# EUROPEAN PATENT APPLICATION

(11) **EP 1 553 091 A1**
(43) Date of publication of application: **13.07.2005**
(21) Application number: 04726765.3
(22) Date of filing: 09.04.2004
(51) Int. Cl.: C07D 263/32, C07D 417/14, C07D 333/20, C07D 413/12, C07D 417/06

(54) **HETEROAROMATIC PENTACYCLIC COMPOUND AND MEDICINAL USE THEREOF**

(30) Priority: 09.04.2003 JP 2003105267; 03.06.2003 JP 2003157590
(71) Applicant: Japan Tobacco Inc., Tokyo 105-8422 (JP)
(72) Inventor: IKEMOTO, Tomoyuki, Takatsuki-shi, Osaka 569-1125 (JP); TANAKA, Masahiro, Takatsuki-shi, Osaka 569-1125 (JP); YUNO, Takeo, Takatsuki-shi, Osaka 569-1125 (JP); SAKAMOTO, Johei, Takatsuki-shi, Osaka 569-1125 (JP); NAKANISHI, Hiroyuki, Takatsuki-shi, Osaka 569-1125 (JP); NAKAGAWA, Yuichi, Takatsuki-shi, Osaka 569-1125 (JP); OHTA, Takeshi, Takatsuki-shi, Osaka 569-1125 (JP); SAKATA, Shohei, Takatsuki-shi, Osaka 569-1125 (JP); MORINAGA, Hisayo, Takatsuki-shi, Osaka 569-1125 (JP)
(74) Representative: von Kreisler, Alek, Dipl.-Chem.
(86) International application number: PCT/JP2004/005119
(87) International publication number: WO 2004/089918

(57) **Abstract**

A 5-membered heteroaromatic ring compound represented by the formula [I] 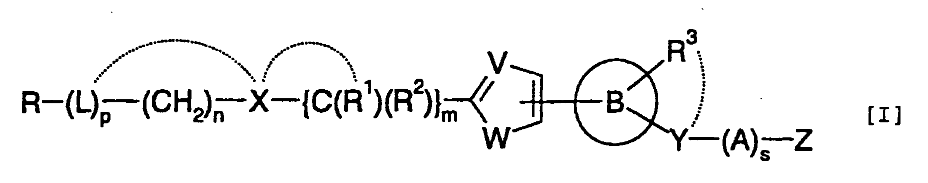 wherein V is CH or N; W is S or O; R¹ and R² are each H etc.; X is -N(R⁴)-, -O-, -S-, -SO₂-N(R⁵)-, -CO-N(R⁷)- etc.; L is 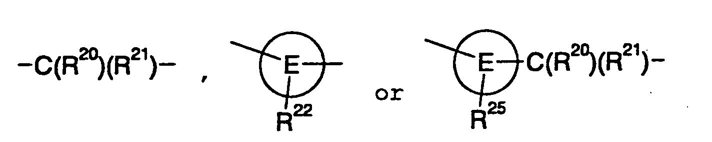 wherein R²⁰, R²¹, R²² and R²⁵ are each H etc.; E is aryl or heteroaromatic ring group; R is -COOH etc.; B is aryl etc.; R³ is H etc.; Y is -C(R¹³)(R¹⁴)-N(R¹²)-, -C(R¹³)(R¹⁴)-O-, -N(R¹¹)-, -O- etc.; A is alkylene; and Z is aryl etc., a prodrug thereof and a pharmaceutically acceptable salt thereof.

The compound [I] has a superior protein tyrosine phosphatase 1B inhibitory activity and is useful as a therapeutic agent for diabetes, diabetic complications, hyperlipidemia, obesity and the like.

## Description

### TECHNICAL FIELD OF THE INVENTION

The present invention relates to a novel 5-membered heteroaromatic ring compound. More particularly, the present invention relates to a 5-membered heteroaromatic ring compound having a protein tyrosine phosphatase 1B (PTP1B) inhibitory activity, a pharmaceutically acceptable salt thereof, and a pharmaceutical composition containing the same.

### BACKGROUND OF THE INVENTION

Diabetes causes various dysbolisms mainly characterized by a chronic hyperglycemic state. It shows a broad range of symptoms based on hyperglycemia, such as mouth dryness, polydipsia, diuresis, loss of body weight and the like. When such a state of hyperglycemia lasts for a long time, it is known to cause all kinds of complications such as retinopathy, nephropathy, neuropathy, cardiac infarction and cerebral infarction based on arteriosclerosis, and the like.

Diabetes is generally classified into four types: type I diabetes (IDDM; insulin dependent diabetes mellitus) accompanying absolute insulin deficiency due to damage or destruction of β cell of pancreas; type II diabetes (NIDDM; non-insulin dependent diabetes mellitus) accompanying relative insulin deficiency due to insulin resistance and decreased insulin secretion; special diabetes secondarily developed by abnormality in gene, other diseases and the like; and gestational diabetes. In some cases, patients diagnosed with type II diabetes shortly after the onset may come to show decreased insulin secretion with the progression of the disease and ultimately lead to type I diabetes.

To treat diabetes, it is essential to improve the above-mentioned hyperglycemic conditions and prevent the onset and progression of complications. For this end, there have been tried diet therapy, exercise therapy and the like, as well as treatments using a variety of pharmaceutical agents with the aim of controlling the blood glucose level to be within the normal range. As the representative existing pharmaceutical agents, there are mentioned insulin preparations, insulin secretagogues (sulfonylurea agent, sulfonamide agent, phenylalanine derivative etc.) that promote insulin secretion from β cell of pancreas, α-glucosidase inhibitors that delay sugar absorption, biguanide agents considered to have a liver glyconeogensis inhibitory action, wherein the detail of the mechanism is unknown, insulin sensitizers (thiazolidinedione derivative etc.) that promote differentiation from fibroblast to adipose cell via a peroxisome proliferator-activated receptor (PPAR) agonist action to increase the number of insulin sensitive cells and the like. However, all these are problematic in terms of effectiveness and safety (e.g., secondary failure due to fatigue of pancreatic β cell, risk of hypoglycemia and promotion of obesity associated with insulin secretagogue, edema and adverse influence on the heart based thereon, anemia, overeating, promotion of obesity, presence of non-responder associated with insulin sensitizer and the like) and they fail to achieve sufficient control of blood glucose.

Referring to glucose metabolism in living organism, materials to be the energy source and constituent components of living organism have been intermittently taken into the body and, for example, brain continuously consumes glucose. In this situation, the blood glucose level is maintained at a constant level, and blood glucose control is enabled by hormone involved in the blood glucose control, metabolism in organ and interaction for exchange of saccharide and the like between organs. Of these, the action of insulin, which is a hormone particularly responsible for the blood glucose control, is significant and disorder thereof, or lower insulin resistance and decreased insulin secretion are considered to be deeply involved in diabetes.

Insulin is secreted from β cell of pancreas, binds with insulin receptor, which is a receptor type tyrosine kinase present on the membrane surface of its target cell or skeletal muscle cell and adipose cell, after which the tyrosine residue in the intracellular domain is self-phosphorylated. Thereafter, the tyrosine residue such as IRS (insulin receptor substrate), APS (adapter protein containing PH and SH2 domain) and the like, which is a substrate of an insulin receptor, is phosphorylated and PI₃ kinase-Akt path is activated, which in turn transfers a glucose transporter onto the cell membrane, causing glucose uptake and decreased blood glucose concentration. On the other hand, tyrosine phosphatase is also present, which performs tyrosine dephosphorylation to negatively control intracellular signaling by insulin, thereby suppressing the activation. As mentioned above, tyrosine phosphorylation plays a major role in the action of insulin. In consideration of the fact that tyrosine phosphorylation is determined by the balance of the activity of tyrosine kinase (phosphorylation enzyme) and that of tyrosine phosphatase (dephosphorylation enzyme), tyrosine phosphatase is considered to directly play a significant role in controlling insulin signaling together with tyrosine kinase.

At present, tyrosine phosphatase forms a large gene family, and more than seven dozen kinds of isozymes have been reported. Of these, protein tyrosine phosphatase 1B (PTP1B) is considered to be a major phosphatase involved in insulin signaling. Particularly, because gene expression of PTP1B increases in high glucose culture and changes in intracellular localization thereof decreases tyrosine phosphorylation of insulin receptor and IRS-1 and induces insulin resistance (J. Biol. Chem., 1995, Vol. 270, pp. 7724-7730; J. Biochem. (Tokyo), 1998, Vol. 123, pp. 813-820), introduction of wild type PTP1B inhibits translocation of glucose transporter GLUT4, such effect was not found in phosphatase activity defective mutants, and insulin sensitivity was enhanced in PTP1B knockout mice and the mice became obesity resistant to a high fat diet (Science, 1999, Vol. 283, pp. 1544-1548), there is a possibility that this enzyme may become one target to enhance the action of from activation of insulin receptor to glucose uptake that insulin is responsible for. In fact, vanadic acid conventionally known as a tyrosine phosphatase inhibitor shows an insulin-like action in animal tests and the like.

Accordingly, a drug that suppresses and/or inhibits activation of such tyrosine phosphatase, particularly PTP1B, enhances the action up to glucose uptake by inhibiting an insulin receptor activated signal from being negatively controlled through dephosphorylation, and can be a new type of therapeutic agent for diabetes, which decreases blood glucose based on direct enhancement of insulin action. In addition, application to various therapeutic agents for diseases such as obesity, neurodegenerative disease and the like is expected.

There are various reports in recent years on compounds aiming at treatment of diseases, such as diabetes and the like, by inhibiting such protein tyrosine phosphatase.

For example, WO00/17211 discloses a phosphonic acid derivative having a PTP1B inhibitory activity. However, this publication does not disclose a compound having a structure of the compound of the present invention, not to mention any description suggestive thereof.

JP-T-11-508919 (U.S. Patent No. 5,770,620) discloses an arylacrylic acid derivative useful as a protein tyrosine phosphatase inhibitor. However, this publication does not disclose a compound having a structure of the compound of the present invention, not to mention any description suggestive thereof.

WO98/27092 (U.S. Patent No. 6,080,772) discloses a thiazole compound having a protein tyrosine phosphatase inhibitory action. However, this publication does not disclose a compound having a structure of the compound of the present invention, not to mention any description suggestive thereof.

WO99/58522 discloses naphtho[2,3-B]heteroar-4-yl derivative, WO99/58511 discloses an oxa/thiazole-aryl-carboxylic acid derivative; WO99/58521 and U.S. Patent No. 6,110,962 disclose a 11-aryl-benzo[B]naphtho[2,3-D]furan and 11-aryl-benzo[B]naphtho[2,3-D]thiophene derivatives; WO99/58518 discloses a biphenyl-oxo-acetic acid derivative; WO99/61419 discloses a 2,3,5-substituted biphenyl derivative; WO99/58520 discloses a biphenyl-sulfonyl-aryl-carboxylic acid derivative; WO99/61435 discloses benzothiophene, benzofuran and indole derivatives; U.S. Patent No. 6,103,708 discloses furan, benzofuran and thiophene derivatives; U.S. Patent No. 6,110,963 discloses an aryl-oxo-acetic acid derivative; U.S. Patent No. 6,001,867 discloses a 1-aryl-dibenzothiophene derivative; U.S. Patent No. 6,057,316 discloses a 4-aryl-1-oxa-9-thia-cyclopenta[B]fluorene derivative; U.S. Patent No. 6,063,815 discloses a benzophenone derivative, each of which having a protein tyrosine phosphatase inhibitory action. However, these publications do not disclose a compound having a structure of the compound of the present invention, not to mention any description suggestive thereof.

As a compound having a thiazole, thiophene or oxazole structure, the following have been reported.

WO00/45635 discloses a 2-substituted thiazole derivative. However, this compound has a carbamoyl group at the terminal of the 2-position substituent of a thiazole ring, and this publication does not disclose a compound having a structure of the compound of the present invention, not to mention any description suggestive thereof.

The compound of this publication is useful as an antibacterial agent or an analgesic agent, and this publication does not disclose usefulness as a PTP1B inhibitor, not to mention any description suggestive thereof.

JP-T-2000-504039 discloses a 2-anilino-4-phenylthiazole derivative. However, the compound of this publication has an anilino group substituted by a hydroxyl group or a carboxyl group at the 2-position of thiazole ring, and phenyl group at the 4-position, wherein the phenyl group at the 4-position has a substituent at the 2-position. This publication does not disclose a compound having a structure of the compound of the present invention, not to mention any description suggestive thereof. In addition, the compound of this publication is useful as a CRF (corticotropin releasing factor) antagonist. This publication does not disclose usefulness as a PTP1B inhibitor, not to mention any description suggestive thereof.

JP-A-4-154773 discloses a thiazole derivative of the formula 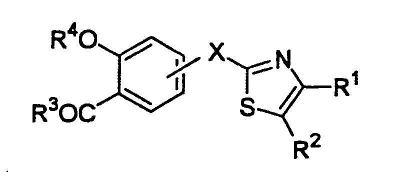 wherein R¹ and R² are the same or different and each is a hydrogen atom, a halogen atom, a lower alkyl group, a phenyl group, a substituted phenyl group, a pyridyl group or a substituted pyridyl group, R³ is a hydroxyl group, a lower alkoxy group or a group represented by -N(R⁵)(R⁶) wherein R⁵ and R⁶ are the same or different and each is a hydrogen atom or a lower alkyl group, R⁴ is a hydrogen atom or a lower alkyl group, and X is an amino group, an amide group, a carbonyl group, an alkylene group, an oxygen atom or a sulfur atom. However, this publication does not disclose a compound having a structure of the compound of the present invention, not to mention any description suggestive thereof. In addition, the compound of this publication is useful as an anti-inflammatory agent. This publication does not disclose usefulness as a PTP1B inhibitor, not to mention any description suggestive thereof.

WO94/08982 discloses a 4-phenylthiazole derivative. However, the compound of this publication has a phenyl group at the 4-position of thiazole ring, and phenyl group at the 4-position, wherein the phenyl group at the 4-position has a substituent at the 2-position. This publication does not disclose a compound having a structure of the compound of the present invention, not to mention any description suggestive thereof. In addition, the compound of this publication is useful as a noxious organism eliminator. This publication does not disclose usefulness as a PTP1B inhibitor, not to mention any description suggestive thereof.

WO02/39997 discloses a compound represented by the formula 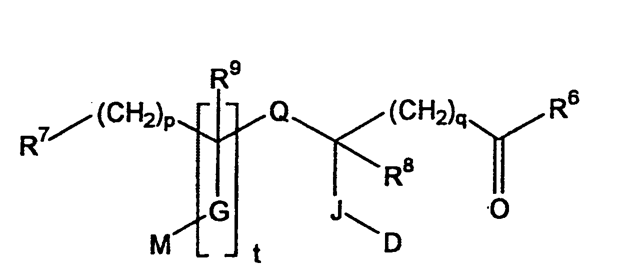 wherein R⁶ is a hydroxyl group or a protective prodrug moiety, R⁷ is a hydrogen atom, a carboxy group, an arylaminocarbonyl group, an aroyl group, an aryl group, an alkylaminocarbonyl group, an aminocarbonyl group, an alkenylaminocarboxy group, a hydroxyl group, an alkoxy group, ether, thiol, an amino group-containing hetero ring group or a protective prodrug moiety, R⁸ is a hydrogen atom or alkyl group that may bond with D to form a ring, R⁹ is a lower alkyl group or a hydrogen atom, Q is a bond, an oxygen atom, a sulfur atom, CR³OH, CR³SH, CR³NR^{3a}R^{3b}, NR³, (CR³R^{3a})ₙ, O(CR³R^{3b})ₙ or (CR³R^{3a})ₙO(CR^{3b}R^{3c})ₙ wherein n is 0 or an integer of 1 to 3, R³, R^{3a}, R^{3b} and R^{3c} are each independently a hydrogen atom, an optionally substituted straight chain, a cyclic or branched chain C₁₋₆ alkyl group, a C₂₋₆ alkenyl group, an acyl group, an arylalkyl group, aryloxycarbonyl group, an arylaminocarbonyl group, an arylalkylsulfonyl group or an aryl group), G is a linking moiety, M is an anchor moiety, J is a bond, an alkylene group, an alkenylene group or an alkynylene group, D is a hydrogen atom, an alkoxy group, amine, an alkyl group, an alkenyl group, an alkynyl group, an aryl group or a heteroaryl group that is bonded with G, M or Q to form a ring, t is 0 or 1, p is 0 or an integer of 1 to 5, and q is 0 or an integer of 1 to 3],
and
a compound represented by the formula 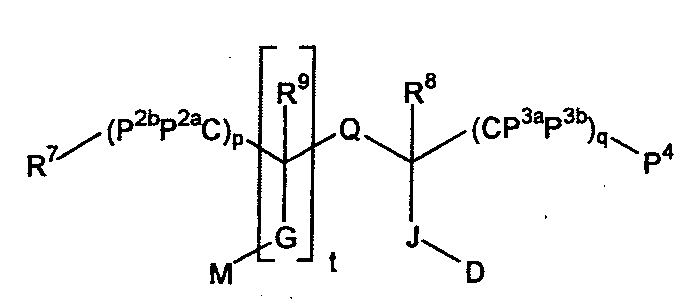 wherein P⁴ is a carboxy group, a cleavable prodrug moiety, COOP^{4'}, (CH₂)₁₋₄SP^{4'} or C(O)NP^{4'}P^{4''}, R⁷ is a hydrogen atom, a carboxy group, an optionally substituted lower alkyl ester, a lower alkenyl ester, an ester added with a secondary amine substituted by lower alkyl, an arylaminocarbonyl group, an aroyl group, an aryl group, an alkylaminocarbonyl group, an aminocarbonyl group, COOR^{7'}, CONR^{7'}R^{7''}, a hydroxyl group, ether, thiol, an amino group, (CH₂)₁₋₄SR^{7'}, a hetero ring group or a cleavable prodrug moiety, P^{4'}, P^{4''}, R^{7'} and R^{7''} are each independently a hydrogen atom, a C₁₋₆ alkyl group, a C₂₋₆ alkenyl group, a C₂₋₆ alkynyl group or an optionally substituted aryl group, R⁸ is a hydrogen atom, an alkyl group or a covalent bond with D, R⁹ is a lower alkyl group or a hydrogen atom, Q is a bond, an oxygen atom, a sulfur atom, CR³OH, CR³SH, CR³NR^{3a}R^{3b}, NR³, (CR³R^{3a})ₙ, O(CR³R^{3b})ₙ or (CR³R^{3a})ₙO(CR^{3b}R^{3c})ₙ wherein n is 0 or an integer of 1 to 3, R³, R^{3a}, R^{3b} and R^{3c} are each independently a hydrogen atom, an optionally substituted C₁₋₆ straight chain or branched chain alkyl group, a C₂₋₆ straight chain or branched chain alkenyl group, an aryloxycarbonyl group, an arylaminocarbonyl group, an arylalkylsulfonyl group, an arylalkyl group, an optionally substituted acyl group, an aryl group or a C₃₋₈ ring optionally substituted by 4 heteroatoms at maximum, P^{2a}, P^{2b}, P^{3a} and P^{3b} are each independently a hydrogen atom or an optionally substituted straight chain, branched chain or cyclic C₁₋₅ alkyl group, G is a linking moiety, M is an anchor moiety, J is a bond, an alkylene group, an alkenylene group or an alkynylene group, D is a hydrogen atom, an alkyl group, an alkenyl group, an alkynyl group or an aryl group or it may be bonded with G, M or Q to form a ring, t is 0 or 1, p is 0 or an integer of 1 to 5, and q is 0 or an integer of 1 to 3 and as examples of the anchor moiety in each formula, a thiazole group and an oxazole group having, as a substituent, an aryl group or a heteroaryl group substituted by -NR'R'', - CONR'R'', -S(O)₂NR'R'', -S(O)₀₋₂R', -NR'R'', -O(CR'R'')₀₋₂CF₃, -COR', -CO₂R' and -OR' wherein R' and R'' are each independently a hydrogen atom, a C₁₋₆ alkyl group, a C₂₋₆ alkenyl group, a C₂₋₆ alkynyl group or an optionally substituted aryl group, and as examples of the linking moiety, a covalent bond and a C₁₋₆ alkyl group are described.

Furthermore, a compound represented by the formula 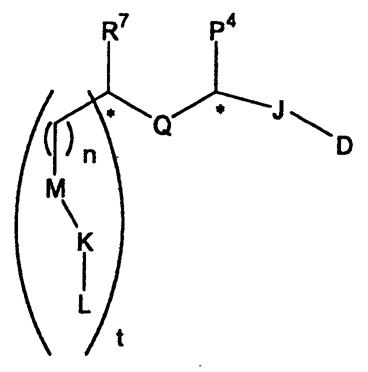 wherein M is a carbon ring group, a hetero ring group or CONR'R" wherein R' and R" are each independently a hydrogen atom, a C₁₋₆ alkyl group, a C₂₋₆ alkenyl group, a C₂₋₆ alkynyl group or an optionally substituted aryl group, Q is a bond, an oxygen atom, a sulfur atom, CR³OH, CR³SH, CR³NR^{3a}R^{3b}, NR³, (CR³R^{3a})ₙ, O(CR³R^{3b})ₙ or (CR³R^{3a})ₙO(CR^{3b}R^{3c})ₙ wherein n is 0 or an integer of 1 to 3, R³, R^{3a}, R^{3b} and R^{3c} are each independently a hydrogen atom, an optionally substituted branched chain, a cyclic or a straight chain C₁₋₆ alkyl group, a C₂₋₆ alkenyl group, an acyl group, an arylalkyl group, an aryloxycarbonyl group, an arylaminocarbonyl group, an arylalkylsulfonyl group or an aryl group, K is an independently selected secondary linking moiety, L is an independently selected secondary anchor moiety, P⁴ is a hydrogen atom, a carboxy group, (CH₂)₁₋₄SP^{4'}, a cleavable prodrug moiety, COOP^{4'} or CONP^{4'}P^{4''}, R⁷ is a hydrogen atom, a carboxy group, an aroyl group, an aryl group, COOR^{7'}, C(O)NR^{7'}R^{7''}, a hydroxyl group, ether, thiol, (CH₂)₁₋₄SR^{7'}, a hetero ring group or a cleavable prodrug moiety, P^{4'}, P^{4''}, R^{7'} and R^{7''} are each independently a hydrogen atom, a C₁₋₆ alkyl group, a C₂₋₆ alkenyl group, a C₂₋₆ alkynyl group or an optionally substituted aryl group, n is 0 or an integer of 1 to 4, D is a hydrogen atom, an alkyl group, an alkoxy group, an alkenyl group, amine, a hydroxyl group, an alkynyl group, an aryl group or a heteroaryl group, and t is 0 or 1, is described and that it is described that the secondary linking moiety has a covalent bond, and the secondary anchor moiety has an optionally substituted aryl group.

However, this publication does not disclose a compound having a structure of the compound of the present invention, not to mention any description suggestive thereof.

In addition, the compound of this publication is useful as an angiotensin converting enzyme (ACE)-2 regulating agent. This publication does not disclose usefulness as a PTP1B inhibitor, not to mention any description suggestive thereof.

As reports on a compound having a thiazole or oxazole structure, which aims at treating not only diabetes but also hyperlipidemia by inhibiting PTP1B, the following can be mentioned.

JP-A-2003-231679 discloses an azole compound represented by the formula 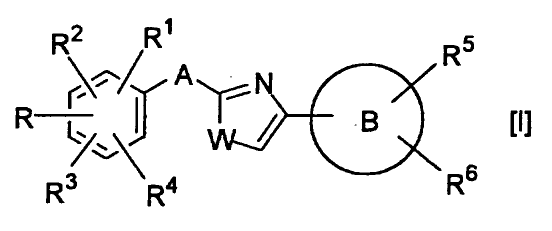 having a PTP1B inhibitory activity, and teaches that this compound is useful as a therapeutic agent for hyperlipidemia. However, the compound of this publication is essentially characterized in that nitrogen atom or carbonyl group at the substituent A should be adjacent to thiazole or carbon atom at the 2-position of oxazole, or A is an alkylene; a bond between A and benzene ring is essential; and nitrogen atom, oxygen atom, sulfur atom or carbonyl group at the substituent R⁵ should be adjacent to ring B, or R⁶ is linked to a ring structure via an alkylene or a single bond is essential, and this publication does not disclose a compound having a structure of the compound of the present invention. In addition, this publication does not describe that the PTP1B inhibitory activity of this compound is higher than the activity of other protein tyrosine phosphatases.

As reports on a method using plural therapeutic drugs for diabetes or other diseases in combination, the following can be mentioned.

JP-A-9-67271 discloses a combination of an insulin sensitizer with an insulin preparation, an insulin secretagogue an α-glucosidase inhibitor, a biguanide agent and the like. However, this publication does not disclose a PTP1B inhibitor or an inhibitor of receptor tyrosine kinase negative regulator such as the compound of the present invention, not to mention any description suggestive thereof.

WO02/100846 discloses a thiophene derivative compound having the formula 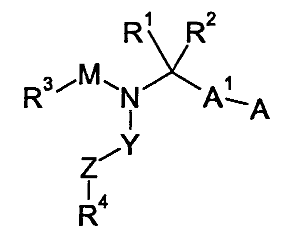 wherein M is selected from -SO₂-, -C=O-, -C=S-, etc.; A¹ is selected from a bond, a C₁₋₆ alkyl, a C₂₋₆ alkenyl, etc.; A is selected from COOR⁵, CO-COOR⁵, PO₃R⁵R⁵, etc., wherein each R⁵ is independently selected from a hydrogen atom and C₁₋₆ alkyl; R¹ and R² are independently selected from a hydrogen atom, a C₁₋₆ alkyl, a C₆₋₁₂ aryl, etc.; R³ is selected from a C₆₋₁₂ aryl, a C₃₋₁₀ heterocycle, a C₆₋₁₂ aralkyl, etc.; Y is selected from a bond, -CH₂-, -CO-, etc.; Z is selected from a C₁₋₆ alkyl, a C₂₋₆ alkenyl, a C₂₋₆ alkynyl, etc.; R⁴ is selected from a hydrogen atom, halogen, CN, NO₂, etc.; or a salt thereof.

WO 02/34711 discloses a thiophene derivative compound having the formula 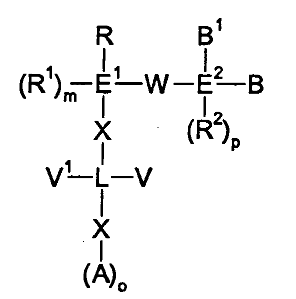 wherein
E¹ and L are each independently selected from a 5- to 7-membered saturated or unsaturated carbon ring, a 5- to 7-membered saturated or unsaturated hetero ring, a bicyclic saturated or unsaturated carbon ring, etc.; R is selected from -CH=CH-R², -C≡C-R², -C(R²)=CH, etc.; R¹ is selected from a hydrogen atom, -R, -NO₂, etc.; m is 1 or higher; R² is selected from a hydrogen atom, a halogen, an alkyl, etc.; W is selected from a direct bond, -CHR²-, -CH=CR²-, etc.; E² is selected from a 5- to 7- membered saturated or unsaturated carbon ring, a 5-to 7- membered saturated or unsaturated hetero ring, a bicyclic ring system, etc.; each X is independently selected from a direct bond, a substituted or unsubstituted C₁₋₄ methylene chain, O, etc., wherein said X at different places may be the same or different; B is selected from a hydrogen atom, -halo, -CN, etc.; B¹ is selected from B, wherein said B¹ and B may be the same or different; p is 1 or higher depending upon the size of the ring; A is selected from R¹ when o is 1, except that when L is a cyclic ring of more than 5 atoms, o is 1 or higher depending upon the size of the ring; V and V¹ are each independently selected from R¹ and N-alkyl substituted carboxamidyl (CONHR) wherein the alkyl group may be straight, branched, cyclic, or bicyclic, etc.; and n is 0 to 4.

WO 98/28264 discloses a thiophene derivative compound having the following formula 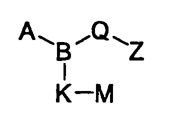 wherein B is N; A is selected from a C₁₋₆ alkylsulfonyl, a C₃₋₇ cycloalkylsulfonyl, a C₃₋₇ cycloalkyl-C₁₋₆ alkylsulfonyl, each of which is optionally mono-, di- or tri- substituted with a hydroxy group, a C₁₋₄ alkyl or a halogen atom; Q is a -C₂₋₈ alkylene-W-C₁₋₃ alkylene- or a -C₃₋₈ alkylene-, said -C₃₋₈ alkylene- is optionally substituted with up to four substituents independently selected from a fluorine atom, a C₁₋₄ alkyl, -X-C₁₋₅ alkylene-, etc., wherein X is a 5- or 6- membered aromatic ring optionally having one or two heteroatoms selected independently from an oxygen atom, a nitrogen atom and a sulfur atom; Z is carboxyl, a C₁₋₆ alkoxycarbonyl, tetrazolyl, etc.; K is a bond, a C₁₋₈ alkylene, a thio(C₁₋₄)alkylene, etc., wherein said C₁₋₈ alkylene is optionally mono-unsaturated and said K is optionally mono-, di- or tri-substituted with a fluorine atom, methyl or chlorine atom; and M is -Ar, -Ar¹-V-AP, -Ar¹-S-AP, etc., wherein Ar, Ar¹ and AP are each independently selected from a partially saturated, fully saturated or fully unsaturated 5- to 8- membered ring optionally having one to four heteroatoms selected from an oxygen atom, a sulfur atom and a nitrogen atom, etc.; or a pharmaceutically acceptable salt thereof or a prodrug thereof.

Furthermore, WO 01/26656 (JP-A-2003-511416) discloses a compound having the following formula 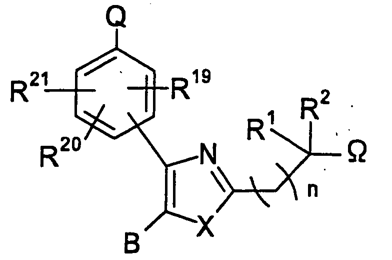 wherein Ω is -NR⁴⁶R⁴⁷ or -OR⁴⁸, wherein R⁴⁶ and R⁴⁷ are each independently selected from a hydrogen atom, an alkyl, a cycloalkyl, etc., and R⁴⁸ is selected from a hydrogen atom, an alkyl, an alkynyl, etc.; B is a hydrogen atom or an alkyl; Q is selected from a hydrogen atom, -OR²², -SR²², etc., wherein R²² is selected from a hydrogen atom, an alkyl, an aryl optionally substituted with an alkyl, a hydroxyl group, a halogen atom, etc., etc.; R¹⁹, R²⁰ and R²¹ are each independently selected from a hydrogen atom, a halogen atom, a hydroxy group, etc.; X is NH or S; N is 0 to 6; R¹ and R² are each selected from a hydrogen atom, an alkyl and a cycloalkyl.

WO 97/30053 discloses a compound having the following formula 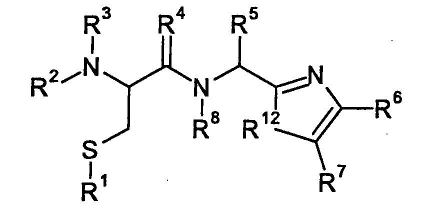 wherein R¹ is a hydrogen atom, a lower alkyl, a cycloalkylthio or a lower alkylthio, and R² and R³ are each independently a hydrogen atom or a lower alkyl; or R¹ and R² may form -CH₂-, -CO- or -C(CH₃)₂-; R⁴ is H2 or O; R⁵ is selected from a hydrogen atom, a substituted or unsubstituted lower alkyl, a lower alkenyl, etc.; R⁶ and R⁷ are each independently selected from a hydrogen atom, -C(O)NHCHR¹³CO₂R¹⁴, a substituted or unsubstituted lower alkyl, etc., wherein R¹³ is selected from a substituted or unsubstituted lower alkyl, etc., and R¹⁴ is a hydrogen atom or lower alkyl, or R⁶ and R⁷ may form an aryl or a heterocyclyl; R⁸ and R⁹ are each independently selected from a hydrogen atom, a substituted or unsubstituted lower alkyl, a cycloalkyl, etc.; and R¹² is NR⁹, S or O; or a pharmaceutically acceptable salt thereof.

WO 99/21555 and JP-A-2000-302680 disclose a compound having the following formula 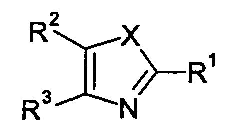 wherein R¹ is selected from a hydrogen atom, a hydrocarbon group which may be substituted, a heterocyclic group which may be substituted, etc.; at least one of R² and R³ is a hydrogen atom, a pyridyl which may be substituted or an aromatic hydrocarbon group which may be substituted, and the other is a pyridyl which may be substituted; and X is a sulfur atom which may be oxidized, an oxygen atom or a group represented by the formula NR⁴, wherein R⁴ is a hydrogen atom, a hydrocarbon group which may be substituted or an acyl; or a salt thereof, which may be N-oxidized.

JP-A-5-202040 discloses a compound having the following formula 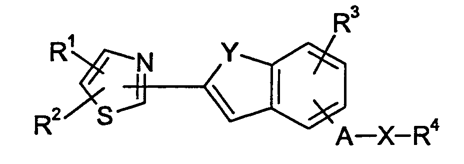 wherein R¹ is an alkyl which may be substituted, or a cycloalkyl, tricycloalkyl or arylheterocyclic group which may be substituted; and R² is a hydrogen atom or a halogen atom, or R¹ and R², together with the adjacent atom, form a cycloalkene ring or an N-containing heterocyclic ring; R³ is selected from a hydrogen atom, a halogen atom, OH, etc.; R⁴ is selected from a hydrogen atom, an acyl group, CN, etc.; A is an alkylene, an alkenylene or a single bond; X is a single bond, O or S; and Y is O or S, or a salt thereof.

However, these publications do not disclose a compound having a structure of the compound of the present invention, not to mention any description suggestive thereof.

### SUMMARY OF THE INVENTION

The present invention aims at providing a compound having a superior selective PTP1B inhibitory activity and an inhibitory activity of receptor tyrosine kinase negative regulator based thereon, which is useful as a therapeutic agent for diabetes, a therapeutic agent for hyperlipidemia, obesity or diabetic complication, and a therapeutic agent for a disease such as neurodegenerative disease and the like.

It is an object of the present invention to provide a PTP1B inhibitor, a therapeutic agent for diabetes, a therapeutic agent for hyperlipidemia, a therapeutic agent for obesity or a therapeutic agent for diabetic complication.

The present inventors have conducted intensive studies with the aim of achieving the above-mentioned object and found that a 5-membered heteroaromatic ring compound represented by the following formula [I] has a high PTP1B inhibitory activity but a lower inhibitory activity against other protein tyrosine phosphatases, namely, a superior selective PTP1B inhibitory activity absent in conventional compounds, and useful as a PTP1B inhibitor, a therapeutic agent for diabetes, a therapeutic agent for hyperlipidemia, a therapeutic agent for obesity or a therapeutic agent for diabetic complication, which resulted in the completion of the present invention.

The present invention relates to compounds represented by the following [1] to [113] and pharmaceutical use thereof.
[1] A 5-membered heteroaromatic ring compound represented by the formula [I] 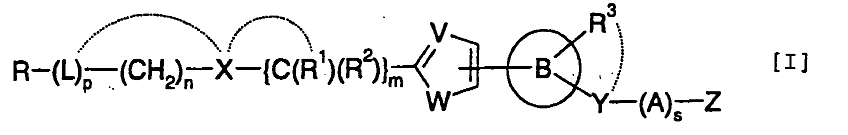 wherein
   V is =N- or =CH-;
   W is -S- or -O-;
   m is 0, 1 or 2;
   R¹ and R² are each independently a hydrogen atom or a C₁₋₄ alkyl group;
   X is -N(R⁴)-, -N(R⁵)-CO-O-, -SO₂-N(R⁵)-, -N(R⁵)-SO₂-, -N(R⁶)-SO₂-N(R⁵)-, -CO-N (R⁷) -, -N (R⁸)-CO-, -N (R⁹)-CO-N (R⁵) -, -N (R¹⁰)-(CH₂)ₖ-N(R⁵)-, -N(R¹⁰)-(CH₂)ₖ-N(R⁵)-CO-, -C(R¹⁰)=N-N(R⁷)-, -N(R¹⁰)-(CH₂)ₖ-CH(R⁶)-, -O-, -S- or -SO₂-
   wherein
   k is 0 or an integer of 1 to 4,
   R⁴, R⁵, R⁶, R⁷, R⁸, R⁹ and R¹⁰ are each independently
   (1) a hydrogen atom,
   (2) a C₁₋₆ alkyl group
      wherein said C₁₋₆ alkyl group is optionally substituted by
      (a) an optionally substituted aryl group,
      (b) an optionally substituted heteroaromatic ring group,
      (c) a carboxy group,
      (d) a C₁₋₄ alkoxycarbonyl group,
      (e) -CO-N(R¹⁵)(R¹⁶)
         wherein R¹⁵ and R¹⁶ are each independently a hydrogen atom, an optionally substituted aryl group, an optionally substituted heteroaromatic ring group, a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group, or may form an indoline ring together with the nitrogen atom bonded thereto or may form a 5- to 7-membered hetero ring optionally containing at least one heteroatom selected from the group consisting of nitrogen atom, oxygen atom and sulfur atom),
      (f) -N(R¹⁵)(R¹⁶)
         wherein R¹⁵ and R¹⁶ are as defined above,
      (g) -O-R¹⁷
         wherein R¹⁷ is a hydrogen atom, an optionally substituted aryl group, an optionally substituted heteroaromatic ring group or a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group,
      (h) -CO-R¹⁷
         wherein R¹⁷ is as defined above,
      (i) -SO₂-R¹⁷
         wherein R¹⁷ is as defined above or
      (j) a C₃₋₇ cycloalkyl group,
   (3) -CO-N(R¹⁵)(R¹⁶)
      wherein R¹⁵ and R¹⁶ are as defined above,
   (4) -SO₂-N(R¹⁵)(R¹⁶)
      wherein R¹⁵ and R¹⁶ are as defined above,
   (5) -CO-R¹⁷
      wherein R¹⁷ is as defined above,
   (6) -SO₂-R¹⁷
      wherein R¹⁷ is as defined above,
   (7) an optionally substituted aryl group,
   (8) an optionally substituted heteroaromatic ring group, or
   (9) R⁴ and R¹ are optionally linked to form 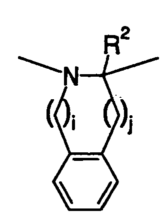 wherein i and j are each independently 0, 1 or 2,
   (10) R⁵ and R⁹ are optionally linked to form 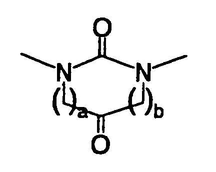 wherein a and b are each independently 0, 1 or 2,
   (11) R⁵ and R¹⁰ are optionally linked to form 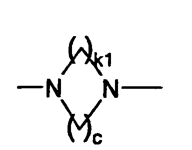 wherein k1 and c are each independently 0 or an integer of 1 to 4,
   (12) R⁵ and R¹⁰ are optionally linked to form 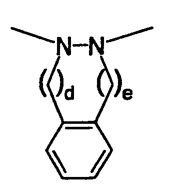 wherein d and e are each independently 0, 1 or 2,
   (13) R⁶ and R¹⁰ are optionally linked to form 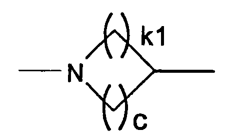 wherein k1 and c are as defined above, or
   (14) R⁷ and R¹⁰ are optionally linked to form 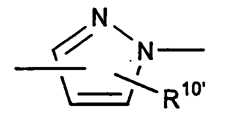 wherein R^{10'} is a hydrogen atom or a C₁₋₆ alkyl group;
      n is 0 or an integer of 1 to 4;
      p is 0 or 1;
      L is 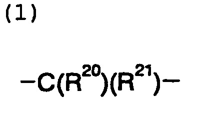 wherein
      R²⁰ is
      (a) a hydrogen atom,
      (b) a C₁₋₆ alkyl group, or
      (c) optionally linked with R⁴ or R⁸ to form 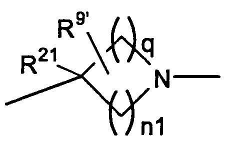 wherein n1 and q are each independently 0 or an integer of 1 to 4, R^{9'} is a hydrogen atom, a hydroxyl group, a C₁₋₆ alkyl group, a carboxy group or a C₁₋₆ alkoxy group, or
      (d) optionally linked with R⁴ to form 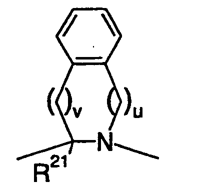
      wherein u and v are each independently 0, 1 or 2,
      R²¹ is a hydrogen atom, a C₁₋₆ alkyl group wherein said C₁₋₆ alkyl group is optionally substituted by an optionally substituted aryl group or an optionally substituted heteroaromatic ring group, an optionally substituted aryl group or an optionally substituted heteroaromatic ring group, 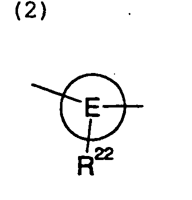 wherein
      E is an aryl group or a heteroaromatic ring group,
      R²² is
      (a) a hydrogen atom,
      (b) a halogen atom,
      (c) a C₁₋₄ alkyl group,
      (d) a C₁₋₄ alkoxy group optionally substituted by a carboxy group,
      (e) a C₁₋₆ alkylthio group,
      (f) a nitro group,
      (g) -N(R²³)(R²⁴)
         wherein R²³ and R²⁴ are each independently a hydrogen atom, a C₁₋₆ alkyl group, a C₁₋₄ alkylcarbonyl group wherein said C₁₋₄ alkylcarbonyl group is optionally substituted by an amino group, a C₁₋₄ alkylamino group or a di(C₁₋₄ alkyl) amino group, or a C₁₋₄ alkylsulfonyl group, or
      (h) optionally linked with R⁴ to form 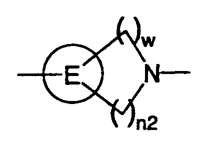 wherein n2 and w are each independently 0 or an integer of 1 to 3,
      (i) optionally linked with R⁴ to form 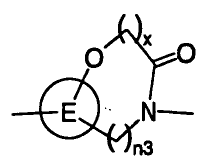 wherein n3 and x are each independently 0 or 1,
      (j) optionally linked with R⁷ to form 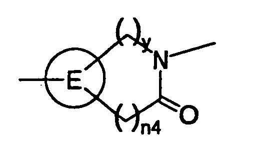 wherein n4 and y are each independently 0, 1 or 2,
      (k) optionally linked with R⁷ to form 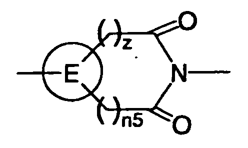 wherein n5 and z are each independently 0 or 1,
      (l) optionally linked with R⁸ to form 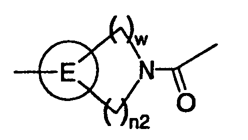 wherein n2 and w are each independently 0 or an integer of 1 to 3, or
      (m) optionally linked with R⁴ to form 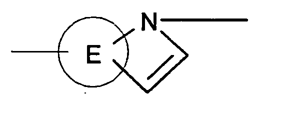 or 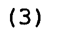 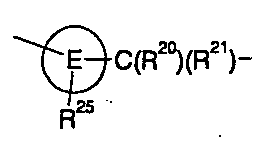
      wherein
      R²⁰, R²¹ and E are as defined above,
      R²⁵ is
      (a) a hydrogen atom,
      (b) a halogen atom,
      (c) a C₁₋₄ alkyl group,
      (d) a C₁₋₄ alkoxy group optionally substituted by a carboxy group,
      (e) a C₁₋₆ alkylthio group,
      (f) a nitro group or
      (g) -N (R²³)(R²⁴)
   wherein R²³ and R²⁴ are as defined above;
   R is -COO(R¹⁹), -A¹-COO(R¹⁹) or -O-A¹-COO(R¹⁹)
   wherein A¹ is a C₁₋₄ alkylene group and R¹⁹ is a hydrogen atom or a C₁₋₄ alkyl group;
   B is an aryl group or a heteroaromatic ring group;
   R³ is
   (1) a hydrogen atom,
   (2) a halogen atom,
   (3) a C₁₋₈ alkyl group,
   (4) a C₁₋₆ alkoxy group,
   (5) a C₁₋₆ alkylamino group,
   (6) a di(C₁₋₆ alkyl) amino group,
   (7) a cyano group,
   (8) a nitro group,
   (9) a C₁₋₄ haloalkyl group,
   (10) -S-R¹⁸ wherein R¹⁸ is a C₁₋₆ alkyl group or an aryl group,
   (11) -SO-R¹⁸ wherein R¹⁸ is a C₁₋₆ alkyl group or an aryl group,
   (12) -SO₂-R¹⁸ wherein R¹⁸ is a C₁₋₆ alkyl group or an aryl group,
   (13) an aryl group or
   (14) a heterocyclic group;
      Y is -O-, -S-, -SO-, -SO₂-, -N(R¹¹)-, -N(R¹²)-CO-, -N(R¹²)-SO₂-, -SO₂-N(R¹²)-, -C(R¹³)(R¹⁴)-, -CO-, -C(R¹³)(R¹⁴)-N(R¹²)-, -CO-N(R¹²)-or -C(R¹³)(R¹⁴)-O-
   wherein
   R¹¹ is
   (1) a hydrogen atom,
   (2) a C₁₋₈ alkyl group
      wherein said C₁₋₈ alkyl group is optionally substituted by substituent(s) selected from the group consisting of
      (a) a C₃₋₇ cycloalkyl group,
      (b) an optionally substituted aryl group,
      (c) an optionally substituted heterocyclic group,
      (d) a hydroxyl group,
      (e) a C₁₋₄ alkylamino group and
      (f) a di(C₁₋₄ alkyl) amino group,
   (3) a C₂₋₄ alkenyl group,
   (4) a C₁₋₄ alkylsulfonyl group,
   (5) a C₁₋₄ alkylcarbonyl group
      wherein said C₁₋₄ alkylcarbonyl group is optionally substituted by a hydroxyl group or a C₁₋₄ alkoxy group, or
   (6) optionally linked with R³ to form 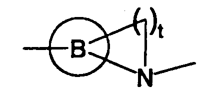 wherein t is an integer of 1 to 4,
   R¹² is
   (1) a hydrogen atom,
   (2) a C₁₋₈ alkyl group
      wherein said C₁₋₈ alkyl group is optionally substituted by substituent(s) selected from the group consisting of
      (a) a C₃₋₇ cycloalkyl group,
      (b) an optionally substituted aryl group,
      (c) an optionally substituted heterocyclic group,
      (d) a hydroxyl group,
      (e) a C₁₋₄ alkylamino group and
      (f) a di(C₁₋₄ alkyl)amino group,
   (3) a C₂₋₄ alkenyl group,
   (4) a C₁₋₄ alkylsulfonyl group or
   (5) a C₁₋₄ alkylcarbonyl group
   wherein said C₁₋₄ alkylcarbonyl group is optionally substituted by a hydroxyl group or a C₁₋₄ alkoxy group,
   R¹³ and R¹⁴ are each independently a hydrogen atom, a C₁₋₄ alkyl group, or optionally form a C₃₋₇ cycloalkane together with the carbon atom bonded thereto, or optionally form, together with the carbon atom bonded thereto, a 5- to 7-membered hetero ring optionally having at least one heteroatom selected from the group consisting of nitrogen atom, oxygen atom and sulfur atom, provided that,
   when m is 0, p is 1 and L is 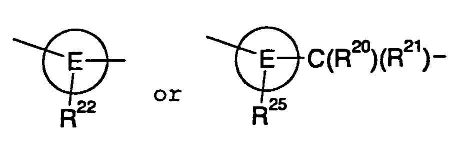 wherein R²⁰ and R²¹ are each a hydrogen atom, E is a phenyl group, R²² is a hydrogen atom, a halogen atom, a C₁₋₄ alkyl group, a C₁₋₄ alkoxy group or a nitro group, R²⁵ is a hydrogen atom, a halogen atom, a C₁₋₄ alkyl group, a C₁₋₄ alkoxy group or a nitro group,
   Y should be
   -C(R¹³)(R¹⁴)-N(R¹²)-, -CO-N(R¹²)- or -C(R¹³)(R¹⁴)-O- wherein R¹², R¹³ and R¹⁴ are as defined above;
   s is 0 or 1;
   A is a C₁₋₄ alkylene group optionally substituted by a C₃₋₇ cycloalkyl group;
   Z is
   (1) a C₃₋₇ cycloalkyl group
      wherein said C₃₋₇ cycloalkyl group is optionally substituted by an aryl group wherein said aryl group is optionally substituted by a halogen atom or a C₁₋₆ alkyl group, or a heteroaromatic ring group wherein said heteroaromatic ring group is optionally substituted by a halogen atom or a C₁₋₆ alkyl group,
   (2) an aryl group
      wherein said aryl group is optionally substituted by substituent(s) selected from the group consisting of
      (a) a heterocyclic group optionally substituted by a C₁₋₄ alkyl group or a C₁₋₄ alkylcarbonyl group,
      (b) a C₃₋₇ cycloalkyl group optionally substituted by a hydroxyl group, an oxo group, a halogen atom or a C₁₋₆ alkyl group,
      (c) a carboxy group,
      (d) a halogen atom,
      (e) a C₁₋₈ alkyl group,
      (f) a C₁₋₄ haloalkyl group,
      (g) a C₁₋₄ alkylamino group,
      (h) a di(C₁₋₄ alkyl) amino group,
      (i) a C₁₋₆ alkylthio group,
      (j) a C₁₋₄ alkoxy group,
      (k) a C₁₋₄ alkylcarbonyl group and
      (l) a nitro group,
   (3) a heteroaromatic ring group
      wherein said heteroaromatic ring group is optionally substituted by substituent(s) selected from the group consisting of
      (a) a heterocyclic group optionally substituted by a C₁₋₄ alkyl group or a C₁₋₄ alkylcarbonyl group,
      (b) a C₃₋₇ cycloalkyl group optionally substituted by a hydroxyl group, an oxo group, a halogen atom or a C₁₋₆ alkyl group,
      (c) a carboxy group,
      (d) a halogen atom,
      (e) a C₁₋₈ alkyl group,
      (f) a C₁₋₄ haloalkyl group,
      (g) a C₁₋₄ alkylamino group,
      (h) a di(C₁₋₄ alkyl) amino group,
      (i) a C₁₋₆ alkylthio group,
      (j) a C₁₋₄ alkoxy group,
      (k) a C₁₋₄ alkylcarbonyl group and
      (l) an aryl group optionally substituted by a halogen atom or a C₁₋₄ haloalkyl group,
   (4) an indanyl group or
   (5) a piperazinyl group
      wherein said piperazinyl group is optionally substituted by substituent(s) selected from the group consisting of
      (a) a phenyl group,
      (b) a phenyl C₁₋₄ alkyl group,
      (c) a benzoyl group optionally substituted by a halogen atom and
      (d) a phenyl C₁₋₄ alkoxycarbonyl group or a prodrug thereof, or a pharmaceutically acceptable salt thereof.
[2] The 5-membered heteroaromatic ring compound of [1], which is represented by the formula [I] 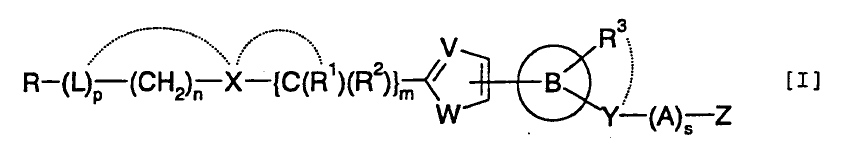 wherein
   V is =N- or =CH-;
   W is -S- or -O-;
   m is 0, 1 or 2;
   R¹ and R² are each independently a hydrogen atom or a C₁₋₄ alkyl group;
   X is -N(R⁴)-, -N(R⁵)-CO-O-, -SO₂-N(R⁵)-, -N(R⁵)-SO₂-, -N (R⁶)-SO₂-
   N(R⁵)-, -CO-N (R⁷)-, -N (R⁸)-CO-, -N (R⁹)-CO-N (R⁵)-, -N (R¹⁰)-(CH₂)ₖ-
   N(R⁵)-, -O-, -S- or -SO₂-
   wherein
   k is 0 or an integer of 1 to 4,
   R⁴, R⁵, R⁶, R⁷, R⁸, R⁹ and R¹⁰ are each independently
   (1) a hydrogen atom,
   (2) a C₁₋₆ alkyl group
      wherein said C₁₋₆ alkyl group is optionally substituted by
      (a) an optionally substituted aryl group,
      (b) an optionally substituted heteroaromatic ring group,
      (c) a carboxy group,
      (d) a C₁₋₄ alkoxycarbonyl group,
      (e) -CO-N(R¹⁵)(R¹⁶)
         wherein R¹⁵ and R¹⁶ are each independently a hydrogen atom, an optionally substituted aryl group, an optionally substituted heteroaromatic ring group, a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group, or may form an indoline ring together with the nitrogen atom bonded thereto or may form a 5- to 7-membered hetero ring optionally containing at least one heteroatom selected from the group consisting of nitrogen atom, oxygen atom and sulfur atom),
      (f) -N(R¹⁵)(R¹⁶)
         wherein R¹⁵ and R¹⁶ are as defined above,
      (g) -O-R¹⁷
         wherein R¹⁷ is a hydrogen atom, an optionally substituted aryl group, an optionally substituted heteroaromatic ring group or a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group,
      (h) -CO-R¹⁷
         wherein R¹⁷ is as defined above,
      (i) -SO₂-R¹⁷
         wherein R¹⁷ is as defined above or
      (j) a C₃₋₇ cycloalkyl group,
   (3) -CO-N(R¹⁵) (R¹⁶)
      wherein R¹⁵ and R¹⁶ are as defined above,
   (4) -SO₂-N(R¹⁵)(R¹⁶)
      wherein R¹⁵ and R¹⁶ are as defined above,
   (5) -CO-R¹⁷
      wherein R¹⁷ is as defined above,
   (6) -SO₂-R¹⁷
      wherein R¹⁷ is as defined above,
   (7) an optionally substituted aryl group,
   (8) an optionally substituted heteroaromatic ring group, or
   (9) R⁴ and R¹ are optionally linked to form 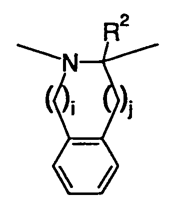 wherein i and j are each independently 0, 1 or 2,
   (10) R⁵ and R⁹ are optionally linked to form 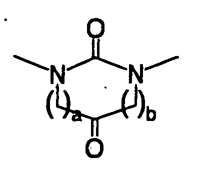 wherein a and b are each independently 0, 1 or 2,
   (11) R⁵ and R¹⁰ are optionally linked to form 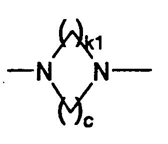 wherein k1 and c are each independently 0 or an integer of 1 to 4, or
   (12) R⁵ and R¹⁰ are optionally linked to form 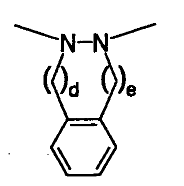 wherein d and e are each independently 0, 1 or 2;
      n is 0 or an integer of 1 to 4;
      p is 0 or 1;
      L is 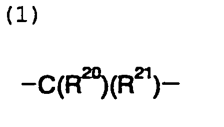 wherein
      R²⁰ is
      (a) a hydrogen atom,
      (b) a C₁₋₆ alkyl group, or
      (c) optionally linked with R⁴ to form 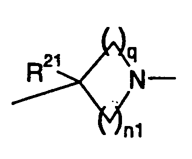 wherein n1 and q are each independently 0 or an integer of 1 to 4, or
      (d) optionally linked with R⁴ to form 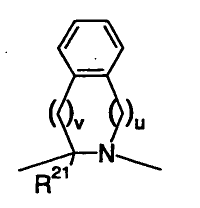
      wherein u and v are each independently 0, 1 or 2,
      R²¹ is a hydrogen atom, a C₁₋₆ alkyl group wherein said C₁₋₆ alkyl group is optionally substituted by an optionally substituted aryl group or an optionally substituted heteroaromatic ring group, an optionally substituted aryl group or an optionally substituted heteroaromatic ring group, 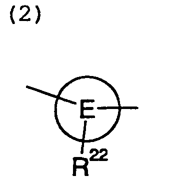 wherein
      E is an aryl group or a heteroaromatic ring group,
      R²² is
      (a) a hydrogen atom,
      (b) a halogen atom,
      (c) a C₁₋₄ alkyl group,
      (d) a C₁₋₄ alkoxy group optionally substituted by a carboxy group,
      (e) a C₁₋₆ alkylthio group,
      (f) a nitro group,
      (g) -N(R²³)(R²⁴)
         wherein R²³ and R²⁴ are each independently a hydrogen atom, a C₁₋₆ alkyl group, a C₁₋₄ alkylcarbonyl group wherein said C₁₋₄ alkylcarbonyl group is optionally substituted by an amino group, a C₁₋₄ alkylamino group or a di(C₁₋₄ alkyl)amino group, or a C₁₋₄ alkylsulfonyl group, or
      (h) optionally linked with R⁴ to form 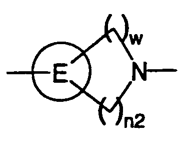 wherein n2 and w are each independently 0 or an integer of 1 to 3,
      (i) optionally linked with R⁴ to form 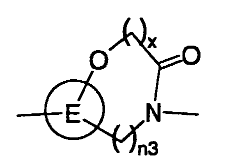 wherein n3 and x are each independently 0 or 1,
      (j) optionally linked with R⁷ to form 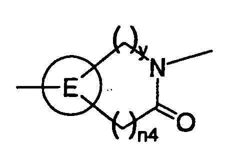 wherein n4 and y are each independently 0, 1 or 2,
      (k) optionally linked with R⁷ to form 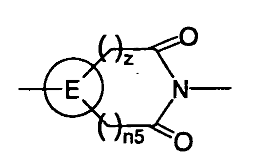 wherein n5 and z are each independently 0 or 1, or
      (l) optionally linked with R⁸ to form 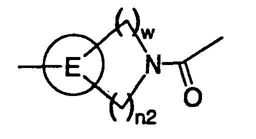
      wherein n2 and w are each independently 0 or an integer of 1 to 3, or 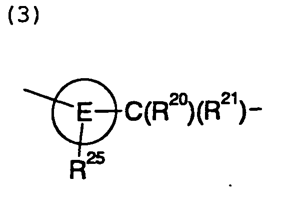 wherein
      R²⁰, R²¹ and E are as defined above,
      R²⁵ is
      (a) a hydrogen atom,
      (b) a halogen atom,
      (c) a C₁₋₄ alkyl group,
      (d) a C₁₋₄ alkoxy group optionally substituted by a carboxy group,
      (e) a C₁₋₆ alkylthio group,
      (f) a nitro group or
      (g) -N(R²³)(R²⁴)
      wherein R²³ and R²⁴ are as defined above;
   R is -COO(R¹⁹), -A¹-COO(R¹⁹) or -O-A¹-COO(R¹⁹)
   wherein A¹ is a C₁₋₄ alkylene group and R¹⁹ is a hydrogen atom or a C₁₋₄ alkyl group;
   B is an aryl group or a heteroaromatic ring group;
   R³ is
   (1) a hydrogen atom,
   (2) a halogen atom,
   (3) a C₁₋₈ alkyl group,
   (4) a C₁₋₆ alkoxy group,
   (5) a C₁₋₆ alkylamino group,
   (6) a di(C₁₋₆ alkyl)amino group,
   (7) a cyano group,
   (8) a nitro group,
   (9) a C₁₋₄ haloalkyl group,
   (10) -S-R¹⁸ wherein R¹⁸ is a C₁₋₆ alkyl group or an aryl group,
   (11) -SO-R¹⁸ wherein R¹⁸ is a C₁₋₆ alkyl group or an aryl group, or
   (12) -SO₂-R¹⁸ wherein R¹⁸ is a C₁₋₆ alkyl group or an aryl group; Y is -O-, -S-, -SO-, -SO₂-, -N(R¹¹)-, -N(R¹²)-CO-, -N(R¹²)-SO₂-, -SO₂-N(R¹²)-, -C(R¹³)(R¹⁴)-, -CO-, -C(R¹³) (R¹⁴)-N(R¹²)-, -CO-N(R¹²)- or -C(R¹³)(R¹⁴)-O-
   wherein
   R¹¹ is
   (1) a hydrogen atom,
   (2) a C₁₋₈ alkyl group
      wherein said C₁₋₈ alkyl group is optionally substituted by substituent(s) selected from the group consisting of
      (a) a C₃₋₇ cycloalkyl group,
      (b) an optionally substituted aryl group,
      (c) an optionally substituted heterocyclic group,
      (d) a hydroxyl group,
      (e) a C₁₋₄ alkylamino group and
      (f) a di(C₁₋₄ alkyl) amino group,
   (3) a C₂₋₄ alkenyl group,
   (4) a C₁₋₄ alkylsulfonyl group,
   (5) a C₁₋₄ alkylcarbonyl group
      wherein said C₁₋₄ alkylcarbonyl group is optionally substituted by a hydroxyl group or a C₁₋₄ alkoxy group, or
   (6) optionally linked with R³ to form 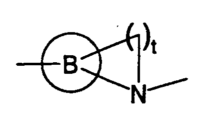
   wherein t is an integer of 1 to 4,
   R¹² is
   (1) a hydrogen atom,
   (2) a C₁₋₈ alkyl group
      wherein said C₁₋₈ alkyl group is optionally substituted by substituent(s) selected from the group consisting of
      (a) a C₃₋₇ cycloalkyl group,
      (b) an optionally substituted aryl group,
      (c) an optionally substituted heterocyclic group,
      (d) a hydroxyl group,
      (e) a C₁₋₄ alkylamino group and
      (f) a di(C₁₋₄ alkyl)amino group,
   (3) a C₂₋₄ alkenyl group,
   (4) a C₁₋₄ alkylsulfonyl group or
   (5) a C₁₋₄ alkylcarbonyl group
   wherein said C₁₋₄ alkylcarbonyl group is optionally substituted by a hydroxyl group or a C₁₋₄ alkoxy group,
   R¹³ and R¹⁴ are each independently a hydrogen atom, a C₁₋₄ alkyl group, or optionally form a C₃₋₇ cycloalkane together with the carbon atom bonded thereto, or optionally form, together with the carbon atom bonded thereto, a 5- to 7-membered hetero ring optionally having at least one heteroatom selected from the group consisting of nitrogen atom, oxygen atom and sulfur atom, provided that,
   when m is 0, p is 1 and L is 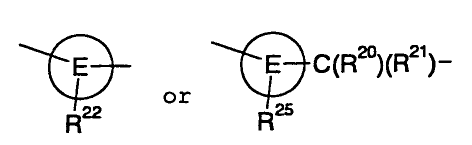 wherein R²⁰ and R²¹ are each a hydrogen atom, E is a phenyl group, R²² is a hydrogen atom, a halogen atom, a C₁₋₄ alkyl group, a C₁₋₄ alkoxy group or a nitro group, R²⁵ is a hydrogen atom, a halogen atom, a C₁₋₄ alkyl group, a C₁₋₄ alkoxy group or a nitro group,
   Y should be
   -C(R¹³)(R¹⁴)-N(R¹²)-, -CO-N(R¹²)- or -C(R¹³)(R¹⁴)-O- wherein R¹², R¹³ and R¹⁴ are as defined above;
   s is 0 or 1;
   A is a C₁₋₄ alkylene group optionally substituted by a C₃₋₇ cycloalkyl group;
   Z is
   (1) a C₃₋₇ cycloalkyl group
      wherein said C₃₋₇ cycloalkyl group is optionally substituted by an aryl group wherein said aryl group is optionally substituted by a halogen atom or a C₁₋₆ alkyl group, or a heteroaromatic ring group wherein said heteroaromatic ring group is optionally substituted by a halogen atom or a C₁₋₆ alkyl group,
   (2) an aryl group
      wherein said aryl group is optionally substituted by substituent(s) selected from the group consisting of
      (a) a heterocyclic group optionally substituted by a C₁₋₄ alkyl group or a C₁₋₄ alkylcarbonyl group,
      (b) a C₃₋₇ cycloalkyl group optionally substituted by a hydroxyl group, an oxo group, a halogen atom or a C₁₋₆ alkyl group,
      (c) a carboxy group,
      (d) a halogen atom,
      (e) a C₁₋₈ alkyl group,
      (f) a C₁₋₄ haloalkyl group,
      (g) a C₁₋₄ alkylamino group,
      (h) a di(C₁₋₄ alkyl) amino group,
      (i) a C₁₋₆ alkylthio group,
      (j) a C₁₋₄ alkoxy group, and
      (k) a C₁₋₄ alkylcarbonyl group,
   (3) a heteroaromatic ring group
      wherein said heteroaromatic ring group is optionally substituted by substituent(s) selected from the group consisting of
      (a) a heterocyclic group optionally substituted by a C₁₋₄ alkyl group or a C₁₋₄ alkylcarbonyl group,
      (b) a C₃₋₇ cycloalkyl group optionally substituted by a hydroxyl group, an oxo group, a halogen atom or a C₁₋₆ alkyl group,
      (c) a carboxy group,
      (d) a halogen atom,
      (e) a C₁₋₈ alkyl group,
      (f) a C₁₋₄ haloalkyl group,
      (g) a C₁₋₄ alkylamino group,
      (h) a di(C₁₋₄ alkyl) amino group,
      (i) a C₁₋₆ alkylthio group,
      (j) a C₁₋₄ alkoxy group,
      (k) a C₁₋₄ alkylcarbonyl group and
      (l) an aryl group optionally substituted by a halogen atom or a C₁₋₄ haloalkyl group,
   (4) an indanyl group or
   (5) a piperazinyl group
      wherein said piperazinyl group is optionally substituted by substituent(s) selected from the group consisting of
      (a) a phenyl group,
      (b) a phenyl C₁₋₄ alkyl group,
      (c) a benzoyl group optionally substituted by a halogen atom and
      (d) a phenyl C₁₋₄ alkoxycarbonyl group or a prodrug thereof, or a pharmaceutically acceptable salt thereof.
[3] The 5-membered heteroaromatic ring compound of [1],
   wherein, in the formula [I],
   R³ is
   (1) a hydrogen atom,
   (2) a halogen atom,
   (3) a C₁₋₆ alkyl group or
   (4) a C₁₋₄ alkoxy group;
      Y is -O-, -N(R¹¹)-, -N(R¹²)-CO-, -C(R¹³)(R¹⁴)-, -C(R¹³)(R¹⁴)-N(R¹²)-, -CO-N(R¹²)- or -C(R¹³)(R¹⁴)-O-
   wherein
   R¹¹ is
   (1) a hydrogen atom,
   (2) a C₁₋₈ alkyl group, or
   (3) optionally linked with R³ to form 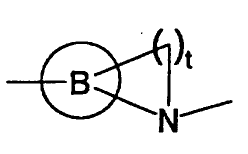 wherein t is 3,
      R¹² is a hydrogen atom or a C₁₋₈ alkyl group,
      R¹³ and R¹⁴ are each a hydrogen atom
      provided that
      when m is 0, p is 1, and L is 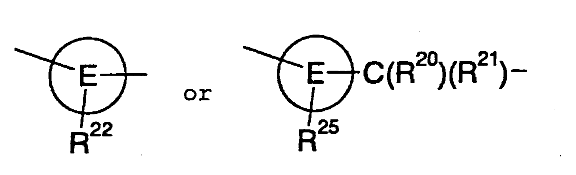 wherein R²⁰ and R²¹ are each a hydrogen atom, E is a phenyl group, R²² is a hydrogen atom, a C₁₋₄ alkoxy group or a nitro group, R²⁵ is a hydrogen atom, a C₁₋₄ alkoxy group or a nitro group,
      Y should be
      -C(R¹³)(R¹⁴)-N(R¹²)-, -CO-N(R¹²)- or -C(R¹³)(R¹⁴)-O- wherein R¹², R¹³ and R¹⁴ are as defined above;
      s is 0 or 1;
      A is a C₁₋₄ alkylene group;
      Z is an aryl group substituted by substituent(s) selected from the group consisting of
      (a) a C₃₋₇ cycloalkyl group optionally substituted by 1 to 3 substituent(s) selected from the group consisting of a halogen atom and a C₁₋₆ alkyl group,
      (b) a halogen atom,
      (c) C₁₋₈ alkyl group,
      (d) a C₁₋₄ haloalkyl group,
      (e) a di(C₁₋₄ alkyl)amino group,
      (f) a C₁₋₆ alkylthio group and
      (g) a C₁₋₄ alkylcarbonyl group,
      a prodrug thereof, or a pharmaceutically acceptable salt thereof.
[4] The 5-membered heteroaromatic ring compound of [3], wherein V is =N- and W is -S- or -O-, or V is =CH- and W is -S-, or a prodrug thereof, or a pharmaceutically acceptable salt thereof.
[5] The 5-membered heteroaromatic ring compound of [4], wherein Z is a phenyl group substituted by a C₁₋₈ alkyl group, or a prodrug thereof, or a pharmaceutically acceptable salt thereof.
[6] The 5-membered heteroaromatic ring compound of [5], wherein Y is -C(R¹³)(R¹⁴)-N(R¹²)- or -C(R¹³)(R¹⁴)-O- wherein R¹², R¹³ and R¹⁴ are as defined in [3] and s is 0, or Y is -O- or -N(R¹¹)-wherein R¹¹ is as defined in [3], s is 1, and A is a methylene group, or a prodrug thereof, or a pharmaceutically acceptable salt thereof.
[7] The 5-membered heteroaromatic ring compound of [6], wherein V is =CH-, W is -S-, and the position of substitution of B on the thiophene ring formed by V together with W is the 4-position or 5-position, or a prodrug thereof, or a pharmaceutically acceptable salt thereof.
[8] The 5-membered heteroaromatic ring compound of [7], wherein B is a phenyl group, or a prodrug thereof, or a pharmaceutically acceptable salt thereof.
[9] The 5-membered heteroaromatic ring compound of [8], wherein m is 1;
   R¹ and R² are each a hydrogen atom;
   X is -N(R⁴)- or -O-
   wherein R⁴ is a C₁₋₆ alkyl group optionally substituted by
   (a) an aryl group,
   (b) an heteroaromatic ring group optionally substituted by 1 to 3 C₁₋₈ alkyl groups or
   (c) -CO-N(R¹⁵) (R¹⁶)
   wherein R¹⁵ and R¹⁶ are each independently a hydrogen atom or an aryl group wherein said aryl group is optionally substituted by 1 to 3 C₁₋₈ alkyl groups;
   n is 0 or 1;
   p is 0 or 1;
   L is

   (1) -C(R²⁰)(R²¹)-

   wherein R²⁰ is linked with R⁴ to form 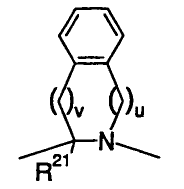 wherein u is 1 and v is 1,
   R²¹ is a hydrogen atom, or 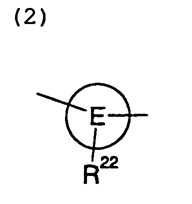 wherein E is an heteroaromatic ring group and R²² is a hydrogen atom;
   R is -COO(R¹⁹)
   wherein R¹⁹ is a hydrogen atom, or a prodrug thereof, or a pharmaceutically acceptable salt thereof.
[10] The 5-membered heteroaromatic ring compound of [9], wherein X is -N(R⁴) wherein R⁴ is as defined in [9], n is 1 and p is 0, or a prodrug thereof, or a pharmaceutically acceptable salt thereof.
[11] The 5-membered heteroaromatic ring compound of [10], wherein R⁴ is a methyl group substituted by a heteroaromatic ring group optionally substituted by one C₁₋₈ alkyl group, or a prodrug thereof, or a pharmaceutically acceptable salt thereof.
[12] The 5-membered heteroaromatic ring compound of [11], wherein the heteroaromatic ring defined in [11] is a thiazolyl group, an oxazolyl group or a benzimidazolyl group, or a prodrug thereof, or a pharmaceutically acceptable salt thereof.
[13] The 5-membered heteroaromatic ring compound of [10], wherein R⁴ is a methyl group substituted by -CO-N(R¹⁵)(R¹⁶) wherein R¹⁵ is a hydrogen atom and R¹⁶ is an aryl group optionally substituted by one C₁₋₈ alkyl group, or a prodrug thereof, or a pharmaceutically acceptable salt thereof.
[14] The 5-membered heteroaromatic ring compound of [6], wherein V is =N-, W is -S-, and the position of substitution of B on the thiazole ring formed by V together with W is the 4-position, or a prodrug thereof, or a pharmaceutically acceptable salt thereof.
[15] The 5-membered heteroaromatic ring compound of [14], wherein B is a phenyl group, or a prodrug thereof, or a pharmaceutically acceptable salt thereof.
[16] The 5-membered heteroaromatic ring compound of [15], wherein
   m is 0 or 1;
   R¹ and R² are each independently a hydrogen atom or a C₁₋₄ alkyl group;
   X is -N(R⁴)-, -N (R⁵) -CO-O-, -SO₂-N(R⁵)-, -CO-N (R⁷)-, -N (R⁹)-CO- N(R⁵)-, -N(R¹⁰)-(CH₂)ₖ-N(R⁵)-, -O-, -S- or -SO₂-
   wherein
   R⁴ is
   (1) a hydrogen atom,
   (2) a C₁₋₆ alkyl group
      wherein said C₁₋₆ alkyl group is optionally substituted by
      (a) an aryl group optionally substituted by 1 to 3 substituent(s) selected from the group consisting of a halogen atom, a C₁₋₈ alkyl group and a C₁₋₄ haloalkyl group,
      (b) a heteroaromatic ring group optionally substituted by 1 to 3 C₁₋₈ alkyl group,
      (c) a carboxy group,
      (d) a C₁₋₄ alkoxycarbonyl group,
      (e) -CO-N(R¹⁵)(R¹⁶)
         wherein R¹⁵ and R¹⁶ are each independently a hydrogen atom, an aryl group wherein said aryl group is optionally substituted by 1 to 3 substituent(s) selected from the group consisting of a C₁₋₈ alkyl group, a C₁₋₄ alkoxy group, a carboxy group and a di(C₁₋₄ alkyl) amino group, a heteroaromatic ring group, a C₁₋₆ alkyl group wherein said C₁₋₆ alkyl group is optionally substituted by an aryl group, or may form a 5- to 7-membered hetero ring optionally containing at least one heteroatom selected from the group consisting of nitrogen atom, oxygen atom and sulfur atom, together with the nitrogen atom bonded thereto,
      (f) -N(R¹⁵) (R¹⁶)
         wherein R¹⁵ and R¹⁶ are each independently a hydrogen atom or an aryl group,
      (g) -O-R¹⁷
         wherein R¹⁷ is an aryl group, or
      (h) a C₃₋₇ cycloalkyl group,
   (3) -CO-N(R¹⁵) (R¹⁶)
      wherein R¹⁵ and R¹⁶ are each independently a hydrogen atom, an aryl group wherein said aryl group is optionally substituted by 1 to 3 C₁₋₈ alkyl groups, a C₁₋₆ alkyl group, or may form an indoline ring together with the nitrogen atom bonded thereto, or may form a 5- to 7-membered hetero ring optionally containing at least one heteroatom selected from the group consisting of nitrogen atom, oxygen atom and sulfur atom,
   (4) -SO₂-N(R¹⁵)(R¹⁶)
      wherein R¹⁵ and R¹⁶ are each independently an aryl group or a C₁₋₆ alkyl group,
   (5) -CO-R¹⁷
      wherein R¹⁷ is an aryl group wherein said aryl group is optionally substituted by 1 to 3 substituent(s) selected from the group consisting of a C₁₋₈ alkyl group and a C₁₋₄ alkoxy group, a heteroaromatic ring group or a C₁₋₆ alkyl group wherein said C₁₋₆ alkyl group is optionally substituted by an aryl group optionally substituted by 1 to 3 substituent(s) selected from the group consisting of a halogen atom and a C₁₋₈ alkyl group, a heteroaromatic ring group, a C₁₋₄ alkoxy group optionally substituted by an aryl group or an aryloxy group optionally substituted by 1 to 3 C₁₋₈ alkyl groups,
   (6) -SO₂-R¹⁷
      wherein R¹⁷ is an aryl group,
   (7) an aryl group, or
   (8) optionally linked with R¹ to form 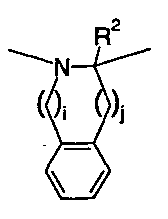
   wherein i and j are each 1,
   R⁵ is a hydrogen atom or an aryl group or a C₁₋₆ alkyl group optionally substituted by a C₃₋₇ cycloalkyl group,
   R⁷ is a hydrogen atom or a C₁₋₆ alkyl group,
   R⁹ is a hydrogen atom or a C₁₋₆ alkyl group,
   k is 2,
   R¹⁰ is
   (1) a hydrogen atom,
   (2) a C₁₋₆ alkyl group, or
   (3) optionally linked with R⁵ to form 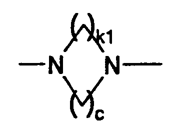 wherein k1 and c are each 2;
      n is 0 or an integer of 1 to 3;
      p is 0 or 1;
      L is

      (1) -C(R²⁰)(R²¹)-

      wherein
      R²⁰ is
      (a) a hydrogen atom,
      (b) a C₁₋₆ alkyl group, or
      (c) optionally linked with R⁴ to form 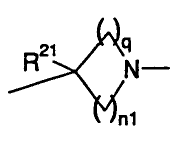 wherein n1 and q are each independently an integer of 1 to 3, or
      (d) optionally linked with R⁴ to form 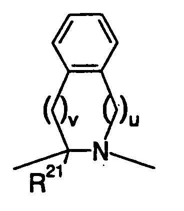
      wherein u is 1 and v is 1,
      R²¹ is a hydrogen atom, a C₁₋₆ alkyl group wherein said C₁₋₆ alkyl group is optionally substituted by an aryl group optionally substituted by 1 to 3 halogen atoms, or an aryl group or 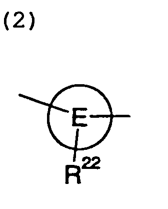 wherein
      E is an aryl group or a heteroaromatic ring group,
      R²² is
      (a) a hydrogen atom,
      (b) a C₁₋₄ alkoxy group optionally substituted by a carboxy group,
      (c) a nitro group,
      (d) -N(R²³)(R²⁴)
         wherein R²³ and R²⁴ are each independently a hydrogen atom, a C₁₋₄ alkylcarbonyl group wherein said C₁₋₄ alkylcarbonyl group is optionally substituted by a C₁₋₄ alkylamino group or a di(C₁₋₄ alkyl)amino group or a C₁₋₄ alkylsulfonyl group, or
      (e) optionally linked with R⁴ to form 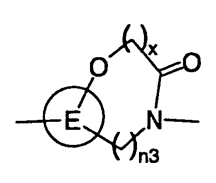
      wherein n3 is 0 and x is 1;
      R is -COO(R¹⁹)
      wherein R¹⁹ is a hydrogen atom or a C₁₋₄ alkyl group, or a prodrug thereof, or a pharmaceutically acceptable salt thereof.
[17] The 5-membered heteroaromatic ring compound of [16], wherein m is 1 and R¹ and R² are each a hydrogen atom, or a prodrug thereof, or a pharmaceutically acceptable salt thereof.
[18] The 5-membered heteroaromatic ring compound of [17],
   wherein X is -N(R⁴) wherein R⁹ is as defined in [16], n is 1 and p is 0, or a prodrug thereof, or a pharmaceutically acceptable salt thereof.
[19] The 5-membered heteroaromatic ring compound of [18],
   wherein
   R⁴ is a C₁₋₆ alkyl group optionally substituted by
   (a) an aryl group optionally substituted by 1 to 3 substituent(s) selected from the group consisting of a halogen atom, a C₁₋₈ alkyl group and a C₁₋₄ haloalkyl group,
   (b) a heteroaromatic ring group optionally substituted by 1 to 3 C₁₋₈ alkyl groups,
   (c) a carboxy group,
   (d) a C₁₋₄ alkoxycarbonyl group,
   (e) -CO-N(R¹⁵)(R¹⁶)
      wherein R¹⁵ and R¹⁶ are each independently a hydrogen atom, an aryl group wherein said aryl group is optionally substituted by 1 to 3 substituent(s) selected from the group consisting of a C₁₋₈ alkyl group, a C₁₋₄ alkoxy group, a carboxy group and a di(C₁₋₄ alkyl) amino group, a heteroaromatic ring group, a C₁₋₆ alkyl group wherein said C₁₋₆ alkyl group is optionally substituted by an aryl group, or may form a 5- to 7-membered hetero ring optionally containing at least one heteroatom selected from the group consisting of nitrogen atom, oxygen atom and sulfur atom, together with the nitrogen atom bonded thereto,
   (f) -N(R¹⁵)(R¹⁶)
      wherein R¹⁵ and R¹⁶ are each independently a hydrogen atom or an aryl group,
   (g) -O-R¹⁷
      wherein R¹⁷ is an aryl group, or
   (h) a C₃₋₇ cycloalkyl group,
      or a prodrug thereof, or a pharmaceutically acceptable salt thereof.
[20] The 5-membered heteroaromatic ring compound of [19],
   wherein R⁴ is a methyl group substituted by a heteroaromatic ring group optionally substituted by one C₁₋₈ alkyl group, or a prodrug thereof, or a pharmaceutically acceptable salt thereof.
[21] The 5-membered heteroaromatic ring compound of [20],
   wherein the heteroaromatic ring defined in [20] is a thiazolyl group, an oxazolyl group, a benzimidazolyl group, a pyridyl group or a quinolyl group, or a prodrug thereof, or a pharmaceutically acceptable salt thereof.
[22] The 5-membered heteroaromatic ring compound of [19], wherein R⁴ is a methyl group substituted by -CO-N(R¹⁵)(R¹⁶)
   wherein R¹⁵ is a hydrogen atom and R¹⁶ is an aryl group optionally substituted by one C₁₋₈ alkyl group, or a prodrug thereof, or a pharmaceutically acceptable salt thereof.
[23] The 5-membered heteroaromatic ring compound of any of [1] to [22], which is selected from the group consisting of the following compounds, or a prodrug thereof, or a pharmaceutically acceptable salt thereof:
   (1) 5-{4-[4-({[4-(1-ethylpropyl)phenyl]isopropylamino}methyl)phenyl]thiazol-2-ylmethoxy}nicotinic acid;
   (2) 4-{4-[4-({[4-(1-ethylpropyl)phenyl]isopropylamino}methyl)phenyl]thiazol-2-ylmethoxy}benzoic acid;
   (3) 6-{4-[4-({[4-(1-ethylpropyl)phenyl]isopropylamino}methyl)phenyl]thiazol-2-ylmethoxy}nicotinic acid;
   (4) 5-{4-[4-({methyl[4-(1-propylbutyl)phenyl]amino}methyl)phenyl]thiazol-2-ylmethoxy}nicotinic acid;
   (5) 4-{4-[4-({methyl[4-(1-propylbutyl)phenyl]amino}methyl)phenyl]thiazol-2-ylmethoxy}benzoic acid;
   (6) 6-[4-(4-{[(4-isopropylphenyl)(1-propylbutyl)amino]methyl}phenyl)thiazol-2-ylmethoxy]nicotinic acid;
   (7) 5-[4-(4-{[(4-isopropylphenyl)(1-propylbutyl)amino]methyl}phenyl)thiazol-2-ylmethoxy]nicotinic acid;
   (8) 5-[4-(4-{[isobutyl(4-isopropylphenyl)amino]methyl}phenyl)thiazol-2-ylmethoxy]nicotinic acid;
   (9) 4-[4-(4-{[(4-isopropylphenyl)(1-propylbutyl)amino]methyl}phenyl)thiazol-2-ylmethoxy]benzoic acid;
   (10) 3-[4-(4-{[(4-isopropylphenyl)(1-propylbutyl)amino]methyl}phenyl)thiazol-2-ylmethoxy]benzoic acid;
   (11) 6-[4-(4-{[(1-ethylpropyl)(4-isopropylphenyl)amino]methyl}phenyl)thiazol-2-ylmethoxy]nicotinic acid;
   (12) 5-[4-(4-{[(1-ethylpropyl)(4-isopropylphenyl)amino]methyl}phenyl)thiazol-2-ylmethoxy]nicotinic acid;
   (13) 4-{4-[4-({[4-(1-ethylpropyl)phenyl]isopropylamino}methyl)phenyl]thiazol-2-ylmethylthio}benzoic acid;
   (14) 4-{4-[4-({methyl[4-(1-propylbutyl)phenyl]amino}methyl)phenyl]thiazol-2-ylmethylthio}benzoic acid;
   (15) 4-(methyl-{4-[4-({methyl[4-(1-propylbutyl)phenyl]amino}methyl)phenyl]thiazol-2-ylmethyl}sulfamoyl)benzoic acid;
   (16) sodium 4-(methyl{4-[4-({methyl[4-(1-propylbutyl)phenyl]amino}methyl)phenyl]thiazol-2-ylmethyl}sulfamoyl)butyrate;
   (17) 4-{[(4-{4-[(4-cyclohexylphenylamino)methyl]phenyl}thiazol-2-yl)methylamino]methyl}benzoic acid;
   (18) 4-({[4-(4-{[(4-cyclohexylphenyl)methylamino]methyl}phenyl)thiazol-2-yl]methylamino}methyl)benzoic acid;
   (19) 4-[(methyl-{4-[4-({methyl[4-(1-propylbutyl)phenyl]amino}methyl)phenyl]thiazol-2-yl}amino)methyl]benzoic acid;
   (20) 4-[({4-[4-({[4-(1-ethylpropyl)phenyl]isopropylamino}methyl)phenyl]thiazol-2-yl)methylamino)methyl]benzoic acid;
   (21) (S)-({4-[4-({[4-(1-ethylpropyl)phenyl]isopropylamino}methyl)phenyl]thiazol-2-ylmethyl}methylamino)phenylacetic acid;
   (22) (S)-2-({4-[4-({[4-(1-ethylpropyl)phenyl]isopropylamino}methyl)phenyl]thiazol-2-ylmethyl}methylamino)-3-phenylpropionic acid;
   (23) {benzyl[4-(4-{[(4-tert-butylphenyl)isobutylamino]methyl}phenyl)thiazol-2-ylmethyl]amino}acetic acid;
   (24) {benzyl[4-(4-{[(4-chlorophenyl)isobutylamino]methyl}phenyl)thiazol-2-ylmethyl]amino}acetic acid;
   (25) (benzyl{4-[4-({methyl[4-(1-propylbutyl)phenyl]amino}methyl)phenyl]thiazol-2-ylmethyl}amino)acetic acid;
   (26) (benzyl{4-[4-({[4-(1-ethylpropyl)phenyl]isopropylamino}methyl)phenyl]thiazol-2-ylmethyl}amino)acetic acid;
   (27) (1-[4-(4-([(4-tert-butylphenyl)isobutylamino]methyl}phenyl)thiazol-2-ylmethyl]-3-phenylureido}acetic acid;
   (28) {benzoyl-[4-(4-{[(4-tert-butylphenyl)isobutylamino]methyl}phenyl)thiazol-2-ylmethyl]amino}acetic acid;
   (29) [[4-(4-{[(4-tert-butylphenyl)isobutylamino]methyl}phenyl)thiazol-2-ylmethyl](pyridin-2-ylcarbonyl)amino]acetic acid;
   (30) [[4-(4-{[(4-tert-butylphenyl)isobutylamino]methyl}phenyl)thiazol-2-ylmethyl](pyridin-3-ylcarbonyl)amino]acetic acid;
   (31) {benzenesulfonyl-[4-(4-{[(4-tert-butylphenyl)isobutylamino]methyl}phenyl)thiazol-2-ylmethyl]amino}acetic acid;
   (32) 2-{4-[4-({[4-(1-ethylpropyl)phenyl]isopropylamino}methyl)phenyl]thiazol-2-ylmethyl}-1,2,3,4-tetrahydroisoquinoline-3-carboxylic acid;
   (33) (S)-2-{4-[4-({[4-(1-ethylpropyl)phenyl]isopropylamino}methyl)phenyl]thiazol-2-ylmethyl}-1,2,3,4-tetrahydroisoquinoline-3-carboxylic acid;
   (34) (S)-2-{4-[4-({methyl[4-(1-propylbutyl)phenyl]amino}methyl)phenyl]thiazol-2-ylmethyl}-1,2,3,4-tetrahydroisoquinoline-3-carboxylic acid;
   (35) (S)-2-[4-(4-{[(1-ethylpropyl)-(4-isopropylphenyl)amino]methyl}phenyl)thiazol-2-ylmethyl]-1,2,3,4-tetrahydroisoquinoline-3-carboxylic acid;
   (36) 4-({4-[4-(4-cyclohexylphenoxymethyl)phenyl]thiazol-2-yl}methylamino)benzoic acid;
   (37) 4-[({4-[4-(4-cyclohexylphenoxymethyl)phenyl]thiazol-2-yl}methylamino)methyl]benzoic acid;
   (38) 4-{[(4-{4-[4-(1,1-dimethylpropyl)phenoxymethyl]phenyl}thiazol-2-yl)methylamino]methyl}benzoic acid;
   (39) 4-{[methyl-(4-{4-[4-(1-propylbutyl)phenoxymethyl]phenyl}thiazol-2-yl)amino]methyl}benzoic acid;
   (40) sodium 4-{[methyl(4-{4-[4-(1-propylbutyl)phenoxymethyl]phenyl}thiazol-2-yl)amino]methyl}benzoate;
   (41) (S)-[methyl-(4-{4-[4-(1-propylbutyl)phenoxymethyl]phenyl}thiazol-2-ylmethyl)amino]phenylacetic acid;
   (42) (S)-2-[methyl-(4-{4-[4-(1-propylbutyl)phenoxymethyl]phenyl}thiazol-2-ylmethyl)amino]-3-phenylpropionic acid;
   (43) (benzyl{4-[4-(2-tert-butyl-4-methylphenoxymethyl)phenyl]thiazol-2-ylmethyl}amino)acetic acid;
   (44) [benzyl(4-{4-[4-(1-propylbutyl)phenoxymethyl]phenyl}thiazol-2-ylmethyl)amino]acetic acid;
   (45) 2-(4-{4-[4-(1-propylbutyl)phenoxymethyl]phenyl}thiazol-2-ylmethyl)-1,2,3,4-tetrahydroisoquinoline-3-carboxylic acid;
   (46) (S)-2-(4-{4-[4-(1-propylbutyl)phenoxymethyl]phenyl}thiazol-2-ylmethyl)-1,2,3,4-tetrahydroisoquinoline-3-carboxylic acid;
   (47) (R)-2-(4-{4-[4-(1-propylbutyl)phenoxymethyl]phenyl}thiazol-2-ylmethyl)-1,2,3,4-tetrahydroisoquinoline-3-carboxylic acid;
   (48) 5-(4-{4-[4-(1-propylbutyl)phenoxymethyl]phenyl}thiazol-2-ylmethoxy)nicotinic acid;
   (49) 4-(4-{4-[4-(1-propylbutyl)phenoxymethyl]phenyl}thiazol-2-ylmethoxy)benzoic acid;
   (50) 4-[methyl-(4-{4-[4-(1-propylbutyl)phenoxymethyl]phenyl}thiazol-2-ylmethyl)sulfamoyl]benzoic acid;
   (51) 4-[methyl-(4-{4-[4-(1-propylbutyl)phenoxymethyl]phenyl}thiazol-2-ylmethyl)sulfamoyl]butyric acid;
   (52) 4-({4-[4-(4-cyclohexylphenylcarbamoyl)phenyl]thiazol-2-yl}methylamino)benzoic acid;
   (53) 4-[({4-[4-(4-cyclohexylphenylcarbamoyl)phenyl]thiazol-2-yl}methylamino)methyl]benzoic acid;
   (54) [benzyl(4-{4-[4-(1-propylbutyl)phenylcarbamoyl]phenyl}thiazol-2-ylmethyl)amino]acetic acid;
   (55) [benzyl(4-{4-[4-(1-propylbutyl)benzylcarbamoyl]phenyl}thiazol-2-ylmethyl)amino]acetic acid;
   (56) {benzyl[4-(4-{methyl[4-(1-propylbutyl)benzyl]carbamoyl}phenyl)thiazol-2-ylmethyl]amino}acetic acid;
   (57) {benzyl[4-(4-{ethyl-[4-(1-propylbutyl)benzyl]carbamoyl}phenyl)thiazol-2-ylmethyl]amino}acetic acid;
   (58) 5-{4-[4-({(2-hydroxy-2-methylpropyl)-[4-(1-propylbutyl)phenyl]amino}methyl)phenyl]thiazol-2-ylmethoxy}nicotinic acid;
   (59) [[4-(4-{[(4-tert-butylphenyl)isobutylamino]methyl}phenyl)thiazol-2-ylmethyl](morpholine-4-carbonyl)amino]acetic acid;
   (60) sodium 5-{4-[4-({[4-(1-ethylpropyl)phenyl]isopropylamino}methyl)phenyl]thiazol-2-ylmethoxy}-nicotinate;
   (61) sodium (S)-2-(4-{4-[4-(1-propylbutyl)phenoxymethyl]phenyl}thiazol-2-ylmethyl)-1,2,3,4-tetrahydroisoquinoline-3-carboxylate;
   (62) sodium 5-(4-{4-[4-(1-propylbutyl)phenoxymethyl]phenyl}thiazol-2-ylmethoxy)nicotinate;
   (63) 3-{4-[4-({[4-(1-ethylpropyl)phenyl]isopropylamino}methyl)phenyl]thiazol-2-ylmethoxy}-5-methoxy-benzoic acid;
   (64) (1-{4-[4-({[4-(1-ethylpropyl)phenyl]isopropylamino}methyl)phenyl]thiazol-2-ylmethyl}-3-phenylureido)acetic acid;
   (65) (1-{4-[4-({[4-(1-ethylpropyl)phenyl]isopropylamino}methyl)phenyl]thiazol-2-ylmethyl}-3-p-tolylureido)acetic acid;
   (66) [1-{4-[4-({[4-(1-ethylpropyl)phenyl]isopropylamino}methyl)phenyl]thiazol-2-ylmethyl}-3-(4-isopropylphenyl)ureido]acetic acid;
   (67) (1-{4-[4-({[4-(1-ethylpropyl)phenyl]isopropylamino}methyl)phenyl]thiazol-2-ylmethyl}-3-methyl-3-phenylureido)acetic acid;
   (68) 5-{4-[4-({isopropyl[4-(1-propylbutyl)phenyl]amino}methyl)phenyl]thiazol-2-ylmethoxy}nicotinic acid;
   (69) [3-phenyl-1-(4-{4-[4-(1-propylbutyl)phenoxymethyl]phenyl}thiazol-2-ylmethyl)ureido]acetic acid;
   (70) (3-(2,6-dimethylphenyl)-1-{4-[4-({[4-(1-ethylpropyl)phenyl]isopropylamino}methyl)phenyl]thiazol-2-ylmethyl}ureido)acetic acid;
   (71) ({4-[4-({[4-(1-ethylpropyl)phenyl]isopropylamino}methyl)phenyl]thiazol-2-ylmethyl}phenylcarbamoylmethylamino)acetic acid;
   (72) (1-{4-[4-({[4-(1-ethylpropyl)phenyl]isopropylamino}methyl)phenyl]thiazol-2-ylmethyl}-3-isopropyl-ureido)acetic acid;
   (73) 3-{4-[4-({isopropyl[4-(1-propylbutyl)phenyl]amino}methyl)phenyl]thiazol-2-ylmethoxy}isoxazole-5-carboxylic acid;
   (74) 5-[4-(4-{[(2-ethylbutyl)-(4-isopropylphenyl)amino]methyl}phenyl)thiazol-2-ylmethoxy]nicotinic acid;
   (75) [{4-[4-({[4-(1-ethylpropyl)phenyl]isopropylamino}methyl)phenyl]thiazol-2-ylmethyl}-(piperidine-1-carbonyl)amino]acetic acid;
   (76) 2-(4-{4-[4-(1-propylbutyl)phenoxymethyl]phenyl}thiazol-2-ylmethoxy)nicotinic acid;
   (77) [{4-[4-({[4-(1-ethylpropyl)phenyl]isopropylamino}methyl)phenyl]thiazol-2-ylmethyl}-(p-tolylcarbamoylmethyl)amino]acetic acid;
   (78) {{4-[4-({[4-(1-ethylpropyl)phenyl]isopropylamino}methyl)phenyl]thiazol-2-ylmethyl}-[(4-isopropylphenylcarbamoyl)methyl]amino}acetic acid;
   (79) (1-{4-[4-({isopropyl[4-(1-propylbutyl)phenyl]amino}methyl)phenyl]thiazol-2-ylmethyl}-3-methyl-3-phenylureido)acetic acid;
   (80) (1-{4-[4-({isopropyl[4-(1-propylbutyl)phenyl]amino}methyl)phenyl]thiazol-2-ylmethyl}-3-p-tolylureido)acetic acid;
   (81) ((2,3-dihydro-indole-1-carbonyl)-{4-[4-({isopropyl[4-(1-propylbutyl)phenyl]amino}methyl)phenyl]thiazol-2-ylmethyl}amino)acetic acid;
   (82) 3-(4-{4-[4-(1-propylbutyl)phenoxymethyl]phenyl}thiazol-2-ylmethoxy)pyridine-2-carboxylic acid;
   (83) {[4-(4-{[(2-ethylbutyl)-(4-isopropylphenyl)amino]methyl}phenyl)thiazol-2-ylmethyl]phenylcarbamoylmethylamino}acetic acid;
   (84) {[4-(4-{[(2-ethylbutyl)-(4-isopropylphenyl)amino]methyl}phenyl)thiazol-2-ylmethyl]-[(4-isopropylphenylcarbamoyl)methyl]amino}acetic acid;
   (85) 4-(2-{carboxymethyl[4-(4-{[(2-ethylbutyl)-(4-isopropylphenyl)amino]methyl}phenyl)thiazol-2-ylmethyl]amino}acetylamino)benzoic acid;
   (86) 6-(4-{4-[4-(1-propylbutyl)phenoxymethyl]phenyl}thiazol-2-ylmethoxy)pyridine-2-carboxylic acid;
   (87) 5-{4-[4-({isobutyl[4-(1-propylbutyl)phenyl]amino}methyl)phenyl]thiazol-2-ylmethoxy}nicotinic acid;
   (88) (1-{4-[4-({isobutyl[4-(1-propylbutyl)phenyl]amino}methyl)phenyl]thiazol-2-ylmethyl}-3-phenylureido)acetic acid;
   (89) 5-{4-[4-({[4-(1-ethylpropyl)phenyl]isopropylamino}methyl)phenyl]thiazol-2-ylmethoxy}nicotinic acid methyl ester;
   (90) 4-amino-3-{4-[4-({[4-(1-ethylpropyl)phenyl]isopropylamino}methyl)phenyl]thiazol-2-ylmethoxy}benzoic acid;
   (91) 3-{4-[4-({[4-(1-ethylpropyl)phenyl]isopropylamino}methyl)phenyl]thiazol-2-ylmethoxy}benzoic acid;
   (92) 3-({4-[4-({[4-(1-ethylpropyl)phenyl]isopropylamino}methyl)phenyl]thiazol-2-ylmethyl}amino)benzoic acid;
   (93) 3-{4-[4-({[4-(1-ethylpropyl)phenyl]isopropylamino}methyl)phenyl]thiazol-2-ylmethoxy}-4-nitro-benzoic acid;
   (94) ((1H-benzimidazol-2-ylmethyl)-{4-[4-({isopropyl[4-(1-propylbutyl)phenyl]amino}methyl)phenyl]thiazol-2-ylmethyl}amino)acetic acid;
   (95) [phenylcarbamoylmethyl(4-{4-[4-(1-propylbutyl)phenoxymethyl]phenyl}thiazol-2-ylmethyl)amino]acetic acid;
   (96) [(4-{4-[4-(1-propylbutyl)phenoxymethyl]phenyl}thiazol-2-ylmethyl)-(pyridin-3-ylcarbamoylmethyl)amino]acetic acid;
   (97) [[(4-dimethylaminophenylcarbamoyl)methyl]-(4-{4-[4-(1-propylbutyl)phenoxymethyl]phenyl}thiazol-2-ylmethyl)amino]acetic acid;
   (98) [[(4-methoxyphenylcarbamoyl)methyl]-(4-{4-[4-(1-propylbutyl)phenoxymethyl]phenyl}thiazol-2-ylmethyl)amino]acetic acid;
   (99) [[(isopropylphenylcarbamoyl)methyl]-(4-{4-[4-(1-propylbutyl)phenoxymethyl]phenyl}thiazol-2-ylmethyl)amino]acetic acid;
   (100) [(4-{4-[4-(1-propylbutyl)phenoxymethyl]phenyl}thiazol-2-ylmethyl)-(pyridin-2-ylcarbamoylmethyl)amino]acetic acid;
   (101) [(2-oxo-2-pyrrolidin-1-yl-ethyl)-(4-{4-[4-(1-propylbutyl)phenoxymethyl]phenyl}thiazol-2-ylmethyl)amino]acetic acid;
   (102) [(4-methylthiazol-2-ylmethyl)-(4-{4-[4-(1-propylbutyl)phenoxymethyl]phenyl}thiazol-2-ylmethyl)amino]acetic acid;
   (103) 4-(3-cyclohexylmethyl-3-{4-[4-({isopropyl[4-(1-propylbutyl)phenyl]amino}methyl)phenyl]thiazol-2-ylmethyl}ureido)benzoic acid;
   (104) 4-(3-isobutyl-3-{4-[4-({isopropyl[4-(1-propylbutyl)phenyl]amino}methyl)phenyl]thiazol-2-ylmethyl}ureido)benzoic acid;
   (105) (1-{4-[4-({isobutyl[4-(1-propylbutyl)phenyl]amino}methyl)phenyl]thiazol-2-ylmethyl}-3-methyl-3-phenylureido)acetic acid;
   (106) ({4-[4-({isobutyl[4-(1-propylbutyl)phenyl]amino}methyl)phenyl]thiazol-2-ylmethyl}phenylcarbamoylmethylamino)acetic acid;
   (107) {{4-[4-((isobutyl[4-(1-propylbutyl)phenyl]amino}methyl)phenyl]thiazol-2-ylmethyl}-[(4-isopropylphenylcarbamoyl)methyl]amino}acetic acid;
   (108) 4-acetylamino-3-{4-[4-({isopropyl[4-(1-propylbutyl)phenyl]amino}methyl)phenyl]thiazol-2-ylmethoxy}benzoic acid;
   (109) 4-(2-dimethylaminoacetylamino)-3-{4-[4-({isopropyl[4-(1-propylbutyl)phenyl]amino}methyl)phenyl]thiazol-2-ylmethoxy}benzoic acid;
   (110) ((1H-benzimidazol-2-ylmethyl}-{4-[4-({[4-(1-ethylpropyl)phenyl]isopropylamino}methyl)phenyl]thiazol-2-ylmethyl}amino)acetic acid;
   (111) [(1H-benzimidazol-2-ylmethyl)-(4-{4-[4-(1-propylbutyl)phenoxymethyl]phenyl}thiazol-2-ylmethyl)amino]acetic acid;
   (112) 3-{4-[4-({isopropyl[4-(1-propylbutyl)phenyl]amino}methyl)phenyl]thiazol-2-ylmethoxy}-4-methanesulfonylaminobenzoic acid;
   (113) 4-isobutyrylamino-3-{4-[4-({isopropyl[4-(1-propylbutyl)phenyl]amino}methyl)phenyl]thiazol-2-ylmethoxy}benzoic acid;
   (114) 4-{4-[4-({[4-(1-ethylpropyl)phenyl]isopropylamino}methyl)phenyl]thiazol-2-ylmethyl}-3-oxo-3,4-dihydro-2H-benzo[1,4]oxazine-6-carboxylic acid;
   (115) 4-{4-[4-({[4-(1-ethylpropyl)phenyl]isopropylamino}methyl)phenyl]thiazol-2-ylmethyl}-3-oxo-3,4-dihydro-2H-benzo[1,4]oxazine-8-carboxylic acid;
   (116) ({4-[4-({isopropyl[4-(1-propylbutyl)phenyl]amino}methyl)phenyl]thiazol-2-ylmethyl}-{methylphenylaminosulfonyl}amino)acetic acid;
   (117) ([(4-isopropylphenylcarbamoyl)methyl]-{4-[4-({isopropyl[4-(1-propylbutyl)phenyl]amino}methyl)phenyl]thiazol-2-ylmethyl}amino)acetic acid;
   (118) ([(3,5-dimethylphenylcarbamoyl)methyl]-{4-[4-({isopropyl[4-(1-propylbutyl)phenyl]amino}methyl)phenyl]thiazol-2-ylmethyl}amino)acetic acid;
   (119) ([(4-dimethylaminophenylcarbamoyl)methyl]-{4-[4-((isopropyl [4-(1-propylbutyl)phenyl]amino}methyl)phenyl]thiazol-2-ylmethyl}amino)acetic acid;
   (120) ((benzylcarbamoylmethyl)-{4-[4-({isopropyl[4-(1-propylbutyl)phenyl]amino}methyl)phenyl]thiazol-2-ylmethyl}amino)acetic acid;
   (121) [{4-[4-({isopropyl[4-(1-propylbutyl)phenyl]amino}methyl)phenyl]thiazol-2-ylmethyl}-[2-(morpholin-4-yl)-2-oxo-ethyl]amino]acetic acid;
   (122) [{4-[4-({[4-(1-ethylpropyl)phenyl]isopropylamino}methyl)phenyl]thiazol-2-ylmethyl}-(2-phenoxyethyl)amino]acetic acid;
   (123) [{4-[4-({[4-(1-ethylpropyl)phenyl]isopropylamino}methyl)phenyl]thiazol-2-ylmethyl}-(2-phenylamino-ethyl)amino]acetic acid;
   (124) 2-carboxymethoxy-5-({4-[4-({[4-(1-ethylpropyl)phenyl]isopropylamino}methyl)phenyl]thiazol-2-ylmethyl}methylamino)benzoic acid;
   (125) 2-carboxymethoxy-5-[methyl(4-{4-[4-(1-propylbutyl)phenoxymethyl]phenyl}thiazol-2-ylmethyl)amino]benzoic acid;
   (126) 3-{4-[4-({isopropyl[4-(1-propylbutyl)phenyl]amino}methyl)phenyl]thiazol-2-ylmethoxy}-4-(2-methylamino-acetylamino)benzoic acid;
   (127) [(4-tert-butylthiazol-2-ylmethyl)-(4-{4-[4-(1-propylbutyl)phenoxymethyl]phenyl}thiazol-2-ylmethyl)amino]acetic acid;
   (128) [phenyl(4-{4-[4-(1-propylbutyl)phenoxymethyl]phenyl}thiazol-2-ylmethyl)amino]acetic acid;
   (129) [(2-phenoxyacetyl)-(4-{4-[4-(1-propylbutyl)phenoxymethyl]phenyl}thiazol-2-ylmethyl)amino]acetic acid;
   (130) [(4-{4-[4-(1-propylbutyl)phenoxymethyl]phenyl}thiazol-2-ylmethyl)-(2-p-tolyloxy-acetyl)amino]acetic acid;
   (131) (ethoxycarbonylmethyl{4-[4-({isopropyl[4-(1-propylbutyl)phenyl]amino}methyl)phenyl]thiazol-2-ylmethyl}amino)acetic acid dihydrochloride;
   (132) ((4-tert-butylthiazol-2-ylmethyl)-{4-[4-({[4-(1-ethylpropyl)phenyl]isopropylamino}methyl)phenyl]thiazol-2-ylmethyl}amino)acetic acid;
   (133) 6-{4-[4-({isopropyl[4-(1-propylbutyl)phenyl]amino}methyl)phenyl]thiazol-2-ylmethoxy}pyridine-2-carboxylic acid;
   (134) [(2-benzyloxy-acetyl)-(4-{4-[4-(1-propylbutyl)phenoxymethyl]phenyl}thiazol-2-ylmethyl)amino]acetic acid;
   (135) [[2-(4-isopropylphenoxy)acetyl]-(4-{4-[4-(1-propylbutyl)phenoxymethyl]phenyl}thiazol-2-ylmethyl)amino]acetic acid;
   (136) [(4,5-dimethylthiazol-2-ylmethyl)-(4-{4-[4-(1-propylbutyl)phenoxymethyl]phenyl}thiazol-2-ylmethyl)amino]acetic acid;
   (137) 5-(4-{5-[4-(1-propylbutyl)phenoxymethyl]thiophen-2-yl}thiazol-2-ylmethoxy)nicotinic acid;
   (138) ((4-tert-butylthiazol-2-ylmethyl)-{4-[6-({isopropyl[4-(1-propylbutyl)phenyl]amino}methyl)benzoxazol-2-yl]thiazol-2-ylmethyl}amino)acetic acid;
   (139) ((1H-benzimidazol-2-ylmethyl)-{4-[6-({isopropyl[4-(1-propylbutyl)phenyl]amino}methyl)benzoxazol-2-yl]thiazol-2-ylmethyl}amino)acetic acid;
   (140) 5-{4-[6-({isopropyl[4-(1-propylbutyl)phenyl]amino}methyl)benzoxazol-2-yl]thiazol-2-ylmethoxy}nicotinic acid;
   (141) [(5-tert-butylthiazol-2-ylmethyl)-(4-{4-[4-(1-propylbutyl)phenoxymethyl]phenyl}thiazol-2-ylmethyl)amino]acetic acid;
   (142) [{4-[4-({isopropyl[4-(1-propylbutyl)phenyl]amino}methyl)phenyl]thiazol-2-ylmethyl}-(2-oxo-2-piperidin-1-yl-ethyl)amino]acetic acid;
   (143) 6-[methyl-(4-{4-[4-(1-propylbutyl)phenoxymethyl]phenyl}thiazol-2-ylmethyl)amino]pyridine-2-carboxylic acid;
   (144) ({4-[6-({isopropyl[4-(1-propylbutyl)phenyl]amino}methyl)benzoxazol-2-yl]thiazol-2-ylmethyl}amino)acetic acid;
   (145) (S)-(carboxymethyl{4-[4-({isopropyl[4-(1-propylbutyl)phenyl]amino}methyl)phenyl]thiazol-2-ylmethyl}amino)phenylacetic acid;
   (146) ((4,5-dimethylthiazol-2-ylmethyl)-{4-[4-({isopropyl[4-(1-propylbutyl)phenyl]amino}methyl)phenyl]thiazol-2-ylmethyl}amino)acetic acid;
   (147) [(5-tert-butyloxazol-2-ylmethyl)-(4-{4-[4-(1-propylbutyl)phenoxymethyl]phenyl}thiazol-2-ylmethyl)amino]acetic acid;
   (148) [(1-methyl-1H-benzimidazol-2-ylmethyl)-(4-{4-[4-(1-propylbutyl)phenoxymethyl]phenyl}thiazol-2-ylmethyl)amino]acetic acid;
   (149) (isobutoxycarbonylmethyl{4-[4-({isopropyl[4-(1-propylbutyl)phenyl]amino}methyl)phenyl]thiazol-2-ylmethyl}amino)acetic acid dihydrochloride;
   (150) ({4-[4-({isopropyl[4-(1-propylbutyl)phenyl]amino}methyl)phenyl]thiazol-2-ylmethyl}propoxycarbonylmethylamino)acetic acid dihydrochloride;
   (151) 1-(4-{4-[4-(1-propylbutyl)phenoxymethyl]phenyl}thiazol-2-ylmethyl)piperidine-4-carboxylic acid hydrochloride;
   (152) 1-{4-[4-({[4-(1-ethylpropyl)phenyl]isopropylamino}methyl)phenyl]thiazol-2-ylmethyl}piperidine-4-carboxylic acid;
   (153) 4-[4-(4-{4-[4-(1-propylbutyl)phenoxymethyl]phenyl}thiazol-2-ylmethyl)piperazin-1-yl]benzoic acid;
   (154) 4-(4-{4-[4-({[4-(1-ethylpropyl)phenyl]isopropylamino}methyl)phenyl]thiazol-2-ylmethyl}-piperazin-1-yl)benzoic acid;
   (155) 2-[4-(4-{4-[4-(1-propylbutyl)benzyloxy]phenyl}thiazol-2-ylmethyl)piperazin-1-yl]benzoic acid;
   (156) 3-[4-(4-{4-[4-(1-propylbutyl)benzyloxy]phenyl}thiazol-2-ylmethyl)piperazin-1-yl]benzoic acid;
   (157) -4-(4-{1-[4-(1-propylbutyl)benzyl]-1H-indol-3-yl}thiazol 2-ylmethoxy)benzoic acid;
   (158) 4-{4-[1-(4-isopropylbenzyl)-5-(1-propylbutyl)-1H-benzimidazol-2-yl]thiazol-2-ylmethoxy}benzoic acid;
   (159) 4-{4-[1-(6-methylpyridin-2-ylmethyl)-5-(1-propylbutyl)-1H-benzimidazol-2-yl]thiazol-2-ylmethoxy}benzoic acid;
   (160) 2-(4-{4-[4-(1-propylbutyl)phenoxymethyl]phenyl}thiazol-2-ylmethyl)-1,2,3,4-tetrahydroisoquinoline-7-carboxylic acid;
   (161) 4-[1-(4-{4-[4-(1-propylbutyl)benzyloxy]phenyl}thiazol-2-ylmethyl)piperidin-4-yl]benzoic acid;
   (162) 3-[1-(4-{4-[4-(1-propylbutyl)benzyloxy]phenyl}thiazol-2-ylmethyl)piperidin-4-yl]benzoic acid;
   (163) 6-[(4-{4-[4-(1-propylbutyl)phenoxymethyl]phenyl}thiazol-2-ylmethyl)carbamoyl]nicotinic acid;
   (164) 2-[1-(4-{4-[4-(1-propylbutyl)benzyloxy]phenyl}thiazol-2-ylmethyl)piperidin-4-yl]benzoic acid;
   (165) 1-(4-{1-[4-(1-propylbutyl)benzyl]-1H-indol-3-yl}thiazol-2-ylmethyl)piperidine-4-carboxylic acid;
   (166) 2-(4-{1-[4-(1-propylbutyl)benzyl]-1H-indol-3-yl}thiazol-2-ylmethyl)-1,2,3,4-tetrahydroisoquinoline-7-carboxylic acid;
   (167) 3-[(4-{1-[4-(1-propylbutyl)benzyl]-1H-indol-3-yl}thiazol-2-ylmethyl)amino]benzoic acid;
   (168) 6-[(2-aminoethyl)(4-{4-[4-(1-propylbutyl)phenoxymethyl]phenyl}thiazol-2-ylmethyl)carbamoyl]nicotinic acid hydrochloride;
   (169) 2-(4-{1-[4-(1-propylbutyl)benzyl]-1H-benzimidazol-2-yl}thiazol-2-ylmethyl)-1,2,3,4-tetrahydroisoquinoline-7-carboxylic acid;
   (170) 3-[4-(4-{1-[4-(1-propylbutyl)benzyl]-1H-benzimidazol-2-yl}thiazol-2-ylmethyl)piperazin-1-yl]benzoic acid;
   (171) 4-[1-(4-{1-[4-(1-propylbutyl)benzyl]-1H-benzimidazol-2-yl}thiazol-2-ylmethyl)piperidin-4-yl]benzoic acid;
   (172) 3-[4-(4-{1-[4-(1-propylbutyl)benzyl]-1H-indol-3-yl}thiazol-2-ylmethyl)piperazin-1-yl]benzoic acid;
   (173) 3-(1-{4-[4-(3,4-dichloro-benzyloxy)phenyl]thiazol-2-ylmethyl}piperidin-4-yl)benzoic acid;
   (174) 3-(1-{4-[4-(3,5-bis-trifluoromethylbenzyloxy)phenyl]thiazol-2-ylmethyl}piperidin-4-yl)benzoic acid;
   (175) 3-(1-{4-[4-(4-butoxy-benzyloxy)phenyl]thiazol-2-ylmethyl}piperidin-4-yl)benzoic acid;
   (176) 5-methyl-2-(4-{4-[4-(1-propylbutyl)phenoxymethyl]phenyl}thiazol-2-ylmethyl)-2H-pyrazole-3-carboxylic acid;
   (177) 5-methyl-1-(4-{4-[4-(1-propylbutyl)phenoxymethyl]phenyl}thiazol-2-ylmethyl)-1H-pyrazole-3-carboxylic acid;
   (178) 5-tert-butyl-1-(4-{4-[4-(1-propylbutyl)phenoxymethyl]phenyl}thiazol-2-ylmethyl)-1H-pyrazole-3-carboxylic acid;
   (179) 5-tert-butyl-2-(4-{4-[4-(1-propylbutyl)phenoxymethyl]phenyl}thiazol-2-ylmethyl)-2H-pyrazole-3-carboxylic acid;
   (180) (2S,4R)-4-hydroxy-1-(4-{1-[4-(1-propylbutyl)benzyl]-1H-indol-3-yl}thiazol-2-ylmethyl)pyrrolidine-2-carboxylic acid;
   (181) 4-[4-(4-{4-[4-(1-propylbutyl)phenoxymethyl]phenyl}thiazol-2-ylmethyl)piperidin-1-yl]benzoic acid;
   (182) 1-(4-{1-[4-(1-propylbutyl)benzyl]-1H-indol-3-yl}thiazol-2-ylmethyl)-1H-indole-3-carboxylic acid;
   (183) 1-(4-{2-phenyl-1-[4-(1-propylbutyl)benzyl]-1H-indol-3-yl}thiazol-2-ylmethyl)piperidine-4-carboxylic acid;
   (184) 3-(1-{4-[4-(4-methyl-3-nitrobenzyloxy)phenyl]thiazol-2-ylmethyl}piperidin-4-yl)benzoic acid;
   (185) 2-{4-[1-(4-isopropylbenzyl)-6-(morpholin-4-yl)-1H-benzimidazol-2-yl]thiazol-2-ylmethyl}-1,2,3,4-tetrahydroisoquinoline-7-carboxylic acid;
   (186) 3-(4-{4-[1-(4-isopropylbenzyl)-6-(morpholin-4-yl)-1H-benzimidazol-2-yl]thiazol-2-ylmethyl}-piperazin-1-yl)benzoic acid;
   (187) {benzyl[4-(4-{methyl[4-(1-propylbutyl)benzyl]amino}phenyl)oxazol-2-ylmethyl]amino}acetic acid;
   (188) 5-(4-{4-[4-(1-propylbutyl)benzyloxy]phenyl}thiazol-2-ylmethoxy)nicotinic acid;
   (189) 4-(4-{4-[4-(1-propylbutyl)benzyloxy]phenyl}thiazol-2-ylmethoxy)benzoic acid;
   (190) 4-(4-{4-[4-(1-propylbutyl)benzyloxy]phenyl}thiazol-2-ylmethylthio)benzoic acid;
   (191) 4-{4-[4-(4-cyclohexylbenzyloxy)phenyl]thiazol-2-ylmethylthio}benzoic acid;
   (192) 4-{4-[4-(4-cyclohexylbenzyloxy)phenyl]thiazol-2-ylmethanesulfonyl}benzoic acid;
   (193) 4-[methyl-(4-{5-methyl-2-[4-(1-propylbutyl)benzyloxy]phenyl}thiazol-2-ylmethyl)sulfamoyl]benzoic acid;
   (194) 4-[methyl-(4-{5-methyl-2-[4-(1-propylbutyl)benzyloxy]phenyl}thiazol-2-ylmethyl)amino]benzoic acid;
   (195) (benzyl{4-[5-methyl-2-(4-trifluoromethylbenzyloxy)phenyl]thiazol-2-ylmethyl}amino)acetic acid;
   (196) [benzyl(4-{5-methyl-2-[4-(1-propylbutyl)benzyloxy]phenyl}thiazol-2-ylmethyl)amino]acetic acid;
   (197) [benzyl(4-{4-[4-(1-propylbutyl)benzyloxy]phenyl}thiazol-2-ylmethyl)amino]acetic acid;
   (198) [benzyl(4-{4-[4-(1-ethylpropyl)benzyloxy]phenyl}thiazol-2-ylmethyl)amino]acetic acid;
   (199) (benzyl{4-[5-tert-butyl-2-(4-isobutylbenzyloxy)phenyl]thiazol-2-ylmethyl}amino)acetic acid;
   (200) [benzyl(4-{5-chloro-2-[4-(1-propylbutyl)benzyloxy]phenyl}thiazol-2-ylmethyl)amino]acetic acid;
   (201) [(4-{4-[4-(1-ethylpropyl)benzyloxy]phenyl}thiazol-2-ylmethyl)-(4-fluoro-benzyl)amino]acetic acid;
   (202) [(4-{4-[4-(1-ethylpropyl)benzyloxy]phenyl}thiazol-2-ylmethyl)-(4-isopropylbenzyl)amino]acetic acid;
   (203) [(4-{4-[4-(1-ethylpropyl)benzyloxy]phenyl}thiazol-2-ylmethyl)-(4-trifluoromethylbenzyl)amino]acetic acid;
   (204) [(4-chlorobenzyl)-(4-{4-[4-(1-ethylpropyl)benzyloxy]phenyl}thiazol-2-ylmethyl)amino]acetic acid;
   (205) [(3,5-dimethylbenzyl)-(4-{4-[4-(1-ethylpropyl)benzyloxy]phenyl}thiazol-2-ylmethyl)amino]acetic acid;
   (206) [(4-{4-[4-(1-propylbutyl)benzyloxy]phenyl}thiazol-2-ylmethyl)-pyridin-2-ylmethylamino]acetic acid;
   (207) [(4-{4-[4-(1-ethylpropyl)benzyloxy]phenyl}thiazol-2-ylmethyl)-pyridin-2-ylmethylamino]acetic acid;
   (208) [(4-{5-methyl-2-[4-(1-propylbutyl)benzyloxy]phenyl}thiazol-2-ylmethyl)-pyridin-2-ylmethylamino]acetic acid;
   (209) [benzoyl-(4-{4-[4-(1-propylbutyl)benzyloxy]phenyl}thiazol-2-ylmethyl)amino]acetic acid;
   (210) [(4-{4-[4-(1-ethylpropyl)benzyloxy]phenyl}thiazol-2-ylmethyl)-(4-methylbenzoyl)amino]acetic acid;
   (211) [(4-methoxybenzoyl)-(4-{4-[4-(1-propylbutyl)benzyloxy]phenyl}thiazol-2-ylmethyl)amino]acetic acid;
   (212) 2-(4-{5-methyl-2-[4-(1-propylbutyl)benzyloxy]phenyl}thiazol-2-ylmethyl)-1,2,3,4-tetrahydroisoquinoline-3-carboxylic acid;
   (213) (S)-2-(4-{4-[4-(1-propylbutyl)benzyloxy]phenyl}thiazol-2-ylmethyl)-1,2,3,4-tetrahydroisoquinoline-3-carboxylic acid;
   (214) {benzyl[4-(4-{[4-(2,2-dimethylpropyl)benzyl]methylamino}phenyl)thiazol-2-ylmethyl]amino}acetic acid;
   (215) {benzyl[4-(4-{[trans-4-(4-tert-butylphenyl)-cyclohexylmethyl]methylamino}phenyl)thiazol-2-ylmethyl]amino}acetic acid;
   (216) [benzyl(4-{4-[(4-cyclohexylbenzyl)methylamino]phenyl}thiazol-2-ylmethyl)amino]acetic acid;
   (217) 3-[benzyl(4-{4-[(4-cyclohexylbenzyl)methylamino]phenyl}thiazol-2-ylmethyl)amino]propionic acid;
   (218) (benzyl{4-[4-({2-[4-(2,2-dimethylpropyl)phenyl]ethyl}methylamino)phenyl]thiazol-2-ylmethyl}amino)acetic acid;
   (219) {benzyl[4-(4-{methyl[4-(trans-4-methylcyclohexyl)benzyl]amino}phenyl)thiazol-2-ylmethyl]amino}acetic acid;
   (220) {benzyl[4-(4-{[4-(cis-4-fluorocyclohexyl)benzyl]methylamino}phenyl)thiazol-2-ylmethyl]amino}acetic acid;
   (221) {benzyl[4-(4-{[trans-4-(4-chlorophenyl)cyclohexylmethyl]methylamino}phenyl)thiazol-2-ylmethyl]amino}acetic acid;
   (222) {benzyl[4-(4-{[4-(4,4-dimethylcyclohexyl)benzyl]methylamino}phenyl)thiazol-2-ylmethyl]amino}acetic acid;
   (223) {benzyl[4-(4-{methyl[4-(1-propylbutyl)benzyl]amino}phenyl)thiazol-2-ylmethyl]amino}acetic acid;
   (224) (benzyl{4-[4-(biphenyl-4-ylmethylmethylamino)phenyl]thiazol-2-ylmethyl}amino)acetic acid;
   (225) sodium [benzyl(4-{4-[(4-cyclohexylbenzyl)methylamino]phenyl}thiazol-2-ylmethyl)amino]acetate;
   (226) [benzyl(4-{4-[(4-isobutylbenzyl)methylamino]phenyl}thiazol-2-ylmethyl)amino]acetic acid;
   (227) sodium {benzyl[4-(4-{methyl[4-(1-propylbutyl)benzyl]amino}phenyl)thiazol-2-ylmethyl]amino}acetate;
   (228) {benzyl[4-(4-{[4-(2,2-dimethylpropylthio)benzyl]methylamino}phenyl)thiazol-2-ylmethyl]amino}acetic acid;
   (229) {benzyl[4-(4-{methyl[4-(3-methylbutylthio)benzyl]amino}phenyl)thiazol-2-ylmethyl]amino}acetic acid;
   (230) [benzyl(4-{4-[(4-dipropylaminobenzoyl)methylamino]phenyl}thiazol-2-ylmethyl)amino]acetic acid;
   (231) [(4-{4-[ethyl(4-isopropylbenzyl)amino]phenyl}thiazol-2-ylmethyl)-(4-isopropylbenzyl)amino]acetic acid;
   (232) [(4-isopropylbenzyl)-(4-{4-[isopropyl-(4-isopropylbenzyl)amino]phenyl}thiazol-2-ylmethyl)amino]acetic acid;
   (233) [(4-tert-butylbenzyl)-(4-{4-[isopropyl-(4-isopropylbenzyl)amino]phenyl}thiazol-2-ylmethyl)amino]acetic acid;
   (234) [(4-chlorobenzyl)-(4-{4-[(4-isobutylbenzyl)methylamino]phenyl}thiazol-2-ylmethyl)amino]acetic acid;
   (235) {{4-[4-(benzylmethylamino)phenyl]thiazol-2-ylmethyl}-[4-(1-propylbutyl)benzyl]amino}acetic acid;
   (236) [{4-[4-(benzylmethylamino)phenyl]thiazol-2-ylmethyl}-(4-chloro-benzyl)amino]acetic acid;
   (237) [{4-[4-(benzylmethylamino)phenyl]thiazol-2-ylmethyl}-(2-chloro-benzyl)amino]acetic acid;
   (238) [{4-[4-(benzylmethylamino)phenyl]thiazol-2-ylmethyl}-(3,4-dichloro-benzyl)amino]acetic acid;
   (239) {[4-(4-{methyl[4-(trans-4-methylcyclohexyl)benzyl]amino}phenyl)thiazol-2-ylmethyl]pyridin-2-ylmethylamino}acetic acid;
   (240) {[4-(4-{methyl[4-(1-propylbutyl)benzyl]amino}phenyl)thiazol-2-ylmethyl]pyridin-2-ylmethylamino}acetic acid;
   (241) ({4-[4-(benzylmethylamino)phenyl]thiazol-2-ylmethyl}naphthalen-1-ylmethylamino)acetic acid;
   (242) ({4-[4-(benzylmethylamino)phenyl]thiazol-2-ylmethyl}quinolin-2-ylmethylamino)acetic acid;
   (243) ((2-benzo[b]thiophen-3-yl-acetyl)-{4-[4-(benzylmethylamino)phenyl]thiazol-2-ylmethyl}amino)acetic acid
   (244) [[2-(4-chlorophenyl)acetyl]-(4-{4-[(4-isobutylbenzyl)methylamino]phenyl}thiazol-2-ylmethyl)amino]acetic acid;
   (245) {(4-{4-[(4-isobutylbenzyl)methylamino]phenyl}thiazol-2-ylmethyl)-[2-(4-isopropylphenyl)acetyl]amino}acetic acid;
   (246) [(4-{4-[(4-isobutylbenzyl)methylamino]phenyl}thiazol-2-ylmethyl)-(3-methyl-butyryl)amino]acetic acid;
   (247) {1-[4-(4-{[4-(2,2-dimethylpropyl)benzyl]methylamino}phenyl)thiazol-2-ylmethyl]-3-phenylureido}acetic acid;
   (248) [benzoyl-(4-{4-[(4-isobutylbenzyl)methylamino]phenyl}thiazol-2-ylmethyl)amino]acetic acid;
   (249) {(4-methylbenzoyl)-[4-(4-{methyl[4-(1-propylbutyl)benzyl]amino}phenyl)thiazol-2-ylmethyl]amino}acetic acid;
   (250) sodium {(4-isopropylbenzoyl)-[4-(4-{methyl[4-(1-propylbutyl)benzyl]amino}phenyl)thiazol-2-ylmethyl]amino}acetate;
   (251) sodium (S)-{3-[4-(4-{methyl[4-(1-propylbutyl)benzyl]amino}phenyl)thiazol-2-yl]-3,4-dihydro-1H-isoquinolin-2-yl}-acetate;
   (252) 1-(4-{4-[(4-cyclohexylbenzyl)methylamino]phenyl}thiazol-2-ylmethyl)piperidine-4-carboxylic acid;
   (253) 1-(4-{4-[(4-cyclohexylbenzyl)methylamino]phenyl}thiazol-2-ylmethyl)piperidine-3-carboxylic acid;
   (254) 1-(4-{4-[(4-cyclohexylbenzyl)methylamino]phenyl}thiazol-2-ylmethyl)-4-phenylpiperidine-4-carboxylic acid;
   (255) 1-(4-{4-[(4-cyclohexylbenzyl)methylamino]phenyl}thiazol-2-ylmethyl)-4-(3-methylbutyl)piperidine-4-carboxylic acid;
   (256) 1-[4-(4-{methyl[4-(trans-4-methylcyclohexyl)benzyl]amino}phenyl)thiazol-2-ylmethyl]-4-phenylpiperidine-4-carboxylic acid;
   (257) 1-[4-(4-{[trans-4-(4-chloro-phenyl)-cyclohexylmethyl]methylamino}phenyl)thiazol-2-ylmethyl]-4-phenylpiperidine-4-carboxylic acid;
   (258) 2-[4-(4-(methyl[4-(trans-4-methylcyclohexyl)benzyl]amino}phenyl)thiazol-2-ylmethyl]-1,2,3,4-tetrahydroisoquinoline-3-carboxylic acid;
   (259) 2-(4-{4-[(4-isobutylbenzyl)methylamino]phenyl}thiazol-2-ylmethyl)-1,2,3,4-tetrahydroisoquinoline-3-carboxylic acid;
   (260) 2-[4-(4-{methyl[4-(1-propylbutyl)benzyl]amino}phenyl)thiazol-2-ylmethyl]-1,2,3,4-tetrahydroisoquinoline-3-carboxylic acid;
   (261) 2-[4-(4-{[4-(2,2-dimethylpropyl)benzyl]methylamino}phenyl)thiazol-2-ylmethyl]-1,2,3,4-tetrahydroisoquinoline-3-carboxylic acid;
   (262) 5-{[(4-{4-[(4-cyclohexylbenzyl)methylamino]phenyl}thiazol-2-yl)methylamino]methyl}furan-2-carboxylic acid;
   (263) 2-[(4-{4-[(4-cyclohexylbenzyl)methylamino]phenyl}thiazol-2-ylmethyl)amino]-3-phenylpropionic acid;
   (264) [(4-{4-[(4-cyclohexylbenzyl)methylamino]phenyl}thiazol-2-ylmethyl)amino]phenylacetic acid;
   (265) 2-[(4-{4-[(4-cyclohexylbenzyl)methylamino]phenyl}thiazol-2-ylmethyl)amino]propionic acid;
   (266) 3-(4-chlorophenyl)-2-[(4-{4-[(4-isobutylbenzyl)methylamino]phenyl}thiazol-2-ylmethyl)amino]propionic acid;
   (267) [(4-{4-[(4-cyclohexylbenzyl)methylamino]phenyl}thiazol-2-ylmethyl)methylamino]acetic acid;
   (268) 3-[(4-{4-[(4-cyclohexylbenzyl)methylamino]phenyl}thiazol-2-ylmethyl)methylamino]propionic acid;
   (269) 4-{[(4-{4-[(4-cyclohexylbenzyl)methylamino]phenyl}thiazol-2-ylmethyl)methylamino]methyl}benzoic acid;
   (270) 4-[(4-{4-[(4-cyclohexylbenzyl)methylamino]phenyl}thiazol-2-ylmethyl)methylamino]benzoic acid;
   (271) 6-[(4-{4-[(4-cyclohexylbenzyl)methylamino]phenyl}thiazol-2-ylmethyl)methylamino]nicotinic acid;
   (272) 2-[(4-{4-[(4-isobutylbenzyl)methylamino]phenyl}thiazol-2-ylmethyl)methylamino]-3-phenylpropionic acid;
   (273) (S)-2-{methyl[4-(4-{methyl[4-(1-propylbutyl)benzyl]amino}phenyl)thiazol-2-ylmethyl]amino}-3-phenylpropionic acid;
   (274) (S)-{methyl[4-(4-{methyl[4-(1-propylbutyl)benzyl]amino}phenyl)thiazol-2-ylmethyl]amino}phenylacetic acid;
   (275) {[4-(4-{[4-(2,2-dimethylpropyl)benzyl]methylamino}phenyl)thiazol-2-ylmethyl]methylamino}phenylacetic acid;
   (276) 2-{[4-(4-{[4-(2,2-dimethylpropyl)benzyl]methylamino}phenyl)thiazol-2-ylmethyl]methylamino}-3-phenylpropionic acid;
   (277) {carboxymethyl[4-(4-{methyl[4-(1-propylbutyl)benzyl]amino}phenyl)thiazol-2-ylmethyl]amino}acetic acid;
   (278) [(4-{4-[(4-cyclohexylbenzyl)methylamino]phenyl}thiazol-2-ylmethyl)-(3-methylbutyl)amino]acetic acid;
   (279) {(3-methylbutyl)-[4-(4-{methyl[4-(trans-4-methylcyclohexyl)benzyl]amino}phenyl)thiazol-2-ylmethyl]amino}acetic acid;
   (280) [(4-{4-[(4-isobutylbenzyl)methylamino]phenyl}thiazol-2-ylmethyl)-(3-methylbutyl)amino]acetic acid;
   (281) 5-[4-(4-{methyl[4-(1-propylbutyl)benzyl]amino}phenyl)thiazol-2-ylmethoxy]nicotinic acid;
   (282) 4-[4-(4-{methyl[4-(1-propylbutyl)benzyl]amino}phenyl)thiazol-2-ylmethoxy]benzoic acid;
   (283) 4-[4-(4-{methyl[4-(1-propylbutyl)benzyl]amino}phenyl)thiazol-2-ylmethylthio]benzoic acid;
   (284) 4-[(4-{4-[(4-cyclohexylbenzyl)methylamino]phenyl}thiazol-2-ylmethyl)sulfamoyl]benzoic acid;
   (285) 4-{[4-(4-{[4-(4,4-dimethylcyclohexyl)benzyl]methylamino}phenyl)thiazol-2-ylmethyl]methylsulfamoyl}benzoic acid;
   (286) {[4-(4-{[4-(4,4-dimethylcyclohexyl)benzyl]methylamino}phenyl)thiazol-2-ylmethyl]methylsulfamoyl}acetic acid;
   (287) 4-[(4-(4-[(4-cyclohexylbenzyl)methylamino]phenyl}thiazol-2-ylmethyl)methylsulfamoyl]benzoic acid;
   (288) 3-[(4-{4-[(4-cyclohexylbenzyl)methylamino]phenyl}thiazol-2-ylmethyl)methylsulfamoyl]benzoic acid;
   (289) [(4-{4-[(4-cyclohexylbenzyl)methylamino]phenyl}thiazol-2-ylmethyl)methylsulfamoyl]acetic acid;
   (290) 4-{[4-(4-{[4-(4,4-dimethylcyclohexyl)benzyl]methylamino}phenyl)thiazol-2-ylmethyl]methylsulfamoyl}butyric acid;
   (291) [(4-{4-[(4-cyclohexylbenzyl)methylamino]phenyl}thiazol-2-ylmethyl)isobutyl-sulfamoyl]acetic acid;
   (292) N-(4-{4-[(4-cyclohexylbenzyl)methylamino]phenyl}thiazol-2-ylmethyl)oxamic acid;
   (293) {benzyl[4-(4-{[4-(1-propylbutyl)benzyl]methylaminocarbonyl}phenyl)thiazol-2-ylmethyl]amino}acetic acid;
   (294) N-(4-{4-[(4-cyclohexylbenzyl)methylamino]phenyl}thiazol-2-ylmethyl)-N-methylterephthalamic acid;
   (295) {benzyl[4-(4-{methyl[4-(trans-4-methylcyclohexyl)benzyl]amino}phenyl)thiazol-2-ylmethoxycarbonyl]amino}acetic acid;
   (296) [3-(4-{4-[(4-cyclohexylbenzyl)methylamino]phenyl}thiazol-2-ylmethyl)-3-methyl-ureido]acetic acid;
   (297) (cyclohexylmethyl{4-[6-(3,4-dichlorobenzyloxy)benzoxazol-2-yl]thiazol-2-yl}amino)acetic acid;
   (298) [4-(4-{4-[(4-cyclohexylbenzyl)methylamino]phenyl}thiazol-2-ylmethyl)piperazin-1-yl]acetic acid;
   (299) sodium (S)-2-(4-{4-[4-(1-propylbutyl)benzyloxy]phenyl}thiazol-2-ylmethyl)-1,2,3,4-tetrahydroisoquinoline-3-carboxylate;
   (300) sodium 5-(4-{4-[4-(1-propylbutyl)benzyloxy]phenyl}thiazol-2-ylmethoxy)nicotinate;
   (301) 5-(4-{2-methyl-4-[4-(1-propylbutyl)benzyloxy]phenyl}thiazol-2-ylmethoxy)nicotinic acid;
   (302) 5-(4-{3-methoxy-4-[4-(1-propylbutyl)benzyloxy]phenyl}thiazol-2-ylmethoxy)nicotinic acid;
   (303) [(4-{2-methyl-4-[4-(1-propylbutyl)benzyloxy]phenyl}thiazol-2-ylmethyl)phenylcarbamoylmethylamino]acetic acid;
   (304) [[(4-isopropylphenylcarbamoyl)methyl]-(4-{2-methyl-4-[4-(1-propylbutyl)benzyloxy]phenyl}thiazol-2-ylmethyl)amino]acetic acid;
   (305) 2-(4-{4-[4-(1-propylbutyl)benzyloxy]phenyl}thiazol-2-ylmethyl)-1,2,3,4-tetrahydroisoquinoline-3-carboxylic acid;
   (306) [(4-{3-methoxy-4-[4-(1-propylbutyl)benzyloxy]phenyl}thiazol-2-ylmethyl)phenylcarbamoylmethylamino]acetic acid;
   (307) [[(4-isopropylphenylcarbamoyl)methyl]-(4-{3-methoxy-4-[4-(1-propylbutyl)benzyloxy]phenyl}thiazol-2-ylmethyl)amino]acetic acid;
   (308) [(4-{2-methyl-4-[4-(1-propylbutyl)benzyloxy]phenyl}thiazol-2-ylmethyl)thiazol-4-ylmethylamino]acetic acid;
   (309) [(4-{2-methyl-4-[4-(1-propylbutyl)benzyloxy]phenyl}thiazol-2-ylmethyl)-(2-methylthiazol-4-ylmethyl)amino]acetic acid hydrochloride;
   (310) [(benzylcarbamoylmethyl)-(4-{2-methyl-4-[4-(1-propylbutyl)benzyloxy]phenyl}thiazol-2-ylmethyl)amino]acetic acid hydrochloride;
   (311) [(1H-benzimidazol-2-ylmethyl)-(4-{2-methyl-4-[4-(1-propylbutyl)benzyloxy]phenyl}thiazol-2-ylmethyl)amino]acetic acid;
   (312) [(4-tert-butylthiazol-2-ylmethyl)-(4-{1-[4-(1-propylbutyl)benzyl]-1,2,3,4-tetrahydroquinolin-6-yl}thiazol-2-ylmethyl)amino]acetic acid;
   (313) 1-(4-{4-[4-(1-propylbutyl)benzyloxy]phenyl}thiazol-2-ylmethyl)piperidine-4-carboxylic acid hydrochloride;
   (314) 4-[4-(4-{4-[4-(1-propylbutyl)benzyloxy]phenyl}thiazol-2-ylmethyl)piperazin-1-yl]benzoic acid;
   (315) 4-{4-[5-(1-ethylpropyl)-1-(4-isopropylbenzyl)-1H-benzimidazol-2-yl]thiazol-2-ylmethoxy}benzoic acid ethyl ester;
   (316) 4-{4-[5-(1-ethylpropyl)-1-(4-isopropylbenzyl)-1H-benzimidazol-2-yl]thiazol-2-ylmethoxy}benzoic acid;
   (317) 4-{4-[1-(4-acetylbenzyl)-5-(1-ethylpropyl)-1H-benzimidazol-2-yl]thiazol-2-ylmethoxy}benzoic acid;
   (318) 4-{4-[1-(4-acetylbenzyl)-6-(1-ethylpropyl)-1H-benzimidazol-2-yl]thiazol-2-ylmethoxy}benzoic acid;
   (319) 4-{4-[1-cyclohexylmethyl-5-(1-ethylpropyl)-1H-benzimidazol-2-yl]thiazol-2-ylmethoxy}benzoic acid;
   (320) [(1-methyl-1H-benzimidazol-2-ylmethyl)-(5-{4-[4-(1-propylbutyl)phenoxymethyl]phenyl}thiophen-2-ylmethyl)amino]acetic acid;
   (321) 5-(4-{4-[4-(1-propylbutyl)phenoxymethyl]phenyl}thiophen-2-ylmethoxy)nicotinic acid;
   (322) 5-{4-[4-({isopropyl[4-(1-propylbutyl)phenyl]amino}methyl)phenyl]thiophen-2-ylmethoxy}nicotinic acid;
   (323) 5-{4-[4-({[4-(1-ethylpropyl)phenyl]isopropylamino}methyl)phenyl]thiophen-2-ylmethoxy}nicotinic acid;
   (324) 5-(5-{4-[4-(1-propylbutyl)phenoxymethyl]phenyl}thiophen-2-ylmethoxy)nicotinic acid;
   (325) [(1H-benzimidazol-2-ylmethyl)-(4-{4-[4-(1-propylbutyl)phenoxymethyl]phenyl}thiophen-2-ylmethyl)amino]acetic acid;
   (326) 6-(4-{4-[4-(1-propylbutyl)phenoxymethyl]phenyl}thiophen-2-ylmethoxy)pyridine-2-carboxylic acid;
   (327) [(1H-benzimidazol-2-ylmethyl)-(5-{4-[4-(1-propylbutyl)phenoxymethyl]phenyl}thiophen-2-ylmethyl)amino]acetic acid;
   (328) [[(4-isopropylphenylcarbamoyl}methyl]-(5-{4-[4-(1-propylbutyl)phenoxymethyl]phenyl}thiophen-2-ylmethyl)amino]acetic acid;
   (329) [phenylcarbamoylmethyl(5-{4-[4-(1-propylbutyl)phenoxymethyl]phenyl}thiophen-2-ylmethyl)amino]acetic acid;
   (330) ({4-[4-({[4-(1-ethylpropyl)phenyl]isopropylamino}methyl)phenyl]thiophen-2-ylmethyl}phenylcarbamoylmethylamino)acetic acid;
   (331) ((4-tert-butylthiazol-2-ylmethyl)-{4-[4-({[4-(1-ethylpropyl)phenyl]isopropylamino}methyl)phenyl]thiophen-2-ylmethyl}amino)acetic acid;
   (332) [(5-tert-butylthiazol-2-ylmethyl)-(4-{4-[4-(1-propylbutyl)phenoxymethyl]phenyl}thiophen-2-ylmethyl)-amino]acetic acid;
   (333) ((1H-benzimidazol-2-ylmethyl)-{4-[4-({[4-(1-ethylpropyl)phenyl]isopropylamino}methyl)phenyl]thiophen-2-ylmethyl}amino)acetic acid;
   (334) [(4-tert-butylthiazol-2-ylmethyl)-(4-{4-[4-(1-propylbutyl)phenoxymethyl]phenyl}thiophen-2-ylmethyl)amino]acetic acid;
   (335) [phenylcarbamoylmethyl(4-{4-[4-(1-propylbutyl)phenoxymethyl]phenyl}thiophen-2-ylmethyl)amino]acetic acid;
   (336) 5-{5-[4-({[4-(1-ethylpropyl)phenyl]isopropylamino}methyl)phenyl]thiophen-2-ylmethoxy}nicotinic acid;
   (337) 5-{5-[4-({isopropyl[4-(1-propylbutyl)phenyl]amino}methyl)phenyl]thiophen-2-ylmethoxy}nicotinic acid;
   (338) ((4-tert-butylthiazol-2-ylmethyl)-{5-[4-({[4-(1-ethylpropyl)phenyl]isopropylamino}methyl)phenyl]thiophen-2-ylmethyl}amino)acetic acid;
   (339) ((4-tert-butylthiazol-2-ylmethyl)-{5-[4-({isopropyl[4-(1-propylbutyl)phenyl]amino}methyl)phenyl]thiophen-2-ylmethyl}amino)acetic acid;
   (340) ((1H-benzimidazol-2-ylmethyl)-{5-[4-({[4-(1-ethylpropyl)phenyl]isopropylamino}methyl)phenyl]thiophen-2-ylmethyl}amino)acetic acid;
   (341) ((1H-benzimidazol-2-ylmethyl)-{5-[4-({isopropyl[4-(1-propylbutyl)phenyl]amino}methyl)phenyl]thiophen-2-ylmethyl}amino)acetic acid;
   (342) [(4,5-dimethylthiazol-2-ylmethyl)- (4-{4-[4-(1-propylbutyl)phenoxymethyl]phenyl}thiophen-2-ylmethyl)amino]acetic acid;
   (343) (S)-2-{4-[4-({[4-(1-ethylpropyl)phenyl]isopropylamino}methyl)phenyl]thiophen-2-ylmethyl}-1,2,3,4-tetrahydroisoquinoline-3-carboxylic acid;
   (344) {{4-[4-({[4-(1-ethylpropyl)phenyl]isopropylamino}methyl)phenyl]thiophen-2-ylmethyl}-[(4-isopropylphenylcarbamoyl)methyl]amino}acetic acid;
   (345) (S)-2-(5-{4-[4-(1-propylbutyl)phenoxymethyl]phenyl}thiophen-2-ylmethyl)-1,2,3,4-tetrahydroisoquinoline-3-carboxylic acid;
   (346) [[(4-isopropylphenylcarbamoyl)methyl]-(4-{4-[4-(1-propylbutyl)phenoxymethyl]phenyl}thiophen-2-ylmethyl)amino]acetic acid;
   (347) (S)-2-{5-[4-({[4-(1-ethylpropyl)phenyl]isopropylamino}methyl)phenyl]thiophen-2-ylmethyl}-1,2,3,4-tetrahydroisoquinoline-3-carboxylic acid;
   (348) [(5-tert-butyloxazol-2-ylmethyl)-(4-{4-[4-(1-propylbutyl)phenoxymethyl]phenyl}thiophen-2-ylmethyl)amino]acetic acid;
   (349) [(1-methyl-1H-benzimidazol-2-ylmethyl)-(4-{4-[4-(1-propylbutyl)phenoxymethyl]phenyl}thiophen-2-ylmethyl)amino]acetic acid;
   (350) 6-(5-{4-[4-(1-propylbutyl)phenoxymethyl]phenyl}thiophen-2-ylmethoxy)pyridine-2-carboxylic acid;
   (351) 6-{4-[4-({[4-(1-ethylpropyl)phenyl]isopropylamino}methyl)phenyl]thiophen-2-ylmethoxy}-pyridine-2-carboxylic acid;
   (352) ((5-tert-butylthiazol-2-ylmethyl)-{4-[4-({[4-(1-ethylpropyl)phenyl]isopropylamino}methyl)phenyl]thiophen-2-ylmethyl}amino)acetic acid;
   (353) [{4-[4-({[4-(1-ethylpropyl)phenyl]isopropylamino}methyl)phenyl]thiophen-2-ylmethyl}-(1-methyl-1H-benzimidazol-2-ylmethyl)amino]acetic acid;
   (354) [(4-tert-butylthiazol-2-ylmethyl)-(5-{4-[4-(1-propylbutyl)phenoxymethyl]phenyl}thiophen-2-ylmethyl)amino]acetic acid;
   (355) [{5-[4-({[4-(1-ethylpropyl)phenyl]isopropylamino}methyl)phenyl]thiophen-2-ylmethyl}-(1-methyl-1H-benzimidazol-2-ylmethyl)amino]acetic acid;
   (356) [(5-tert-butylthiazol-2-ylmethyl)-(5-{4-[4-(1-propylbutyl)phenoxymethyl]phenyl}thiophen-2-ylmethyl)amino]acetic acid;
   (357) sodium [(1-methyl-1H-benzimidazol-2-ylmethyl)-(5-{4-[4-(1-propylbutyl)phenoxymethyl]phenyl}thiophen-2-ylmethyl)amino]-acetate;
   (358) calcium bis{[(1-methyl-1H-benzimidazol-2-ylmethyl)-(5-{4-[4-(1-propylbutyl)phenoxymethyl]phenyl}thiophen-2-ylmethyl)amino]-acetate};
   (359) [(1-methyl-1H-benzimidazol-2-ylmethyl)-(5-{4-[4-(1-propylbutyl)phenoxymethyl]phenyl}thiophen-2-ylmethyl)amino]acetic acid toluene-4-sulfonate;
   (360) 5-(4-{4-[4-(1-propylbutyl)phenoxymethyl]phenyl}thiophen-2-ylmethoxy)nicotinic acid sulfate;
   (361) [(1-methyl-1H-benzimidazol-2-ylmethyl)-(5-{4-[4-(1-propylbutyl)phenoxymethyl]phenyl}thiophene-2-carbonyl)amino]acetic acid;
   (362) [(5-tert-butylthiazol-2-ylmethyl)-(5-{4-[4-(1-propylbutyl)phenoxymethyl]phenyl}thiophene-2-carbonyl)amino]acetic acid;
   (363) [(4-isopropylbenzyl)-(5-{4-[4-(1-propylbutyl)phenoxymethyl]phenyl}thiophene-2-carbonyl)amino]acetic acid;
   (364) [(4-dimethylaminobenzyl)-(5-{4-[4-(1-propylbutyl)phenoxymethyl]phenyl}thiophene-2-carbonyl)amino]acetic acid;
   (365) [(5-{4-[4-(1-propylbutyl)phenoxymethyl]phenyl}thiophene-2-carbonyl)-pyridin-2-ylmethylamino]acetic acid;
   (366) [(5-{4-[4-(1-propylbutyl)phenoxymethyl]phenyl}thiophene-2-carbonyl)-pyridin-3-ylmethylamino]acetic acid;
   (367) 5-(4-{4-[4-(1-propylbutyl)phenoxymethyl]phenyl}thiophen-2-ylmethoxy)nicotinic acid methanesulfonate;
   (368) [methanesulfonyl-(5-{4-[4-(1-propylbutyl)phenoxymethyl]phenyl}thiophen-2-ylmethyl)amino]acetic acid;
   (369) sodium 5-(4-{4-[4-(1-propylbutyl)phenoxymethyl]phenyl}thiophen-2-ylmethoxy)nicotinate;
   (370) 5-(4-{4-[4-(1-propylbutyl)phenoxymethyl]phenyl}thiophen-2-ylmethoxy)nicotinic acid hydrochloride;
   (371) 4-[4-(5-{4-[4-(1-propylbutyl)phenoxymethyl]phenyl}thiophene-2-carbonyl)piperazin-1-yl]benzoic acid;
   (372) 4-[1-(5-(4-[4-(1-propylbutyl)phenoxymethyl]phenyl}thiophene-2-carbonyl)piperidin-4-yl]benzoic acid;
   (373) [(1-methyl-1H-benzimidazol-2-ylmethyl)-(4-{4-[4-(1-propylbutyl)phenoxymethyl]phenyl}thiophen-2-ylmethyl)amino]acetic acid hydrochloride;
   (374) [(1-methyl-1H-benzimidazol-2-ylmethyl)-(4-{4-[4-(1-propylbutyl)phenoxymethyl]phenyl}thiophen-2-ylmethyl)amino]acetic acid sulfate;
   (375) 4-(2-dimethylaminoacetylamino)-3-(4-{4-[4-(1-propylbutyl)phenoxymethyl]phenyl}thiophen-2-ylmethoxy)benzoic acid;
   (376) 4-isobutyrylamino-3-(4-{4-[4-(1-propylbutyl)phenoxymethyl]phenyl}thiophen-2-ylmethoxy)benzoic acid;
   (377) 4-(4-{4-[4-(1-propylbutyl)phenoxymethyl)phenyl}thiophen-2-ylmethoxy)benzoic acid;
   (378) 4-(methanesulfonylmethylamino)-3-(4-{4-[4-(1-propylbutyl)phenoxymethyl]phenyl}thiophen-2-ylmethoxy)benzoic acid;
   (379) 4-(4-{4-[4-(1-propylbutyl)phenoxymethyl]phenyl}thiophen-2-ylmethylthio)benzoic acid;
   (380) 4-amino-3-(4-{4-[4-(1-propylbutyl)phenoxymethyl]phenyl}thiophen-2-ylmethoxy)benzoic acid;
   (381) 1-{5-[1-(4-cyclohexylbenzyl)-1H-indol-3-yl]thiophen-2-ylmethyl}-4-phenylpiperidine-4-carboxylic acid hydrochloride;
   (382) (benzyl{5-[1-(4-cyclohexylbenzyl)-1H-indol-3-yl]thiophen-2-ylmethyl}amino)acetic acid hydrochloride;
   (383) [(methylphenylsulfamoyl)(4-{4-[4-(1-propylbutyl)phenoxymethyl]phenyl}thiophen-2-ylmethyl)amino]acetic acid;
   (384) [(5-tert-butylthiazol-2-ylmethyl)-(5-{4-[(4-cyclohexylphenyl)methylcarbamoyl]phenyl}thiophen-2-ylmethyl)amino]acetic acid;
   (385) [(5-{4-[(4-cyclohexylbenzyl)ethylamino]phenyl}thiophen-2-ylmethyl)pyridin-2-ylmethylamino]acetic acid;
   (386) [(5-{4-[(4-cyclohexylbenzyl)ethylamino]phenyl}thiophen-2-ylmethyl)-(2-phenoxyethyl)amino]acetic acid;
   (387) 4-{1-[4-(4-{[trans-4-(4-tert-butylphenyl)-cyclohexylmethyl]ethylamino}phenyl)thiophen-2-ylmethyl]piperidin-4-yl}benzoic acid;
   (388) [[2-(4-isopropylphenoxy)acetyl]-(4-{4-[4-(1-propylbutyl)phenoxymethyl]phenyl}thiophen-2-ylmethyl)amino]acetic acid;
   (389) [(4-isopropylbenzoyl)-(4-{4-[4-(1-propylbutyl)phenoxymethyl]phenyl}thiophen-2-ylmethyl)amino]acetic acid;
   (390) [(3-methylbutyl)-(4-{4-[4-(1-propylbutyl)phenoxymethyl]phenyl}thiophen-2-ylmethyl)amino]acetic acid;
   (391) [3-methyl-3-phenyl-1-(4-{4-[4-(1-propylbutyl)phenoxymethyl]phenyl}thiophen-2-ylmethyl)ureido]acetic acid;
   (392) 2-(4-{3-[4-(1-propylbutyl)phenoxymethyl]phenyl}thiophen-2-ylmethyl)-1,2,3,4-tetrahydroisoquinoline-7-carboxylic acid;
   (393) 4-[4-(5-{4-[4-(trans-4-methylcyclohexyl)benzyloxy]phenyl}thiophen-2-ylmethyl)piperazin-1-yl]benzoic acid;
   (394) [(2-chloro-5-trifluoromethylbenzyl)-(5-{4-[4-(1-propylbutyl)phenoxymethyl]phenyl}thiophen-2-ylmethyl)amino]acetic acid;
   (395) 3-{[carboxymethyl(5-{4-[4-(1-propylbutyl)phenoxymethyl]phenyl}thiophen-2-ylmethyl)amino]methyl}benzoic acid;
   (396) [(4-methoxybenzyl)-(5-{4-[4-(1-propylbutyl)phenoxymethyl]phenyl}thiophen-2-ylmethyl)amino]acetic acid;
   (397) [(4-methylthiobenzyl)-(5-{4-[4-(1-propylbutyl)phenoxymethyl]phenyl}thiophen-2-ylmethyl)amino]acetic acid;
   (398) 4-[cyclohexylmethyl(5-{4-[4-(trans-4-methylcyclohexyl)benzyloxy]phenyl}thiophen-2-ylmethyl)sulfamoyl]benzoic acid;
   (399) 4-[3-cyclohexylmethyl-3-(5-{4-[4-(trans-4-methylcyclohexyl)benzyloxy]phenyl}thiophen-2-ylmethyl)ureido]benzoic acid;
   (400) [benzhydryl-(5-{4-[4-(1-propylbutyl)phenoxymethyl]phenyl}thiophen-2-ylmethyl)amino]acetic acid;
   (401) [[2-oxo-2-(4-pyrrolidin-1-yl-phenyl)ethyl]-(5-{4-[4-(1-propylbutyl)phenoxymethyl]phenyl}thiophen-2-ylmethyl)amino]acetic acid ethyl ester hydrochloride;
   (402) [(1-methyl-1H-benzimidazol-Z-ylmethyl)-(5-{4-[4-(1-propylbutyl)phenoxymethyl]phenyl}thiophen-2-ylmethyl)amino]acetic acid ethyl ester;
   (403) 3-(benzyl{5-[1-(4-cyclohexylbenzyl)-2,3-dihydro-1H-indol-5-yl]thiophen-2-ylmethyl}amino)propionic acid;
   (404) [benzyl(4-{4-[2-(2,2-dimethylpropyl)benzimidazol-1-ylmethyl]phenyl}thiophen-2-ylmethyl)amino]acetic acid;
   (405) [(1-methyl-1H-indol-3-ylmethyl)-(4-{4-[4-(1-propylbutyl)phenoxymethyl]phenyl}thiophen-2-ylmethyl)amino]acetic acid;
   (406) [(4-{4-[4-(1-propylbutyl)phenoxymethyl]phenyl}thiophen-2-ylmethyl)quinolin-2-ylmethylamino]acetic acid;
   (407) [benzothiazol-2-ylmethyl(4-{4-[4-(1-propylbutyl)phenoxymethyl]phenyl}thiophen-2-ylmethyl)amino]acetic acid;
   (408) [(1-benzyl-1H-imidazol-2-ylmethyl)-(4-{4-[4-(1-propylbutyl)phenoxymethyl]phenyl}thiophen-2-ylmethyl)amino]acetic acid;
   (409) [(1H-indol-5-ylmethyl)-(4-{4-[4-(1-propylbutyl)phenoxymethyl]phenyl}thiophen-2-ylmethyl)amino]acetic acid;
   (410) [(4-imidazol-1-ylbenzyl)-(4-{4-[4-(1-propylbutyl)phenoxymethyl]phenyl}thiophen-2-ylmethyl)amino]acetic acid;
   (411) [benzofuran-2-ylmethyl(4-{4-[4-(1-propylbutyl)phenoxymethyl]phenyl}thiophen-2-ylmethyl)amino]acetic acid;
   (412) [[2,2']bithiophenyl-5-ylmethyl(4-{4-[4-(1-propylbutyl)phenoxymethyl]phenyl}thiophen-2-ylmethyl)amino]acetic acid;
   (413) [(2-phenyl-1H-imidazol-4-ylmethyl)-(5-{4-[4-(1-propylbutyl)phenoxymethyl]phenyl}thiophen-2-ylmethyl)amino]acetic acid;
   (414) [(3-phenyl-1H-pyrazol-4-ylmethyl)-(5-{4-[4-(1-propylbutyl)phenoxymethyl]phenyl}thiophen-2-ylmethyl)amino]acetic acid;
   (415) [benzoxazol-2-ylmethyl(5-{4-[4-(1-propylbutyl)phenoxymethyl]phenyl}thiophen-2-ylmethyl)amino]acetic acid;
   (416) [benzo[b]thiophen-2-ylmethyl(5-{4-[4-(1-propylbutyl)phenoxymethyl]phenyl}thiophen-2-ylmethyl)amino]acetic acid;
   (417) [(4-phenylthiophen-2-ylmethyl)-(5-{4-[4-(1-propylbutyl)phenoxymethyl]phenyl}thiophen-2-ylmethyl)amino]acetic acid;
   (418) [benzothiazol-2-yl-(5-{4-[4-(1-propylbutyl)phenoxymethyl]phenyl}thiophen-2-ylmethyl)amino]acetic acid;
   (419) [(5-chlorothiophene-2-sulfonyl)-(5-{4-[4-(1-propylbutyl)phenoxymethyl]phenyl}thiophen-2-ylmethyl)amino]acetic acid;
   (420) [(1-diethylcarbamoylmethyl-1H-benzimidazol-2-ylmethyl)-(5-{4-[4-(1-propylbutyl)phenoxymethyl]phenyl}thiophen-2-ylmethyl)amino]acetic acid;
   (421) (S)-({4-[2-(4-cyclohexylbenzyloxy)-5-methylphenyl]thiophen-2-ylmethyl}methylamino)-3-phenylpropionic acid;
   (422) [benzyl(4-{4-[(4-butoxybenzenesulfonyl)ethylamino]phenyl}thiophen-2-ylmethyl)amino]acetic acid;
   (423) N-{4-[2-(4-cyclohexylbenzyloxy)-5-methylphenyl]thiophen-2-ylmethyl}-N-(2-methylbenzothiazol-6-yl)oxamic acid;
   (424) (benzyl{4-[4-(3,5-dichlorophenoxymethyl)phenyl]thiophen-2-ylmethyl}amino)acetic acid;
   (425) [(1-allyl-1H-benzimidazol-2-ylmethyl)-(5-{4-[4-(1-propylbutyl)phenoxymethyl]phenyl}thiophen-2-ylmethyl)amino]acetic acid; and
   (426) [[4-(4-chlorophenyl)thiazol-2-yl]-(5-{4-[4-(1-propylbutyl)phenoxymethyl]phenyl}thiophen-2-ylmethyl)amino]acetic acid.
[24] The 5-membered heteroaromatic ring compound of any of [1] to [23], which is selected from the group consisting of the following compounds, or a prodrug thereof, or a pharmaceutically acceptable salt thereof:
   (1) (S)-2-(4-{4-[4-(1-propylbutyl)phenoxymethyl]phenyl}thiazol-2-ylmethyl)-1,2,3,4-tetrahydroisoquinoline-3-carboxylic acid;
   (2) {{4-[4-({[4-(1-ethylpropyl)phenyl]isopropylamino}methyl)phenyl]thiazol-2-ylmethyl}-[(4-isopropylphenylcarbamoyl)methyl]amino}acetic acid;
   (3) 4-(3-isobutyl-3-{4-[4-({isopropyl[4-(1-propylbutyl)phenyl]amino}methyl)phenyl]thiazol-2-ylmethyl}ureido)benzoic acid;
   (4) ({4-[4-({isobutyl[4-(1-propylbutyl)phenyl]amino}methyl)phenyl]thiazol-2-ylmethyl}phenylcarbamoylmethylamino)acetic acid;
   (5) ([(4-isopropylphenylcarbamoyl)methyl]-{4-[4-({isopropyl[4-(1-propylbutyl)phenyl]amino}methyl)phenyl]thiazol-2-ylmethyl}amino)acetic acid;
   (6) [(4-tert-butylthiazol-2-ylmethyl)-(4-{4-[4-(1-propylbutyl)phenoxymethyl]phenyl}thiazol-2-ylmethyl)amino]acetic acid;
   (7) [(4,5-dimethylthiazol-2-ylmethyl)-(4-{4-[4-(1-propylbutyl)phenoxymethyl]phenyl}thiazol-2-ylmethyl)amino]acetic acid;
   (8) [(5-tert-butylthiazol-2-ylmethyl)-(4-{4-[4-(1-propylbutyl)phenoxymethyl]phenyl}thiazol-2-ylmethyl)amino]acetic acid;
   (9) [[2-(4-isopropylphenoxy)acetyl]-(4-{4-[4-(1-propylbutyl)phenoxymethyl]phenyl}thiazol-2-ylmethyl)amino]acetic acid;
   (10) 4-[4-(4-{4-[4-(1-propylbutyl)phenoxymethyl]phenyl}thiazol-2-ylmethyl)piperazin-1-yl]benzoic acid;
   (11) {benzyl[4-(4-{methyl[4-(1-propylbutyl)benzyl]amino}phenyl)thiazol-2-ylmethyl]amino}acetic acid;
   (12) [(1-methyl-1H-benzimidazol-2-ylmethyl)-5-{4-[4-(1-propylbutyl)phenoxymethyl]phenyl}thiophen-2-ylmethyl)amino]acetic acid;
   (13) 5-(4-{4-[4-(1-propylbutyl)phenoxymethyl]phenyl}thiophen-2-ylmethoxy}nicotinic acid;
   (14) [(1H-benzimidazol-2-ylmethyl)-(4-{4-[4-(1-propylbutyl)phenoxymethyl]phenyl}thiophen-2-ylmethyl)amino]acetic acid;
   (15) [(1H-benzimidazol-2-ylmethyl)-(5-{4-[4-(1-propylbutyl)phenoxymethyl]phenyl}thiophen-2-ylmethyl)amino]acetic acid;
   (16) [[(4-isopropylphenylcarbamoyl)methyl]-(5-{4-[4-(1-propylbutyl)phenoxymethyl]phenyl}thiophen-2-ylmethyl)amino]acetic acid;
   (17) (S)-2-{4-[4-({[4-(1-ethylpropyl)phenyl]isopropylamino}methyl)phenyl]thiophen-2-ylmethyl}-1,2,3,4-tetrahydroisoquinoline-3-carboxylic acid;
   (18) {{4-[4-({[4-(1-ethylpropyl)phenyl]isopropylamino}methyl)phenyl]thiophen-2-ylmethyl}-[(4-isopropylphenylcarbamoyl)methyl)amino}acetic acid;
   (19) [(1-methyl-1H-benzimidazol-2-ylmethyl)-(4-{4-[4-(1-propylbutyl)phenoxymethyl]phenyl}thiophen-2-ylmethyl)amino]acetic acid; and
   (20) [(4-tert-butylthiazol-2-ylmethyl)-(5-{4-[4-(1-propylbutyl)phenoxymethyl]phenyl}thiophen-2-ylmethyl)amino]acetic acid.
[25] A pharmaceutical composition comprising the 5-membered heteroaromatic ring compound of any of [1] to [24], or a prodrug thereof, or a pharmaceutically acceptable salt thereof and a pharmaceutically acceptable carrier.
[26] A pharmaceutical composition for inhibition of a receptor tyrosine kinase negative regulator, which comprises a 5-membered heteroaromatic ring compound of any of [1] to [24], or a prodrug thereof, or a pharmaceutically acceptable salt thereof and a pharmaceutically acceptable carrier.
[27] A pharmaceutical composition for inhibition of protein tyrosine phosphatase 1B, which comprises a 5-membered heteroaromatic ring compound of any of [1] to [24], or a prodrug thereof, or a pharmaceutically acceptable salt thereof and a pharmaceutically acceptable carrier.
[28] A pharmaceutical composition for the prophylaxis or treatment of diabetes, which comprises a 5-membered heteroaromatic ring compound of any of [1] to [24], or a prodrug thereof, or a pharmaceutically acceptable salt thereof and a pharmaceutically acceptable carrier.
[29] A pharmaceutical composition for the prophylaxis or treatment of hyperlipidemia, which comprises a 5-membered heteroaromatic ring compound of any of [1] to [24], or a prodrug thereof, or a pharmaceutically acceptable salt thereof and a pharmaceutically acceptable carrier.
[30] A pharmaceutical composition for the prophylaxis or treatment of obesity, which comprises a 5-membered heteroaromatic ring compound of any of [1] to [24], or a prodrug thereof, or a pharmaceutically acceptable salt thereof and a pharmaceutically acceptable carrier.
[31] A pharmaceutical composition for the prophylaxis or treatment of diabetic complications, which comprises a 5-membered heteroaromatic ring compound of any of [1] to [24], or a prodrug thereof, or a pharmaceutically acceptable salt thereof and a pharmaceutically acceptable carrier.
[32] The pharmaceutical composition of [25], which is used in combination with a therapeutic agent for hyperlipidemia.
[33] The pharmaceutical composition of [32], wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.
[34] The pharmaceutical composition of [33], wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).
[35] The pharmaceutical composition of [25], which is used in combination with a therapeutic agent for diabetes.
[36] The pharmaceutical composition of [35], wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors, antioxidants, carnitine acetyltransferase stimulant, antidepressants, glucocorticoids, retilin, radical formation agonists and transketolase activators.
[37] The pharmaceutical composition of [36], wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.
[38] The pharmaceutical composition of [25], which is used in combination with a therapeutic agent for obesity.
[39] The pharmaceutical composition of [38], wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.
[40] The pharmaceutical composition of [39], wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.
[41] The pharmaceutical composition of [25], which is used in combination with a therapeutic agent for hypertension.
[42] The pharmaceutical composition of [41], wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alphal-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.
[43] The pharmaceutical composition of [42], wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.
[44] The pharmaceutical composition of [25], which is used in combination with a therapeutic agent for thrombosis.
[45] The pharmaceutical composition of [44], wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.
[46] The pharmaceutical composition of [45], wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.
[47] The pharmaceutical composition for the prophylaxis or treatment of diabetes according to [28], which is used in combination with a therapeutic agent for hyperlipidemia.
[48] The pharmaceutical composition of [47], wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.
[49] The pharmaceutical composition of [48], wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).
[50] The pharmaceutical composition for the prophylaxis or treatment of diabetes according to [28], which is used in combination with a different therapeutic agent for diabetes.
[51] The pharmaceutical composition of [50], wherein the different therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors,antioxidants, carnitine acetyltransferase stimulant, antidepressants, glucocorticoids, retilin, radical formation agonists and transketolase activators.
[52] The pharmaceutical composition of [51], wherein the different therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.
[53] The pharmaceutical composition for the prophylaxis or treatment of diabetes according to [28], which is used in combination with a therapeutic agent for obesity.
[54] The pharmaceutical composition of [53], wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.
[55] The pharmaceutical composition of [5'4], wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.
[56] The pharmaceutical composition for the prophylaxis or treatment of diabetes according to [28], which is used in combination with a therapeutic agent for hypertension.
[57] The pharmaceutical composition of [56], wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.
[58] The pharmaceutical composition of [57], wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.
[59] A pharmaceutical composition for the prophylaxis or treatment of diabetes according to [28], which is used in combination with a therapeutic agent for thrombosis.
[60] The pharmaceutical composition of [59], wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations antiplatelet agents and thrombolytic agents.
[61] The pharmaceutical composition of [60], wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.
[62] A pharmaceutical composition for the prophylaxis or treatment of hyperlipidemia according to [29], which is used ir combination with a different therapeutic agent for hyperlipidemia.
[63] The pharmaceutical composition of [62], wherein the different therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.
[64] The pharmaceutical composition of [63], wherein the different therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).
[65] A pharmaceutical composition for the prophylaxis or treatment of hyperlipidemia according to [29], which is used in combination with a therapeutic agent for diabetes.
[66] The pharmaceutical composition of [65], wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors, antioxidants, carnitine acetyltransferase stimulant, antidepressants, glucocorticoids, retilin, radical formation agonists and transketolase activators.
[67] The pharmaceutical composition of [66], wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.
[68] A pharmaceutical composition for the prophylaxis or treatment of hyperlipidemia according to [29], which is used i combination with a therapeutic agent for obesity.
[69] The pharmaceutical composition of [68], wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.
[70] The pharmaceutical composition of [69], wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.
[71] A pharmaceutical composition for the prophylaxis or treatment of hyperlipidemia according to [29], which is used in combination with a therapeutic agent for hypertension.
[72] The pharmaceutical composition of [71], wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.
[73] The pharmaceutical composition of [72], wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.
[74] A pharmaceutical composition for the prophylaxis or treatment of hyperlipidemia according to [29], which is used in combination with a therapeutic agent for thrombosis.
[75] The pharmaceutical composition of [74], wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.
[76] The pharmaceutical composition of [75], wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.
[77] A pharmaceutical composition for the prophylaxis or treatment of obesity according to [30], which is used in combination with a therapeutic agent for hyperlipidemia.
[78] The pharmaceutical composition of [77], wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.
[79] The pharmaceutical composition of [78], wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).
[80] A pharmaceutical composition for the prophylaxis or treatment of obesity according to [30], which is used in combination with a therapeutic agent for diabetes.
[81] The pharmaceutical composition of [80], wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors, antioxidants, carnitine acetyltransferase stimulant, antidepressants, glucocorticoids, retilin, radical formation agonists and transketolase activators.
[82] The pharmaceutical composition of [81], wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.
[83] A pharmaceutical composition for the prophylaxis or treatment of obesity according to [30], which is used in combination with a different therapeutic agent for obesity.
[84] The pharmaceutical composition of [83], wherein the different therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.
[85] The pharmaceutical composition of [84], wherein the different therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.
[86] A pharmaceutical composition for the prophylaxis or treatment of obesity according to [30], which is used in combination with a therapeutic agent for hypertensiona.
[87] The pharmaceutical composition of [86], wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alphal-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.
[88] The pharmaceutical composition of [87], wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.
[89] A pharmaceutical composition for the prophylaxis or treatment of obesity according to [30], which is used in combination with a therapeutic agent for thrombosis.
[90] The pharmaceutical composition of [89], wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.
[91] The pharmaceutical composition of [90], wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.
[92] A pharmaceutical composition for the prophylaxis or treatment of diabetic complications according to [31], which is used in combination with a therapeutic agent for hyperlipidemia.
[93] The pharmaceutical composition of [92], wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.
[94] The pharmaceutical composition of [93], wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).
[95] A pharmaceutical composition for the prophylaxis or treatment of diabetic complications according to [31], which is used in combination with a therapeutic agent for diabetes.
[96] The pharmaceutical composition of [95], wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors, antioxidants, carnitine acetyltransferase stimulant, antidepressants, glucocorticoids, retilin, radical formation agonists and transketolase activators.
[97] The pharmaceutical composition of [96], wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.
[98] A pharmaceutical composition for the prophylaxis or treatment of diabetic complications according to [31], which is used in combination with a therapeutic agent for obesity.
[99] The pharmaceutical composition of [98], wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.
[100] The pharmaceutical composition of [99], wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.
[101] A pharmaceutical composition for the prophylaxis or treatment of diabetic complications according to [31], which is used in combination with a therapeutic agent for hypertension.
[102] The pharmaceutical composition of [101], wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.
[103] The pharmaceutical composition of [102], wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.
[104] A pharmaceutical composition for the prophylaxis or treatment of diabetes according to [31], which is used in combination with a therapeutic agent for thrombosis.
[105] The pharmaceutical composition of [104], wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.
[106] The pharmaceutical composition of [105] wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.
[107] A pharmaceutical composition for inhibition of a receptor tyrosine kinase negative regulator, which is used in combination with a therapeutic agent for hyperlipidemia.
[108] A pharmaceutical composition for inhibition of a receptor tyrosine kinase negative regulator, which is used in combination with a therapeutic agent for diabetes.
[109] A pharmaceutical composition for inhibition of a receptor tyrosine kinase negative regulator, which is used in combination with a therapeutic agent for obesity.
[110] A pharmaceutical composition for inhibition of a receptor tyrosine kinase negative regulator, which is used in combination with a therapeutic agent for hypertension.
[111] A pharmaceutical composition for inhibition of a receptor tyrosine kinase negative regulator, which is used in combination with a therapeutic agent for thrombosis.
[112] A 5-membered heteroaromatic ring compound represented by the formula [II] 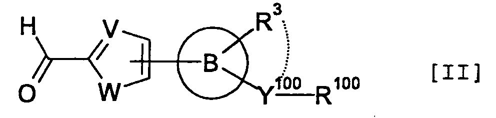 wherein
   Y¹⁰⁰ is -C(R¹³)(R¹⁴)- (R¹³ and R¹⁴ are each as defined in [1]);
   R¹⁰⁰ is a hydroxyl group or a halogen atom, and
   V, W and R³ are each as defined in [1], or a pharmaceutically acceptable salt thereof.
[113] The 5-membered heteroaromatic ring compound of [112],
wherein, in the formula [II], R¹³ and R¹⁴ are each a hydrogen atom, V is =CH- and W is -S-, or a pharmaceutically acceptable salt thereof.

### DETAILED DESCRIPTION OF THE INVENTION

The definition of each substituent and each moiety used in the present specification is as follows.

The "halogen atom" is a fluorine atom, a chlorine atom, a bromine atom or an iodine atom, preferably a fluorine atom or a chlorine atom.

The "C₁₋₄ alkyl group" is a straight chain or branched chain alkyl group having 1 to 4 carbon atoms, which is specifically exemplified by methyl group, ethyl group, propyl group, isopropyl group, butyl group, isobutyl group, sec-butyl group and tert-butyl group.

It is preferably methyl group for R¹ or R², and methyl group or ethyl group for R¹³, R¹⁴, R¹⁹, R²² or R²⁵.

The "C₁₋₆ alkyl group" is a straight chain or branched chain alkyl group having 1 to 6 carbon atoms, which is specifically exemplified by methyl group, ethyl group, propyl group, isopropyl group, butyl group, isobutyl group, sec-butyl group, tert-butyl group, pentyl group, isopentyl group, neopentyl group, tert-pentyl group, 1-ethylpropyl group, hexyl group and the like. Preferred are branched chain alkyl group, methyl group and ethyl group, each having 3 to 6 carbon atoms, such as isopropyl group, isobutyl group, tert-butyl group, isopentyl group, neopentyl group, tert-pentyl group, 1-ethylpropyl group and the like.

Preferably, it is methyl group, ethyl group or isopentyl group for R⁴, methyl group or isobutyl group for R⁵, R⁶, R⁷, R⁸, R⁹ or R¹⁰, methyl group, ethyl group or isopropyl group for R¹⁵ or R¹⁶, methyl group or isobutyl group for R¹⁷, methyl group or isopentyl group for R²⁰ or R²¹, and methyl group, isobutyl group, tert-butyl group, isopentyl group, neopentyl group, tert-pentyl group or 1-ethylpropyl group for R¹⁸, R²³ or R²⁴.

The "C₁₋₈ alkyl group" is a straight chain or branched chain alkyl group having 1 to 8 carbon atoms, which is specifically exemplified by methyl group, ethyl group, propyl group, isopropyl group, butyl group, isobutyl group, sec-butyl group, tert-butyl group, pentyl group, isopentyl group, neopentyl group, tert-pentyl group, 1-ethylpropyl group, hexyl group, 2-ethylbutyl group, 3,3-dimethylbutyl group, heptyl group, 1-propylbutyl group, 3-ethylpentyl group, octyl group and the like. Preferably, it is methyl group, ethyl group, propyl group, isopropyl group or a branched chain alkyl group having 4 to 8 carbon atoms such as isobutyl group, sec-butyl group, tert-butyl group, isopentyl group, neopentyl group, tert-pentyl group, 1-ethylpropyl group, 2-ethylbutyl group, 1-propylbutyl group and the like.

Preferably, it is methyl group, tert-butyl group or 1-ethylpropyl group for R³, and methyl group, ethyl group, propyl group, isopropyl group, isobutyl group, sec-butyl group, tert-butyl group, isopentyl group, neopentyl group, tert-pentyl group, 1-ethylpropyl group, 2-ethylbutyl group or 1-propylbutyl group for R¹¹ or R¹². It is particularly preferably isopropyl group for R¹¹ or R¹².

The "C₁₋₄ haloalkyl group" is a haloalkyl group wherein a straight chain or branched chain alkyl group having 1 to 4 carbon atoms is substituted by the above-defined "halogen atom", which is specifically exemplified by fluoromethyl group, difluoromethyl group, trifluoromethyl group, bromomethyl group, chloromethyl group, 1,2-dichloromethyl group, 2,2-dichloromethyl group, 2,2,2-trifluoroethyl group and the like, with preference given to trifluoromethyl group.

It is preferably trifluoromethyl group for R³.

The "C₁₋₄ alkylene group" is a straight chain or branched chain alkylene group having 1 to 4 carbon atoms, which is specifically exemplified by methylene group, ethylene group, trimethylene group, propylene group, tetramethylene group and the like. Preferably, it is methylene group or ethylene group, more preferably methylene group.

Preferably, it is methylene group or ethylene group for A, and methylene group for A¹.

The "C₁₋₄ alkoxy group" is a straight chain or branched chain alkoxy group having 1 to 4 carbon atoms, which is specifically exemplified by methoxy group, ethoxy group, propoxy group, isopropoxy group, butoxy group, isobutoxy group, tert-butoxy group and the like. Preferably, it is methoxy group.

Preferably, it is methoxy group for R²² or R²⁵.

The "C₁₋₆ alkoxy group" is a straight chain or branched chain alkoxy group having 1 to 6 carbon atoms, which is specifically exemplified by methoxy group, ethoxy group, propoxy group, isopropoxy group, butoxy group, isobutoxy group, tert-butoxy group, pentyloxy group, isopentyloxy group, neopentyloxy group, tert-pentyloxy group, 1-ethylpropoxy group, hexyloxy group and the like. It is preferably methoxy group, ethoxy group or branched chain alkoxy group having 3 to 6 carbon atoms (e.g., isopropoxy group, isobutoxy group, tert-butoxy group, isopentyloxy group, neopentyloxy group, tert-pentyloxy group, 1-ethylpropoxy group etc.).

Preferably, it is methoxy group, ethoxy group, isopropoxy group, isobutoxy group, tert-butoxy group, isopentyloxy group, neopentyloxy group, tert-pentyloxy group or 1-ethylpropoxy group for R³.

The "aryl group" is an aromatic hydrocarbon group having 6 to 14 carbon atoms, which is specifically exemplified by phenyl group, naphthyl group, biphenylyl group (e.g., 2-biphenylyl group, 3-biphenylyl group, 4-biphenylyl group etc.), anthryl group and the like. Preferably, it is phenyl group, naphthyl group or biphenylyl group, more preferably phenyl group.

Preferably, it is phenyl group or biphenylyl group for Z, phenyl group for B or E, phenyl group or naphthyl group for R⁴, R⁵, R⁶, R⁷, R⁸, R⁹, R¹⁰, R¹⁵, R¹⁶, R¹⁷, R¹⁸ or R²¹. It is particularly preferably phenyl group for Z.

The "heterocyclic group" is "a saturated or unsaturated 5- to 7-membered heterocyclic group containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, pyridyl group, pyridazinyl group, pyrimidinyl group, pyrazinyl group, tetrahydrofuryl group, tetrahydrothienyl group, pyrrolidinyl group, pyrazolidinyl group, imidazolidinyl group, oxazolidinyl group, thiazolidinyl group, tetrahydropyranyl group, dioxanyl group, piperidinyl group, piperazinyl group, morpholinyl group and the like, preferably a heterocyclic group selected from the group consisting of

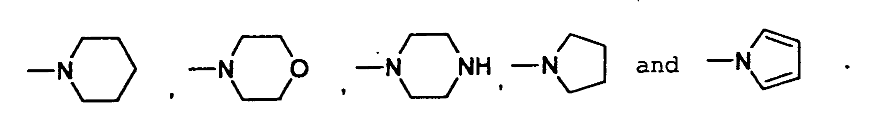

The "heteroaromatic ring group" is "a 5- to 14-membered mono or fused heteroaromatic ring group containing 1 to 3 heteroatoms selected from the group consisting of nitrogen atom, oxygen atom and sulfur atom, which is specifically exemplified by furyl group, thienyl group, pyrrolyl group, oxazolyl group, isoxazolyl group, thiazolyl group, isothiazolyl group, imidazolyl group, pyrazolyl group, 1,3,4-thiadiazolyl group, pyridyl group, pyridazinyl group, pyrimidinyl group, pyrazinyl group, indolyl group, isoindolyl group, benzofuranyl group, benzothienyl group, benzimidazolyl group, benzothiazolyl group, benzoxazolyl group, indolizinyl group, quinolyl group, isoquinolyl group, quinazolinyl group, cinnolinyl group, quinoxalinyl group, phthalazinyl group, acridinyl group, phenazinyl group, naphthyridinyl group and the like. Preferably, "a 5- to 10-membered mono or fused heteroaromatic ring group containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, 1,3,4-thiadiazolyl group, pyridyl group, pyridazinyl group, pyrimidinyl group, pyrazinyl group, indolyl group, isoindolyl group, benzofuranyl group, benzothienyl group, benzimidazolyl group, benzothiazolyl group, benzoxazolyl group, indolizinyl group, quinolyl group, isoquinolyl group and the like.

Preferably, it is thienyl group, thiazolyl group, pyrazolyl group, 1,3,4-thiadiazolyl group, pyridyl group, pyridazinyl group, pyrimidinyl group, indolyl group, benzimidazolyl group, benzothiazolyl group or benzoxazolyl group for B, furyl group, isoxazolyl group or pyridyl group for E, thienyl group or pyridyl group for Z, and oxazolyl group, thiazolyl group, pyridyl group, benzothienyl group, benzimidazolyl group or quinolyl group for R⁴, R⁵, R⁶, R⁷, R⁸, R⁹, R¹⁰, R¹⁵, R¹⁶, R¹⁷ or R²¹.

The "C₃₋₇ cycloalkyl group" is a cycloalkyl group having 3 to 7 carbon atoms, which is specifically exemplified by cyclopropyl group, cyclobutyl group, cyclopentyl group, cyclohexyl group and cycloheptyl group. Preferably, it is cycloalkyl group having 5 to 7 carbon atoms, particularly preferably cyclohexyl group.

Preferably, it is cyclohexyl group for Z.

The "C₂₋₄ alkenyl group" is a straight chain or branched chain alkenyl group having 2 to 4 carbon atoms, which is specifically exemplified by vinyl group, 1-propenyl group, allyl group, 1-methyl-2-propenyl group, 1-butenyl group, 2-butenyl group, 3-butenyl group and the like.

Preferably, it is allyl group for R¹¹ or R¹².

The "C₁₋₄ alkylsulfonyl group" is that wherein the "alkyl" moiety is alkylsulfonyl group having the above-defined "C₁₋₄ alkyl group", which is specifically exemplified by methylsulfonyl group, ethylsulfonyl group, propylsulfonyl group, isopropylsulfonyl group, butylsulfonyl group, isobutylsulfonyl group, sec-butylsulfonyl group and tert-butylsulfonyl group.

Preferably, it is methylsulfonyl group (i.e., mesyl group) for R¹¹, R¹², R²³ or R²⁴.

The "C₁₋₄ alkylcarbonyl group" is that wherein the "alkyl" moiety is alkylcarbonyl group having the above-defined "C₁₋₄ alkyl group", with preference given to acetyl group, propionyl group, butyryl group, isobutyryl group, valeryl group, isovaleryl group, pivaloyl group and the like.

Preferably, it is acetyl group for R¹¹ or R¹², and acetyl group or isobutyryl group for R²³ or R²⁴.

The "C₁₋₄ alkoxycarbonyl group" is alkoxycarbonyl group wherein the "alkoxy" moiety is the above-defined "C₁₋₄ alkoxy group", which is specifically exemplified by methoxycarbonyl group, ethoxycarbonyl group, propoxycarbonyl group, isopropoxycarbonyl group, butoxycarbonyl group, isobutoxycarbonyl group, tert-butoxycarbonyl group and the like.

The "aryloxy group" is aryloxy group wherein the "aryl" moiety is the above-defined "aryl group", which is specifically exemplified by phenoxy group, naphthyloxy group and the like.

The "aralkyl group" is a "C₁₋₄ alkyl group" substituted by "aryl group", wherein the "aryl group" and "C₁₋₄ alkyl group" are as defined above. The "aralkyl group" is specifically exemplified by benzyl group and the like.

The "C₁₋₄ alkylamino group" is alkylamino group wherein the "alkyl" moiety is the above-defined "C₁₋₄ alkyl group", which is specifically exemplified by methylamino group, ethylamino group, propylamino group, isopropylamino group, butylamino group, isobutylamino group, sec-butylamino group, tert-butylamino group and the like.

The "C₁₋₆ alkylamino group" is alkylamino group wherein the "alkyl" moiety is the above-defined "C₁₋₆ alkyl group", which is specifically exemplified by methylamino group, ethylamino group, propylamino group, isopropylamino group, butylamino group, isobutylamino group, sec-butylamino group, tert-butylamino group, pentylamino group, isopentylamino group, neopentylamino group, tert-pentylamino group, hexylamino group and the like.

Preferably, it is methylamino group, ethylamino group, isopropylamino group, isobutylamino group, sec-butylamino group, tert-butylamino group, isopentylamino group, neopentylamino group or tert-pentylamino group for R²² or R²⁵.

The "di(C₁₋₄ alkyl)amino group" is di(alkyl)amino group wherein the "alkyl" moiety is the above-defined "C₁₋₄ alkyl group", which is specifically exemplified by dimethylamino group, diethylamino group, dipropylamino group, diisopropylamino group, dibutylamino group, diisobutylamino group, di(sec-butyl)amino group, di(tert-butyl)amino group, N-ethyl-N-methylamino group, N-methyl-N-propylamino group, N-ethyl-N-propylamino group and the like.

The "di(C₁₋₆ alkyl) amino group" is di(alkyl)amino group wherein the "alkyl" moiety is the above-defined "C₁₋₄ alkyl group", which is specifically exemplified by dimethylamino group, diethylamino group, dipropylamino group, diisopropylamino group, dibutylamino group, diisobutylamino group, di(sec-butyl)amino group, di(tert-butyl)amino group, dipentylamino group, diisopentylamino group, di(tert-pentyl)amino group, dihexylamino group, N-ethyl-N-methylamino group, N-methyl-N-propylamino group, N-ethyl-N-propylamino group and the like.

Preferably, it is dimethylamino group, diethylamino group, dipropylamino group, dibutylamino group, dipentylamino group, diisopentylamino group, dihexylamino group, N-ethyl-N-methylamino group, N-methyl-N-propylamino group or N-ethyl-N-propylamino group for R²² or R²⁵.

The "C₁₋₆ alkylthio group" is alkylthio group wherein the "alkyl" moiety is the above-defined "C₁₋₆ alkyl group", which is specifically exemplified by methylthio group, ethylthio group, propylthio group, isopropylthio group, butylthio group, isobutylthio group, sec-butylthio group, tert-butylthio group, pentylthio group, isopentylthio group, neopentylthio group, tert-pentylthio group, 1-ethylpropylthio group, hexylthio group and the like.

Preferably, it is methylthio group for R²² or R²⁵.

The "di(C₁₋₆ alkyl)aminocarbonyl group" is di(alkyl)aminocarbonyl group wherein the "di(C₁₋₆ alkyl)amino" moiety is the above-defined "di(C₁₋₆ alkyl)amino group", which is specifically exemplified by dimethylaminocarbonyl group, diethylaminocarbonyl group, dipropylaminocarbonyl group, diisopropylaminocarbonyl group, dibutylaminocarbonyl group, diisobutylaminocarbonyl group, di(sec-butyl)aminocarbonyl group, di(tert-butyl)aminocarbonyl group, dipentylaminocarbonyl group, diisopentylaminocarbonyl group, di(tert-pentyl)aminocarbonyl group, dihexylaminocarbonyl group, N-ethyl-N-methylaminocarbonyl group, N-methyl-N-propylaminocarbonyl group, N-ethyl-N-propylaminocarbonyl group and the like.

When R¹⁹ of the -COO(R¹⁹) moiety for R is hydrogen atom, this carboxy group may form a salt. As the salt, alkali metal salts (e.g., potassium salt, sodium salt etc.), alkaline earth metal salts (e.g., calcium salt, magnesium salt etc.) and the like can be mentioned, with preference given to sodium salt and calcium salt.

The "C₁₋₆ alkyl group" for R⁴, R⁵, R⁶, R⁷, R⁸, R⁹ or R¹⁰ is optionally substituted by optionally substituted aryl group, optionally substituted heteroaromatic ring group, carboxy group, C₁₋₄ alkoxycarbonyl group, -CO-N(R¹⁵)(R¹⁶), -N(R¹⁵)(R¹⁶), -O-R¹⁷, -CO-R¹⁷, -SO₂-R¹⁷ or C₃₋₇ cycloalkyl group. Such "C₁₋₆ alkyl group" may be C₁₋₆ alkyl group substituted by plural substituents like, for example, benzhydryl group. Preferably, the number of substituent is 1. When the "C₁₋₆ alkyl group" is substituted, the "C₁₋₆ alkyl group" moiety is preferably methyl group.

The "aryl group" moiety of the "optionally substituted aryl group", which is a substituent on the "C₁₋₆ alkyl group" for R⁴, R⁵, R⁶, R⁷, R⁸, R⁹ or R¹⁰ is the above-defined "aryl group", preferably, phenyl group or naphthyl group.

The "optionally substituted aryl group", which is a substituent on the "C₁₋₆ alkyl group" for R⁴, R⁵, R⁶, R⁷, R⁸, R⁹ or R¹⁰ is optionally substituted by 1 to 3 substituents selected from the following. As such substituent, halogen atom, C₁₋₈ alkyl group, C₁₋₈ alkyl group substituted by di(C₁₋₆ alkyl)aminocarbonyl group, C₁₋₄ haloalkyl group, C₁₋₄ alkoxy group, C₂₋₄ alkenyl group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group, C₁₋₆ alkylthio group, heteroaromatic ring group optionally substituted by halogen atom, aralkyl group and the like can be mentioned. Preferable substituents include halogen atoms (e.g., fluorine atom, chlorine atom etc.), C₁₋₈ alkyl groups (e.g., methyl group, isopropyl group, tert-butyl group, 1-propylbutyl group etc.), C₁₋₄ haloalkyl groups (e.g., trifluoromethyl group etc.), C₂₋₄ alkenyl groups (e.g., allyl group etc.), C₁₋₆ alkylthio groups (e.g., methylthio group etc.), C₁₋₈ alkyl group (e.g., methyl group etc.) substituted by di(C₁₋₆ alkyl)aminocarbonyl group (e.g., diethylaminocarbonyl group etc.), heteroaromatic ring group (e.g., thienyl group, imidazolyl group etc.) optionally substituted by halogen atom (e.g., chlorine atom etc.) and aralkyl groups (e.g., benzyl group etc.).

The "optionally substituted heteroaromatic ring group", which is a substituent on the "C₁₋₆ alkyl group" for R⁴, R⁵, R⁶, R⁷, R⁸, R⁹ or R¹⁰ is the above-defined "heteroaromatic ring group", which is preferably oxazolyl group, thiazolyl group, pyridyl group, benzothienyl group, benzimidazolyl group or quinolyl group.

The "optionally substituted heteroaromatic ring group", which is a substituent on the "C₁₋₆ alkyl group" for R⁴, R⁵, R⁶, R⁷, R⁸, R⁹ or R¹⁰ is optionally substituted by 1 to 3 substituents selected from the following. As such substituent, halogen atom, C₁₋₈ alkyl group, C₁₋₈ alkyl group substituted by di(C₁₋₆ alkyl) aminocarbonyl group, C₁₋₄ haloalkyl group, C₁₋₄ alkoxy group, C₂₋₄ alkenyl group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group, C₁₋₆ alkylthio group, aryl group optionally substituted by halogen atom, heteroaromatic ring group optionally substituted by halogen atom, aralkyl group and the like can be mentioned. Preferable substituents include halogen atoms (e.g., fluorine atom, chlorine atom etc.), C₁₋₈ alkyl groups (e.g., methyl group, isopropyl group, tert-butyl group, 1-propylbutyl group etc.), C₁₋₄ haloalkyl groups (e.g., trifluoromethyl group etc.), C₂₋₄ alkenyl groups (e.g., allyl group etc.), C₁₋₆ alkylthio groups (e.g., methylthio group etc.), C₁₋₈ alkyl group (e.g., methyl group etc.) substituted by di(C₁₋₆ alkyl)aminocarbonyl group (e.g., diethylaminocarbonyl group etc.), aryl group (e.g., phenyl group etc.) optionally substituted by halogen atom (e.g., chlorine atom etc.), heteroaromatic ring group (e.g., thienyl group, imidazolyl group etc.) optionally substituted by halogen atom (e.g., chlorine atom etc.) and aralkyl groups (e.g., benzyl group etc.).

The "C₁₋₄ alkoxycarbonyl group", which is a substituent on the "C₁₋₆ alkyl group" for R⁴, R⁵, R⁶, R⁷, R⁸, R⁹ or R¹⁰ is the above-defined "C₁₋₄ alkoxycarbonyl group", which is preferably ethoxycarbonyl group, propoxycarbonyl group or isobutoxycarbonyl group.

The "C₃₋₇ cycloalkyl group", which is a substituent on the "C₁₋₆ alkyl group" for R⁴, R⁵, R⁶, R⁷, R⁸, R⁹ or R¹⁰ is the above-defined "C₃₋₇ cycloalkyl group", which is preferably cyclohexyl group.

The "optionally substituted aryl group" for R⁴, R⁵, R⁶, R⁷, R⁸, R⁹ or R¹⁰ is optionally substituted by 1 to 3 substituents selected from the following. As such substituent, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group, heteroaromatic ring group optionally substituted by halogen atom, aralkyl group and the like can be mentioned. Preferable substituents include halogen atoms (e.g., fluorine atom, chlorine atom etc.), C₁₋₈ alkyl groups (e.g., methyl group, isopropyl group, tert-butyl group, 1-propylbutyl group etc.), C₁₋₄ haloalkyl groups (e.g., trifluoromethyl group etc.), heteroaromatic ring groups (e.g., thienyl group, imidazolyl group etc.) optionally substituted by halogen atom (e.g., chlorine atom etc.) and aralkyl groups (e.g., benzyl group etc.).

The "optionally substituted heteroaromatic ring group" for R⁴, R⁵, R⁶, R⁷, R⁸, R⁹ or R¹⁰ is optionally substituted by 1 to 3 substituents selected from the following. As such substituent, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group, aryl group optionally substituted by halogen atom, heteroaromatic ring group optionally substituted by halogen atom, aralkyl group and the like can be mentioned. Preferable substituents include halogen atoms (e.g., fluorine atom, chlorine atom etc.), C₁₋₈ alkyl groups (e.g., methyl group, isopropyl group, tert-butyl group, 1-propylbutyl group etc.), C₁₋₄ haloalkyl groups (e.g., trifluoromethyl group etc.), aryl group (e.g., phenyl group etc.) optionally substituted by halogen atom (e.g., chlorine atom etc.), heteroaromatic ring group (e.g., thienyl group, imidazolyl group etc.) optionally substituted by halogen atom (e.g., chlorine atom etc.), and aralkyl group (e.g., benzyl group etc.).

The "optionally substituted aryl group" for R¹⁵, R¹⁶ or R¹⁷" is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl) amino group, 1-pyrrolidinyl group, 1-piperidyl group, morpholino group, C₁₋₄ alkoxycarbonyl group and the like can be mentioned. Preferable substituents include halogen atoms (e.g., chlorine atom etc.), C₁₋₈ alkyl groups (e.g., methyl group, isopropyl group etc.), C₁₋₄ alkoxy groups (e.g., methoxy group etc.), carboxy groups and di(C₁₋₄ alkyl)amino groups (e.g., dimethylamino group etc.).

The "optionally substituted heteroaromatic ring group" for R¹⁵, R¹⁶ or R¹⁷ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl) amino group, 1-pyrrolidinyl group, 1-piperidyl group, morpholino group, C₁₋₄ alkoxycarbonyl group and the like can be mentioned. Preferable substituents include halogen atoms (e.g., chlorine atom etc.), C₁₋₈ alkyl groups (e.g., methyl group, isopropyl group etc.), C₁₋₄ alkoxy groups (e.g., methoxy group etc.), carboxy group and di(C₁₋₄ alkyl)amino groups (e.g., dimethylamino group etc.).

The "C₁₋₆ alkyl group" for R¹⁵, R¹⁶ or R¹⁷ is optionally substituted by optionally substituted aryl group, optionally substituted heteroaromatic ring group, C₁₋₄ alkoxy group optionally substituted by aryl group or optionally substituted aryloxy group. As the "C₁₋₆ alkyl group" moiety when the "C₁₋₆ alkyl group" is substituted is preferably methyl group.

The "aryl group" of the "optionally substituted aryl group", which is a substituent on the "C₁₋₆ alkyl group" for R¹⁵, R¹⁶ or R¹⁷ is the above-defined "aryl group", preferably phenyl group or naphthyl group.

The "optionally substituted aryl group", which is a substituent on the "C₁₋₆ alkyl group" for R¹⁵, R¹⁶ or R¹⁷ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group and the like can be mentioned. Preferable substituents include halogen atoms (e.g., chlorine atom etc.), C₁₋₈ alkyl groups (e.g., methyl group, isopropyl group etc.), C₁₋₄ alkoxy groups (e.g., methoxy group etc.), carboxy group, di(C₁₋₄ alkyl) amino groups (e.g., dimethylamino group etc.), 1-pyrrolidinyl group, 1-piperidyl group and morpholino group.

The "heteroaromatic ring group" moiety of the "optionally substituted heteroaromatic ring group", which is a substituent on the "C₁₋₆ alkyl group" for R¹⁵, R¹⁶ or R¹⁷ is the above-defined "heteroaromatic ring group", preferably oxazolyl group, thiazolyl group, pyridyl group, benzothienyl group, benzimidazolyl group or quinolyl group.

The "optionally substituted heteroaromatic ring group", which is a substituent on the "C₁₋₆ alkyl group" for R¹⁵, R¹⁶ or R¹⁷ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl) amino group, 1-pyrrolidinyl group, 1-piperidyl group, morpholino group, C₁₋₄ alkoxycarbonyl group and the like can be mentioned. Preferable substituents include halogen atoms (e.g., chlorine atom etc.), C₁₋₈ alkyl groups (e.g., methyl group, isopropyl group etc.), C₁₋₄ alkoxy groups (e.g., methoxy group etc.), carboxy group, di(C₁₋₄ alkyl)amino groups (e.g., dimethylamino group etc.), 1-pyrrolidinyl group, 1-piperidyl group and morpholino group.

The "C₁₋₄ alkoxy group" moiety of the "C₁₋₄ alkoxy group optionally substituted by aryl group", which is a substituent on the "C₁₋₆ alkyl group" for R¹⁵, R¹⁶ or R¹⁷ is the above-defined "C₁₋₄ alkoxy group", preferably methoxy group.

The "aryl group" moiety of the "C₁₋₄ alkoxy group optionally substituted by aryl group", which is a substituent on the "C₁₋₆ alkyl group" for R¹⁵, R¹⁶ or R¹⁷ is the above-defined "aryl group", preferably phenyl group.

The "aryloxy group" moiety of the "optionally substituted aryloxy group", which is a substituent on the "C₁₋₆ alkyl group" for R¹⁵, R¹⁶ or R¹⁷ is the above-defined "aryloxy group", preferably phenoxy group.

The "optionally substituted aryloxy group", which is a substituent on the "C₁₋₆ alkyl group" for R¹⁵, R¹⁶ or R¹⁷ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group and the like can be mentioned. Preferable substituents include halogen atom (e.g., chlorine atom etc.), C₁₋₈ alkyl group (e.g., methyl group, isopropyl group etc.), C₁₋₄ alkoxy group (e.g., methoxy group etc.), carboxy group, di(C₁₋₄ alkyl)amino group (e.g., dimethylamino group etc.), 1-pyrrolidinyl group, 1-piperidyl group and morpholino group.

The "5- to 7-membered hetero ring optionally containing at least one heteroatom selected from the group consisting of nitrogen atom, oxygen atom and sulfur atom", which is formed by R¹⁵ and R¹⁶ together with the nitrogen atom bonded thereto is preferably a "saturated or unsaturated 5- to 7-membered hetero ring containing 1 to 3 heteroatoms selected from the group consisting of nitrogen atom, oxygen atom and sulfur atom", which is specifically exemplified by a hetero ring selected from the group consisting of

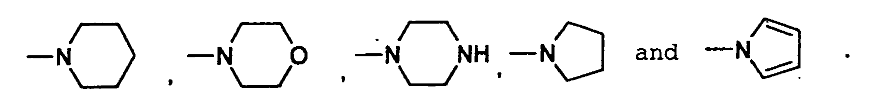

The "C₁₋₆ alkyl group" for R²¹ is optionally substituted by optionally substituted aryl group or optionally substituted heteroaromatic ring group.

The "aryl group" moiety of the "optionally substituted aryl group", which is a substituent on the "C₁₋₆ alkyl group" for R²¹ is the above-defined "aryl group", preferably phenyl group.

The "optionally substituted aryl group", which is a substituent on the "C₁₋₆ alkyl group" for R²¹ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group and the like can be mentioned. Preferable substituents include halogen atoms (e.g., chlorine atom, fluorine atom etc.), C₁₋₈ alkyl groups (e.g., methyl group etc.) and C₁₋₄ haloalkyl groups (e.g., trifluoromethyl group etc.).

The "heteroaromatic ring group" moiety of the "optionally substituted heteroaromatic ring group", which is a substituent on the "C₁₋₆ alkyl group" for R²¹ is the above-defined "heteroaromatic ring group", preferably oxazolyl group, thiazolyl group, pyridyl group, benzothienyl group, benzimidazolyl group or quinolyl group.

The "optionally substituted heteroaromatic ring group", which is a substituent on the "C₁₋₆ alkyl group" for R²¹ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group and the like can be mentioned. Preferable substituents include halogen atom (e.g., chlorine atom, fluorine atom etc.), C₁₋₈ alkyl group (e.g., methyl group etc.) and C₁₋₄ haloalkyl group (e.g., trifluoromethyl group etc.).

The "optionally substituted aryl group" for R²¹ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl)amino group, 1-pyrrolidinyl group, 1-piperidyl group, morpholino group, C₁₋₄ alkoxycarbonyl group and the like can be mentioned. Preferable substituents include halogen atom (e.g., chlorine atom, fluorine atom etc.), C₁₋₈ alkyl group (e.g., methyl group etc.) and C₁₋₄ haloalkyl group (e.g., trifluoromethyl group etc.).

The "optionally substituted heteroaromatic ring group" for R²¹ is optionally substituted by 1 to 3 substituents selected from the following. As such substituents, halogen atom, C₁₋₈ alkyl group, C₁₋₄ haloalkyl group, C₁₋₄ alkoxy group, carboxy group, hydroxyl group, cyano group, nitro group, amino group, C₁₋₄ alkylamino group, di(C₁₋₄ alkyl) amino group, 1-pyrrolidinyl group, 1-piperidyl group, morpholino group, C₁₋₄ alkoxycarbonyl group and the like can be mentioned. Preferable substituents include halogen atom (e.g., chlorine atom, fluorine atom etc.), C₁₋₈ alkyl group (e.g., methyl group etc.) and C₁₋₄ haloalkyl group (e.g., trifluoromethyl group etc.).

The "C₁₋₄ alkylcarbonyl group" for R²³ or R²⁴ is optionally substituted by amino group, C₁₋₄ alkylamino group or di(C₁₋₄ alkyl)amino group.

The "C₁₋₄ alkylamino group", which is a substituent on the "C₁₋₆ alkyl group" for R²³ or R²⁴ is the above-defined "C₁₋₄ alkylamino group", preferably methylamino group.

The "di(C₁₋₄ alkyl)amino group", which is a substituent on the "C₁₋₆ alkyl group" for R²³ or R²⁴ is the above-defined "di(C₁₋₄ alkyl)amino group", preferably dimethylamino group.

The "C₁₋₈ alkyl group" for R¹¹ or R¹² is optionally substituted by a substituent selected from the group consisting of C₃₋₇ cycloalkyl group, optionally substituted aryl group, optionally substituted hetero ring group, hydroxyl group, C₁₋₄ alkylamino group and di(C₁₋₄ alkyl)amino group.

The "aryl group" moiety of the "optionally substituted aryl", which is a substituent on the "C₁₋₆ alkyl group" for R¹¹ or R¹² is the above-defined "aryl group", preferably phenyl group.

The "optionally substituted aryl group", which is a substituent on the "C₁₋₈ alkyl group" for R¹¹ or R¹² is optionally substituted by 1 to 3 substituents selected from the following. As such substituents, halogen atom, C₁₋₄ alkyl group, C₁₋₄ haloalkyl group, C₁₋₄ alkoxy group, carboxy group, hydroxyl group, cyano group, nitro group, amino group, C₁₋₄ alkoxycarbonyl group and the like can be mentioned. Preferable substituents include halogen atom and C₁₋₄ haloalkyl group.

The "heterocyclic group" moiety of the "optionally substituted heterocyclic group", which is a substituent on the "C₁₋₈ alkyl group" for R¹¹ or R¹² is "a saturated or unsaturated 5- to 7-membered heterocyclic group containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, pyridyl group, pyridazinyl group, pyrimidinyl group, pyrazinyl group, tetrahydrofuryl group, tetrahydrothienyl group, pyrrolidinyl group, pyrazolidinyl group, imidazolidinyl group, oxazolidinyl group, thiazolidinyl group, tetrahydropyranyl group, dioxanyl group, piperidinyl group, piperazinyl group, morpholinyl group and the like, preferably tetrahydropyranyl group.

The "optionally substituted heterocyclic group", which is a substituent on the "C₁₋₈ alkyl group" for R¹¹ or R¹² is optionally substituted by 1 to 3 substituents selected from the following. As such substituents, halogen atom, C₁₋₄ alkyl group, C₁₋₄ haloalkyl group, C₁₋₄ alkoxy group, carboxy group, hydroxyl group, cyano group, nitro group, amino group, C₁₋₄ alkoxycarbonyl group and the like can be mentioned.

The "C₁₋₄ alkylamino group", which is a substituent on the "C₁₋₈ alkyl group" for R¹¹ or R¹² is the above-defined "C₁₋₄ alkylamino group", preferably methylamino group.

The "di(C₁₋₄alkyl)amino group", which is a substituent on the "C₁₋₈ alkyl group" for R¹¹ or R¹² is the above-defined "di(C₁₋₄ alkyl)amino group", preferably dimethylamino group.

The "C₁₋₄alkylcarbonyl group" for R¹¹ or R¹² is optionally substituted by hydroxyl group or C₁₋₄ alkoxy group.

The "C₁₋₄ alkoxy group", which is a substituent on the "C₁₋₄ alkylcarbonyl group" for R¹¹ or R¹² is the above-defined "C₁₋₄ alkoxy group".

The "C₃₋₇ cycloalkane" formed by R¹³ and R¹⁴ together with the carbon atom bonded thereto is cycloalkane having 3 to 7 carbon atoms, which is specifically exemplified by cyclopropane, cyclobutane, cyclopentane, cyclohexane and cycloheptane. Preferred is cycloalkane having 5 to 7 carbon atoms, particularly preferably cyclopentane or cyclohexane.

The "5- to 7-membered hetero ring optionally having at least one heteroatom selected from the group consisting of nitrogen atom, oxygen atom and sulfur atom", which is formed by R¹³ and R¹⁴ together with the carbon atom bonded thereto is preferably a "5- to 7-membered hetero ring optionally containing at least one heteroatom selected from the group consisting of nitrogen atom, oxygen atom and sulfur atom", which is specifically exemplified by tetrahydropyrane, thiane and the like, particularly preferably tetrahydropyrane.

The C₁₋₄ alkylene group for A is optionally substituted by C₃₋₇ cycloalkyl group. As such C₃₋₇ cycloalkyl group, the above-defined "C₃₋₇ cycloalkyl group" can be mentioned.

The "C₁₋₄ alkylene group optionally substituted by C₃₋₇ cycloalkyl group" for A is preferably 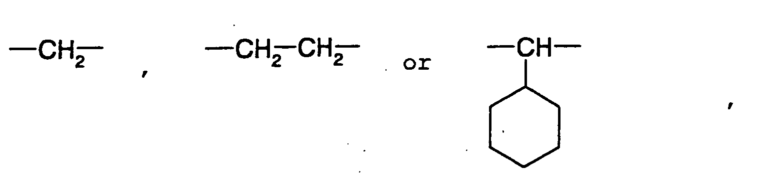 more preferably -CH₂-.

The "C₃₋₇ cycloalkyl group" for Z is preferably cyclopentyl group or cyclohexyl group, more preferably cyclohexyl group.

The "C₃₋₇ cycloalkyl group" for Z is optionally substituted by aryl group (this aryl group is optionally substituted by 1 to 5 (preferably 1 to 3) halogen atoms or C₁₋₆ alkyl group) or heteroaromatic ring group (this heteroaromatic ring group is optionally substituted by 1 to 5 (preferably 1 to 3) halogen atoms or C₁₋₆ alkyl group). Such substituent of the "C₃₋₇ cycloalkyl group" is preferably phenyl group optionally substituted by 1 to 3 halogen atoms or C₁₋₆ alkyl group.

The "aryl group" for Z is preferably phenyl group or biphenylyl group (e.g., 2-biphenylyl group, 3-biphenylyl group, 4-biphenylyl group), more preferably phenyl group.

The "aryl group" for Z is optionally substituted by 1 to 5 (preferably 1 to 3) substituents selected from the following.
(a) heterocyclic group optionally substituted by C₁₋₄ alkyl group or C₁₋₄ alkylcarbonyl group,
(b) C₃₋₇ cycloalkyl group optionally substituted by hydroxyl group, oxo group, halogen atom or C₁₋₆ alkyl group,
(c) carboxy group,
(d) halogen atom,
(e) C₁₋₈ alkyl group,
(f) C₁₋₄ haloalkyl group,
(g) C₁₋₄ alkylamino group,
(h) di(C₁₋₄ alkyl) amino group,
(i) C₁₋₆ alkylthio group,
(j) C₁₋₄ alkoxy group,
(k) C₁₋₄ alkylcarbonyl group and
(l) nitro group.

As such substituents, preferred are C₁₋₈ alkyl group.

The "heterocyclic group" of the "heterocyclic group optionally substituted by C₁₋₄ alkyl group or C₁₋₄ alkylcarbonyl group", which is a substituent on the "aryl group" for Z is preferably a "saturated or unsaturated 5- to 7-membered heterocyclic group optionally containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, pyridyl group, pyridazinyl group, pyrimidinyl group, pyrazinyl group, tetrahydrofuryl group, tetrahydrothienyl group, pyrrolidinyl group, pyrazolidinyl group, imidazolidinyl group, oxazolidinyl group, thiazolidinyl group, tetrahydropyranyl group, dioxanyl group, piperidinyl group, piperazinyl group, morpholinyl group and the like. Preferred are piperidinyl group, morpholinyl group, piperazinyl group, pyrrolidinyl group, pyrrolyl group and tetrahydropyranyl group.

The substituent on the "heterocyclic group" is preferably C₁₋₄ alkyl group or C₁₋₄ alkylcarbonyl group.

The "C₃₋₇ cycloalkyl" of the "C₃₋₇ cycloalkyl group optionally substituted by hydroxyl group, oxo group, halogen atom or C₁₋₆ alkyl group", which is a substituent on the "aryl group" for Z is preferably cyclopentyl group or cyclohexyl group, more preferably cyclohexyl group. The "C₃₋₇ cycloalkyl group" is optionally substituted by 1 to 5 (preferably 1 to 3) substituents selected from the group consisting of hydroxyl group, oxo group, halogen atom and C₁₋₆ alkyl group. As the substituent on the "C₃₋₇ cycloalkyl group" is preferably halogen atom or C₁₋₄ alkyl group.

As the "halogen atom", which is a substituent on the "aryl group" for Z, the above-defined "halogen atom" can be mentioned, which is preferably a fluorine atom, a chlorine atom or a bromine atom, particularly preferably a chlorine atom.

As the "C₁₋₈ alkyl group", which is the substituent on the "aryl group" for Z, the above-defined "C₁₋₈ alkyl group" can be mentioned. Preferably, it is isopropyl group, isobutyl group, sec-butyl group, tert-butyl group, isopentyl group, neopentyl group, tert-pentyl group, 1-ethylpropyl group or 1-propylbutyl group, more preferably isopentyl group, neopentyl group, tert-pentyl group, 1-ethylpropyl group or 1-propylbutyl group, particularly preferably neopentyl group, 1-ethylpropyl group or 1-propylbutyl group.

As the "C₁₋₄ haloalkyl group", which is the substituent on the "aryl group" for Z, the above-defined "C₁₋₄ haloalkyl group" can be mentioned, with preference given to trifluoromethyl group.

As the "C₁₋₄ alkylamino group", which is the substituent on the "aryl group" for Z, the above-defined "C₁₋₄ alkylamino group" can be mentioned, with preference given to methylamino group, ethylamino group and isopropylamino group.

As the "di(C₁₋₄ alkyl)amino group", which is the substituent on the "aryl group" for Z, the above-defined "di(C₁₋₄ alkyl)amino group" can be mentioned, with preference given to dimethylamino group, diethylamino group and dipropylamino group.

As the "C₁₋₆ alkylthio group", which is the substituent on the "aryl group" for Z, the above-defined "C₁₋₆ alkylthio group" can be mentioned, with preference given to isopentylthio group.

As the "C₁₋₄ alkoxy group", which is the substituent on the "aryl group" for Z, the above-defined "C₁₋₄ alkoxy group" can be mentioned.

As the "C₁₋₄ alkylcarbonyl group", which is the substituent on the "aryl group" for Z, the above-defined "C₁₋₄ alkylcarbonyl group" can be mentioned, with preference given to acetyl group.

The "heteroaromatic ring group" for Z is preferably a "5-to 10-membered mono or fused heteroaromatic ring group containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, pyridyl group, pyridazinyl group, pyrimidinyl group, pyrazinyl group, indolyl group, isoindolyl group, benzofuranyl group, benzothienyl group, benzoimidazolyl group, benzothiazolyl group, benzoxazolyl group and the like. Particularly preferred are thiazolyl group and pyridyl group.

The "heteroaromatic ring group" for Z is optionally substituted by 1 to 5 (preferably 1 to 3) substituents selected from the following.
(a) heterocyclic group optionally substituted by C₁₋₄ alkyl group or C₁₋₄ alkylcarbonyl group,
(b) C₃₋₇ cycloalkyl group optionally substituted by hydroxyl group, oxo group, halogen atom or C₁₋₆ alkyl group,
(c) carboxy group,
(d) halogen atom,
(e) C₁₋₈ alkyl group,
(f) C₁₋₄ haloalkyl group,
(g) C₁₋₄ alkylamino group,
(h) di(C₁₋₄ alkyl) amino group,
(i) C₁₋₆ alkylthio group,
(j) C₁₋₄ alkoxy group,
(k) C₁₋₄ alkylcarbonyl group and
(l) aryl group optionally substituted by halogen atom or C₁₋₄ haloalkyl group.

As such substituents, preferred are (a) heterocyclic group, (b) C₁₋₈ alkyl group and (c) aryl group optionally substituted by halogen atom or C₁₋₄ haloalkyl group.

The "heterocyclic group" of the "heterocyclic group optionally substituted by C₁₋₄ alkyl group or C₁₋₄ alkylcarbonyl group", which is a substituent on the "heteroaromatic ring group" for Z is preferably, a "saturated or unsaturated 5- to 7-membered heterocyclic group optionally containing 1 to 3 heteroatoms selected from the group consisting of nitrogen atom, oxygen atom and sulfur atom", which is specifically exemplified by furyl group, thienyl group, pyrrolyl group, oxazolyl group, isoxazolyl group, thiazolyl group, isothiazolyl group, imidazolyl group, pyrazolyl group, pyridyl group, pyridazinyl group, pyrimidinyl group, pyrazinyl group, tetrahydrofuryl group, tetrahydrothienyl group, pyrrolidinyl group, pyrazolidinyl group, imidazolidinyl group, oxazolidinyl group, thiazolidinyl group, tetrahydropyranyl group, dioxanyl group, piperidinyl group, piperazinyl group, morpholinyl group and the like. Preferred are piperidinyl group, morpholinyl group, piperazinyl group, pyrrolidinyl group, pyrrolyl group and tetrahydropyranyl group.

The substituent on the "heterocyclic group" is preferably C₁₋₄ alkyl group or C₁₋₄ alkylcarbonyl group.

The "C₃₋₇ cycloalkyl" of the "C₃₋₇ cycloalkyl group optionally substituted by hydroxyl group, oxo group, halogen atom or C₁₋₆ alkyl group", which is a substituent on the "heteroaromatic ring group" for Z is preferably cyclopentyl group or cyclohexyl group, more preferably cyclohexyl group.

The "C₃₋₇ cycloalkyl group" is optionally substituted by 1 to 5 (preferably 1 to 3) substituents selected from the group consisting of hydroxyl group, oxo group, halogen atom and C₁₋₆ alkyl group. The substituent on the "C₃₋₇ cycloalkyl group" is preferably halogen atom or C₁₋₄ alkyl group.

As the "halogen atom", which is the substituent on the "heteroaromatic ring group" for Z, the above-defined "halogen atom" can be mentioned, with preference given to fluorine atom, chlorine atom and bromine atom, particularly preferably chlorine atom.

As the "C₁₋₈ alkyl group", which is the substituent on the "heteroaromatic ring group" for Z, the above-defined "C₁₋₈ alkyl group" can be mentioned. Preferred are isopropyl group, isobutyl group, sec-butyl group, tert-butyl group, isopentyl group, neopentyl group, tert-pentyl group, 1-ethylpropyl group and 1-propylbutyl group, more preferred are isopentyl group, neopentyl group, tert-pentyl group, 1-ethylpropyl group and 1-propylbutyl group, particularly preferred are neopentyl group, 1-ethylpropyl group and 1-propylbutyl group.

As the "C₁₋₄ haloalkyl group", which is the substituent on the "heteroaromatic ring group" for Z, the above-defined "C₁₋₄ haloalkyl group" can be mentioned, with preference given to trifluoromethyl group.

As the "C₁₋₄ alkylamino group", which is the substituent on the "heteroaromatic ring group" for Z, the above-defined "C₁₋₄ alkylamino group" can be mentioned, with preference given to methylamino group, ethylamino group and isopropylamino group.

As the "di(C₁₋₄ alkyl)amino group", which is the substituent on the "heteroaromatic ring group" for Z, the above-defined "di(C₁₋₄ alkyl)amino group" can be mentioned, with preference given to dimethylamino group, diethylamino group and dipropylamino group.

As the "C₁₋₆ alkylthio group", which is the substituent on the "heteroaromatic ring group" for Z, the above-defined "C₁₋₆ alkylthio group" can be mentioned, with preference given to isopentylthio group.

As the "C₁₋₄ alkoxy group", which is the substituent on the "heteroaromatic ring group" for Z, the above-defined "C₁₋₄ alkoxy group" can be mentioned.

As the "C₁₋₄ alkylcarbonyl group", which is the substituent on the "heteroaromatic ring group" for Z, the above-defined "C₁₋₄ alkylcarbonyl group" can be mentioned, with preference given to acetyl group.

As the "aryl group optionally substituted by halogen atom or C₁₋₄ haloalkyl group", which is the substituent on the "heteroaromatic ring group" for Z, "phenyl group optionally substituted by halogen atom or C₁₋₄ haloalkyl group" can be preferably mentioned.

The "piperazinyl group" for Z is optionally substituted by 1 to 5 (preferably 1 to 3) substituents selected from the following.
(a) phenyl group,
(b) phenyl C₁₋₄ alkyl group,
(c) benzoyl group optionally substituted by halogen atom and
(d) phenyl C₁₋₄ alkoxycarbonyl group.

The "phenyl C₁₋₄ alkyl group", which is a substituent on the "piperazinyl group" for Z is phenylalkyl group having the above-defined "C₁₋₄ alkyl group" as the "alkyl moiety", which is specifically exemplified by benzyl group, phenethyl group, 1-phenylethyl group, 3-phenylpropyl group and the like. Preferably, it is benzyl group.

The "benzoyl group optionally substituted by halogen atom", which is a substituent on the "piperazinyl group" for Z, is preferably benzoyl group optionally substituted by 1 to 5 of the above-defined "halogen atom", which is specifically exemplified by chlorobenzoyl group, bromobenzoyl group and the like.

The "phenyl C₁₋₄ alkoxycarbonyl group", which is a substituent on the "piperazinyl group" for Z is a "phenylalkoxycarbonyl group having the above-defined C₁₋₄ alkoxy group as the alkoxy moiety, which is specifically exemplified by benzyloxycarbonyl group and the like. Preferred is benzyloxycarbonyl group.

In the formula [I], preferable substituents include the following.
V is preferably =N- or =CH-.
W is preferably -S-.
m is preferably 1 or 2.
R¹ and R² are preferably each a hydrogen atom.

X is preferably the following (1) to (4).

(1) -N(R⁴)-, -SO₂-N(R⁵)-, -CO-N (R⁷)- or -O-

wherein R⁴ is a hydrogen atom or a C₁₋₆ alkyl group, R⁵ is a hydrogen atom or a C₁₋₆ alkyl group and R⁷ is a hydrogen atom or a C₁₋₆ alkyl group,

(2) -N(R⁴)-

wherein R⁴ is a C₁₋₆ alkyl group substituted by
(a) aryl group optionally substituted by 1 to 3 substituents selected from the group consisting of halogen atom, a C₁₋₈ alkyl group and a C₁₋₄ haloalkyl group,
(b) heteroaromatic ring group optionally substituted by 1 to 3 C₁₋₈ alkyl groups,
(c) carboxy group,
(d) C₁₋₄ alkoxycarbonyl group,
(e) -CO-N(R¹⁵)(R¹⁶)
   wherein R¹⁵ and R¹⁶ are each independently a hydrogen atom, an aryl group (this aryl group is optionally substituted by 1 to 3 substituents selected from the group consisting of C₁₋₈ alkyl group, C₁₋₄ alkoxy group, carboxy group and di(C₁₋₄ alkyl) amino group), a heteroaromatic ring group, a C₁₋₆ alkyl group (this C₁₋₆ alkyl group is optionally substituted by aryl group), or may form a 5- to 7-membered hetero ring optionally containing at least one heteroatom selected from the group consisting of nitrogen atom, oxygen atom and sulfur atom, which is formed together with the nitrogen atom bonded thereto,
(f) -N(R¹⁵)(R¹⁶)
   wherein R¹⁵ and R¹⁶ are each independently a hydrogen atom or an aryl group,
(g) -O-R¹⁷
   wherein R¹⁷ is an aryl group or
(h) C₃₋₇ cycloalkyl group,

   (3) -N(R⁴)-

   wherein R⁴ is
(a) -CO-N(R¹⁵)(R¹⁶) wherein R¹⁵ and R¹⁶ are each independently a hydrogen atom, an aryl group (this aryl group is optionally substituted by 1 to 3 C₁₋₈ alkyl groups), a C₁₋₆ alkyl group, or may form an indoline ring together with the nitrogen atom bonded thereto, or may form a 5- to 7-membered hetero ring optionally containing at least one heteroatom selected from the group consisting of nitrogen atom, oxygen atom and sulfur atom,
(b) -SO₂-N(R¹⁵)(R¹⁶)
   wherein R¹⁵ and R¹⁶ are each independently an aryl group or a C₁₋₆ alkyl group,
(c) -CO-R¹⁷
   wherein R¹⁷ is an aryl group (this aryl group is optionally substituted by 1 to 3 substituents selected from the group consisting of C₁₋₈ alkyl group and C₁₋₄ alkoxy group), a heteroaromatic ring group or a C₁₋₆ alkyl group (this C₁₋₆ alkyl group is an aryl group optionally substituted by 1 to 3 substituents selected from the group consisting of a halogen atom and a C₁₋₈ alkyl group, a heteroaromatic ring group, a C₁₋₄ alkoxy group optionally substituted by aryl group or an aryloxy group optionally substituted by 1 to 3 C₁₋₈ alkyl groups,
(d) -SO₂-R¹⁷
   wherein R¹⁷ is an aryl group or
(e) aryl group
   or

   (4) -N(R⁸)-CO-, -N(R⁹)-CO-N(R⁵)- or -N(R¹⁰)-(CH₂)ₖ-N(R⁵)-

   wherein
   R⁵ is a C₁₋₆ alkyl group optionally substituted by hydrogen atom or aryl group,
   R⁸ is a hydrogen atom or C₁₋₆ alkyl group,
   R⁹ is linked with R⁵ to form 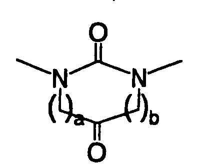 wherein a and b are each independently 0, 1 or 2,
   k is 0 or an integer of 1 to 4,
   R¹⁰ is linked with R⁵ to form 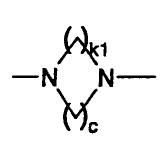 wherein k1 and c are each independently 0 or an integer of 1 to 4 or 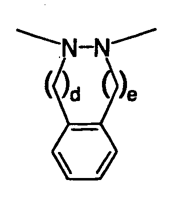 wherein d and e are each independently 0, 1 or 2.
   n is preferably 0 or an integer of 1 to 3.
   P is preferably 0 or 1.
   L is preferably the following (1) to (4).

   (1) -C(R²⁰)(R²¹)-

   wherein
   R²⁰ is linked with R⁴ to form 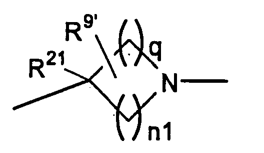 wherein n1 and q are each independently 0 or an integer of 1 to 4 and R^{9'} is a hydrogen atom, a hydroxyl group, a C₁₋₆ alkyl group, a carboxy group or a C₁₋₆ alkoxy group or 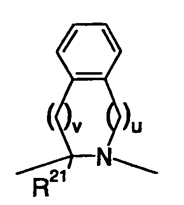 wherein u and v are each independently 0, 1 or 2,
   R²¹ is a hydrogen atom, 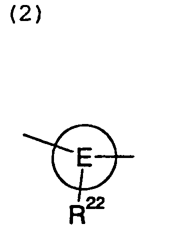 wherein E is a phenyl group, a furyl group, an isoxazolyl group or a pyridyl group and R²² is a hydrogen atom, 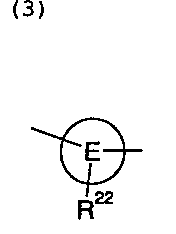 wherein
   E is a phenyl group,
   R²² is
   (a) linked with R⁴ to form 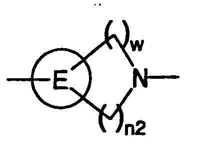 wherein n2 and w are each independently 0 or an integer of 1 to 3,
   (b) linked with R⁷ to form 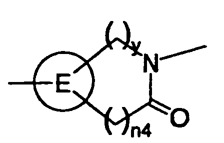 wherein n4 and y are each independently 0, 1 or 2,
   (c) linked with R⁷ to form 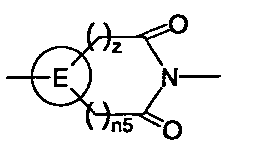 wherein n5 and z are each independently 0 or 1 or
   (d) linked with R⁸ to form 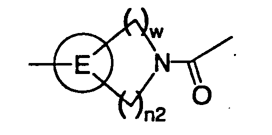
   wherein n2 and w are each independently 0 or an integer of 1 to 3 or 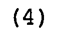 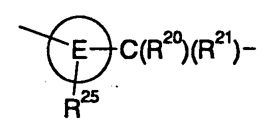 wherein
   R²⁰ is linked with R⁴ to form 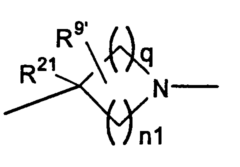 wherein n1 and q are each independently 0 or an integer of 1 to 4 and R^{9'} is a hydrogen atom, a hydroxyl group, a C₁₋₆ alkyl group, a carboxy group or a C₁₋₆ alkoxy group,
   R²¹ is a hydrogen atom,
   E is a phenyl group, a furyl group, an isoxazolyl group or a pyridyl group, and
   R²⁵ is a hydrogen atom.
   R is preferably -COO(R¹⁹), -A¹-COO(R¹⁹) or -O-A¹-COO(R¹⁹)
   wherein A¹ is a C₁₋₄ alkylene group and R¹⁹ is a hydrogen atom or a C₁₋₄ alkyl group.

B is a phenyl group or a heteroaromatic ring group (this heteroaromatic ring group is preferably selected from the group consisting of thienyl group, thiazolyl group, pyrazolyl group, 1,3,4-thiadiazolyl group, pyridyl group, pyridazinyl group, pyrimidinyl group, indolyl group, benzimidazolyl group, benzothiazolyl group and benzoxazolyl group). When V is =CH-, the position of substitution of B on the thiophene or furan ring formed by V together with W is preferably the 4-position or 5-position, and when V is =N-, the position of substitution of B on the thiazole or oxazole ring formed by V together with W is preferably the 4-position.
R³ is preferably a hydrogen atom.

Y is preferably -C (R¹³)(R¹⁴)-N(R¹²)-, -C(R¹³)(R¹⁴)-O-, -N(R¹¹)- or -O-
wherein R¹¹ is a hydrogen atom or a C₁₋₈ alkyl group, R¹² is a hydrogen atom or a C₁₋₈ alkyl group, and R¹³ and R¹⁴ are each a hydrogen atom.

s is preferably 0 or 1.

A is preferably a methylene group.

Y, s and A are preferably the following (1) or (2).
(1) Y is -C(R¹³)(R¹⁴)-N(R¹²)- or -C(R¹³)(R¹⁴)-O-
   wherein R¹² is a hydrogen atom or a C₁₋₈ alkyl group and R¹³ and R¹⁴ are each a hydrogen atom,
   and
   s is 0.
(2) Y is -N(R¹¹)- or -O-
wherein R¹¹ is a hydrogen atom or a C₁₋₈ alkyl group, and
s is 1 and A is a methylene group.

Z is preferably an aryl group substituted by substituents selected from the group consisting of,
(a) C₃₋₇ cycloalkyl group optionally substituted by 1 to 3 substituents selected from the group consisting of halogen atom and C₁₋₆ alkyl group,
(b) halogen atom,
(c) C₁₋₈ alkyl group,
(d) C₁₋₄ haloalkyl group,
(e) di(C₁₋₄ alkyl) amino group,
(f) C₁₋₆ alkylthio group and
(g) C₁₋₄ alkylcarbonyl group.

Z is more preferably a phenyl group substituted by a C₁₋₈ alkyl group (this C₁₋₈ alkyl group is selected from the group consisting of isopropyl group, isobutyl group, sec-butyl group, tert-butyl group, isopentyl group, neopentyl group, tert-pentyl group, 1-ethylpropyl group and 1-propylbutyl group).

Z is particularly preferably a phenyl group substituted by 1-ethylpropyl group or 1-propylbutyl group.

In the formula [I], particularly preferable substituents include the following.
V is particularly preferably =N- or =CH-.
W is particularly preferably -S-.
m is particularly preferably 1.
R¹ and R² are each preferably a hydrogen atom.
X, n, p and L are particularly preferably the following (1) to (7).
(1) X is -N(R⁴)
wherein R⁴ is a C₁₋₆ alkyl group substituted by heteroaromatic ring group optionally substituted by 1 to 3 C₁₋₈ alkyl groups and
n is 1 and p is 0.
(2) X is -N(R⁴)
wherein R⁴ is -CO-N(R¹⁵)(R¹⁶)
wherein R¹⁵ and R¹⁶ are each independently a C₁₋₆ alkyl group substituted by hydrogen atom, an aryl group (this aryl group is optionally substituted by 1 to 3 substituents selected from the group consisting of C₁₋₈ alkyl group, C₁₋₄ alkoxy group, carboxy group and di(C₁₋₄ alkyl) amino group), a heteroaromatic ring group, a C₁₋₆ alkyl group (this C₁₋₆ alkyl group is optionally substituted by aryl group), or may form a 5- to 7-membered hetero ring optionally containing at least one heteroatom selected from the group consisting of nitrogen atom, oxygen atom and sulfur atom, together with the nitrogen atom bonded thereto
and
n is 1 and p is 0.
(3) X is -N(R⁴)-
wherein R⁴ is -CO-N(R¹⁵)(R¹⁶)
wherein R¹⁵ and R¹⁶ are each independently a hydrogen atom, an aryl group (this aryl group is optionally substituted by 1 to 3 C₁₋₈ alkyl groups), or a C₁₋₆ alkyl group, or may form an indoline ring together with the nitrogen atom bonded thereto, or may form a 5 to 7-membered hetero ring optionally containing at least one heteroatom selected from the group consisting of nitrogen atom, oxygen atom and sulfur atom
and
n is 1 and p is 0.
(4) X is -N(R⁴)-, -SO₂-N(R⁵)-, -CO-N (R⁷)- or -O-
wherein R⁴ is a hydrogen atom or a C₁₋₆ alkyl group, R⁵ is a hydrogen atom or a C₁₋₆ alkyl group, and R⁷ is a hydrogen atom or a C₁₋₆ alkyl group, and
n is 0, p is 1 and L is 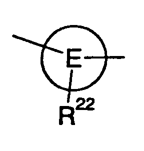 wherein E is a pyridyl group and R²² is a hydrogen atom.
(5) X is -N(R¹⁰)-(CH₂)ₖ-N(R⁵)-
wherein k is 0 or an integer of 1 to 4, and
R¹⁰ is linked with R⁵ to form 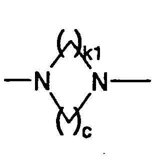 wherein k1 and c are each independently 0 or an integer of 1 to 4,
and
n is 0 or an integer of 1 to 4, p is 1 and L is 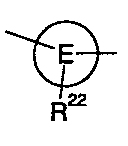 wherein E is a phenyl group, a furyl group, an isoxazolyl group or a pyridyl group and R²² is a hydrogen atom.
(6) X is -N(R⁴)-
and
n is 0 or an integer of 1 to 4, p is 1 and L is 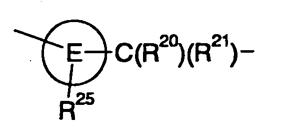 wherein
R²⁰ is linked with R⁴ to form 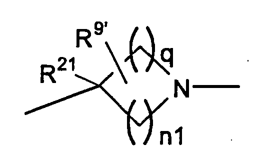 wherein n1 and q are each independently 0 or an integer of 1 to 4 and R^{9'} is a hydrogen atom, a hydroxyl group, a C₁₋₆ alkyl group, a carboxy group or a C₁₋₆ alkoxy group,
R²¹ is a hydrogen atom,
E is a phenyl group, a furyl group, an isoxazolyl group or a pyridyl group, and
R²⁵ is a hydrogen atom.
(7) X is -N(R⁴)-
and
n is 0 or an integer of 1 to 3, p is 1 and L is 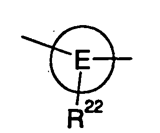 wherein
E is a phenyl group,
R²² is linked with R⁴ to form 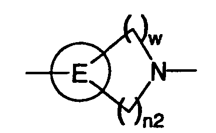 wherein n2 and w are each independently 0 or an integer of 1 to 3.
R is -COO(R¹⁹)
wherein R¹⁹ is a hydrogen atom or a C₁₋₄ alkyl group, is particularly preferable.
B is particularly preferably a phenyl group. When V is =CH-, the position of substitution of B on the thiophene or furan ring formed by V together with W is preferably the 4-position or 5-position, and when V is =N-, the position of substitution of B on the thiazole or oxazole ring formed by V together with W is particularly preferably the 4-position.
R³ is particularly preferably a hydrogen atom.
Y, s and A are each particularly preferably the following (1) or (2).
(1) Y is -C(R¹³)(R¹⁴)-N(R¹²)- or -C(R¹³)(R¹⁴)-O-
wherein R¹² is a hydrogen atom or a C₁₋₈ alkyl group and R¹³ and R¹⁴ are each a hydrogen atom,
and
s is 0.
(2) Y is -N(R¹¹)- or -O-
wherein R¹¹ is a hydrogen atom or a C₁₋₈ alkyl group,
and
s is 1 and A is a methylene group.
Z is particularly preferably a phenyl group substituted by 1-ethylpropyl group or 1-propylbutyl group.

In the formula [II], preferable substituents include the following.
V is preferably =CH-.
W is preferably -S-.
Y¹⁰⁰ is preferably -CH₂-.
R¹⁰⁰ is preferably a hydroxyl group or a chlorine atom.
B is preferably a phenyl group.
The position of substitution of B on the thiophene ring formed by V together with W is preferably the 4-position.
R³ is preferably a hydrogen atom.

The "pharmaceutically acceptable salt" may be any salt as long as it forms a non-toxic salt with a compound of the above-mentioned the formula [I], and can be obtained by reaction with inorganic acids such as hydrochloric acid, sulfuric acid, phosphoric acid, hydrobromic acid and the like; organic acids such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid, benzylsulfonic acid and the like; inorganic bases such as sodium hydroxide, potassium hydroxide, calcium hydroxide, magnesium hydroxide, ammonium hydroxide and the like; organic bases such as methylamine, diethylamine, triethylamine, triethanolamine, ethylenediamine, tris(hydroxymethyl)methylamine, guanidine, choline, cinchonine, N-methyl-D-glucamine and the like; or amino acids such as lysin, histidine, arginine, alanine and the like. The present invention also encompasses water-containing products, hydrates and solvates of each compound.

There exist various isomers of the compound represented by the above-mentioned formula [I]. For example, E-form and Z-form are present as geometric isomers, and when an asymmetric carbon atom exists, enantiomer and diastereomer are present as stereoisomers based thereon. In some cases, a tautomer can be present. Accordingly, the present invention encompasses all these isomers and mixtures thereof.

The present invention also encompasses prodrug and metabolite of the compound represented by the formula [I].

A "prodrug" is a derivative of the compound of the present invention, which has a group capable of chemical or metabolic decomposition, and shows inherent efficacy upon restoration to the original compound after administration to a living organism. It includes complexes and salts free of a covalent bond.

For example, an ester derivative known as a prodrug in the field of pharmaceutical agents can be used, which is specifically an ester derivative wherein the -COOR⁷ moiety for R is represented by the following formula.

-CO₂CH₃, -CO₂CH₂CH₃;

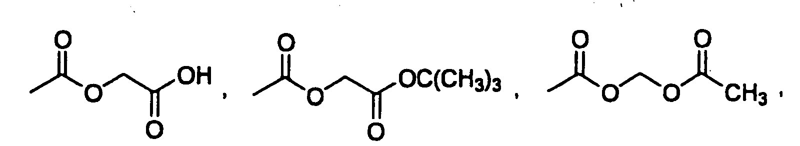

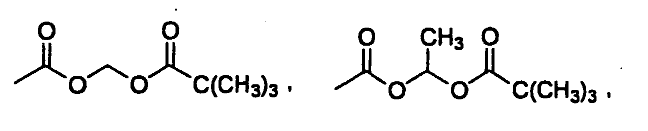

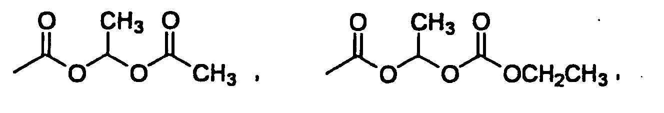

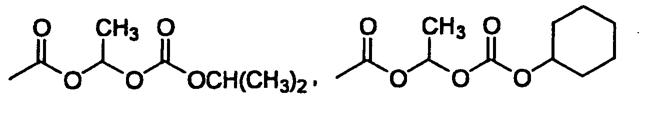

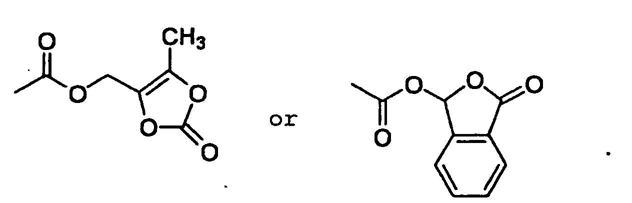

When the compound of the present invention is used as a pharmaceutical preparation, it is generally admixed with pharmacologically acceptable carrier, excipient, diluent, extending agent, disintegrant, stabilizer, preservative, buffer, emulsifier, aromatic, coloring agent, sweetener, thickener, corrigent, dissolution aids, and other additive generally known *per se*, which are specifically water, vegetable oil, alcohol such as ethanol, benzyl alcohol etc., polyethylene glycol, glycerol triacetate, gelatin, lactose, carbohydrate such as starch and the like, magnesium stearate, talc, lanolin, vaseline and the like, and systemically or topically, orally or parenterally administered in the form of tablet, pill, powder, granule, suppository, injection, eye drop, liquid, capsule, troche, aerosol, elixir, suspension, emulsion, syrup and the like by conventional methods.

While the dose of the compound of the present invention varies depending on age, body weight, symptoms, disease to be treated, administration method and the like, it is generally 1 mg to 1,000 mg for one dose to an adult, which is administered once a day to several times a day.

The compound [I] of the present invention can be administered as a PTP1B inhibitor or an inhibitor of receptor tyrosine kinase negative regulator, a drug for the prophylaxis or treatment of diabetes, a drug for the prophylaxis or treatment of diabetic complications (retinopathy, nephropathy, neuropathy, syndrome X or metabolic syndrome, ischemic heart diseases (cardiac infarction, angina pectoris etc.) and strokes (cerebral infarction, cerebral hemorrhage, subarachnoid hemorrhage etc.), a drug for the prophylaxis or treatment of hyperlipidemia, a drug for the prophylaxis or treatment of obesity, a drug for the prophylaxis or treatment of neurodegenerative diseases (macula edema, macular degeneration etc.), a drug for the prophylaxis or treatment of diseases mediated by PTP1B and the like to mammals (human, mouse, rat, rabbit, dog, cat, bovine, swine, simian etc.).

The compound [I] of the present invention has a superior and selective PTP1B inhibitory action. By the "superior and selective PTP1B inhibitory action" is meant that the PTP1B inhibitory activity is stronger as compared to other protein tyrosine phosphatases (e.g., T-cell protein tyrosine phosphatase (TCPTP) inhibitory activity), which specifically means, for example, IC₅₀ for PTP1B is as low as 1/10 or below that for TCPTP, more preferably 1/60 or below, particularly preferably 1/300 or below. Thus, the compound [I] of the present invention can be used as a PTP1B inhibitor, an inhibitor of receptor tyrosine kinase negative regulator, a drug for the prophylaxis or treatment of diabetes, a drug for the prophylaxis or treatment of diabetic complications, a drug for the prophylaxis or treatment of hyperlipidemia, a drug for the prophylaxis or treatment of obesity, neurodegenerative disease and the like or a drug for the prophylaxis or treatment of diseases mediated by PTP1B, which causes a fewer side effects.

The "inhibitor of receptor tyrosine kinase negative regulator" refers to a pharmaceutical agent that inhibits a factor that regulates intracellular signaling toward a negative direction, which signaling stems from activation of a receptor tyrosine kinase upon binding with a ligand, thereby enhancing the signal. In the case of an insulin receptor, for example, which is one of the receptor tyrosine kinases, wherein, as results of activation of the receptor as bound with insulin, intracellular region of the receptor itself, IRS (insulin receptor substrate) and the like are phosphorylated and intracellular signaling causing glucose uptake occurs, a pharmaceutical agent that inhibits a factor that regulates such signaling towards the negative direction is an inhibitor of receptor tyrosine kinase negative regulator, wherein a representative example is a PTP1B inhibitor. The inhibitor of receptor tyrosine kinase negative regulator is different from existing insulin sensitivity enhancers (PPAR agonists such as thiazolidindione derivative etc.). To be precise, because an inhibitor of receptor tyrosine kinase negative regulator, as represented by PTP1B inhibitor, directly acts on an intracellular signal induced by insulin, high efficacy is expected in mammals (non-obesity type diabetic patients etc.) that exhibit insufficient efficacy by the administration of existing insulin sensitizers, and side effects of existing insulin sensitizers have can be avoided.

The compound [I] of the present invention can be used in combination with other therapeutic agents for diabetes for the purpose of prophylaxis or treatment of diabetes or diabetic complications (particularly, microangio complications, in other words, retinopathy, nephropathy, neuropathy etc.) and concurrently administered to mammals. In the present invention, the "therapeutic agents for diabetes" encompasses therapeutic agents for diabetic complications (particularly, microangio complications). In addition, the compound [I] of the present invention can be used in combination with other therapeutic agents for hyperlipidemia for the purpose of prophylaxis or treatment of hyperlipidemia and concurrently administered to mammals. Furthermore, the compound [I] of the present invention can be used in combination with other therapeutic agents for obesity for the purpose of prophylaxis or treatment of obesity and concurrently administered to mammals.

Further, the compound [I] of the present invention can be used in combination with therapeutic agents for diabetes, therapeutic agents for hyperlipidemia, therapeutic agents for obesity, therapeutic agents for hypertension and/or therapeutic agents for thrombosis for the purpose of prophylaxis or treatment of diabetic complications (particularly, large artery complications, in other words, syndrome X or metabolic syndrome, ischemic heart diseases (cardiac infarction, angina pectoris etc.), strokes (cerebral infarction, cerebral hemorrhage, subarachnoid hemorrhage etc.) etc.) and concurrently administered to mammals.

When the compound is used in combination with other therapeutic agents for diabetes, other therapeutic agent for hyperlipidemia, other therapeutic agents for obesity, other therapeutic agent for hypertension or other therapeutic agent for thrombosis (hereinafter to be referred to as a concomitant pharmaceutical agent), the compound of the present invention may be simultaneously administered along with a concomitant pharmaceutical agent, or may be administered with time intervals. When the compound is used in combination with a concomitant pharmaceutical agent, the compound of the present invention and a concomitant pharmaceutical agent can be administered as a pharmaceutical composition containing them. Alternatively, a pharmaceutical composition containing the compound of the present invention and a pharmaceutical composition containing a concomitant pharmaceutical agent may be administered separately. The administration route of each pharmaceutical composition may be the same or different.

When the compound is used in combination with a concomitant pharmaceutical agent and when the concomitant pharmaceutical agent has a different action mechanism from the compound of the present invention (e.g., insulin secretagogue, biguanide agent, α-glucosidase inhibitor, insulin preparation, insulin sensitivity enhancer, inhibitor of receptor tyrosine kinase negative regulator other than PTP1B inhibitor etc.), an additive prophylaxis or treatment effect based on respective actions of the compound of the present invention and the concomitant pharmaceutical agent, as well as an amplified prophylaxis or treatment effect combining the both actions are expected. Thus, in each case of the compound of the present invention and the concomitant pharmaceutical agent, it is expected to have high efficacy by administration of fewer dose compared to that administered alone and, as a result, to solve side effect that cannot be avoided in case administered alone. Therefore, the compound of the present invention can be administered at a single dose of 1 mg to 1,000 mg, or even a smaller dose, once a day to several times a day. The concomitant pharmaceutical agent can be administered at a dose generally employed for the prophylaxis or treatment of diabetes or diabetic complications, hyperlipidemia or obesity, or even at a smaller dose.

As other therapeutic agents for diabetes that are used as concomitant pharmaceutical agents, insulin secretagogues (ATP-dependent potassium (K(ATP)) channel blockers (sulfonylureas, sulfonamides, phenylalanine derivatives, etc)), biguanides, a-glucosidase inhibitors, insulin formulations, insulin analogs, insulin sensitivity enhancers (peroxisome proliferators-activated receptor (PPAR)-gamma agonists such as thiazolidinedione derivatives (glitazones)), IL-11, anti-CD25 (IL-2 Receptor) agents (such as monoclonal antibodies), angiotensin (AT1) antagonists, angiotensin-converting enzyme (ACE) inhibitors, aldose reductase inhibitors,antioxidants (protein kinase activators/reverse transcriptase inhibitors), carnitine acetyltransferase stimulant, antidepressants, glucocorticoids, retilin, radical formation agonists and transketolase activatorscan be mentioned. Also can be mentioned are beta3-adrenoceptor agonists, insulin sensitizers, nuclear receptor modulators, peroxisome proliferator-activated receptors, PPAR-alpha agonists, PPAR-gamma antagonists, PPAR-delta ligands (agonists or antagonists), fibrates, drugs acting on retinoid receptors (retinoids, etc.), protein tyrosine phosphatase (PTP) inhibitors (other than compounds of the invention), protein tyrosine phosphatase-1B (PTP1B) inhibitors (other than compounds of the invention), T-cell protein tyrosine phosphatase (TCPTP) inhibitors (other than compounds of the invention), leukocyte common antigen-related protein (LAR) inhibitors, SH2-containing inositol phosphatase 2 (SHIP2) inhibitors, c-Jun NH2-terminal kinase (JNK) inhibitors, insulin receptor agonists, insulin receptor kinase stimulants, insulin-like growth factor (IGF)-1 antagonists, dipeptidyl-peptidase IV inhibitors, glucagon-like peptide 1 (GLP1) agonists, GLP1, leptin, ATP-dependent potassium channel activators; VPAC2 (receptor for vasoactive intestinal peptide (VIP)) agonists, P2Y agonists; beta amyloid protein antagonists, apoptosis inhibitors, TGR79 agonists, GPR40 agonists, sodium-glucose co-transporter inhibitors (SGLT-2 inhibitors, etc.), glycogen phosphorylase inhibitors, fructose-biphosphatase inhibitors, phosphoenolpyruvate carboxykinase (PEPCK) inhibitors, pyruvate dehydrogenase kinase inhibitors, glucose-6-phosphatase inhibitors, glycogen synthase kinase (GSK)-3 inhibitors, glucokinase activators, glucagon antagonists, signal transducer and activator of transcription (STAT)-3 modulators, forkhead transcription factor (FOXO-1) inhibitors, interleukin (IL) agonists such as IL-10 agonists and IL-4 agonists, IL-6 production inhibitors, tumor necrosis factor (TNF)-alpha production inhibitors, monocyte chemoattractant protein (MCP)-1 expression inhibitors, adipocytokine modulators, plasminogen activator inhibitor type 1 (PAI-1) production inhibitors, adiponectin production enhancers, adiponectin receptor agonists, adiponectin, resistine production inhibitors, resistine receptor antagonists, alpha 2 adrenoreceptor antagonists, beta2-adrenoceptor antagonists, 5-HT6 antagonists, serine carboxypeptidase inhibitor, ATP-binding cassette A1 (ABCA1) expression enhancers, retinoid X receptor (RXR) agonists, liver X receptor (LXR) ligands (agonists or antagonists), adypocite fatty acid binding protein (FABP) (aP2) inhibitors, cholesterol antagonists, adenosine A2B antagonists, alpha1-adrenoreceptor agonists, alpha2-adrenoreceptors, carnitine O-palmitoyltransferase inhibitors, AMP-activated protein kinase (AMPK) activators, glucose transporter (GLUT)-4 translocation activators, glucocorticoid receptor antagonists, glucocorticoid receptor modulators, 11beta-hydroxysteroid dehydrogenase inhibitors, growth hormone release inhibitors, somatostatin analogs, somatostatin sst5 agonists, somatostatin sst2 agonists, growth hormone-releasing factors (GHRF), ghrelin production inhibitors, ghrelin receptor antagonists, kinase inhibitors, PTPN1 expression inhibitor oligonucleotides, antioxidants, nitric oxide scavengers, free radical scavengers, heme oxygenase (HO)-1 inducers, EGFR (erbB1) inhibitors, phosphodiesterase III inhibitors, phosphodiesterase IV inhibitors, acetyl-CoA carboxylase (ACC) inhibitors, carnitine palmitoyltransferase (CPT) 1 inhibitors, glucose sensor activators, microsomal triglyceride transfer protein (MTP) inhibitors, diacylglycerol acyltransferase (DGAT) inhibitors, statins, cholesteryl ester transfer protein (CETP) inhibitors, any other glucose lowering agents, anti-CD3 agents (such as monoclonal antibodies), glutamate decarboxylase stimulants, combination of gastrin and epidermal growth factor (EGF), calcineurin inhibitors, antioxidants (metalloporphyrins, etc.), NAD+ ADP-ribosyltransferase (poly(ADP-ribose)polymerase; PARP) inhibitors, immunostimulants, chemokine receptor agonist, chemokine receptor antagonist, vanadium complexes, p38 protein kinase inhibitors, advanced glycation end product (AGE) inhibitors (Maillard's reaction inhibitors), low molecular weight (LMW) heparins, inducible nitric oxide synthase inhibitors, anti-platelet-derived growth factor (PDGF) agents (such as monoclonal antibodies), angiogenesis inhibitors (apoptosis inducers, endothelin (ETA) antagonists, etc.), somatostatin sst2 antagonists, growth factor modulators, cell adhesion inhibitors, amadorase inhibitors, transforming growth factor (TGF)-beta receptor antagonists, TGF-beta receptor signal inhibitors, TGF-beta production inhibitors, anti-TGF-beta antibodies, anti-TGF-beta receptor antibodies, anti-lectin-like oxidized low-density lipoprotein receptor (LOX)-1 agents (such as monoclonal antibodies), Edge receptor antagonists, Edge receptor signal inhibitors, neurotrophic factor enhancers, neurotrophic agents, nerve growth factor (NGF), NGF agonists, vascular endothelial growth factor (VEGF), neurotrophin (NT)-3, NT-3 inducers, protein kinase (PK) C beta inhibitors, drugs acting on gamma-aminobutyric acid (GABA)-mediated transmission, 5-HT reuptake inhibitors; 5-HT2A antagonists, norepinephrine reuptake inhibitors, drugs acting on vanilloid receptors, cannabinoid receptor agonists, IL-6, N-methyl-D-aspartate (NMDA) antagonists, prostaglandin E1, prostacyclin analogs, pyruvate dehydrogenase activators, nitric oxide synthase (NOS) 3 expression enhancers, heat shock protein general agonists, sodium channel blockers, NAALADase inhibitors, general pump inhibitors, sonic hedgehog-IgG, VEGF receptor antagonists, VEGF receptor signal inhibitors, pigment epithelial-derived factor (PEDF), anti-VEGF monoclonal antibodies, matrix metalloproteinase inhibitors, methionine aminopeptidase-2 inhibitors, somatostatin sst1 agonists, tyrosine kinase inhibitors, endothelial growth factor antagonists, vitronectin (alphavbeta3, alphavbeta5, etc.) antagonists, hypoxia-inducible factor (HIF) inhibitors, neuropilin-1 receptor antagonists, angiopoietin-2 receptor antagonists, angiopoietin-2 production inhibitors, angiopoietin-1 receptor agonists, angiopoietin-1 inducers, and the like. For example, nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide are used in combination as concomitant pharmaceutical agents for the compound of the present invention. Also used as concomitant pharmaceutical agents are balaglitazone, rivoglitazone, isaglitazone (netoglitazone), cryptolepine hydrochloride, triproamylin (pramlintide) acetate, mitiglinide calcium hydrate, muraglitazar, tesaglitazar, oxeglitazar, insulin glulisine, insulin detemir, hexyl-insulin monoconjugate 2, AeroDose insulin inhaler, AIR-insulin,' Technosphere/insulin, rDNA insulin, any other inhaled, pulmonary or oral human insulin, exendin-4, sulphostin, liraglutide, recombinant glucagon-like peptide-1 (7-36) amide (AC-2592), dexlipotam, cyclipostin R, ramipril, dehydrotrametenolic acid, famoxin, methoxime-3,4-dephostatin, demethylasterriquinone B-1, adiponectin (human), salacinol, leporin B, TAK-654, MBX-102, ADD-9918, ADD-9922, NN-304, NN-344, BIM-51077, LABI (NN-1215), SNAC/Insulin, LAF-237, 823093, 825964, 815541, P93/01, SSR-162329, LY-510929 (LY-929), LBL-752 (DRF-MDX8, DRF-4158), GW-677954, LY-307161 SR, CJC-1131, SUN-E7001, ZP-10A (AVE-0010), MB-6322 (CS-917), BVT-2498, INGAP peptide, LY-818, TH-9507, ISIS-113715, SR-58611, YM-178, SB-418790 (AZ-40140, GW-427353), N-5984, NN-2501, RF-1051, HMR-1426, NOX-700, ATL-962, 869682, R-1439, R-765, GW-501516, GI-181771, ST-1326, VDO-52, A-348441, BLX-1002, TLK-19781, ZYH-2, BLX-2001, NC-1567, R-1440, (FMS)3-insulin, insulin chain B (9-23) peptide, Genapol-stabilized insulin, insulin transdermal, metreleptin (recombinant methionyl human leptin), islet neogenesis therapy, NBI-6024, NN-344, O-346, DiaPep227 (AVE-0277), TRX-4, rhGAD65, rhIGF-I (rhIGFBP-3), R-1524 (ISA-247, ISAtx-247), TRX-4-TolerMab, MnTE-2-Pyp5+ (AEOL-10113), CTCM-226, irbesartan, telmisartan, candesartan cilexetil (hexetil), pratosartan, pyridoxamine (pyridoxylamine), magnesium lithospermate B (Lithospermic acid B magnesium salt), sulodexide, aminoguanidine (pimagedine), pirfenidone, 1D11, CR-002, T-8, (+)-A-127722 (A-147627, ABT-627), ED-4914, fidarestat, nefazodone hydrochloride, venlafaxine hydrochloride, pregabalin, ruboxistaurin mesilate hydrate, recombinant human nerve growth factor, capsavanil, dronabinol/cannabidiol, atexakin alfa (interleukin-6), memantine hydrochloride, dexlipotam, beraprost sodium, arimoclomol maleate, SX-3201 (AS-3201, SX-3030), TAK-428, QR-333, ruboxistaurin hydrochloride, octreotide acetate, aminoguanidine (pimagedine), pegaptanib octasodium, hyaluronidase, sescandelin, batimastat, recombinant angiopoietin-1, LY-338522, BIM-23190, AdPEDF.11 (Ad(GV)PEDF.11D), VEGF Trap (VEG Trap(R1R2), CVT-3634, and the like. Preferably, other therapeutic agents for diabetes include insulin preparation, insulin secretagogue, biguanide, a-glucosidase inhibitor and insulin sensitivity enhancer, such as insulin, glibenclamide, tolbutamide, nateglinide, metformin hydrochloride, voglibose and pioglitazone hydrochloride.

As other therapeutic agents for hyperlipidemia that are used as concomitant pharmaceutical agents, HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids, can be mentioned. Also can be mentioned are ACAT inhibitors, adenosine A1 agonists, ApoB expression inhibitors, ApoB secretion inhibitors, microsomal triglyceride transfer protein (MTP) inhibitors, HDL-cholesterol increasing agents, cholesteryl ester transfer protein (CETP) inhibitors, ileal bile acid transporter inhibitors, insulin sensitizers, PPAR-alpha agonists, PPAR-gamma agonists, PPAR-delta agonists, squalene synthase inhibitors, testosterone agonists, 11beta-hydroxysteroid dehydrogenase inhibitors, 15-lipoxygenase inhibitors, 5-HT2 antagonists, ABCA1 expression enhancers, ACAT inhibitors, retinoid RXR agonists, liver X receptor (LXR) agonists, acetyl-CoA carboxylase inhibitors, acetyl-CoA thiolase inhibitors, adenosine A2A agonists, adenylate cyclase activators, adypocite FABP (aP2) inhibitors, keratinocyte FABP (k-FABP) inhibitors, aldose reductase inhibitors, angiogenesis inhibitors, anti-ICAM-1 agents, antioxidants, VCAM-1 antagonists, antiestrogens, estrogen agonists, lipid peroxidation inhibitors, ApoB secretion inhibitors, ApoB-100 lowering agents, apoptosis inducers, apoptosis inhibitors, ATP citrate lyase inhibitors, beta3-adrenoceptor agonists, calcium channel blockers, carnitine O-palmitoyltransferase inhibitors, cholesterol esterase inhibitors, drugs acting on farnesoid X receptors (FXR), farnesoid X receptor (FXR) agonists, drugs acting on thyroid hormone receptors, drugs acting on thyroid hormones, thyroid hormones, parathyroid hormones, farnesyl transferase inhibitors, glycogen phosphorylase a inhibitors, lanosterol 14alpha-demethylase inhibitors, lanosterol synthase inhibitors, histamine H1 antagonists, nitric oxide synthase inhibitors, thromboxane A2 antagonists, triacylglycerol lipase inhibitors, alpha-glucosidase inhibitors, LDL-receptor up-regulators, LYPLA1 expression inhibitors, non-steroidal antiinflammatory drugs, PDGFR inhibitors, phospholipase A2 inhibitors, platelet-activating factor (PAF) antagonists, potassium channel activators, prostaglandins, SCAP ligands, squalene epoxidase inhibitors, selective estrogen receptor modulators (SERM), sterol DELTA14-reductase inhibitors, vasopressin Vla antagonists, and the like. For example, lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid) are used in combination as concomitant pharmaceutical agents for the compound of the present invention. Also used as concomitant pharmaceutical agents are pactimibe, eflucimibe, implitapide, large unilamellar vesicles, campestanol ascorbyl phosphate, sitostanol ascorbyl phosphate, amlodipine besylate, GW-493838, RPR-749, BAY-13-9952, CP-346086, BTG-511, JTT-705, LY-518674 (LY-674), ESP-31015 (ETC-1001), ETC-642 (ESP-24228, RLT), DRF-4832, S-8921, LY-510929 (LY-929), GW-501516 (GW-516), FM-VP4, JTT-130, GW-590735, 641597, KRP-101, TAK-475, HE-2200 (3beta,7beta,17beta-androstenetriol), D-003, PLT-732, NS-220, KS-01-019, AZD-7806, AZD-4619, AZD-6610, AZD-8294, ZYH-1, DRF-10945, SMP-797, and the like.

As other therapeutic agent for obesity that are used as concomitant pharmaceutical agents, mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone can be mentioned. Also can be mentioned are drugs acting on cannabinoid receptors, cannabinoid CB1 receptor antagonists, cannabinoid CB1 receptor inverse agonists, ciliary neurotrophic factor (CNTF), growth hormones, growth hormone secretagogues, triacylglycerol lipase inhibitors, ABCA1 expression enhancers, acetyl-CoA carboxylase inhibitors, adypocite FABP (aP2) Inhibitors, aldose reductase inhibitors, alpha-glucosidase inhibitors, alpha-mannosidase inhibitors, ATP citrate lyase inhibitors, adenosine A1 receptor agonists, serine carboxypeptidase inhibitors, 11beta-hydroxysteroid dehydrogenase inhibitors, drugs acting on neuropeptide Y (NPY) receptors, neuropeptide Y2 (NPY Y2) receptor agonists, neuropeptide Y1 (NPY Y1) receptor antagonists, neuropeptide Y5 (NPY Y5) receptor antagonists, CCK antagonists, CCK1 (CCKA) agonists, CCK2 (CCKB/gastrin) antagonists, CRF1 antagonists, CRF2 agonists, drugs acting on melanin-concentrating hormone (MCH) receptors, MCH receptor antagonists, drugs acting on melanocortin receptors, melanocortin MC1 agonists, melanocortin MC3 agonists, melanocortin MC4 agonists, melanocortin MC5 agonists, orexin receptor antagonists, orexin OX-1 antagonists, orexin OX-2 antagonists, neurotensin agonists, tachykinin NK2 antagonists, drugs acting on thyroid hormones, thyroid hormone receptor beta agonists, galanin GAL1 antagonists, GHS (Ghrelin) receptor inverse agonists, glucocorticoid receptor modulators, beta2-adrenoceptor agonists, beta2-adrenoceptor antagonists, beta3-adrenoceptor agonists, alpha2-adrenoceptor antagonists, MAO inhibitors, dopamine D1 antagonists, dopamine D5 antagonists, dopamine reuptake inhibitors, dopamine-releasing drugs, norepinephrine reuptake inhibitors, drugs acting on 5-hydroxytryptamine receptors, 5-HT release.stimulants, 5-HT1A antagonists, 5-HT1B agonists, drugs binding to 5-HT2 receptors, 5-HT2A antagonists, 5-HT2B agonists, 5-HT2C (5-HT1C) agonists, drugs binding to 5-HT6 receptors, 5-HT6 antagonists, drugs binding to histamine H3 receptors, histamine H3 antagonists, opioid antagonists, kappa-opioid antagonists, estrogen agonists, selective estrogen receptor modulators (SERM), apoB secretion inhibitors, microsomal triglyceride transfer protein (MTP) inhibitors, benzodiazepines, GABA(A) BZ site receptor agonists, CEBPA expression inhibitor oligonucleotides, SAPK1 (JNK) inhibitors, DAT inhibitors, NET inhibitors, SERT inhibitors, dipeptidyl-peptidase IV inhibitors, drugs acting on glucagon-like peptide 1 (GLP-1) receptors, tripeptidyl peptidase II inhibitors, drugs acting on peroxisome proliferator-activated receptors (PPAR), PPAR-alpha agonists, PPAR-gamma agonists, PPAR-gamma antagonists, PPAR-gamma partial agonists, PPAR-delta agonists, retinoid RXR agonists, retinoid RXR antagonists, fatty acid synthase inhibitors, glycogen phosphorylase inhibitors, HMG-CoA reductase inhibitors, protein tyrosine phosphatase (PTP)-1B inhibitors (other than compounds of the invention), metalloporphyrins, nitric oxide synthase inhibitors, non-steroidal antiinflammatory drugs, phosphodiesterase III inhibitors, phosphodiesterase PDE3B inhibitors, SGLT-1 inhibitors, SGLT-2 inhibitors, sigma-receptor ligands, and the like. For example, mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate are used in combination as concomitant pharmaceutical agents for the compound of the present invention. Also used as concomitant pharmaceutical agents are rimonabant hydrochloride, SR-147778, CNTF (Ax15) (RG-228, RPN-228), pegylated axokine, GI-181771, SR-146131, SR-125180, AOD-9604, ATL-962, PYY3-36 (AC-162352), N-5984, L-796568, garcinia acid ((-)-Hydroxycitric acid), HMR-1426, P-57, 1954, chromium picolinate/conjugated linoleic acid, S-2367, APD-356, BVT-5182, Ucn II, C-75, gAcrp30, KB-141, NLC-002, BLX-2002, P-64, T-71 and the like.

As other therapeutic agents for hypertension that are used as concomitant pharmaceutical agents, thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators; GABA(A) receptor antagonists and phosphodiesterase V (PDE5A) inhibitors can be mentioned. Also can be mentioned are 5-HT1A receptor agonists, 5-HT1B antagonists, {5-HT2 antagonists,} 5-HT2A antagonists, ACAT inhibitors, adenosine A1 agonists, adenosine A1 antagonists, adenosine A2A agonists, adenosine A2A antagonists, adenylate cyclase activators, adrenergic neuron blockers, AGE inhibitors (Maillard's reaction inhibitors), aldose reductase inhibitors, {aldosterone antagonists,} alkaloids, alpha-adrenoceptor ligands, {alpha-adrenoceptor antagonists, alpha1-adrenoceptor antagonists,} alpha1A-adrenoceptor antagonists, {alpha2-adrenoceptor agonists,} alpha2-adrenoceptor antagonists, beta2-adrenoceptor antagonists, {beta-adrenoceptor antagonists, beta1-adrenoceptor antagonists,} beta2-adrenoceptor agonists, alpha-atrial natriuretic peptides, {AMPA receptor modulators,} angiogenesis inhibitors, apoptosis inhibitors, {angiotensin-I converting enzyme (ACE) inhibitors, angiotensin AT1 antagonists}, angiotensin AT2 antagonists, angiotensin receptor antagonists, antiinflammatory drugs, free radical scavengers, {antioxidants,} calcium channel activators, {calcium channel blockers,} L-type calcium channel blockers, {platelet-activating factor (PAF) antagonists,} cAMP phosphodiesterase inhibitors, cannabinoid CB2 agonists, cannabinoid receptor agonists, vanilloid VR1 receptor agonists, chymase inhibitors, cyclooxygenase-2 inhibitors, dopamine autoreceptor agonists, dopamine receptor agonists, dopamine D1 agonists, {dopamine D2 agonists,} dopamine D1 antagonists, {dopamine D2 antagonists,} dopamine-beta-monooxygenase inhibitors, endothelin receptor antagonists, {endothelin ETA receptor antagonists, endothelin ETB receptor antagonists,} endothelin receptor agonists, endothelin-converting enzyme inhibitors, enkephalinase inhibitors, {GABA(A) receptor antagonists,} glycogen phosphorylase a inhibitors, growth hormone release inhibitors, somatostatin analogs, {guanylate cyclase activators,} histamine H1 agonists, histamine H1 antagonists, hypoxia inducible factor 1-alpha (HIF-1alpha) inhibitors, drugs acting on imidazoline receptors, (imidazoline I1 receptor agonists,} insulin analogs (zinc complexes), insulin sensitizers, {PPAR-alpha agonists,} PPAR-gamma agonists, insulin lowering agents, {K(ATP) channel activators,} K(ATP) channel blockers, lipoxygenase inhibitors, large conductance BK(Ca) channel activators, {Na+/H+ exchange inhibitors,} Na+/K+-ATPase inhibitors, NADPH oxidase inhibitors, neprilysin inhibitors, neuropeptide Y1 (NPY Y1) antagonists, neuropeptide Y4 (NPY Y4) agonists, {nicotinic antagonists,} nitrates, {nitric oxide donors,} non-steroidal antiinflammatory drugs, protein kinase C (PKC) inhibitors, {NOS3 expression enhancers,} oligonucleotides, PDGFR inhibitors, phosphodiesterase inhibitors, phosphodiesterase I inhibitors, phosphodiesterase III inhibitors, phosphodiesterase IV inhibitors, {phosphodiesterase V (PDE5A) inhibitors,} potassium channel activators, {prostacyclin analogs, prostaglandins,} prostanoid IP agonists, prostanoid TP (thromboxane A2) antagonists, thromboxane synthase inhibitors, protease inhibitors, proteasome inhibitors, protein kinase C (PKC) inhibitors, renin inhibitors, {Rho kinase inhibitors,} sigma-receptor antagonists, sodium channel blockers, thiazides, triglyceride lowering agents, vasopressin antagonists, vasopressin V1a antagonists, vasopressin V2 antagonists, and the like. For example, chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa are used in combination as concomitant pharmaceutical agents for the compound of the present invention. Also used as concomitant pharmaceutical agents are omapatrilat, aladotril (alatriopril, fasidotril), AVE-7688 (MDL-107688), 796406, lemildipine, pranidipine, clevidipine, levamlodipine ((S)-amlodipine), ambrisentan, sitaxsentan sodium, SPP-301 (R-639, RO-67-0565), TBC-3711, PMD-2850 (angiotensin vaccine), PMD-3117, Binodenoson, alagebrium chloride, SLV-306, aliskiren fumarate, NV-04, MC-4232, ENO, MDL-72832, BP-554, (+)-OSU-191 (OSU-191-(+), U-86192A), WAY-100339, QF-303B (QF-0303B), QF-307B (QF-0307B), QF-311B (QF-0311B), lidanserin (ZK-33839), AP-1067, AHR-16303B, QF-313B (QF-0313B), S-14280, S-35031, S-35120, PR-1173, RG-14718, CCPA, S-ENBA, Sch-59761, FR-166124, GU-285, MDL-101483, CVT-2995, CVT-3032, CVT-3033, 2HE-NECA (HENECA), CGS-21680, CGS-22492, CGS-22989, SHA-40, APEC, KF-17837, OPB-9195, lithospermic acid B magnesium salt (magnesium lithospermate B), 6-iodoamiloride, 6-protoberberine (PTB-6), CR-2991, HSR-175, CDRI-93/478, DC-015 (DC-028, DL-028A), BAM-2202, GB-67, YM-11133, Abbott-65265, SK&F-104856, MG-1, L-765314, KMUP-880602, A-68828, YC-1, AB-47, C-112, utibaprilat, FPL-63674 (FPL-63674XX, FPL-63547-diacid), FPL-66564, MDL-100173, MDL-27467A, glycopril, ROO 911, SQ-31440, DU-1777, S-nitrosocaptopril (SNOCAP), Y-23785, enalapril nitrate, moexiprilat (RS-10029), temocaprilat (RS-5139), CV-5975, imidaprilat (6366 A), REV-6134, BW-B385C, CGS-26582, SA-6817, SA-7060, Sch-54470, S-allylmercaptocaptopril (CPSSA), RB-106, MDL-101628, mixanpril, fasidotrilat (alatrioprilat), BMS-182657, ER-32897, ER-32935, ER-40121, ER-40133 (E-4030), CGS-28106, CGS-30440, Sch-50690, A-81282 (Abbott-81282), A-81988, GA-0050, GA-0113, ZD-7155, RU-63455, RU-64276, RU-65868, embusartan (Bay-10-6734), BIBS-39, BMS-180560, BMS-181688, BMS-184698, EXP-063, EXP6155, EXP-7711 (IMI), EXP-9270, S-8308, XD-937, XH-148, UP-221-78, UP-275-22, FI-6828K, FR-130739, FR-143187, FR-149581, LR-B/087, RWJ-38970, RWJ-46458, WK-1492 (WK-1492.2K), KR-31080 (SK-1080), KT3-579, KT3-866, KW-3433, LY-285434, LY-301875, LY-302289, L-158338, L-158659, L-158978, L-159093, L-159689, L-161177, L-162154, L-162223, L-162234, L-162393, L-162474, L-163313, L-163579, EMD-69943, EMD-90423, tisartan (CGP-48369), abitesartan (CGP-49870), CP-161418, CP-191166, PD-123177 (EXP-655), PD-123319, PD-150304, SC-50560, SC-51316, SC-51757, SC-51895, SC-54628, SC-54629, U-97018, RWJ-47639, TH-142177, CV-11194, 606A, TA-606, LCY-018, CR-3210, UP-275-13, UR-7198, UR-7280, WK-1260, CL-190133 (CL-332877, CL-190733, CL-190734), WAY-126227, YM-31472, EXP-408, XR-510, L-161021, L-162389, L-162441, L-162537, L-163007, L-163017, L-163958, BMS-248360, DMP-811 (DuP-811, L-708404), SB-203220, LR-B/057, L-159913, L-161816, sesamin, ferulinolol, PF-9104, TZC-1370, O-palmitoyl tilisolol, BG6, TZC-8159, Y-22516, olradipine hydrochloride (S-11568), PCA-50938, H-324/38, ZM-224832, HP-406, BMY-43011, SQ-32321, SQ-32547, SQ-33351, elnadipine, rhynchophylline, SUN-5647, VULM-999, DCDDP, sagandipine, GR-60139, Goe-5584-A, siratiazem hydrobromide LR-A/113), GS-386, sornidipine, MCF-1084, MCF-1113, dibudipine, deuterated nifedipine (ISA-1058), McN-6497 (RWJ-26902), CRL-41695, (4S)-(+)-AE0047, Y-24149, s-petasin, NIP-101, UK-52831, UK-55444, UK-56593, UK-51656, P-1268, BBR-2160, RS-88007, RS-5773, SL-85.1097, 83-0256, 83-0327, S-312 (83-0312), MR-14134, AHR-12742, AHR-16462B, AHR-5360C (AHR-5360), BMY-20014, BMY-20064, BMY-42803, veroxan, labedipinedilol B, labedipinedilol A, FR-172516, vanidipinedilol, xanthonolol, Z-6568, F-1850, Wy-27569, M-54033, HU-308, arvanil, BL-3875, BL-4027, TEI-E00548, rutaecarpine (rutecarpine), PD-139899, FPL-65447AA, Z-12571, Z-7760, (S)-Z-11410, FR-138104, MDL-43925, SK&F-102698, A-292438, A-306552, RPR-117820A, RPR-118031A, J-105510, J-105859, J-112287, BMS-182874, TBC-11040, TBC-11192, TBC-2576, TBC-3214, SB-247083, EMD-122946, EMD-94246, fandosentan potassium (CI-1034, PD-180988), PABSA, YM-62899, YM-91746, LU-302872 (BSF-420627, LU-420627), BMS-187308, IRL-3630, L-747844, RPR-111844, PD-163070, PD-166309, PD-142893, A-192621, CGS-31398, IRL-1620, BQ-485, FR-901366 (WS-009A), KET-011, RES-701-1 (KF-20704), L-744453, L-749329, L-749805, IRL-1737, PD-155080, PD-156252, T-0115 (TA-0115), S-17162, WS-79089B (WS-79089-3, FR-901533), SM-19712, CGS-26303, CGS-26393, CGS-30084, CGS-34043, CGS-34225, CGS-34226, CGS-34753, CGS-35066, KC-12792, SQ-28603, CP-91149, BAY-41-2272, BAY-41-8543, BAY-58-2667, KMUP-1, methylhistaprodifen, suprahistaprodifen, S-23515, S-23757, LNP-509, LNP-911, Zn(car)2C12, DRF-4158 (DRF-MDX8, LBL-752), DY-9804, MJ-355, BPDZ-79, BPDZ-83, YM-099, sarakalim (RS-91309), PNU-96293, NS-1608, CPU-23, PST-2107, apocynin, CGS-24128, CGS-24592, CGS-25155, H-394/84, BIBO-3304, 1229U91 (GR-231118, BW-1229U91, GW-1229), RS-7897, C92-4609 (CAS-1609), CHF-2363 (SN011), PROLI/NO, FR-144420, GEA-3175, DS1, NOC-7, MAHMA MONOate, PF-9404, EDTA (edetic acid), AS-AT1R-ODN mRNA, NX-1975, 5E3623, Sch-51866, Sch-59498, LAS-31180, SK&F-94120, SK&F-95654, Win-63291, DMPPO, DF-100 (A80a), E-4010, zaprinast (M&B-22948), T-1032, iptakalim hydrochloride, ZM-260384, RP-66784, S-0121, BMS-182264, KC-399, KC-515, SG-209, DY-9708, ER-001533, RWJ-26629, U-94968, KI-4032, KRN-4884, KR-30818 (SKP-818), P-1060, LM-3339, MCC-134, AL-0671, AL-0670, Y-26763, PC-286, TAK-636, dioclein, UR-8081, UR-8218, UR-8225, UR-8267, CL-188294, LP-805, KMUP-880708, cromakalim, BW-68C, FR-181157, FR-181877, ER-017996, PSI, MDL-29152, A-62198, A-65317, A-68064, A-82110, A-70461, ICI-219623, MDL-73323, MDL-74147, S-2864, BW-175, BILA-2157, SQ-30774, SQ-31844, SQ-33800, JTP-2438, JTP-2724, JTP-3072, JTP-4129, JTV-505 (JTP-4761), KRI-1177, KRI-1314, CGP-44099A, CGP-54061, CGP-55128A, CGP-56346A, CGP-56962A, CGP-62198A, CP-108671, CP-71362, PD-132002, SC-46944, SC-56525, U-77436, Ro-44-9375, Ro-66-1132 (RO-0661132, RO-X1), RO-X2, SPP-600, CL-331049, WAY-121604, YM-21095, YM-26365, hydroxyfasudil, Y-30141, H-1152 (HMN-1152), CNS-1307, Ono-1505, JTV-605, YM-176770, JNJ-17079166, FR-161282, and the like.

As other therapeutic agents for thrombosis that are used as concomitant pharmaceutical agents, heparin preparations, low molecular weight heparins, heparin analogs, anticoagulants, thrombin inhibitors, anti-thrombin preparations, antiplatelet agents thrombolytic agents and the like can be mentioned. For example, heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate, protein C, and the like are used in combination as concomitant pharmaceutical agents for the compound of the present invention.

In the following, examples of production methods of the compound of the present invention used to practice the present invention are given. However, the production methods of the compound of the present invention are not limited to those exemplified below.

Even in the absence of specific description in the present production methods, efficiently production can be performed by incorporating modification where necessary, such as introducing a protecting group into a functional group, followed by deprotection in a later step, exchanging the order of production method and steps and the like.

The work-up in each step can be performed according to methods generally employed, such as washing by extraction, concentration, filtration, adjustment of pH and the like, wherein isolation and purification can be performed by employing suitable conventional methods such as crystallization, recrystallization, silica gel chromatography, preparative HPLC and the like, which may be a single method or two or more methods in combination.

### Production Method 1

In this Production Method, compound [I] wherein m is 1, R¹ and R² are each a hydrogen atom and X is -N(R⁴)-, -N(R⁵)-COO-, -O- or -S- is produced. 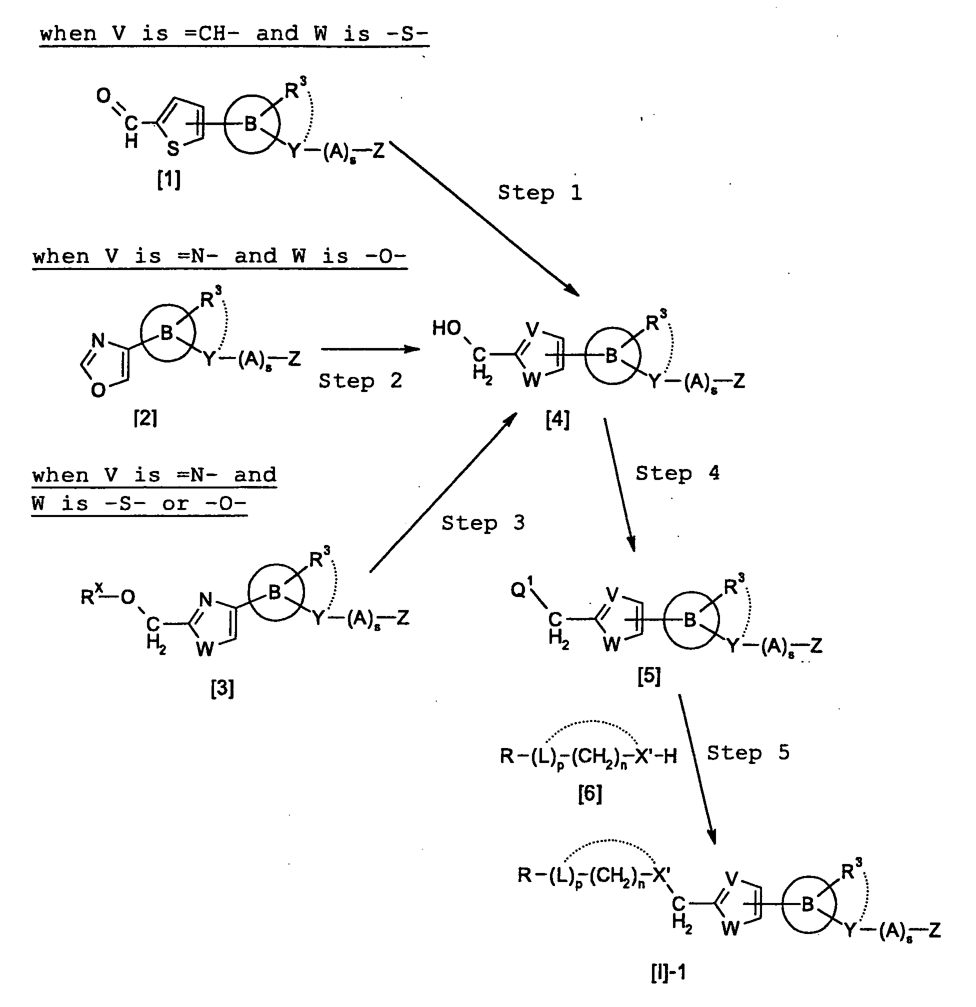 wherein Q¹ is a general leaving group (e.g., halogen atom (bromine atom, iodine atom, chlorine atom etc.), a mesyloxy group, a tosyloxy group, a trifluoromethanesulfonyloxy group etc.), R^{X} is a general hydroxyl-protecting group (e.g., tertbutyldimethylsilyl group, benzoyl group etc.), X' is -N(R⁴)-, -N(R⁵)-CO-O-, -O- or -S- wherein each symbol is as defined above and other symbols are as defined above.

### Step 1

Compound [4] can be obtained by reduction of compound [1] in a solvent using a conventional reducing agent. As the solvent, alcohols (methanol, ethanol etc.), ether solvents (tetrahydrofuran, dioxane etc.), toluene and the like, and a mixed solvent thereof can be mentioned. As the reducing agent, sodium borohydride, lithium borohydride, diborane, lithium aluminum hydride and the like can be mentioned. The reaction can be carried out under cooling to heating.

### Step 2

Compound [4] can be obtained by reacting compound [2] with paraformaldehyde in a solvent in the presence of a base for hydroxymethylation. As the solvent, toluene, ether solvents (tetrahydrofuran, diethyl ether etc.) and the like and a mixed solvent thereof can be mentioned. As the base, a base obtained using alkyllithium (n-butyllithium, sec-butyllithium, tert-butyllithium etc.) and 2,2,6,6-tetramethylpiperidine, lithium diisopropylamine and the like can be mentioned. The reaction can be carried out under cooling.

### Step 3

Compound [4] can be obtained by deprotection of R^{X} of compound [3] according to conventional methods. When R^{X} is a benzoyl group, for example, the object product can be obtained by a reaction similar to the method exemplified in Step 30 of Production Method 19.

### Step 4

Compound [5] can be obtained by substituting a hydroxyl group of compound [4] with a conventional leaving group in a similar manner as in Step 18 of Production Method 10.

### Step 5

Compound [I]-1 can be obtained by reacting compound [5] with compound [6] in a solvent in the presence of base and in the presence or absence of a catalyst. As the solvent, N,N-dimethylacetamide, N,N-dimethylformamide, acetonitrile, ethyl acetate, toluene, alcohol solvents (methanol, ethanol etc.), ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. As the catalyst, potassium iodide, quaternary ammonium salt (tetrabutylammonium iodide, tetrabutylammonium bromide etc.) and the like can be mentioned. The reaction can be carried out under cooling to heating.

### Production Method 2

In this Production Method, of the compounds [6] used in Step 5 of Production Method 1, a compound wherein X' is -N(R⁴)-is produced. 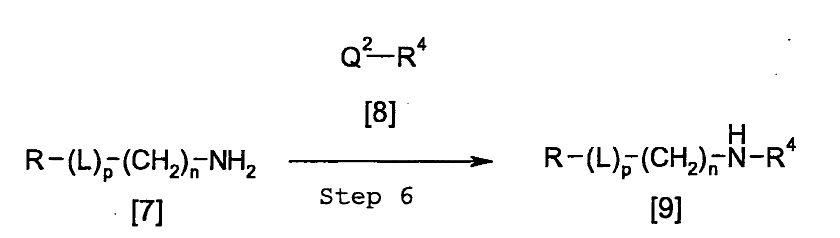 wherein Q² is a general leaving group as described in Production Method 1 and other symbols are as defined above.

### Step 6

Compound [9] can be obtained by reacting compound [7] with compound [8] in a solvent in the presence of a base and in the presence or absence of a catalyst. As the solvent, N,N-dimethylacetamide, N,N-dimethylformamide, acetonitrile, ethyl acetate, toluene, alcohol solvents (methanol, ethanol etc.), ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. As the catalyst, potassium iodide, quaternary ammonium salt (tetrabutylammonium iodide, tetrabutylammonium bromide etc.) and the like can be mentioned. The reaction can be carried out under cooling to heating.

### Production Method 3

In this Production Method, compound [I] wherein V is =CH-, W is -S-, m is 1, R¹ and R² are each a hydrogen atom and X is - N(R⁴)- is produced. 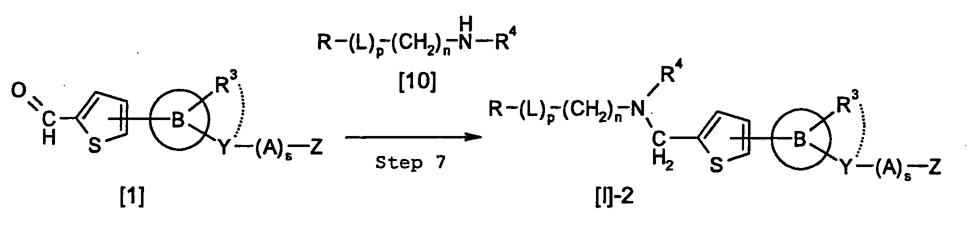 wherein each symbol is as defined above.

### Step 7

Compound [I]-2 can be obtained by reacting compound [1] with compound [10] in a solvent in the presence of a reducing agent and in the presence or absence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane, 1,2-dichloroethane etc.), alcohol solvents (methanol, ethanol etc.), acetic acid and the like and a mixed solvent thereof can be mentioned. As the reducing agent, sodium borohydride, sodium triacetoxyborohydride, sodium cyanoborohydride and the like can be mentioned. As the base, organic bases such as pyridine and the like can be mentioned. The reaction temperature is preferably 0°C - 80°C.

### Production Method 4

In this Production Method, compound [I] wherein V is =CH-, W is -S-, m is 0 and X is -N(R⁸)-CO- is produced. 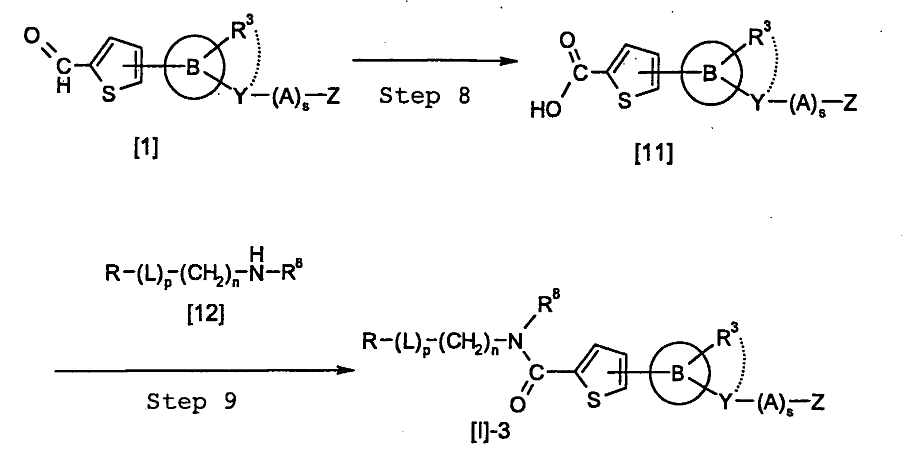 wherein each symbol is as defined above.

### Step 8

Compound [11] can be obtained by oxidation of aldehyde of compound [1] using a conventional oxidizing agent in a solvent. As the solvent, alcohols (methanol, ethanol etc.), ether solvents (tetrahydrofuran, dioxane etc.), acetone, acetic acid, water and the like, and a mixed solvent thereof can be mentioned. As the oxidizing agent, sodium chlorite (where necessary, a chlorine scavenger (sulfamic acid etc.) is added), sodium hypochlorite, potassium permanganate, sodium chromate, silver nitrate, silver oxide, 2,2,6,6-tetramethylpiperidine-N-oxide and the like can be mentioned. The reaction can be carried out under cooling to heating.

### Step 9

Compound [I]-3 can be obtained by amide condensation of compound [11] with compound [12] obtained by a method similar to that to give compound [9] in Production Method 2. The amide condensation can be performed according to conventional methods. For example, a method comprising condensation in a solvent such as N,N-dimethylformamide, acetonitrile, tetrahydrofuran, chloroform, ethyl acetate, methylene chloride, toluene and the like, in the presence of a condensing agent such as dicyclohexylcarbodiimide, 1-ethyl-3-(3-dimethylaminopropyl)carbodiimide hydrochloride, diphenylphosphoryl azide and the like, and in the presence or absence of N-hydroxysuccinimide, 1-hydroxybenzotriazole and the like can be mentioned. The reaction temperature is preferably 0°C - 100°C.

### Production Method 5

In this Production Method, compound [I] wherein m is 1, R¹ and R² are each a hydrogen atom and X is -N(R⁴)- is produced. 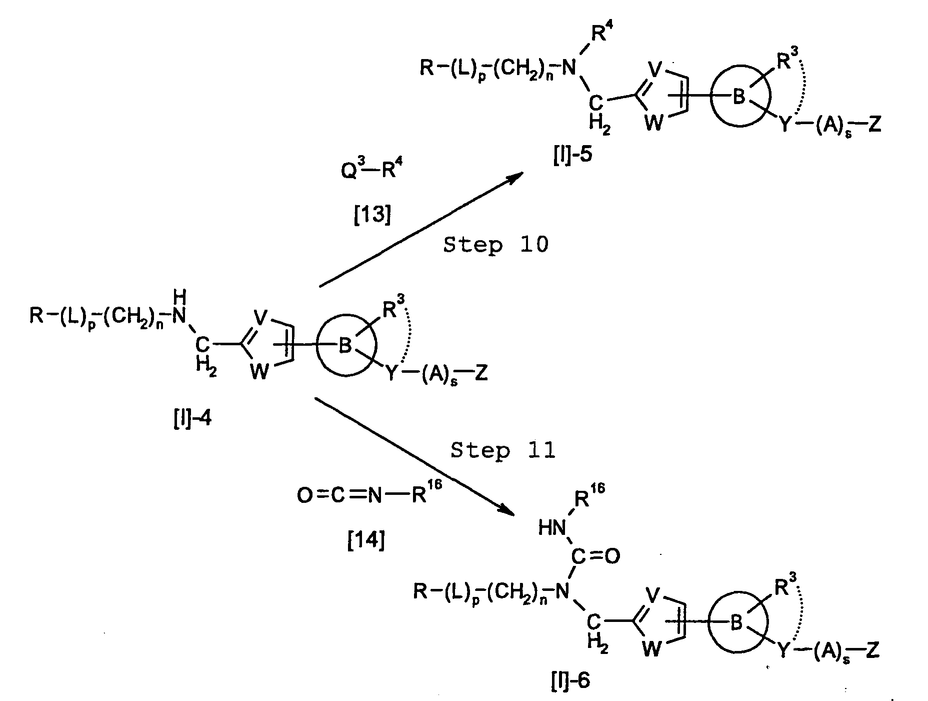 wherein Q³ is a general leaving group as described in Production Method 1 and other symbols are as defined above.

### Step 10

Compound [I]-5 can be obtained by reaction of compound [I]-4 obtained by a method similar to that to give compound [I]-1 in Production Method 1 with compound [13] in a solvent in the presence or absence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. The reaction can be carried out under cooling to heating.

### Step 11

Compound [I]-6 can be obtained by reacting compound [I]-4 with compound [14] in a solvent in the presence or absence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. The reaction can be carried out under cooling to heating.

### Production Method 6

In this Production Method, compound [I] wherein m is 1 or 2 and X is -N(R⁴)- is produced. 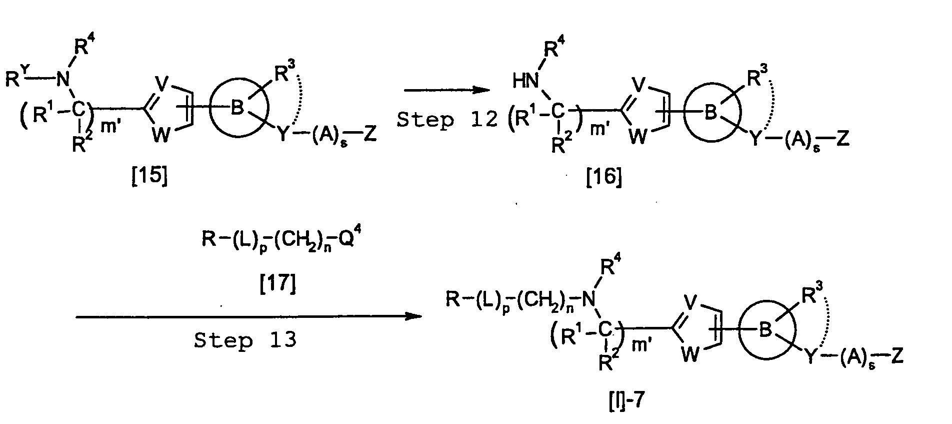 wherein m' is 1 or 2, Q⁴ is a general leaving group as described in Production Method 1, R^{Y} is an amine-protecting group such as tert-butoxycarbonyl group, benzyloxycarbonyl group and the like, and other symbols are as defined above.

### Step 12

Compound [16] can be obtained by deprotection of R^{Y} of compound [15]. The deprotection can be conducted according to conventional methods. For example, when R^{Y} is a tert-butoxycarbonyl group, a method comprising reaction with hydrogen chloride in a solvent such as dioxane and the like can be mentioned. The reaction temperature is preferably 0°C - room temperature.

### Step 13

Compound [I]-7 can be obtained by reacting compound [16] with compound [17] in a solvent in the presence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. The reaction can be carried out under cooling to heating.

### Production Method 7

In this Production Method, compound [I] wherein m is 1 or 2 and X is -SO₂-N(R⁵)- is produced. 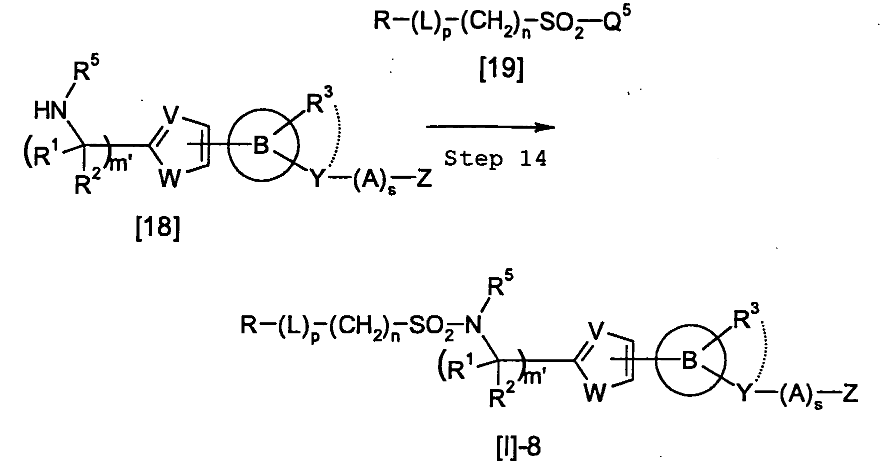 wherein Q⁵ is a halogen atom (bromine atom, iodine atom, fluorine atom, chlorine atom etc.) and other symbols are as defined above.

### Step 14

Compound [I]-8 can be obtained by reacting compound [18] obtained by a method similar to that to give compound [16] in Production Method 6 with compound [19] in a solvent in the presence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.) and the like, and a mixed solvent thereof can be mentioned. As the base, inorganic bases such as potassium carbonate, sodium carbonate and the like and organic bases such as triethylamine, diisopropylethylamine and the like can be mentioned. The reaction can be carried out at room temperature to under heating.

### Production Method 8

In this Production Method, compound [I] wherein m is 1 or 2 and X is -CO-N(R⁷)- is produced. 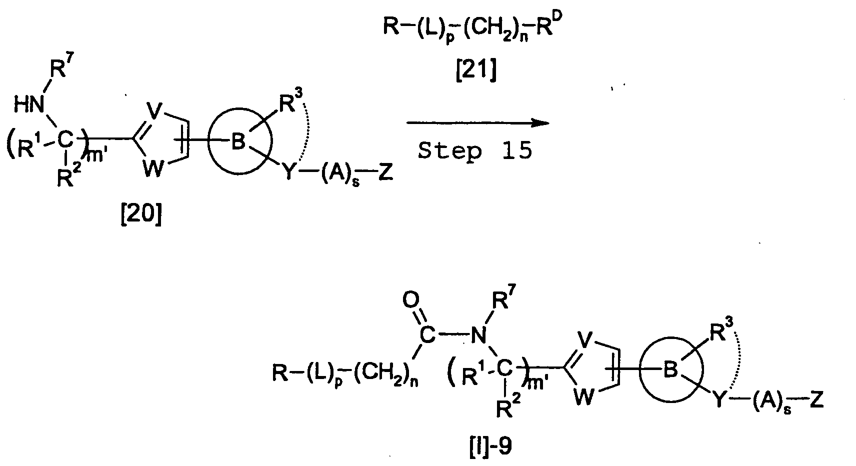 wherein R^{D} is a carboxy group or a formyl group substituted by a halogen atom (bromine atom, iodine atom, fluorine atom, chlorine atom etc.), and other symbols are as defined above.

### Step 15

Compound [I]-9 can be obtained by amide condensation of compound [20] obtained by a method similar to that to give compound [16] in Production Method 6 with compound [21]. The amide condensation can be performed according to conventional methods. For example, in a case of R^{D} being a carboxy group, a method comprising condensation in a solvent such as N,N-dimethylformamide, acetonitrile, tetrahydrofuran, chloroform, ethyl acetate, methylene chloride, toluene and the like, in the presence of a condensing agent such as dicyclohexylcarbodiimide, 1-ethyl-3-(3-dimethylaminopropyl)carbodiimide hydrochloride, diphenylphosphoryl azide and the like, and in the presence or absence of N-hydroxysuccinimide, 1-hydroxybenzotriazole and the like can be mentioned. The reaction can be carried out under cooling to heating. In addition, in a case of R^{D} being a formyl group substituted by a halogen atom, a method comprising condensation in a solvent such as N,N-dimethylformamide, acetonitrile, tetrahydrofuran, chloroform, ethyl acetate, methylene chloride, toluene, water and the like and a mixed solvent thereof, and in the presence of a base such as inorganic bases (sodium hydrogen carbonate, potassium carbonate, potassium hydrogen carbonate, sodium hydroxide, etc.) and organic bases (triethylamine, diisopropylethylamine, pyridine, etc.), can be mentioned as an example. The reaction can be carried out under cooling to heating.

### Production Method 9

In this Production Method, compound [I] wherein m is 1 or 2 and X is -NH-CO-N(R⁵)- is produced. 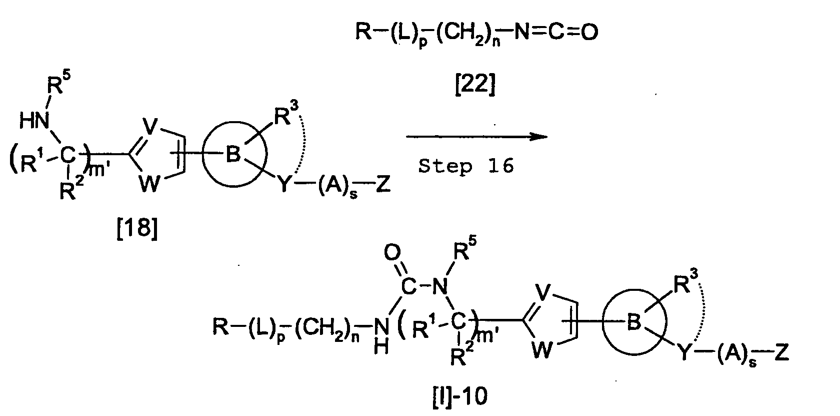 wherein each symbol is as defined above.

### Step 16

Compound [I]-10 can be obtained by reacting compound [18] with compound [22] in a solvent in the presence or absence of a base. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium'carbonate and the like and organic bases such as triethylamine, diisopropylethylamine and the like can be mentioned. The reaction can be carried out under cooling to heating.

### Production Method 10

In this Production Method, of compounds [I], compounds [1] used in Production Methods 1, 3 and 4, compounds [2] used in Production Method 1, compounds [3] used in Production Method 1 and compounds [15] used in Production Method 6, a compound
wherein Y is -CH₂-N(R¹²)-, -CH₂-O- or -CH₂-S- is produced. 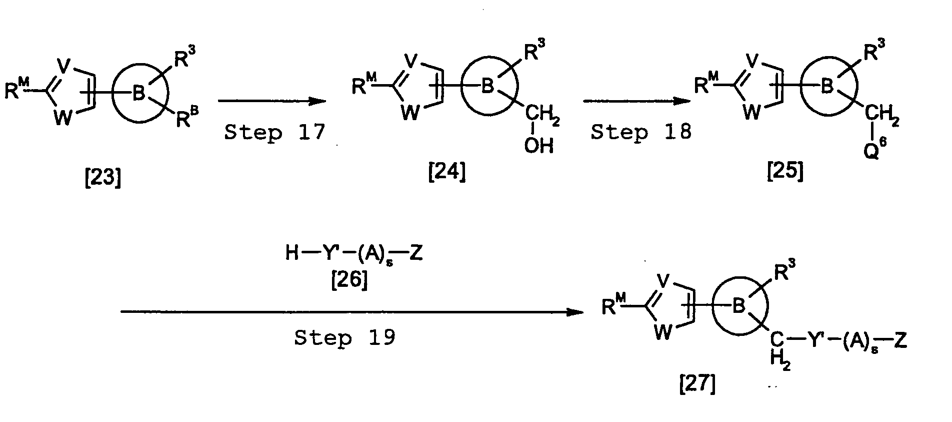 wherein R^{M} is 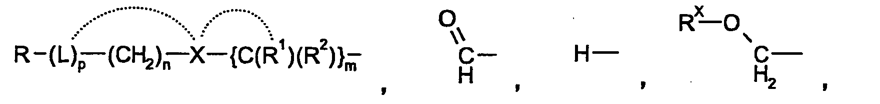 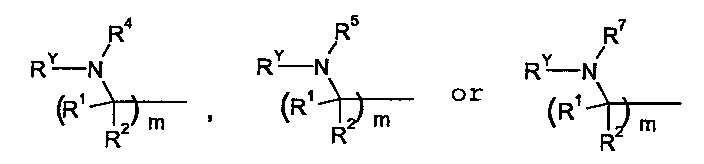 wherein each symbol is as defined above, R^{B} is a carboxy group, a carboxy group substituted by an alkyl group (methyl group, ethyl group etc.) or a formyl group, Q⁶ is a conventional leaving group as described in Production Method 1, Y' is -(R¹²)-, -O- or -S-wherein each symbol is as defined above, and other symbols are as defined above.

### Step 17

Compound [24] can be obtained by activation of compound [23] in a solvent using an activating agent where necessary, and reduction thereof using a conventional reducing agent. As the solvent, alcohols (methanol, ethanol etc.), ether solvents (tetrahydrofuran, dioxane etc.), toluene and the like, and a mixed solvent thereof can be mentioned. As the reducing agent, sodium borohydride, lithium borohydride, diborane, lithium aluminum hydride and the like can be mentioned. As the activating agent, for example, when R^{B} is a carboxy group, carbonyldiimidazole and the like can be mentioned. The reaction can be carried out under cooling to heating.

### Step 18

Compound [25] can be obtained by substituting a hydroxyl group of compound [24] with a conventional leaving group according to conventional methods. For example, conversion to a mesyloxy group, a tosyloxy group and the like can be performed using such sulfonyl chloride in a solvent in the presence of an organic base. As the solvent, toluene, ethyl acetate, halogenated solvents (chloroform, dichloromethane etc.), ether solvents (tetrahydrofuran, dioxane etc.), acetonitrile, N,N-dimethylformamide and a mixed solvent thereof can be mentioned, and as the organic base, pyridine, triethylamine, diisopropylethylamine and the like can be mentioned. The reaction can be carried out under cooling to heating. In addition, conversion to a chlorine atom can be performed using thionyl chloride without solvent or in a solvent. As the solvent, toluene, ethyl acetate, halogenated solvents (chloroform, dichloromethane etc.), ether solvents (tetrahydrofuran, dioxane etc.) and a mixed solvent thereof can be mentioned. As a catalyst, N,N-dimethylformamide may be added. The reaction can be carried out under cooling to heating. In addition, conversion to a chlorine atom can be also performed by converting to a mesyloxy group, a tosyloxy group and the like, and further reacting with an alkali metal chloride (lithium chloride etc.) or quaternary ammonium chloride. As the solvent, acetonitrile, N,N-dimethylformamide and the like can be mentioned. The reaction can be carried out under cooling to heating.

### Step 19

Compound [27] can be obtained by reacting compound [25] with compound [26] in a solvent in the presence of a base and in the presence or absence of a catalyst. As the solvent, N,N-dimethylacetamide, N,N-dimethylformamide, acetonitrile, ethyl acetate, toluene, alcohol solvents (methanol, ethanol etc.), ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine, potassium tert-butoxide and the like can be mentioned. As the catalyst, potassium iodide, quaternary ammonium salts (tetrabutylammonium iodide, tetrabutylammonium bromide etc.) and the like can be mentioned. The reaction can be carried out at room temperature to under heating.

### Production Method 11

In this Production Method, of compounds [I], compounds [1] used in Production Methods 1, 3 and 4, compounds [2] used in Production Method 1, compounds [3] used in Production Method 1 and compounds [15] used in Production Method 6, a compound
wherein Y is -CO-N(R¹²)- is produced. 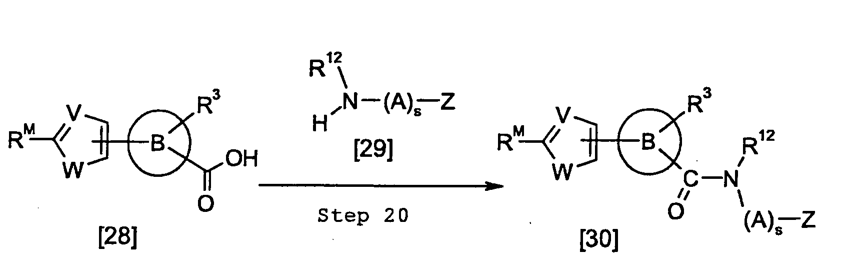 wherein each symbol is as defined above.

### Step 20

Compound [30] can be obtained by amide condensation of compound [28] with compound [29]. Amide condensation can be performed according to conventional methods. For example, a method comprising condensation in a solvent such as N,N-dimethylformamide, acetonitrile, tetrahydrofuran, chloroform, ethyl acetate, methylene chloride, toluene and the like, in the presence of a condensing agent such as dicyclohexylcarbodiimide, 1-ethyl-3-(3-dimethylaminopropyl)carbodiimide hydrochloride, diphenylphosphoryl azide and the like, and in the presence or absence of N-hydroxysuccinimide, 1-hydroxybenzotriazole and the like can be mentioned. The reaction temperature is preferably 0°C - 100°C

### Production Method 12

In this Production Method, of compounds [I], compounds [1] used in Production Methods 1, 3 and 4, compounds [2] used in Production Method 1, compounds [3] used in Production Method 1 and compounds [15] used in Production Method 6, a compound
wherein Y is -N(R¹¹)-, -O- or -S- is produced. 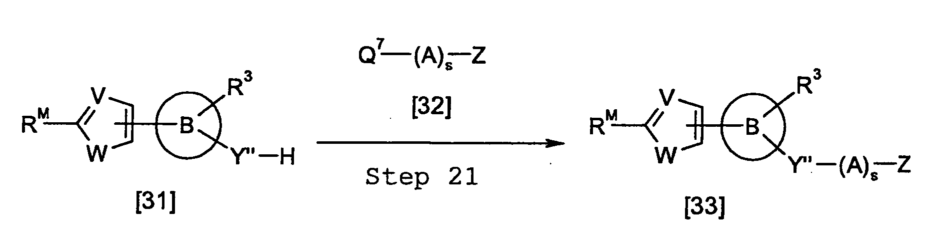 wherein Q⁷ is a general leaving group as described in Production Method 1, Y" is -N(R¹¹)-, -O- or -S- and other symbols are as defined above.

### Step 21

Compound [33] can be obtained by reacting compound [31] with compound [32] in a solvent in the presence of a base and in the presence or absence of a catalyst. As the solvent, N,N-dimethylacetamide, N,N-dimethylformamide, acetonitrile, ethyl acetate, toluene, alcohol solvents (methanol, ethanol etc.), ether solvents (tetrahydrofuran, dioxane etc.), halogenated solvents (chloroform, dichloromethane etc.) and the like and a mixed solvent thereof can be mentioned. As the base, inorganic bases such as potassium carbonate, sodium carbonate and the like and organic bases such as triethylamine, diisopropylethylamine and the like can be mentioned. As the catalyst, potassium iodide, quaternary ammonium salts (tetrabutylammonium iodide, tetrabutylammonium bromide etc.) and the like can be mentioned. The reaction can be carried out at room temperature to under heating.

### Production Method 13

In this Production Method, of compounds [I], compounds [1] used in Production Methods 1, 3 and 4, compounds [2] used in Production Method 1, compounds [3] used in Production Method 1 and compounds [15] used in Production Method 6, a compound
wherein Y is -N(R¹¹)-, s is 1 and A is a methylene group is produced. 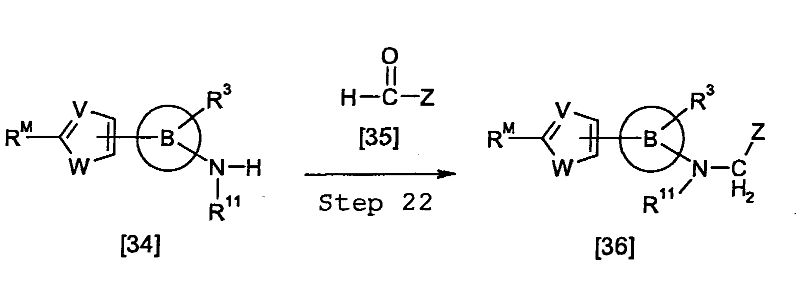 wherein each symbol is as defined above.

### Step 22

Compound [36] can be obtained by reacting compound [34] with compound [35] in a solvent in the presence of a reducing agent. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane etc.), acetic acid and the like and a mixed solvent thereof can be mentioned. As the reducing agent, sodium triacetoxyborohydride, sodium cyanoborohydride and the like can be mentioned. The reaction temperature is preferably 0°C - 80°C.

### Production Method 14

In this Production Method, of compounds [I], compounds [1] used in Production Methods 1, 3 and 4, compounds [2] used in Production Method 1, compounds [3] used in Production Method 1 and compounds [15] used in Production Method 6, a compound
wherein Y is -N(R¹²)-CO- is produced. 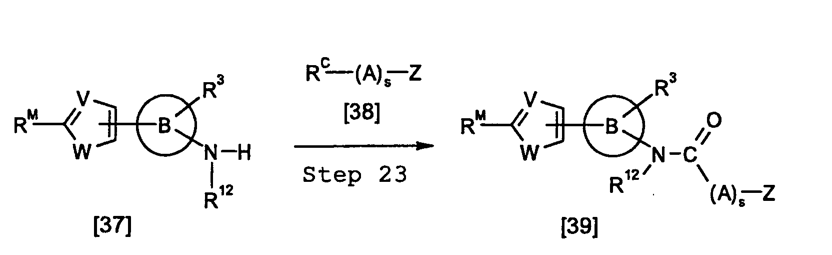 wherein R^{C} is a carboxy group or a formyl group substituted by a halogen atom (bromine atom, iodine atom, fluorine atom, chlorine atom etc.), and other symbols are as defined above.

### Step 23

Compound [39] can be obtained by amide condensation of compound [37] with compound [38]. The amide condensation can be performed according to conventional methods. For example, in a case of R^{C} being a carboxy group, a method comprising condensation in a solvent such as N,N-dimethylformamide, acetonitrile, tetrahydrofuran, chloroform, ethyl acetate, methylene chloride, toluene and the like, in the presence of a condensing agent such as dicyclohexylcarbodiimide, 1-ethyl-3-(3-dimethylaminopropyl)carbodiimide hydrochloride, diphenylphosphoryl azide and the like, and in the presence or absence of N-hydroxysuccinimide, 1-hydroxybenzotriazole and the like can be mentioned. In addition, in a case of R^{C} being a formyl group substituted by a halogen atom, a method comprising condensation in a solvent such as N,N-dimethylformamide, acetonitrile, tetrahydrofuran, chloroform, ethyl acetate, methylene chloride, toluene, water and the like and a mixed solvent thereof, and in the presence of a base such as inorganic bases (sodium hydrogen carbonate, potassium carbonate, potassium hydrogen carbonate, sodium hydroxide, etc.) and organic bases (triethylamine, diisopropylethylamine, pyridine, etc.), can be mentioned as an example. The reaction can be carried out under cooling to heating.

### Production Method 15

In this Production Method, of compound [I], compound [1] used in Production Methods 1, 3 and 4, compound [2] used in Production Method 1, compound [3] used in Production Method 1 and compound [15] used in Production Method 6, a compound
wherein Y is -N(R¹¹)-, s is 1, and A is a methylene group is produced. 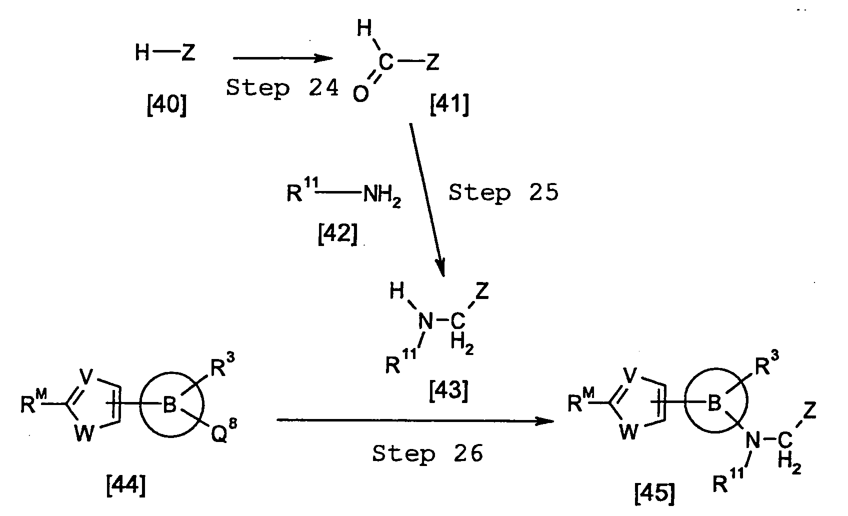 wherein Q⁸ is a general leaving group as described in Production Method 1 and other symbols are as defined above.

### Step 24

Compound [41] can be obtained by introducing a formyl group into compound [40] according to conventional methods. For example, when Z is an aryl group or a heteroaromatic ring group (said aryl group or heteroaromatic ring group is optionally substituted as described above), compound [41] can be synthesized by reacting compound [40] with dichloromethyl methyl ether in a solvent in the presence of a Lewis acid and hydrolyzing the obtained methoxychloromethyl compound. As the solvent, halogenated solvents (chloroform, dichloromethane etc.) and the like can be mentioned. As the Lewis acid, aluminum chloride, titanium tetrachloride, tin tetrachloride and the like can be mentioned. The reaction can be carried out under cooling to room temperature.

### Step 25

Compound [43] can be obtained by reacting compound [41] with compound [42] in a solvent in the presence of a reducing agent. As the solvent, N,N-dimethylacetamide, N,N-dimethylformamide, acetonitrile, ethyl acetate, toluene, ether solvents (tetrahydrofuran, dioxane etc.), halogenated solvents (chloroform, dichloromethane, 1,2-dichloroethane etc.), alcohol solvents (methanol, ethanol etc.), acetic acid and the like and a mixed solvent thereof can be mentioned. As the reducing agent, sodium borohydride, sodium triacetoxyborohydride, sodium cyanoborohydride and the like can be mentioned. The reaction temperature is preferably 0°C - 80°C.

### Step 26

Compound [45] can be obtained by reacting compound [43] with compound [44] in a solvent in the presence of a base and in the presence of a catalyst. As the solvent, toluene, xylene, ether solvents (tetrahydrofuran, dioxane, 1,2-dimethoxyethane etc.) and the like and a mixed solvent thereof can be mentioned. As the base, cesium carbonate, sodium tert-butoxide, tripotassium phosphate and the like can be mentioned. As the catalyst, a palladium complex (e.g., a complex formed using palladium acetate and (R)-(+)-2,2'-bis(diphenylphosphino)-1,1'-binaphthyl) and the like can be mentioned. The reaction can be carried out at room temperature to under heating.

### Production Method 16

In this Production Method, of compound [I], compound [3] used in Production Method 1, compound [15] used in Production Method 6, compound [23] used in Production Method 10, compound [28] used in Production Method 11, compound [31] used in Production Method 12, compound [34] used in Production Method 13, compound [37] used in Production Method 14, and compound [44] used in Production Method 15, a compound wherein V is =N-, W is -S- and the position of substitution of B on the thiazole ring formed by the above-mentioned V and W is the 4-position is produced. 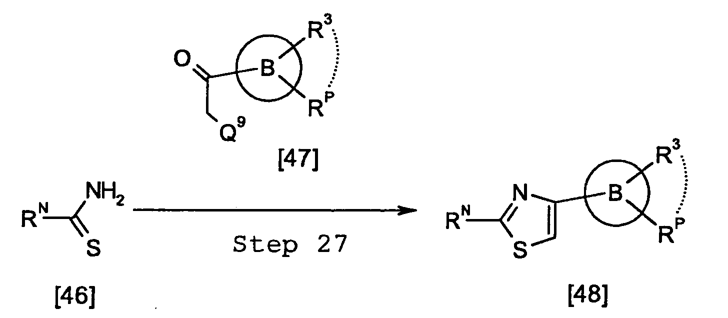 wherein R^{N} is 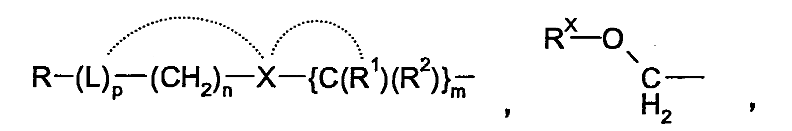 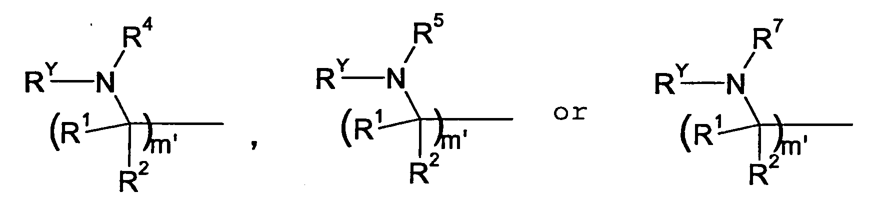 wherein each symbol is as defined above, R^{P} is 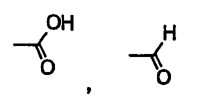 substituted by a halogen atom (bromine atom, iodine atom, fluorine atom, chlorine atom etc.), 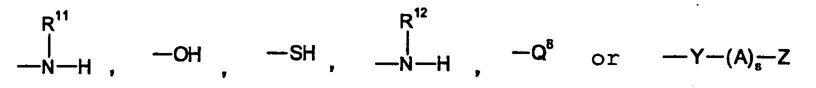 wherein each symbol is as defined above, Q⁹ is a general leaving group as described in Production Method 1 and other symbols are as defined above.

### Step 27

Compound [48] can be obtained by reacting compound [46] with compound [47] in a solvent in the presence or absence of a base. As the solvent, N,N-dimethylacetamide, N,N-dimethylformamide, acetonitrile, ethyl acetate, alcohol solvents (methanol, ethanol etc.), ether solvents (tetrahydrofuran, dioxane etc.), halogenated solvents (chloroform, dichloromethane etc.) and the like and a mixed solvent thereof can be mentioned. As the base, inorganic bases such as sodium hydrogen carbonate and the like and organic bases such as diisopropylethylamine and the like can be mentioned. The reaction can be carried out under cooling to heating.

### Production Method 17

In this Production Method, of compounds [I], compounds [3] used in Production Method 1, compounds [15] used in Production Method 6, compounds [23] used in Production Method 10, compounds [28] used in Production Method 11, compounds [31] used in Production Method 12, compounds [34] used in Production Method 13, compounds [37] used in Production Method 14 and compounds [44] used in Production Method 15, a compound wherein V is =N-, W is -O- and the position of substitution of B on the oxazole ring formed by the above-mentioned V and W is the 4-position is produced. 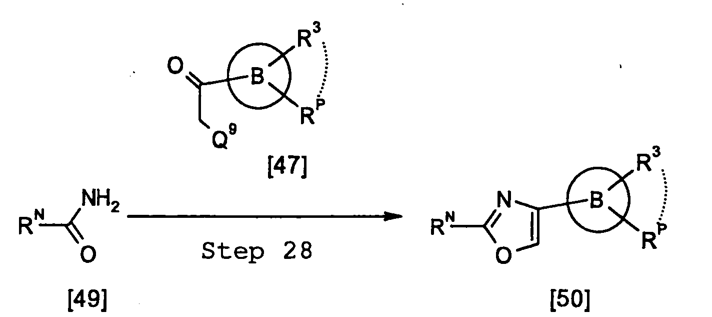 wherein each symbol is as defined above.

### Step 28

Compound [50] can be obtained by reacting compound [49] with compound [47] without solvent or in a solvent. As the solvent, acetonitrile, alcohols (methanol, ethanol, isopropyl alcohol etc.), xylene, toluene and the like and a mixed solvent thereof can be mentioned. The reaction can be carried out under heating.

### Production Method 18

In this Production Method, of compounds [I], compounds [1] used in Production Method 1, 3 and 4, compounds [15] used in Production Method 6, compounds [23] used in Production Method 10, compounds [28] used in Production Method 11, compounds [31] used in Production Method 12, compounds [34] used in Production Method 13, compounds [37] used in Production Method 14 and compounds [44] used in Production Method 15, compound [I] wherein V is =CH- and W is -S- is produced. 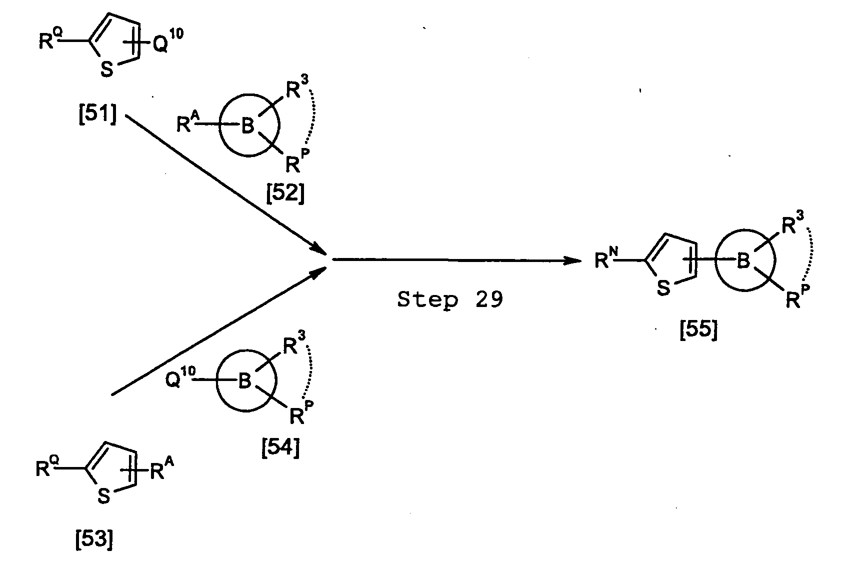 wherein R^{Q} is 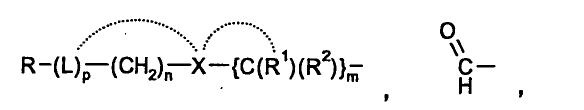 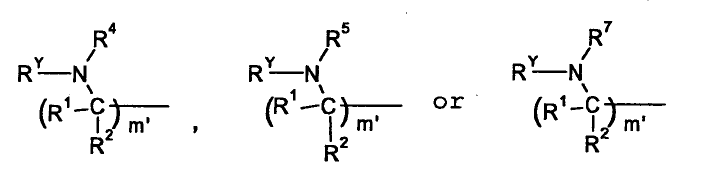 wherein each symbol is as defined above, Q¹⁰ is a general leaving group as described in Production Method 1, R^{A} is a boronic acid group (-B(OH)₂), and other symbols are as defined above.

### Step 29

Compound [55] can be obtained by reacting compound [51] with compound [52] or by reacting compound [53] with compound [54] in a solvent in the presence of a base and in the presence of a catalyst. As the solvent, N,N-dimethylformamide, acetonitrile, alcohol solvents (methanol, ethanol etc.), ether solvents (tetrahydrofuran, 1,2-dimethoxyethane etc.), toluene, water and the like and a mixed solvent thereof can be mentioned. As the base, inorganic bases such as potassium carbonate, potassium hydrogen carbonate, sodium hydrogen carbonate, potassium phosphate and the like and organic bases such as triethylamine and the like can be mentioned. As the catalyst, palladium catalysts (tetrakis(triphenylphosphine)palladium, bis(triphenylphosphine)palladium(II) dichloride, palladium acetate-triphenylphosphine etc.) and the like can be mentioned. The reaction can be carried out at room temperature to under heating.

### Production Method 19

In this Production Method, compound [I] wherein R is -COOH, -A¹-COOH or -O-A¹-COOH is produced. 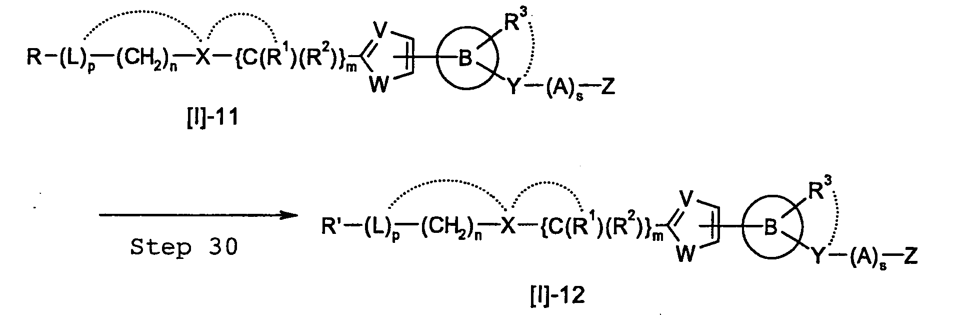 wherein R' is -COOH, -A¹-COOH or -O-A¹-COOH and other symbols are as defined above.

### Step 30

When R¹⁹ for R in compound [I]-11 is a C₁₋₄ alkyl group, compound [I]-12 can be obtained by deprotection according to conventional methods to give carboxylic acid. For example, a method comprising hydrolysis in a solvent in the presence of a base can be mentioned. As the solvent, alcohols (methanol, ethanol, isopropyl alcohol etc.), tetrahydrofuran, 1,2-dimethoxyethane, N,N-dimethylformamide, dimethyl sulfoxide, water and the like and a mixed solvent thereof can be mentioned. As the base, alkali metal hydroxides such as sodium hydroxide and the like, potassium carbonate, sodium carbonate and the like can be mentioned. The reaction can be carried out under cooling to heating.

Moreover, by stirring compound [I] obtained in the above-mentioned Production Methods in a solvent upon addition of an acid or a base, a pharmaceutically acceptable salt of compound [I] can be obtained. As the solvent, 2-butanone, acetone, tetrahydrofuran, ethyl acetate, methanol, ethanol, water, hexane, and a mixed solvent thereof can be mentioned. As the acid, inorganic acids such as hydrochloric acid, sulfuric acid, phosphoric acid and the like and organic acids such as oxalic acid, malonic acid, trifluoroacetic acid, methanesulfonic acid, p-toluenesulfonic acid, benzenesulfonic acid and the like can be mentioned. As the base, inorganic bases such as sodium hydroxide, potassium hydroxide, calcium hydroxide and the like and organic bases such as triethylamine, ethylenediamine, tris(hydroxymethyl)aminomethane, N-methyl-D-glucamine and the like can be mentioned. The reaction can be carried out at room temperature.

Compound [I] obtained above or a pharmaceutically acceptable salt thereof is dissolved or suspended in a solvent at room temperature to under heating and stirred or stood at 0°C-150°C to allow precipitation of crystals, which are added with a poor solvent of the compound or a salt thereof where necessary under cooling to room temperature, and filtered to give crystals of compound [I] or a pharmaceutically acceptable salt thereof. As the solvent, 2-butanone, acetone, tetrahydrofuran, ethyl acetate, methanol, ethanol, water, hexane and a mixed solvent thereof can be mentioned.

The Production Methods described in the present specification are examples of the Production Methods of the compound of the present invention, and compounds other than those explained above can be also produced by combining conventional methods known in the field of organic synthetic chemistry.

### Examples

The compound represented by the formula [I] of the present invention and a production method thereof are explained in detail by referring to Examples, which are not to be construed as limitative.

### Example 1-1

### 5-{4-[4-({[4-(1-Ethylpropyl)phenyl]isopropylamino}methyl)phenyl]thiazol-2-ylmethoxy}nicotinic acid

(1) 4-(2-(Benzoyloxymethyl)thiazol-4-yl)benzoic acid 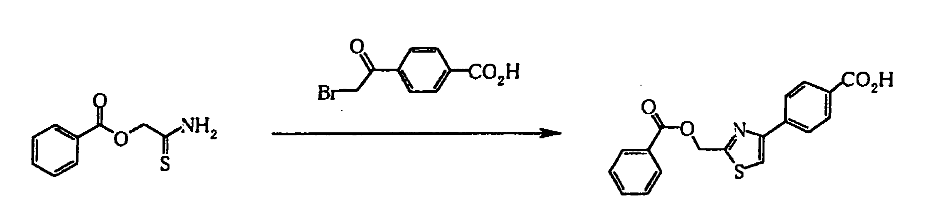
   To a solution of 4-(bromoacetyl)benzoic acid (10 g, 41.14 mmol) in N,N-dimethylformamide (40 ml) was added 2-(benzoyloxy)ethanethioamide (8.03 g, 41.14 mmol) under ice-cooling, and the mixture was stirred at room temperature for 1.5 hr. After the completion of the reaction, sodium hydrogen carbonate (3.46 g, 41.14 mmol) and water were added under ice-cooling and the mixture was stirred at room temperature for 0.5 hr. The precipitates were collected by filtration. The obtained solid was dried under reduced pressure to give the title compound (13.089 g, 93.3%).
(2) (4-(4-Hydroxymethylphenyl)thiazol-2-yl)methyl benzoate 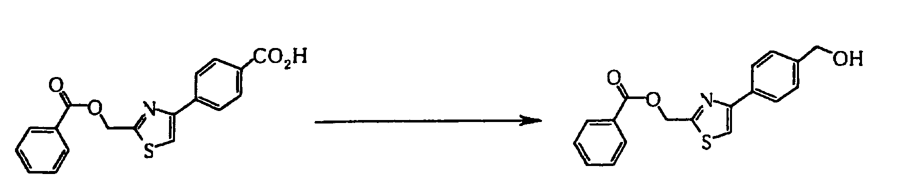
   To a suspension of 4-(2-(benzoyloxymethyl)thiazol-4-yl)benzoic acid (0.5 g, 1.47 mmol) obtained in Example 1-1(1) in tetrahydrofuran (5 ml) was added 1,1'-carbonyldiimidazole (0.358 g, 2.21 mmol) at room temperature and the mixture was stirred at 65°C for 2 hr. Then an aqueous solution of sodium borohydride (0.056 g, 1.47 mmol) was added dropwise at room temperature and the mixture was stirred at the same temperature for 20 min. After the completion of the reaction, ethyl acetate and an aqueous sodium hydrogen carbonate solution were added for extraction, and the organic layer was washed with water and saturated brine and dried over sodium sulfate. The solvent was evaporated and the residue was washed with a mixed solvent of hexane-ethyl acetate and collected by filtration. The obtained solid was dried under reduced pressure to give the title compound (370.7 mg, 77.5%).
(3) (4-(4-Chloromethylphenyl)thiazol-2-yl)methyl benzoate 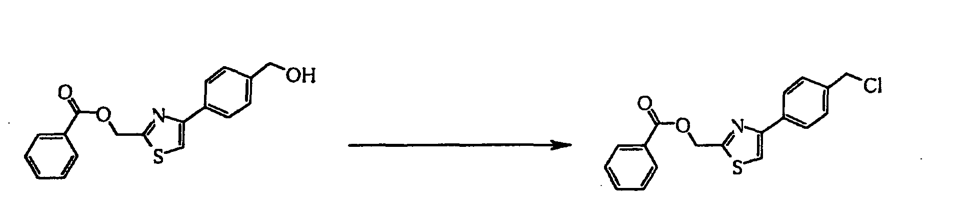
   To a solution of (4-(4-hydroxymethylphenyl)thiazol-2-yl)methyl benzoate (5.55 g, 17.06 mmol) obtained in Example 1-1(2) in chloroform (100 ml) were added thionyl chloride (1.49 ml, 20.67 mmol) and N,N-dimethylformamide (catalytic amount) under ice-cooling. The mixture was stirred at room temperature for 1 hr. After the completion of the reaction, the solvent was evaporated and the residue was concentrated by adding chloroform. Hexane was added and the precipitates were collected by filtration. The obtained solid was dried under reduced pressure to give the title compound (5.804 g, 98.9%).
(4)(4-(4-(N-Isopropyl-4-(1-ethylpropyl)phenylamino)methylphenyl)thiazol-2-yl)methanol 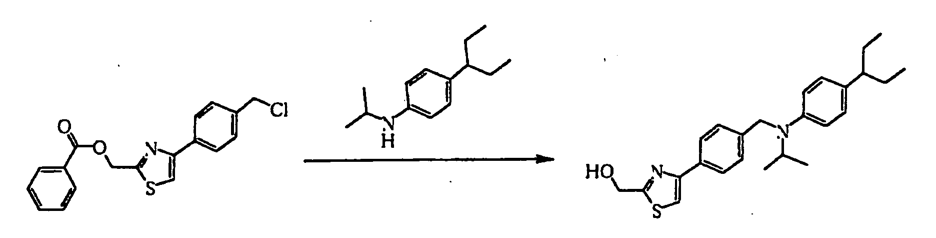
   To a solution of N-isopropyl-4-(1-ethylpropyl)aniline (7.34 g, 35.78 mmol) in N,N-dimethylacetamide (35 ml) were successively added (4-(4-chloromethylphenyl)thiazol-2-yl)methyl benzoate (11.18 g, 32.52 mmol) obtained in Example 1-1(3), potassium carbonate (4.95 g, 35.78 mmol) and potassium iodide (0.54 g, 3.25 mmol) and the mixture was stirred at 80°C for 3 hr. Then potassium carbonate (0.449 g, 3.252 mmol) was added and the mixture was stirred at 70°C for 12 hr. After the completion of the reaction, a mixture of 50% ethyl acetate-hexane and water were added for extraction, and the organic layer was washed successively with water and saturated brine and dried over sodium sulfate. The solvent was evaporated and tetrahydrofuran (60 ml), methanol (60 ml) and a 1N-aqueous sodium hydroxide solution (48.8 ml) were successively added to the obtained residue at room temperature. The mixture was stirred at 75°C for 1.5 hr. After the completion of the reaction, the solvent was evaporated and ethyl acetate and water were added for extraction, and the organic layer was washed successively with water and saturated brine and dried over sodium sulfate and the solvent was evaporated. The obtained residue was purified by silica gel column chromatography (hexane:ethyl acetate=4:1→2:1) to give the title compound (10.664 g, 80.3%).
(5) (4-(4-(N-Isopropyl-4-(1-ethylpropyl)phenylamino)methylphenyl)thiazol-2-yl)methyl chloride hydrochloride 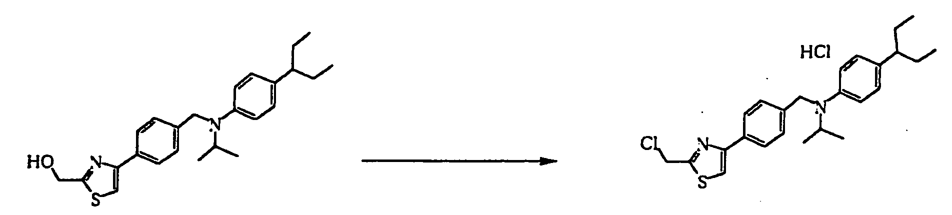
   Thionyl chloride (2.7 ml, 37.1 mmol) and N,N-dimethylformamide (catalytic amount) were added to chloroform (30 ml) at room temperature and a solution of (4-(4-(N-isopropyl-4-(1-ethylpropyl)phenylamino)methylphenyl)thiazol-2-yl)methanol (10.1 g, 24.7 mmol) obtained in Example 1-1(4) in chloroform (30 ml) was added dropwise under ice-cooling. The mixture was stirred at room temperature for 1 hr. After the completion of the reaction, the solvent was evaporated and the residue was concentrated by adding chloroform. Ethyl acetate was added and the mixture was stirred for 0.5 hr. The precipitates were collected by filtration and the obtained solid was dried under reduced pressure to give the title compound (10.23 g, 89.4%).
(6) 5-{4-[4-({[4-(1-Ethyl-propyl)phenyl]isopropyl-amino}methyl)phenyl]thiazol-2-ylmethoxy}nicotinic acid methyl ester 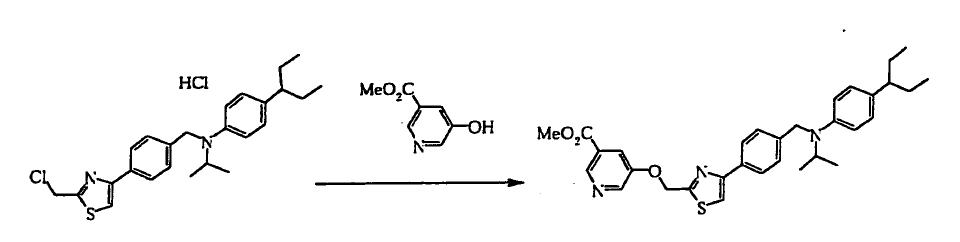
   To a solution of (4-(4-(N-isopropyl-4-(1-ethylpropyl)phenylamino)methylphenyl)thiazol-2-yl)methyl chloride hydrochloride (9.92 g, 21.4 mmol) obtained in Example 1-1(5) and methyl 5-hydroxynicotinate (3.61 g, 23.5 mmol) in N,N-dimethylformamide were added potassium carbonate (6.8 g, 49.2 mmol) and potassium iodide (0.355 g, 2.14 mmol) under ice-cooling, and the mixture was stirred at 60°C for 12 hr. After the completion of the reaction, ethyl acetate and water were added for extraction and the organic layer was washed successively with water and saturated brine and dried over sodium sulfate and the solvent was evaporated. The obtained residue was purified by silica gel column chromatography (hexane:ethyl acetate=8:1) and then (chloroform:ethyl acetate=20:1) to give the title compound (5.32 g, 45.7%).
(7) 5-{4-[4-({[4-(1-Ethylpropyl)phenyl]isopropylamino}methyl)phenyl]thiazol-2-ylmethoxy}nicotinic acid

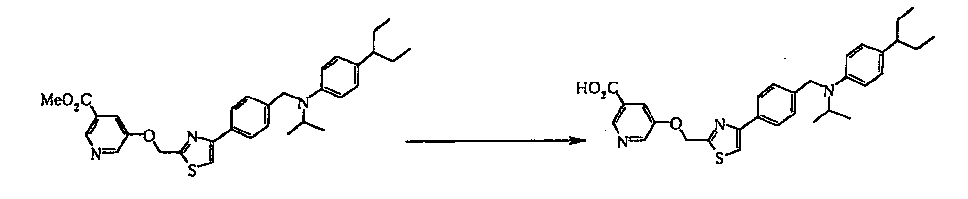

To a solution of 5-{4-[4-({[4-(1-ethylpropyl)-phenyl]isopropylamino}methyl)phenyl]thiazol-2-ylmethoxy}nicotinic acid methyl ester (5.32 g, 9.78 mmol) obtained in Example 1-1(6) in 50% tetrahydrofuran-methanol (32 ml) was added a 2N-aqueous sodium hydroxide solution (9.78 ml) at room temperature and the mixture was stirred at 75°C for 2 hr. After the completion of the reaction, a 2N-hydrochloric acid (9.78 ml) was added under ice-cooling and the precipitates were collected by filtration. The obtained solid was dried under reduced pressure to give the title compound (5.00 g, 96.5%).
¹H NMR (DMSO-d₆, 300 MHz) δ 0.69(t, J=7.3Hz, 6H), 1.16(d, J=6.5Hz, 6H), 1.48-1.31(m, 2H), 1.64-1.48(m, 2H), 2.19-2.06(m, 1H), 4.23(quint, J=6.6Hz, 1H), 5.67(s, 2H), 6.62(d, J=8.7Hz, 2H), 6.89(d, J=8.6Hz, 2H), 7.36(d, J=8.2Hz, 2H), 7.90(d, J=8.2Hz, 2H), 7.99(dd, J=3.0, 1.5Hz, 1H), 8.11(s, 1H), 8.64(d, J=2.9Hz, 1H), 8.72(d, J=1.5Hz, 1H), 13.43(br s, 1H)
melting point: 201.5°C

### Examples 1-2 to 1-186

In the same manner as in Example 1-1 and using other conventional methods where necessary, the compounds of Examples 1-2 to 1-186 were produced. The structural formulas and property values of the obtained compounds, as well as those of Example 1-1, are shown in the following Tables.

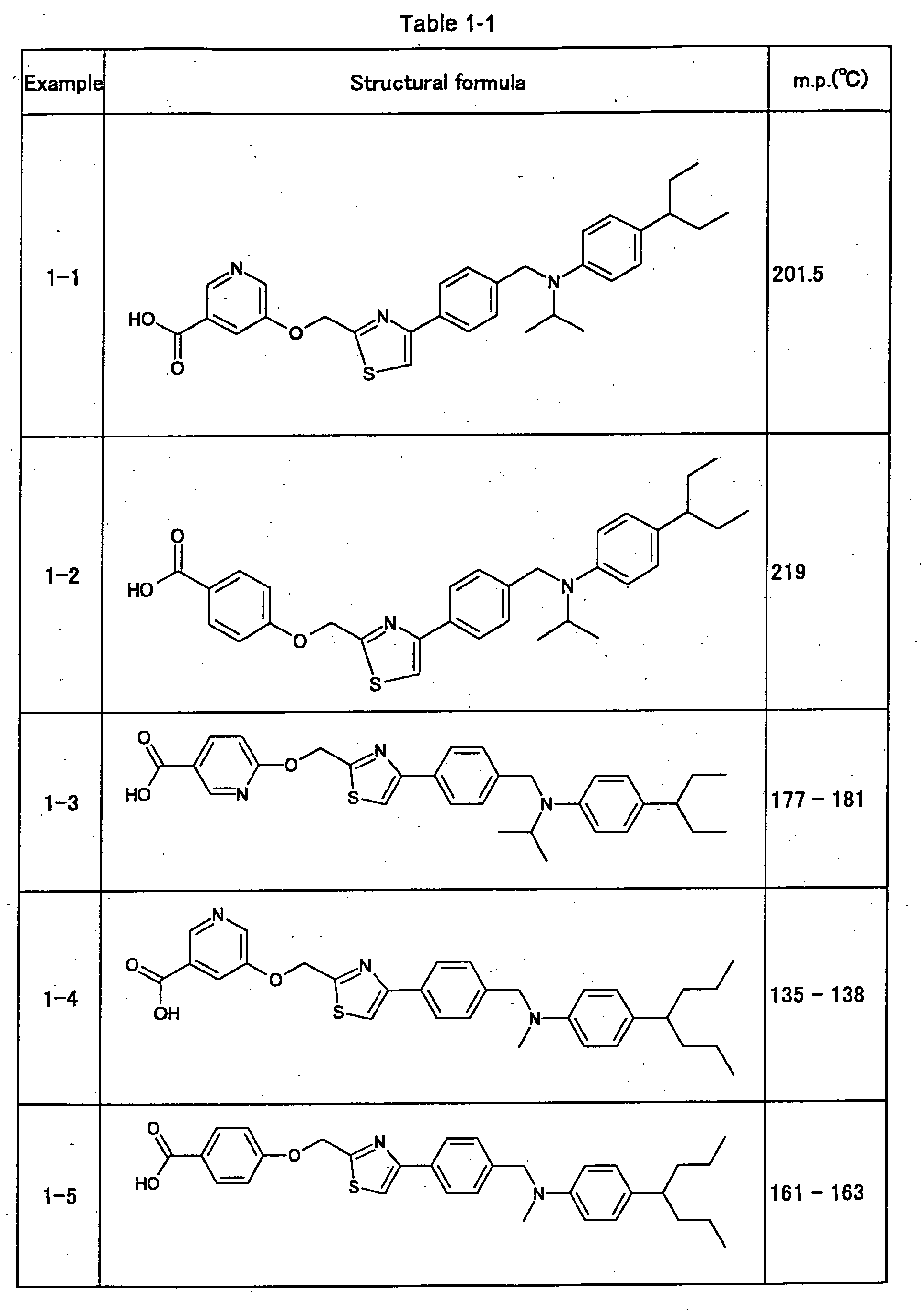

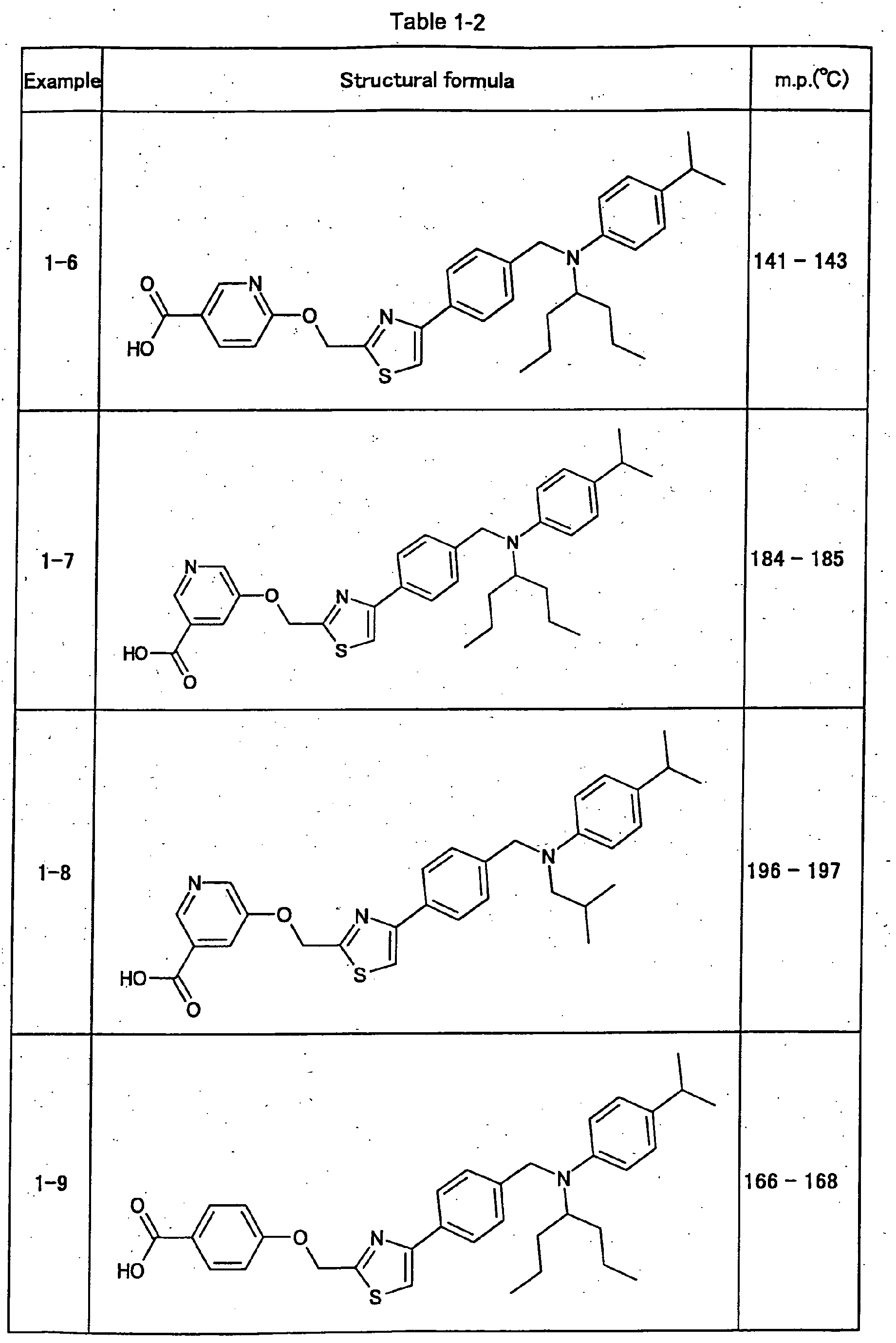

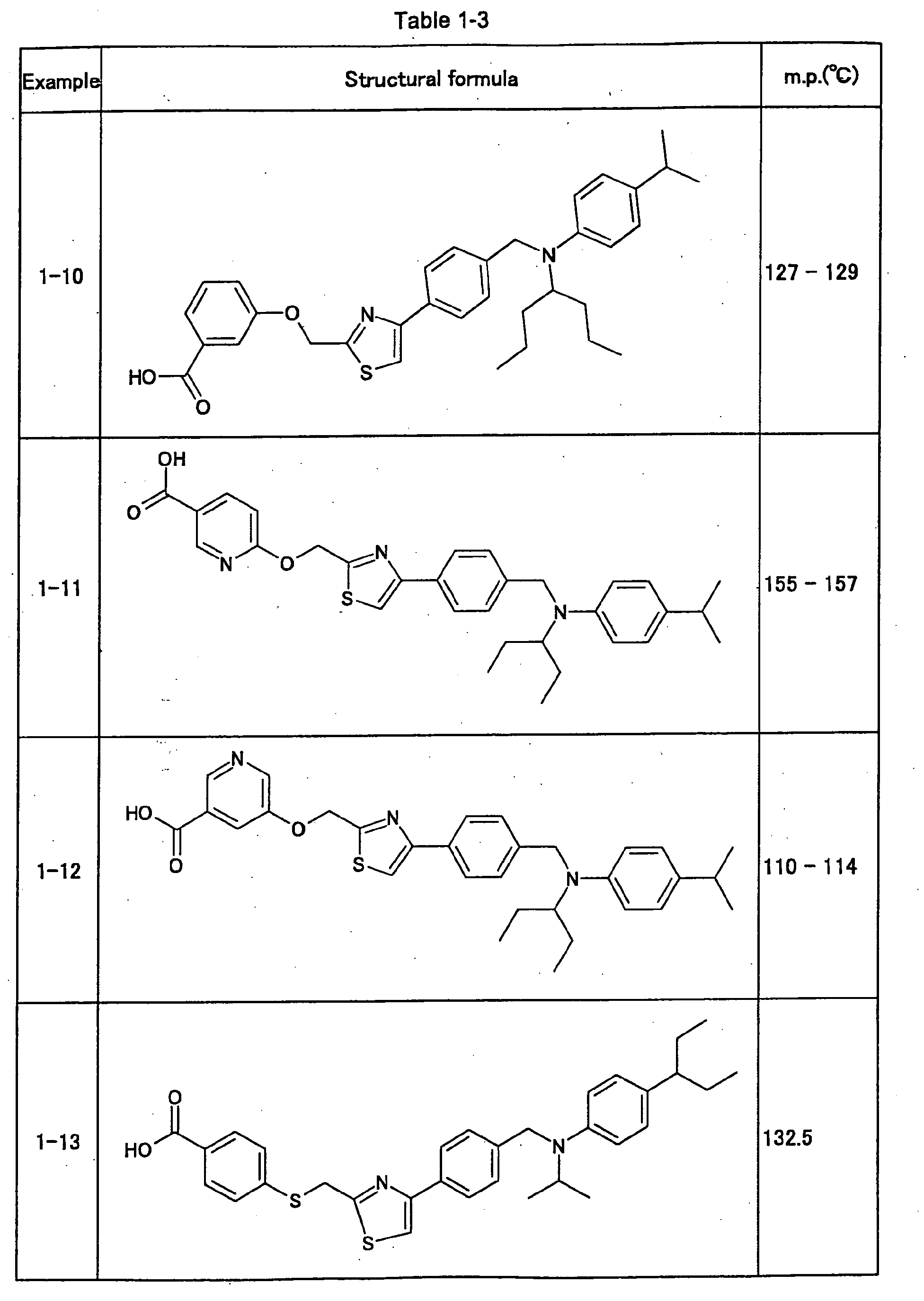

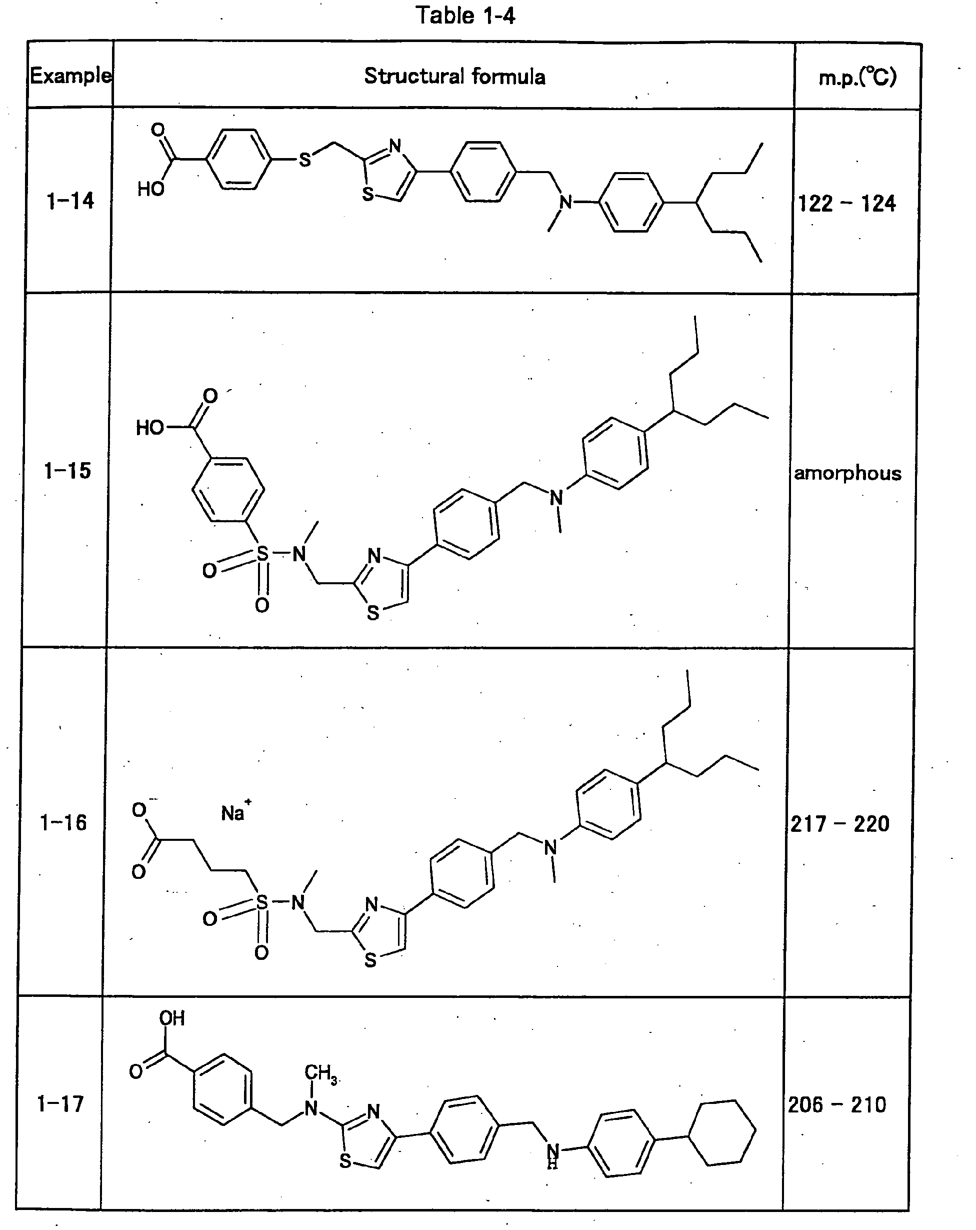

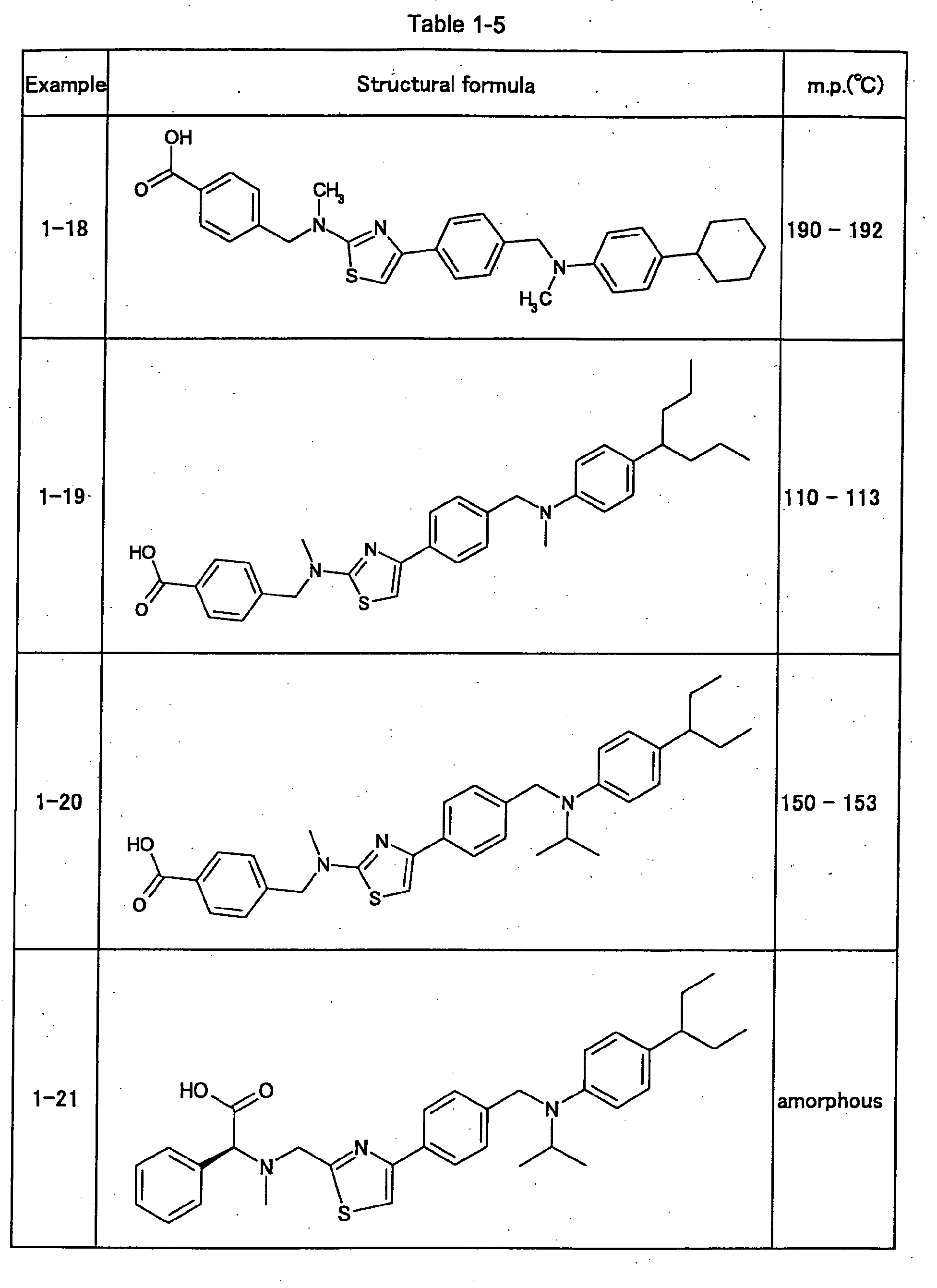

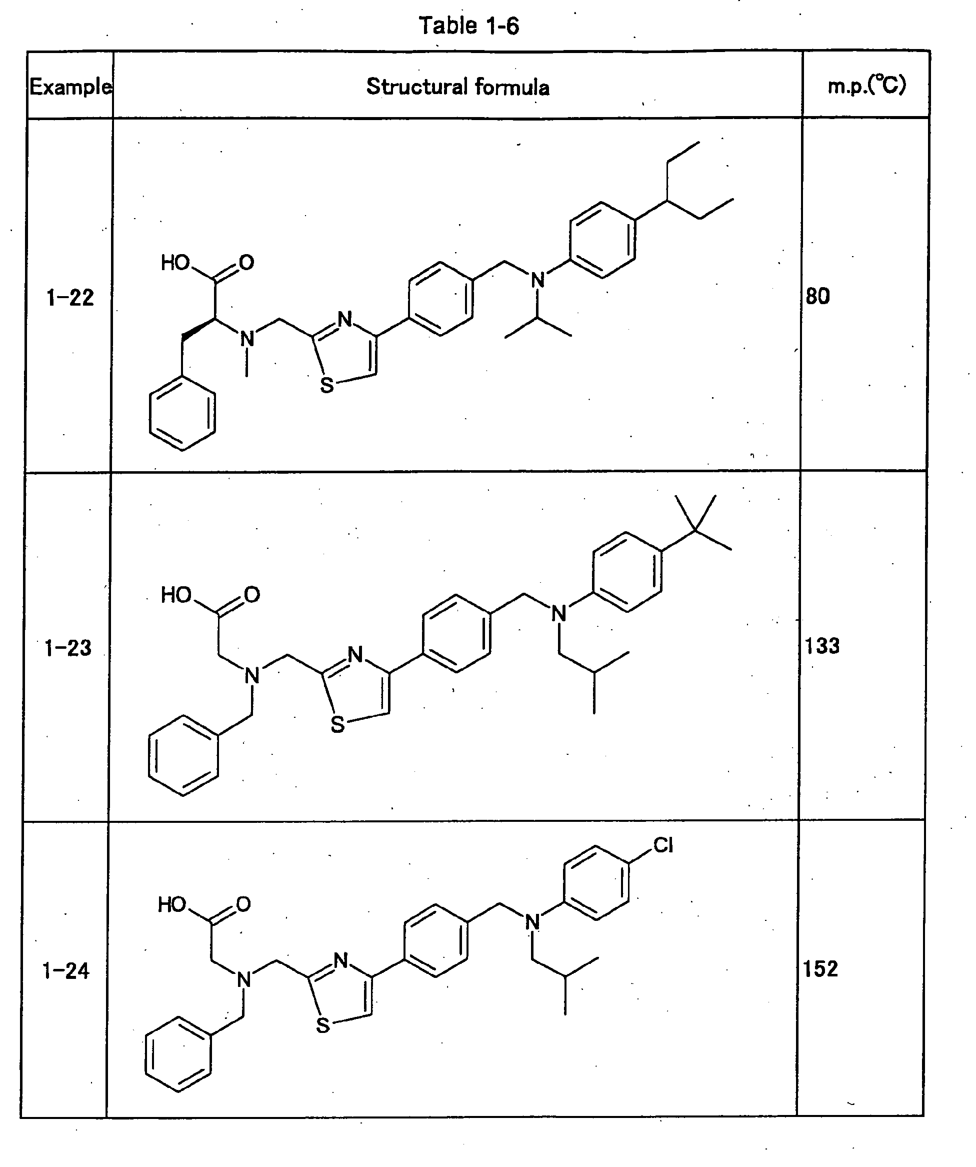

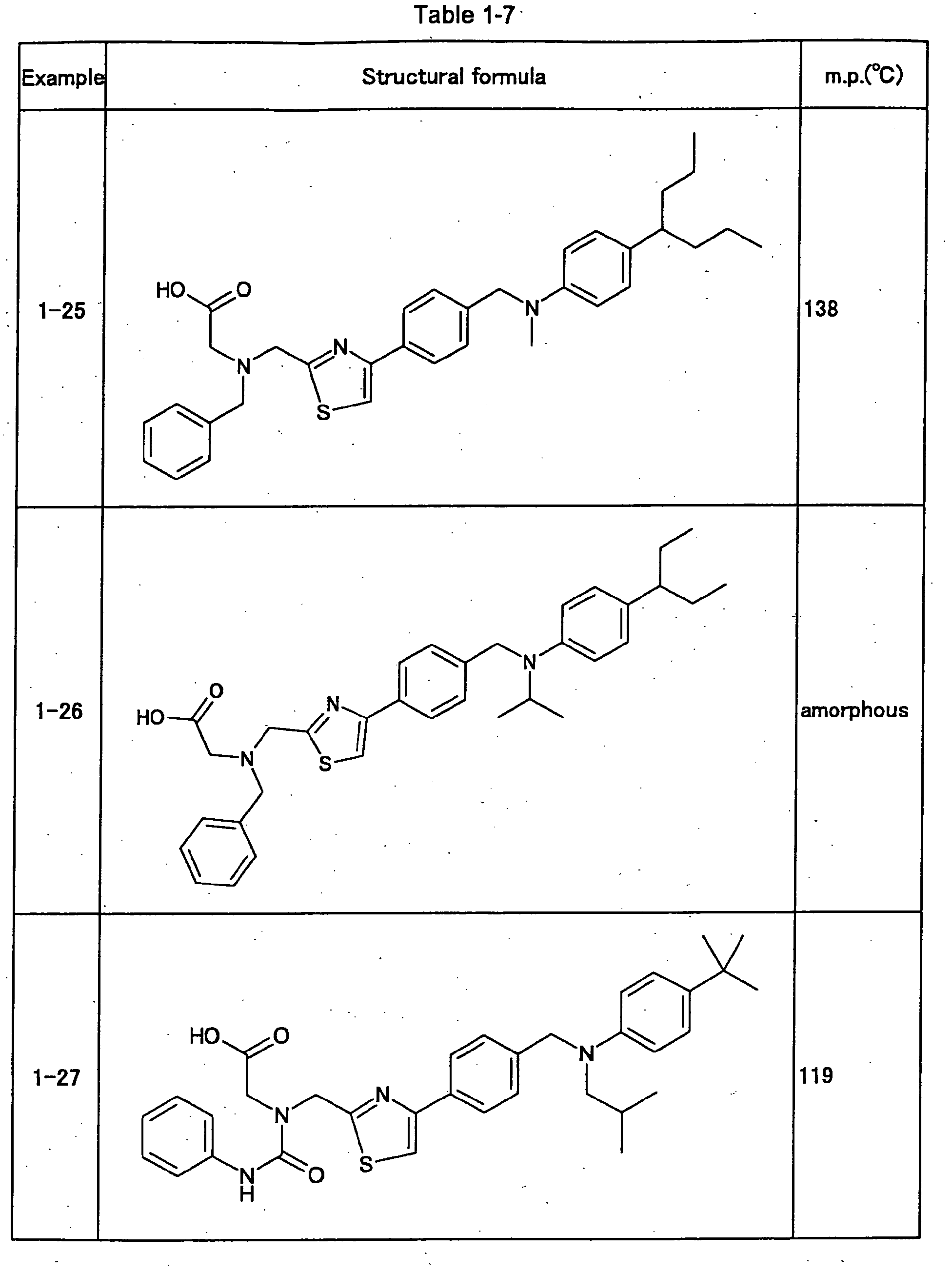

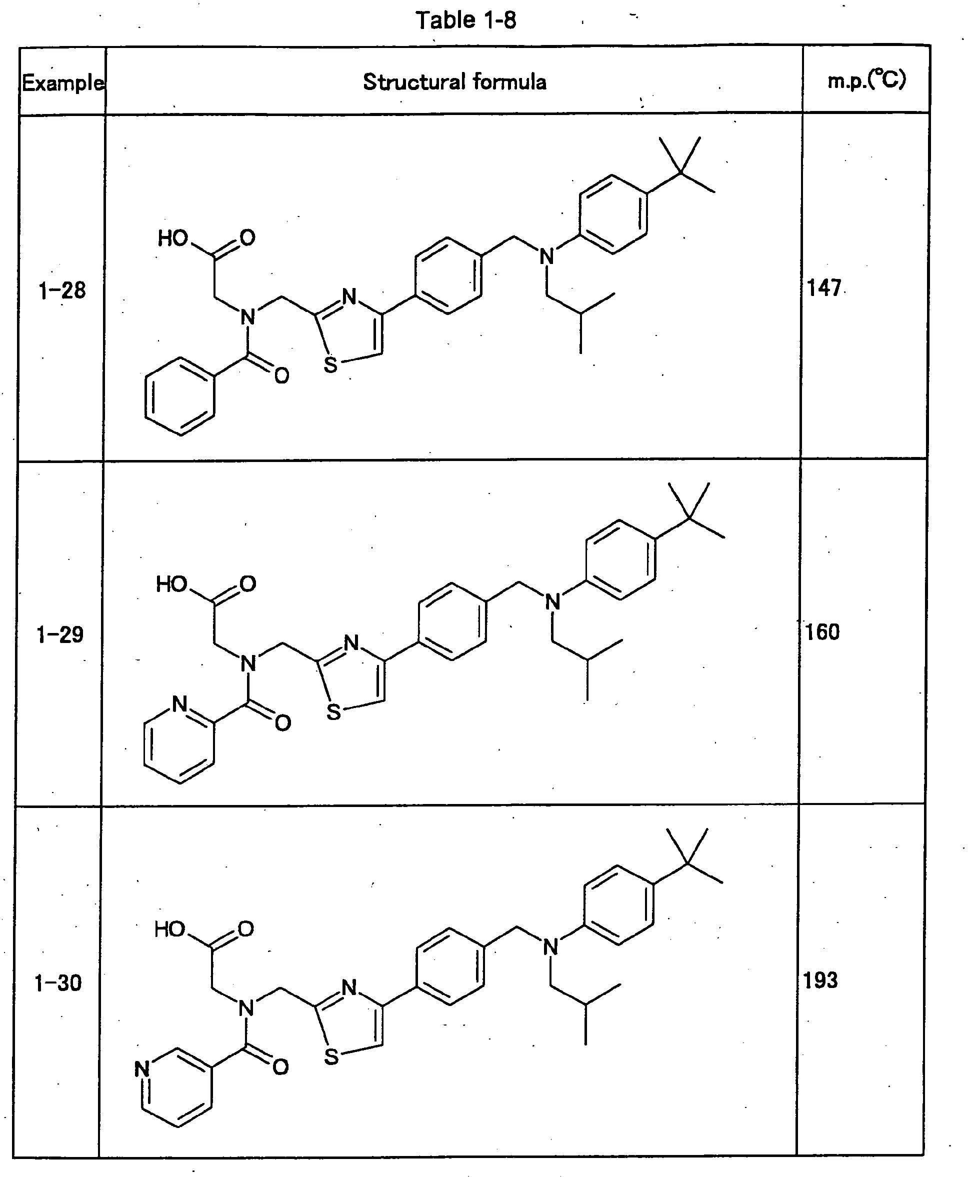

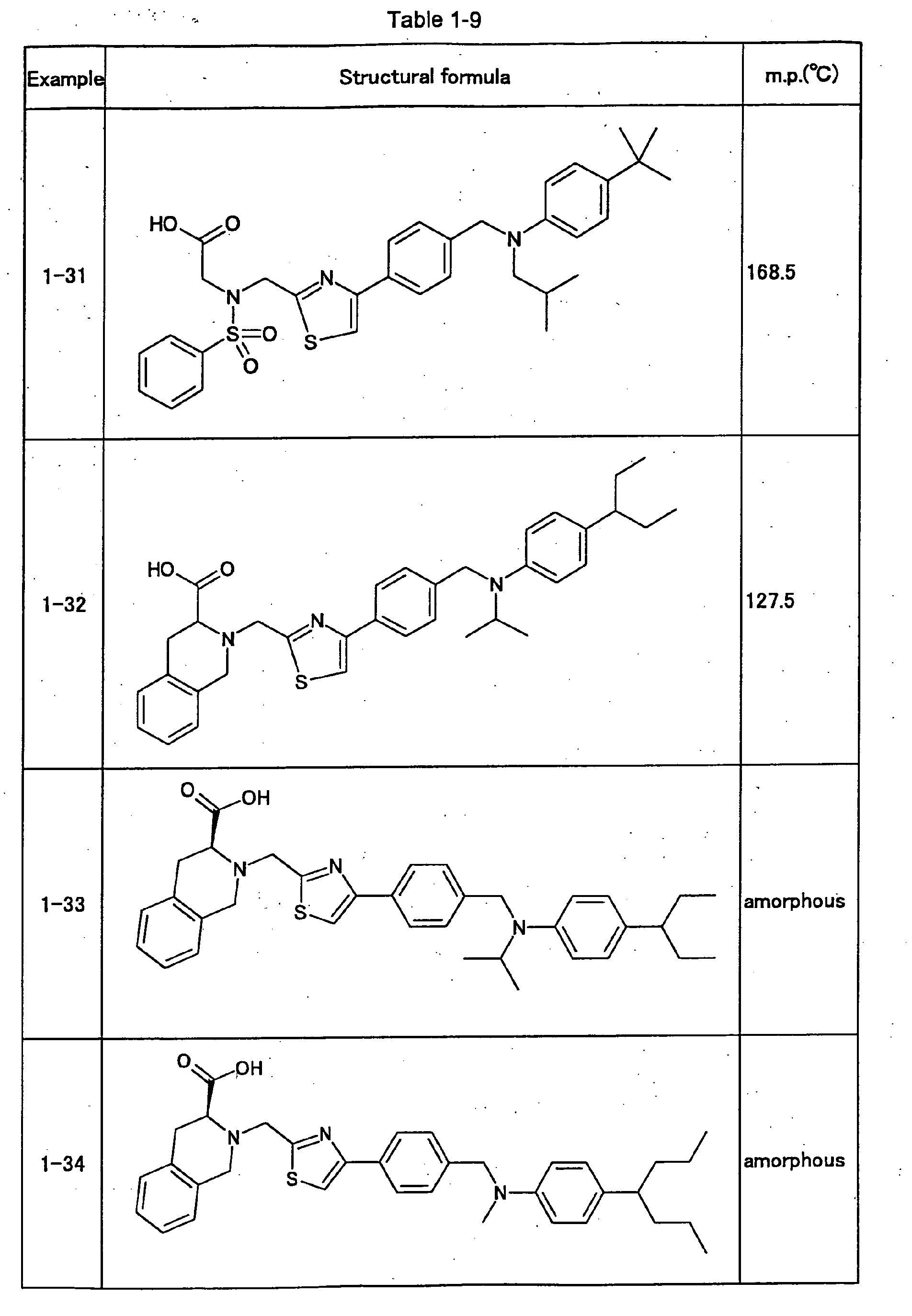

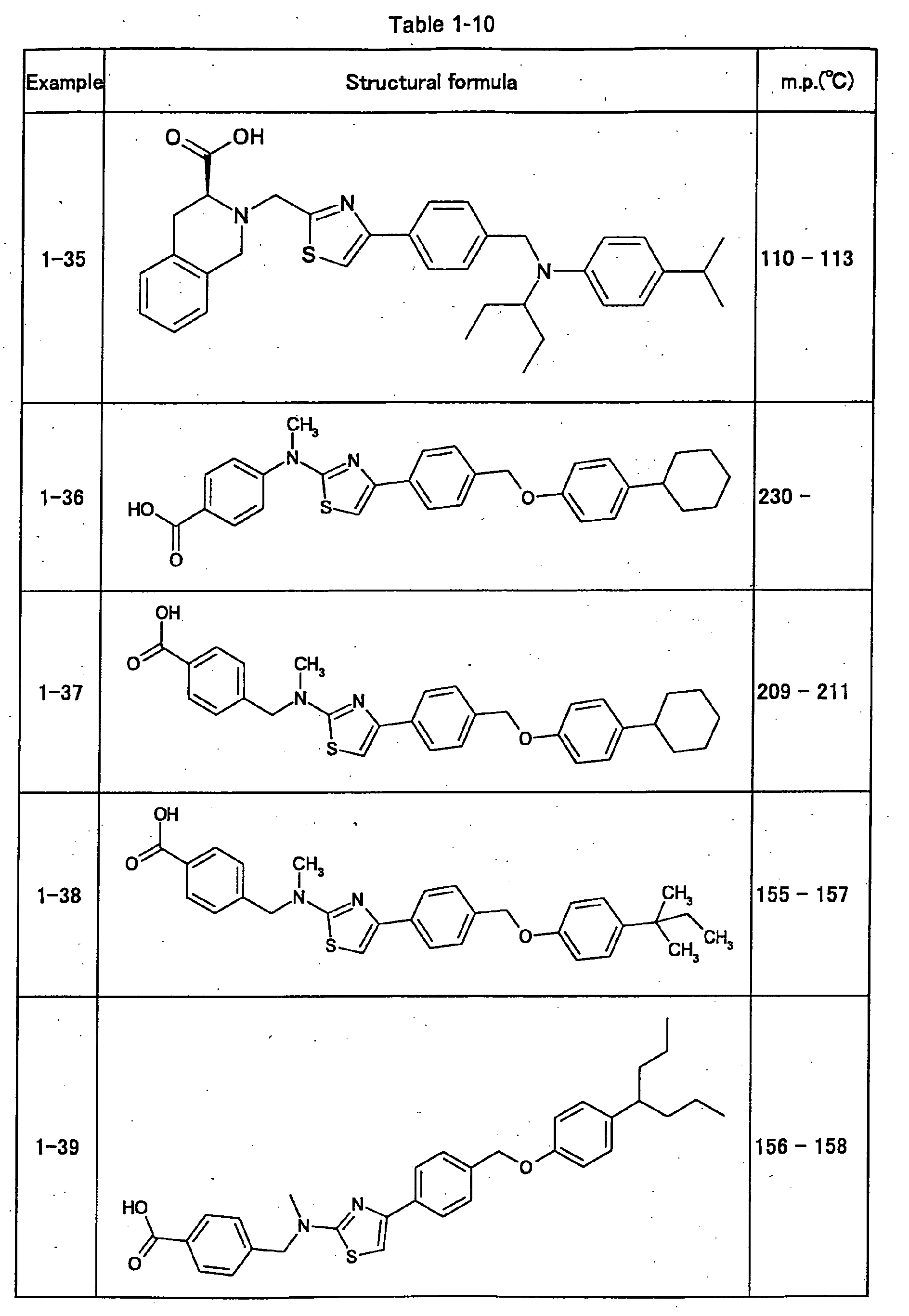

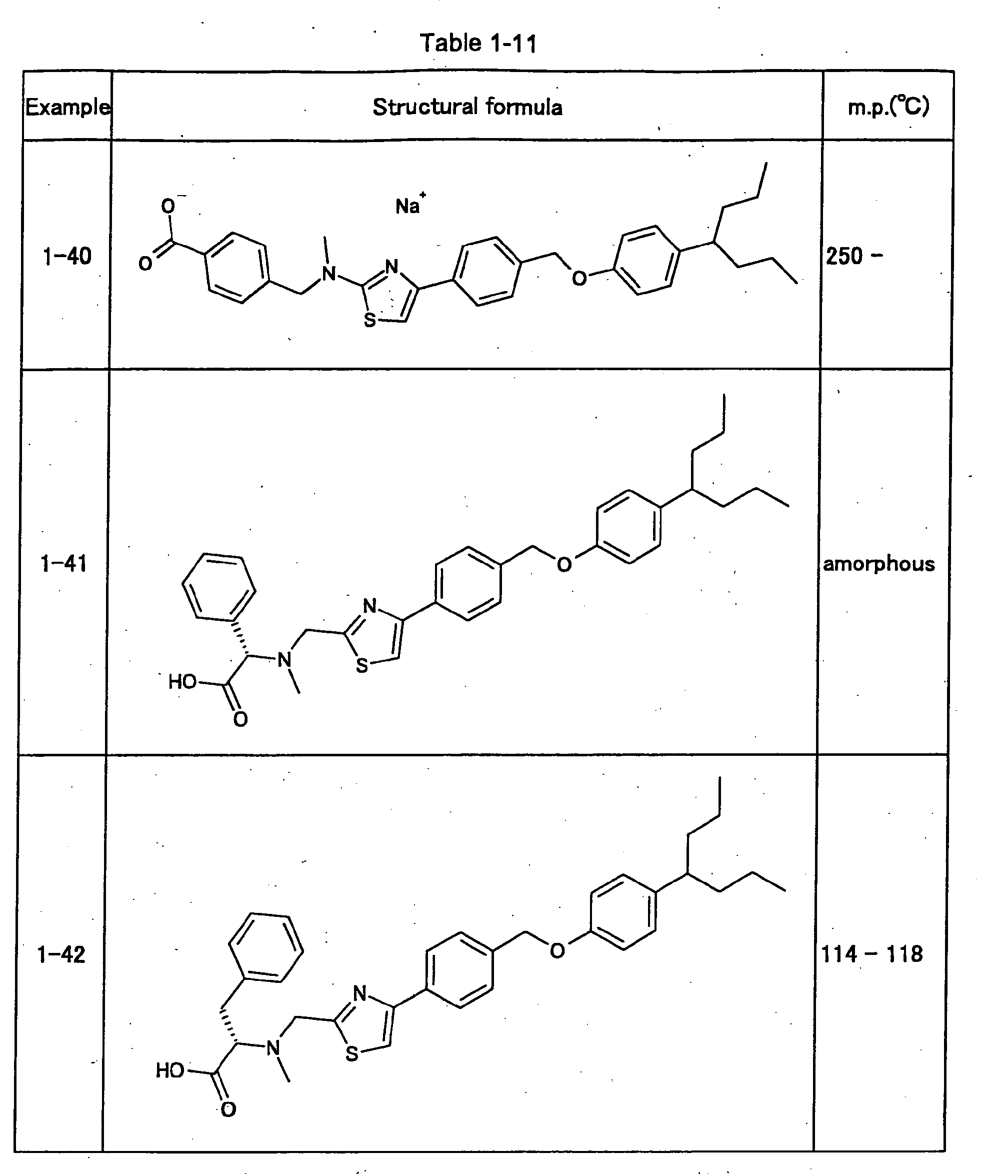

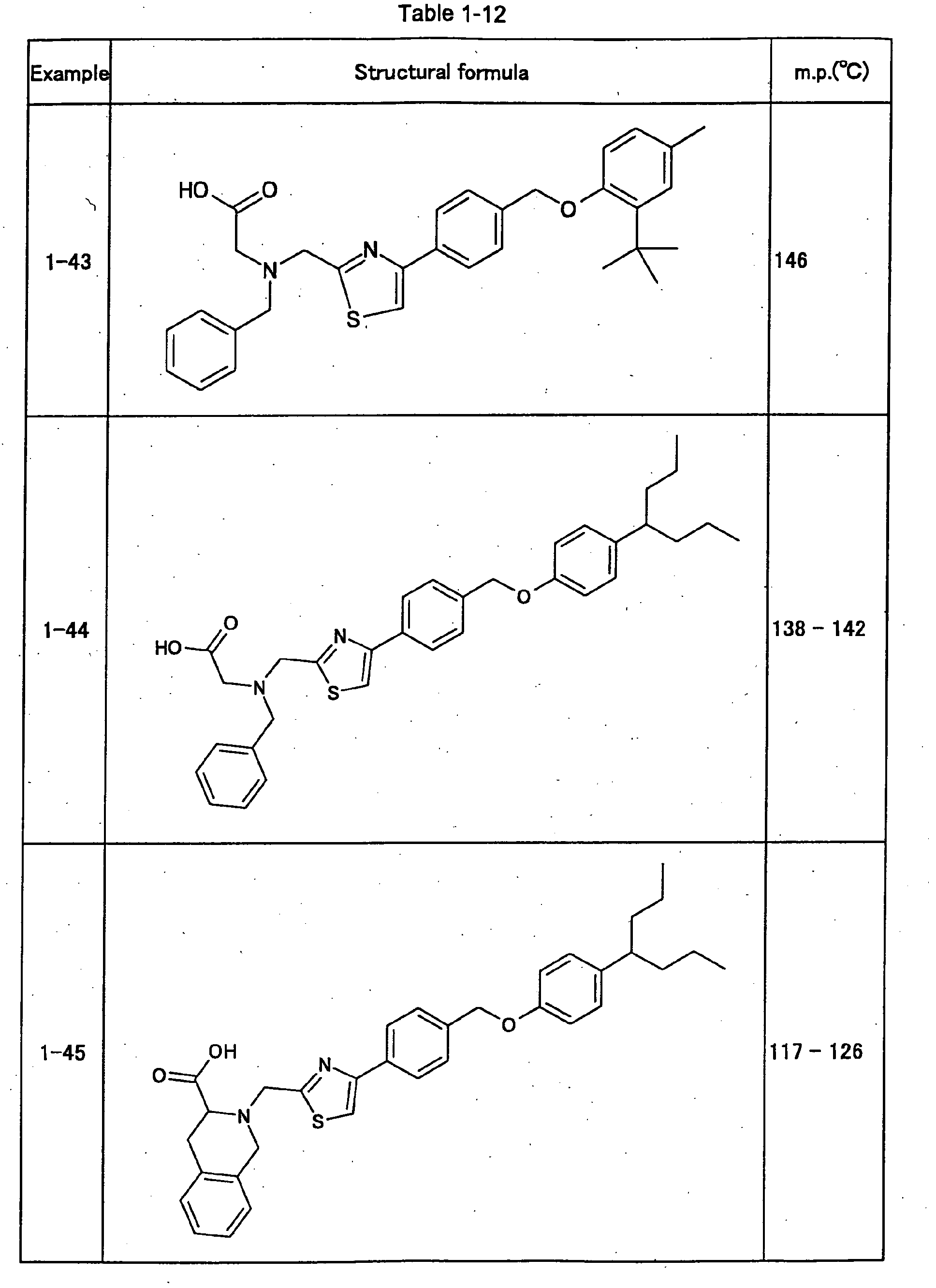

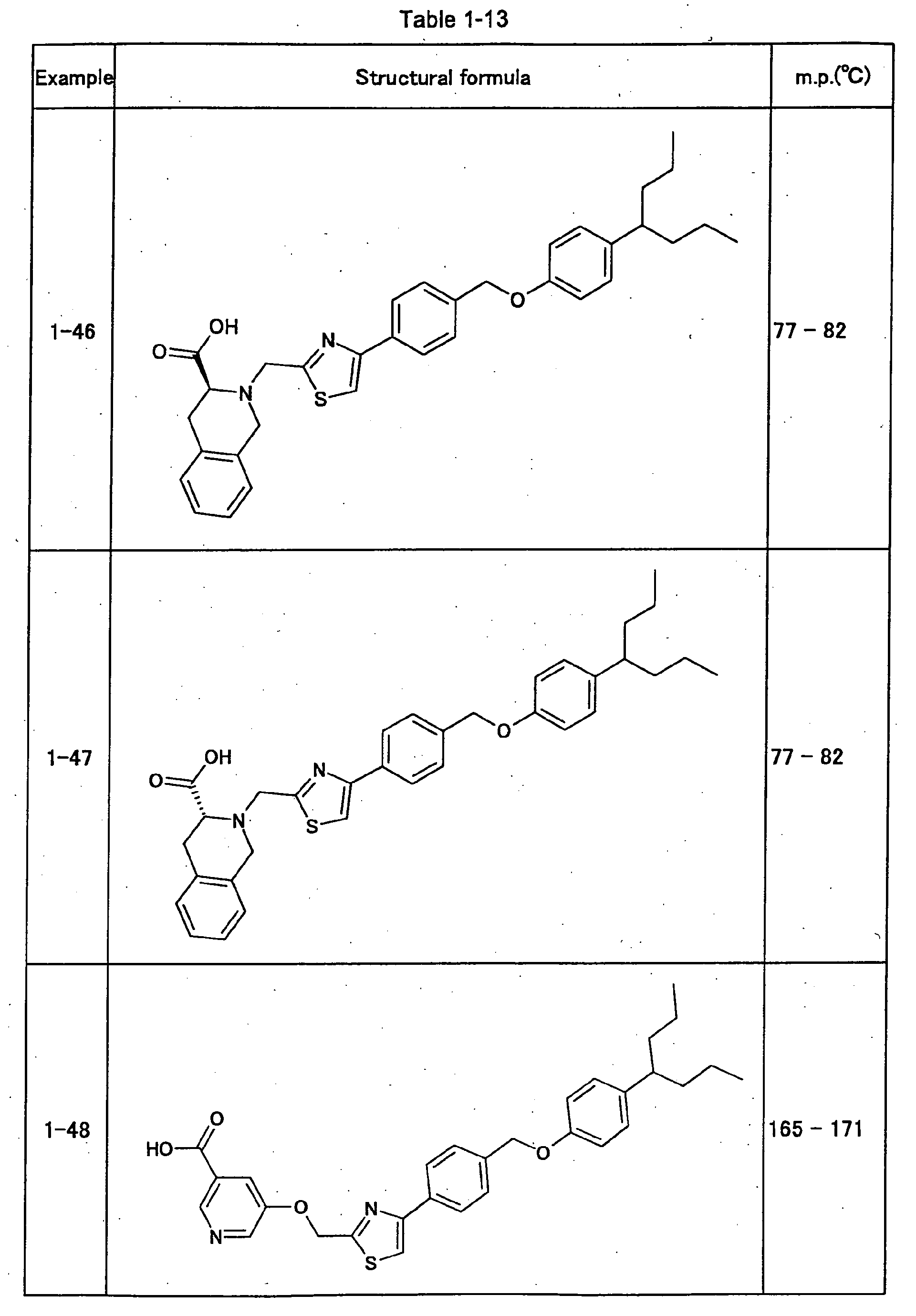

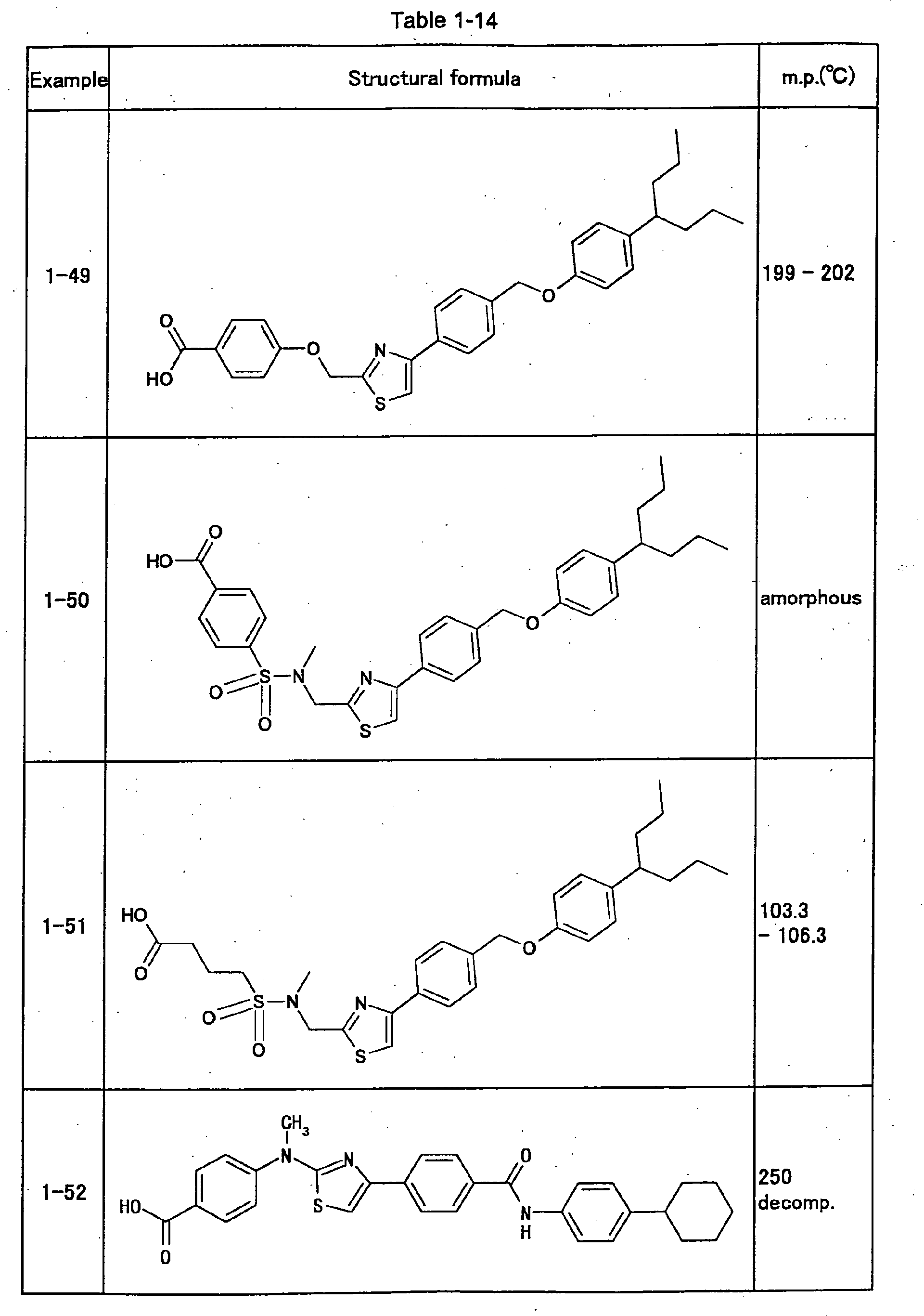

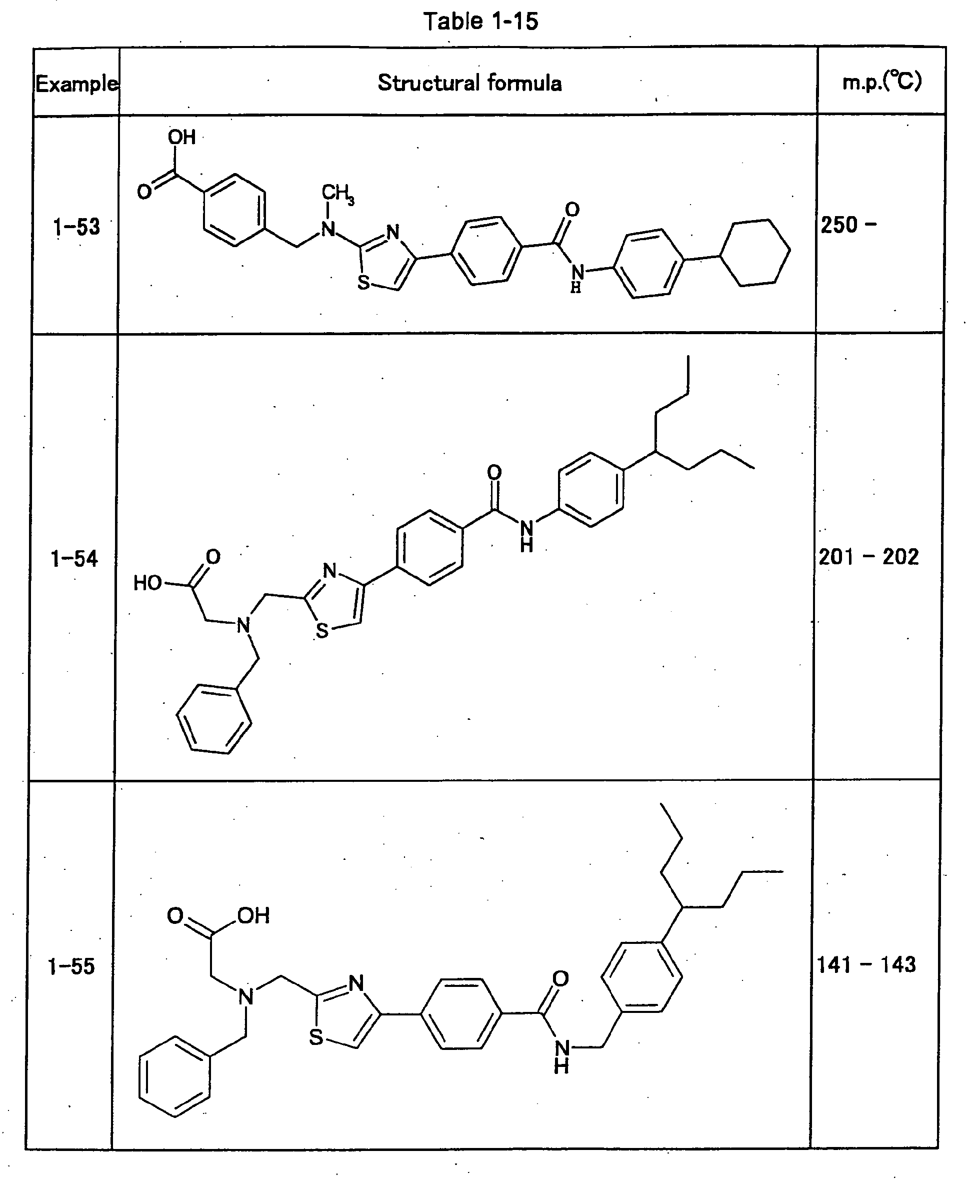

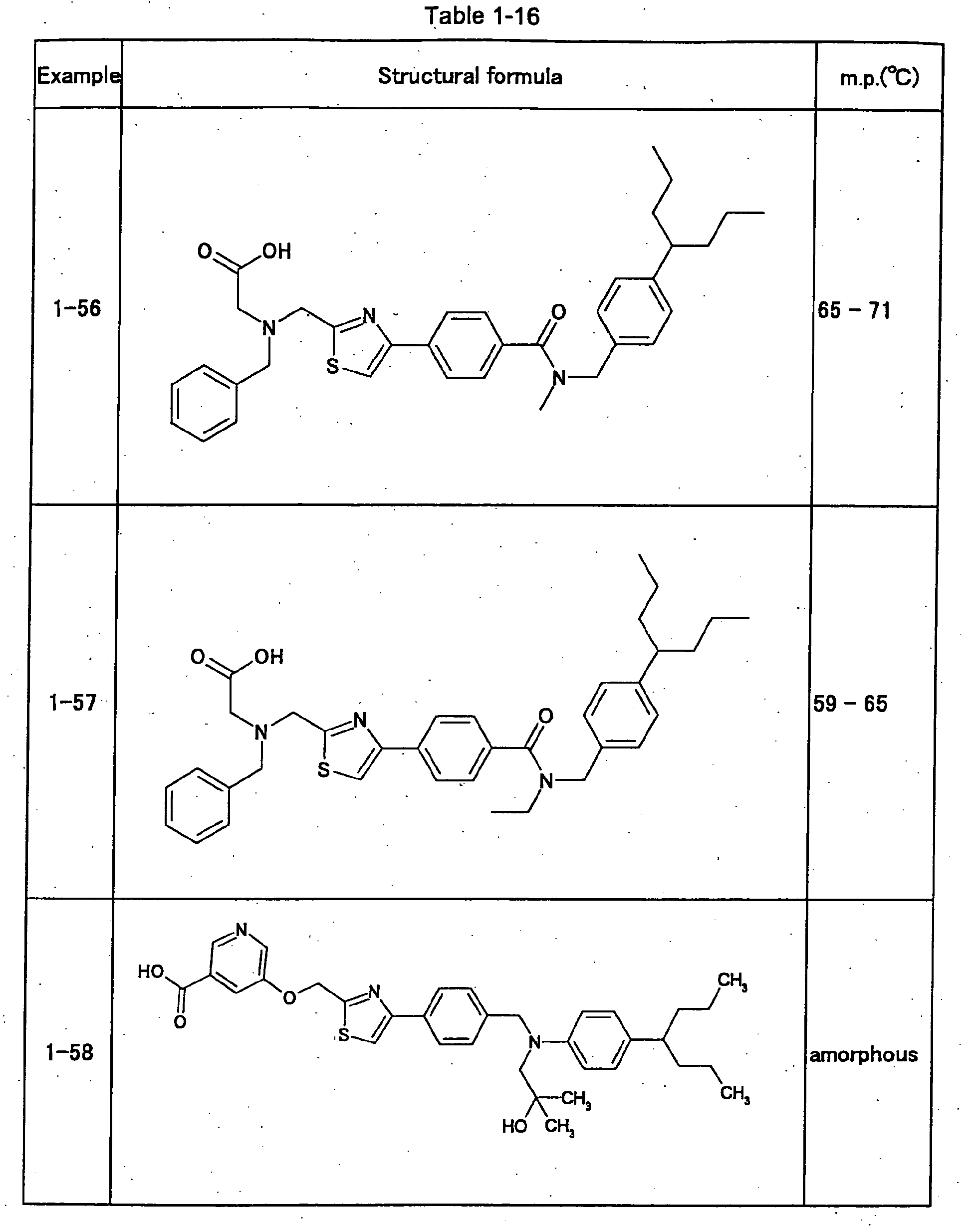

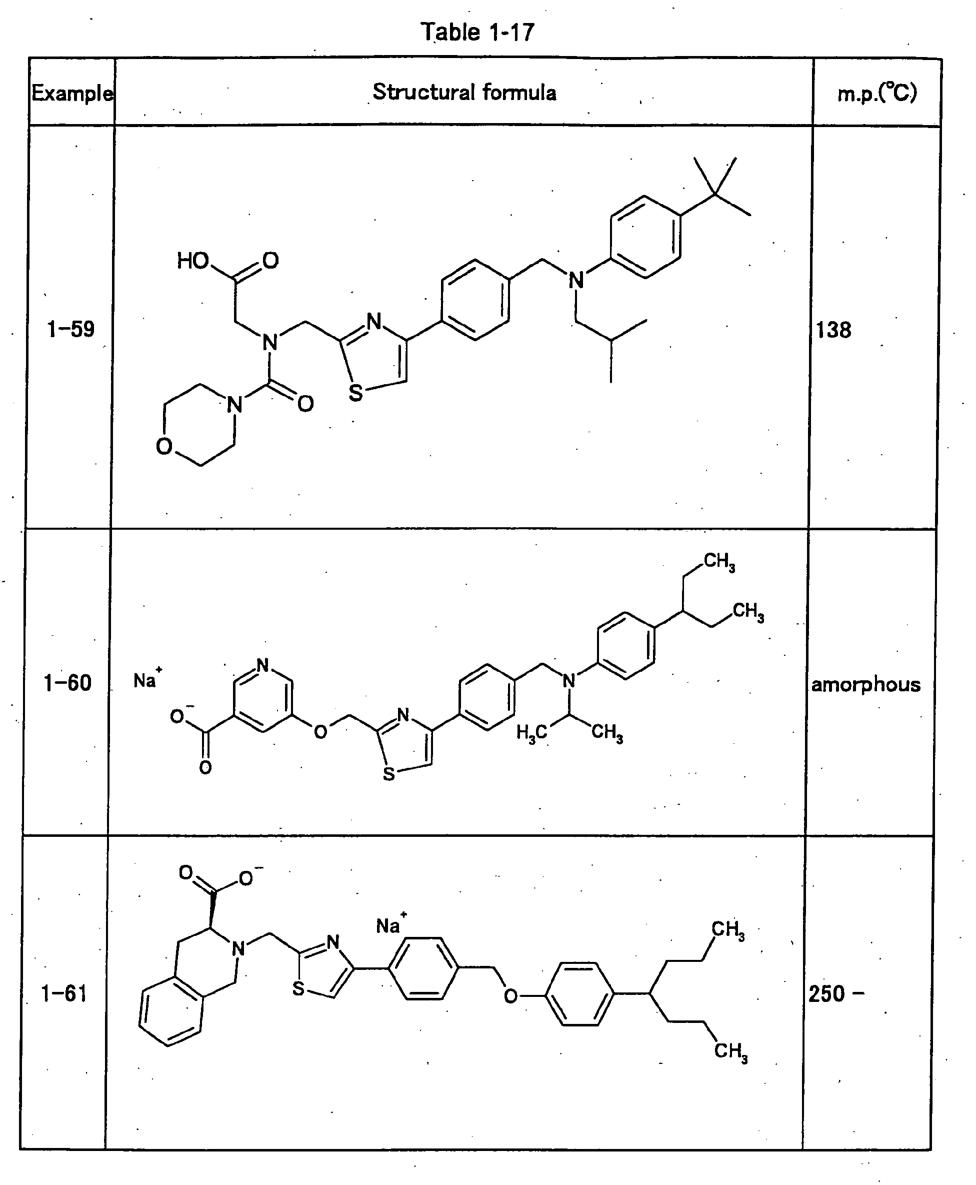

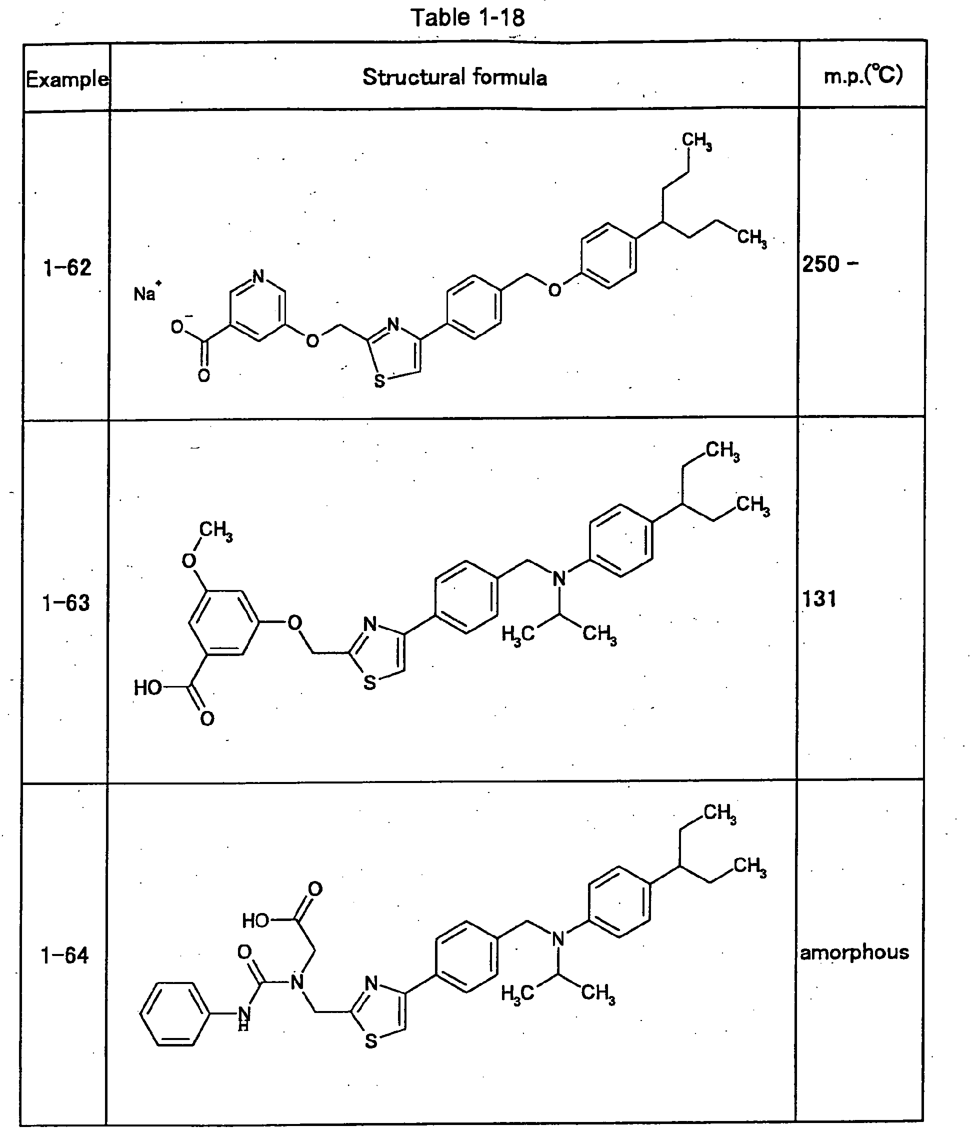

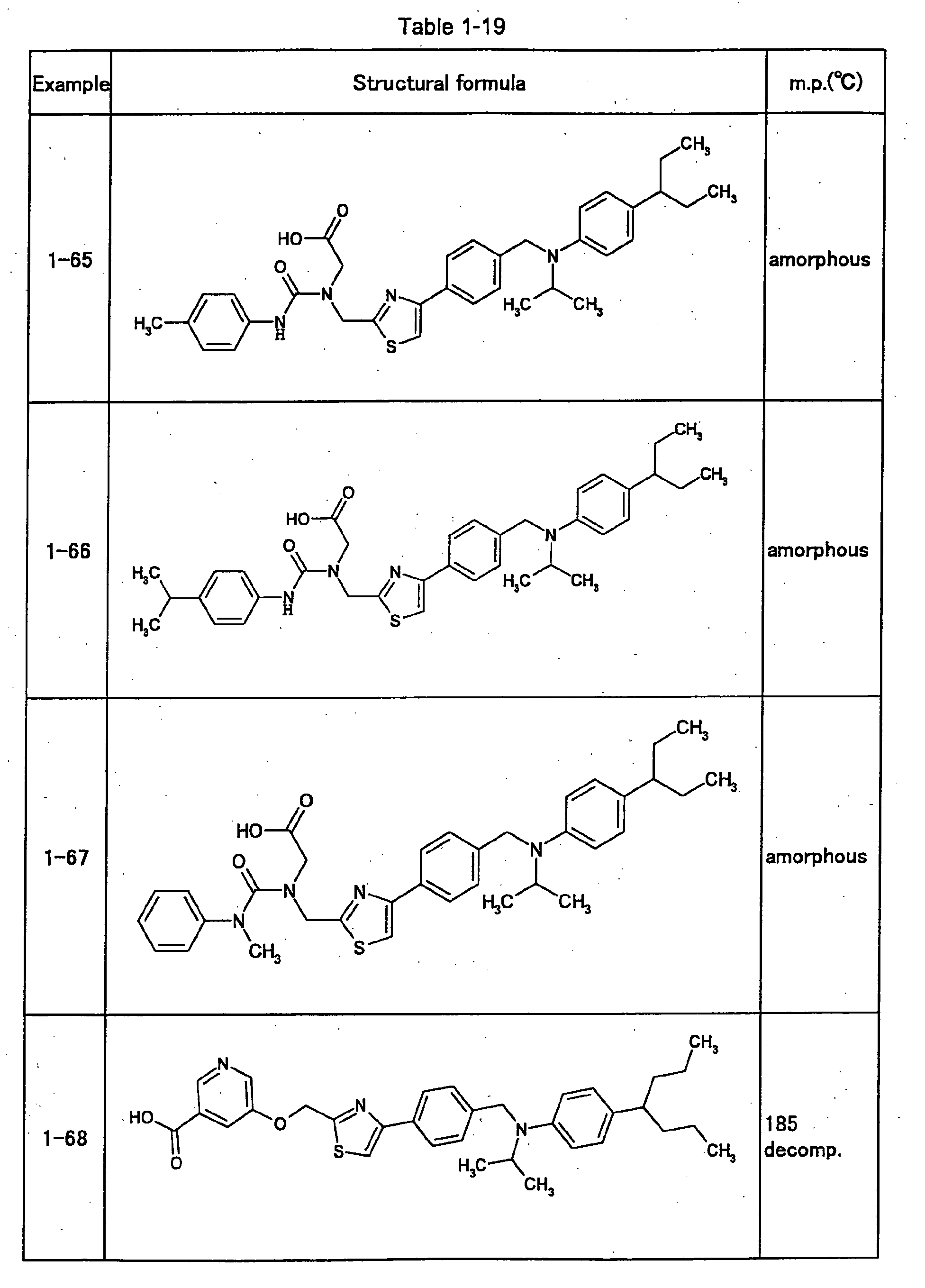

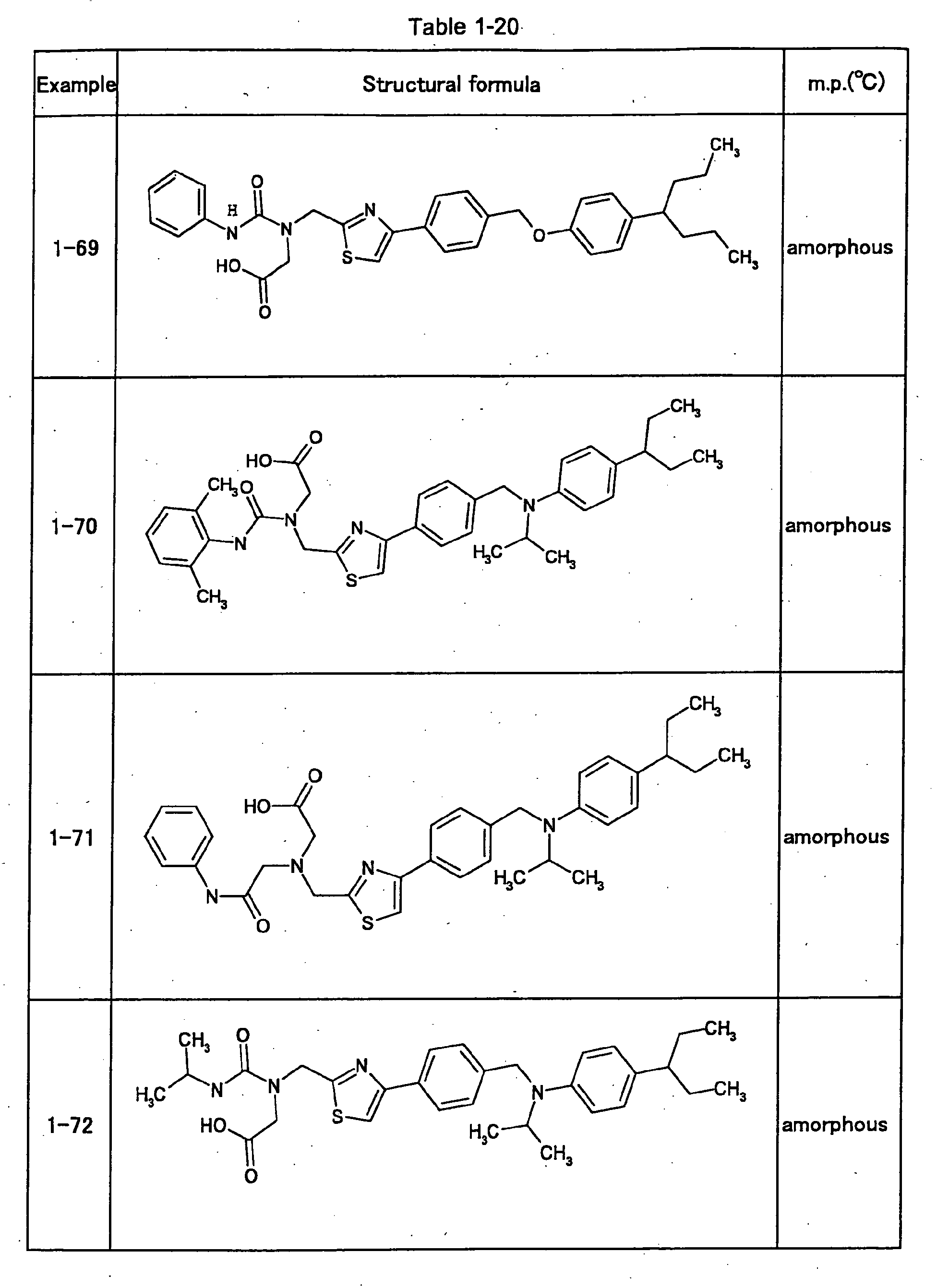

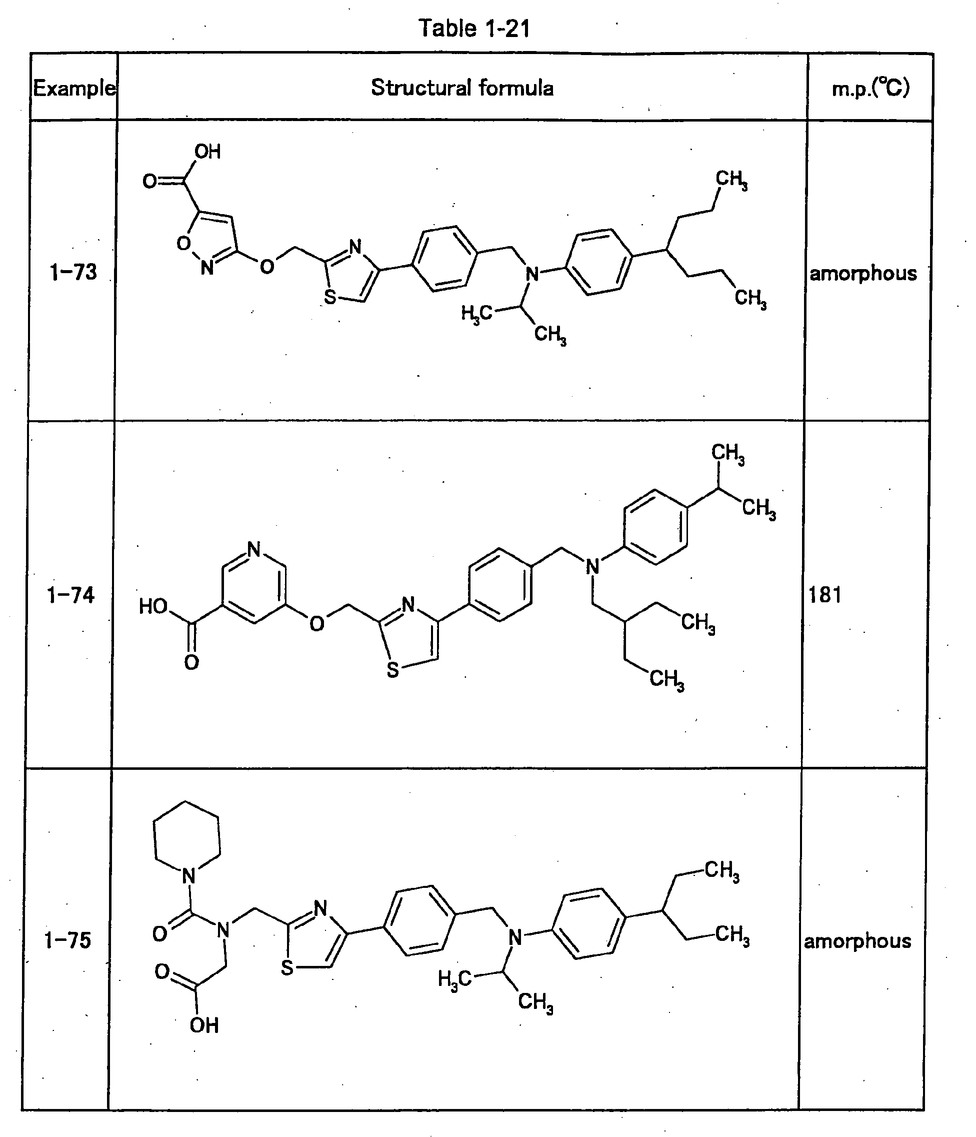

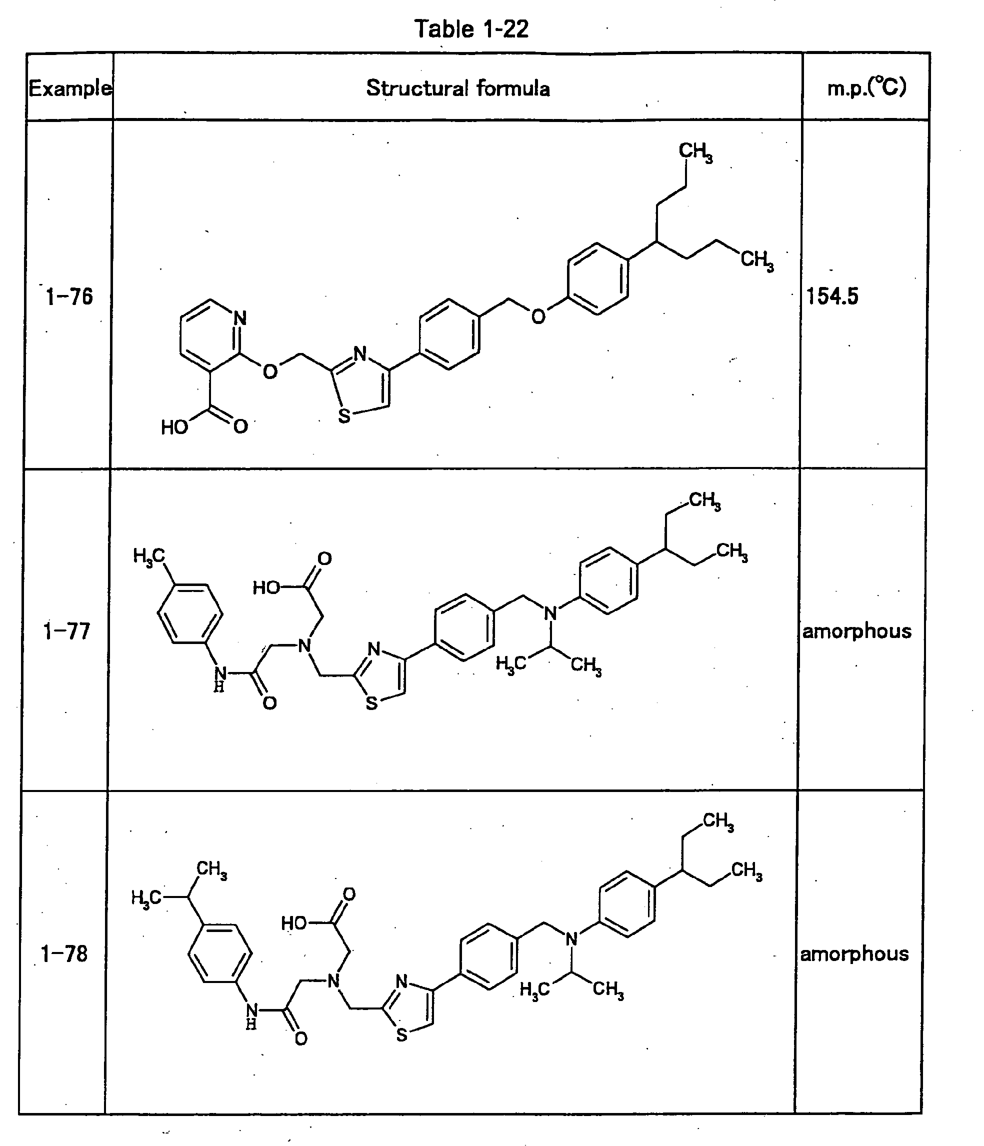

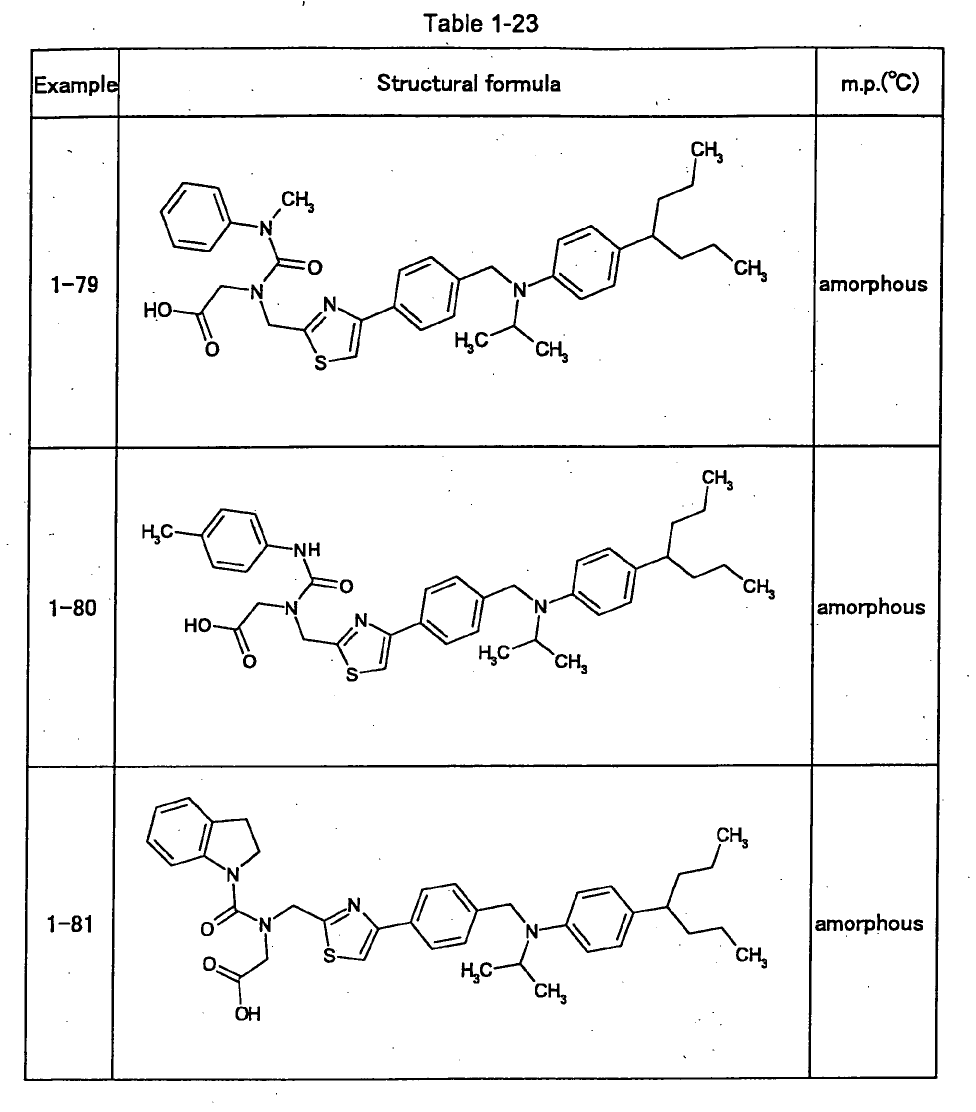

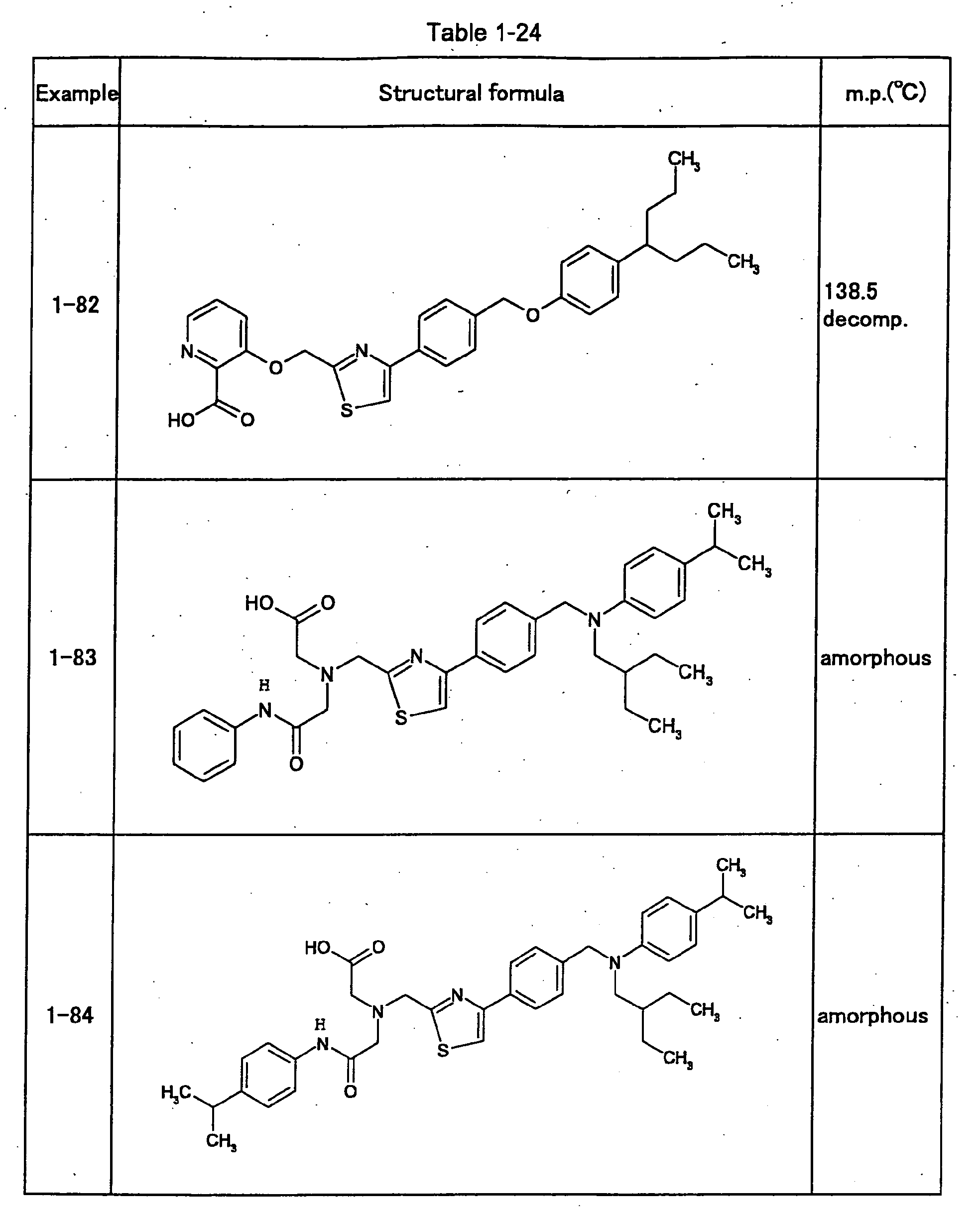

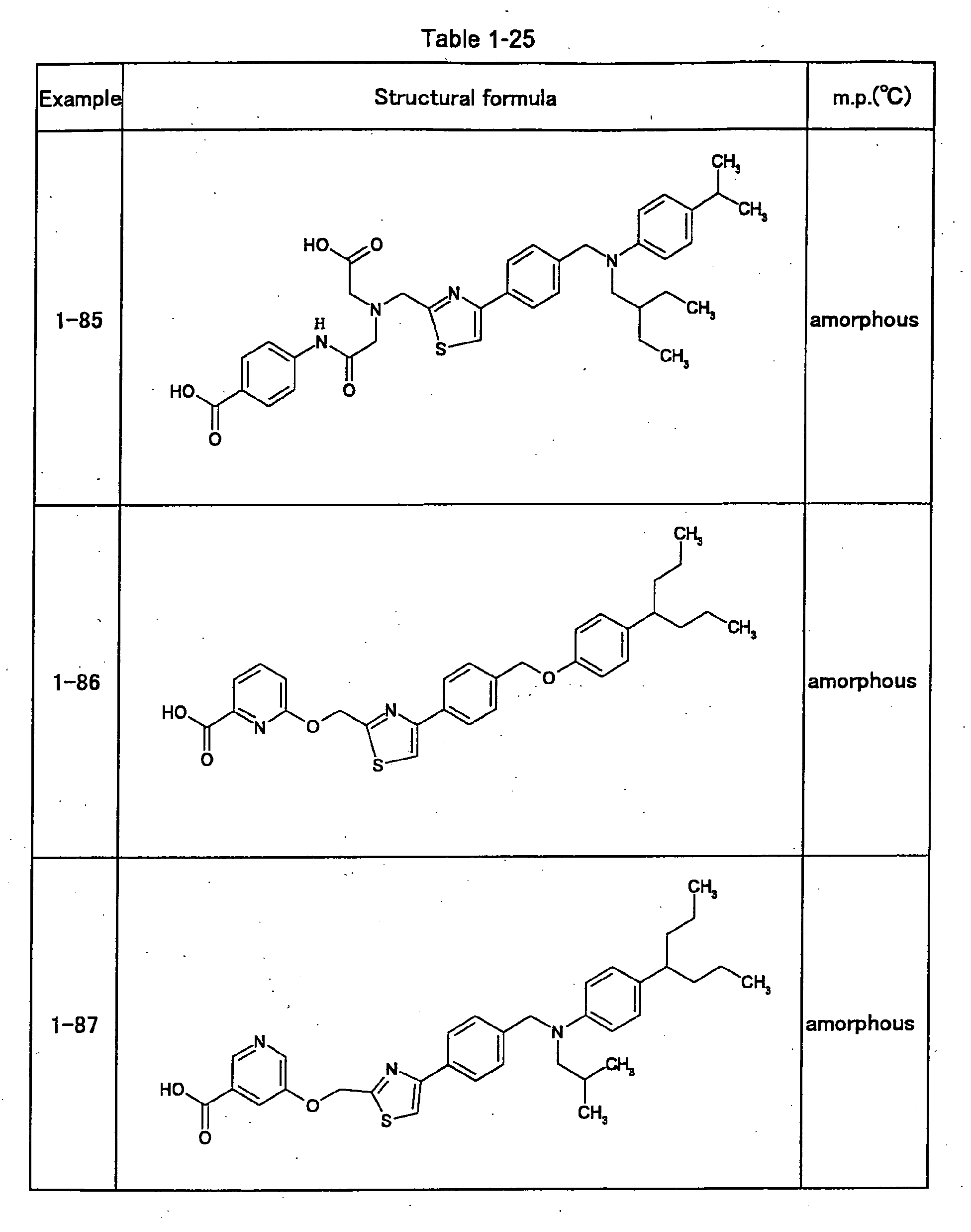

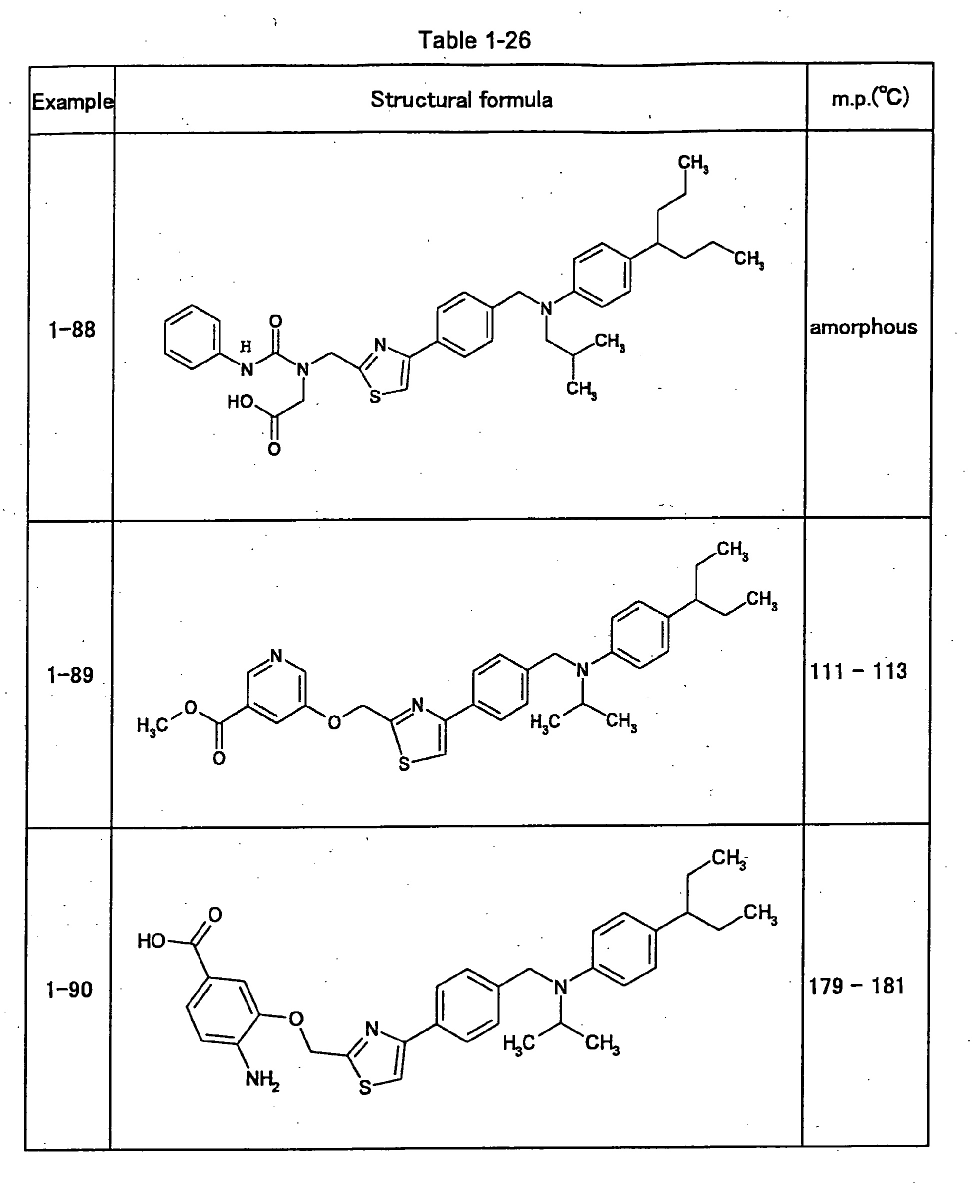

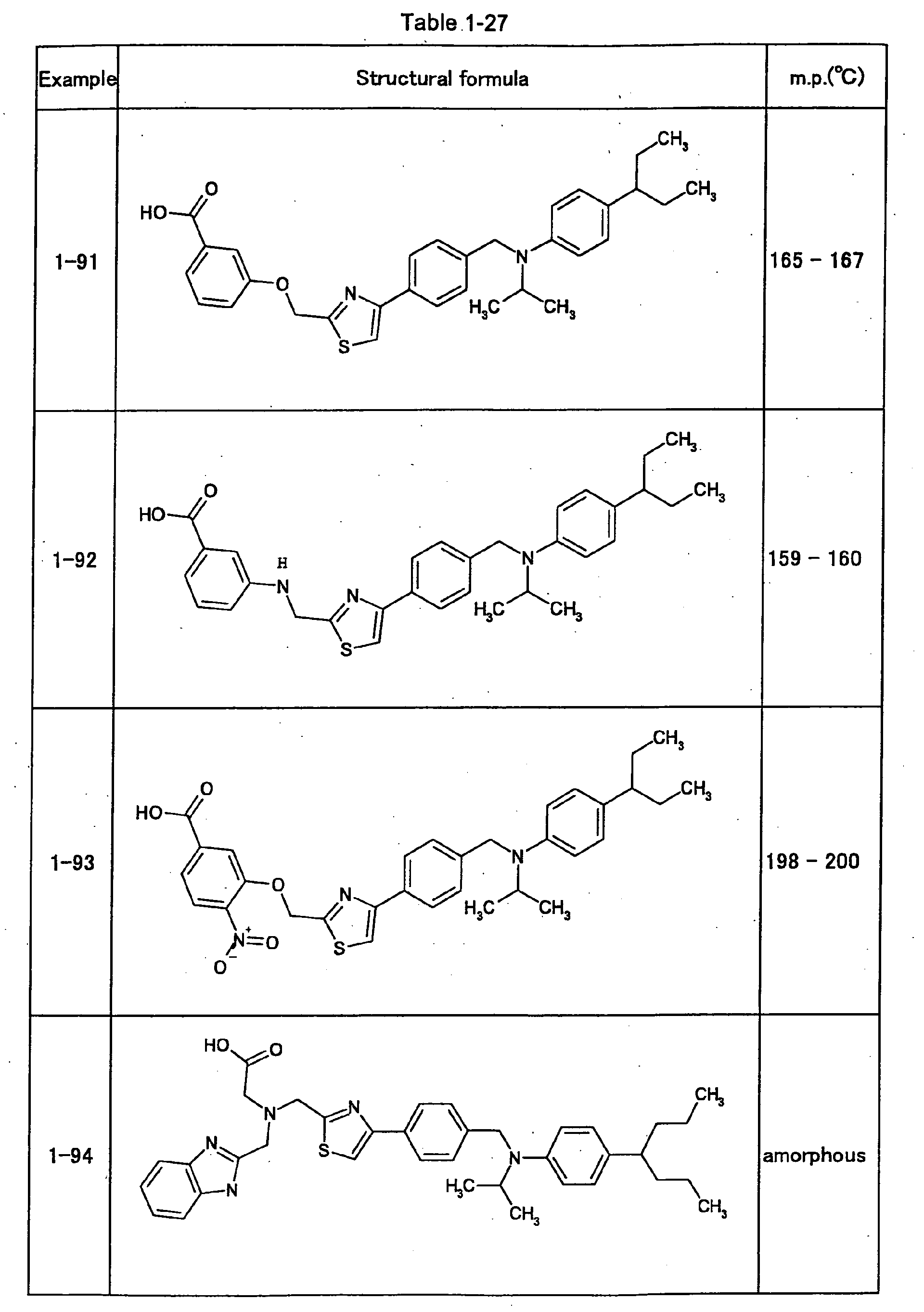

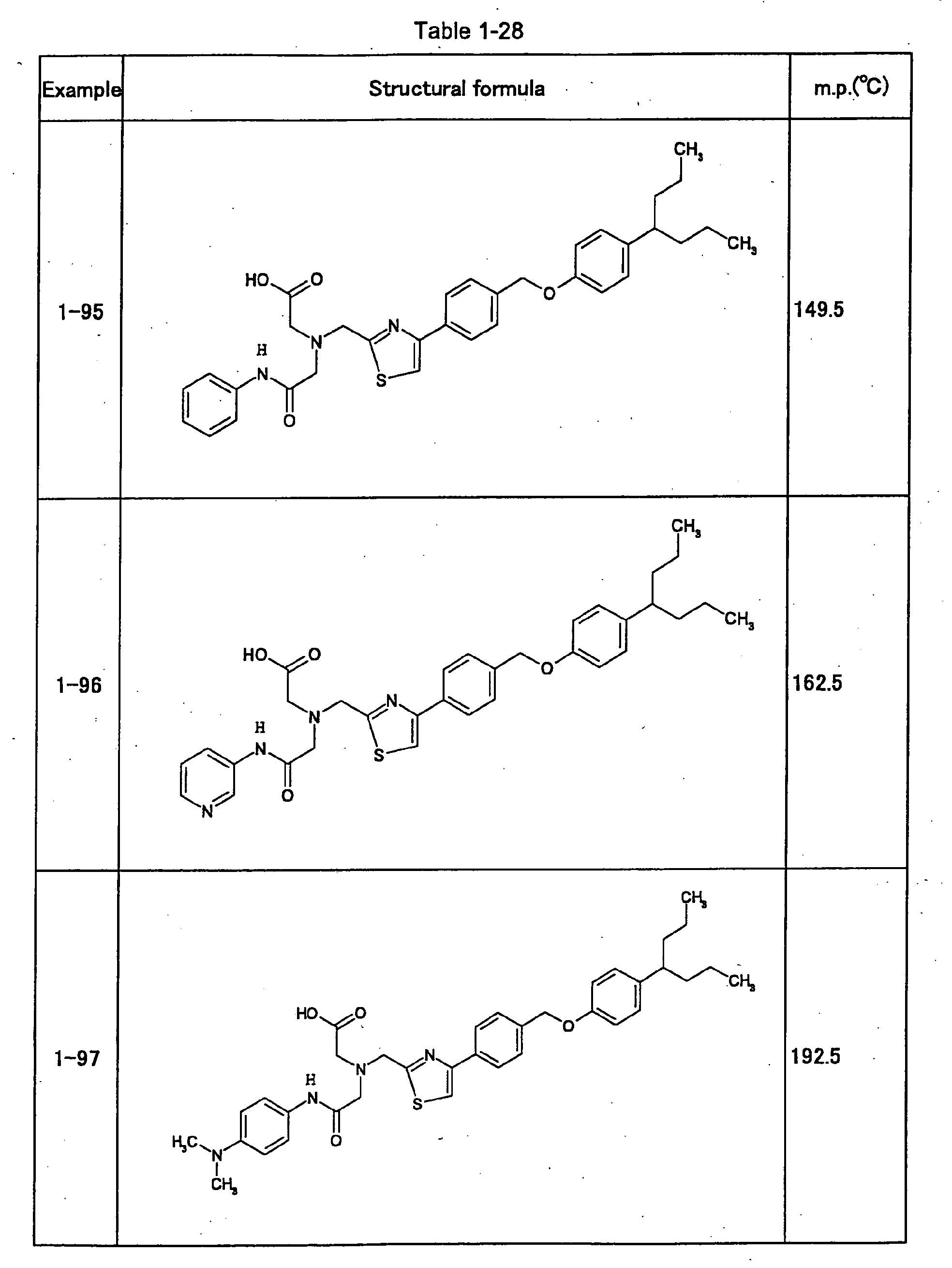

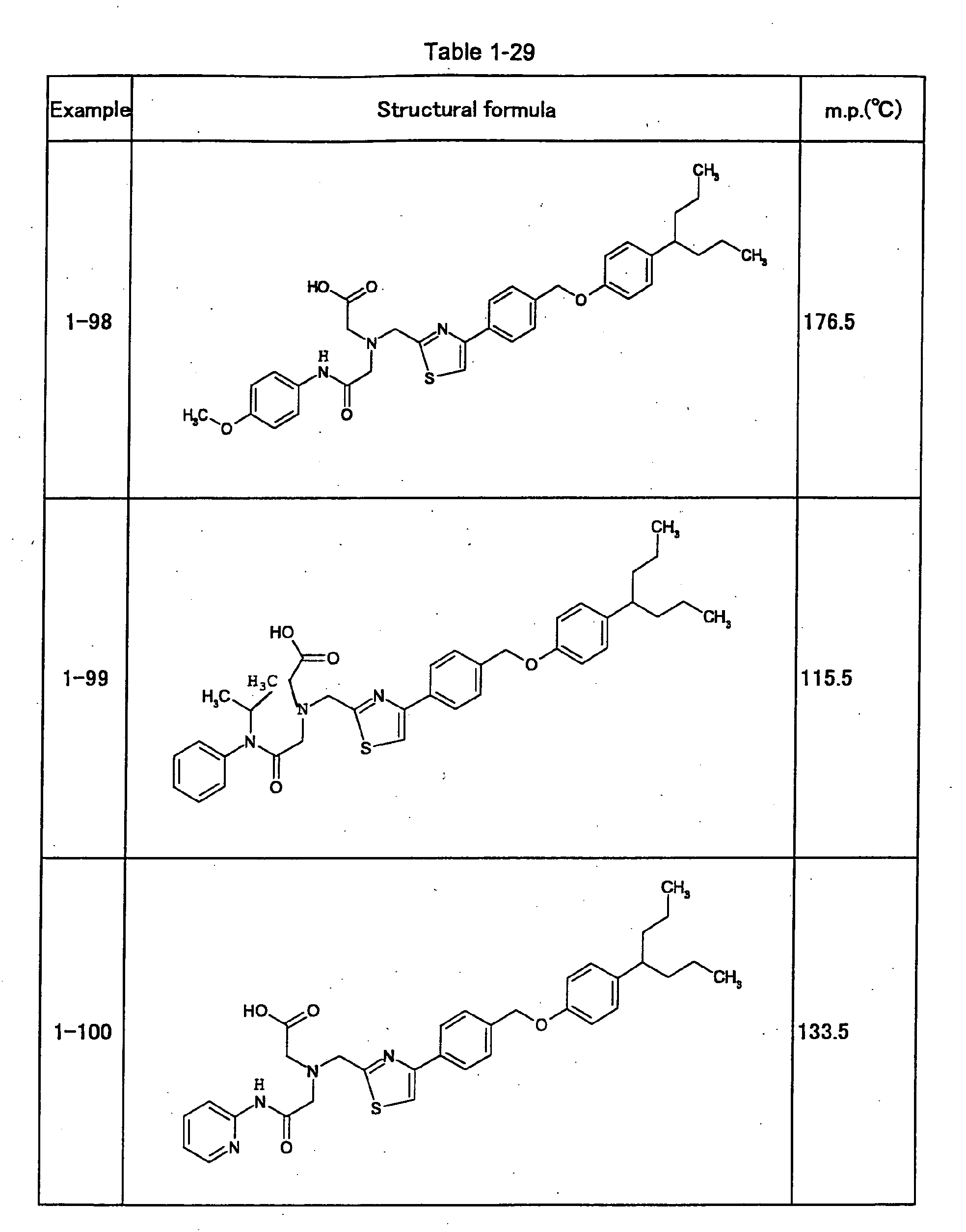

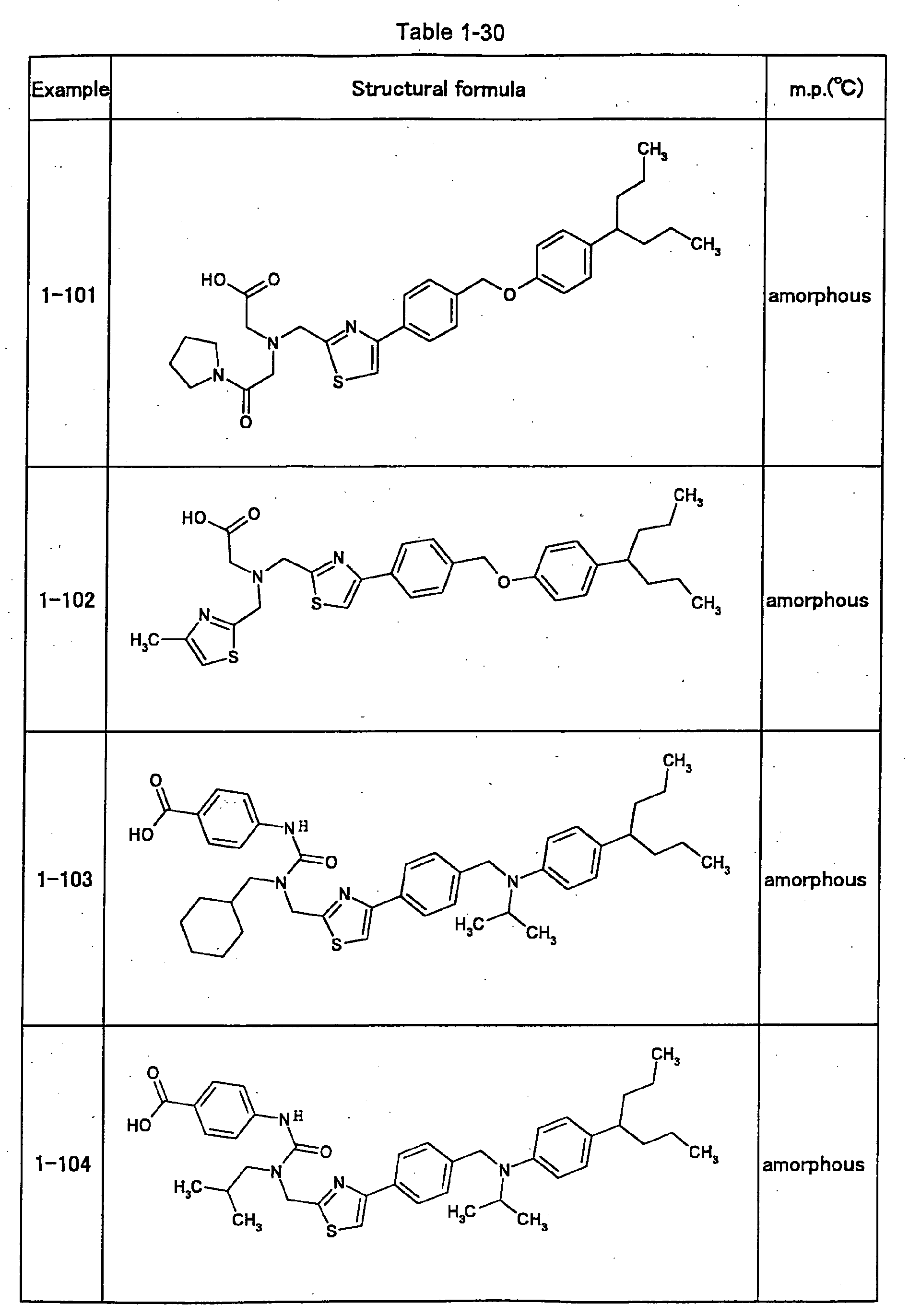

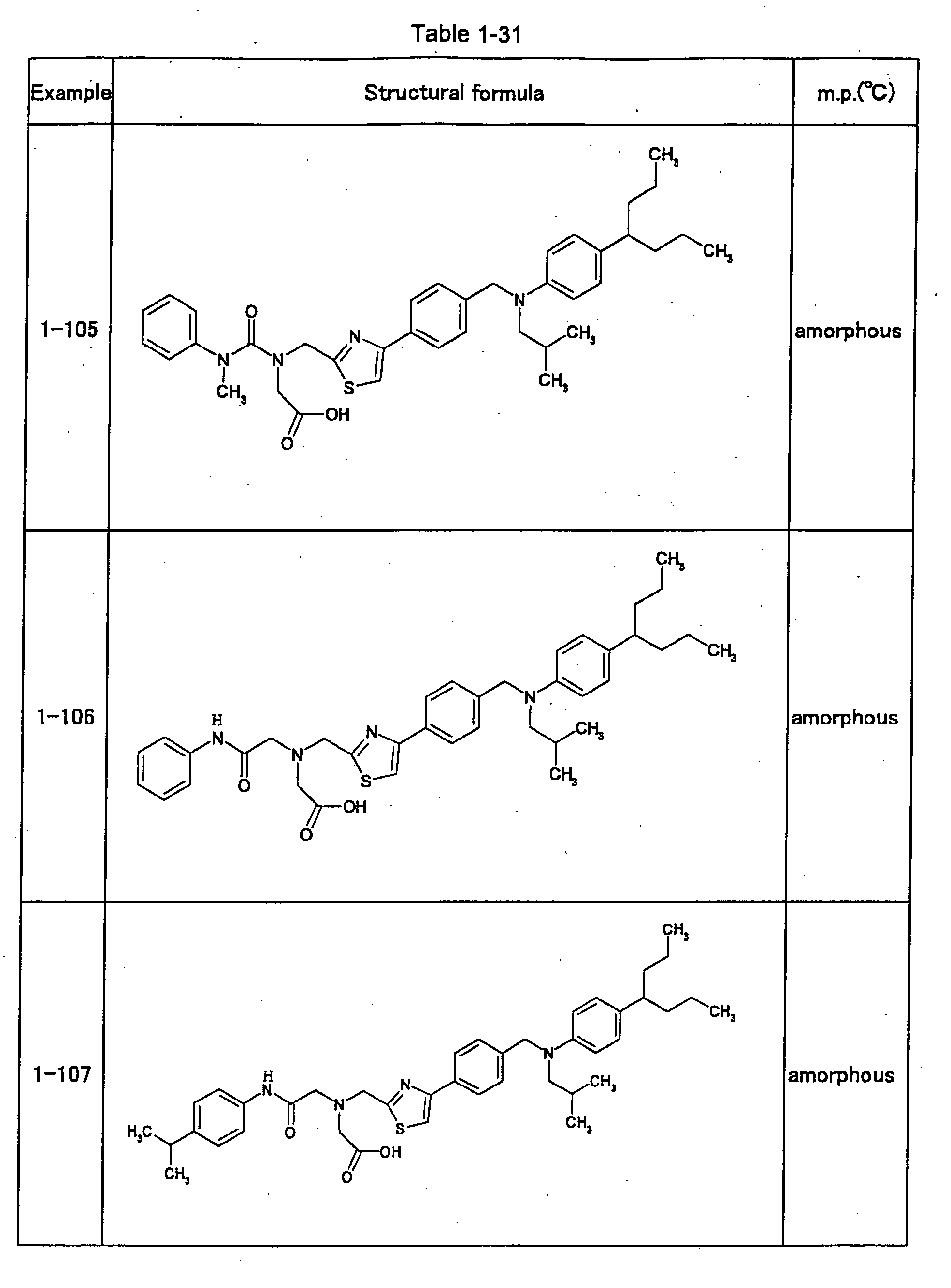

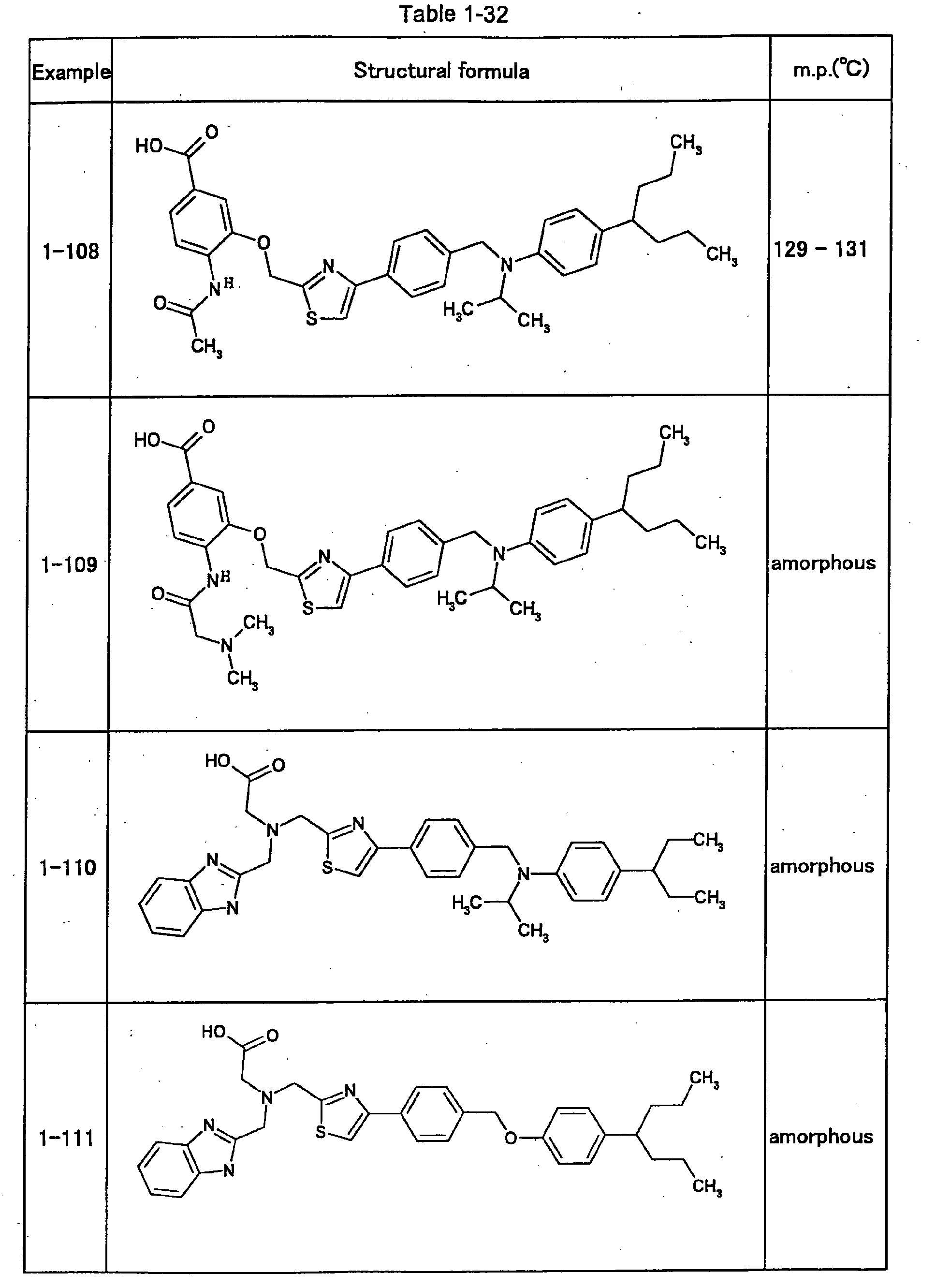

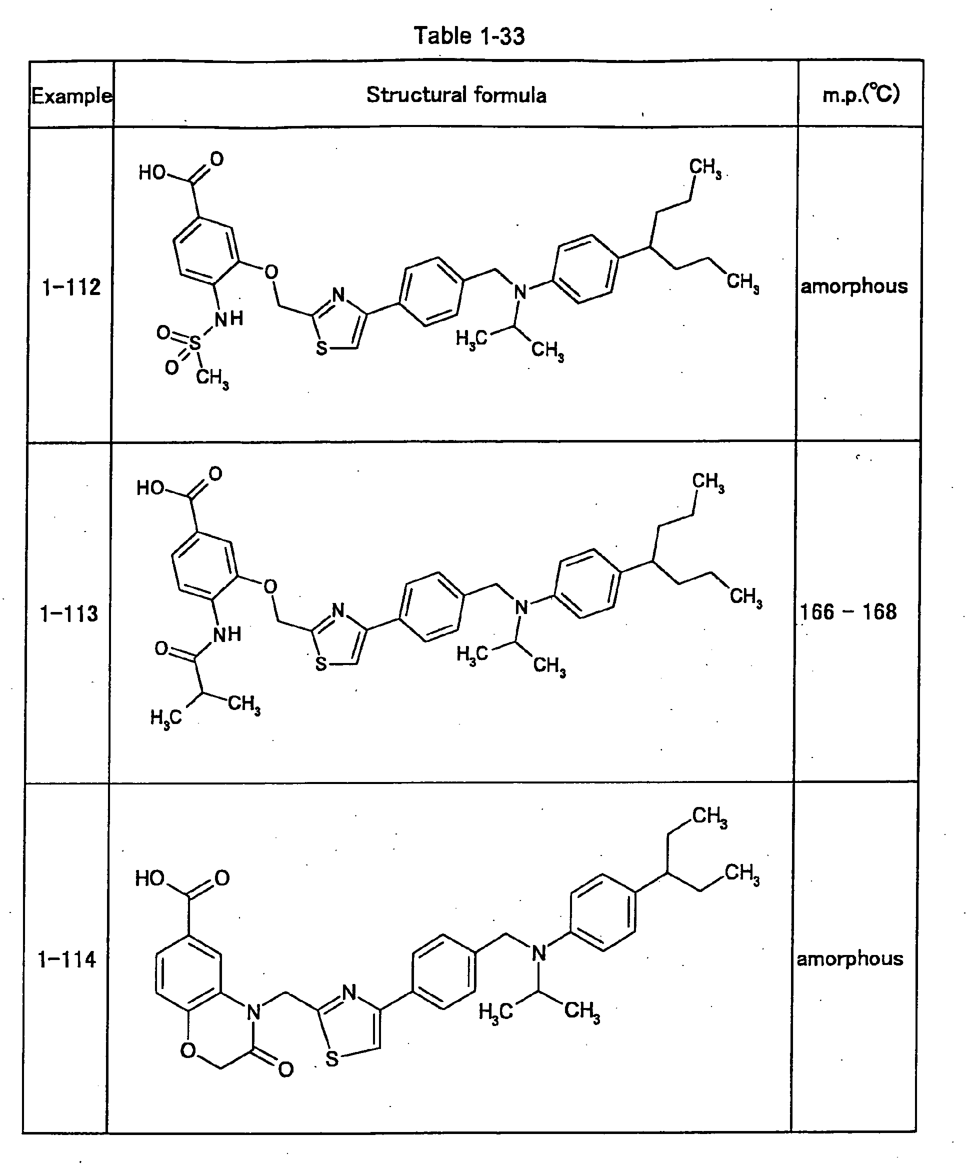

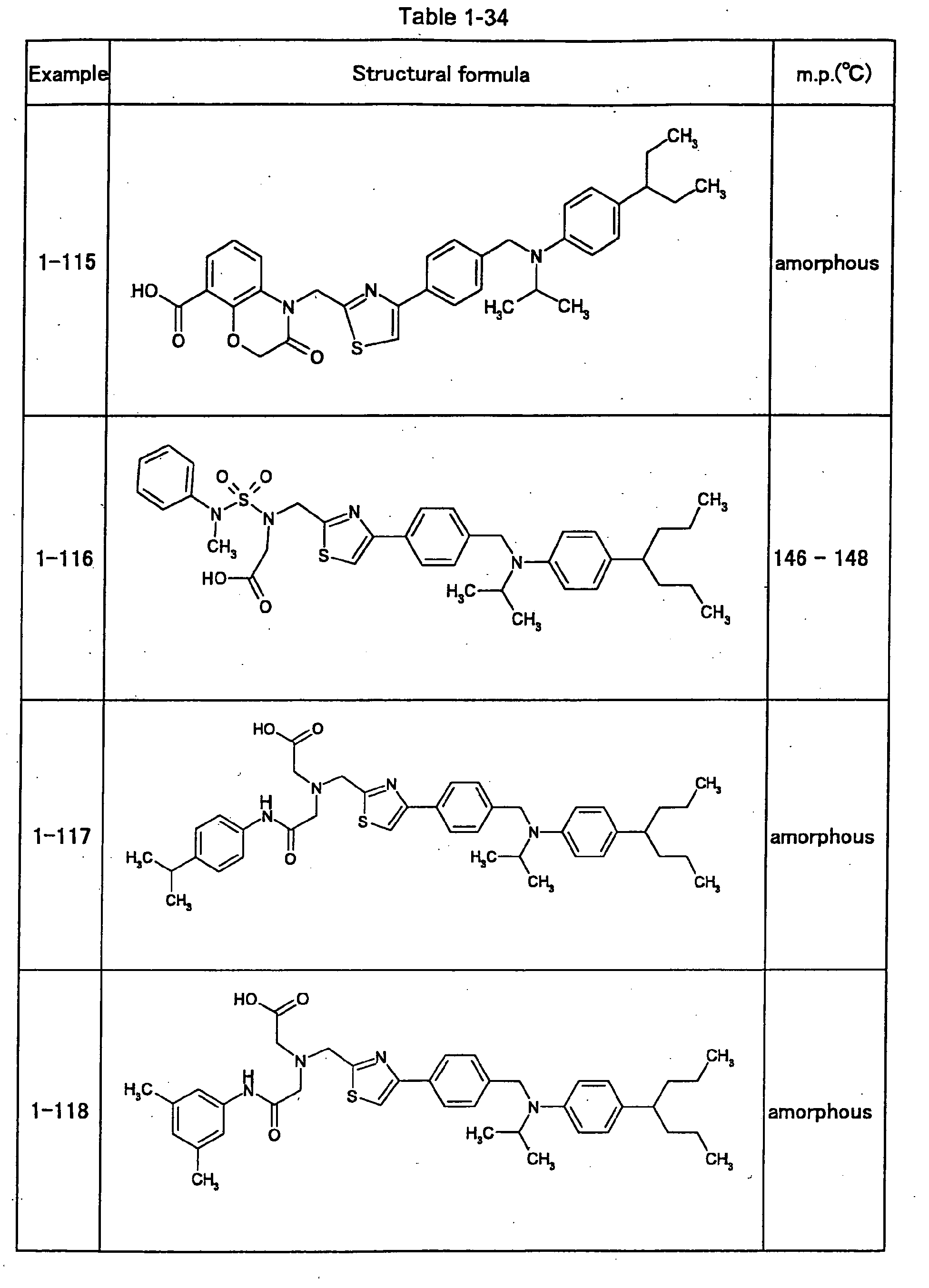

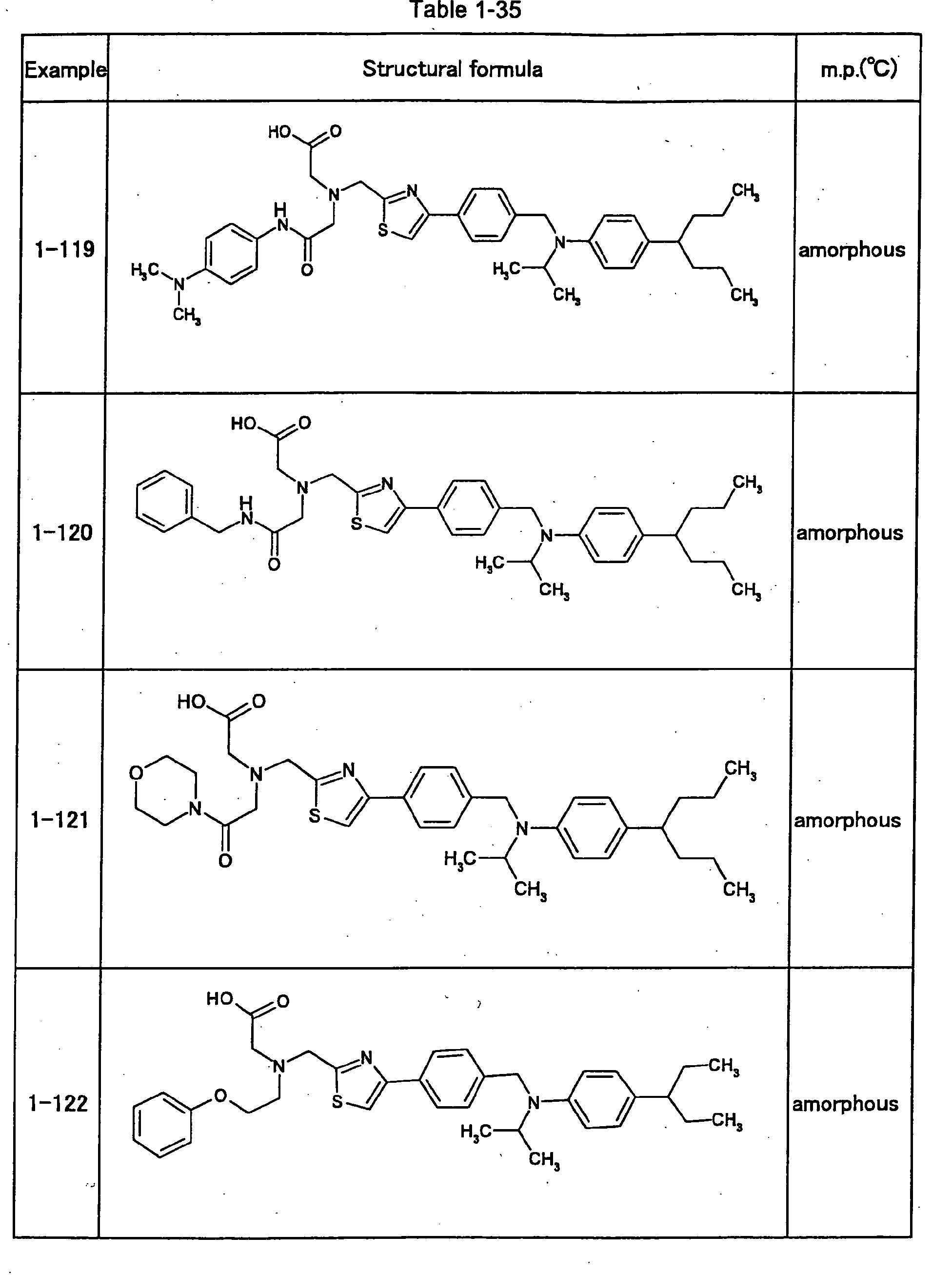

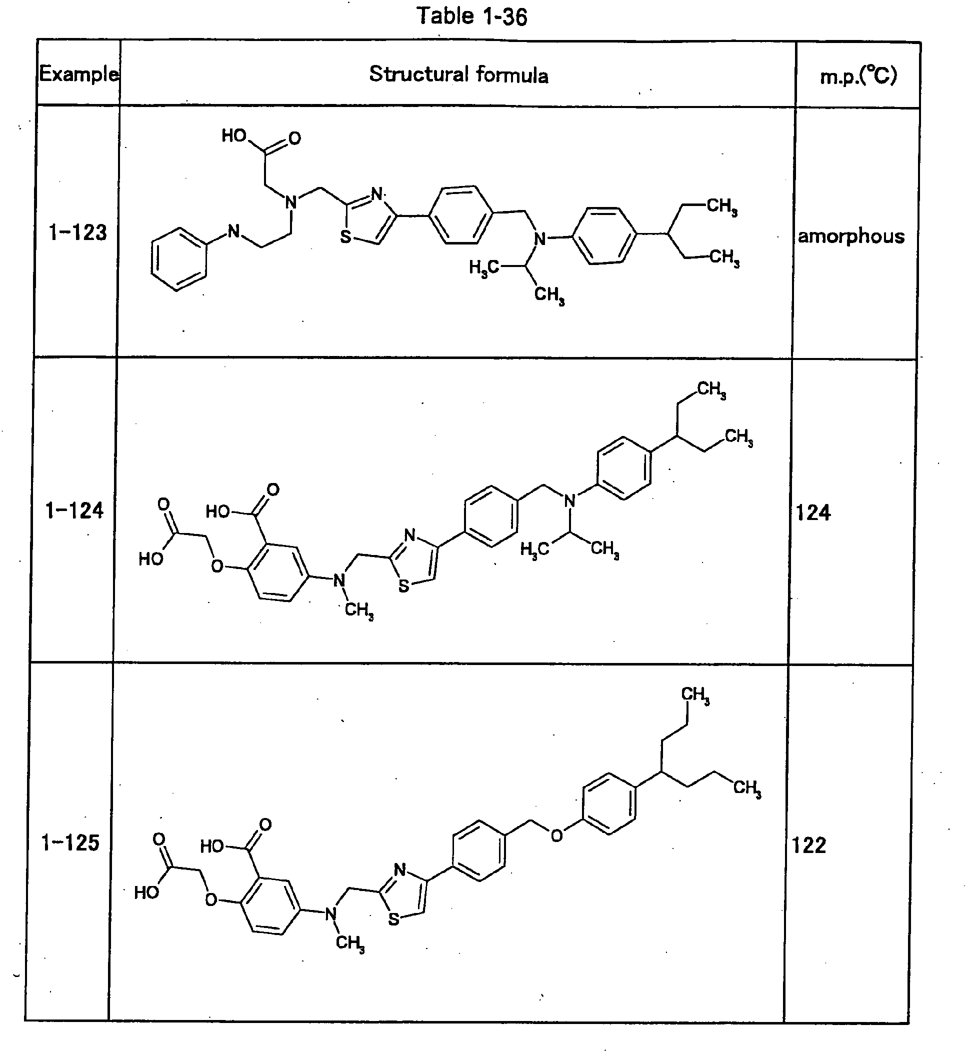

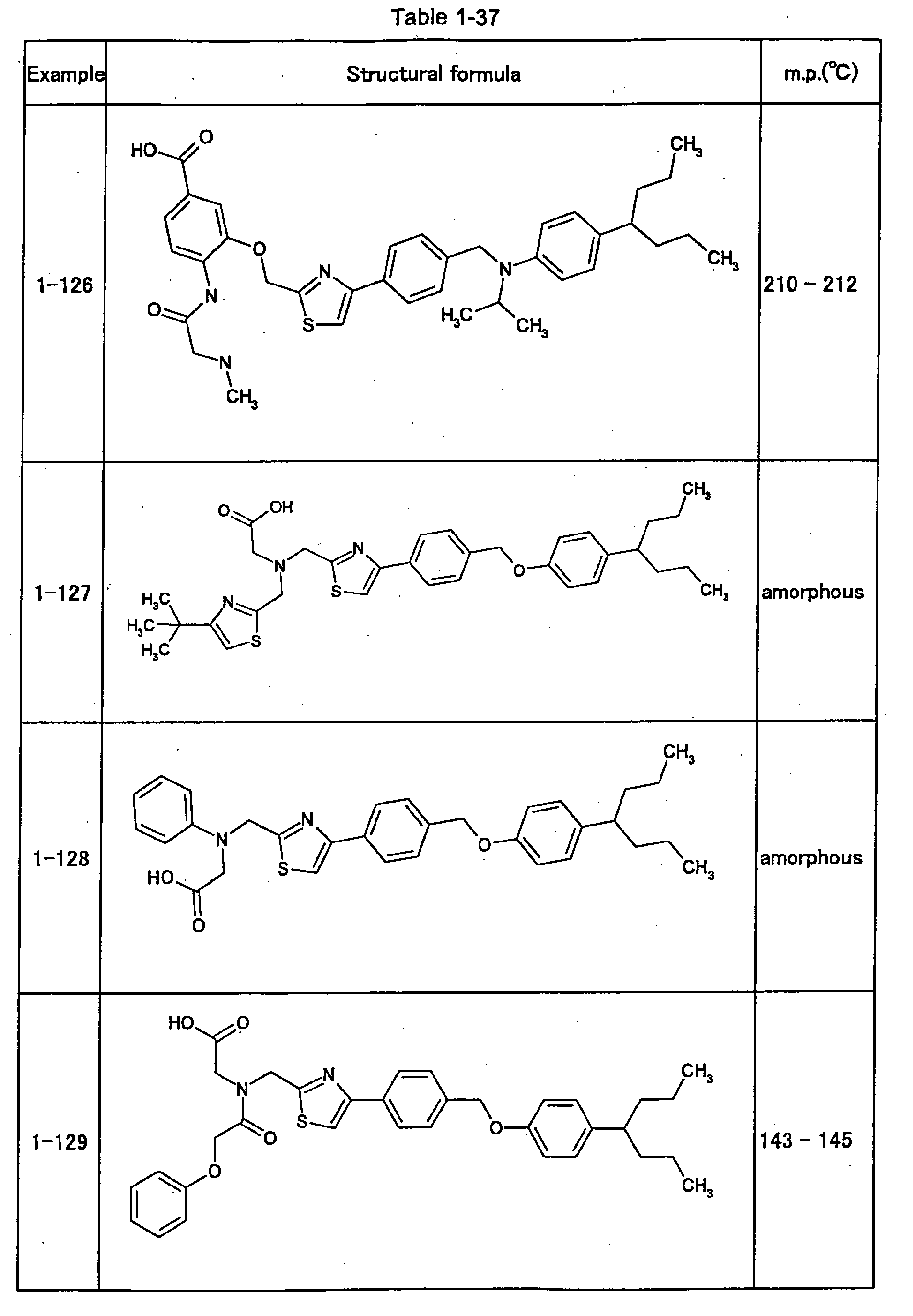

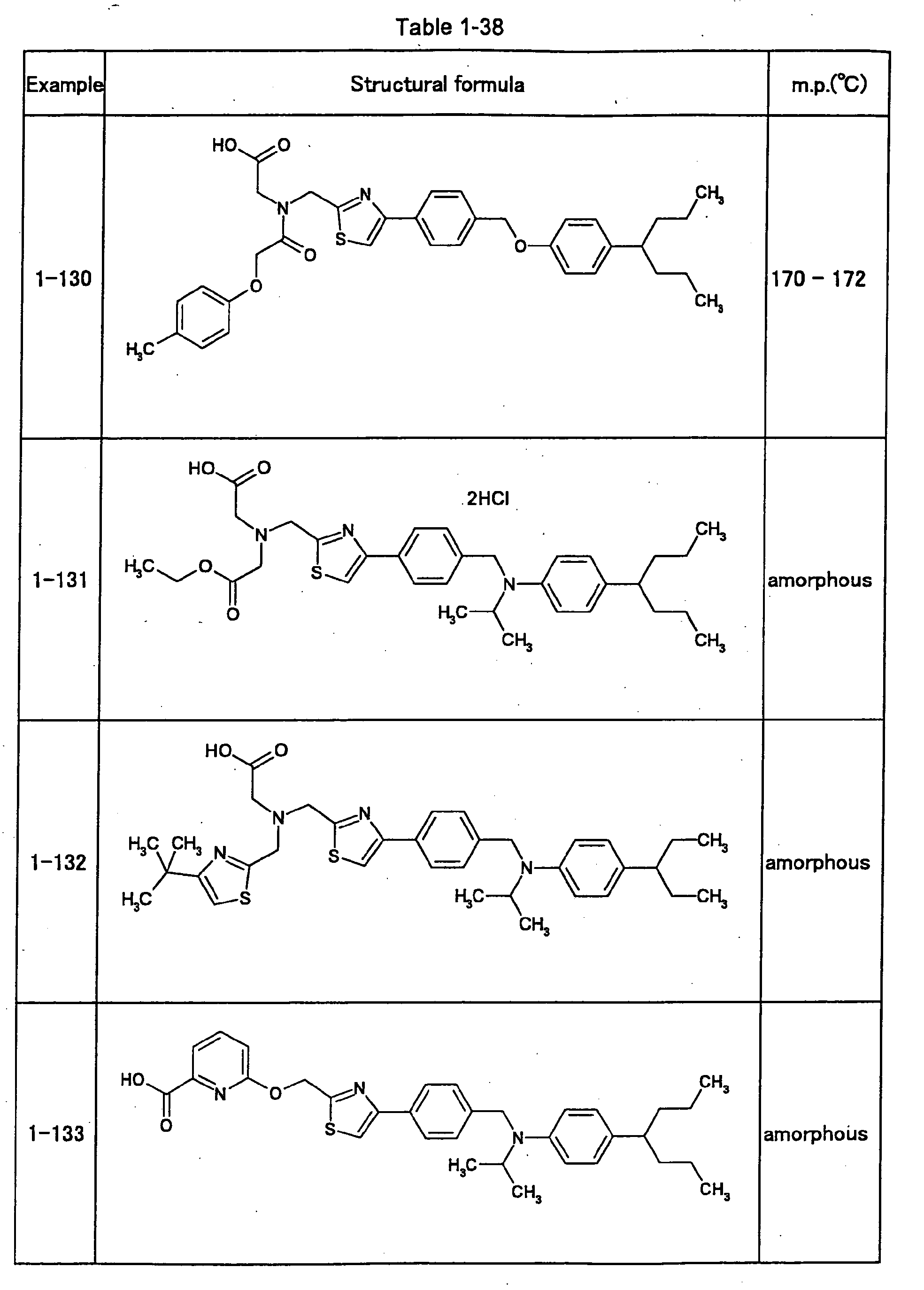

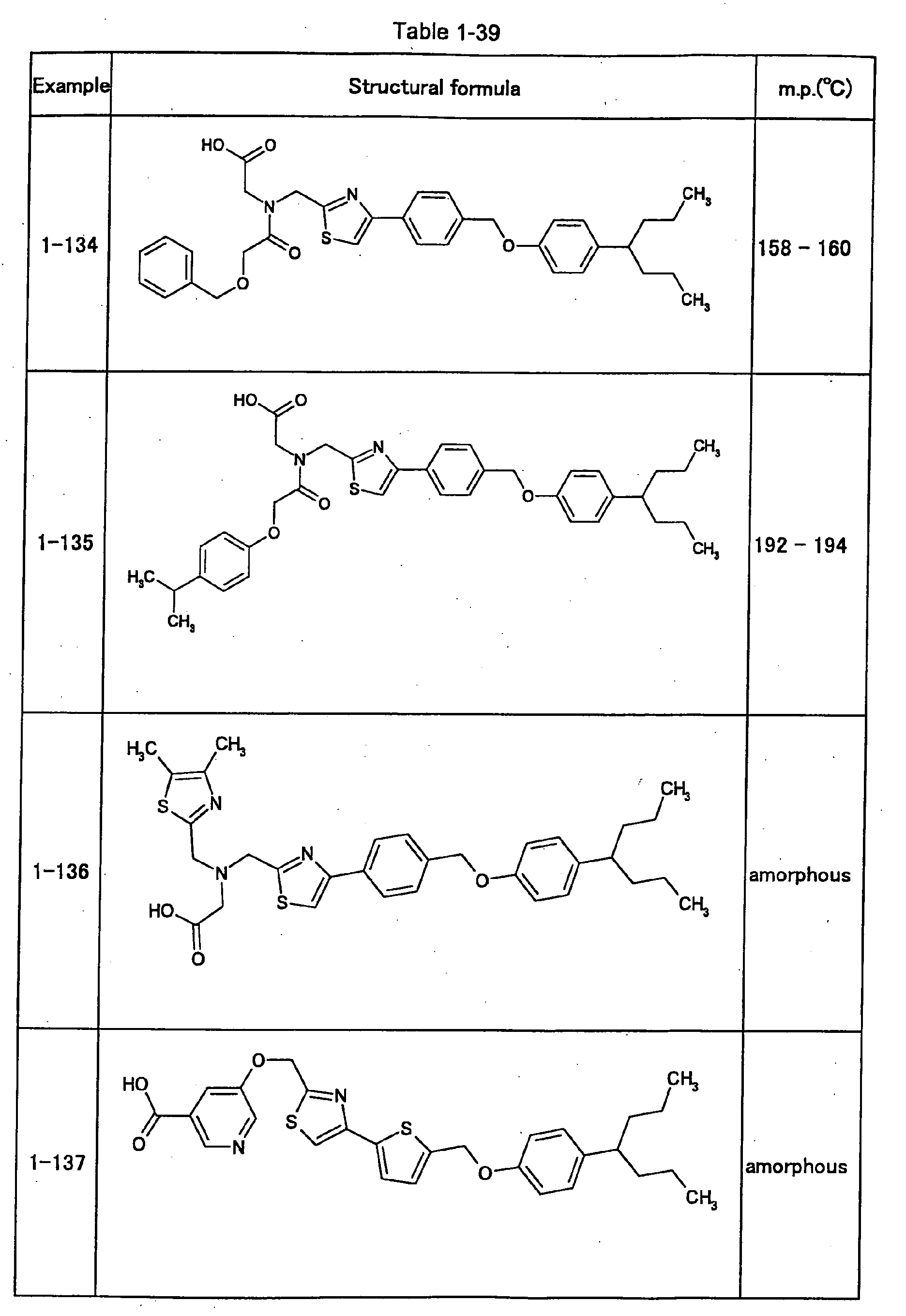

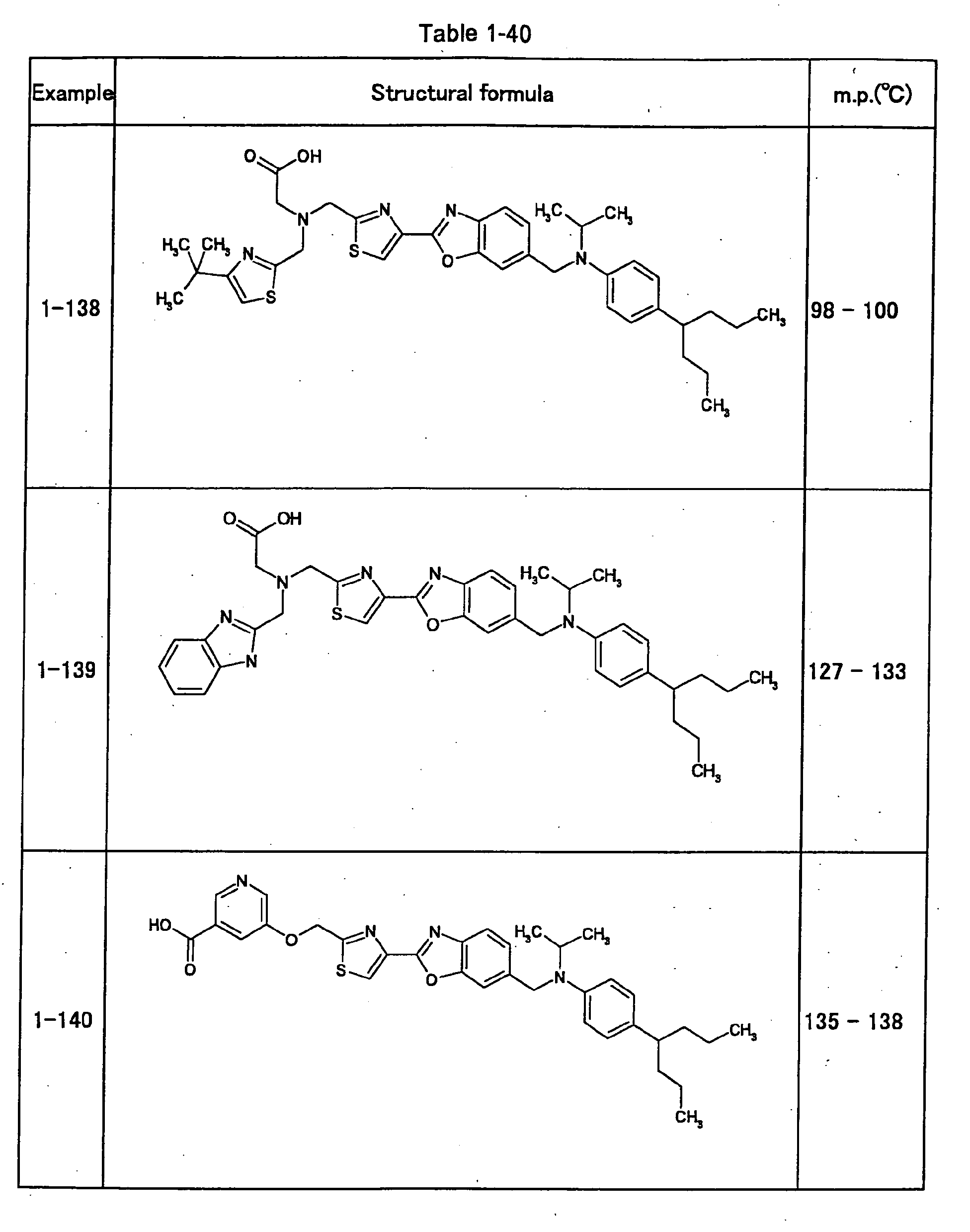

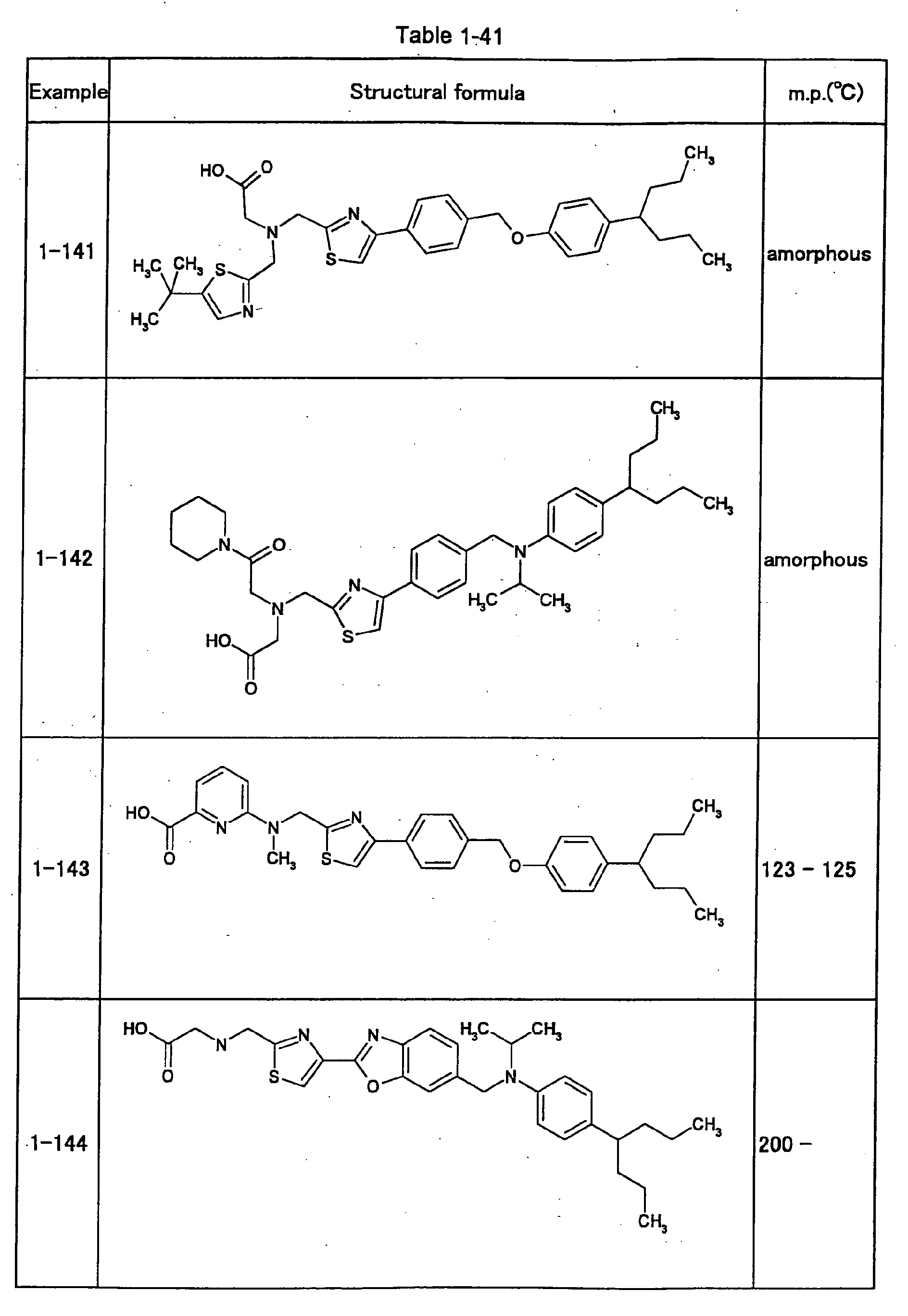

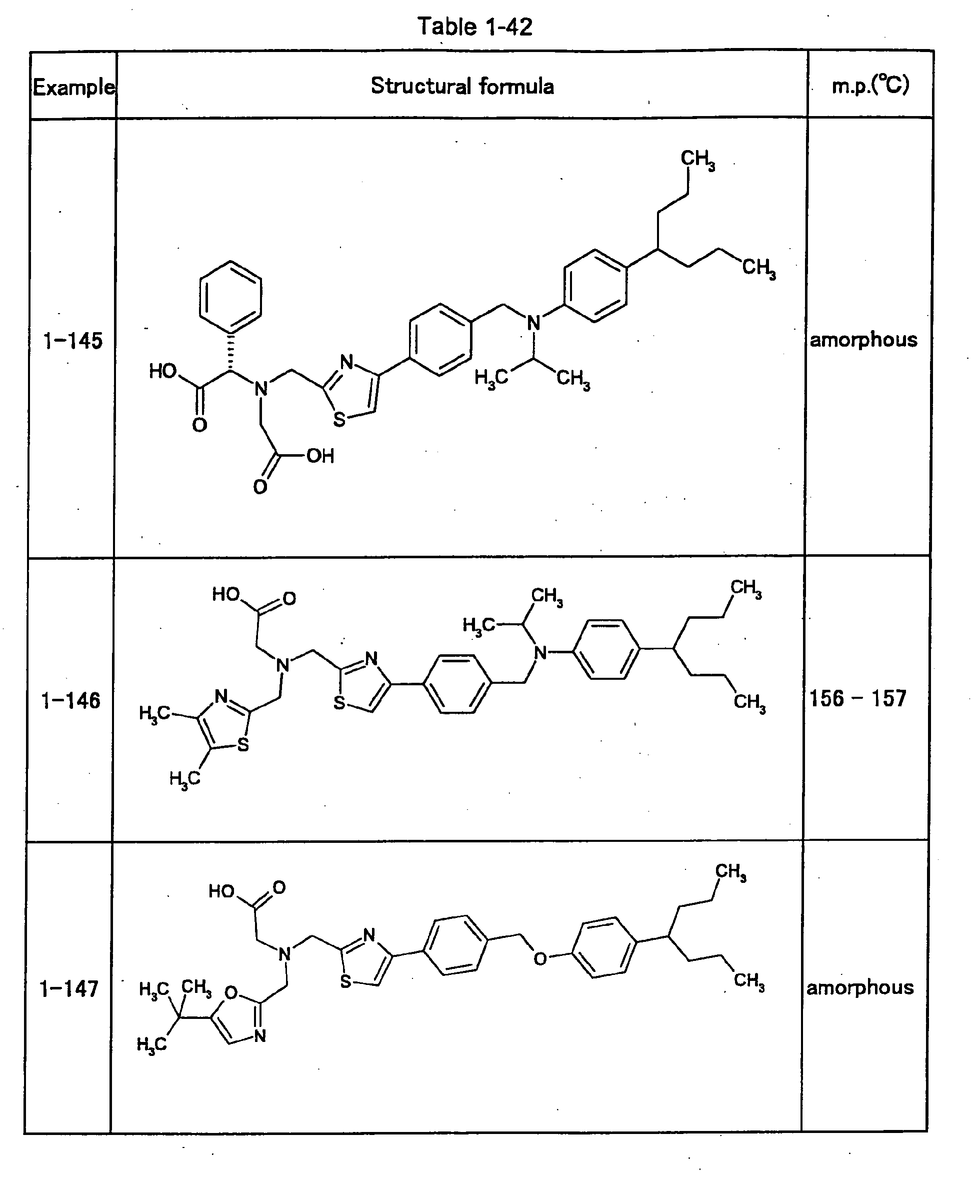

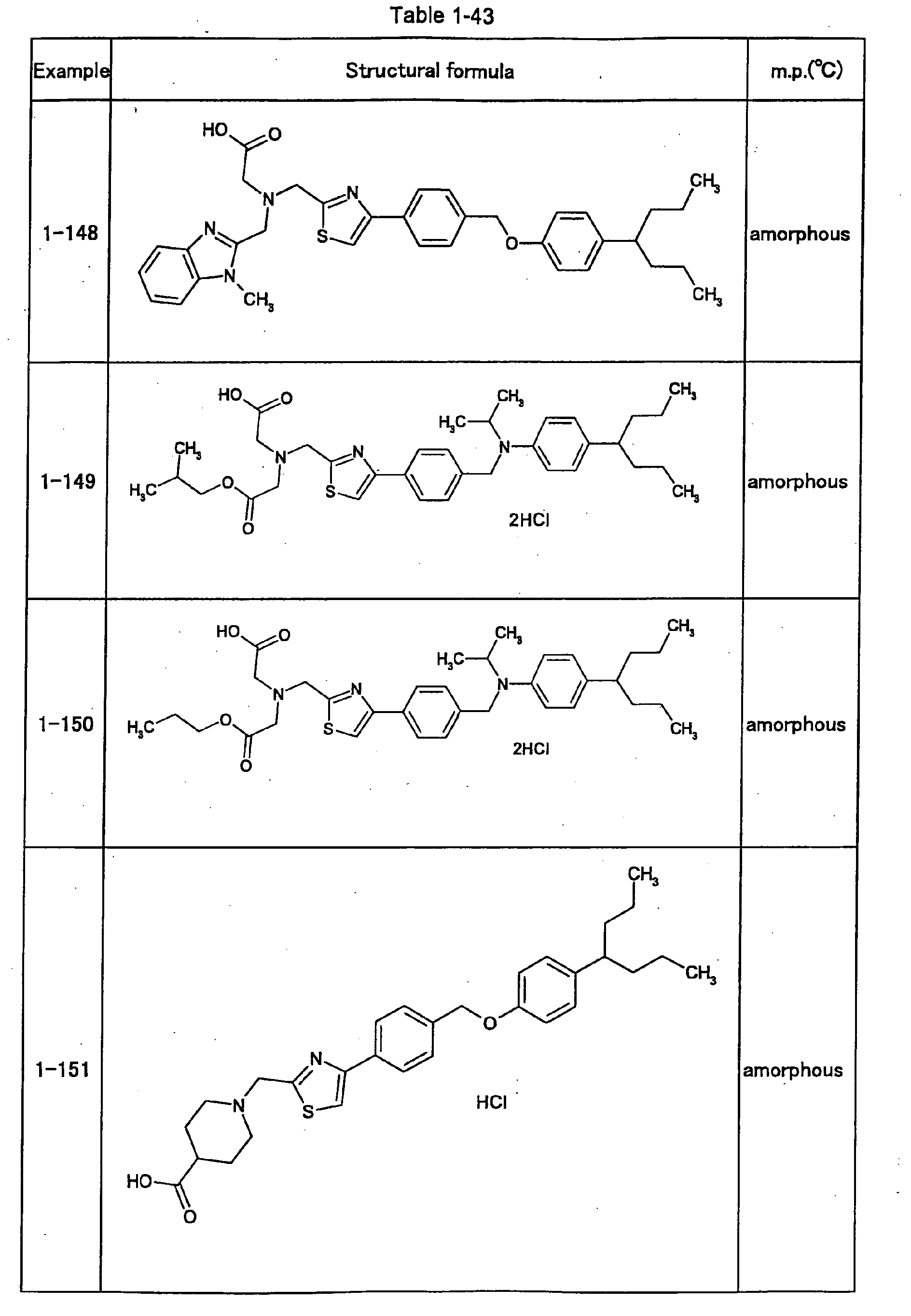

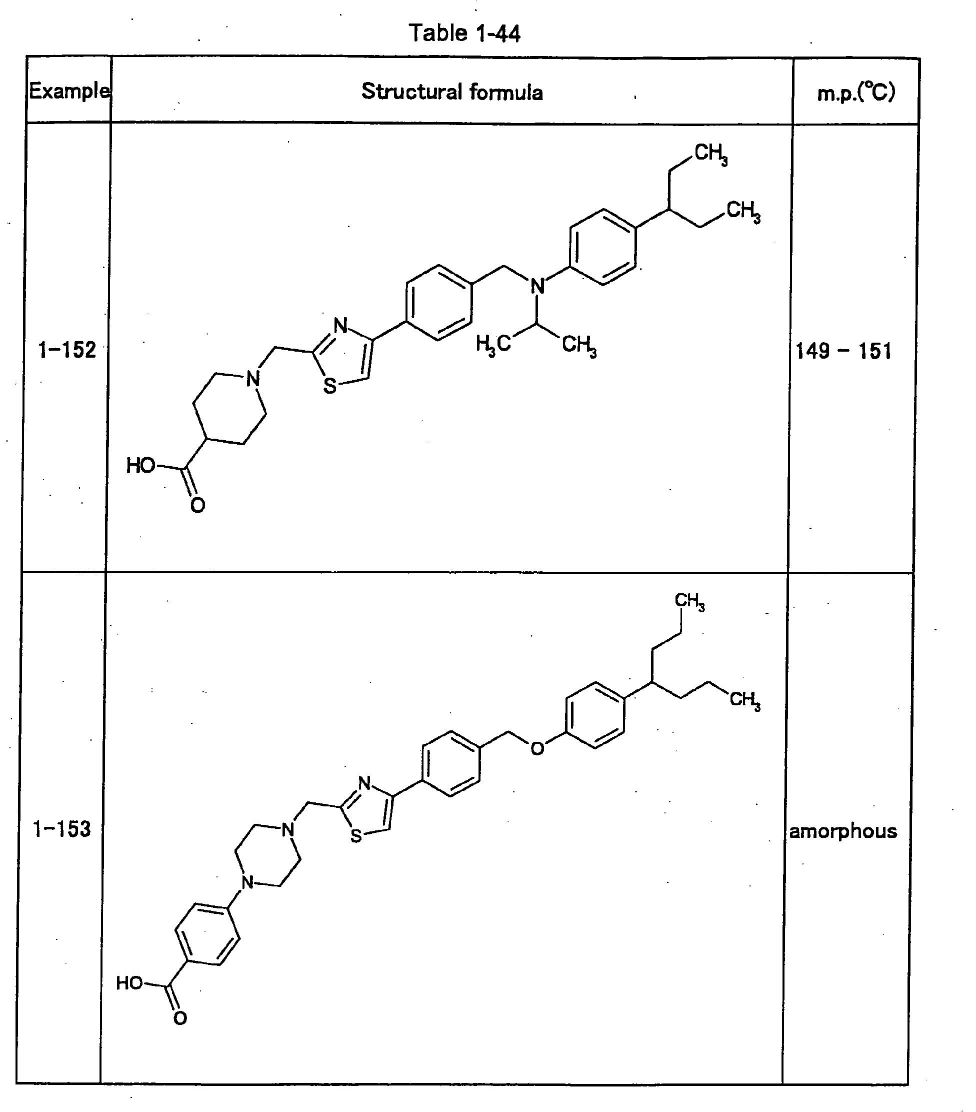

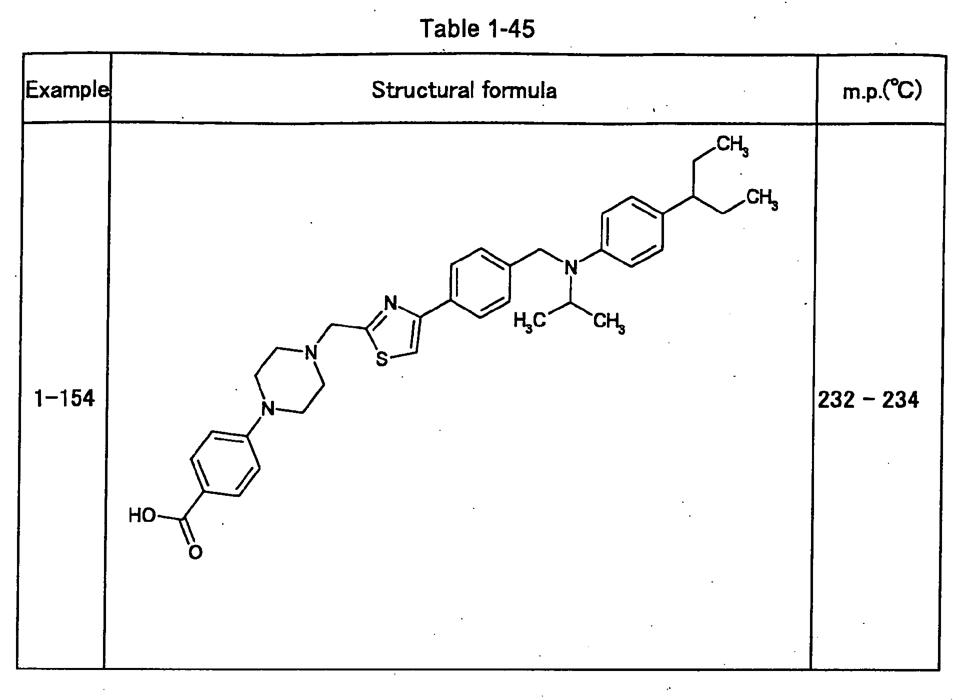

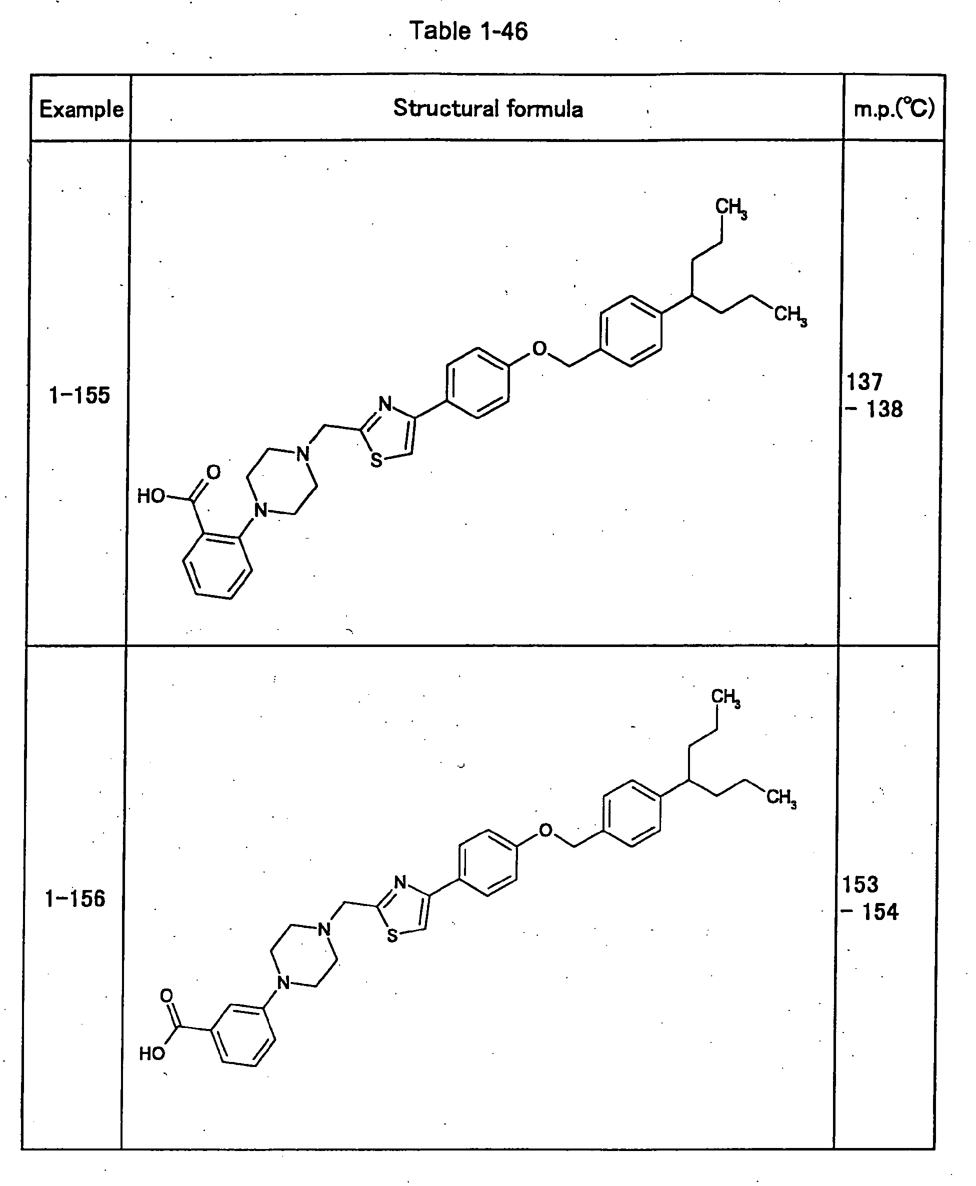

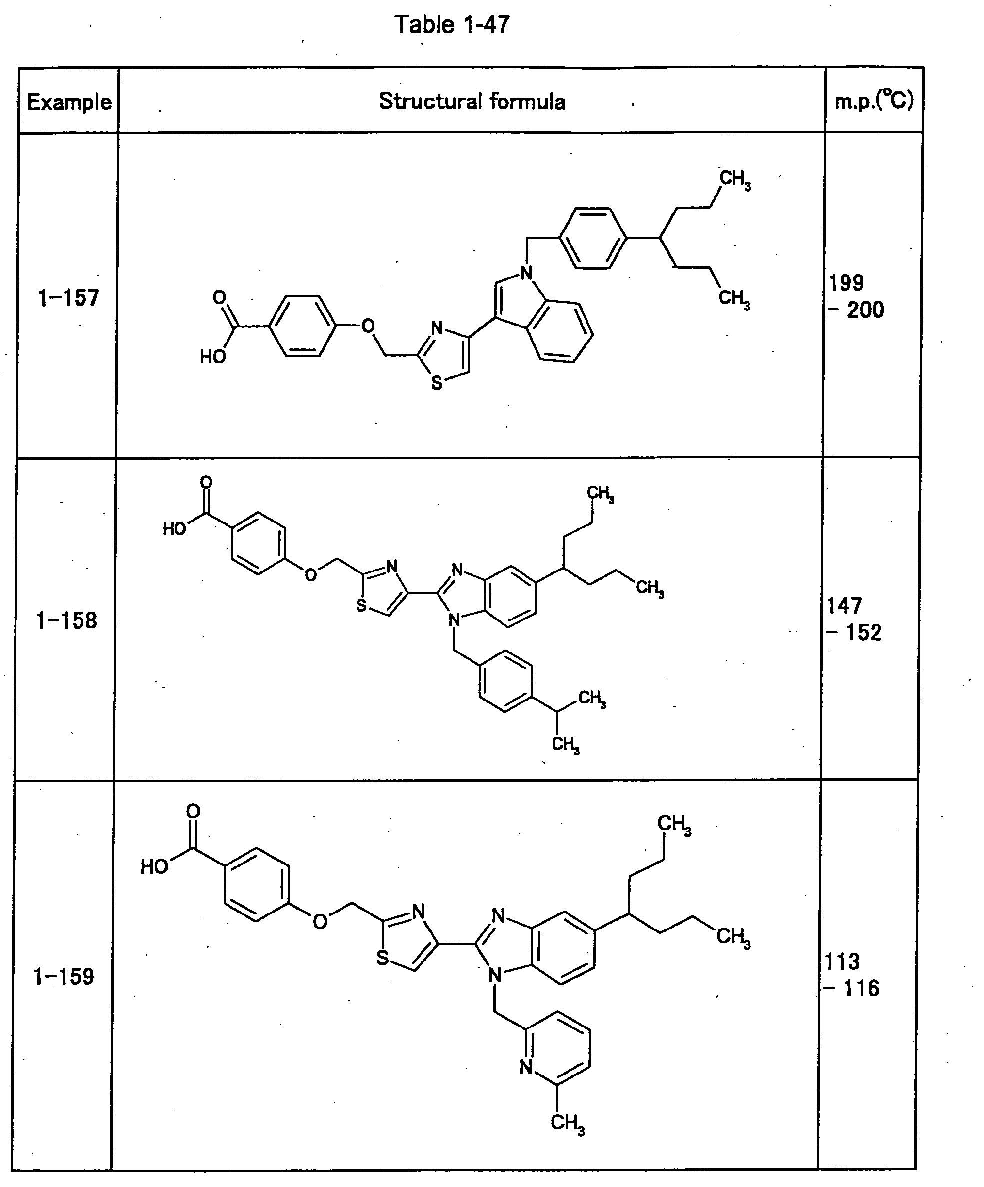

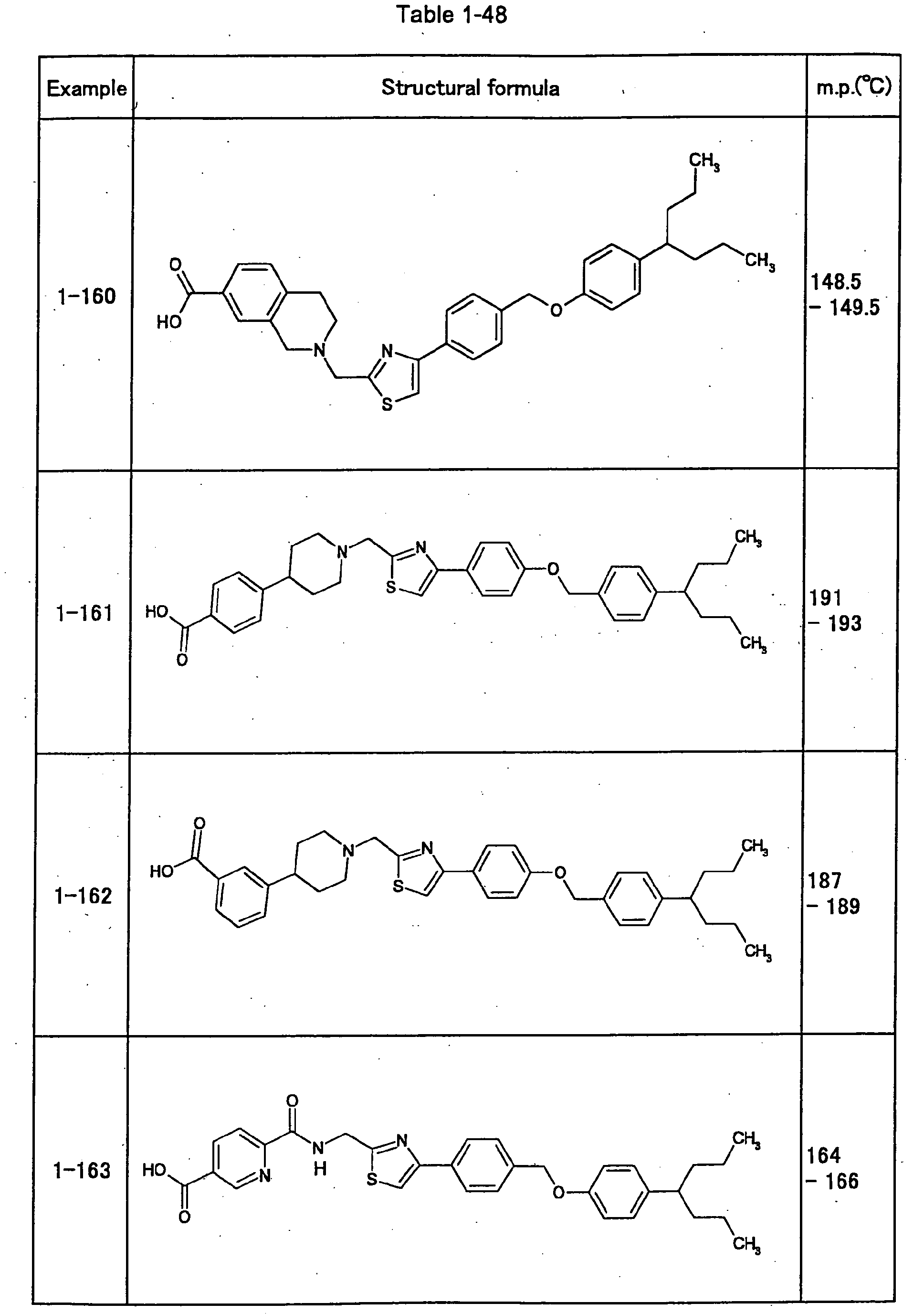

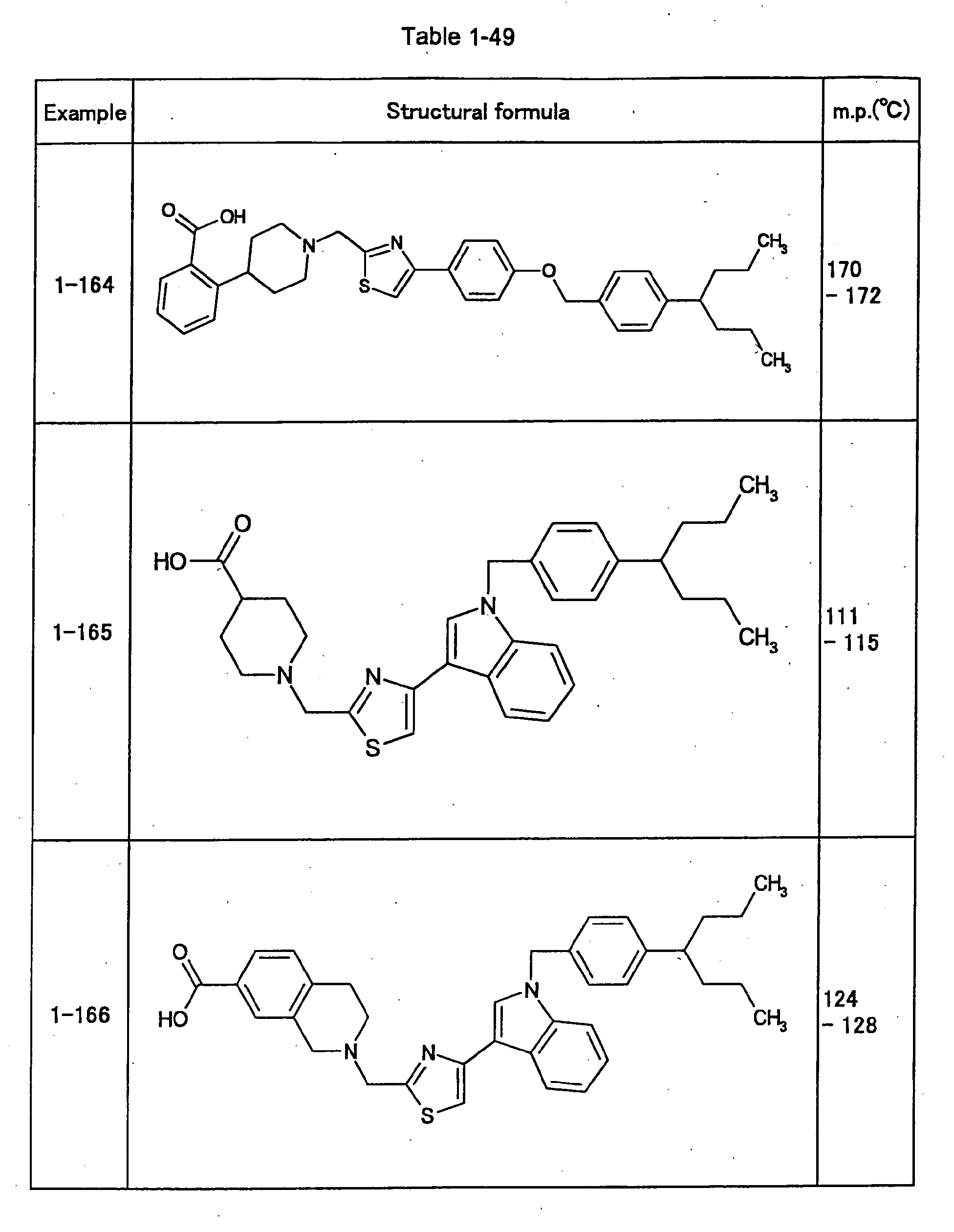

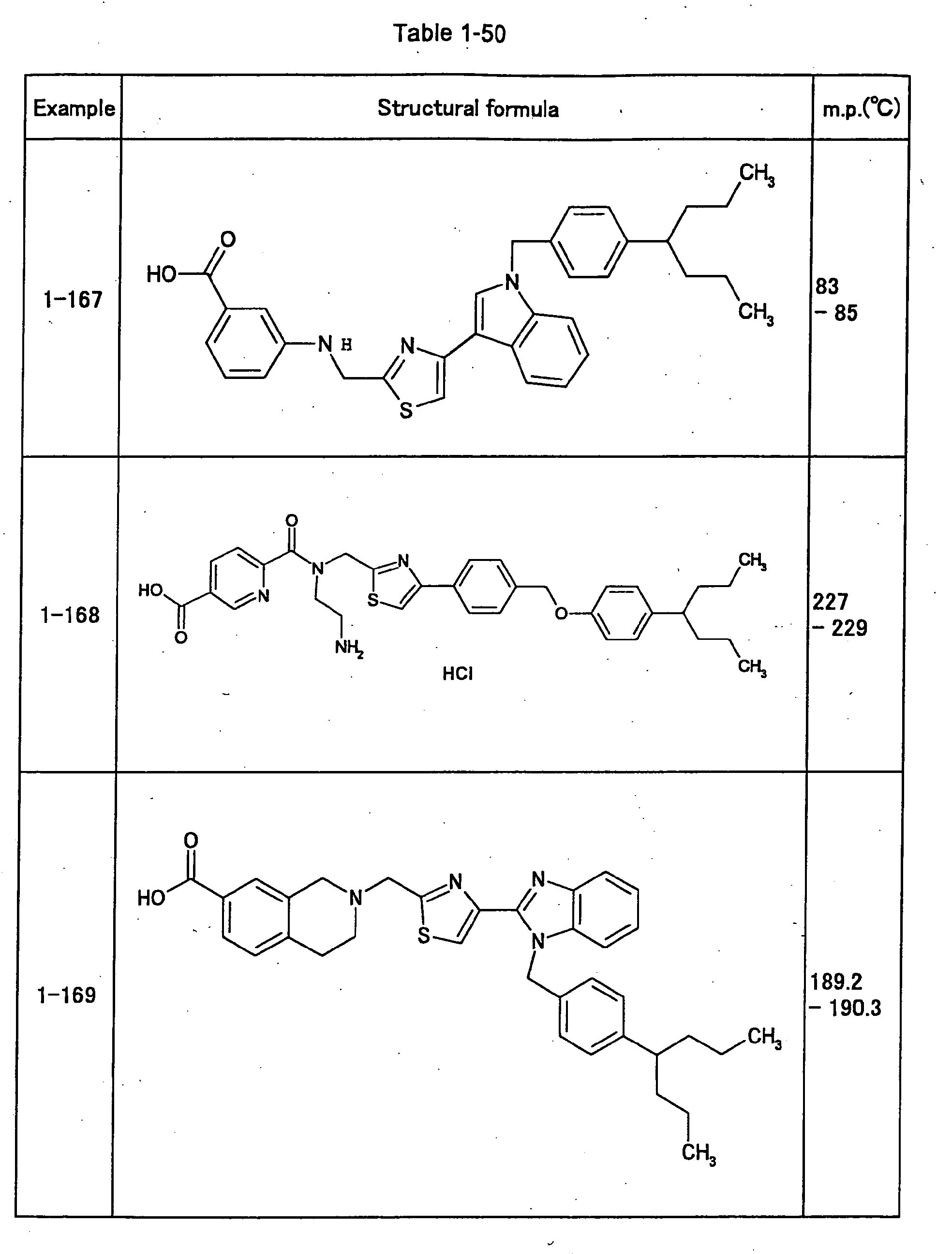

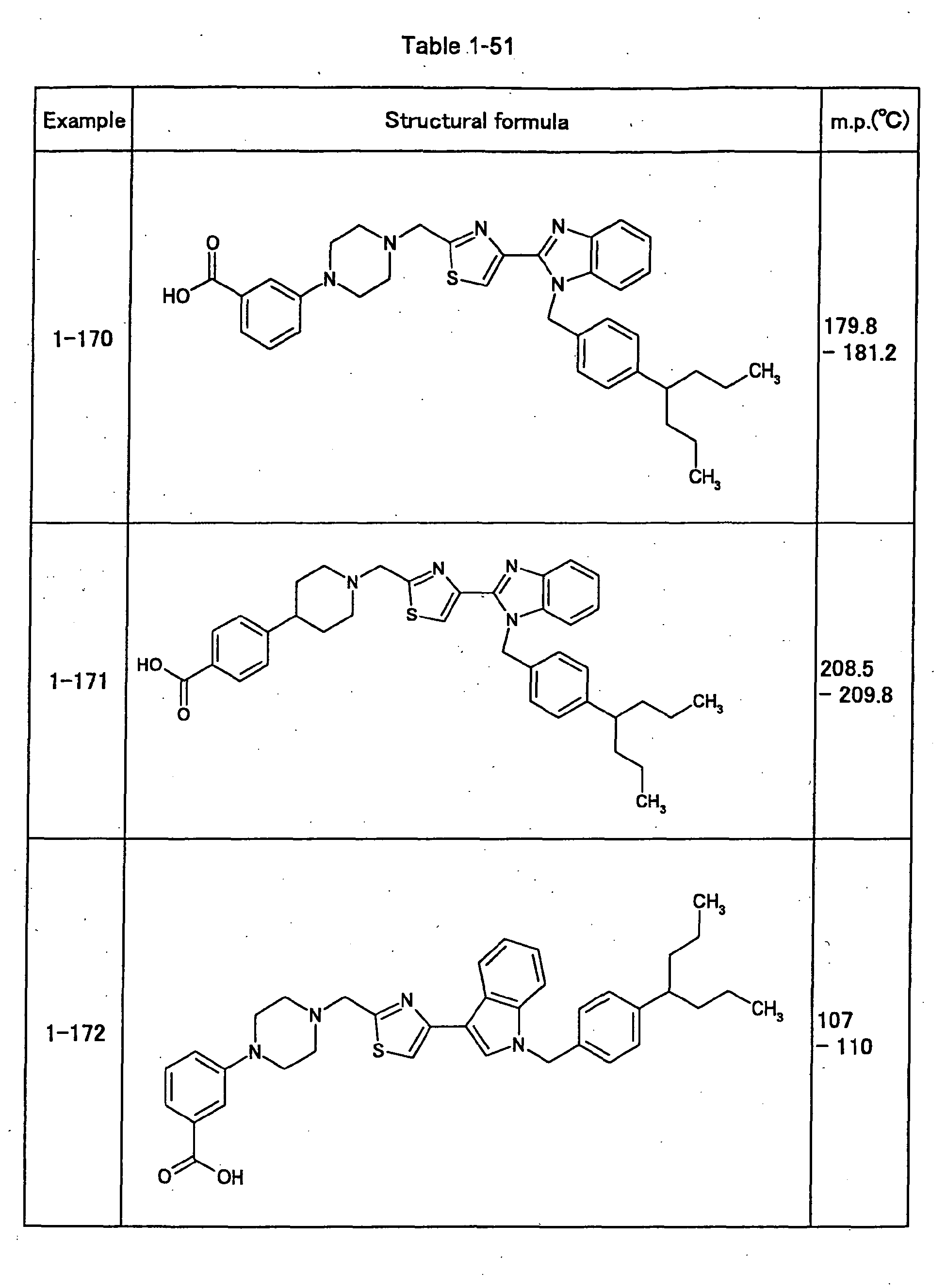

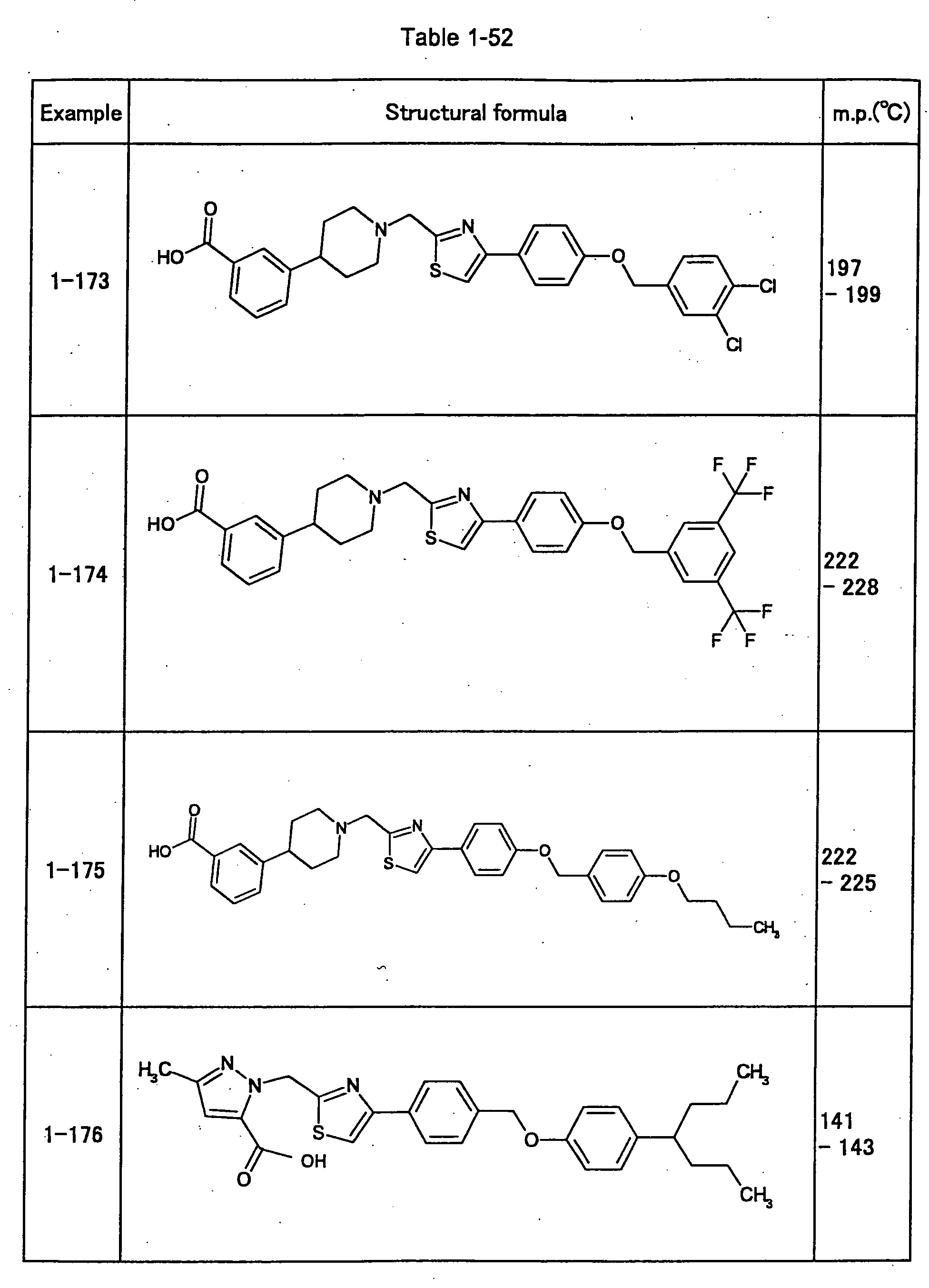

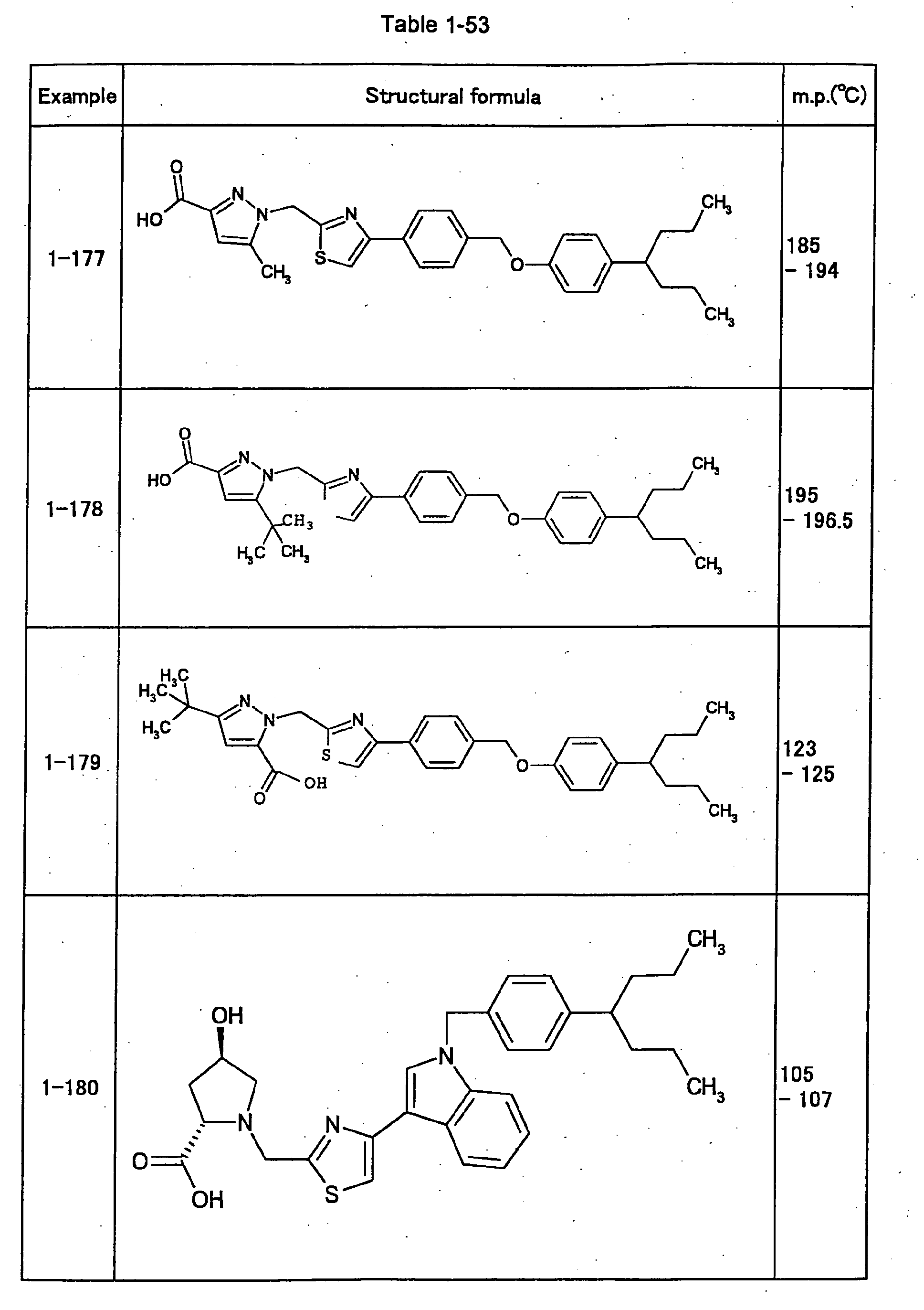

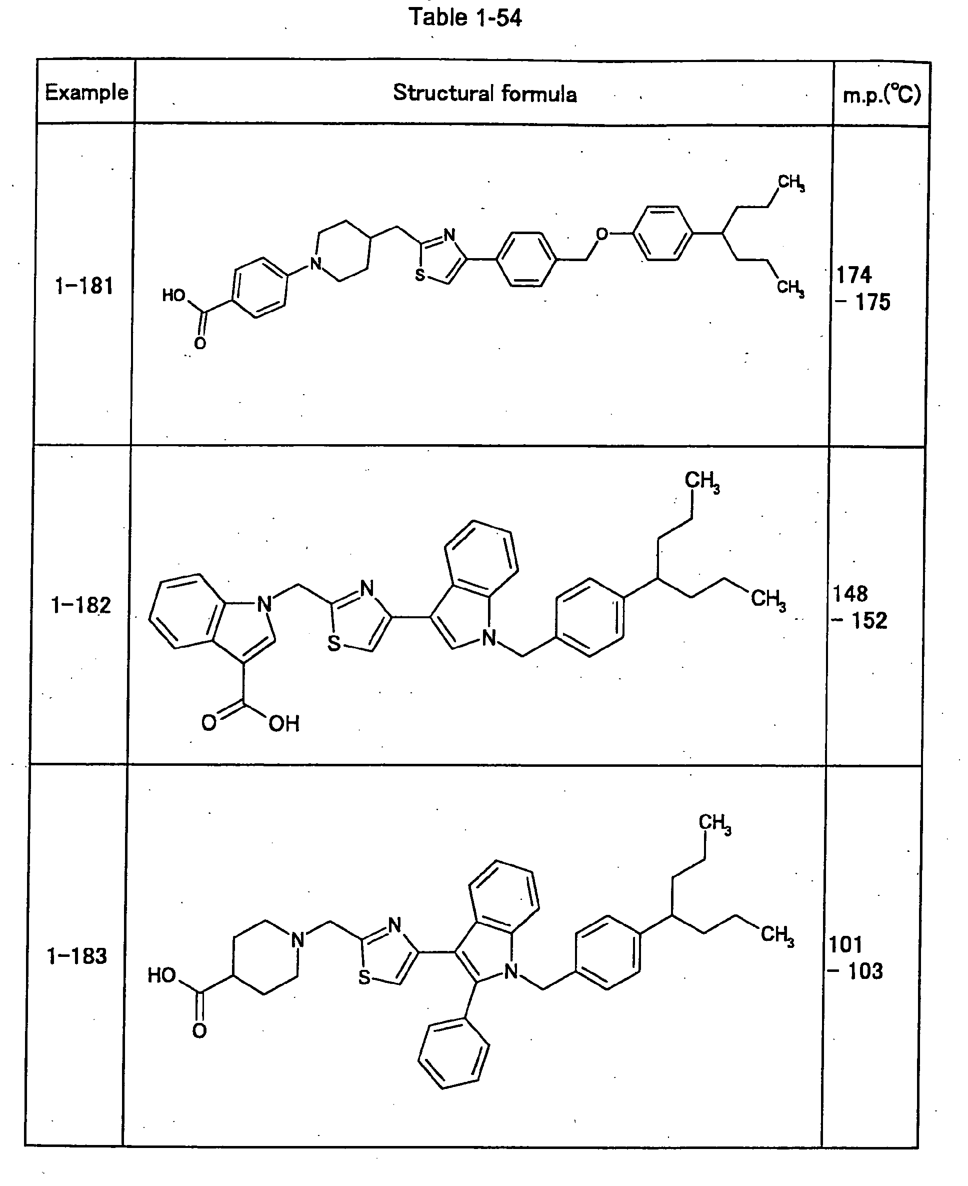

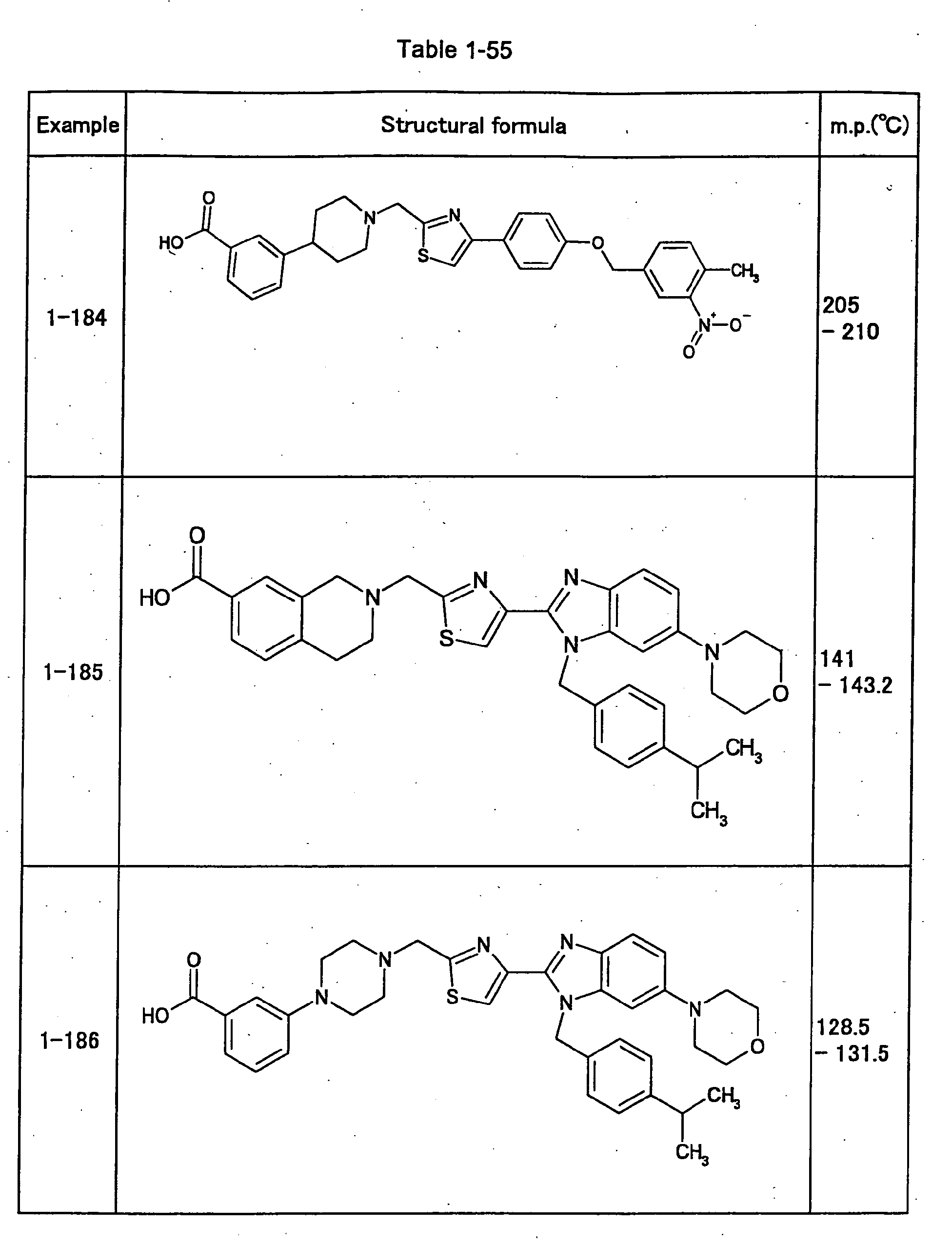

### Example 2-1

### {Benzyl-[4-(4-{methyl[4-(1-propylbutyl)benzyl]amino}phenyl)oxazol-2-ylmethyl]amino}acetic acid

(1) 4-(1-Propylbutyl)benzaldehyde 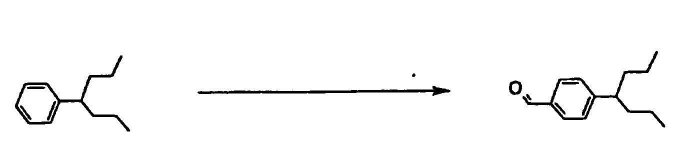
   To a solution of titanium tetrachloride (18.7 ml, 170 mmol) in chloroform (50 ml) was added dropwise a solution of (1-propylbutyl)benzene (10.0 g, 56.7 mmol) and dichloromethyl methyl ether (7.70 ml, 85.1 mmol) in chloroform (40 ml) under ice-cooling, and the mixture was stirred at the same temperature for 1 hr. The reaction mixture was poured into ice (100 g) and the mixture was stirred at room temperature for 1 hr. The organic layer was washed successively with water, a 0.5N aqueous sodium hydroxide solution, water and saturated brine and dried over magnesium sulfate. After filtration, the solvent was removed and the residue was purified by silica gel column chromatography (eluent; ethyl acetate-hexane 1:50) to give the title compound (10.0 g, yield 87%).
(2) N-Methyl-4-(1-propylbutyl)benzylamine hydrochloride 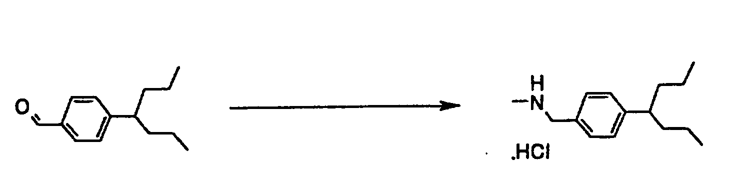
   To a solution of 4-(1-propylbutyl)benzaldehyde (1.00 g, 4.89 mmol) obtained in Example 2-1(1) in ethanol was added a solution (2 mol/l, 2.94 ml) of methylamine in methanol under ice-cooling, and the mixture was stirred at room temperature for 30 min. The solvent was removed and the residue was dissolved in tetrahydrofuran (10 ml). Sodium borohydride (285 mg, 7.53 mmol) was added under ice-cooling, and the mixture was stirred at room temperature for 1 hr. Ethanol was added and the mixture was stirred for 6 hr. The solvent was removed and chloroform (10 ml) was added to the residue. Water and 2N-hydrochloric acid were added under ice-cooling and the mixture was stirred at room temperature for 3 hr. To the organic layer, a saturated aqueous sodium hydrogen carbonate solution and di-tert-butyl dicarbonate (1.28 g, 5.87 mmol) were added. The mixture was stirred for 3 hr. After partitioning, the organic layer was washed with saturated brine and dried over sodium sulfate. The solvent was removed and the residue was purified by silica gel column chromatography (eluent; ethyl acetate-hexane 5:95) to give an oil.
   The obtained oil was dissolved in ethyl acetate (2 ml) and a 4N-hydrogen chloride - ethyl acetate solution (10 ml) was added and the mixture was stirred for 3 hr. Hexane (10 ml) was added and the precipitates were collected by filtration and dried to give the title compound (650 mg, yield 52%).
(3) (4-(4-Bromophenyl)oxazol-2-yl)methanol 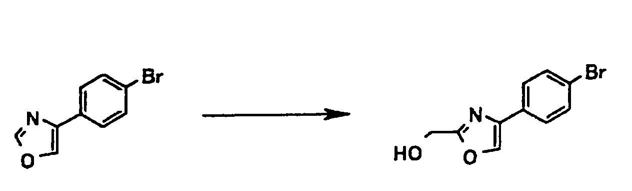
   Under an argon atmosphere, to a solution of 2,2,6,6-tetramethylpiperidine (227 mg, 1.61 mol) in tetrahydrofuran (3.0 ml, 13 v/w) was added n-butyllithium (1.56 M hexane solution, 0.944 ml, 1.47 mmol) at 0°C and the mixture was stirred for 30 min. After cooling to -78°C, 4-(4-bromophenyl)oxazole (300 mg, 1.34 mmol) was added and the mixture was stirred for 45 min. A suspension of paraformaldehyde (100 mg, 3.35 mmol) in tetrahydrofuran (1.5 ml, 15 v/w) was added and the mixture was stirred for 20 min. After heating to room temperature, a saturated aqueous ammonium chloride solution (5.0 ml) was added. The solvent was removed and the mixture was extracted with ethyl acetate (15 ml), washed with saturated brine (10 ml) and dried over sodium sulfate (1.0 g). The solvent was removed and the residue was purified by silica gel column chromatography (developing solvent, chloroform-chloroform:ethyl acetate=8:2) to give the title compound (210 mg, yield 62%).
(4) N-Benzyl-N-((4-(4-bromophenyl)oxazol-2-yl)methyl)glycine ethyl ester 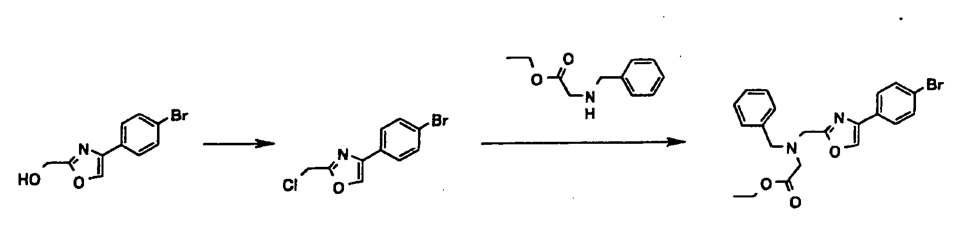
   Under an argon atmosphere, to a suspension of (4-(4-bromophenyl)oxazol-2-yl)methanol (200 mg, 0.787 mmol) obtained in Example 2-1(3) in chloroform (4.0 ml, 20 v/w) was added thionyl chloride (103 mg, 0.866 mmol) at 0°C. The mixture was stirred at room temperature for 1 hr and at 60°C for 1 hr. Thionyl chloride (103 mg, 0.866 mmol) was added at room temperature and the mixture was stirred for 12 hr. After removal of the solvent, acetonitrile (2.0 ml), N-benzylglycine ethyl ester (0.221 ml, 1.18 mmol), potassium carbonate (326 mg, 2.36 mmol) and potassium iodide (13 mg, 0.0787 mmol) were successively added. The mixture was stirred at 60°C for 1 hr. Distilled water (5.0 ml) was added at room temperature, and the mixture was extracted with a mixed solvent of hexane:ethyl acetate=1:1, washed with saturated brine (5.0 ml) and dried over sodium sulfate (1.0 g). The solvent was removed and the residue was purified by silica gel column chromatography (developing solvent; hexane:ethyl acetate=9:1) to give the title compound (220 mg, yield 65%).
(5) {Benzyl-[4-(4-{methyl-[4-(1-propylbutyl)benzyl]amino}phenyl)oxazol-2-ylmethyl]amino}acetic acid ethyl ester 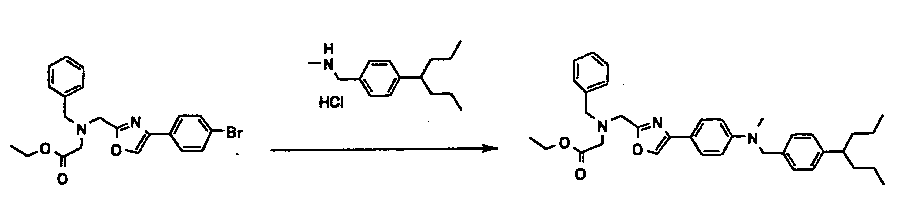
   Under an argon atmosphere, to a solution of N-benzyl-N-((4-(4-bromophenyl)oxazol-2-yl)methyl)glycine ethyl ester (200 mg, 0.466 mmol) obtained in Example 2-1(4) in dioxane (2.0 ml) were successively added N-methyl-4-(1-propylbutyl)benzylamine hydrochloride (131 mg, 0.512 mmol) obtained in Example 2-1(2), (R)-(+)-2,2'-bis(diphenylphosphino)-1,1'-binaphthyl (29.0 mg, 0.0466 mmol), cesium carbonate (334 mg, 1.03 mmol) and palladium acetate (5.20 mg, 0.0233 mmol). The mixture was stirred at 90°C for 18 hr and (R)-(+)-2,2'-bis(diphenylphosphino)-1,1'-binaphthyl (70.0 mg, 0.112 mmol) and palladium acetate (10.0 mg, 0.0445 mmol) were added. The mixture was stirred at 100°C for 3 hr and cesium carbonate (200 mg, 0.614 mmol) was added. The mixture was stirred for 12 hr. 4-Morpholinoaniline (83.0 mg, 0.466 mmol) was added and the mixture was stirred for 4 hr and passed through Celite at room temperature. The solvent was removed and the residue was purified by silica gel column chromatography (developing solvent; hexane:ethyl acetate=9:1) to give the title compound (35.0 mg, yield 13%).
(6) {Benzyl-[4-(4-{methyl-[4-(1-propylbutyl)benzyl]amino}phenyl)oxazol-2-ylmethyl]amino}acetic acid

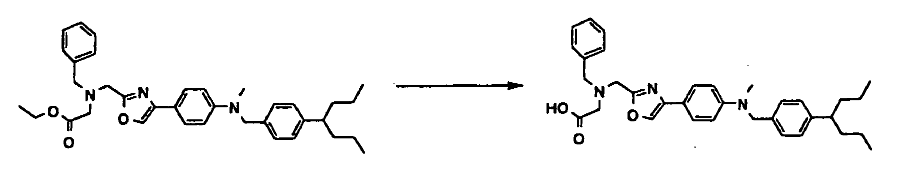

Under an argon atmosphere, to a solution of {benzyl[4-(4-{methyl[4-(1-propylbutyl)benzyl]amino}phenyl)oxazol-2-ylmethyl]amino}acetic acid ethyl ester (30.0 mg, 0.0528 mmol) obtained in Example 2-1(5) in tetrahydrofuran (0.50 ml) were added methanol (0.50 ml) and a 2N aqueous sodium hydroxide solution (0.0528 ml, 0.106 mmol). The mixture was stirred at 70°C for 1.5 hr and 1N hydrochloric acid (0.106 ml, 0.106 mmol) and distilled water (2.0 ml) were successively added at room temperature. The solvent was removed, and hexane (0.2 ml) and distilled water (2.0 ml) were successively added. The precipitates were collected by filtration, and dried to give the title compound (16.0 mg, yield 56%).
¹H NMR (DMSO-d₆, 300 MHz) δ 0.79(t, J=7.3Hz, 6H), 0.95-1.20 (m, 4H), 1.40-1.65(m, 4H), 2.40-2.60(m, 1H), 3.02(s, 3H), 3.81(s, 2H), 3.93(s, 2H), 4.57(s, 2H), 6.78(d, J=8.8Hz, 2H), 7.00-7.40(m, 9H), 7.55(d, J=8.8Hz, 2H), 8.30(s, 1H), 12.30(br s, 1H).
melting point: 129°C-131°C

The structural formula and property value of Example 2-1 is shown in the following Table 2.

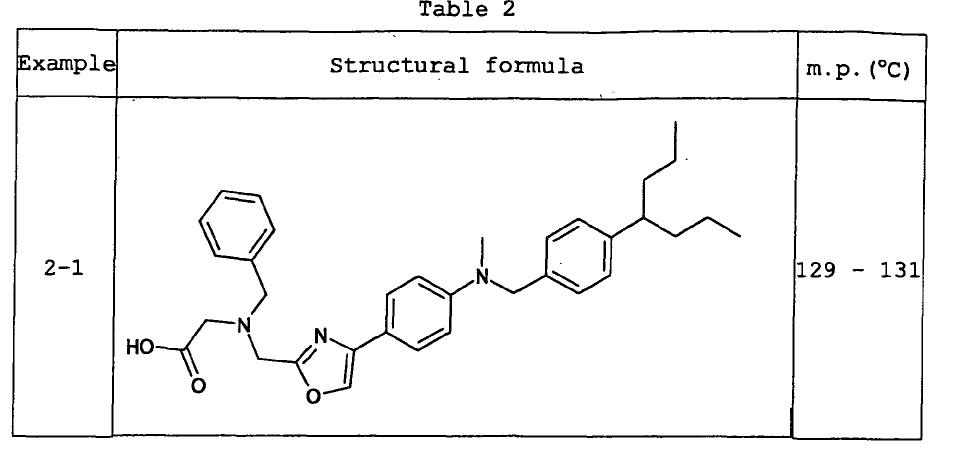

### Example 3-1

### 5-(4-{4-[4-(1-Propylbutyl)benzyloxy]phenyl}thiazol-2-ylmethoxy)nicotinic acid

(1) (4-(4-Hydroxyphenyl)thiazol-2-yl)methyl benzoate 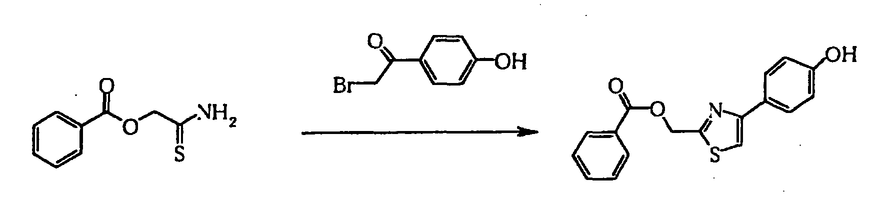
   To a solution of 2-(benzoyloxy)ethanethioamide (4 g, 20.488 mmol) and sodium hydrogen carbonate (1.72 g, 20.488 mmol) in N,N-dimethylacetamide (4 ml) was added dropwise a solution of 2-bromo-4'-hydroxyacetophenone (5 g, purity 88%, 20.488 mmol) in N,N-dimethylacetamide (8 ml) at room temperature, and the mixture was stirred at the same temperature for 2 hr. After the completion of the reaction, ethanol and water were added and the precipitates were collected by filtration and washed with 50% ethanol. The obtained solid was dried under reduced pressure to give the title compound (5.336 g, 84.1%).
(2) (4-(4-(4-(1-Propylbutyl)benzyloxy)phenyl)thiazol-2-yl)methyl benzoate 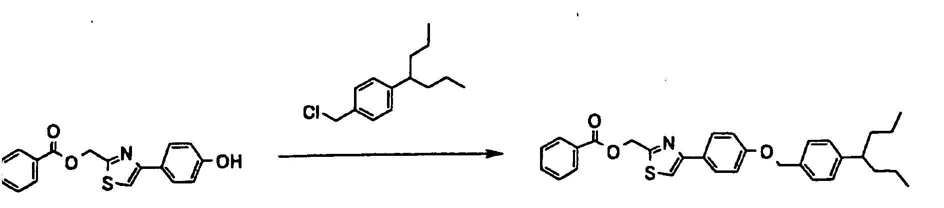
   To a solution of (4-(4-hydroxyphenyl)thiazol-2-yl)methyl benzoate (14.0 g, 44.9 mmol) obtained in Example 3-1(1) in N,N-dimethylacetamide (70 ml) were added 4-(1-propylbutyl)benzyl chloride (10.1 g, 44.9 mmol), potassium carbonate (12.4 g, 89.9 mmol) and potassium iodide (75 mg, 0.45 mmol) and the mixture was stirred at 80°C for 3 hr. After allowing to cool, ethyl acetate was added and the mixture was washed successively with water and saturated brine and dried over magnesium sulfate. After filtration, the solvent was removed and ethanol (120 ml) was added. The mixture was heated to 50°C. After allowing to cool, the precipitated crystals were collected by filtration and dried to give the title compound (19.5 g, yield 87%).
(3) (4-(4-(4-(1-Propylbutyl)benzyloxy)phenyl)thiazol-2-yl)methanol 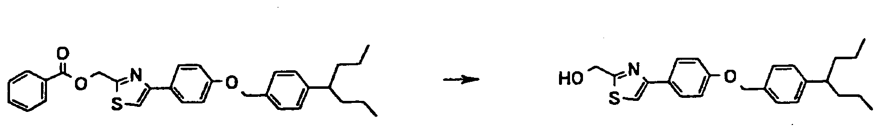
   To (4-(4-(4-(1-propylbutyl)benzyloxy)phenyl)thiazol-2-yl)methyl benzoate (26.5 g, 53.1 mmol) obtained in Example 3-1(2) were added methanol (159 ml), tetrahydrofuran (27 ml) and a 2N-aqueous sodium hydroxide solution (39.8 ml, 79.6 mmol) and the mixture was stirred at 80°C for 1 hr. The solvent was removed and water (265 ml) was added. The mixture was stirred at room temperature for 1 hr. The crystals were collected by filtration, dissolved in toluene (100 ml) and ethyl acetate (10 ml). The mixture was washed with saturated brine and dried over magnesium sulfate and filtered and the solvent was removed to give the title compound (20.5 g, yield 98%).
(4) (4-(4-(4-(1-Propylbutyl)benzyloxy)phenyl)thiazol-2-yl)methyl chloride 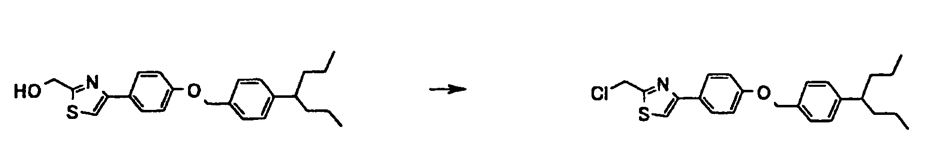
   To a solution of (4-(4-(4-(1-propylbutyl)benzyloxy)phenyl)thiazol-2-yl)methanol (20.5 g, 51.8 mmol) obtained in Example 3-1(3) in chloroform (103 ml) was added thionyl chloride (9.17 g, 77.7 mmol) and the mixture was stirred at room temperature for 1 hr. The solvent was removed to give the title compound (21.9 g, quant.).
(5) 5-(4-{4-[4-(1-Propylbutyl)benzyloxy]phenyl}thiazol-2-ylmethoxy)nicotinic acid methyl ester 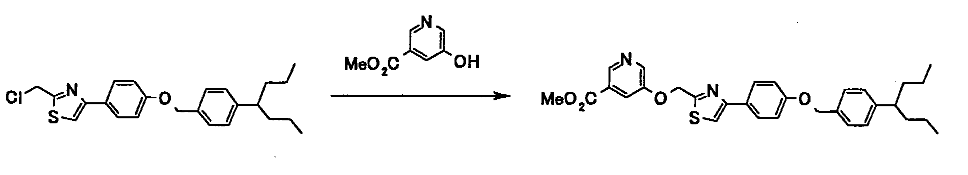
   N,N-Dimethylacetamide (104 ml), methyl 5-hydroxynicotinate (5.25 g, 40.8 mmol), potassium carbonate (13.0 g, 94.2 mmol) and potassium iodide (520 mg, 3.1 mmol) were added to (4-(4-(4-(1-propylbutyl)benzyloxy)phenyl)thiazol-2-yl)methyl chloride (13.0 g, 31.4 mmol) obtained in Example 3-1(4) and the mixture was stirred at 75°C for 2 hr. After allowing to cool, ethyl acetate was added and the mixture was washed successively with water, a saturated aqueous sodium hydrogen carbonate solution, water and saturated brine, and dried over magnesium sulfate. After filtration, the solvent was removed and the residue was purified by silica gel column chromatography (eluent; toluene-ethyl acetate, 5:1). The obtained crude crystals were slurried for washing with ether to give the title compound (9.91 g, yield 59%).
(6) 5-(4-{4-[4-(1-Propylbutyl)benzyloxy]phenyl}thiazol-2-ylmethoxy)nicotinic acid

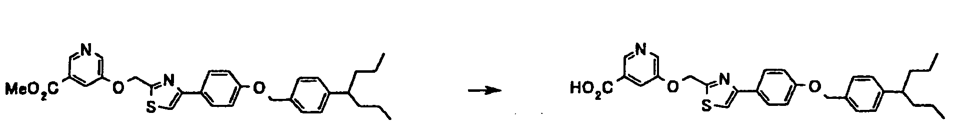

To 5-(4-{4-[4-(1-propylbutyl)benzyloxy]phenyl}thiazol-2-ylmethoxy)nicotinic acid methyl ester (9.50 g, 17.9 mmol) obtained in Example 3-1(5) were added tetrahydrofuran (62.5 ml), ethanol (62.5 ml) and a 2N-aqueous sodium hydroxide solution (17.9 ml, 35.8 mmol), and the mixture was stirred at 70°C for 2.5 hr. 2N-Hydrochloric acid (17.9 ml, 35.8 mmol) was added under ice-cooling and the mixture was concentrated under reduced pressure. Ethanol (19 ml) and water (9.5 ml) were added, and after stirring, the crystals were collected by filtration and dried to give the title compound (9.15 g, yield 99%).
¹H NMR (DMSO-d₆, 400 MHz) δ 0.81(t, J=7.3Hz, 6H), 1.05-1.13(m, 4H), 1.48-1.57(m, 4H), 2.5-2.56(m, 1H), 5.09(s, 2H), 5.66(s, 2H), 7.09(d, J=9.04Hz, 2H), 7.19(d, J=8.32Hz, 2H), 7.38(d, J=8.12Hz, 2H), 7.91(d, J=9.04Hz, 2H), 7.98-7.99(m, 1H), 8.03(s, 1H), 8.64(d, J=3Hz, 1H), 8.72(d, J=1.4Hz, 1H), 13.52(s, 1H)
melting point: 172-174°C

### Examples 3-2 to 3-132

In the same manner as in Example 3-1 and using other conventional methods where necessary, the compounds of Examples 3-2 to 3-132 were produced. The structural formulas and property values of the obtained compounds, as well as those of Example 3-1, are shown in the following Tables.

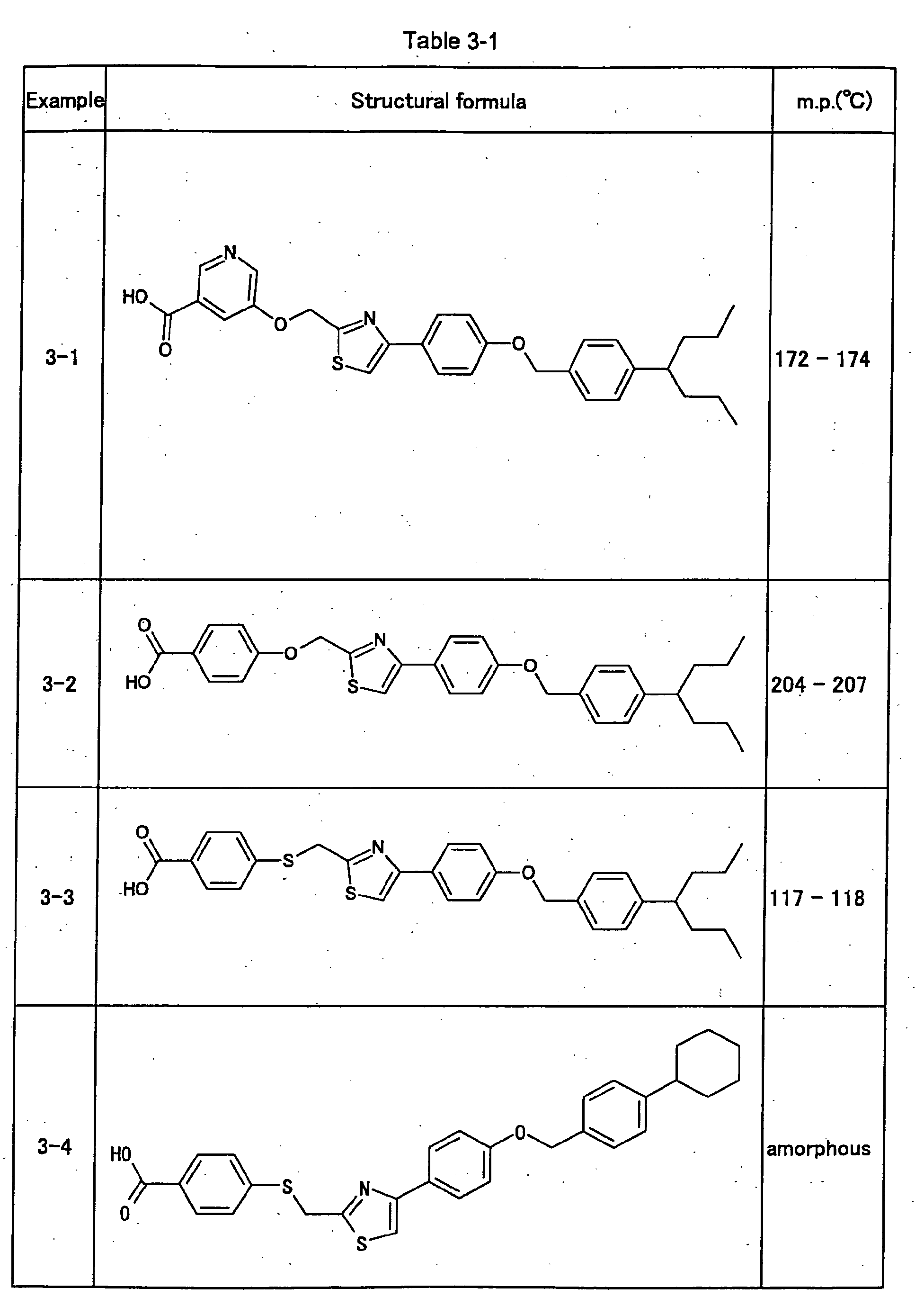

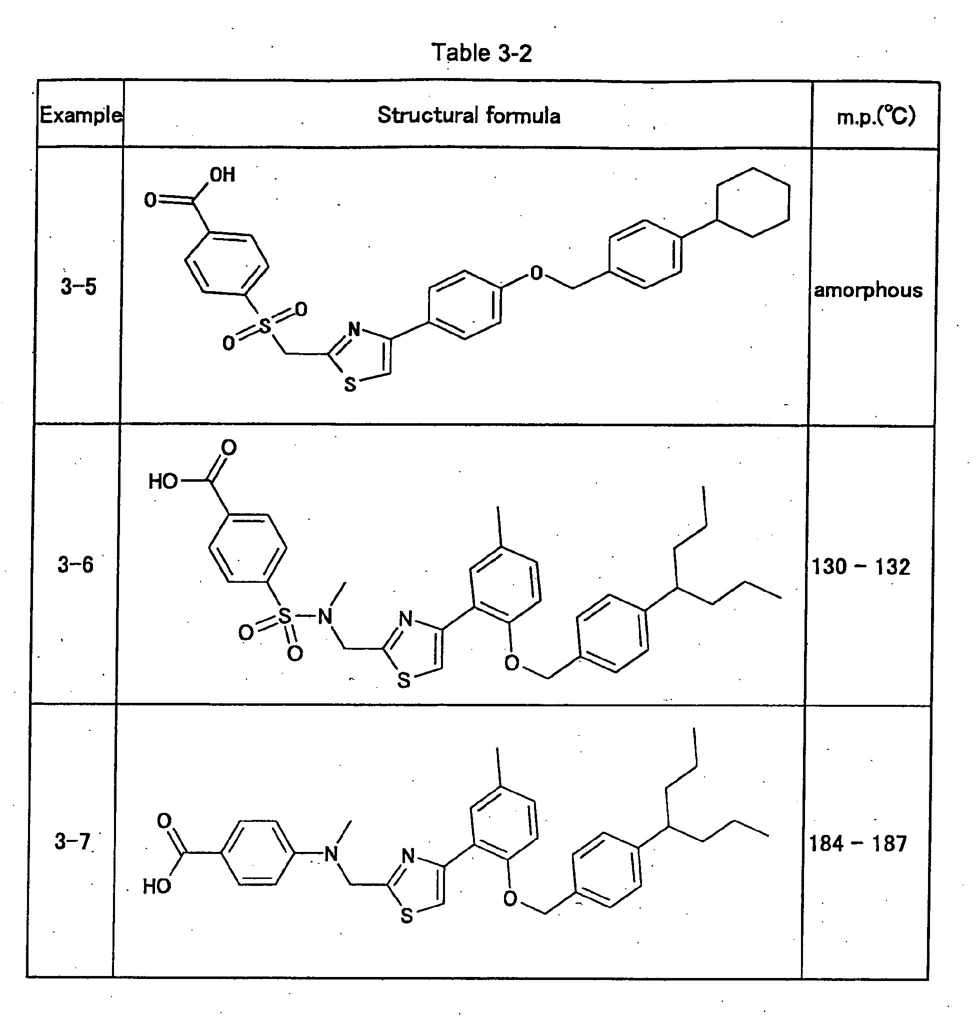

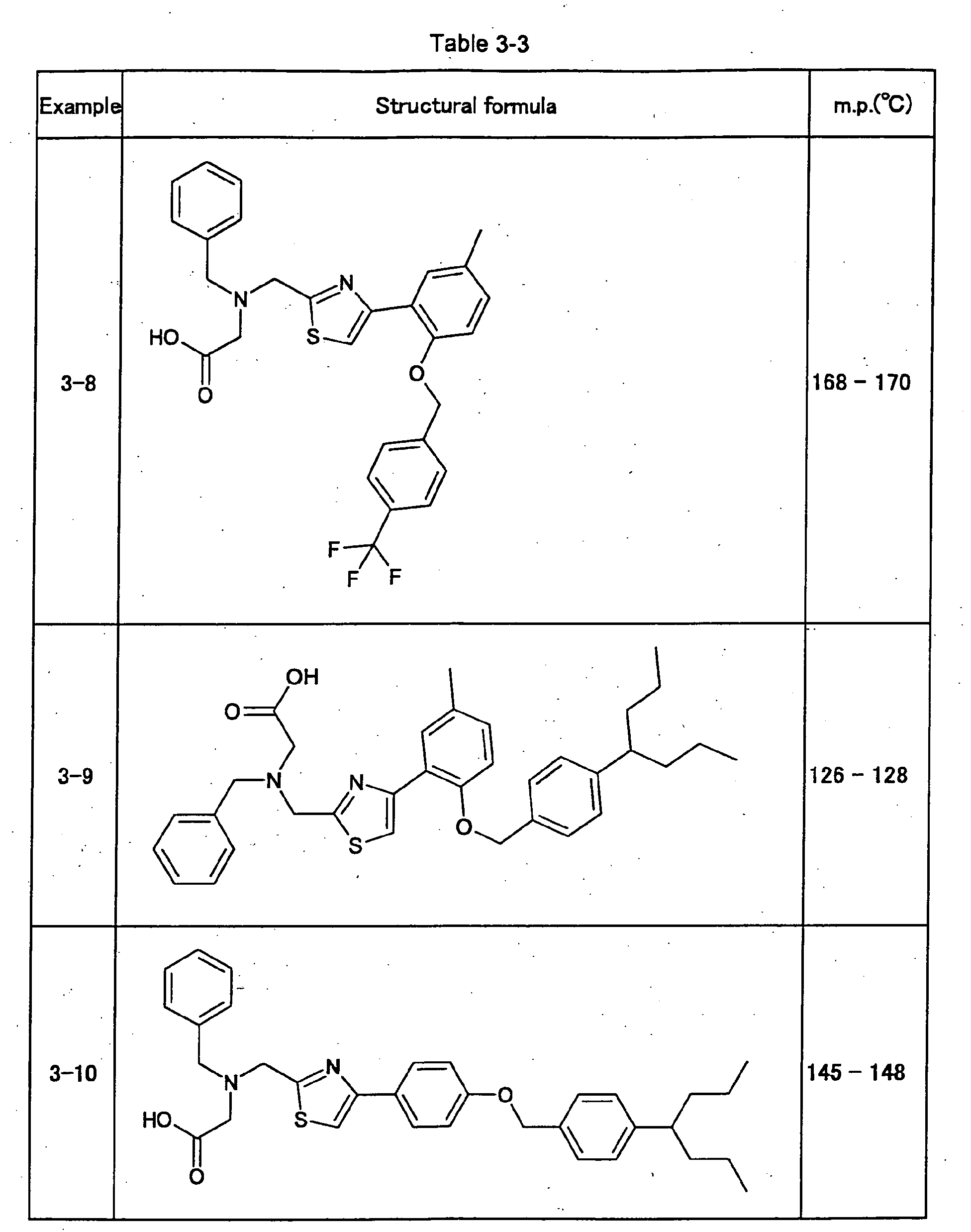

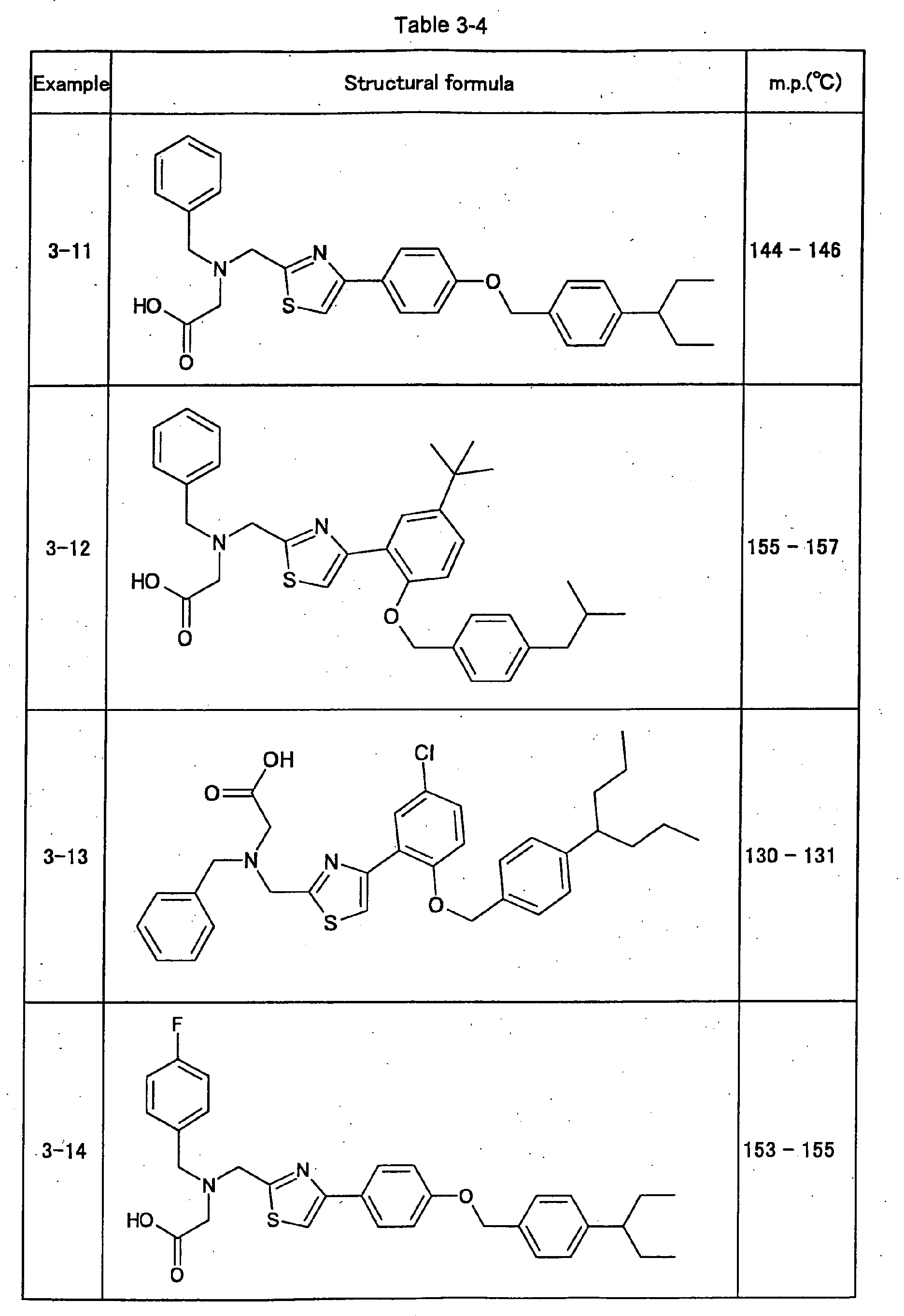

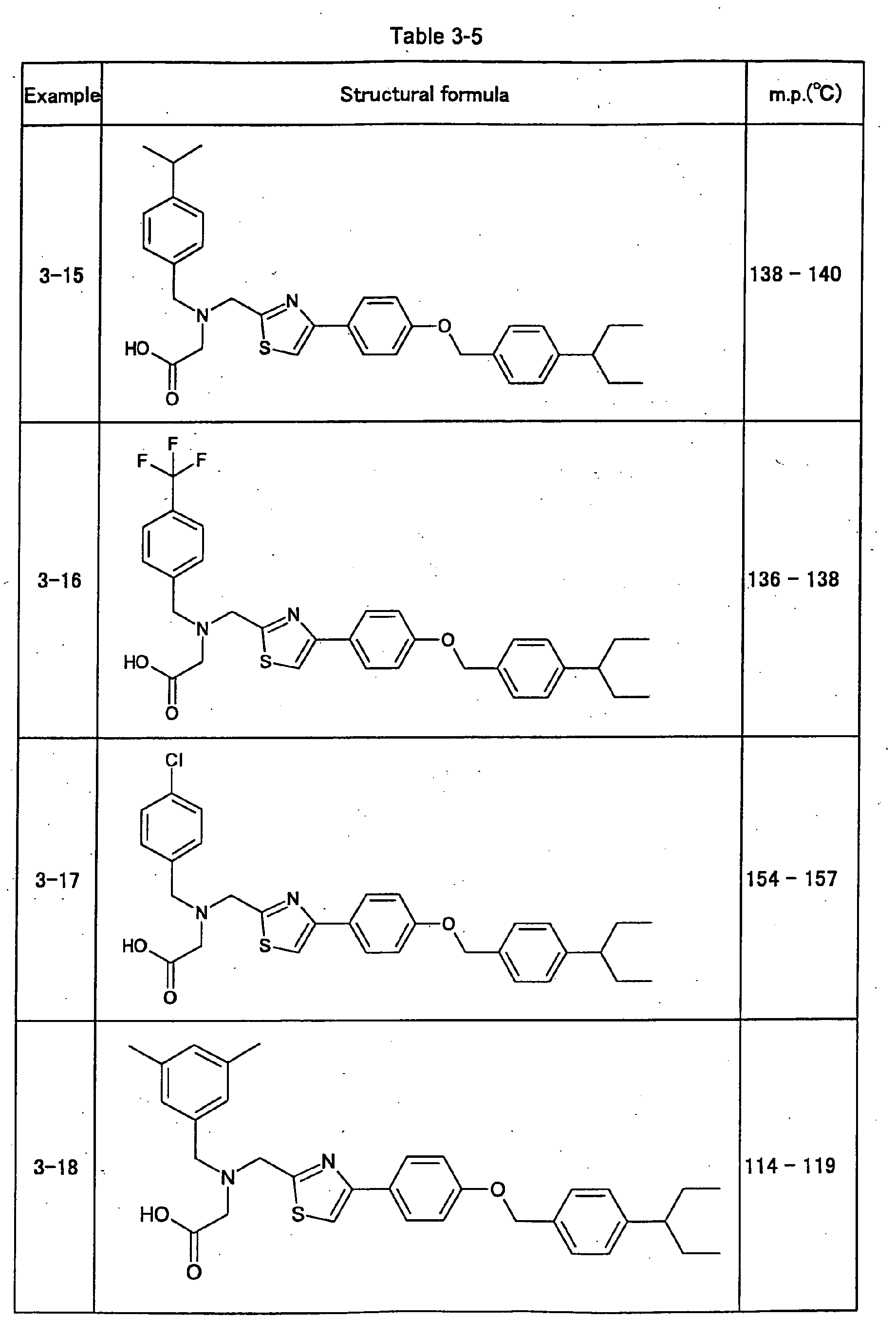

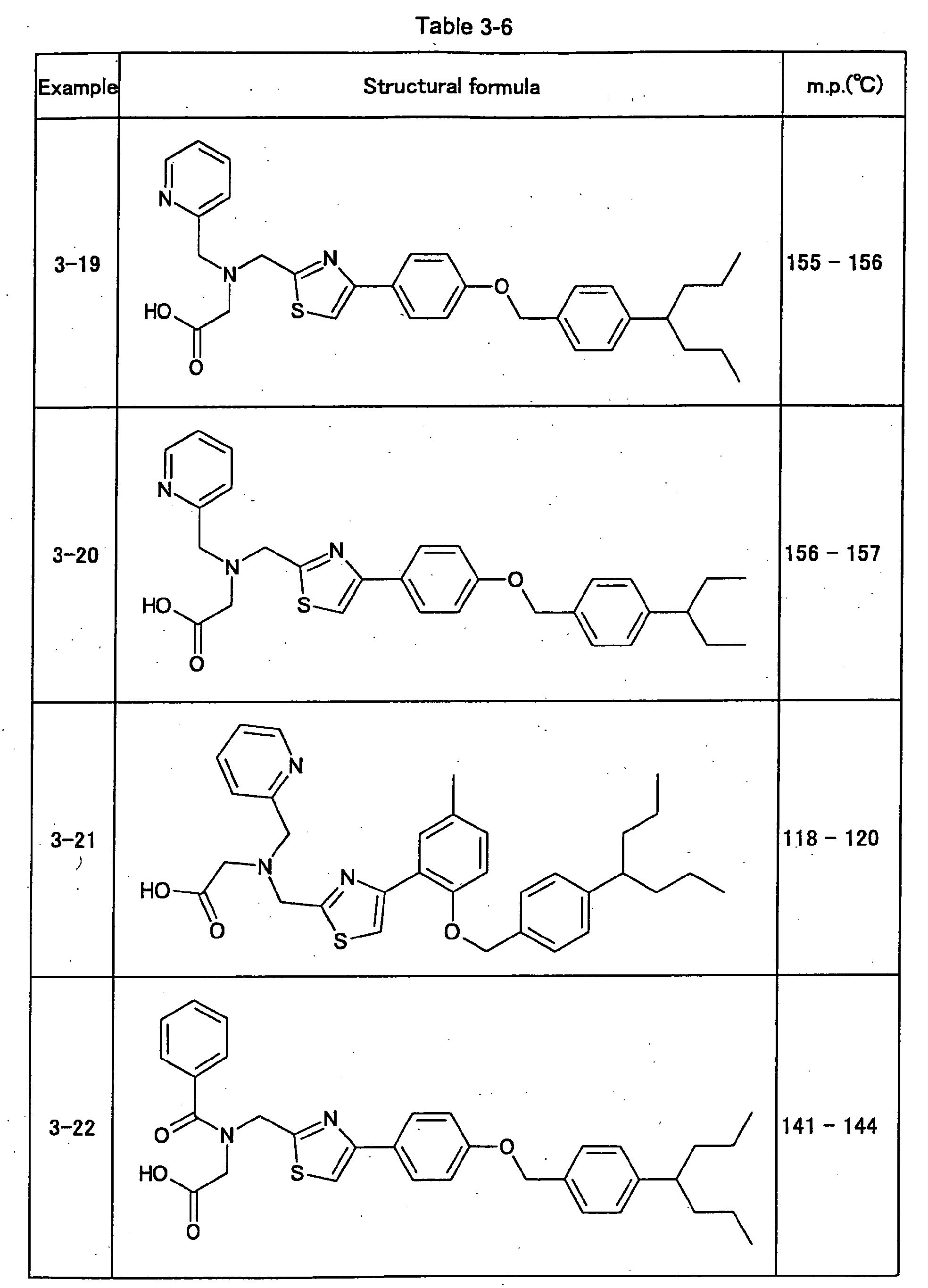

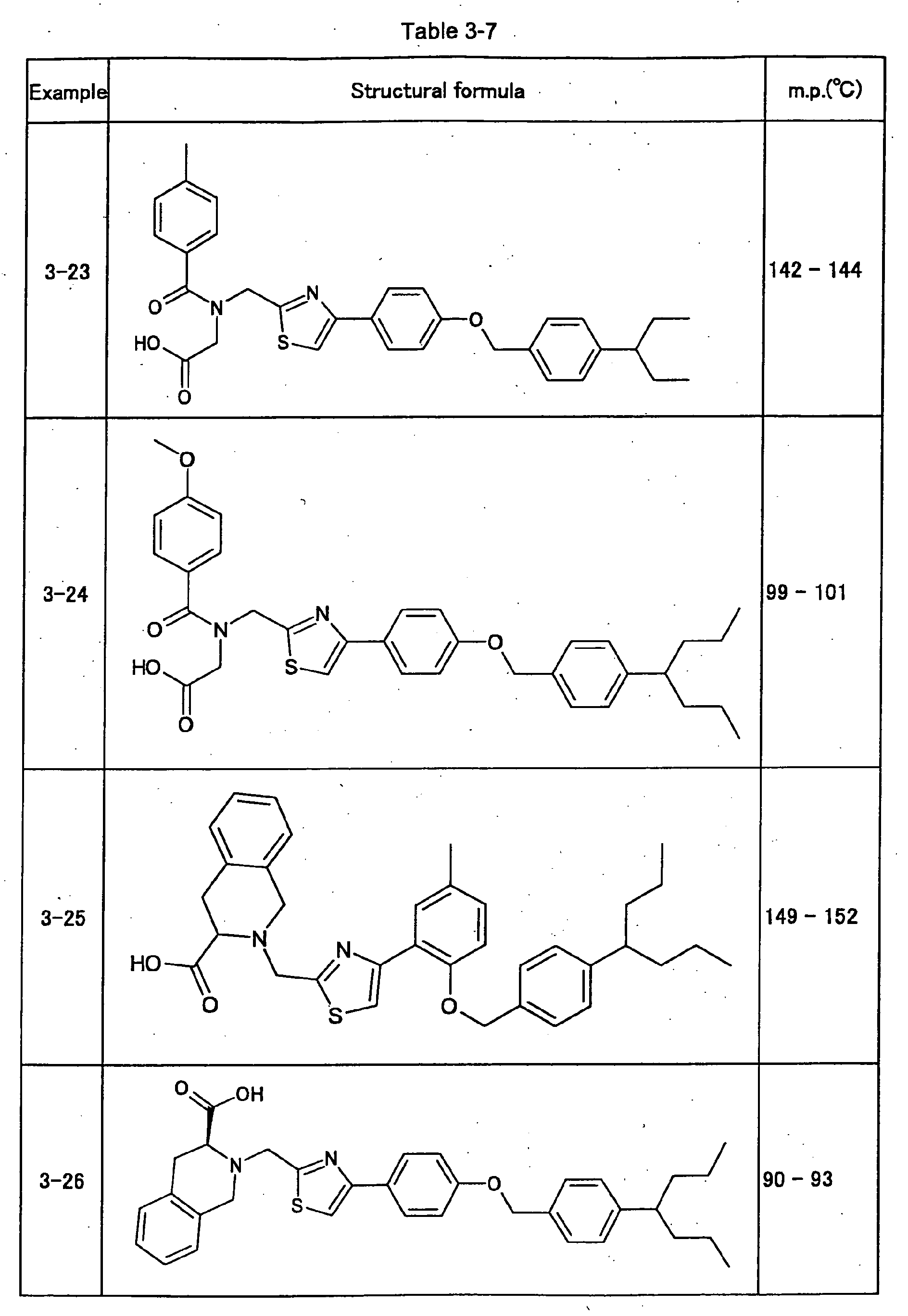

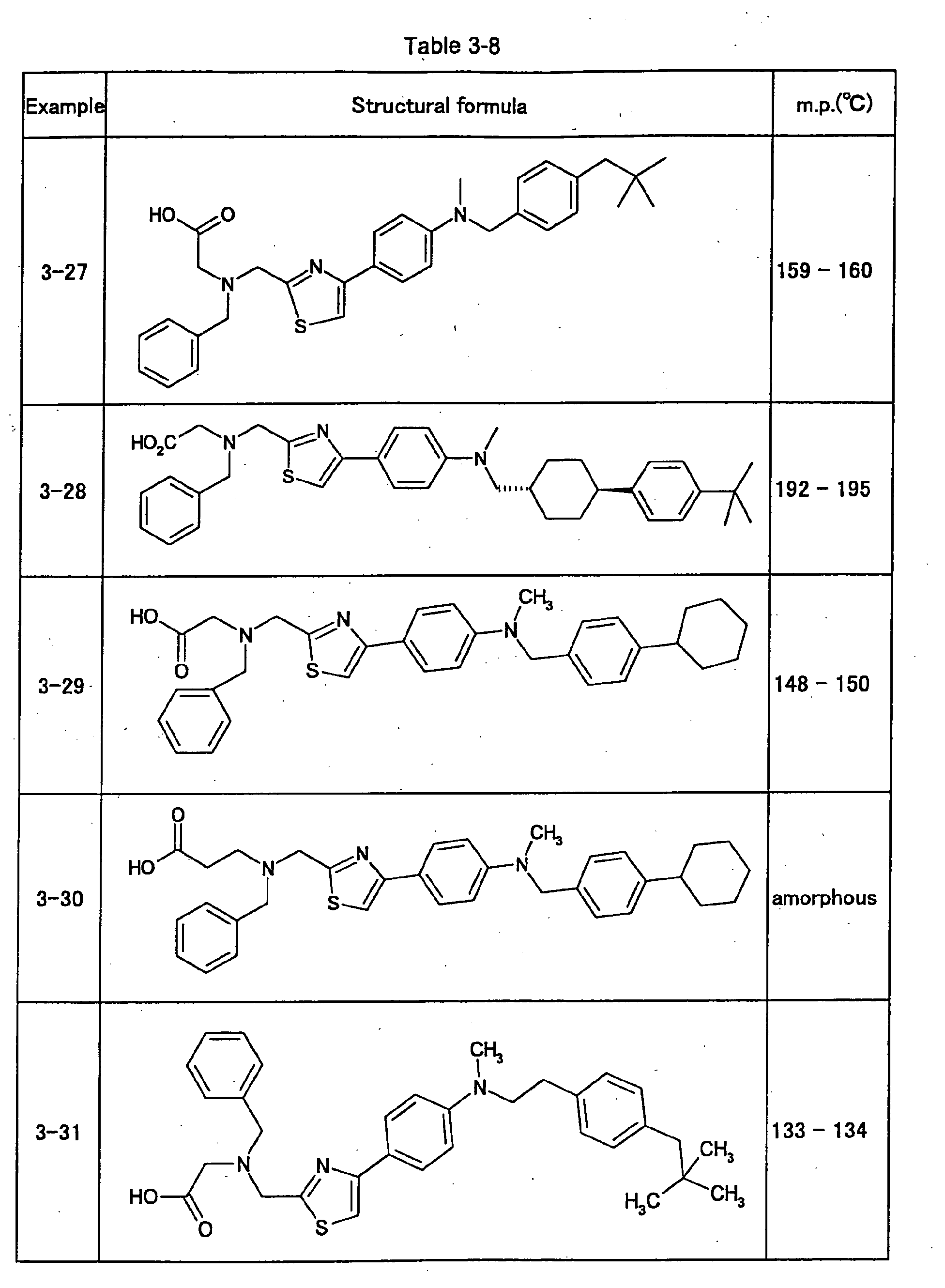

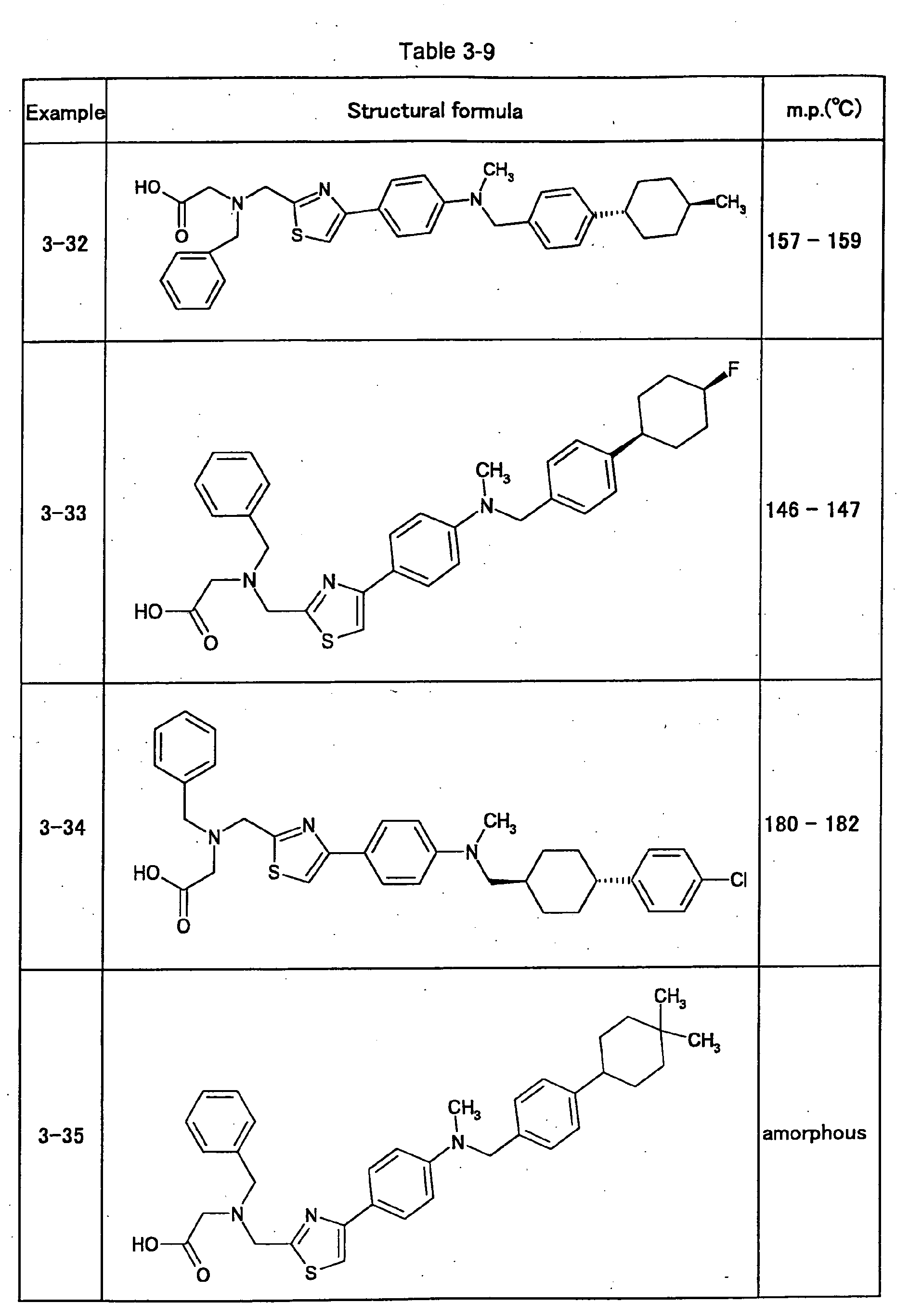

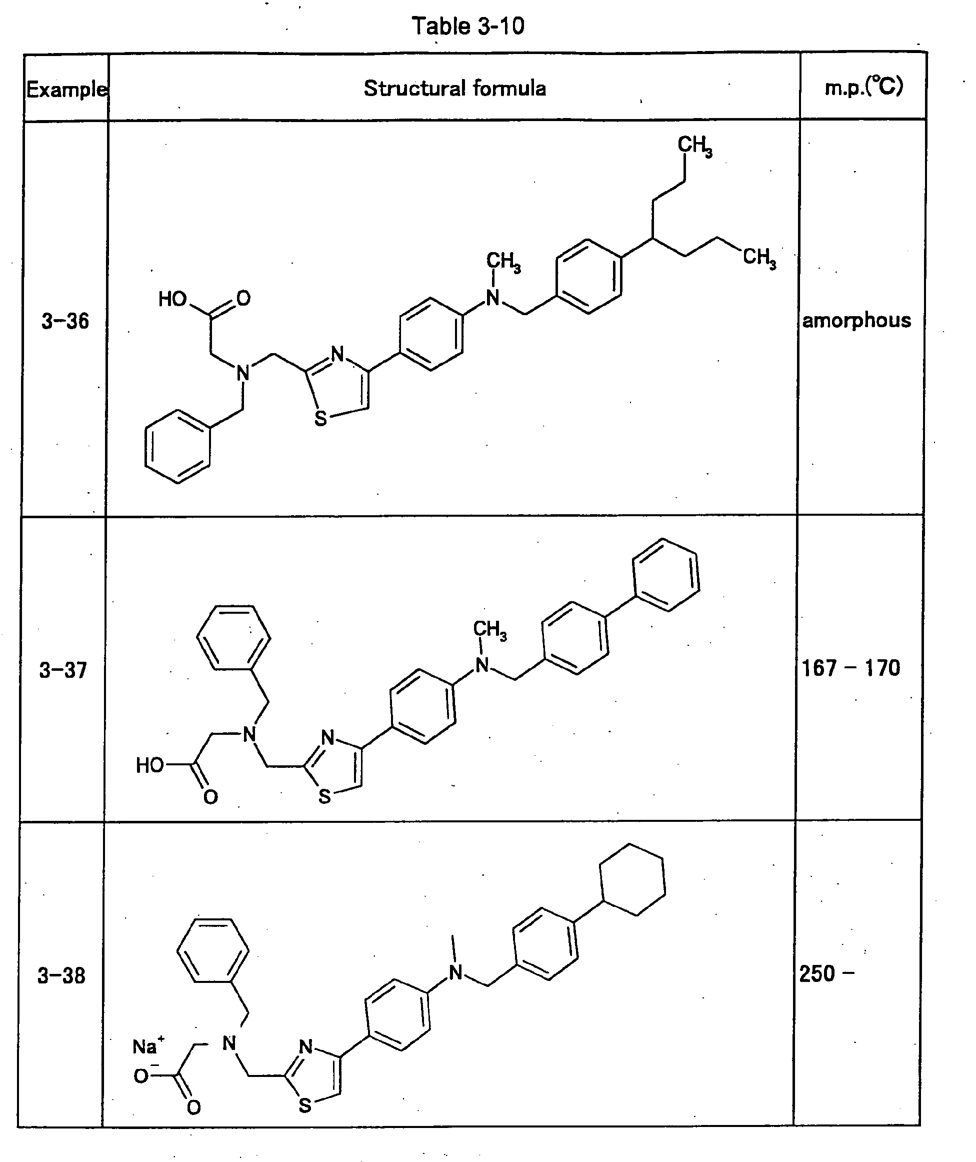

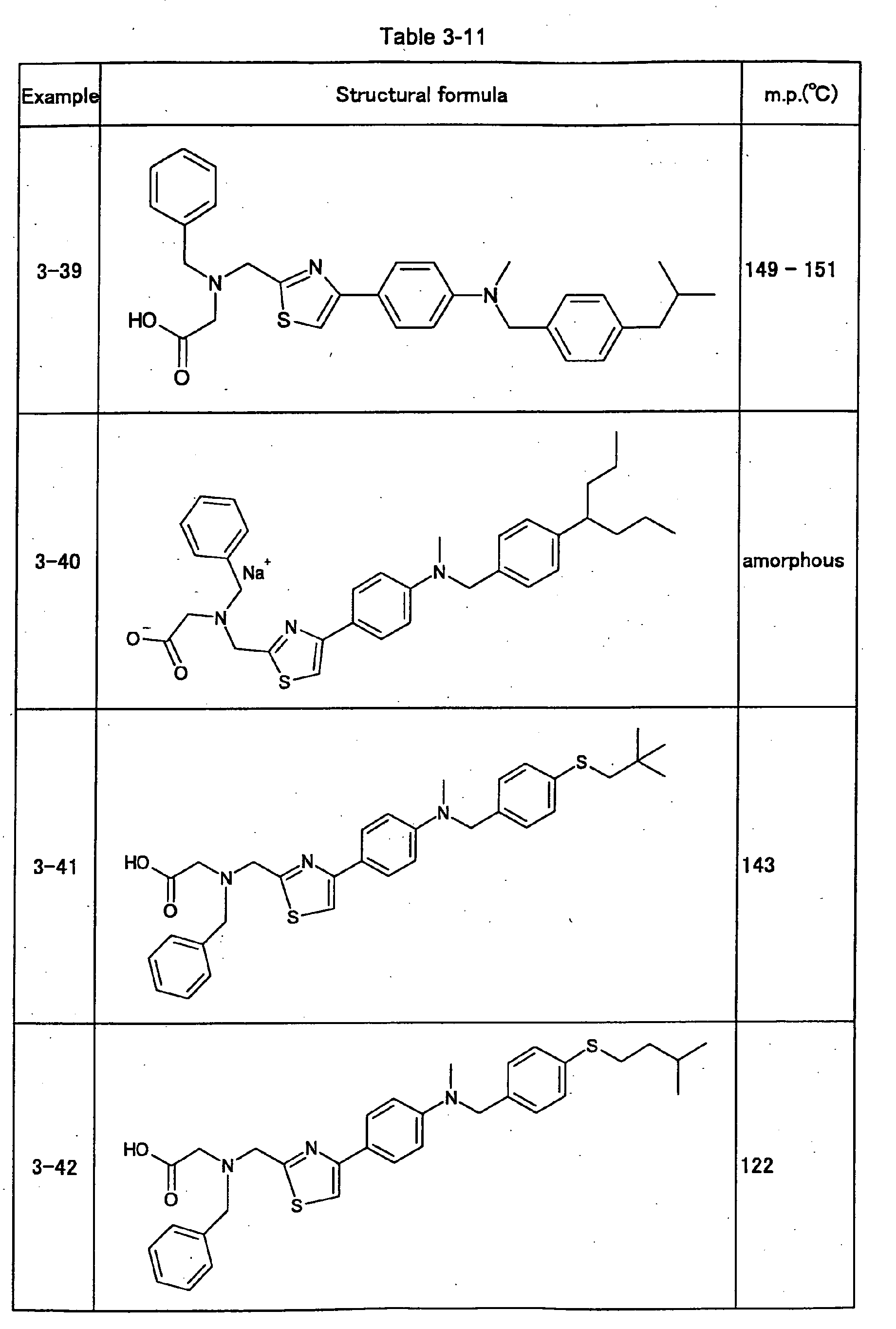

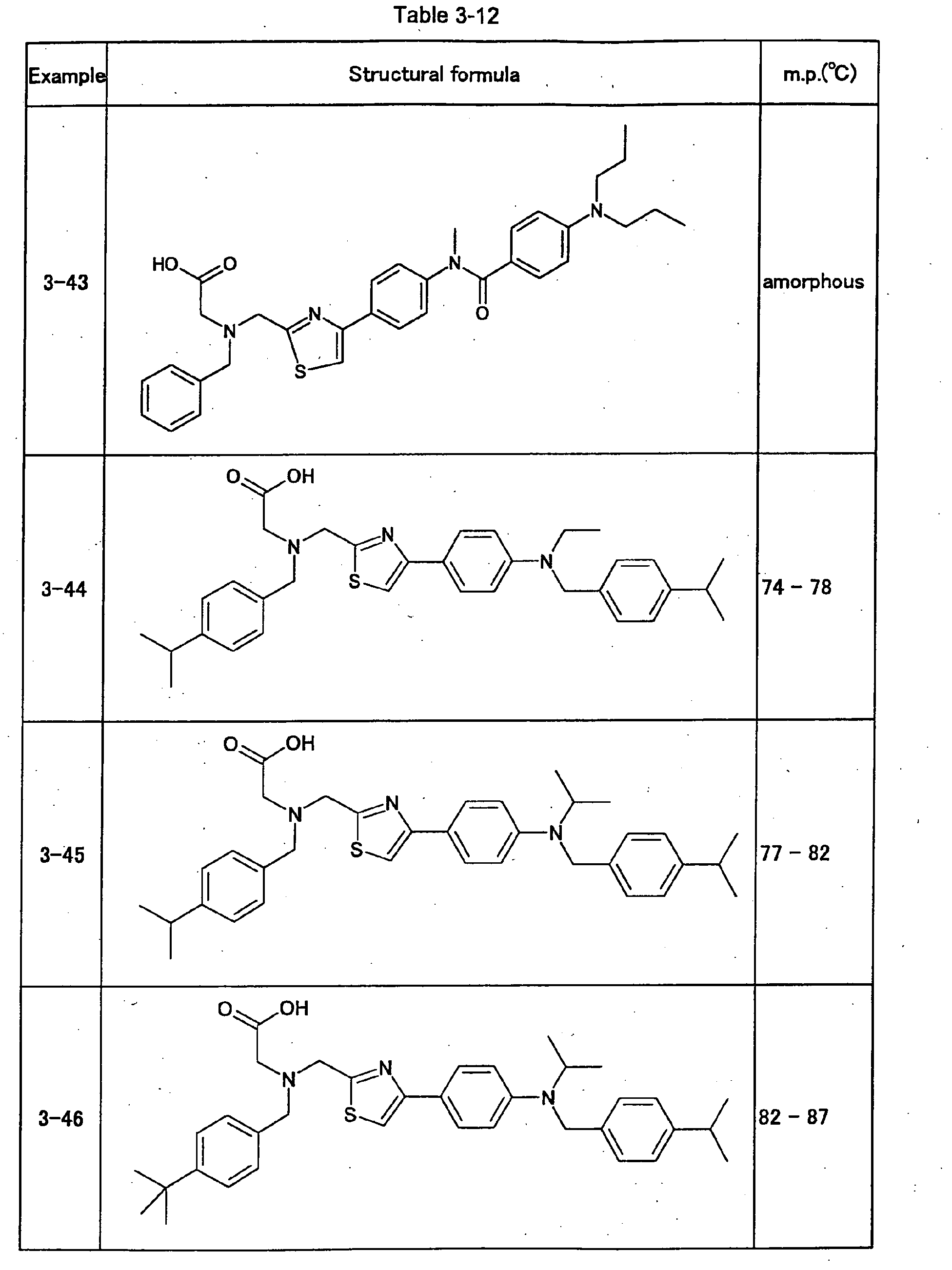

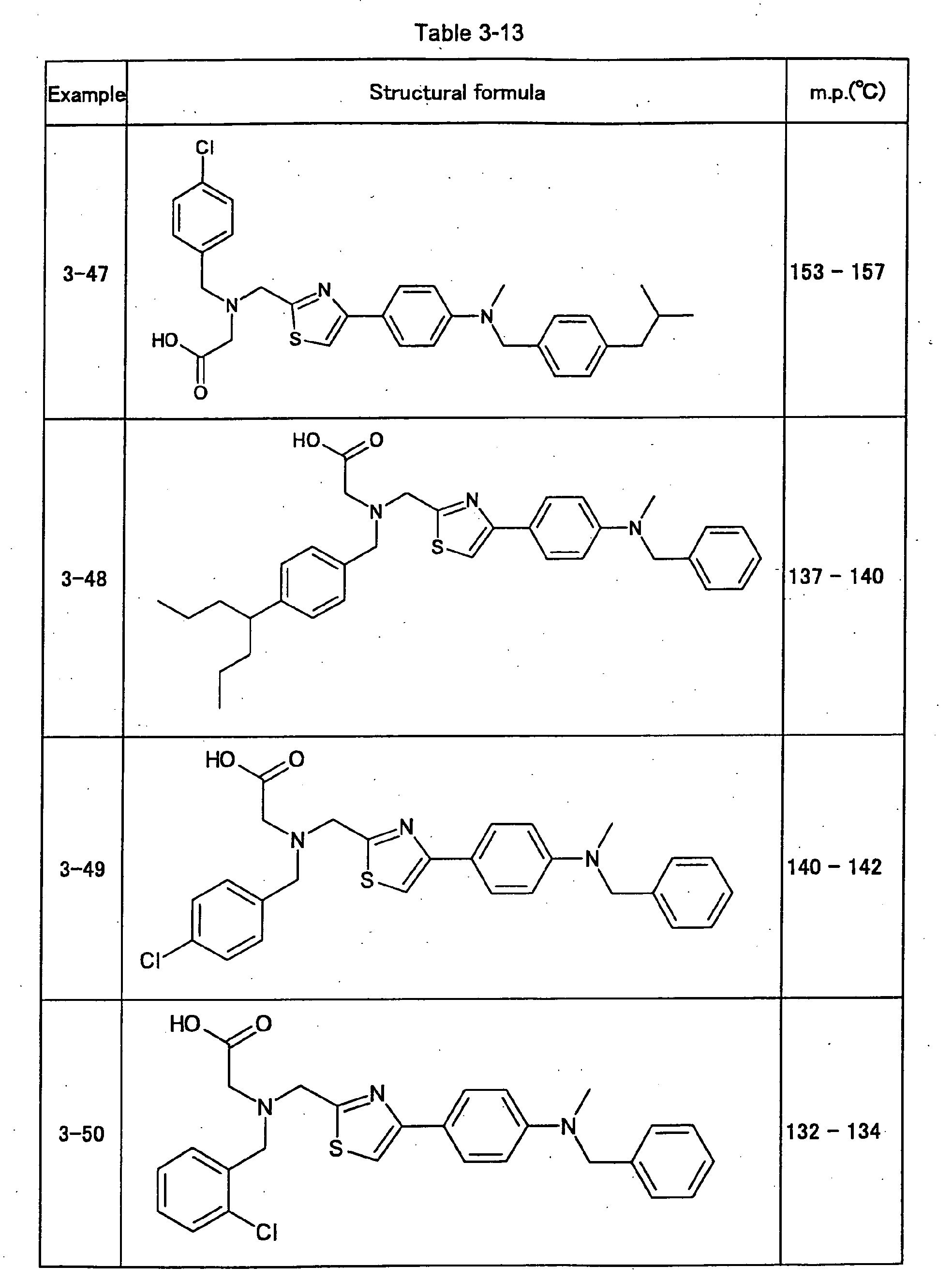

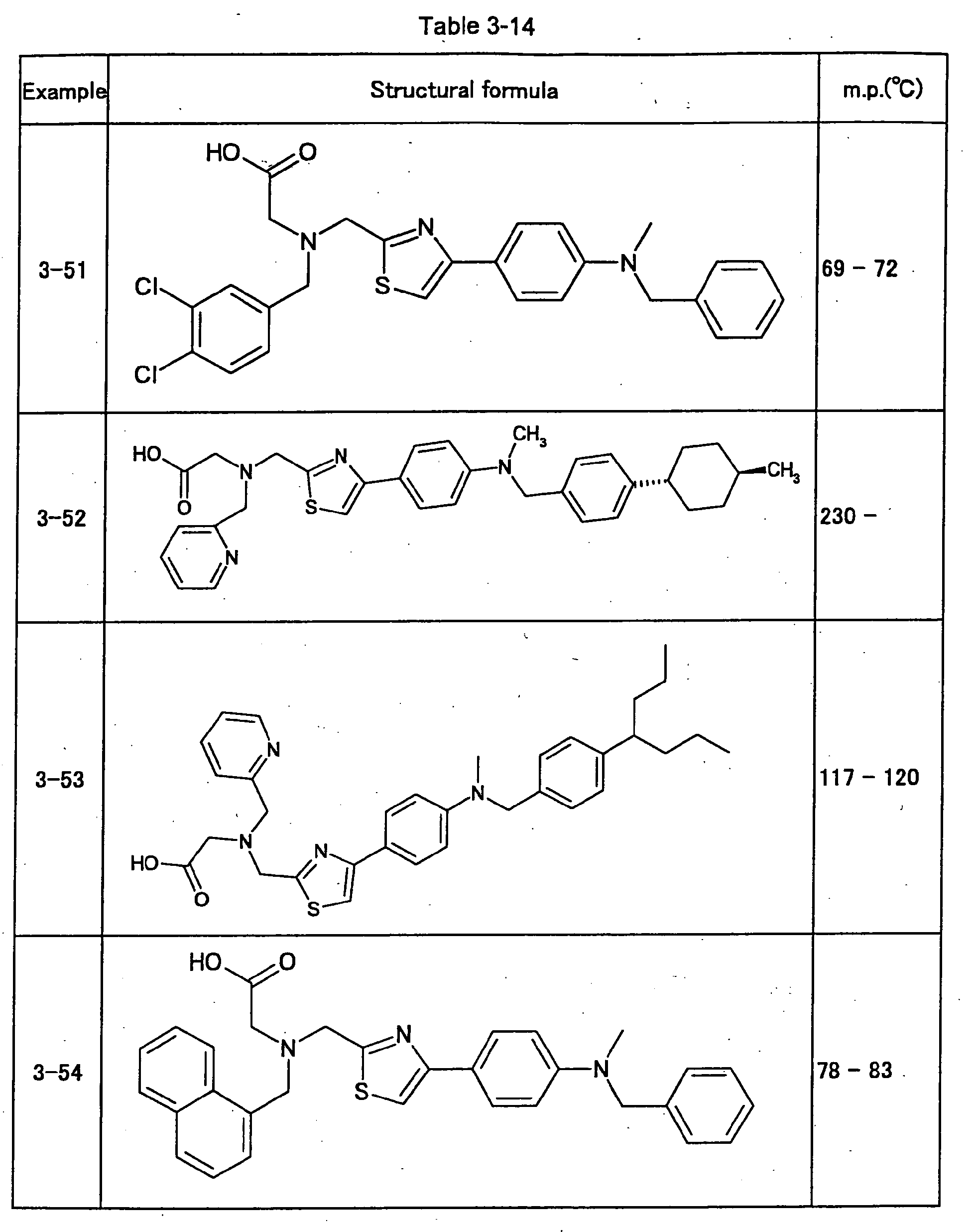

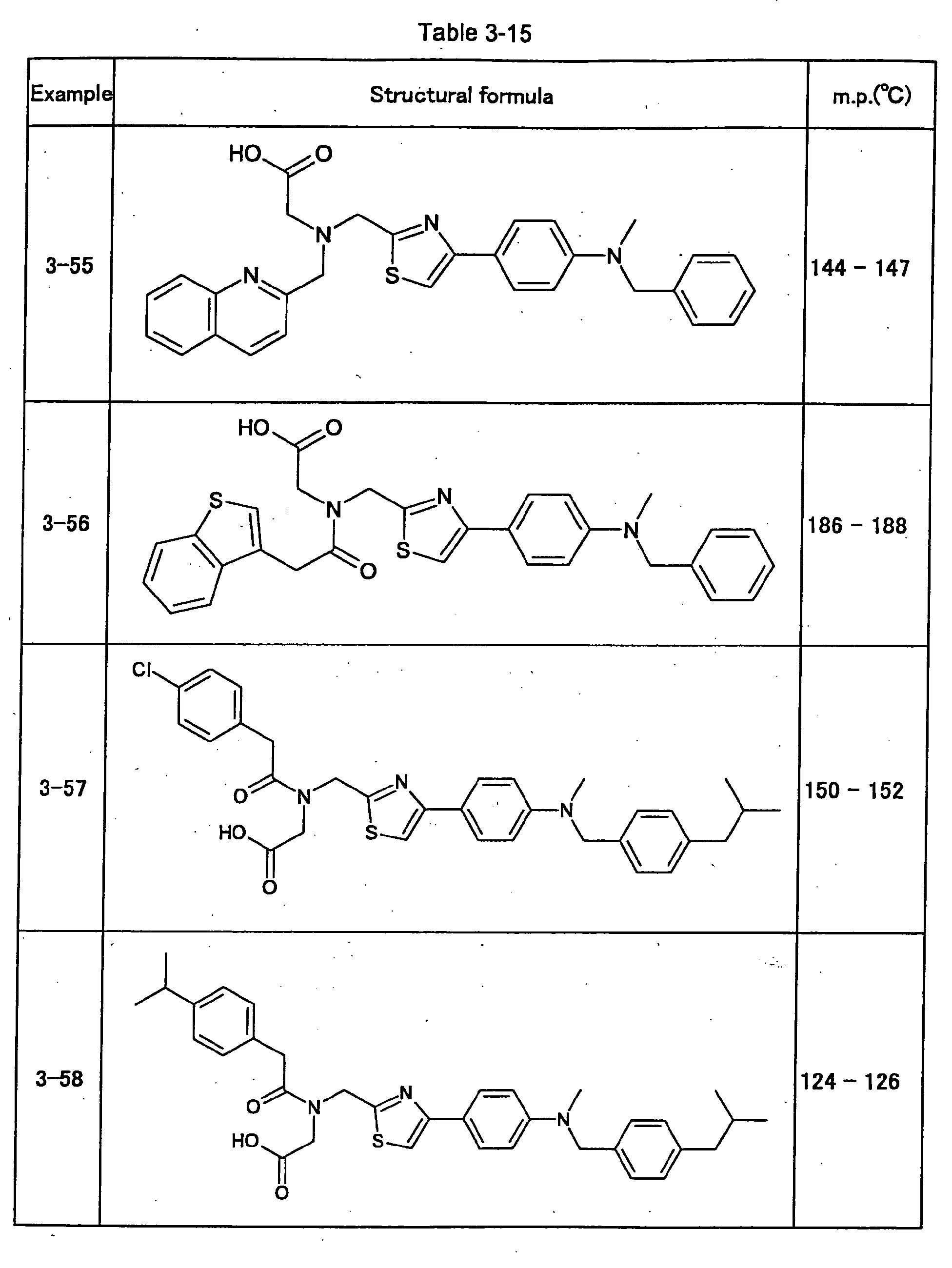

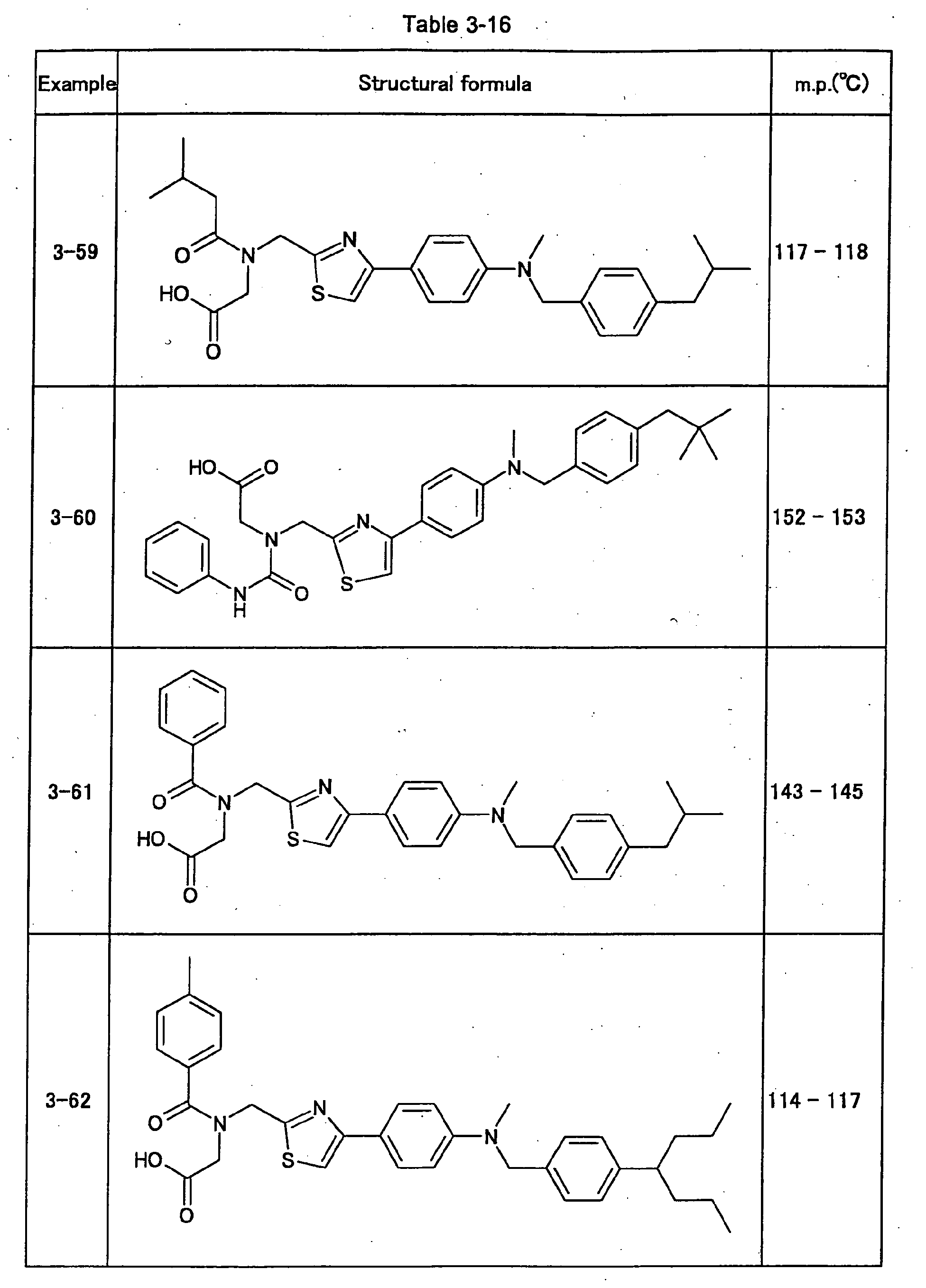

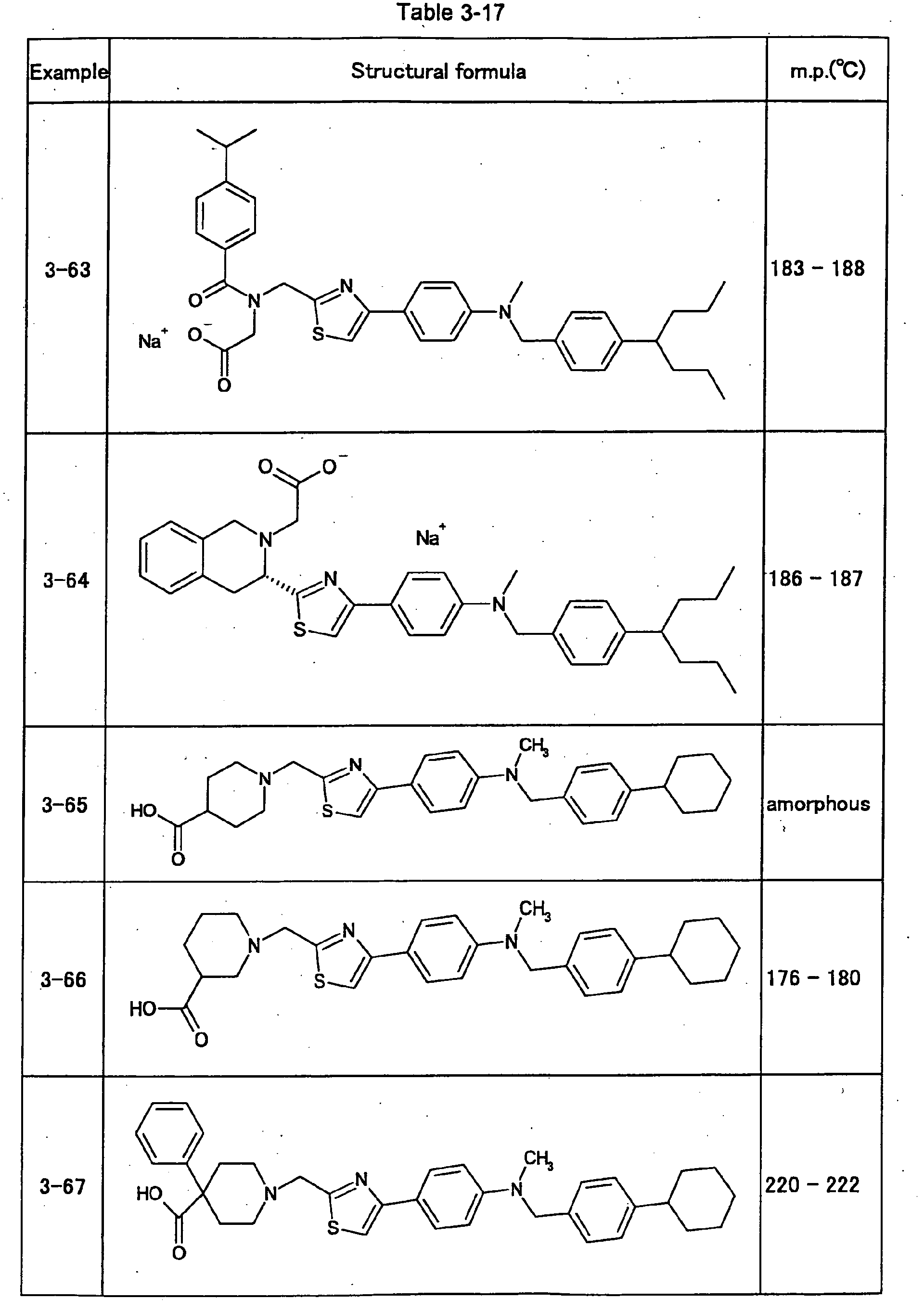

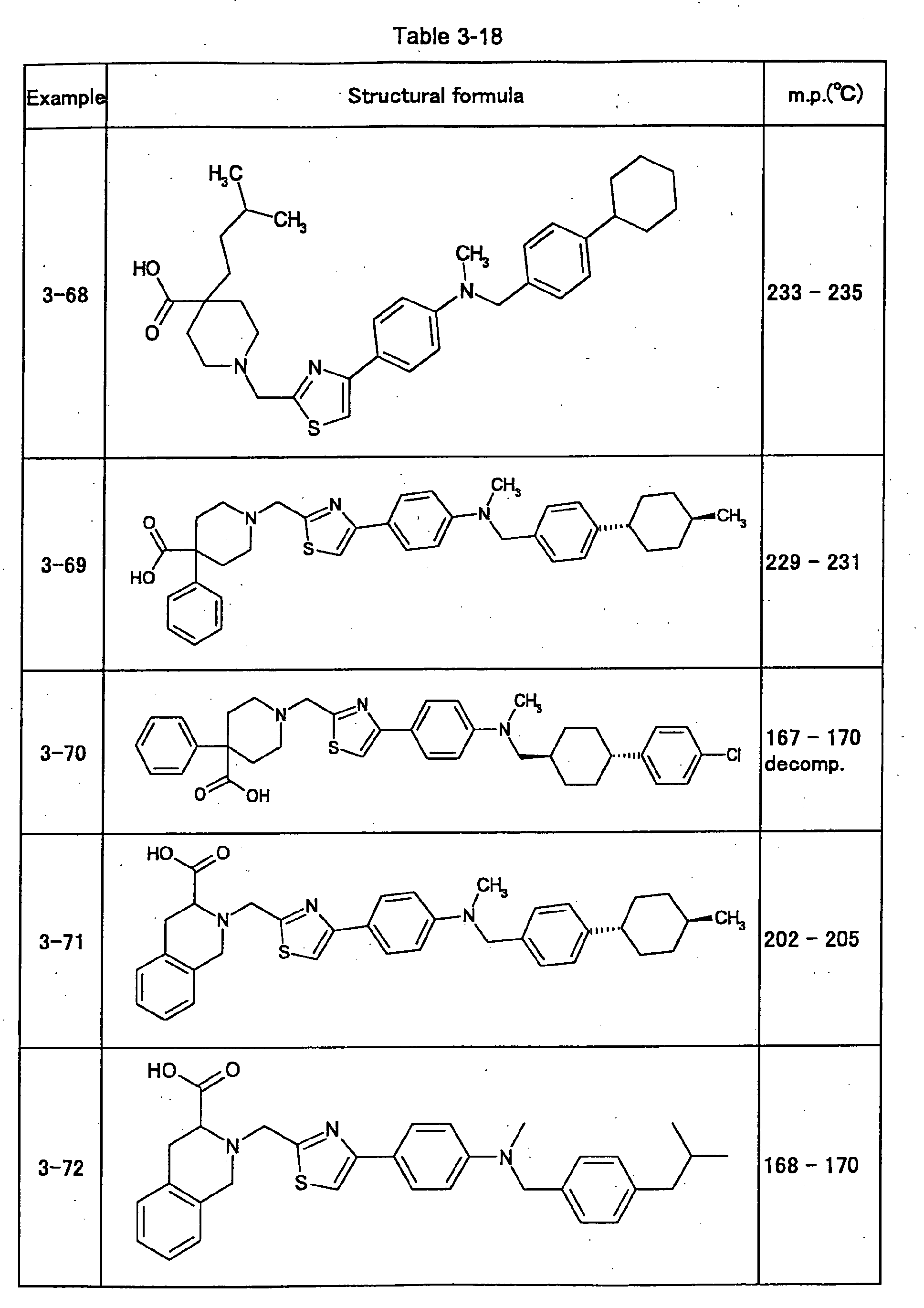

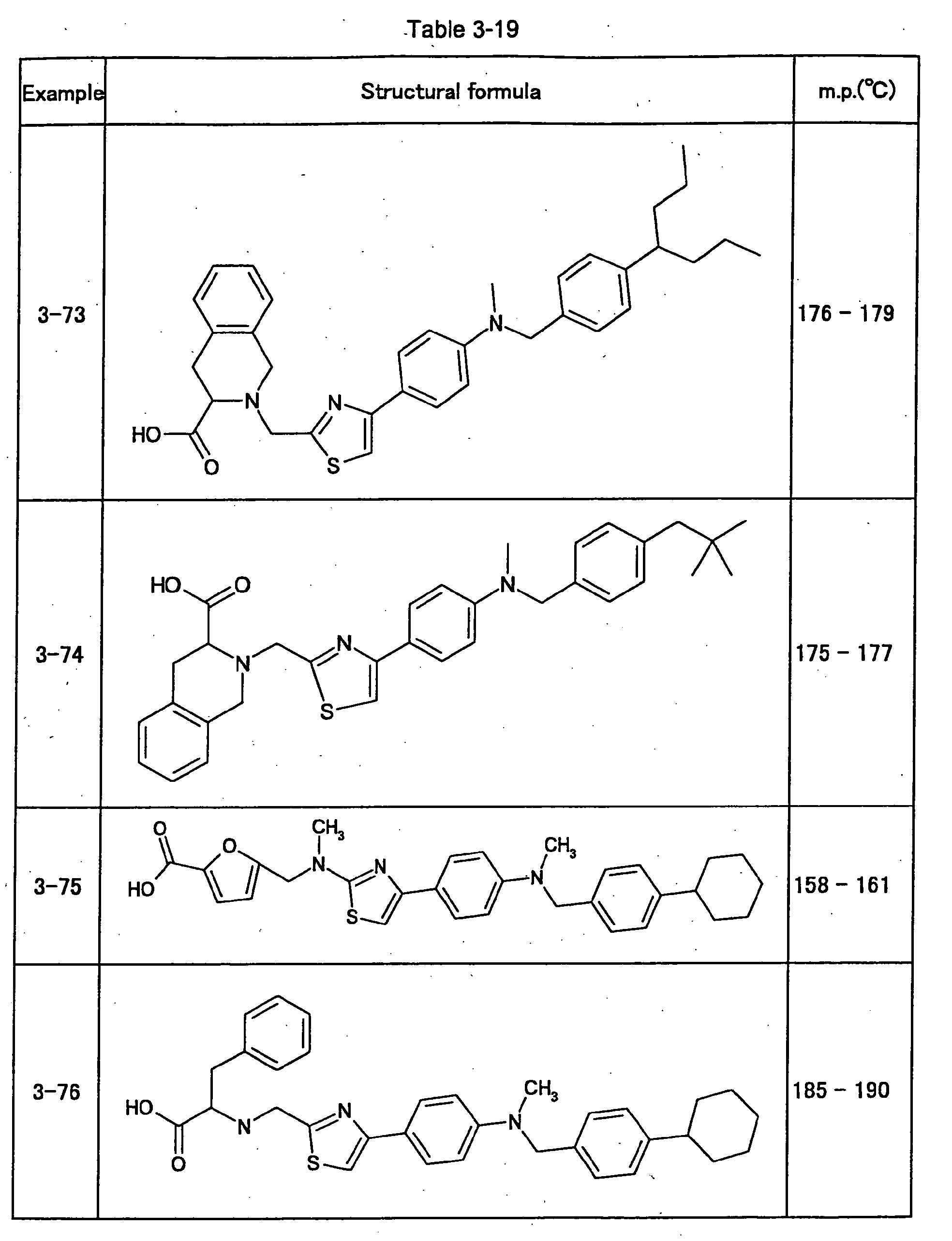

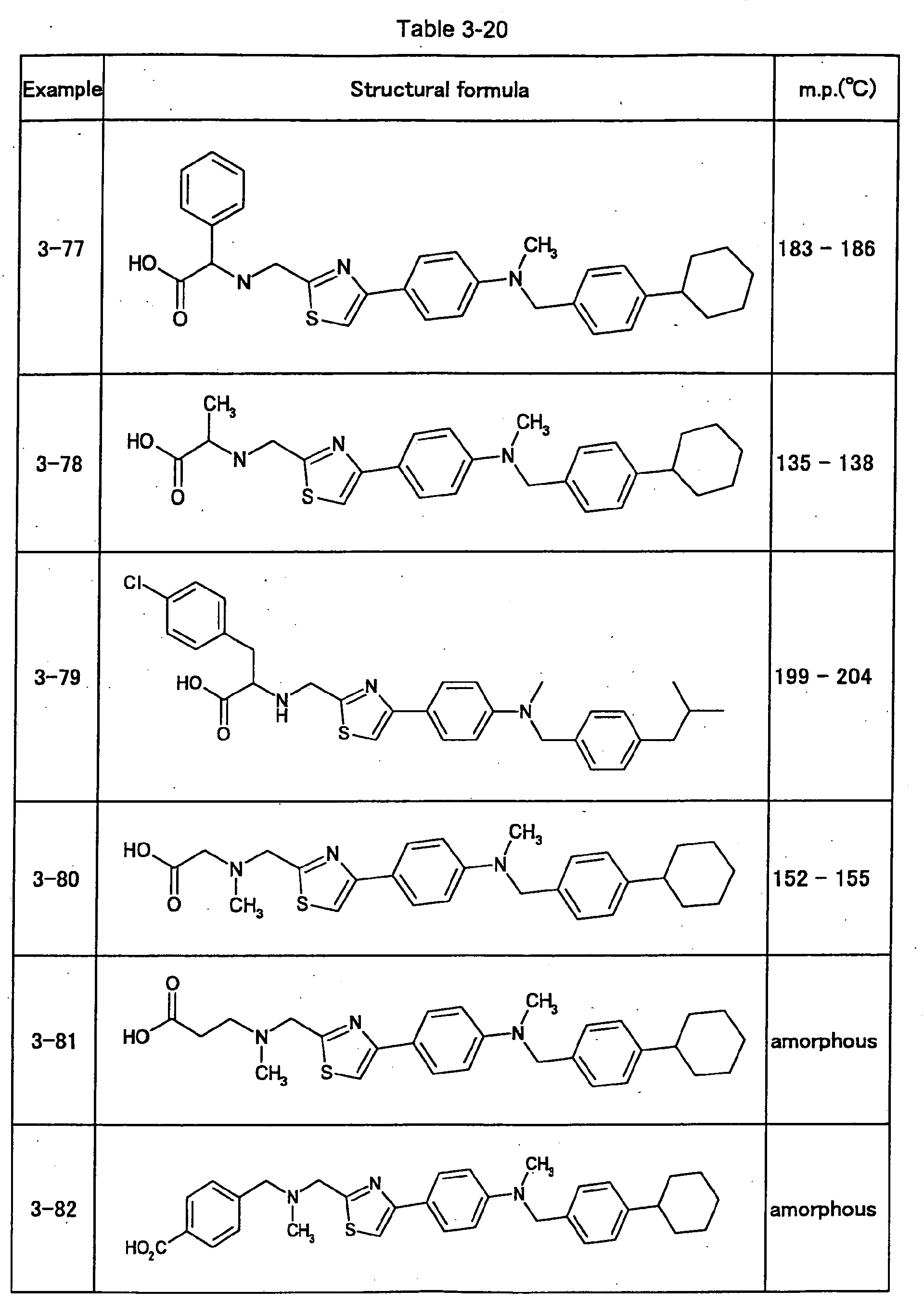

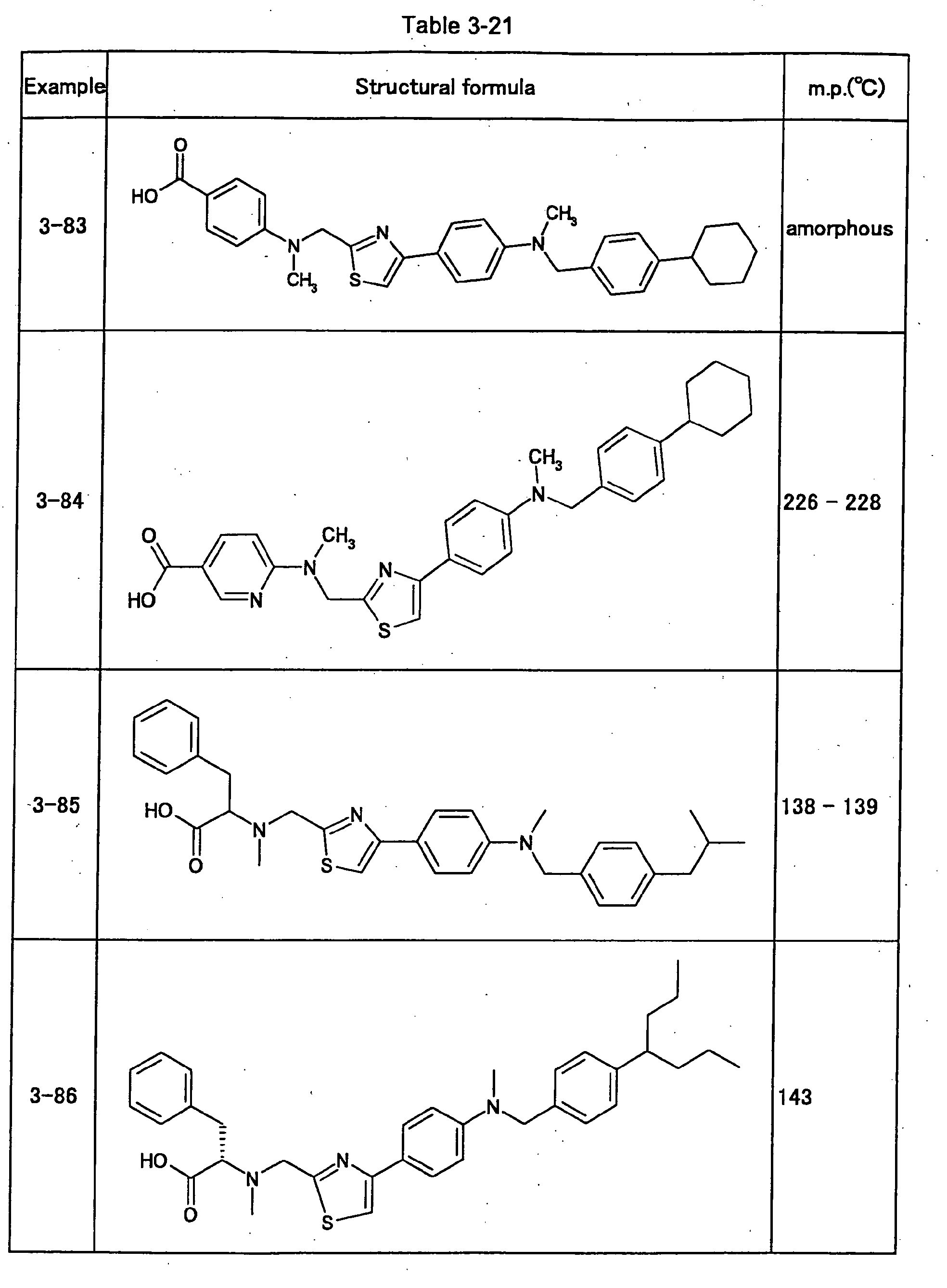

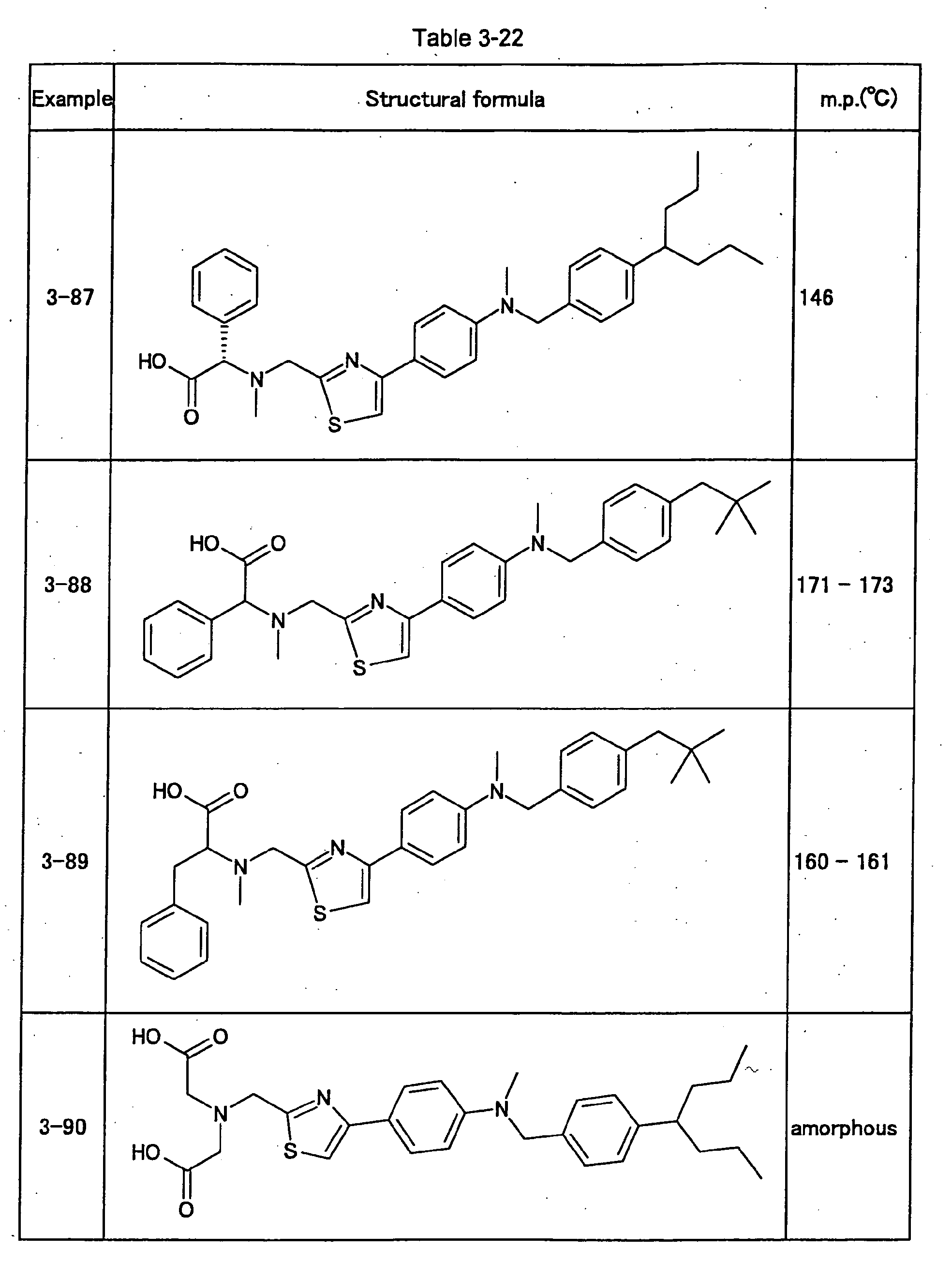

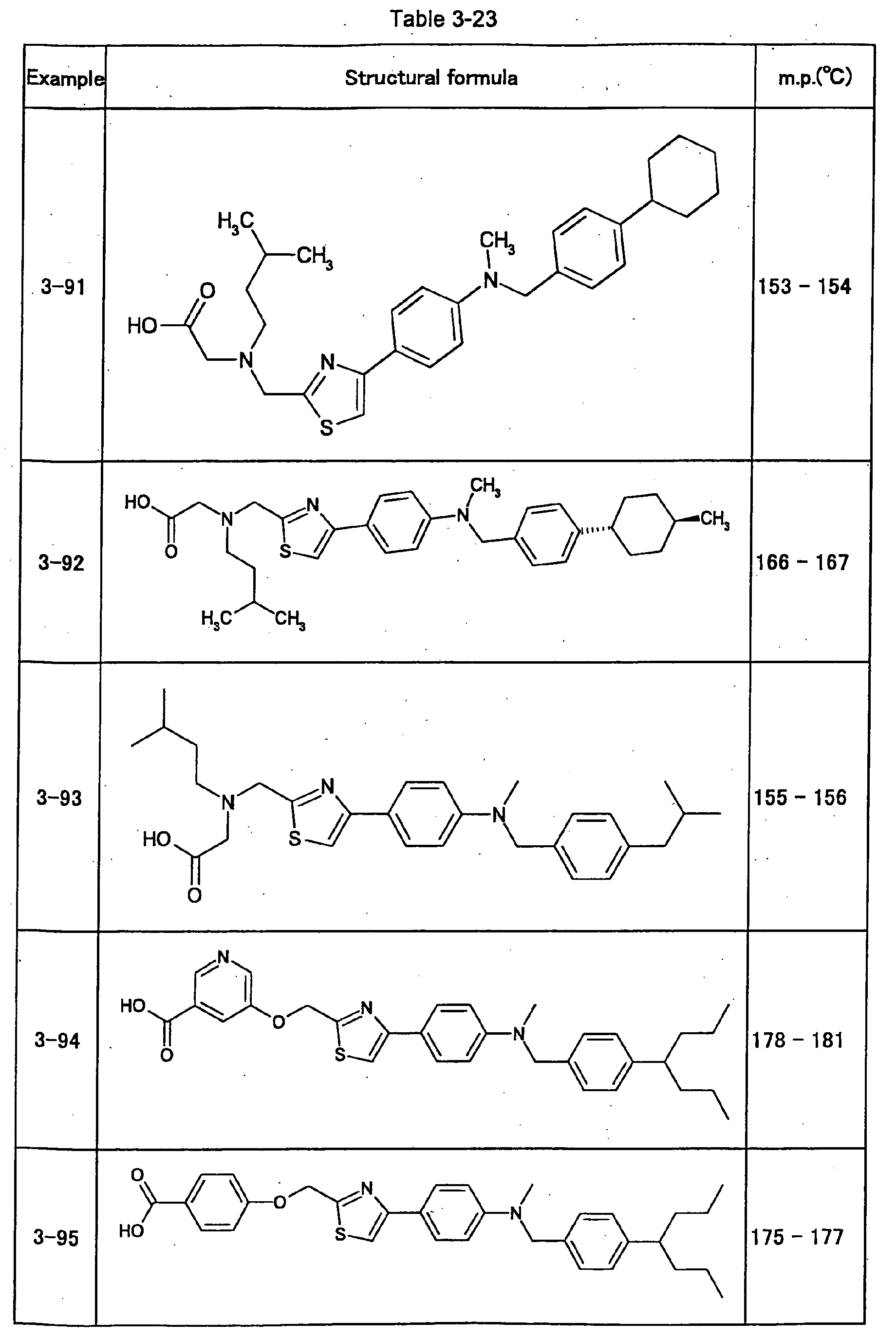

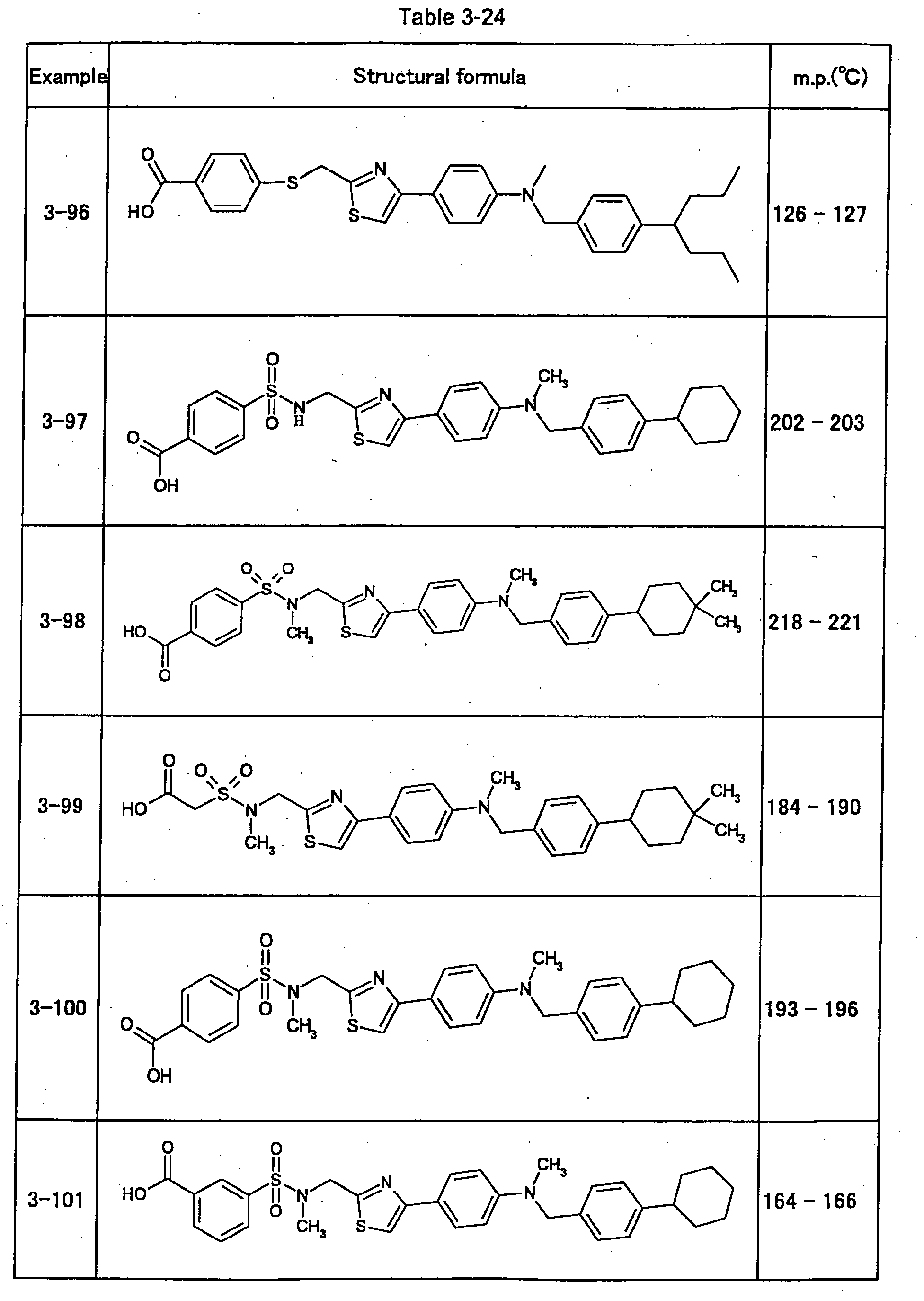

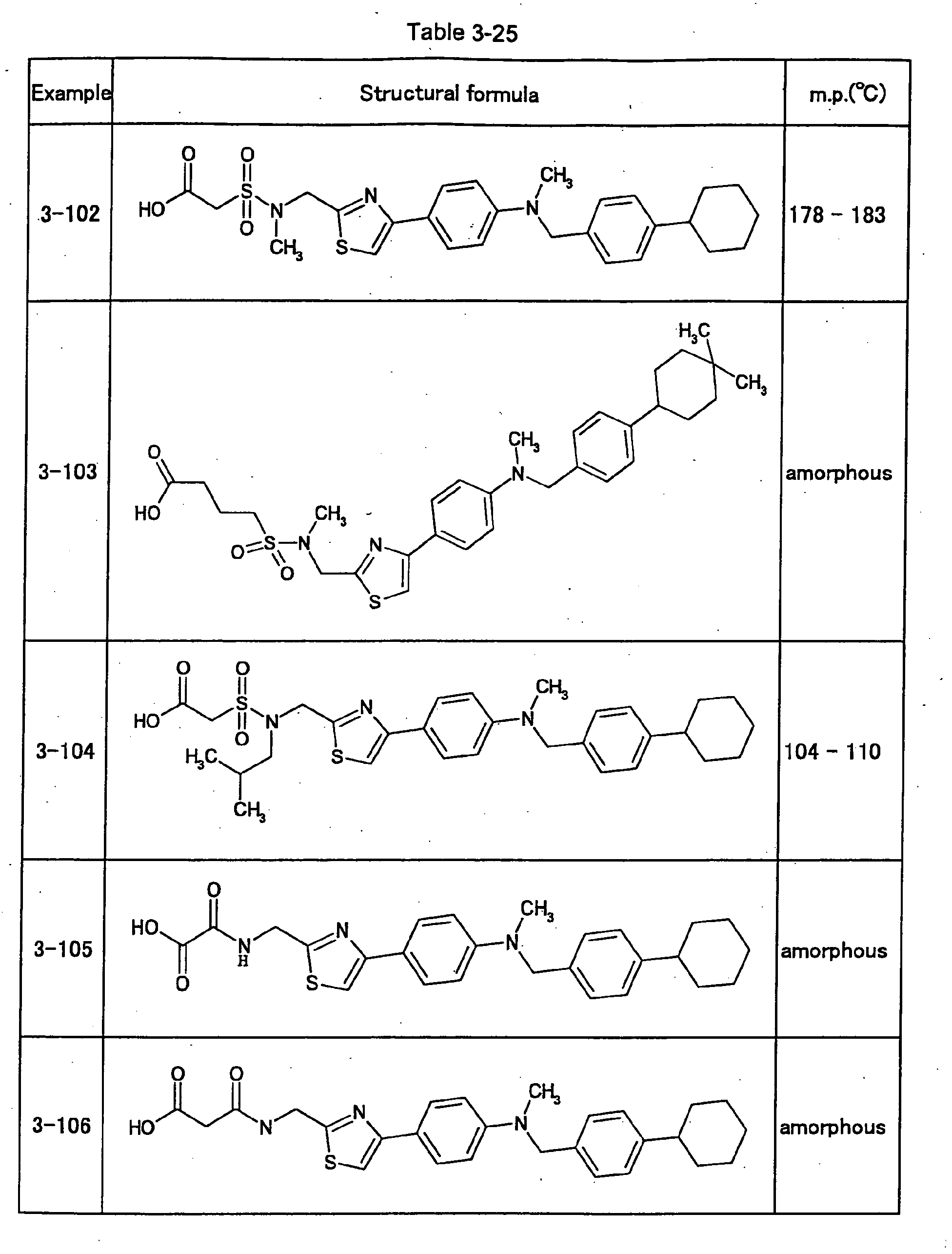

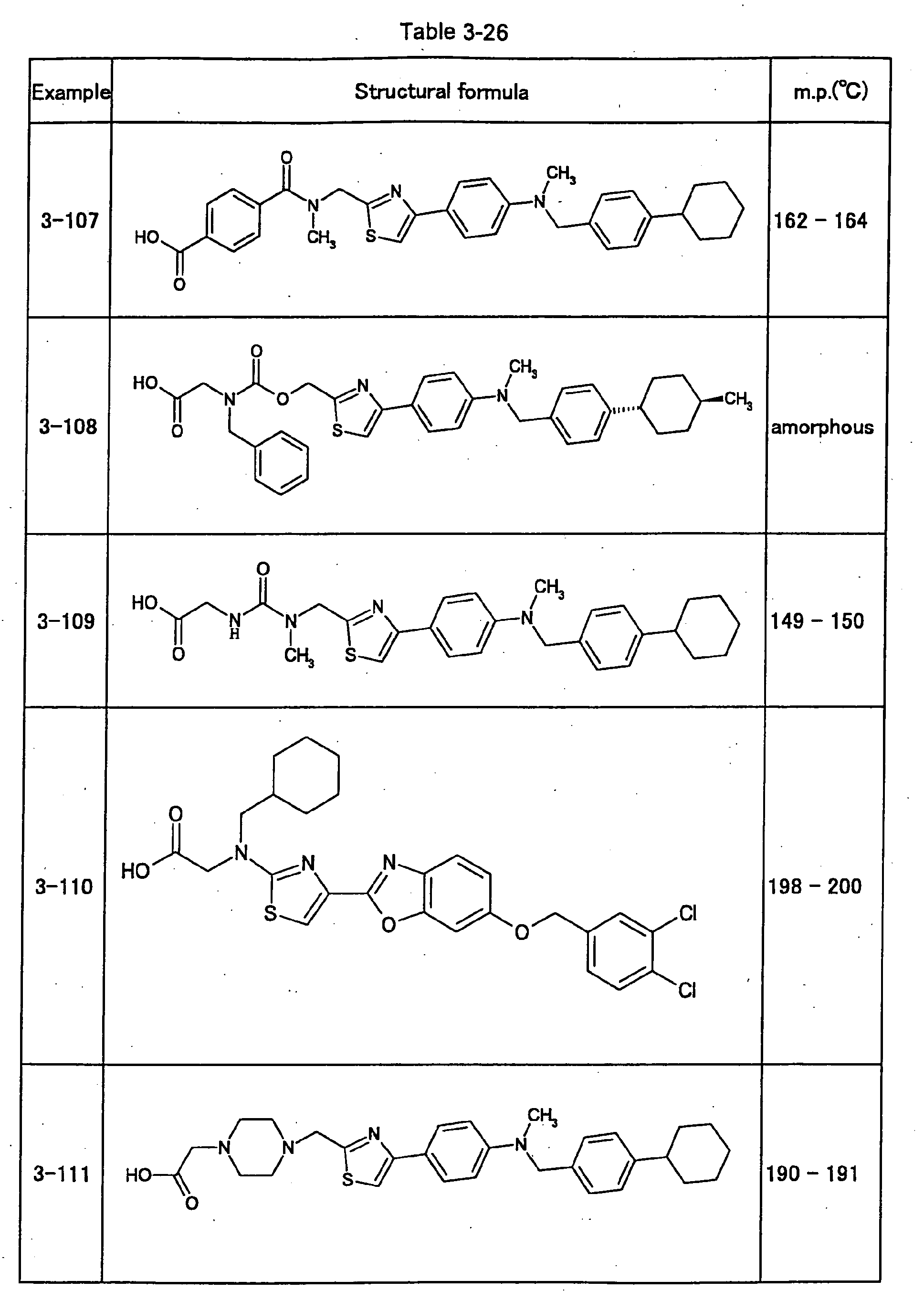

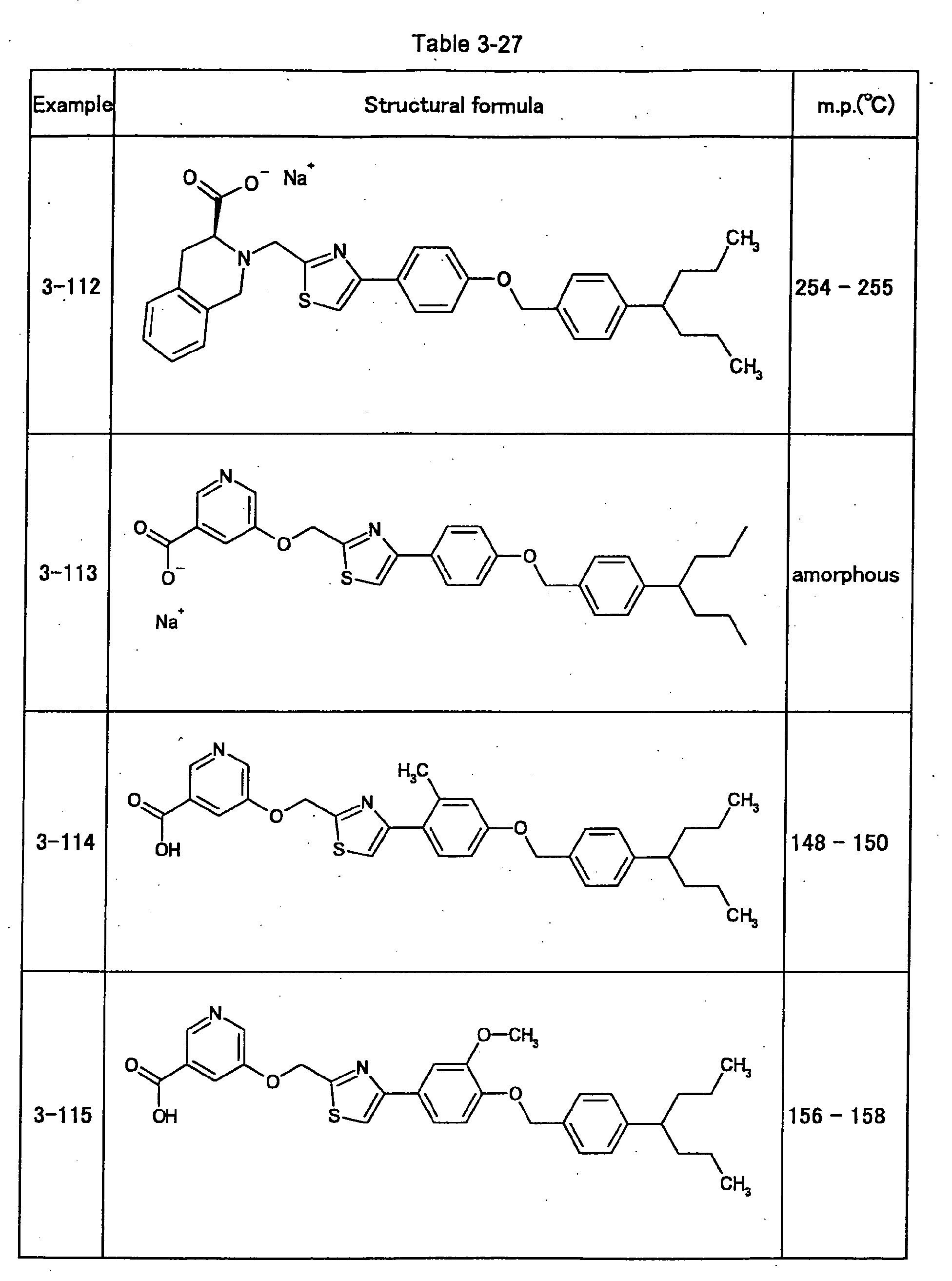

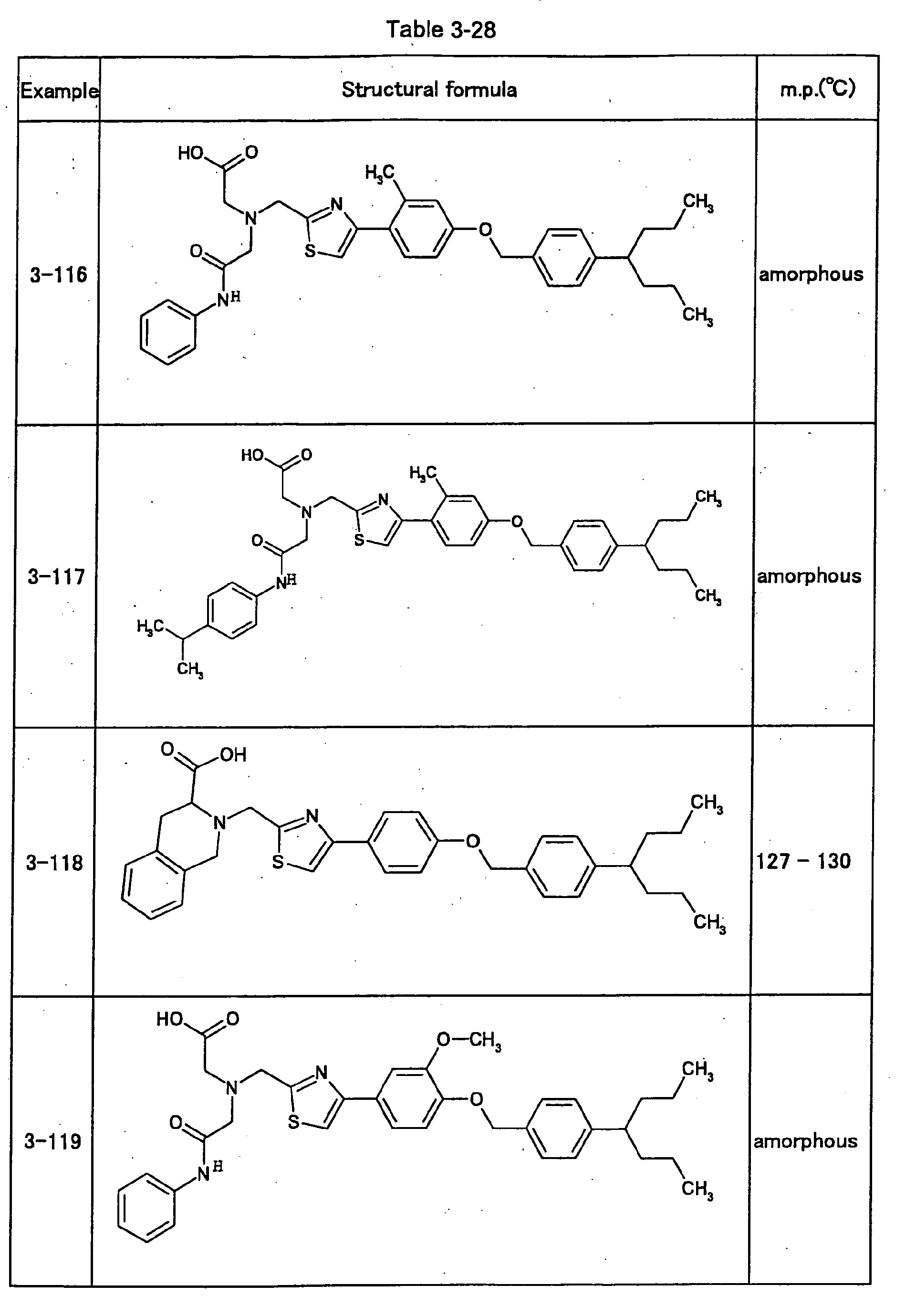

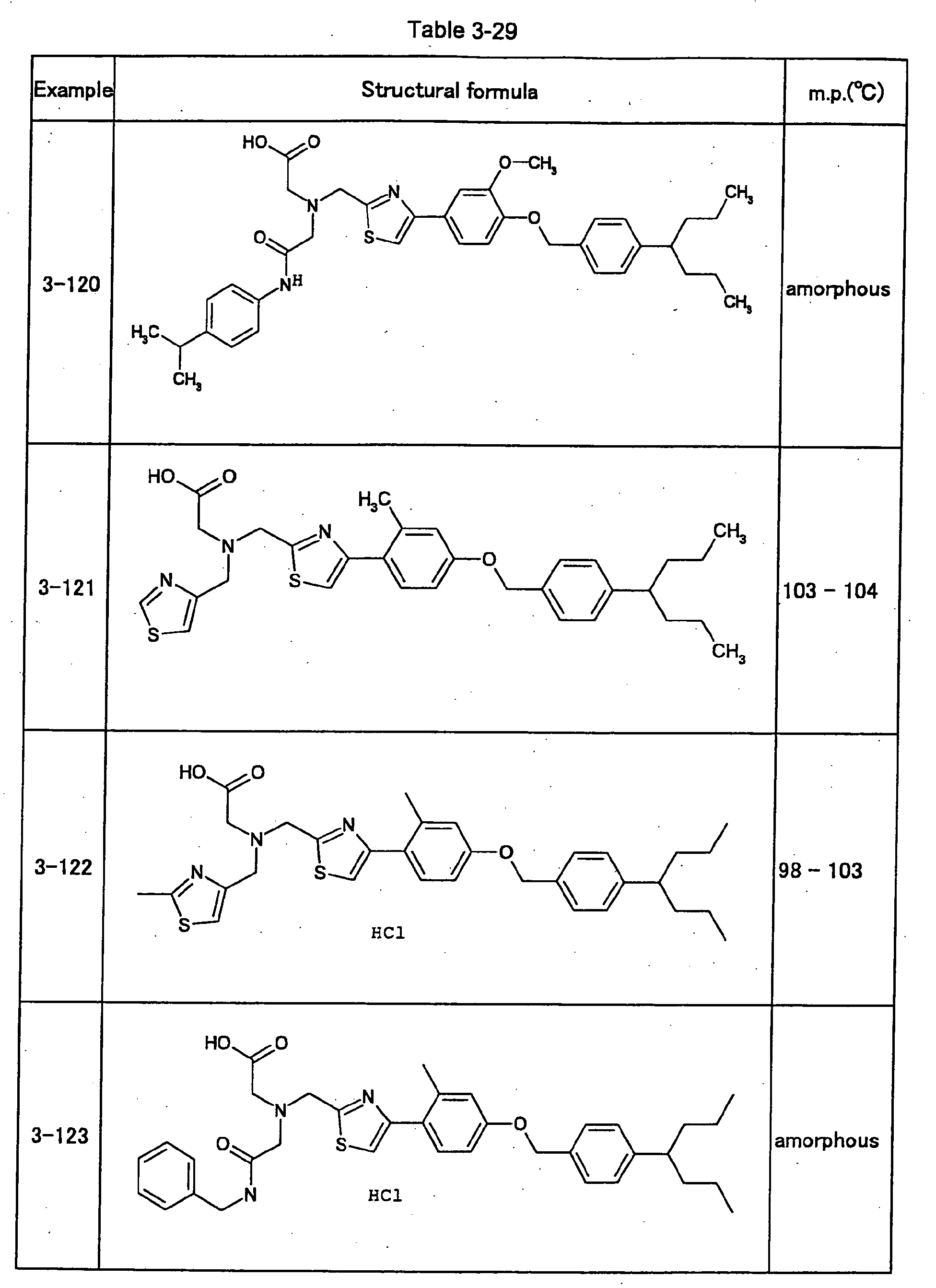

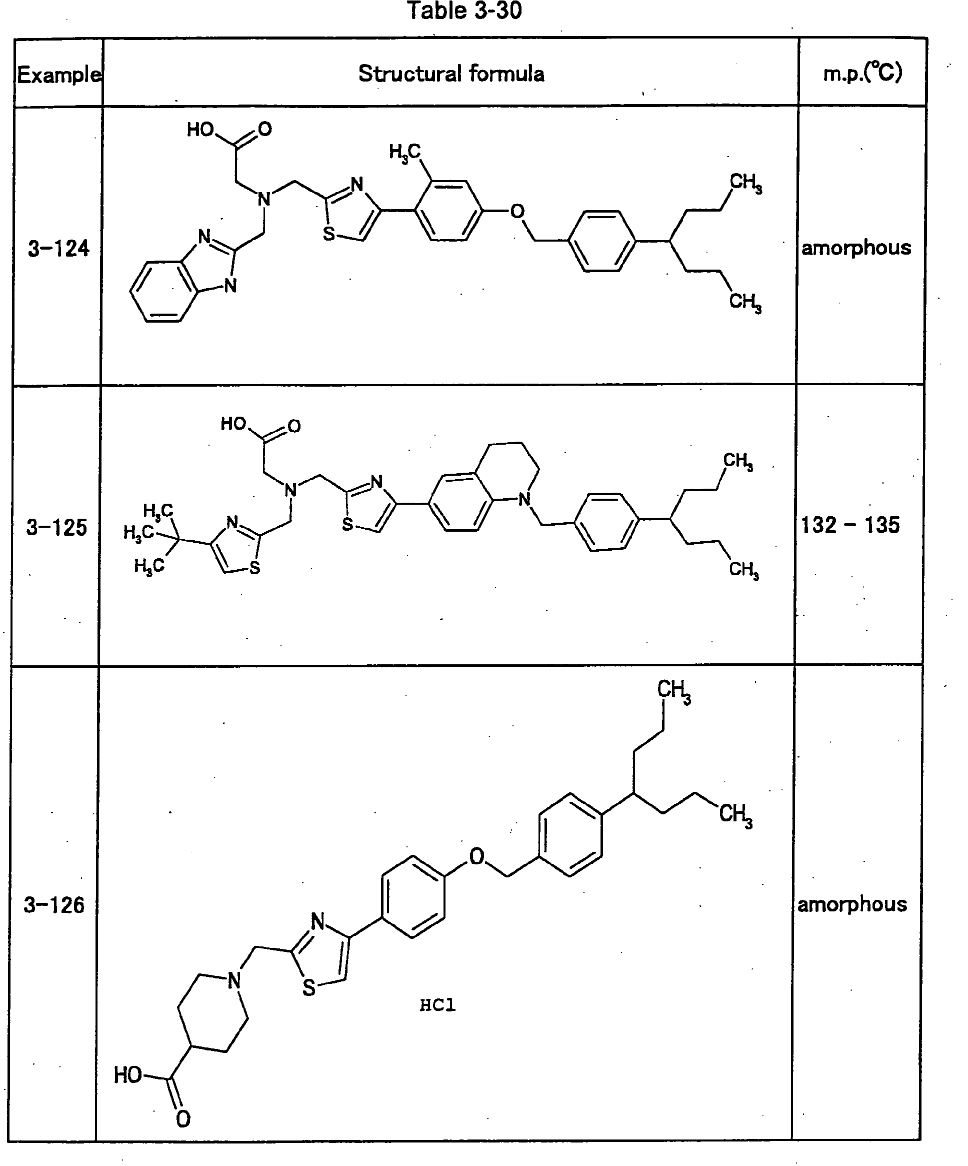

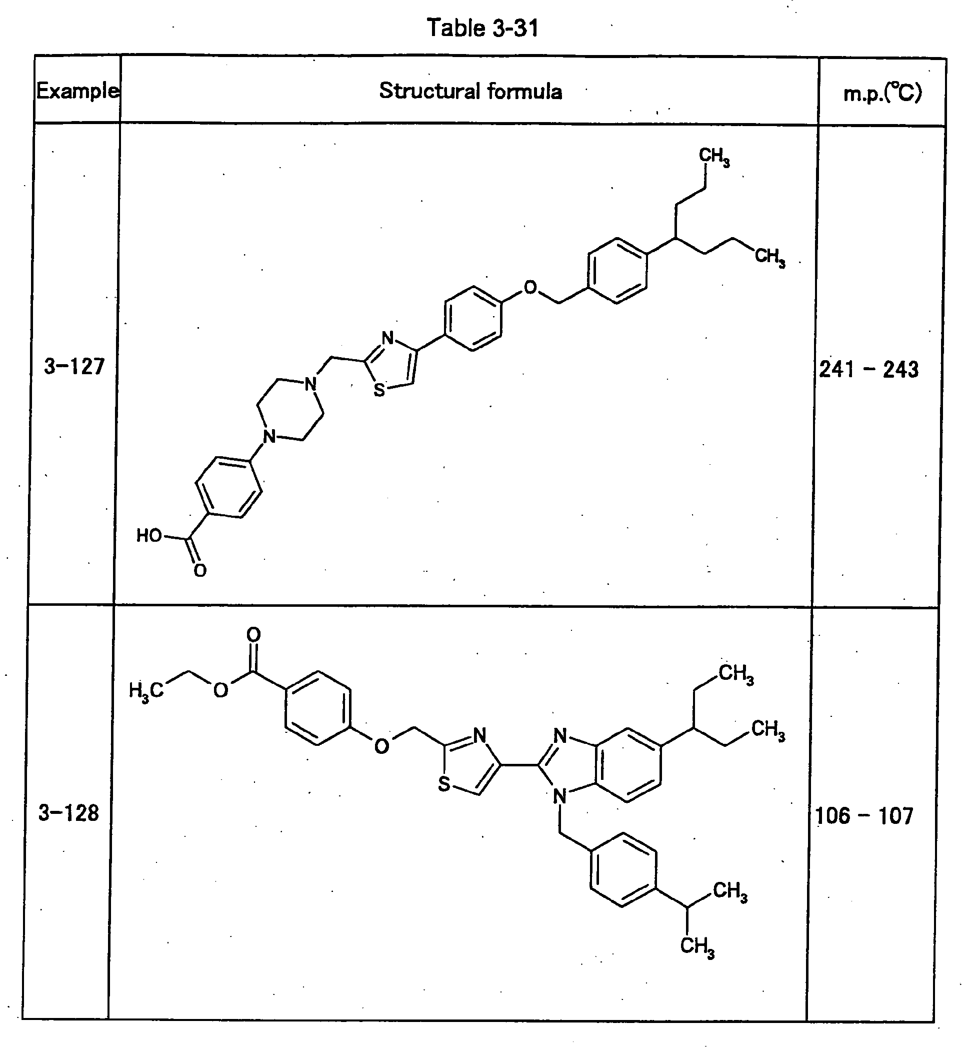

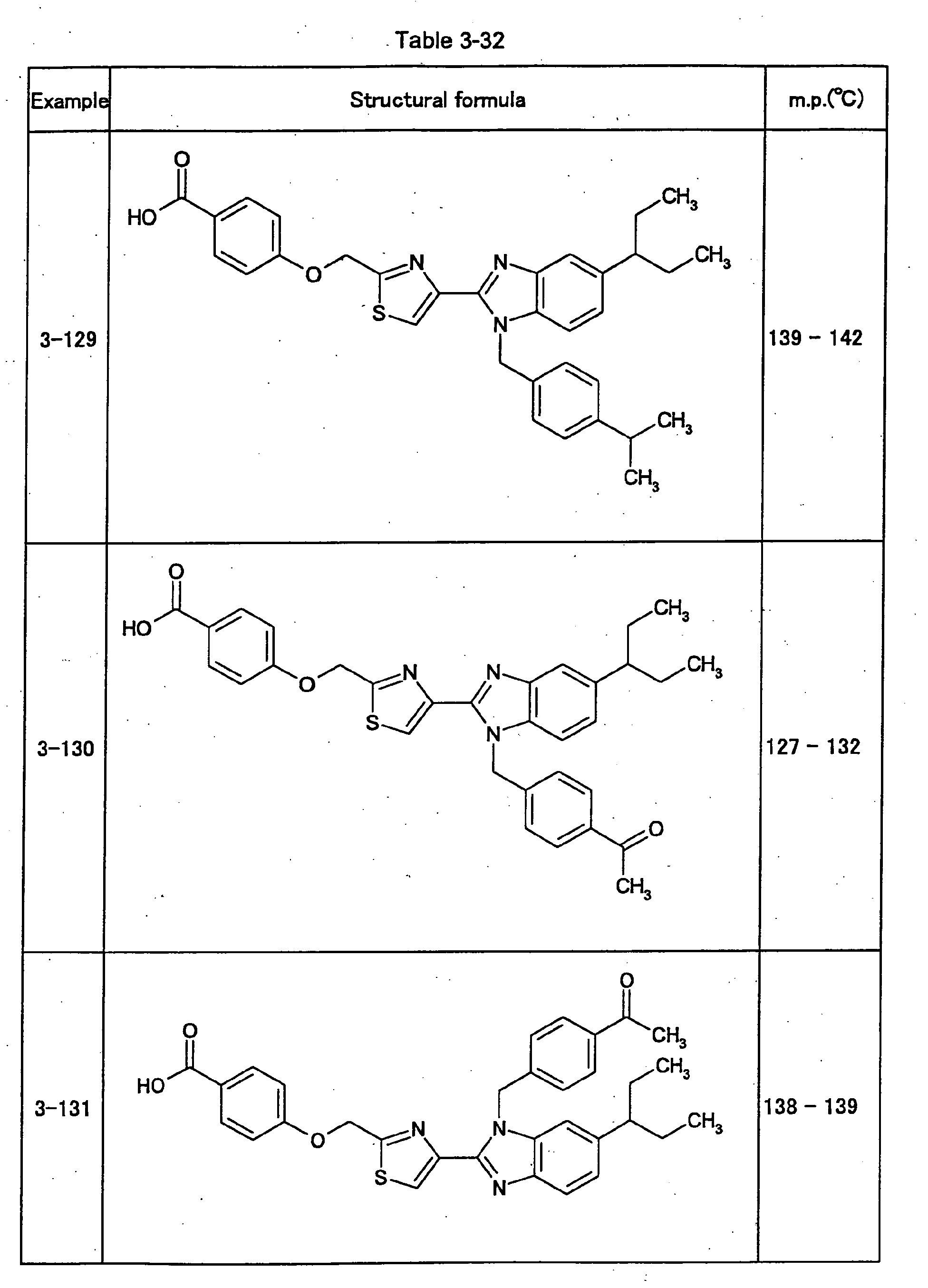

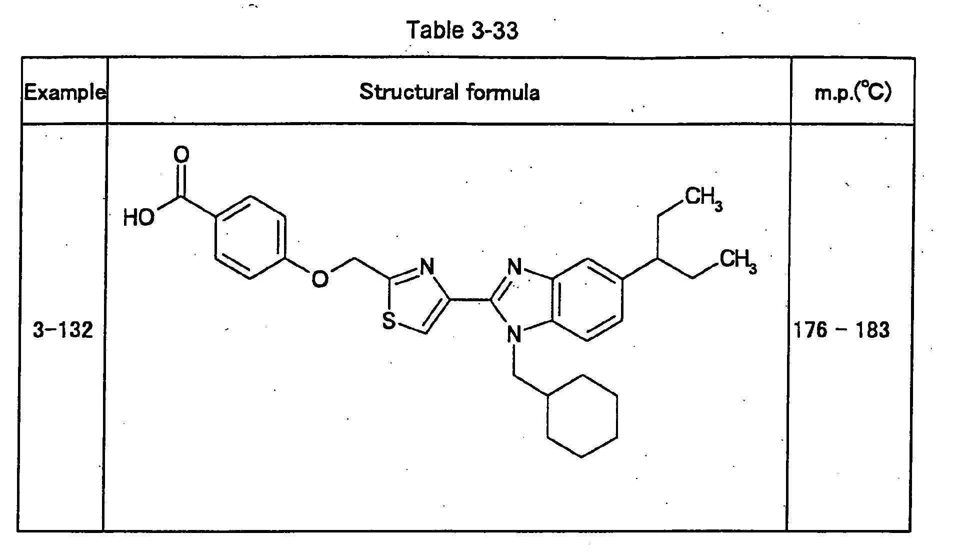

### Example 4-1

### [(1-Methyl-1H-benzimidazol-2-ylmethyl)-5-(4-[4-(1-propylbutyl)phenoxymethyl]phenyl}thiophen-2-ylmethyl]amino]acetic acid

(1) N-(1-Methyl-1H-benzimidazol-2-ylmethyl)glycine ethyl ester hydrochloride 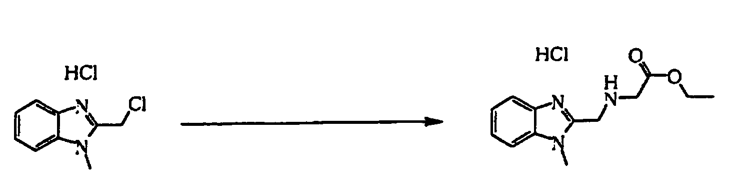
   To a suspension of 2-chloromethyl-1-methyl-1H-benzimidazole hydrochloride (81.135 g, 0.374 mmol) in acetonitrile (800 ml) were added glycine ethyl ester hydrochloride (156.5 g, 1.12 mol), potassium carbonate (284.1 g, 2.06 mol) and potassium iodide (6.2 g, 0.037 mol) and the mixture was stirred at 75°C for 1.5 hr. After the completion of the reaction, ethyl acetate and water were added for extraction, and the organic layer was washed with water and saturated brine and dried over sodium sulfate. The solvent was evaporated, and the obtained residue was dissolved in ethyl acetate. A 4N-hydrochloric acid/ethyl acetate solution (93 ml) was added and the solvent was evaporated. Ethyl acetate was added to the obtained residue and the resulting solids were collected by filtration to give the title compound (74.4 g, 70%).
(2) 5-(4-Hydroxymethylphenyl)thiophene-2-carbaldehyde 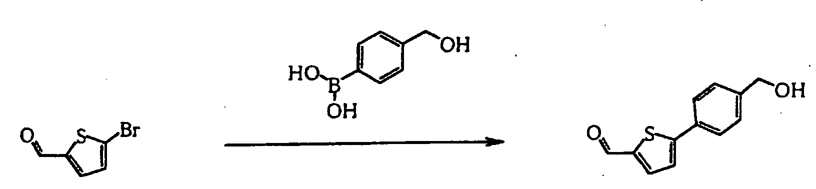
   To a suspension of 5-bromothiophene-2-carbaldehyde (125.7 g, 0.658 mol), 4-hydroxymethylphenylboronic acid (100 g, 0.658 mol) and potassium carbonate (136.43 g, 0.987 mol) in 50% methanol (880 ml) was added bis(triphenylphosphine)palladium(II) dichloride (4.62 g, 6.58 mmol) and the mixture was stirred at 80°C for 1.5 hr. After the completion of the reaction, the resulting solids were collected by filtration. The obtained solid was dried under reduced pressure to give the title compound (137.2 g, 96%).
(3) 5-(4-Chloromethylphenyl)thiophene-2-carbaldehyde 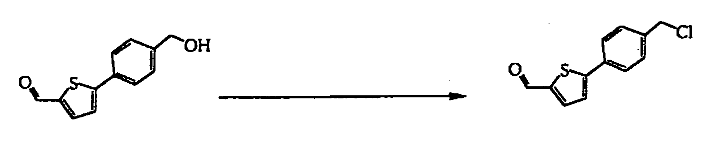
   To a solution of 5-(4-hydroxymethylphenyl)thiophene-2-carbaldehyde (20 g, 91.6 mmol) obtained in Example 4-1(2) and triethylamine (15.3 ml, 110 mmol) in acetonitrile was added dropwise methanesulfonyl chloride (8.51 ml, 110 mmol) under ice-cooling and the mixture was stirred at room temperature for 1 hr. Lithium chloride (7.77 g, 183.3 mmol) was added to the reaction mixture and the mixture was stirred at 55°C for 1 hr. After the completion of the reaction, water was added at room temperature and the resulting solids were collected by filtration. The obtained solid was dried under reduced pressure to give the title compound (19.3 g, 89%).
(4) 5-(4-(4-(1-Propylbutyl)phenoxymethyl)phenyl)thiophene-2-carbaldehyde 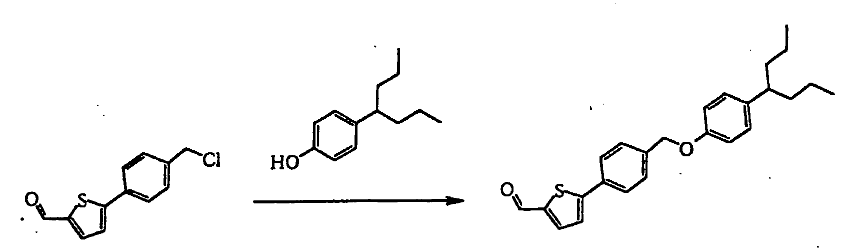
   To a solution of 4-(1-propylbutyl)phenol (15.6 g, 81.1 mmol) in N,N-dimethylacetamide (192 ml) were successively added 5-(4-chloromethylphenyl)thiophene-2-carbaldehyde (19.2 g, 81.1 mmol) obtained in Example 4-1(3), potassium carbonate (22.4 g, 162.2 mmol) and potassium iodide (2.69 g, 16.2 mmol) and the mixture was stirred at 90°C for 1.5 hr. After the completion of the reaction, ethyl acetate and water were added at room temperature and the mixture was extracted. The organic layer was washed successively with water and saturated brine and dried over magnesium sulfate. The solvent was evaporated and the obtained residue was stirred in hexane/diisopropyl ether=3/1. The resulting solids were collected by filtration and the obtained solid was dried under reduced pressure to give the title compound (25.9 g, 81%).
(5) (5-(4-(4-(1-Propylbutyl)phenoxymethyl)phenyl)thiophen-2-yl)methanol 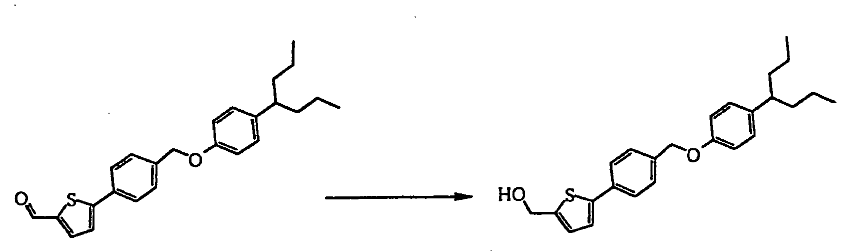
   To a solution of 5-(4-(4-(1-propylbutyl)phenoxymethyl)phenyl)thiophene-2-carbaldehyde (25.9 g, 66 mmol) obtained in Example 4-1(4) in tetrahydrofuran (260 ml) and ethanol (260 ml) was added sodium borohydride (2.5 g, 66 mmol) under ice-cooling and the mixture was stirred at room temperature for 0.5 hr. After the completion of the reaction, the solvent was partially concentrated. To the obtained residue were added ethyl acetate, a saturated aqueous sodium hydrogen carbonate solution and water and the mixture was stirred for 0.5 hr. The mixture was extracted and the organic layer was washed with saturated brine, and dried over magnesium sulfate. The solvent was evaporated and the obtained residue was purified by silica gel column chromatography (chloroform:methanol=50:1→10:1) to give the title compound (23.6 g, 91%).
(6) (5-(4-(4-(1-Propylbutyl)phenoxymethyl)phenyl)thiophen-2-yl)methyl chloride 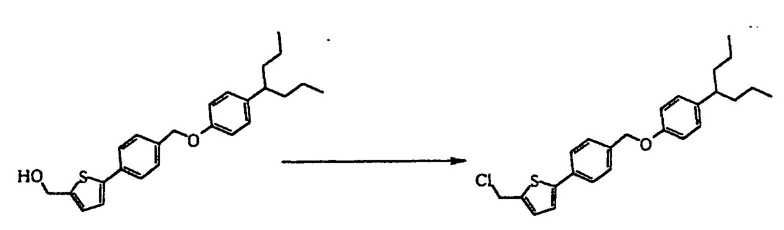
   To a solution of (5-(4-(4-(1-propylbutyl)phenoxymethyl)phenyl)thiophen-2-yl)methanol (23.6 g, 59.8 mmol) obtained in Example 4-1(5) in chloroform (354 ml) was added thionyl chloride (8.73 ml, 119.6 mmol) at room temperature and the mixture was stirred at the same temperature for 1 hr. After the completion of the reaction, the solvent was evaporated and the obtained residue was concentrated by adding chloroform and dried under reduced pressure to give the title compound (24.6 g, 100%).
(7) [(1-Methyl-1H-benzimidazol-2-ylmethyl)-5-{4-[4-(1-propylbutyl)phenoxymethyl]phenyl}thiophen-2-ylmethyl]amino]acetic acid ethyl ester 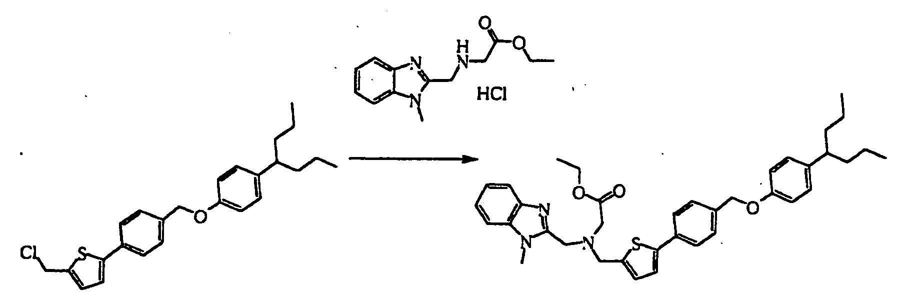
   To a solution of (5-(4-(4-(1-propylbutyl)phenoxymethyl)phenyl)thiophen-2-yl)methyl chloride (0.687 g, 1.66 mmol) obtained in Example 4-1(6) in acetonitrile were added N-(1-methyl-1H-benzimidazol-2-ylmethyl)glycine ethyl ester hydrochloride (0.674 g, 1.83 mmol) obtained in Example 4-1(1), potassium carbonate (0.69 g, 5.0 mmol) and potassium iodide (0.055 g, 0.33 mmol) and the mixture was stirred at 80°C for 1.5 hr. After the completion of the reaction, ethyl acetate and water were added. The mixture was extracted and the organic layer was washed with saturated brine and dried over magnesium sulfate. The solvent was evaporated, and the obtained residue was purified by silica gel column chromatography (chloroform:methanol=100:1→20:1) to give the title compound (0.633 g, 61%).
(8) [(1-Methyl-1H-benzimidazol-2-ylmethyl)-5-{4-[4-(1-propylbutyl)phenoxymethyl]phenyl}thiophen-2-ylmethyl]amino]acetic acid

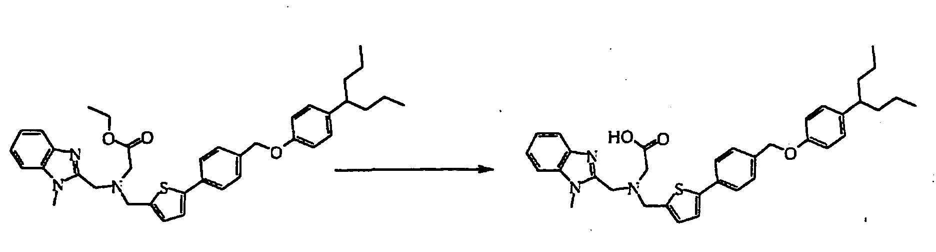

To a solution of [(1-methyl-1H-benzimidazol-2-ylmethyl)-5-{4-[4-(1-propylbutyl)phenoxymethyl]phenyl}thiophen-2-ylmethyl]amino]acetic acid ethyl ester (5 g, 8.01 mmol) obtained in Example 4-1(7) in 50% tetrahydrofuran-ethanol (100 ml) was added a 1N-aqueous sodium hydroxide solution (16 ml) at room temperature, and the mixture was stirred at 80°C for 1 hr. After the completion of the reaction, a 1N-hydrochloric acid (16 ml) was added under ice-cooling, and the mixture was stirred at room temperature. The resulting solids were collected by filtration. The obtained solid was dried under reduced pressure to give the title compound (4.63 g, 97%).
¹H NMR (DMSO-d₆, 300 MHz) δ 0.79 (t, J=7.3Hz, 6H), 0.96-1.18(m, 4H), 1.34-1.60(m, 4H), 3.36(s, 2H), 3.88(s, 3H), 4.04(s, 2H), 4.16(s, 2H), 5.06(s, 2H), 6.92(d, J=8.7Hz, 2H), 7.01(d, J=3.4Hz, 1H), 7.07(d, J=8.7Hz, 2H), 7.14-7.26(m, 2H), 7.35(d, J=3.4Hz, 1H), 7.46(d, J=8.3Hz, 2H), 7.53(d, J=7.5Hz, 1H), 7.58-7.65(m, 3H), 12. 52 (br s, 1H)

### Example 4-40

### [(1-Methyl-1H-benzimidazol-2-ylmethyl)-5-{4-[4-(1-propylbutyl)phenoxymethyl]phenyl}thiophen-2-ylmethyl]amino]acetic acid p-toluenesulfonate

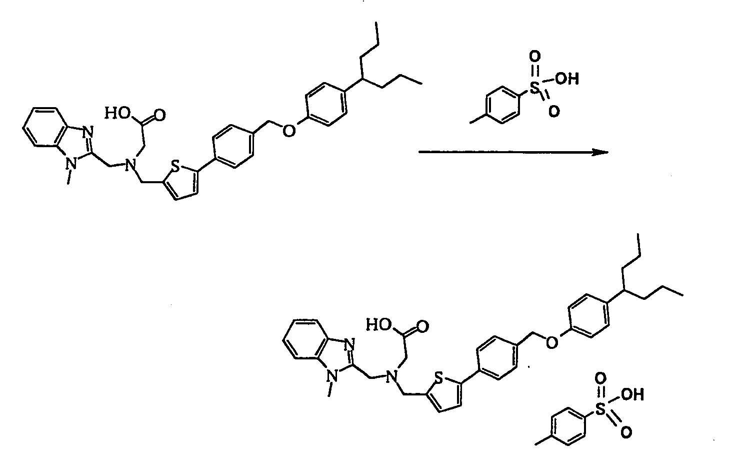

To a suspension of [(1-methyl-1H-benzimidazol-2-ylmethyl)-5-{4-[4-(1-propyl-butyl)phenoxymethyl]phenyl}thiophen-2-ylmethyl]amino]acetic acid (200 mg, 0.336 mmol) obtained in Example 4-1 in 2-butanone (5 ml) was added p-toluenesulfonic acid monohydrate (64 mg, 0.37 mmol) and the mixture was stirred at room temperature for 30 min. The crystals were collected by filtration and dried over to give the title compound (231 mg, 90%).
¹H NMR (DMSO-d₆, 300 MHz) δ 0.79(t, J=7.3Hz, 6H), 0.95-1.20(m, 4H), 1.32-1.60(m, 4H), 2.28(s, 3H), 2.40-2.54(m, 1H), 3.62(s, 2H), 4.00(s, 3H), 4.19(s, 2H), 4.46(s, 2H), 5.05(s, 2H), 6.92(d, J=8.6Hz, 2H), 6.98-7.14(m, 5H), 7.25(d, J=3.6Hz, 1H), 7.36-7.58(m, 8H), 7.76(d, J=9.0Hz, 1H), 7.89(d, J=9.0Hz, 1H)
melting point: 100°C-135°C

### Example 4-2

### 5-(4-{4-[4-(1-Propylbutyl)phenoxymethyl]phenyl}thiophen-2-ylmethoxy)nicotinic acid

(1) 4-(4-Hydroxymethylphenyl)thiophene-2-carbaldehyde 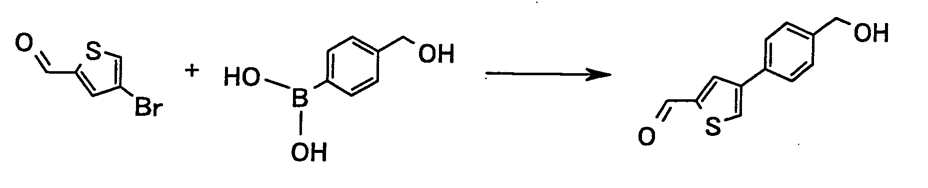
   To a solution of 4-bromothiophene-2-carbaldehyde (100 g, 0.523 mol) in tetrahydrofuran (500 ml) were added bis(triphenylphosphine)palladium(II) dichloride (3.67 g, 52.3 mmol), 4-hydroxymethylphenylboronic acid (79.5 g, 0.523 mol), sodium hydrogen carbonate (66.0 g, 0.786 mol) and water (1000 ml) and the mixture was stirred at an internal temperature of 62-63°C for 3 hr. After allowing to cool, ethyl acetate was added to the reaction mixture. The organic layer was washed with saturated brine and concentrated to give a crude product (140 g). To the obtained crude product were added toluene (400 ml) and hexane (100 ml) and the mixture was stirred. The solids were collected by filtration and dried to give the title compound (95.4 g, 83%).
(2) 4-(4-Chloromethylphenyl)thiophene-2-carbaldehyde 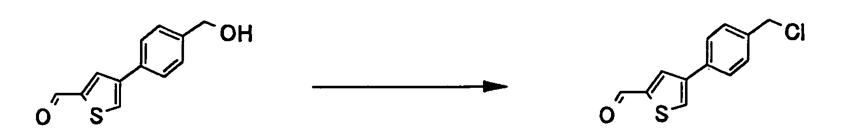
   To a solution of 4-(4-hydroxymethylphenyl)thiophene-2-carbaldehyde (95.4 g, 0.437 mol) and triethylamine (73.1 ml, 0.524 mol) in N,N-dimethylformamide (763 ml) was added dropwise methanesulfonyl chloride (40.6 ml, 0.524 mol) under ice-cooling and the mixture was stirred at room temperature for 2 hr. Lithium chloride (37.1 g, 0.874 mol) was added to the reaction mixture and the mixture was stirred at 60°C for 1 hr, After allowing to cool, water was added and the resulting solids were collected by filtration. The obtained solid was suspended in diisopropyl ether (465 ml) and the mixture was stirred at an internal temperature of 60-65°C for 1.5 hr. After allowing to cool, the solids were collected by filtration and dried to give the title compound (83.2 g, 80%).
(3) (4-(4-(4-(1-Propylbutyl)phenoxymethyl)phenyl)thiophen-2-yl)methanol 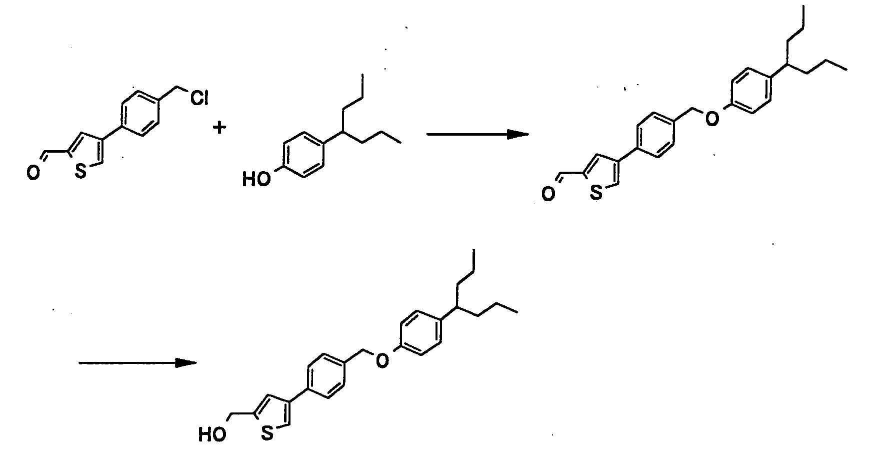
   To a solution of 4-(4-chloromethylphenyl)thiophene-2-carbaldehyde (21.1 g, 88.9 mmol) in N,N-dimethylacetamide (105 ml) were successively added 4-(1-propylbutyl)phenol (18.7 g, 97.8 mmol), potassium carbonate (36.8 g, 267 mmol) and potassium iodide (1.47 g, 8.89 mmol) and the mixture was stirred at 90°C for 1.5 hr. After the completion of the reaction, ethyl acetate and water were added at room temperature. The mixture was extracted and the organic layer was washed successively with water and saturated brine and dried over magnesium sulfate. The solvent was evaporated and the obtained residue (68 g) was dissolved in ethanol (100 ml). Sodium borohydride (37.8 g, 88.9 mmol) was added under ice-cooling and the mixture was stirred at room temperature for 1 hr. Water and ethyl acetate were added to the reaction mixture and, the organic layer was washed successively with a 1N-aqueous sodium hydroxide solution, water and saturated brine and dried over magnesium sulfate. The solvent was evaporated and the obtained residue was purified by silica gel column chromatography (chloroform:methanol=100:1→50:1) to give a crude product (29.9 g). The crude product (14.5 g) was purified by silica gel column chromatography (ethyl acetate:hexane=1:5→1:4) to give the title compound (11.9 g).
(4) (4-(4-(4-(1-Propylbutyl)phenoxymethyl)phenyl)thiophen-2-yl)methyl chloride 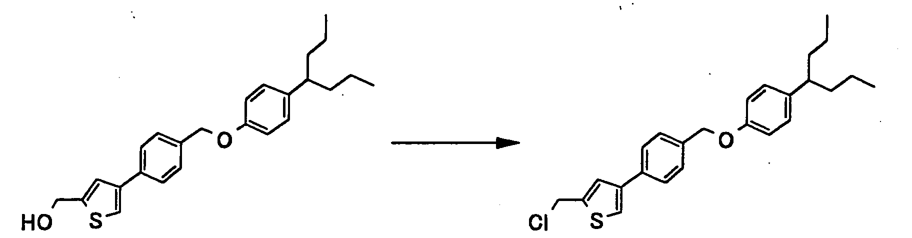
   To a solution of (4-(4-(4-(1-propylbutyl)phenoxymethyl)-phenyl)thiophen-2-yl)methanol (11.9 g, 30.2 mmol) in chloroform (60 ml) was added thionyl chloride (4.40 ml, 60.4 mmol) and the mixture was stirred at room temperature for 30 min. The solvent was evaporated and thionyl chloride (10.0 ml, 137 mmol) was added. The mixture was stirred at room temperature for 20 min. The reaction mixture was concentrated under pressure and the obtained residue was concentrated by adding chloroform to give the title compound (12.5 g, 100%).
(5) 5-(4-{4-[4-(1-Propylbutyl)phenoxymethyl]phenyl}thiophen-2-ylmethoxy)nicotinic acid methyl ester 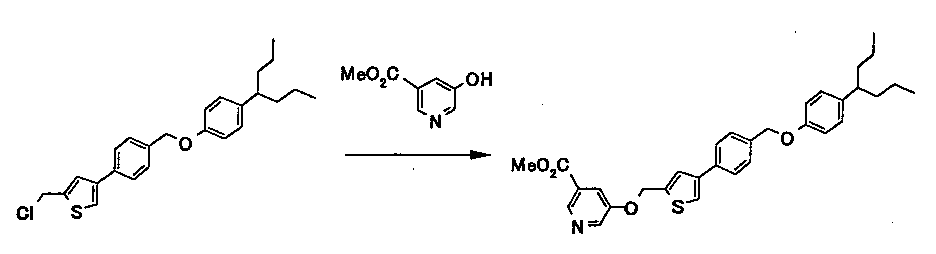
   To (4-(4-(4-(1-propylbutyl)phenoxymethyl)phenyl)thiophen-2-yl)methyl chloride (5.00 g, 12.1 mmol) were added N,N-dimethylacetamide (25 ml), 5-hydroxynicotinic acid methyl ester (2.04 g, 13.3 mmol), cesium carbonate (11.8 g, 36.3 mmol), potassium iodide (201 mg, 1.21 mmol) and tetra-n-butylammonium bromide (390 mg, 1.21 mmol) and the mixture was stirred at 60°C for 1.5 hr. After allowing to cool, ethyl acetate was added and the mixture was washed successively with water and saturated brine. After concentration, the concentrate was purified by silica gel column chromatography (ethyl acetate:hexane=2:5→1:2) to give the title compound (5.19 g, 81%).
(6) 5-(4-{4-[4-(1-Propylbutyl)phenoxymethyl]phenyl}thiophen-2-ylmethoxy)nicotinic acid

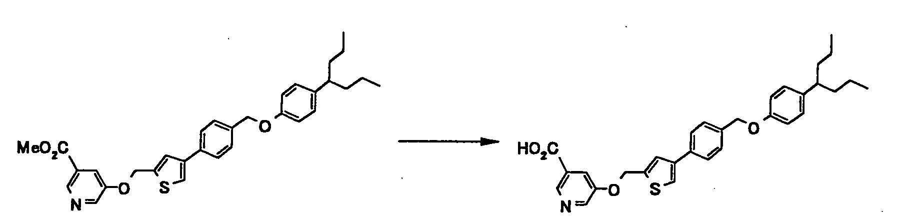

To 5-(4-{4-[4-(1-propylbutyl)phenoxymethyl]phenyl}thiophen-2-ylmethoxy)nicotinic acid methyl ester (5.04 g, 9.52 mmol) were added tetrahydrofuran (25 ml), ethanol (25 ml) and a 2N-aqueous sodium hydroxide solution (5.00 ml, 10 mmol) and the mixture was stirred at 60°C for 1.5 hr. After allowing to cool, 2N-hydrochloric acid (5.00 ml, 10 mmol) and water (30 ml) were added. The precipitated solids were collected by filtration and dried to give the title compound (4.77 g, yield 97%).
¹H NMR (DMSO-d₆, 300 MHz) δ 0.80(t, J=7.3Hz, 6H), 1.00-1.12(m, 4H), 1.38-1.57(m, 4H), 2.40-2.50(m, 1H), 5.07(s, 2H), 5.48(s, 2H), 6.93(d, J=8.6Hz, 2H), 7.07(d, J=8.6Hz, 2H), 7.49(d, J=7.9Hz, 2H), 7.70-7.70(m, 1H), 7.72(d, J=8.3Hz, 2H), 7.88-7.89(m, 1H), 7.91(d, J=1.5Hz, 1H), 8.56(d, J=2.6Hz, 1H), 8.69(d, J=1.5Hz, 1H), 13.41(brs, 1H)
melting point: 157-159°C

### Example 4-41

### 5-(4-{4-[4-(1-Propylbutyl)phenoxymethyl]phenyl}thiophen-2-ylmethoxy)nicotinic acid sulfate

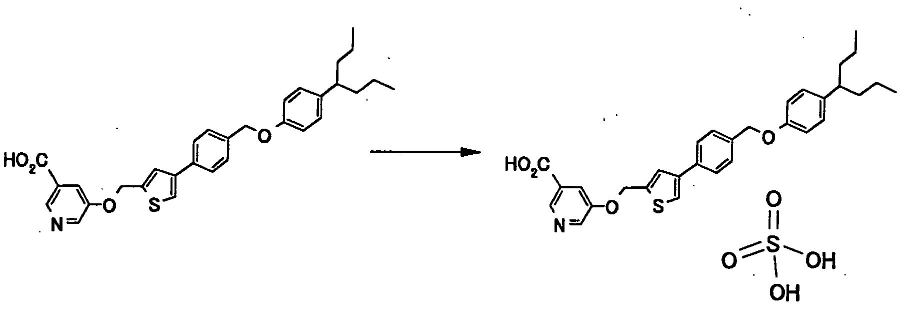

To a suspension of 5-(4-(4-[4-(1-propylbutyl)-phenoxymethyl]phenyl}thiophen-2-ylmethoxy)nicotinic acid (200 mg, 0.388 mmol) obtained in Example 4-2 in 2-butanone (2 ml) was added concentrated sulfuric acid (0.0207 ml, 0.388 mmol) and the mixture was stirred at room temperature for 1 hr. The solids were collected by filtration and dried to give the title compound (224 mg, yield 94%).
¹H NMR (DMSO-d₆, 300 MHz) 0. 80 (t, J=7.3Hz, 6H), 0.98-1.15(m, 4H), 1.38-1.59(m, 4H), 2.39-2.54(m, 1H), 5.07 (s, 2H), 5.52(s, 2H), 6.93(d, J=8.6Hz, 2H), 7.07(d, J=8.7Hz, 2H), 7.49(d, J=8.3Hz, 2H), 7.71(s, 1H), 7.72(d, J=6.4Hz, 2H), 7.93(d, J=1.5Hz, 1H), 8.00(brs, 1H), 8.65-8.67(m, 1H), 8.73-8.76(m, 1H)
melting point: 125°C (dec.)

### Examples 4-3 to 4-39 and 4-42 to 4-107

In the same manner as in Examples 4-1, 4-2, 4-40 and 4-41 and using other conventional methods where necessary, the compounds of Examples 4-3 to 4-39 and Examples 4-42 to 4-107 were produced. The structural formulas and property values of the obtained compounds, as well as those of Examples 4-1, 4-2, 4-40 and 4-41, are shown in the following Tables.

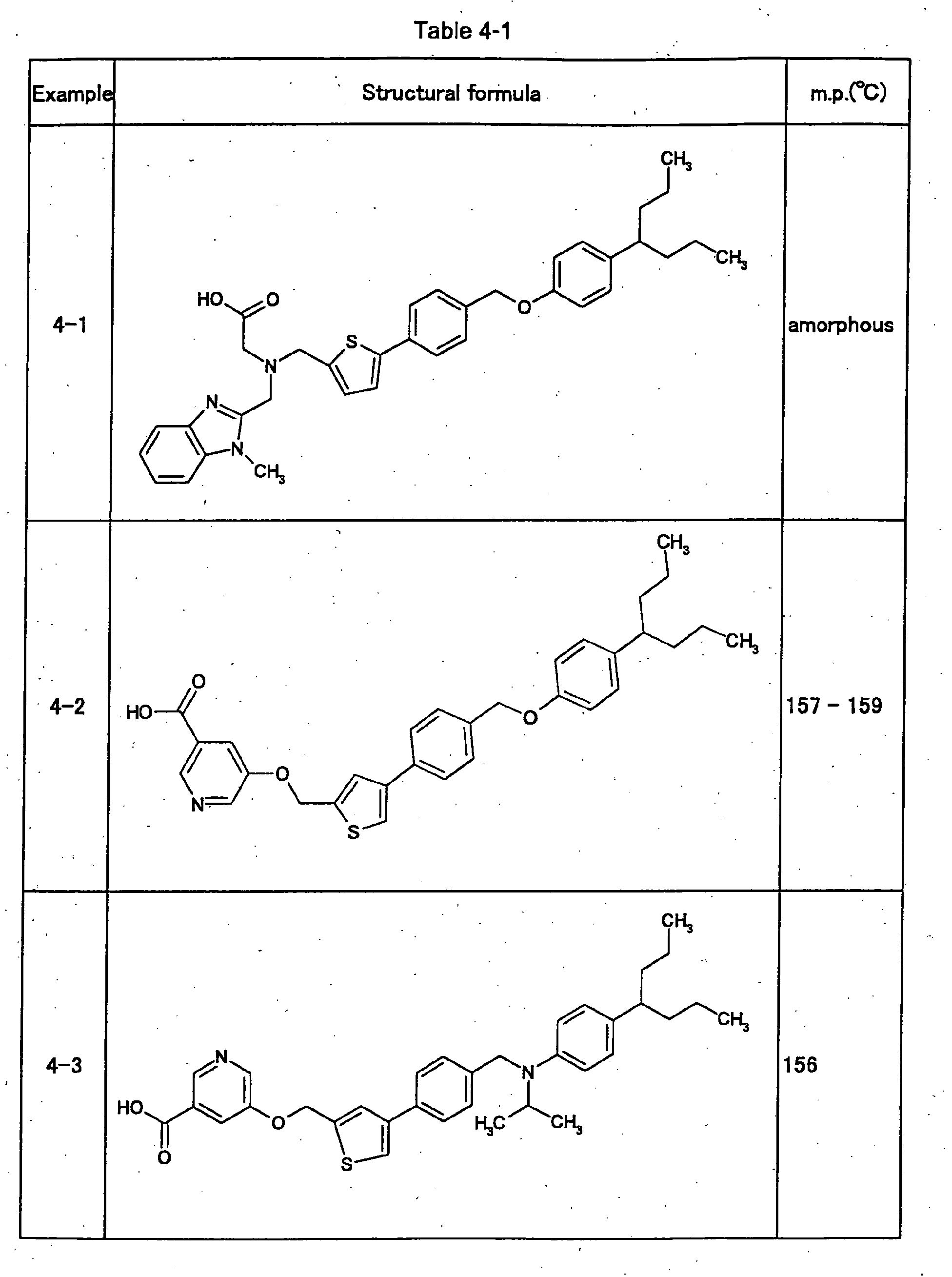

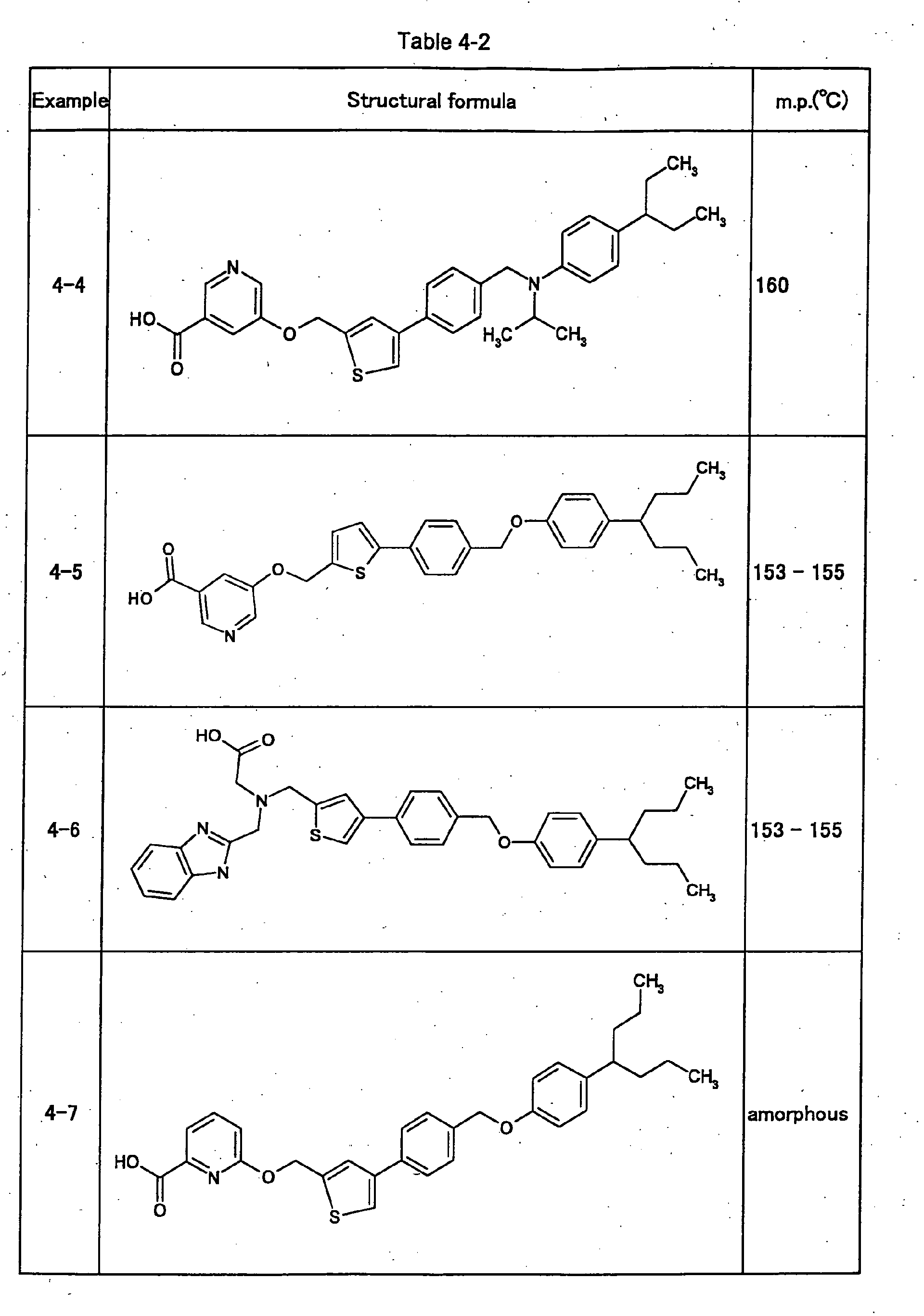

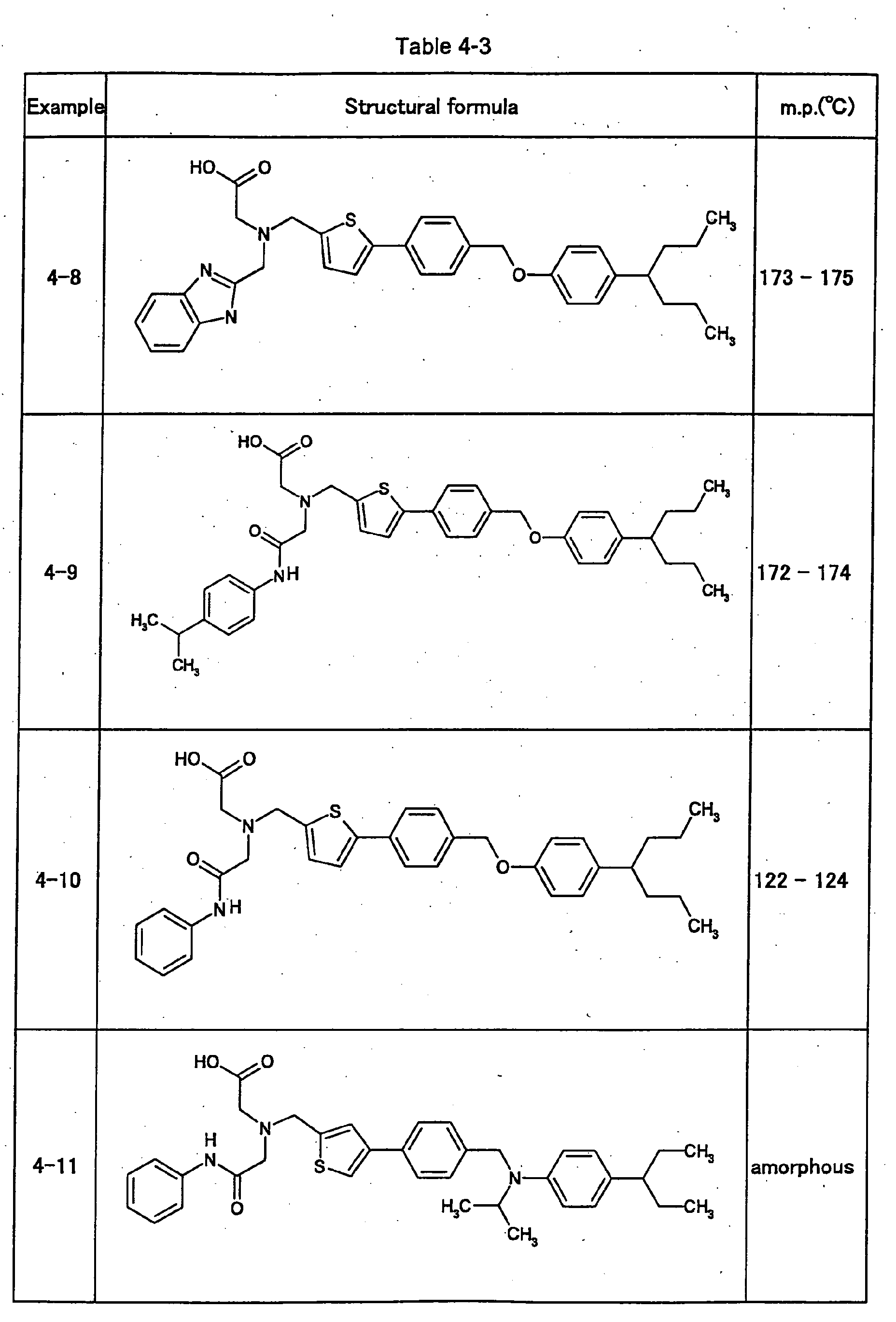

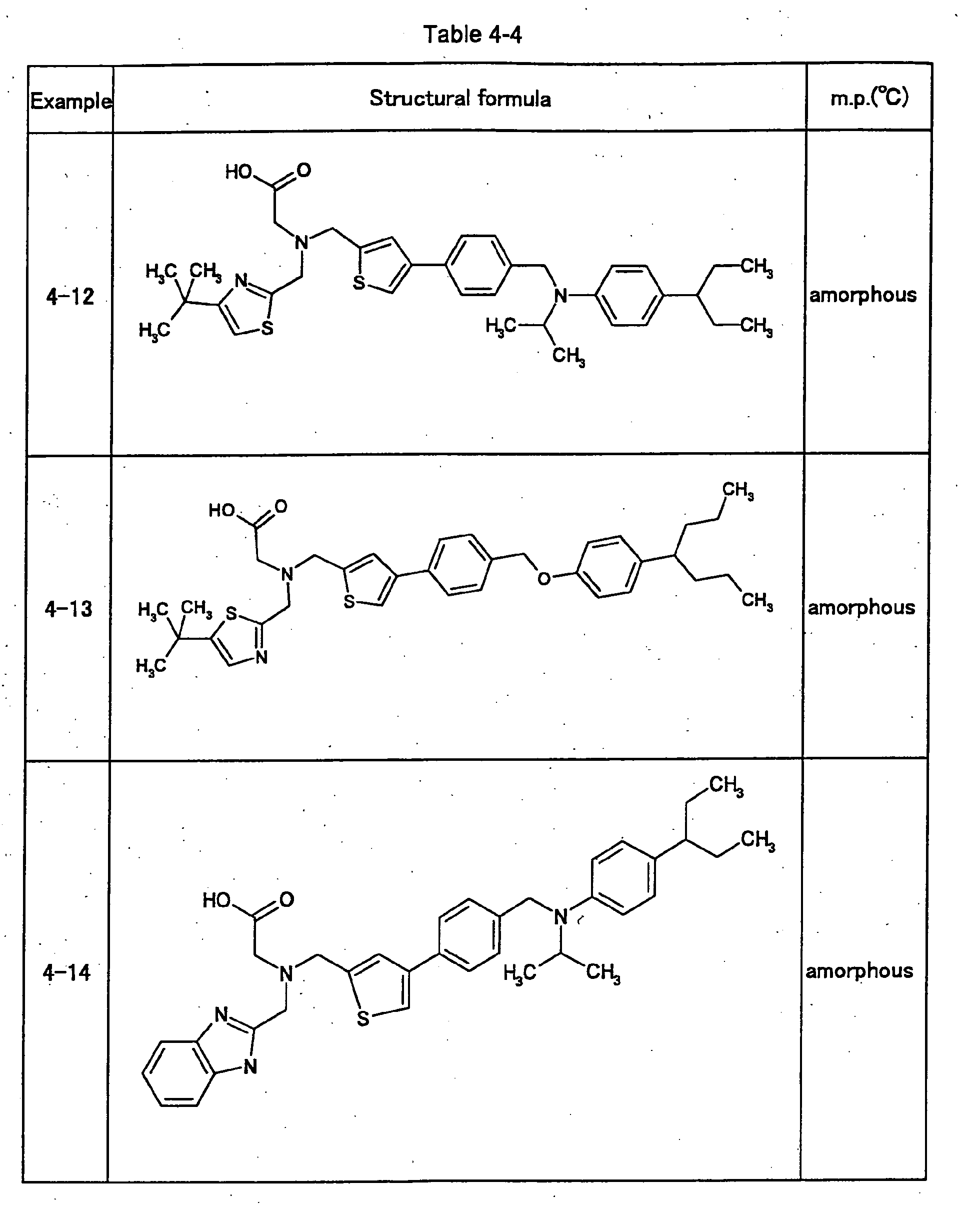

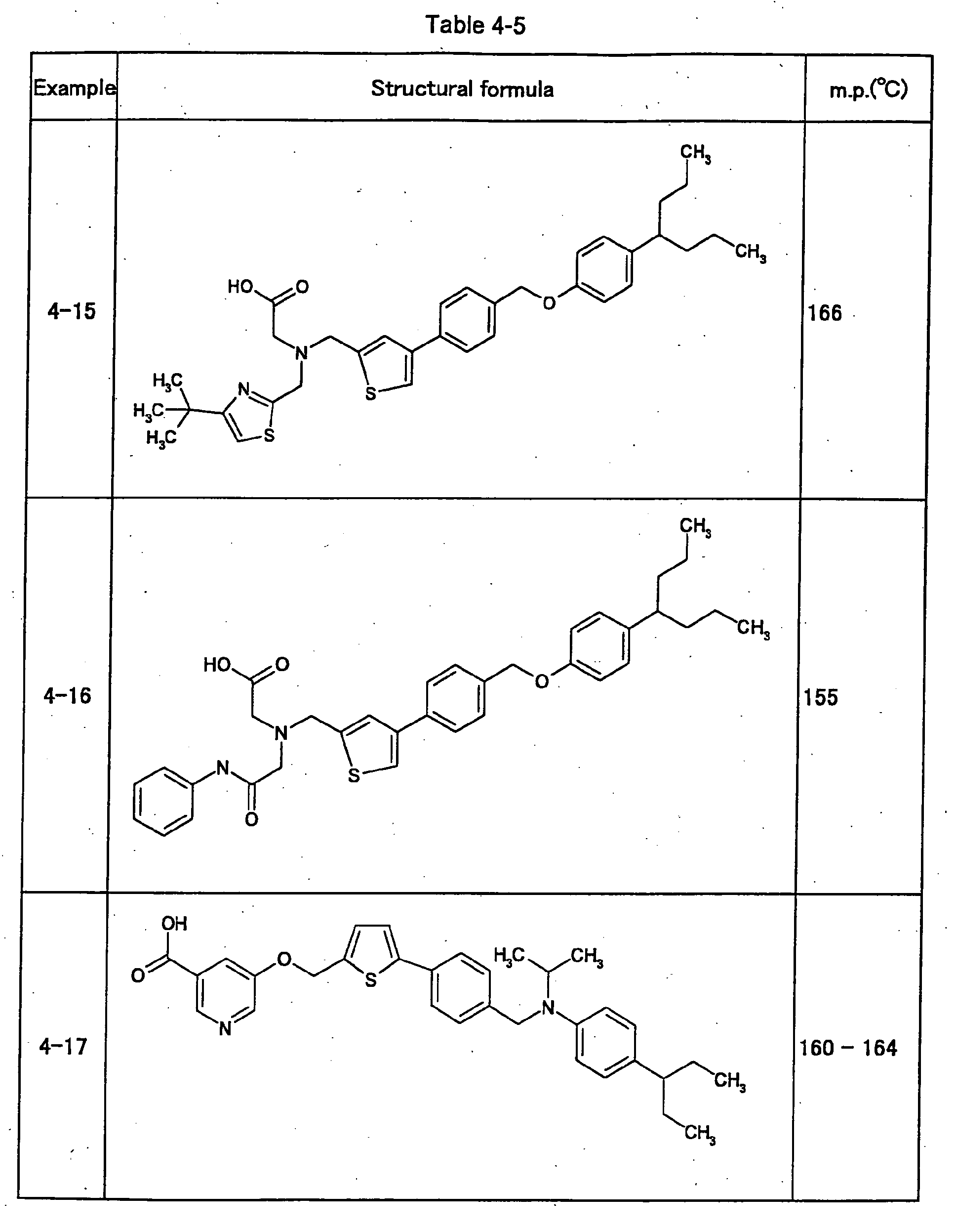

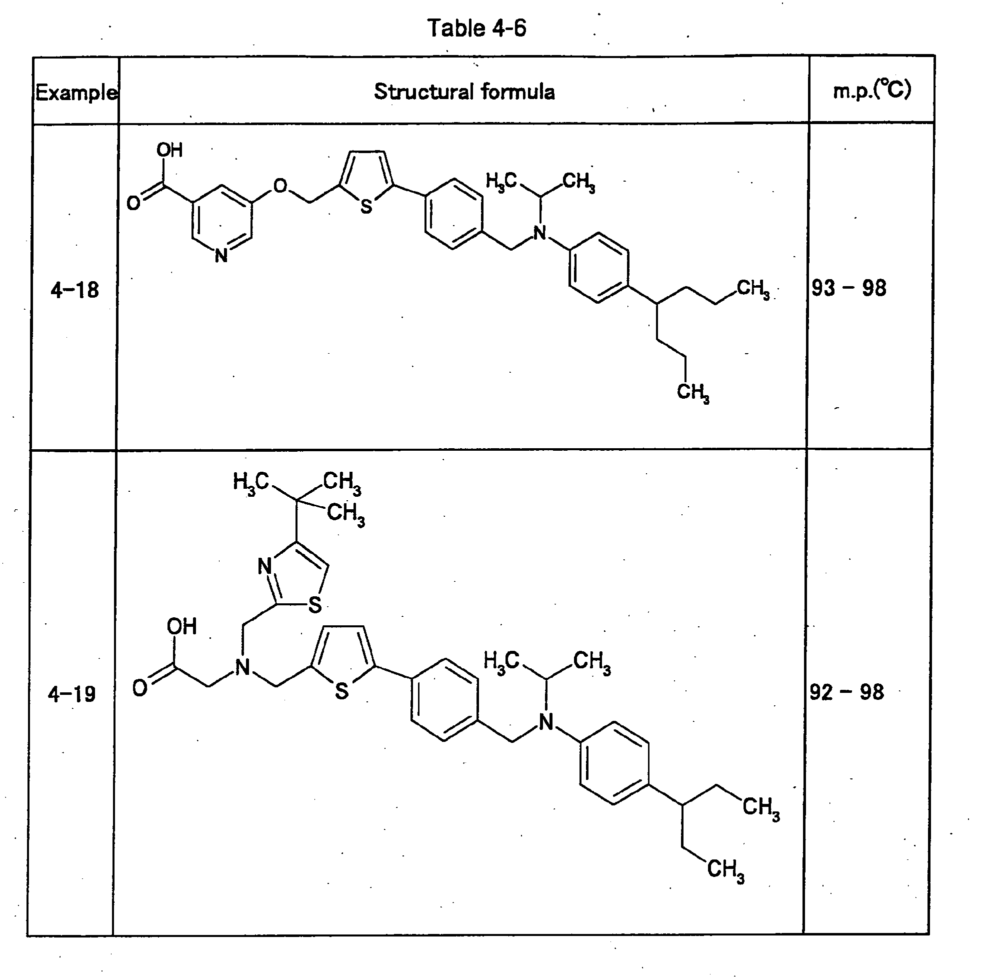

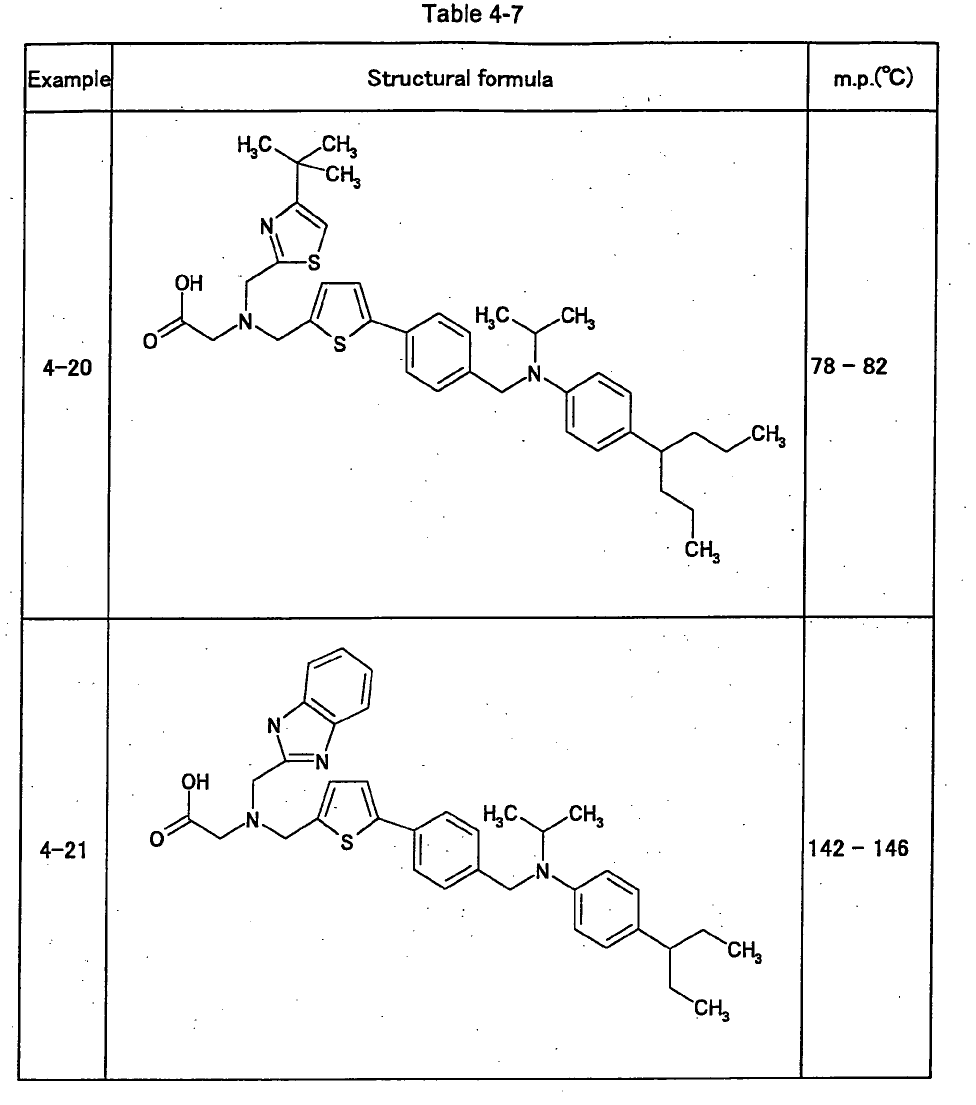

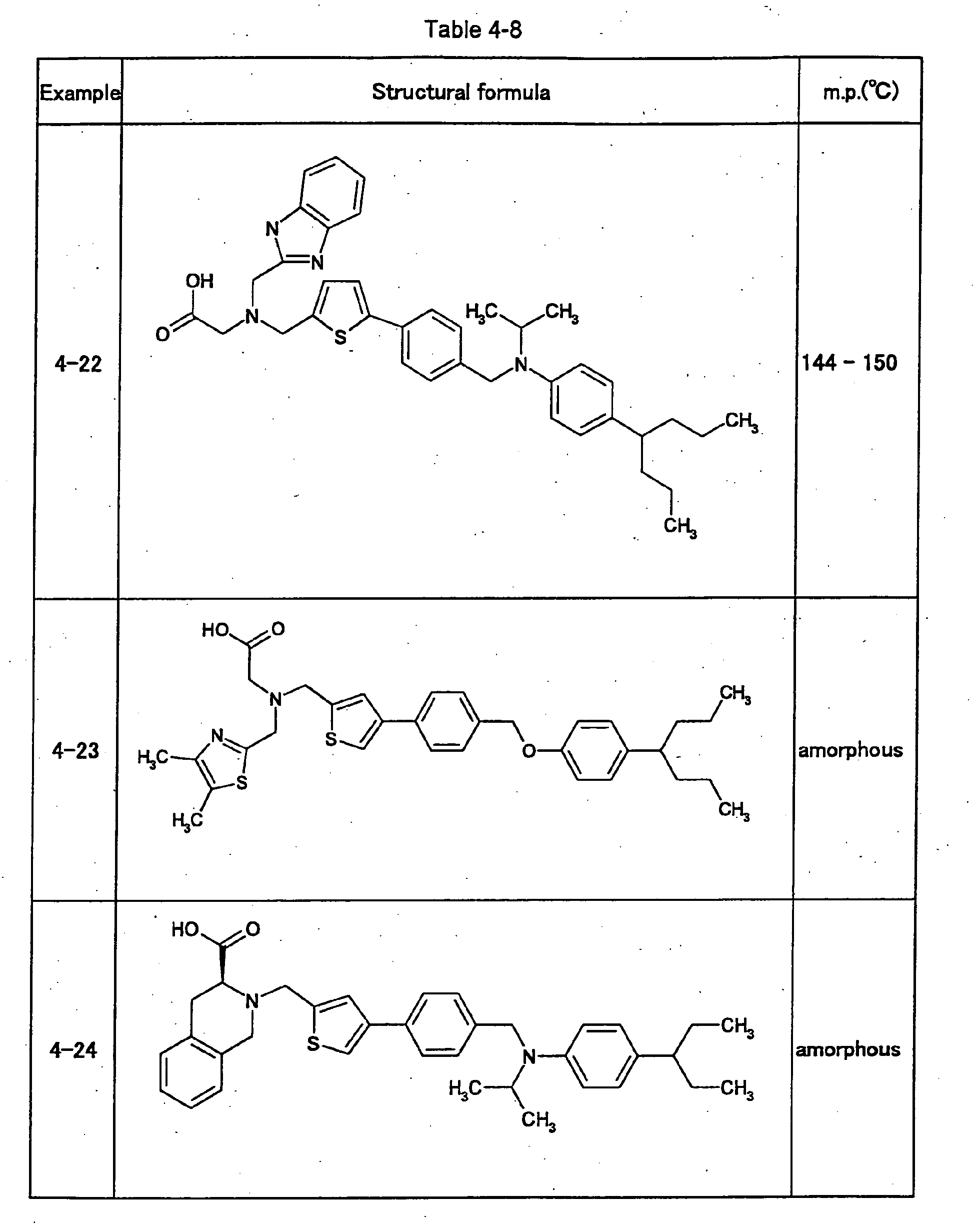

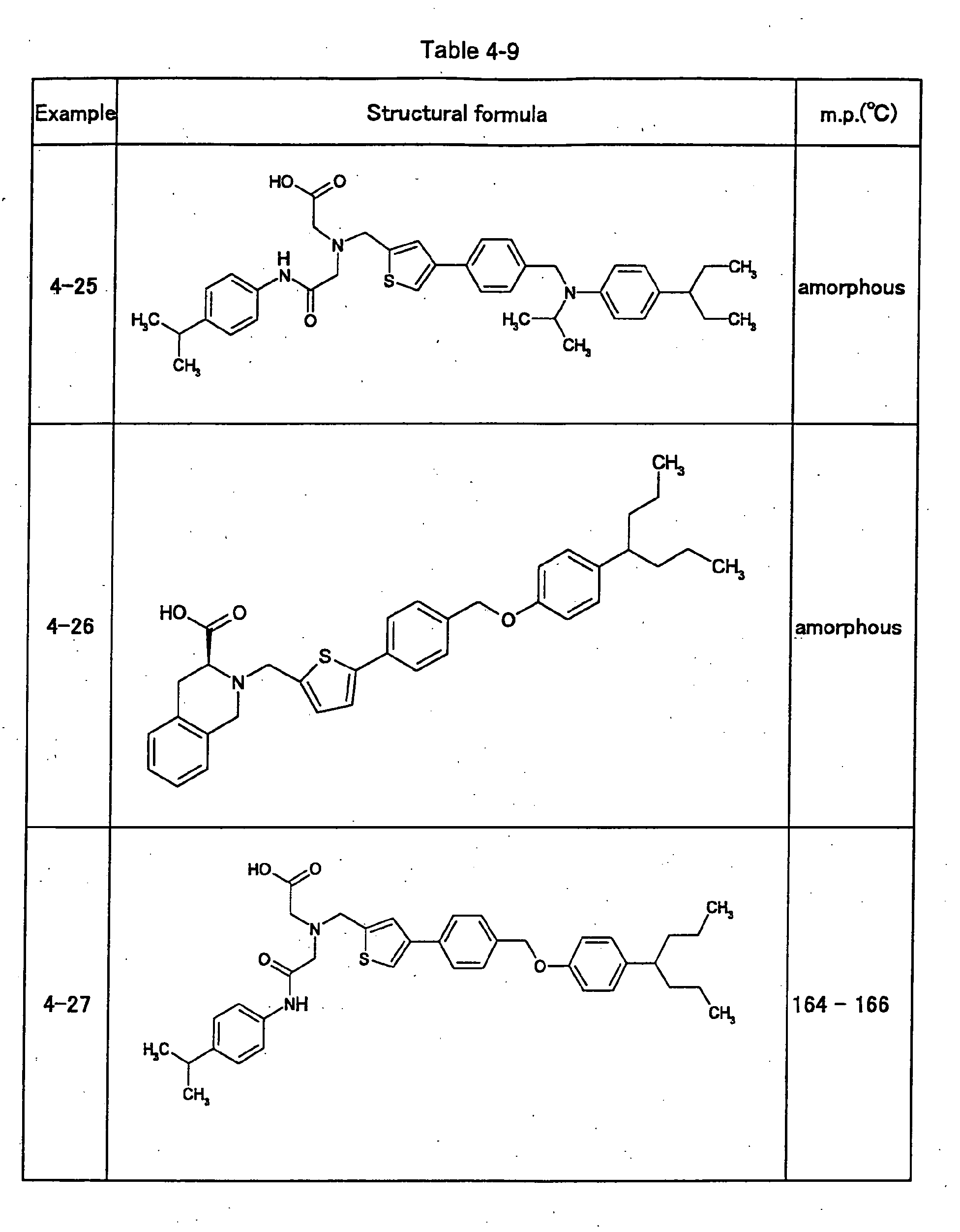

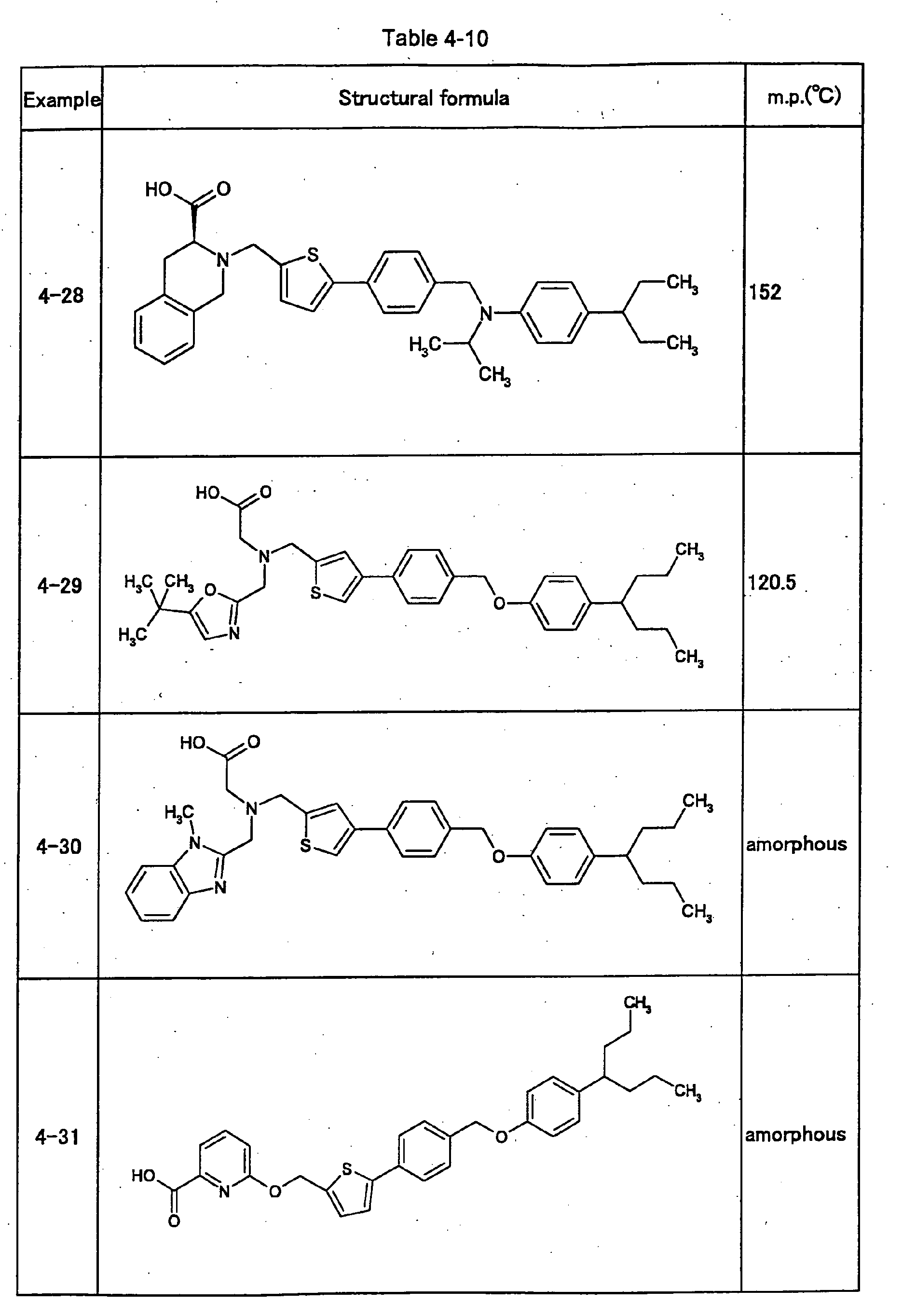

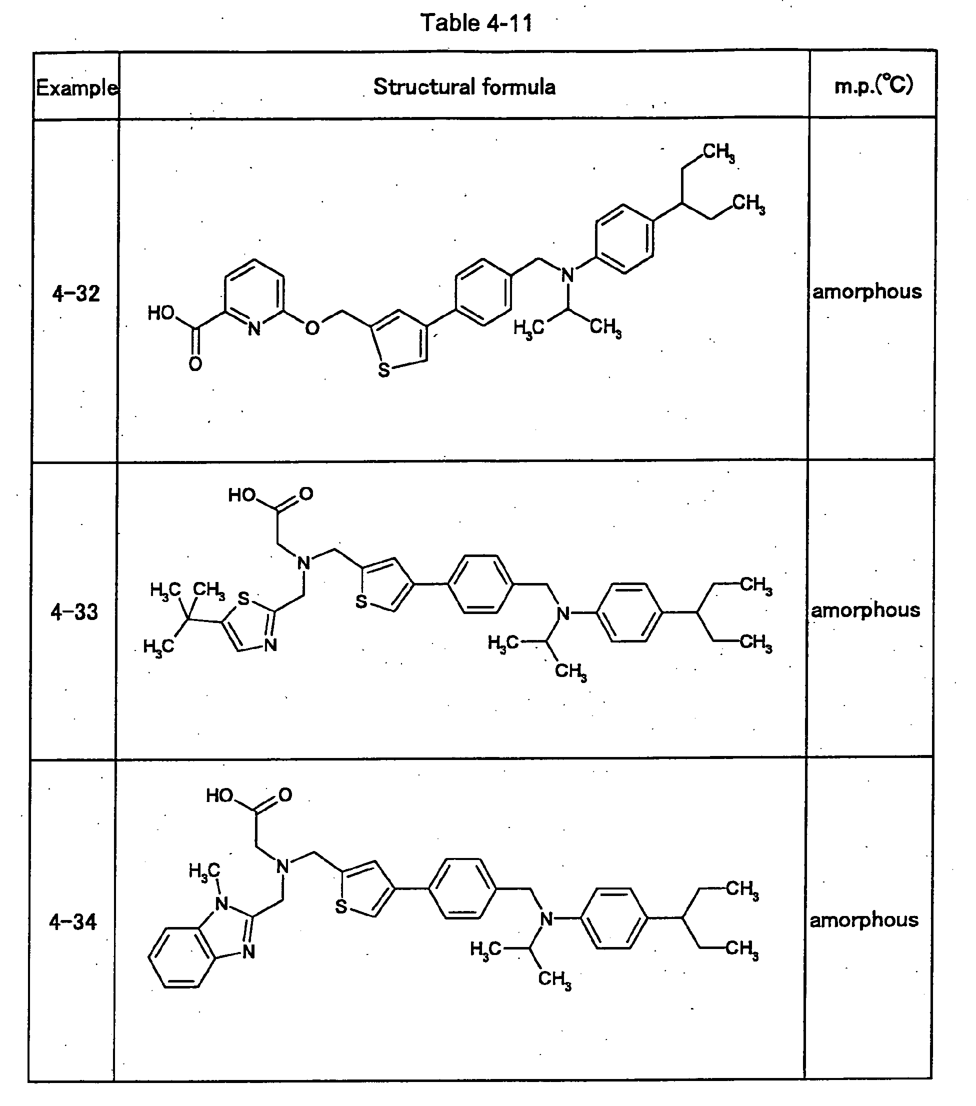

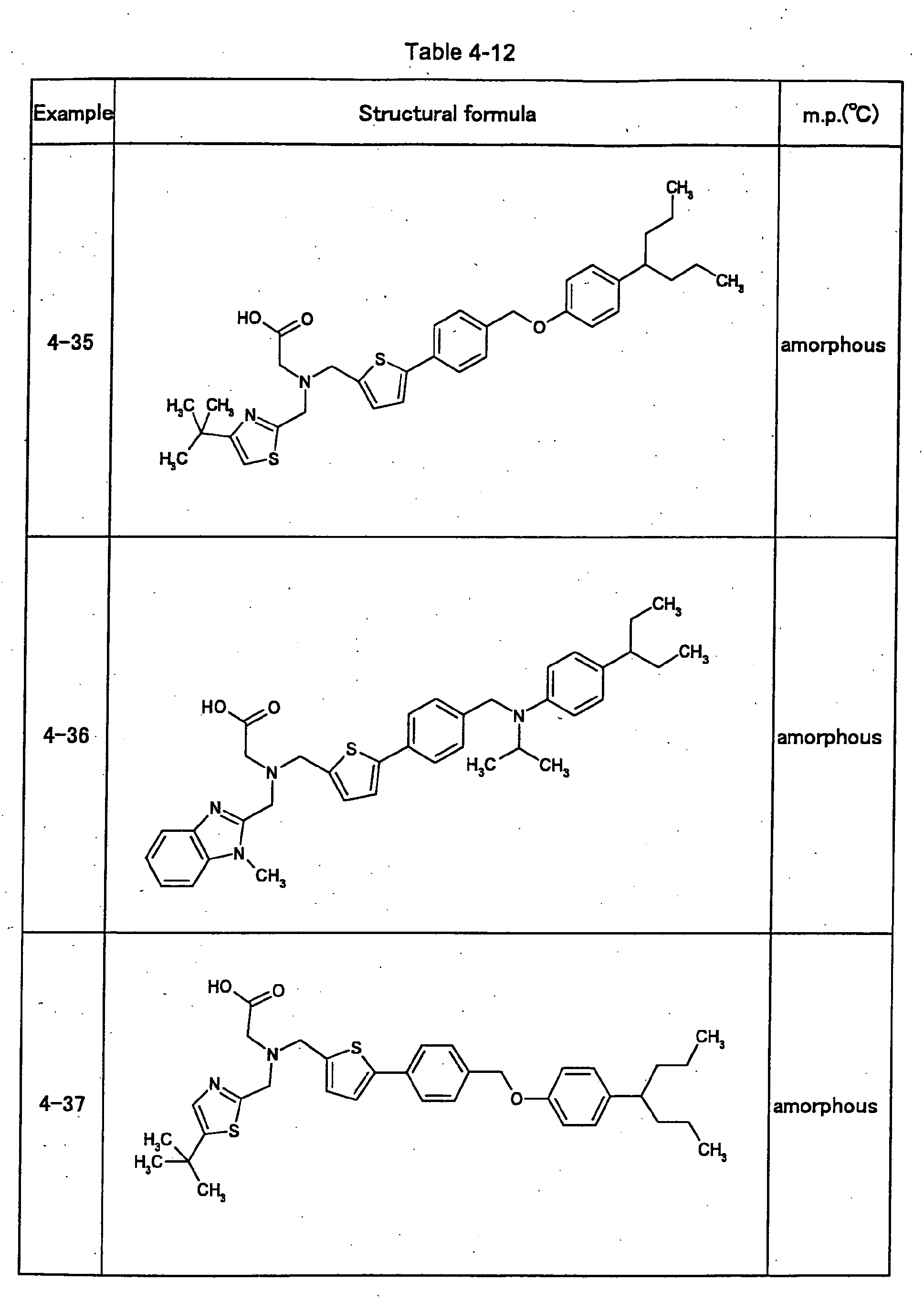

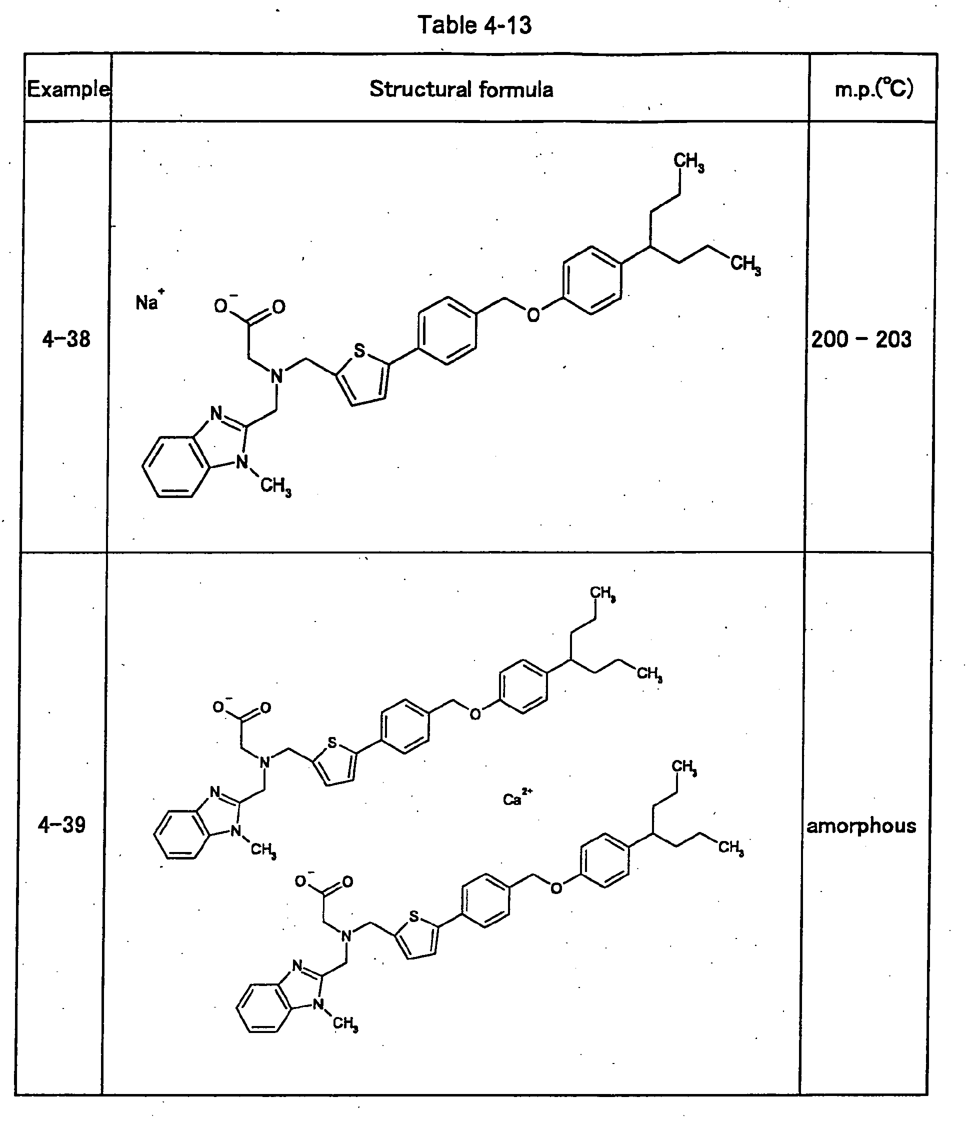

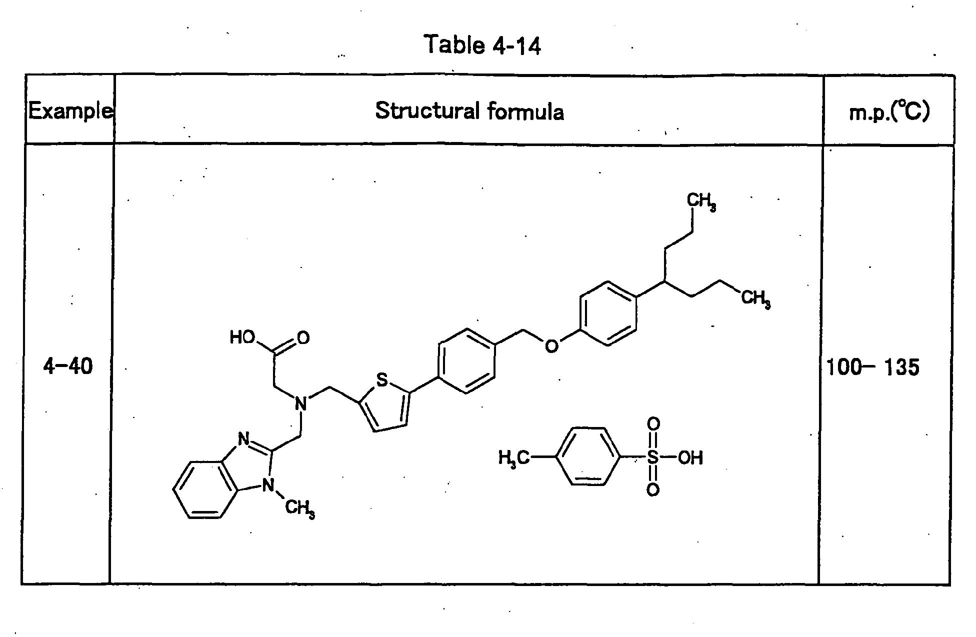

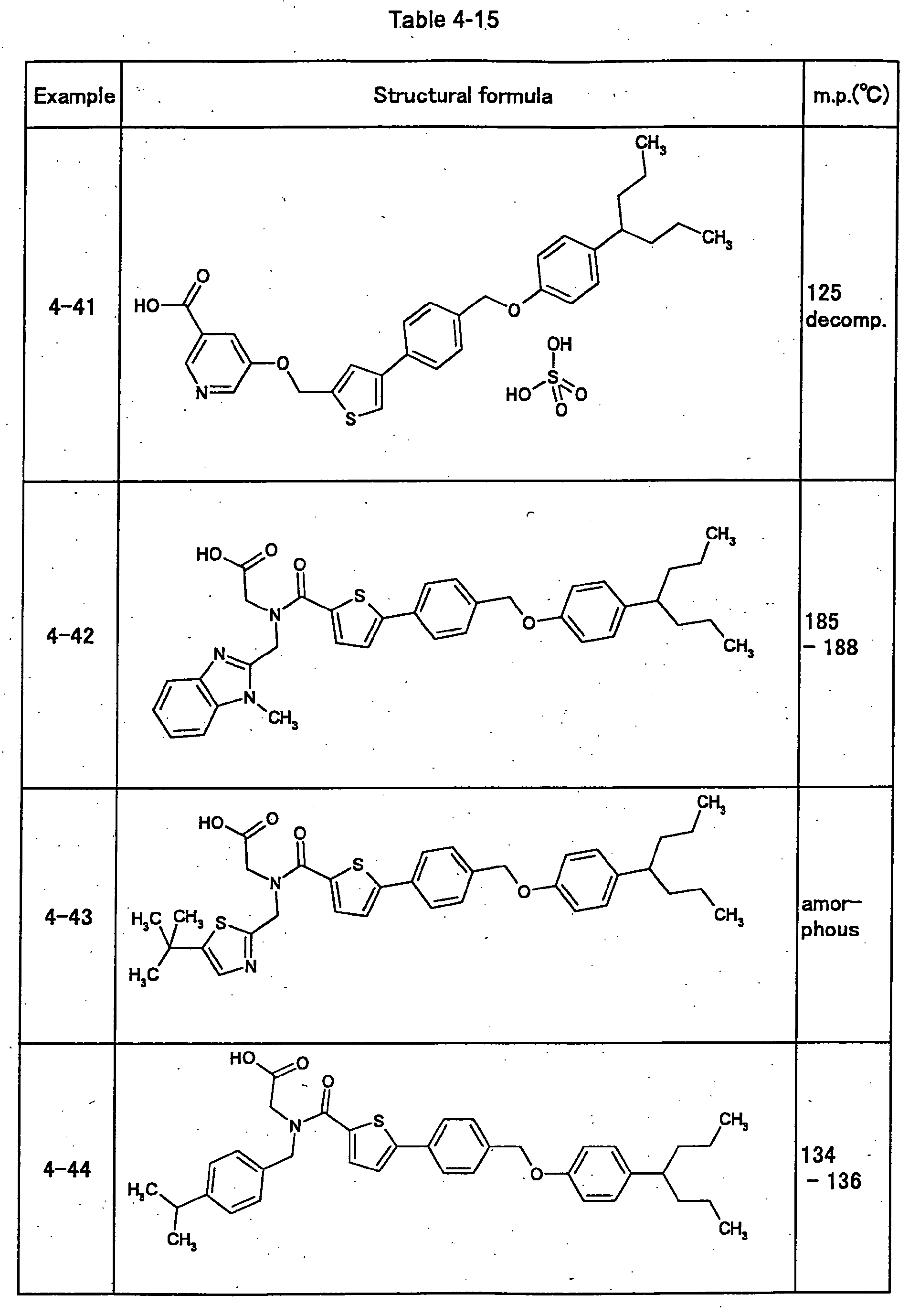

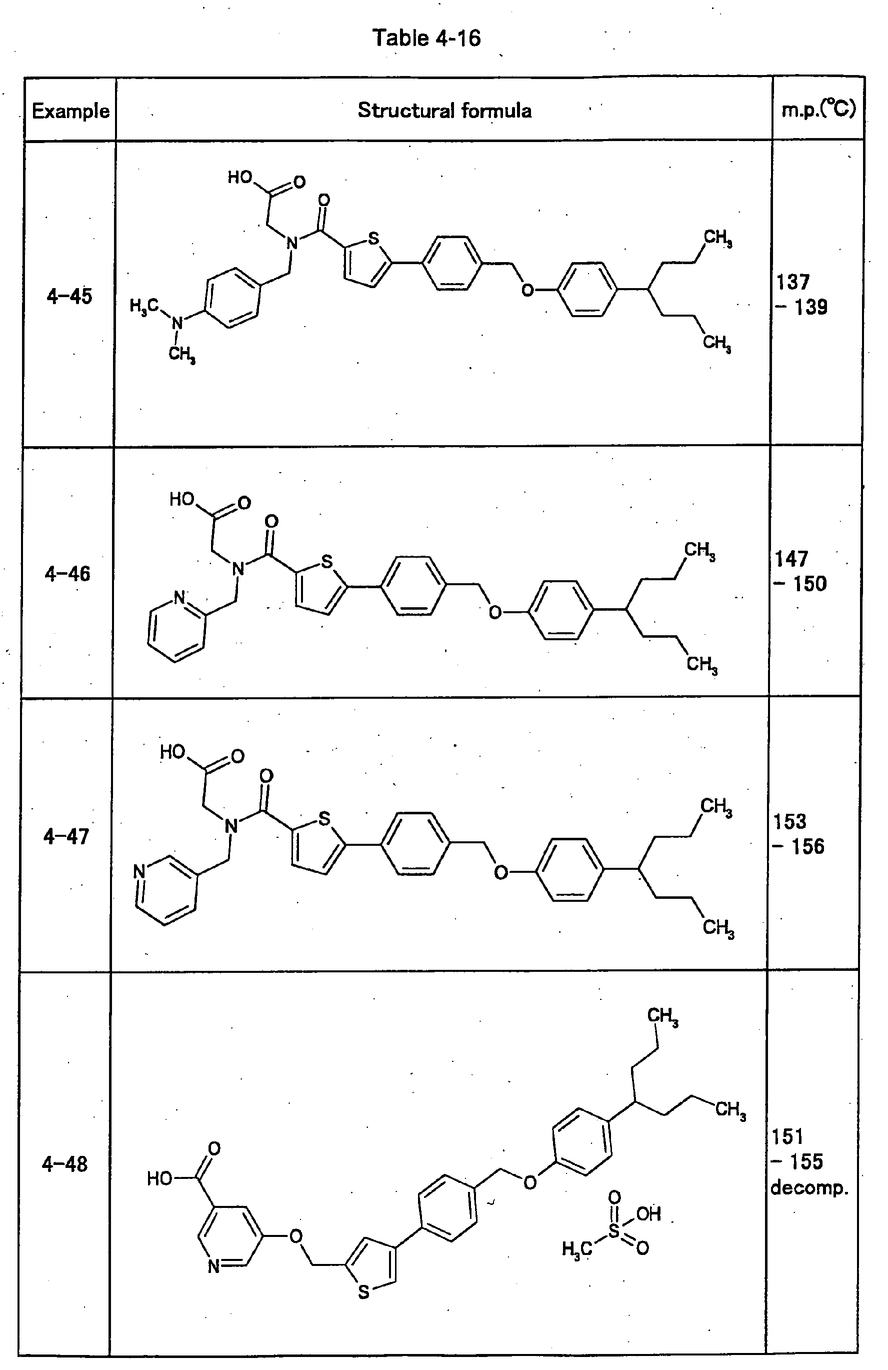

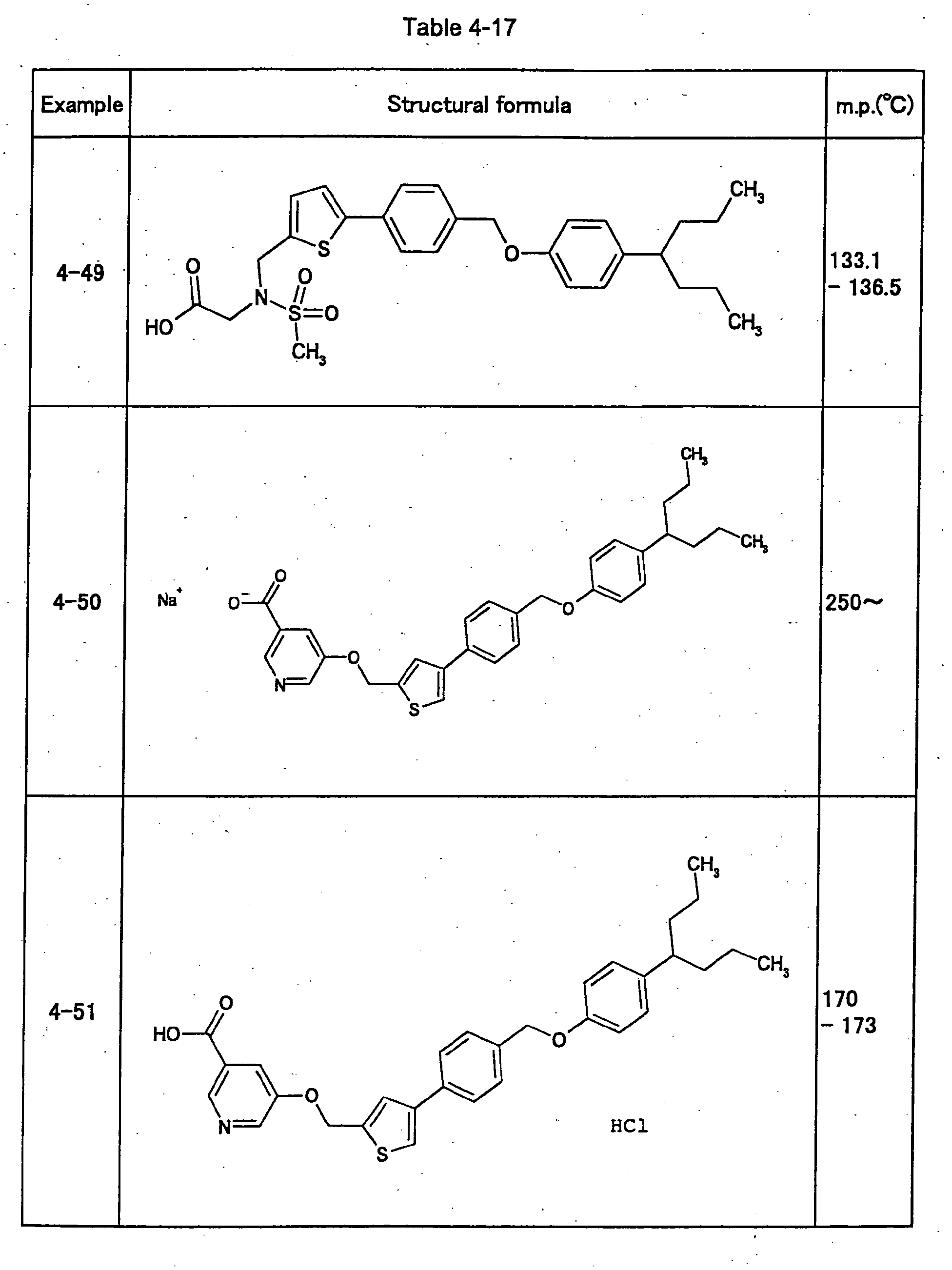

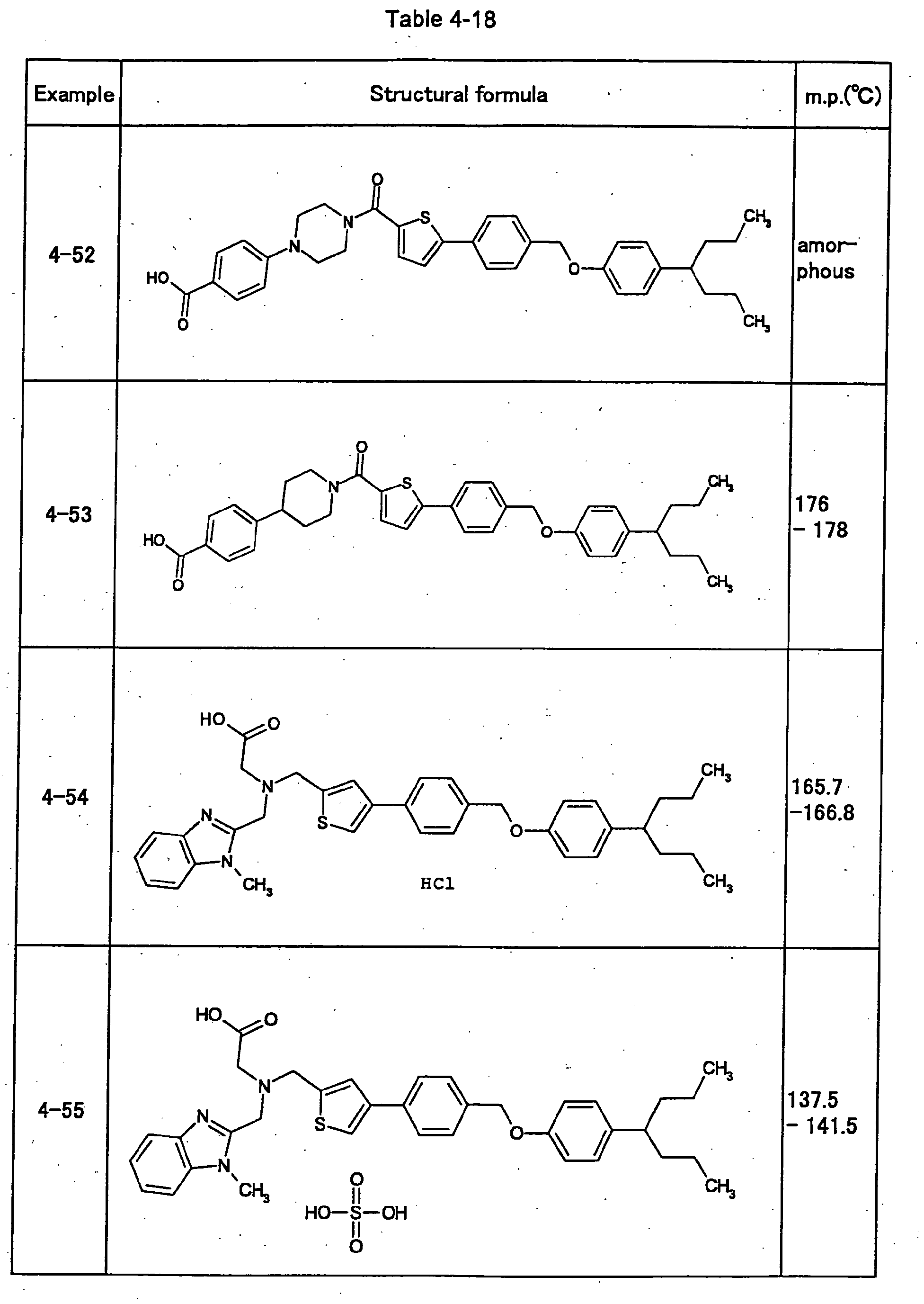

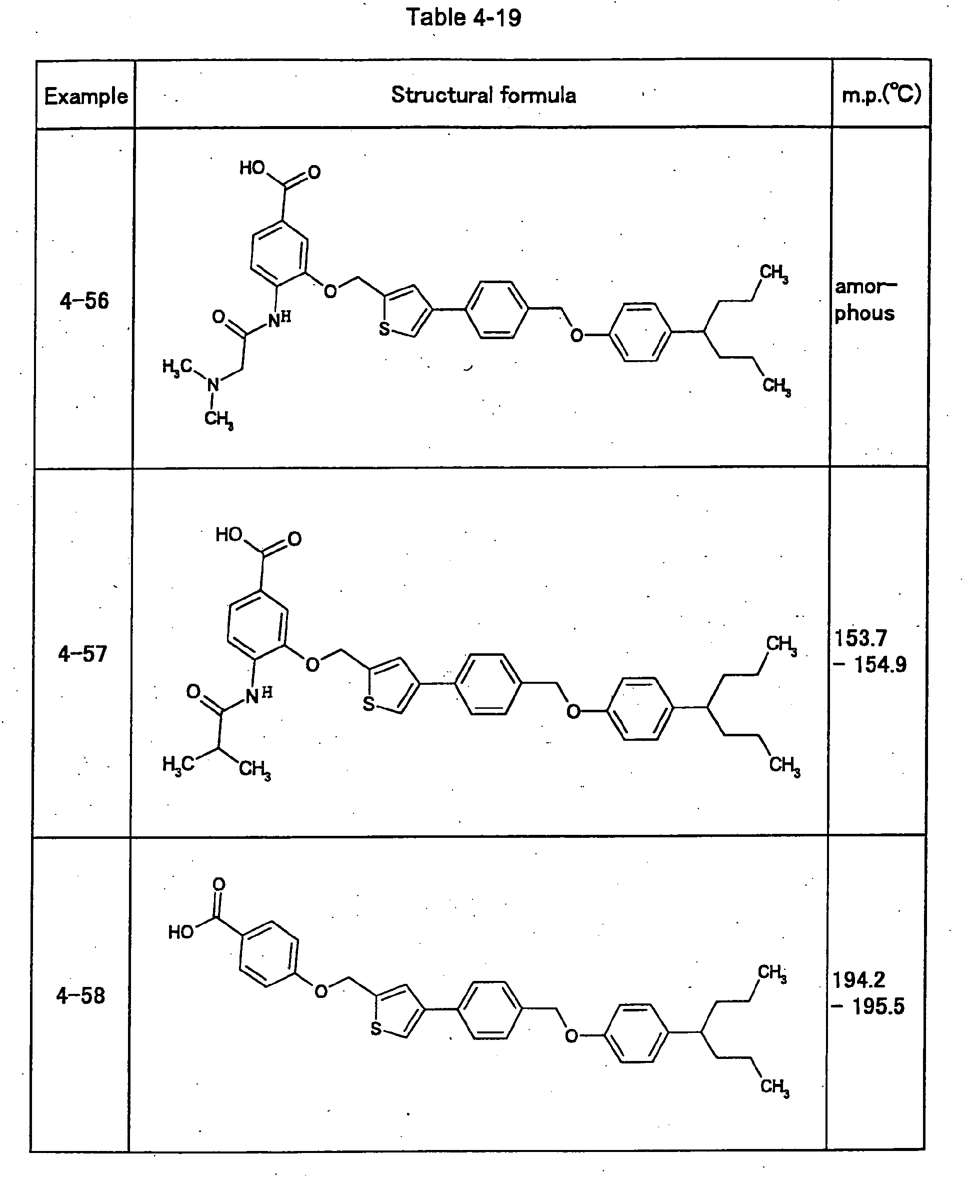

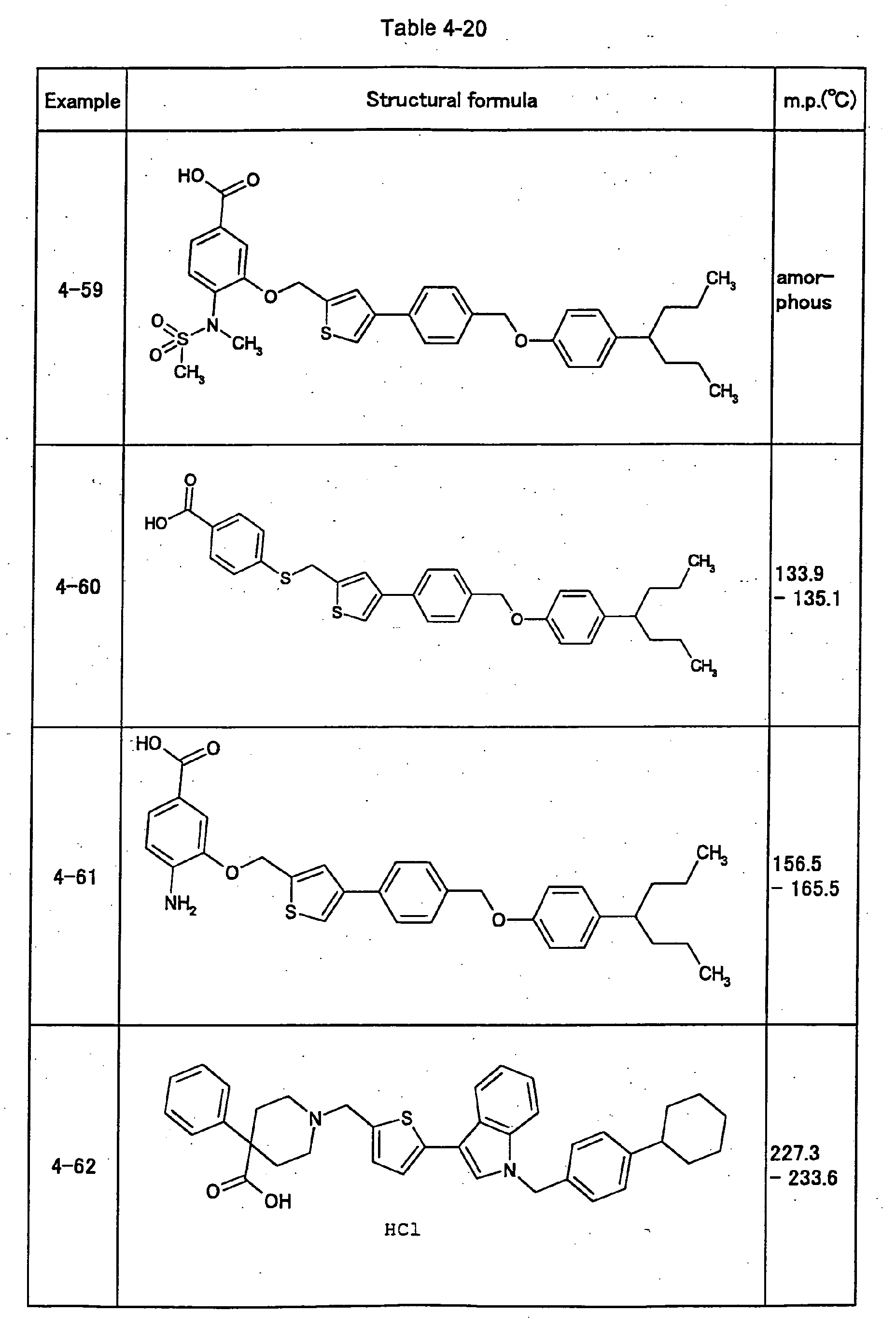

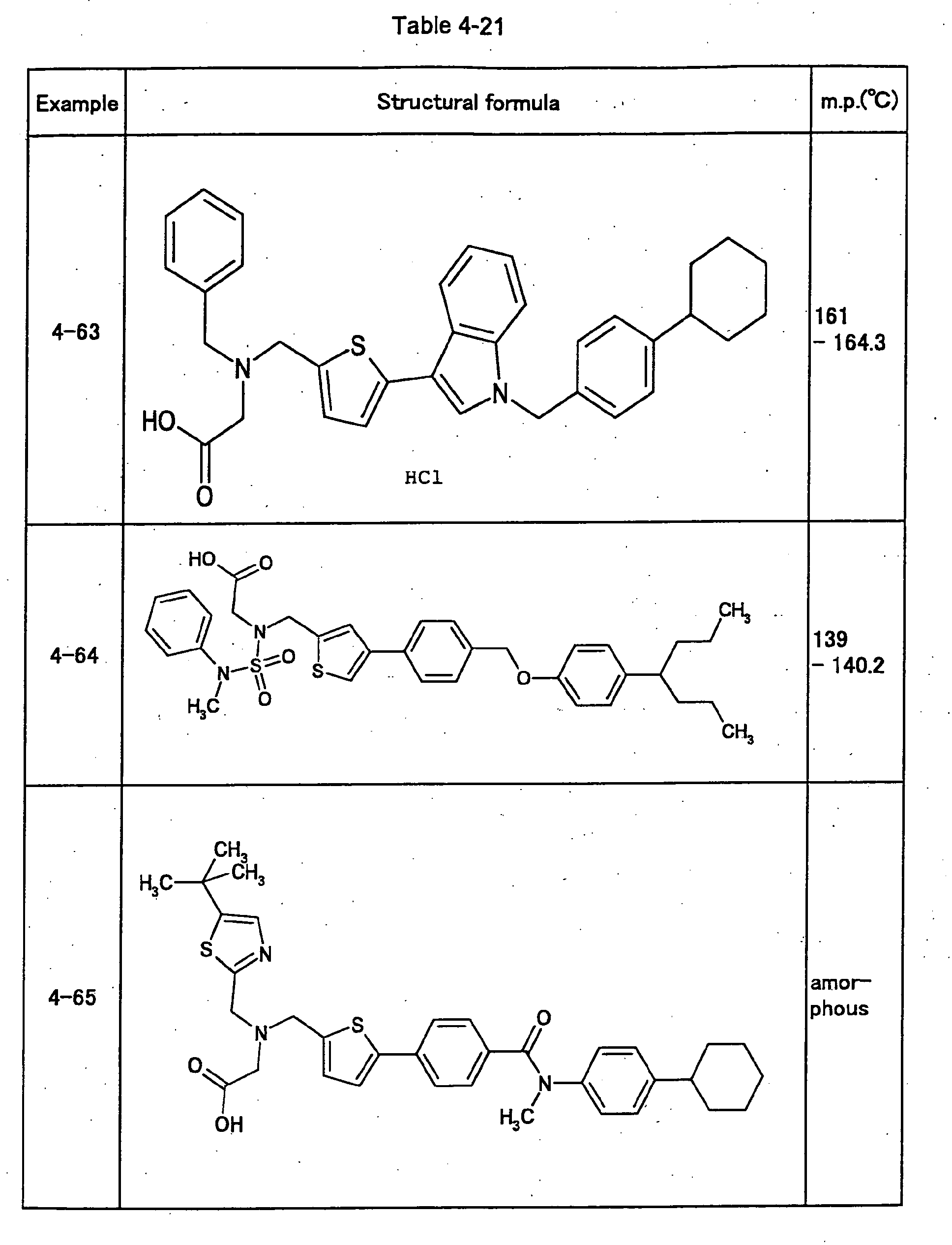

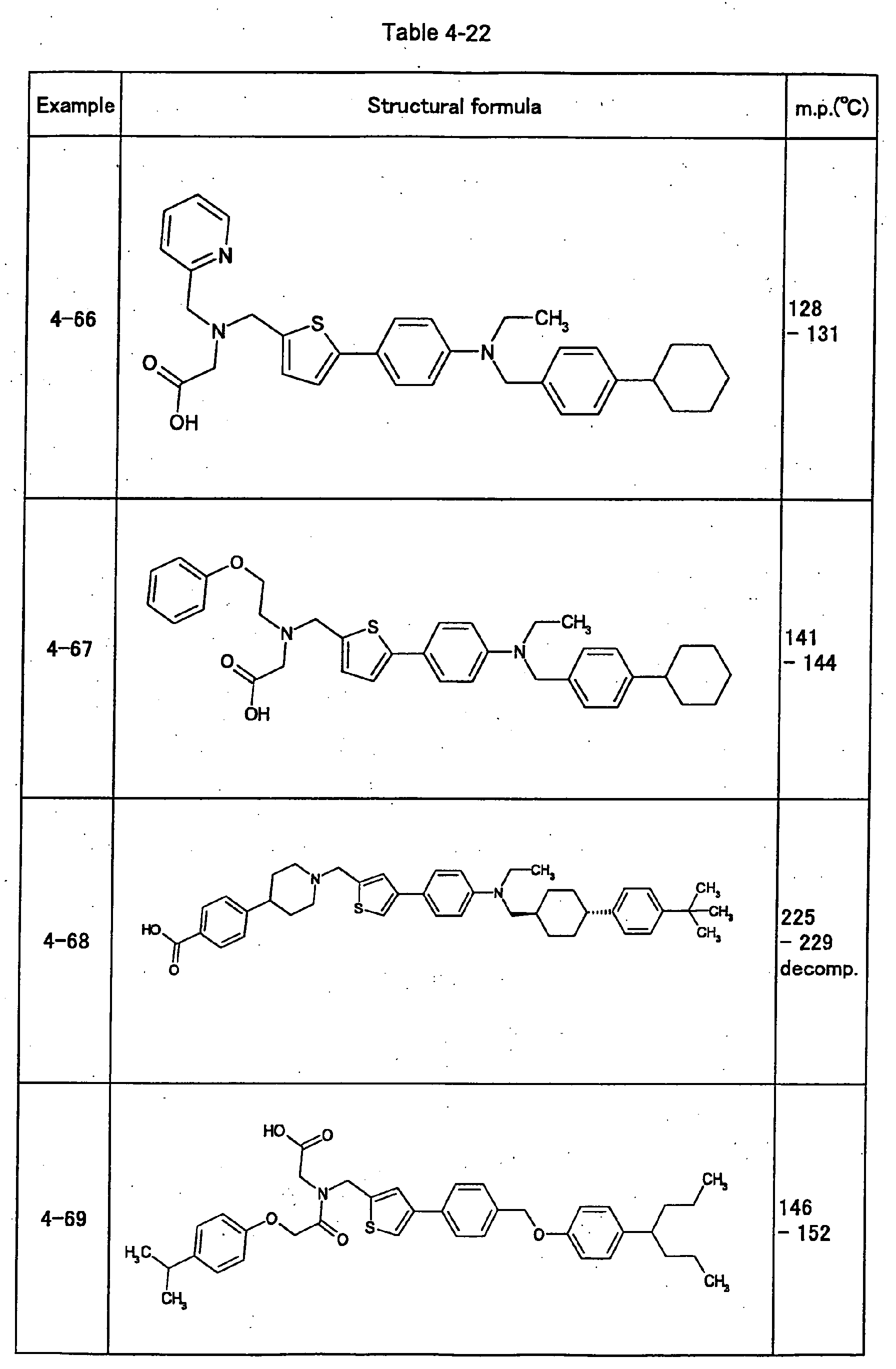

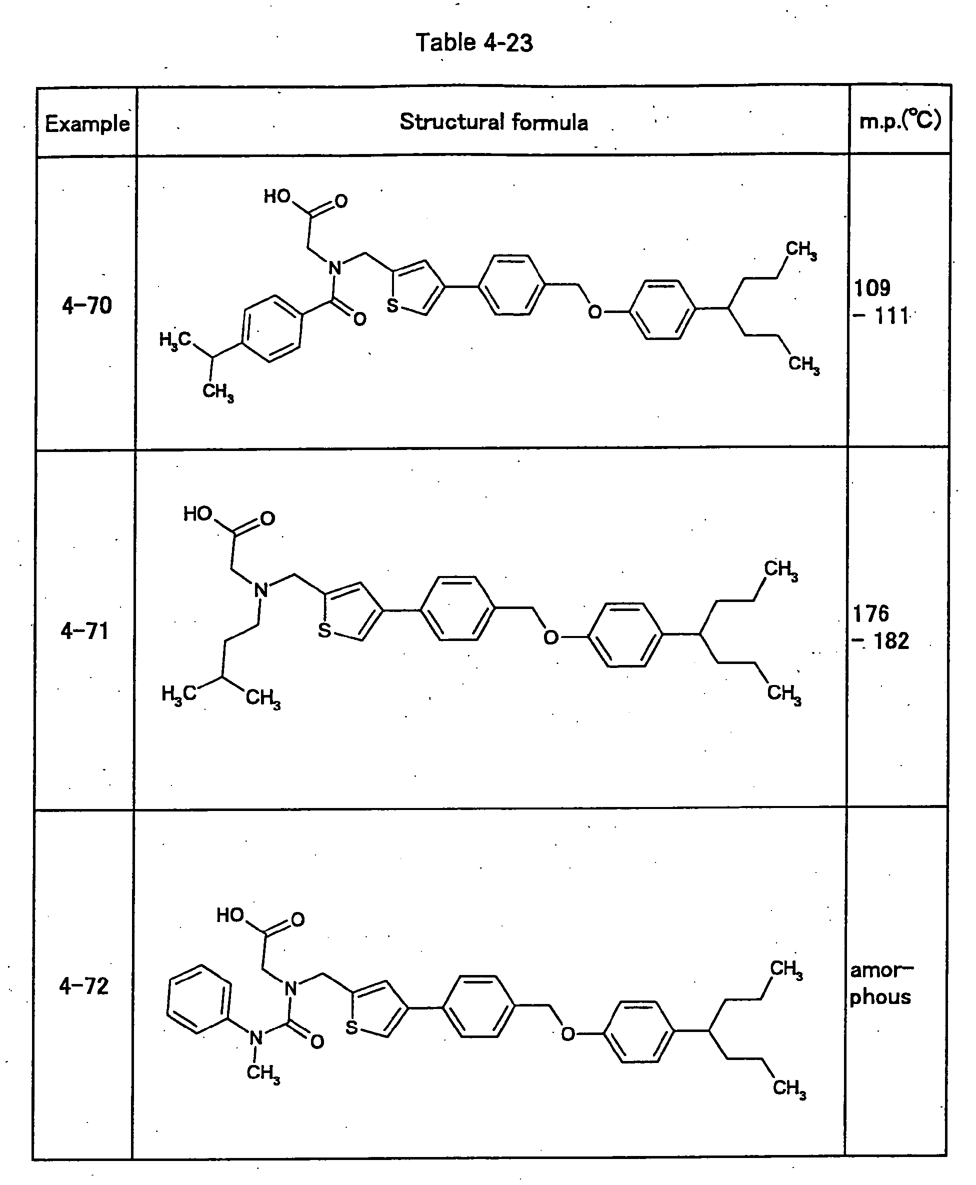

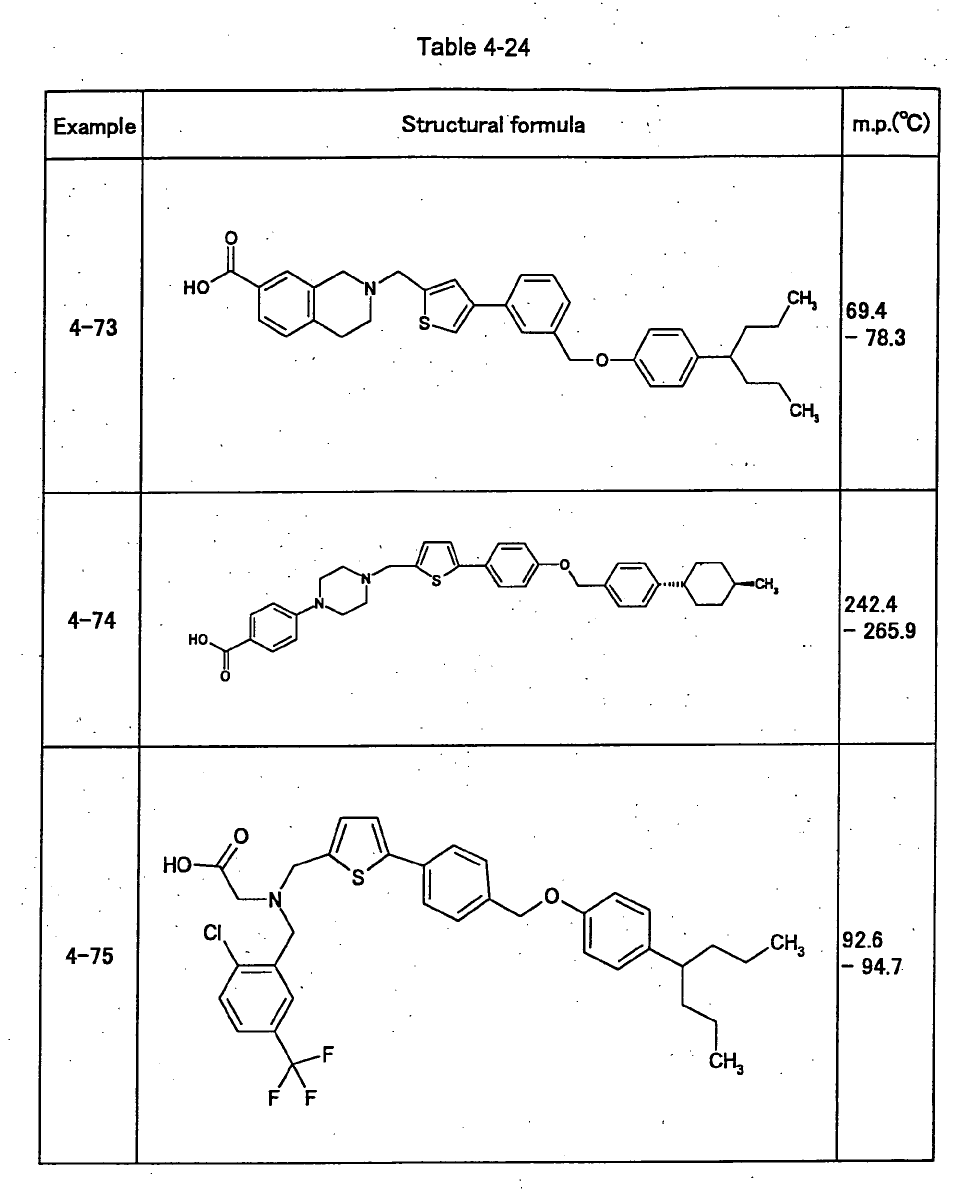

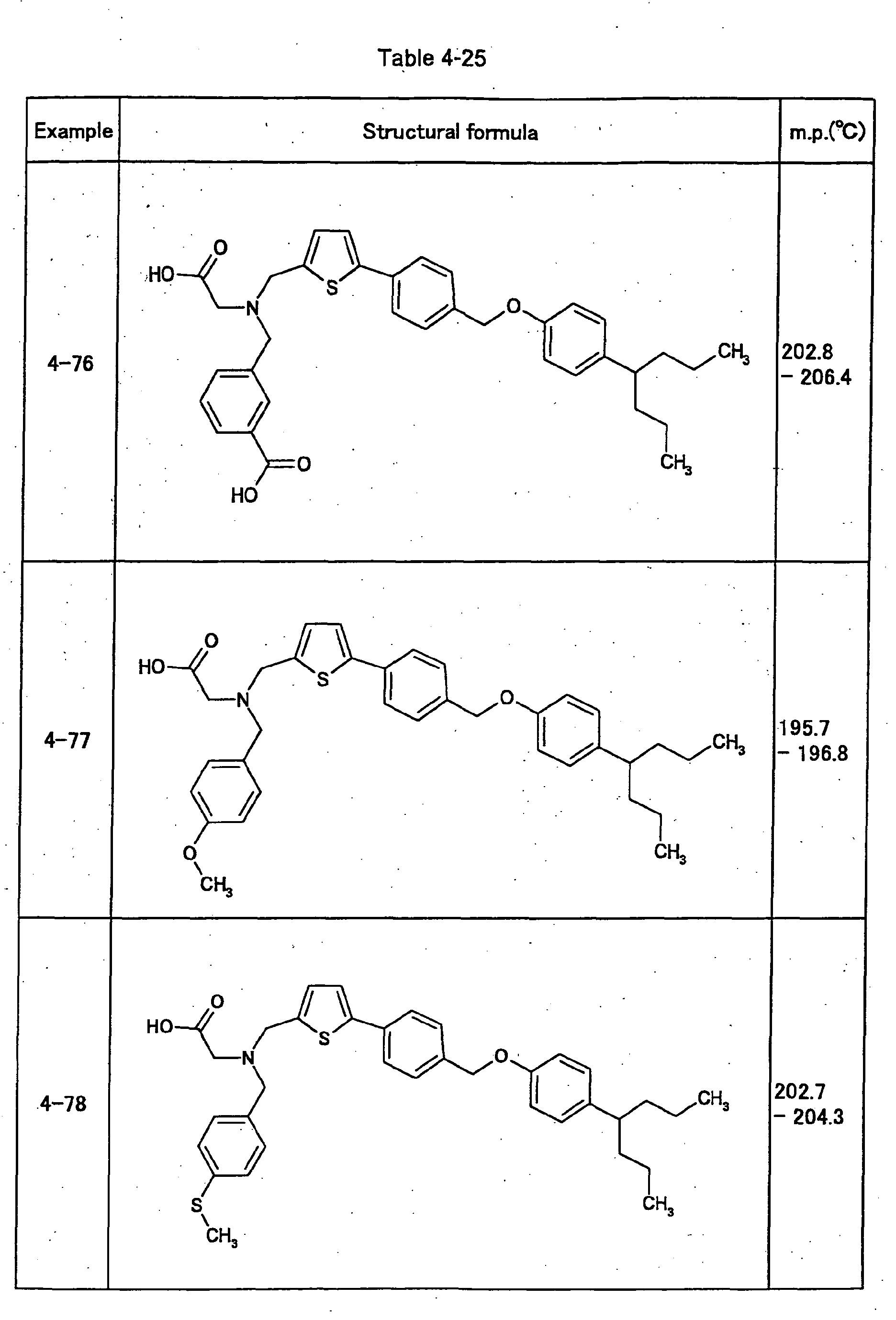

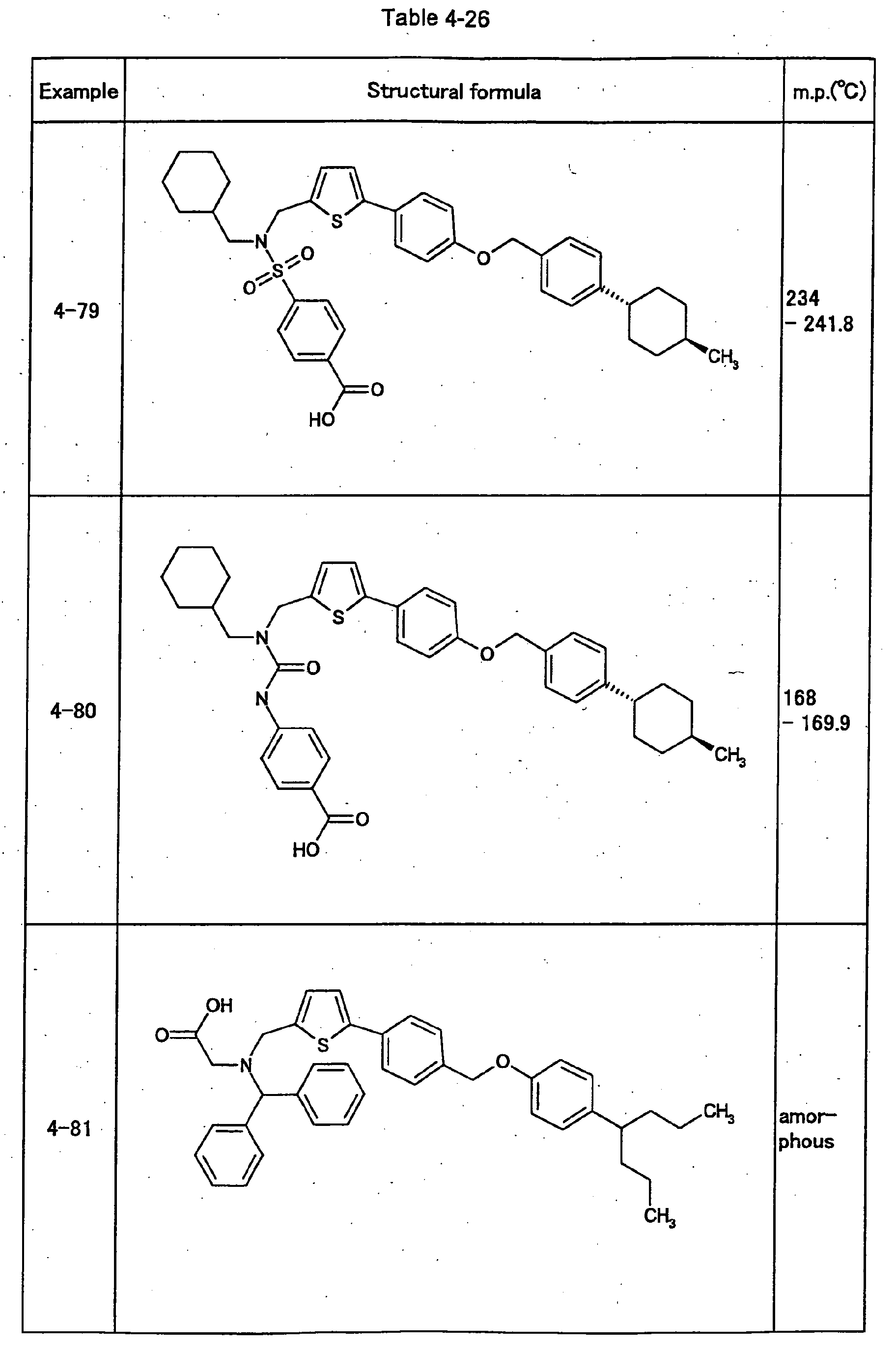

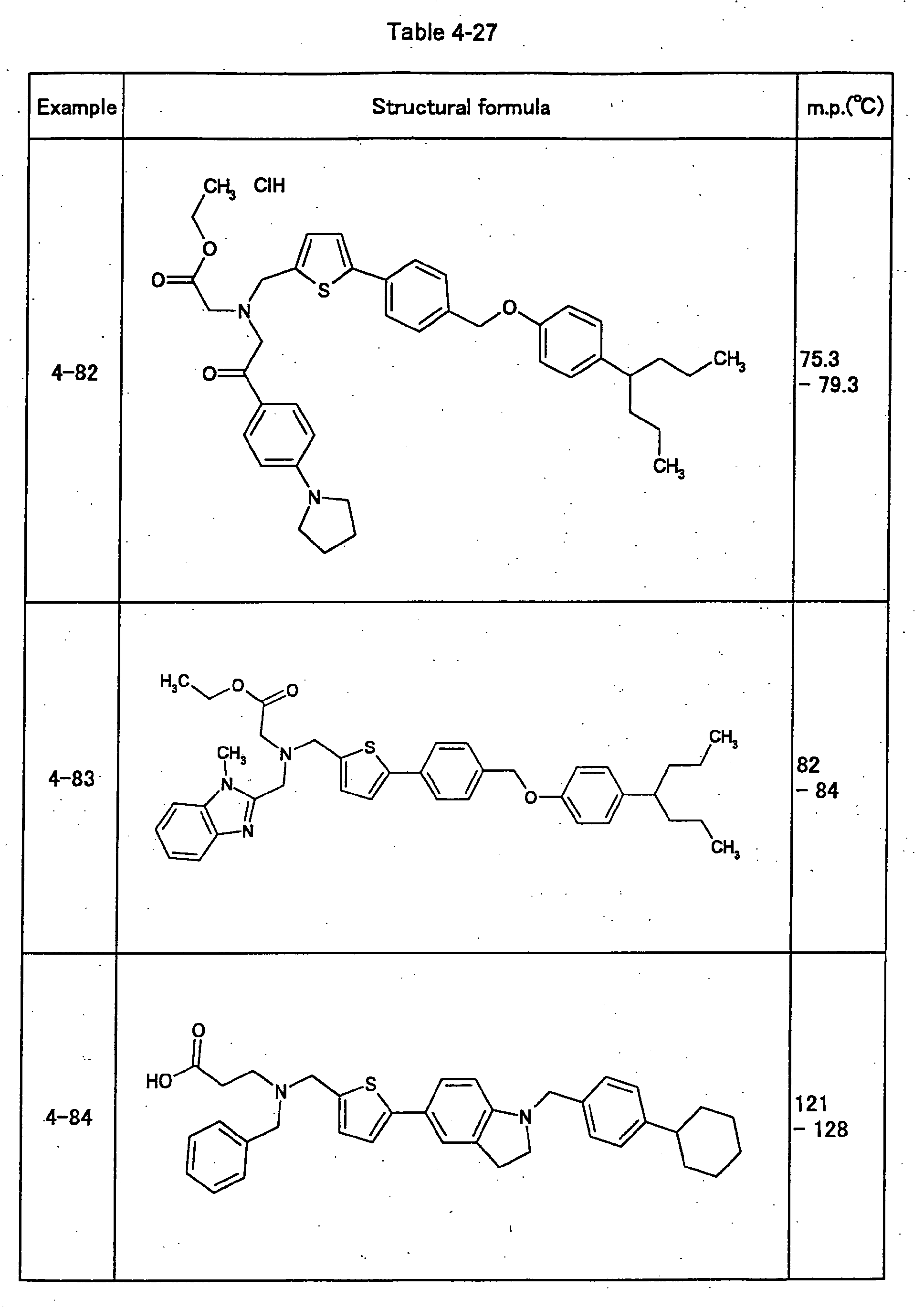

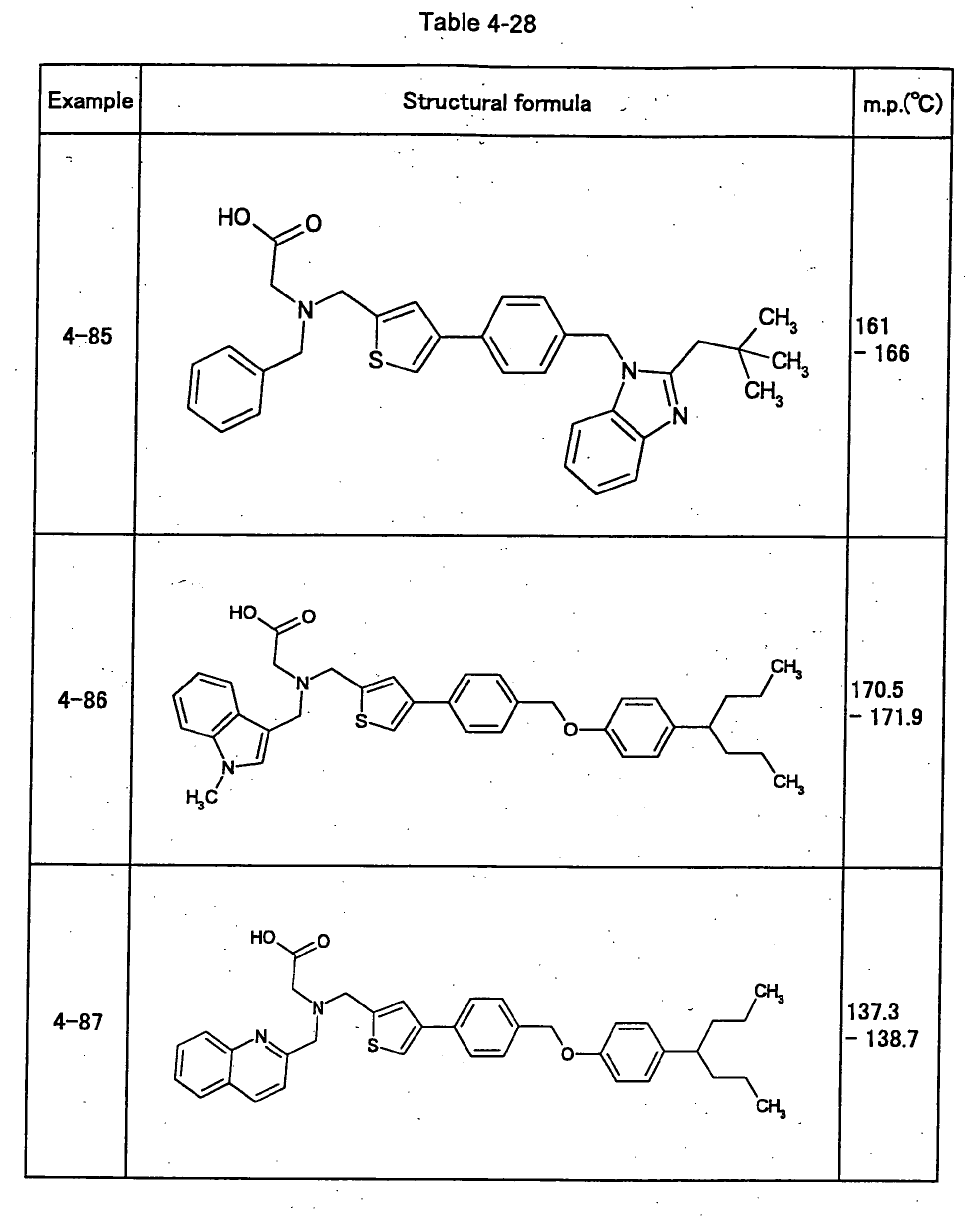

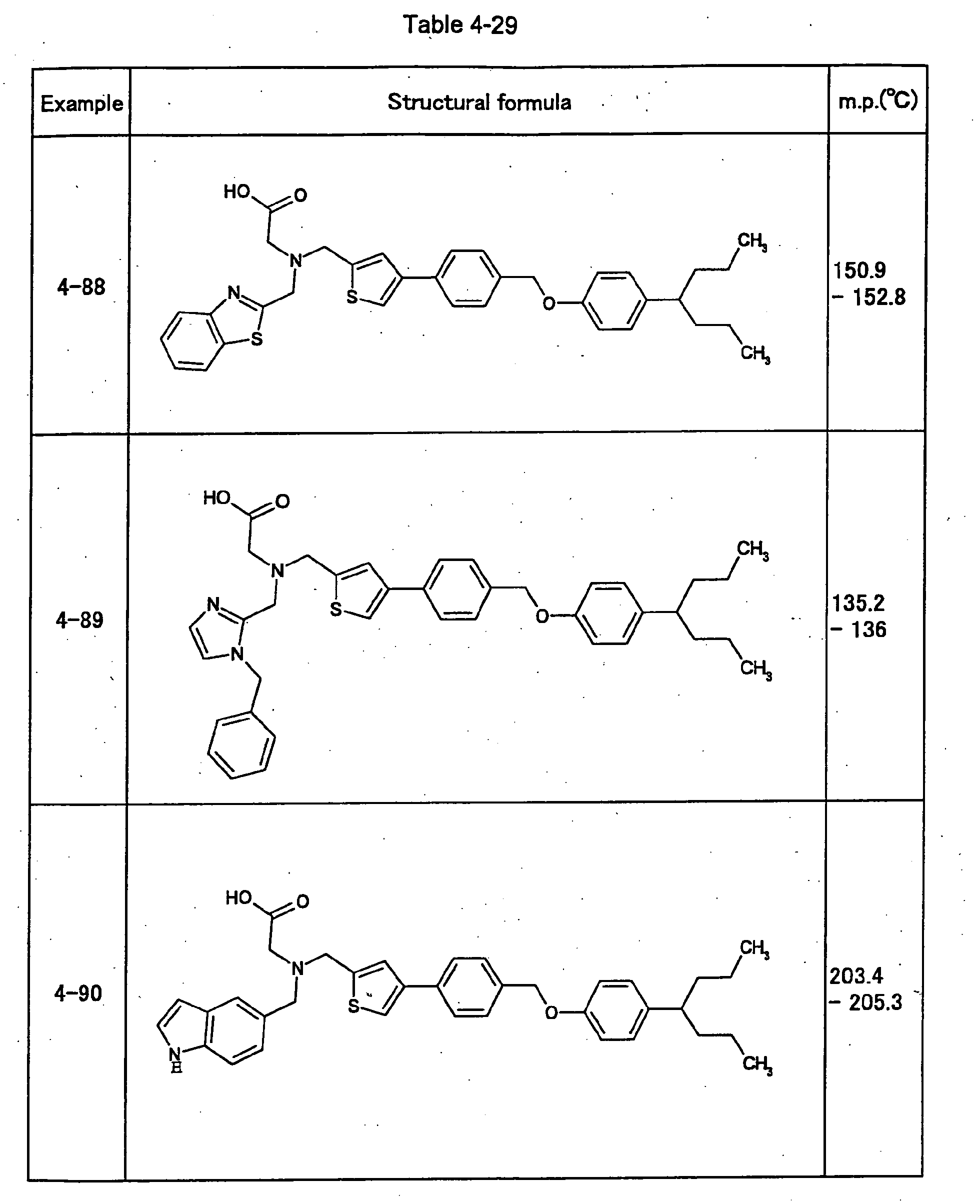

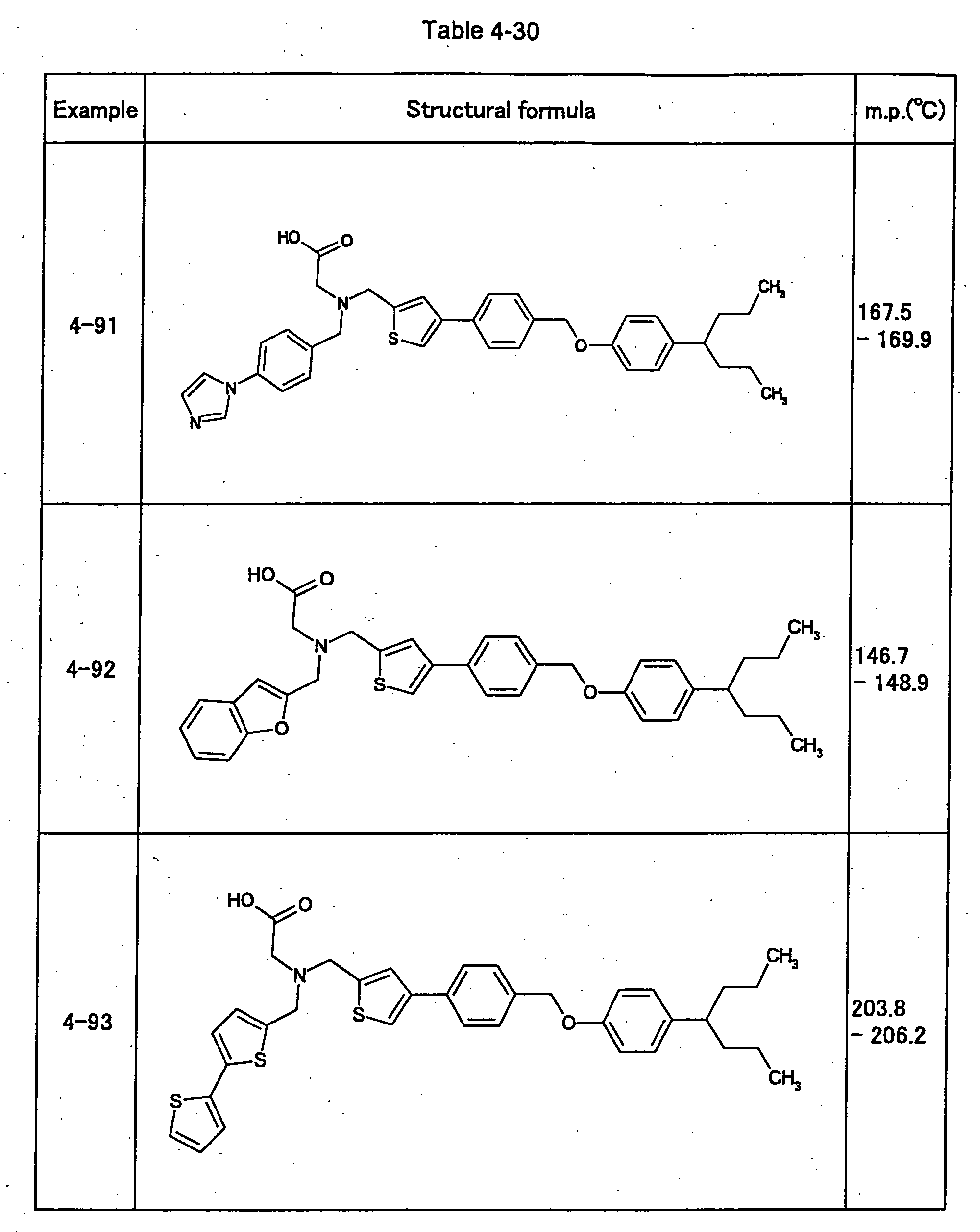

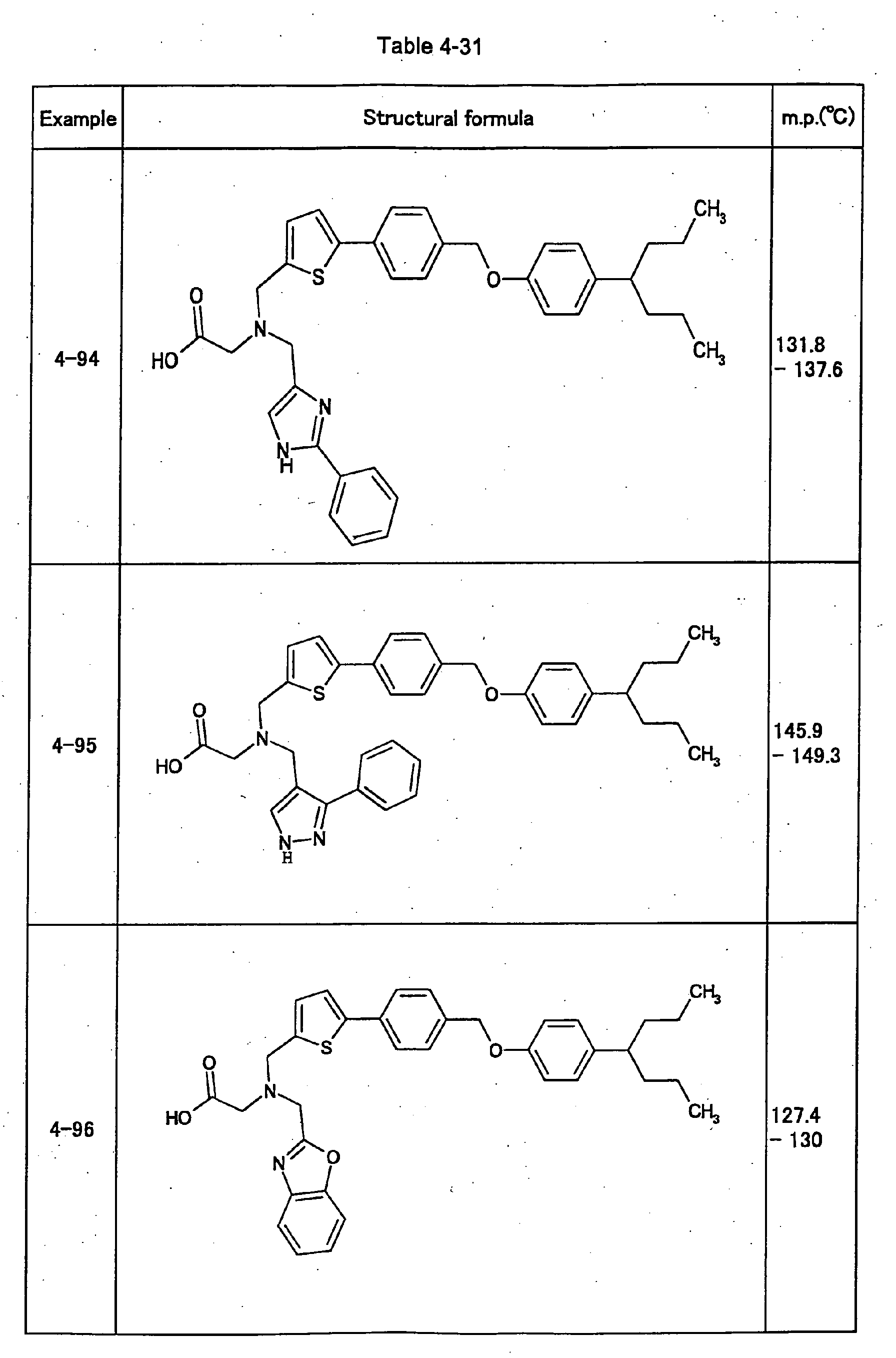

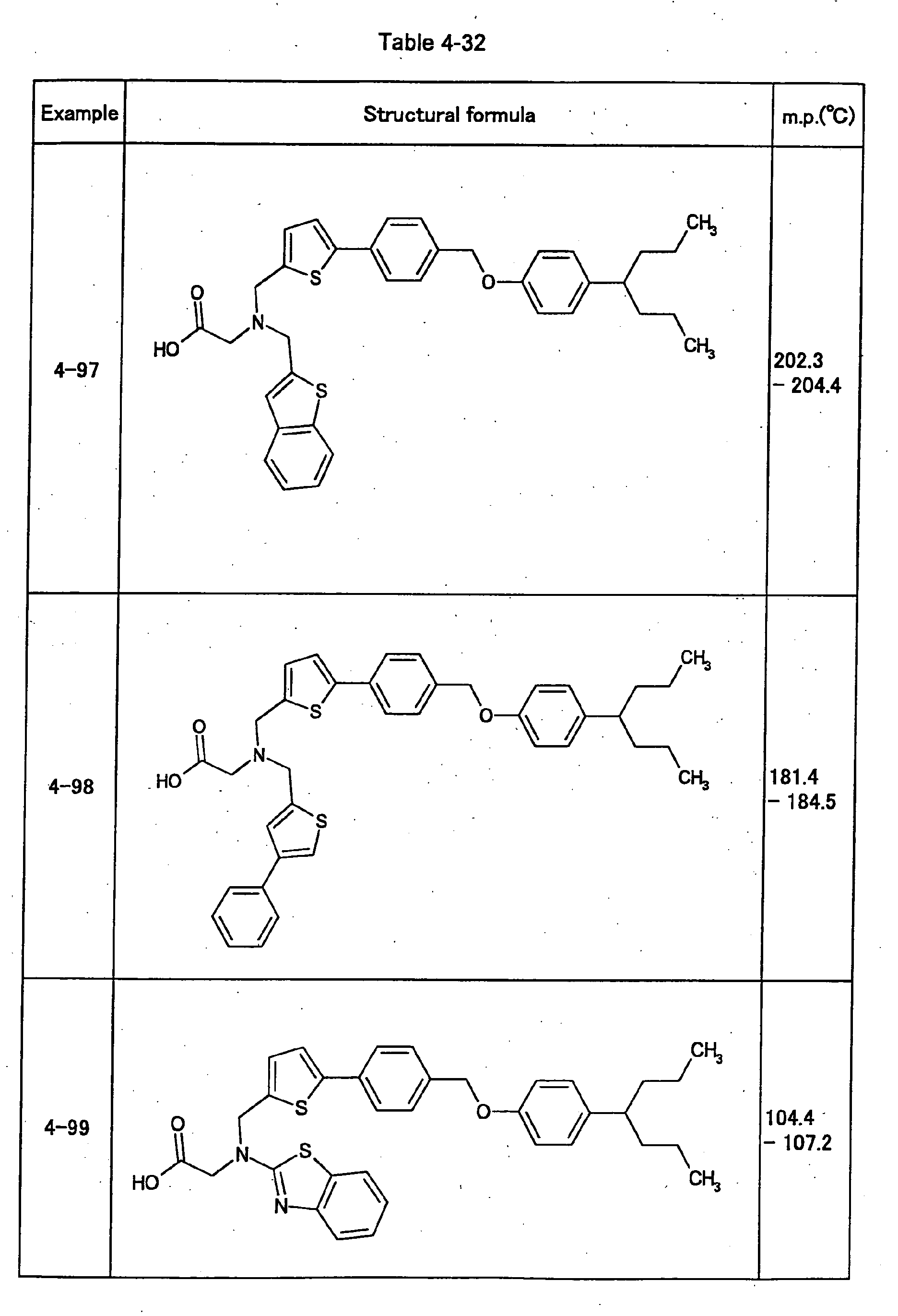

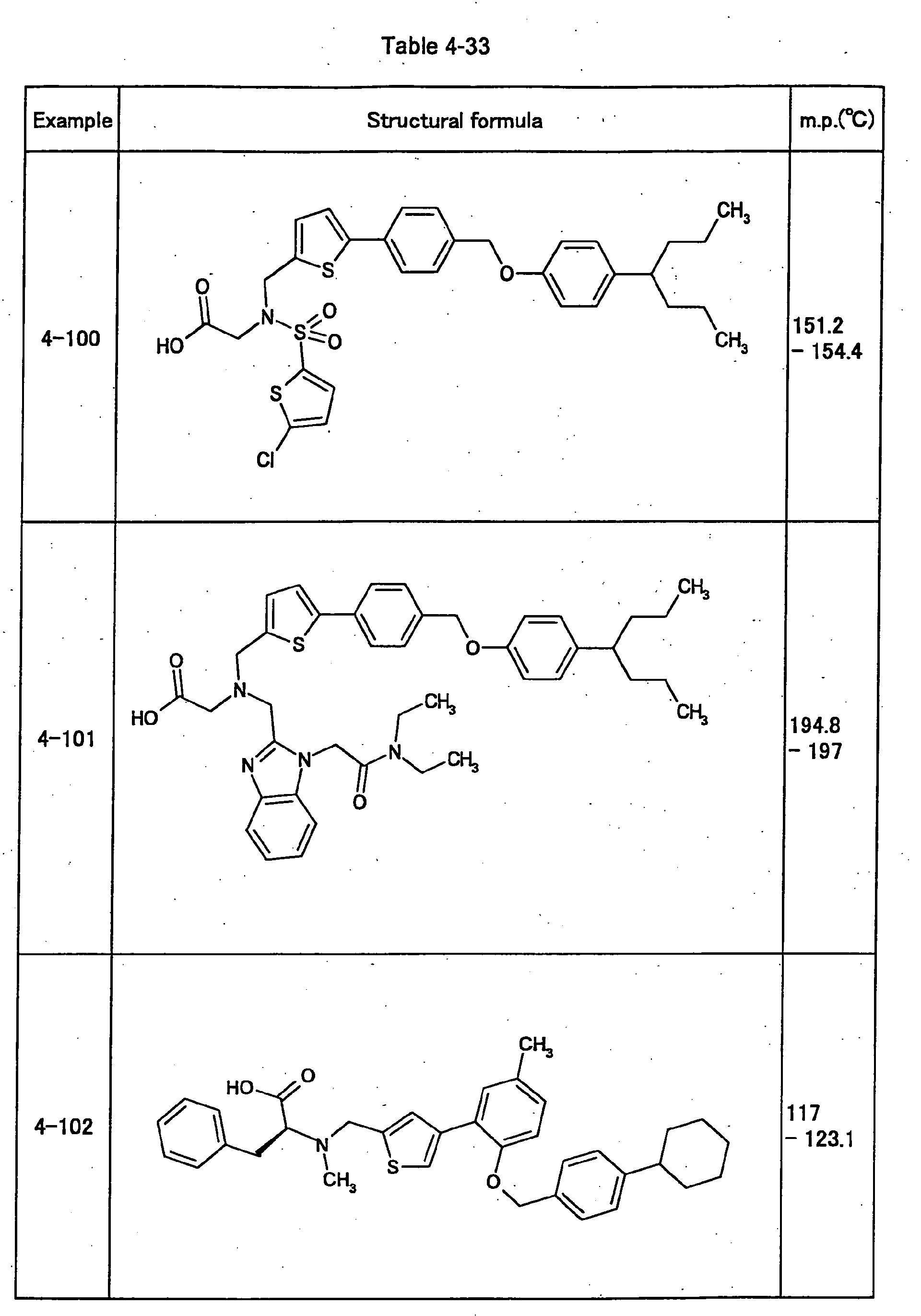

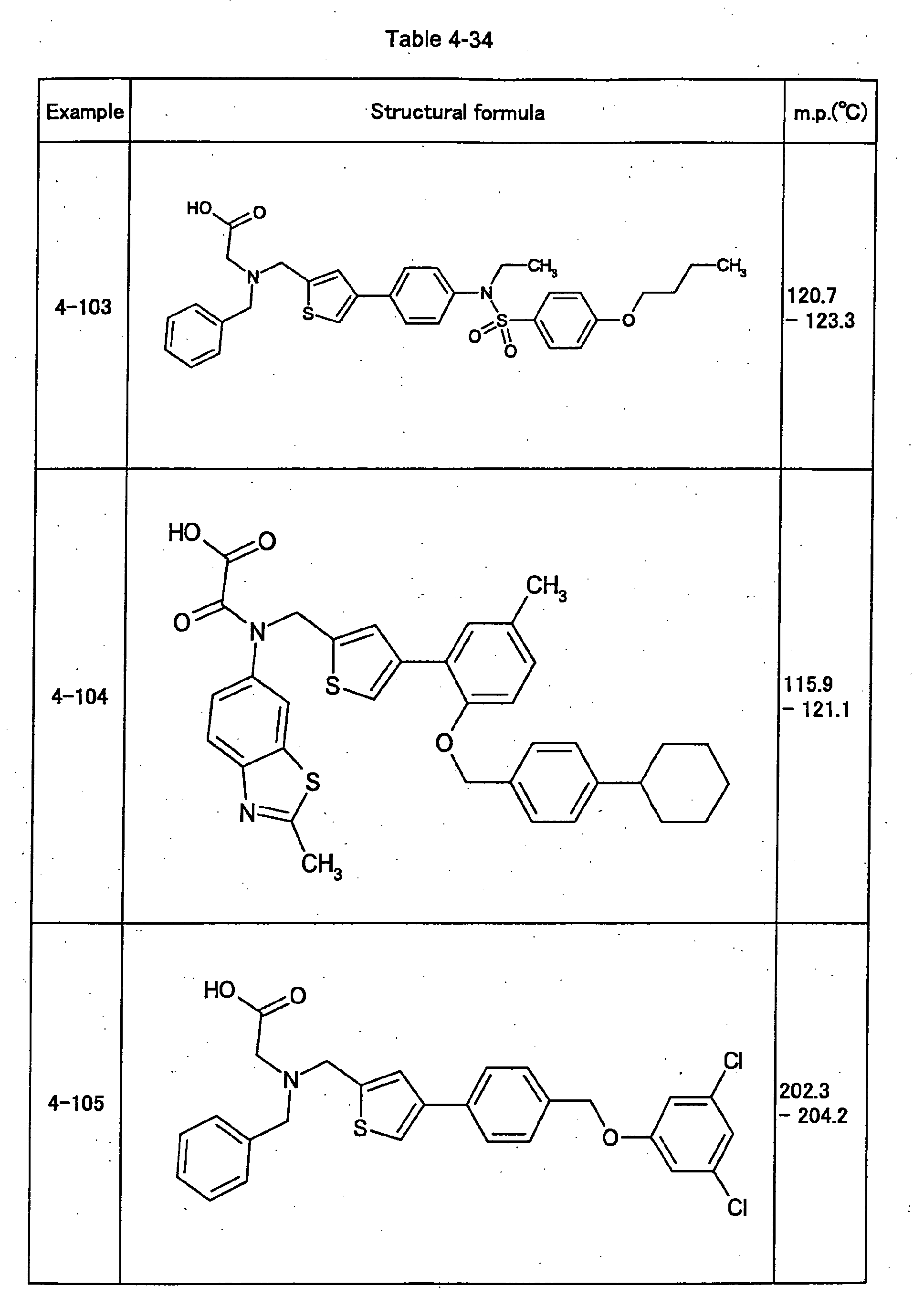

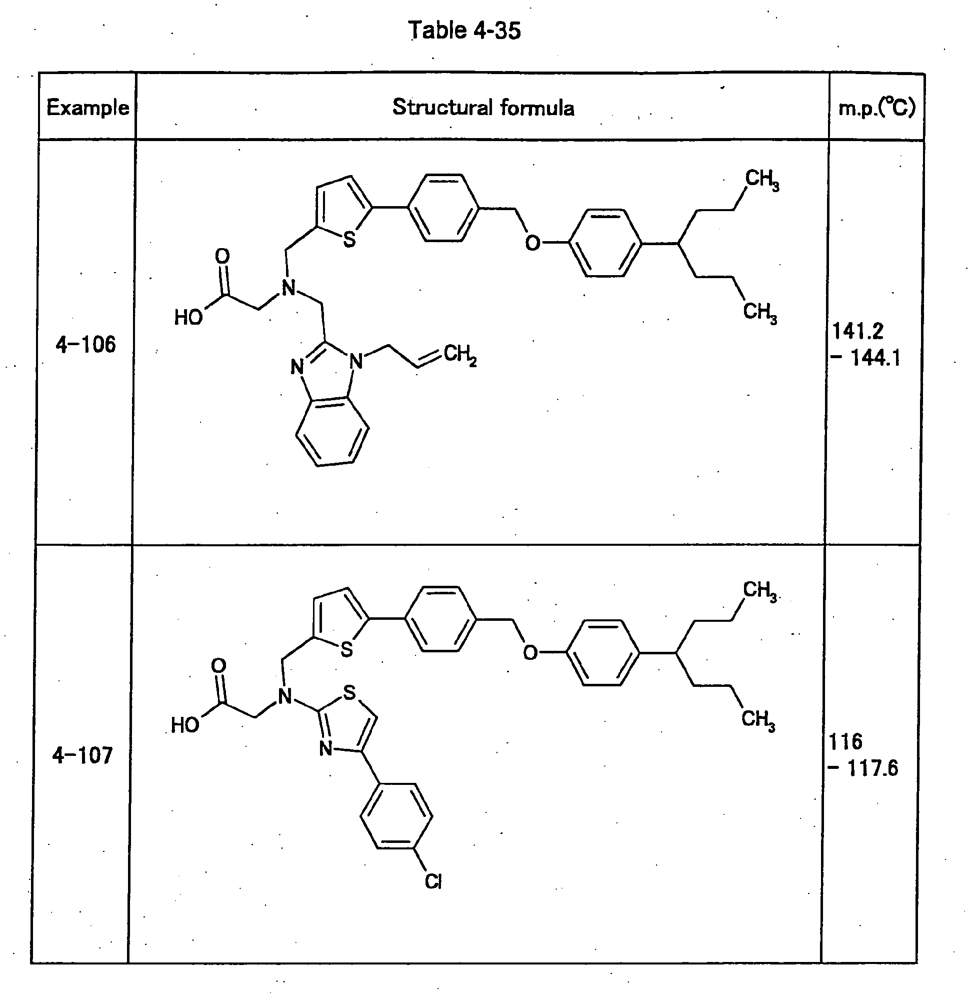

The Formulation Examples are shown in the following, which are not to be construed as limiting the present invention.

### Formulation Examples

| | |
|---|---|
| (a) compound of Ex. 1 | 10 g |
| (b) lactose | 50 g |
| (c) cornstarch | 15 g |
| (d) sodium carboxymethylcellulose | 44 g |
| (e) magnesium stearate | 1 g |

The total amount of (a), (b) and (c) and 30 g of (d) were kneaded with water, dried in vacuo and granulated. The obtained granules were mixed with 14 g of (d) and 1 g of (e) and tableted with a tableting machine to give 1000 tablets containing 10 mg of (a) per tablet.

The test results of protein tyrosine phosphatase 1B inhibitory activity of the present invention are shown in the following.

### Experimental Example 1 (protein tyrosine phosphatase 1B inhibitory activity)

### Preparation of assay buffer:

A 50 mM Tris-HCl buffer (pH 7.5), 50 mM NaCl and 3 mM dithiothreitol (DTT) were prepared.

### Preparation of sample:

10 mM DMSO solutions of 0.1, 0.3, 1, 3 and 10 µM of the test compound were prepared and diluted with the above-mentioned assay buffer to the final dimethyl sulfoxide (DMSO) concentration of not more than 1%. As the control, the assay buffer was used.

### Preparation of substrate:

A synthetic peptide, wherein three tyrosines in 12 amino acids from 1142nd to 1153rd of the sequence of insulin receptor had been phosphorylated, was diluted with the above-mentioned assay buffer to 80 µM.

### Preparation of enzyme:

Recombinant human protein tyrosine phosphatase 1B (manufactured by UBI) was diluted with the above-mentioned assay buffer (1.2 ng/25 µl).

### (Evaluation method)

The sample (10 µl) prepared as mentioned above and a substrate (25 µl) were successively added to a 96 well plate, and the enzyme (25 µl) prepared as mentioned above was added and mixed. The mixture was incubated at room temperature for 60 min and a malachite green (120 µl, Biomol), which is a color reagent, was added. The mixture was further incubated at room temperature for 20 min to allow for color development. An absorbance at 650 nm was measured on a plate reader based on which the protein tyrosine phosphatase 1B inhibitory activity of the test compound was evaluated. The results are shown in Tables 5 and 6.

The T cell protein tyrosine phosphatase inhibitory activity of the compounds of Examples 1-1, 1-27, 1-48, 1-65, 1-66, 1-68, 1-71, 1-77, 1-78, 1-80, 1-86, 1-88, 1-89, 1-94, 1-97, 1-105, 1-109, 1-110, 1-111, 1-119, 1-120, 1-126, 1-130, 1-132, 1-138, 1-139, 1-140, 1-141, 1-146, 1-149, 1-153, 1-155, 1-156, 1-160, 1-161, 1-162, 3-1, 3-117, 3-120, 3-124, 4-1, 4-2, 4-6, 4-8, 4-9, 4-17, 4-18, 4-19, 4-21, 4-27, 4-30, 4-31, 4-34, 4-36 and 4-37 was evaluated and compared with the aforementioned protein tyrosine phosphatase 1B inhibitory activity. As a result, IC₅₀ of protein tyrosine phosphatase 1B was as low as 1/60 or below of IC₅₀ of T cell protein tyrosine phosphatase.

### Experimental Example 2 (hypoglycemic activity)

A 0.5% methyl cellulose suspension of the test compound was orally administered to 6- to 9-week-old male ob/ob mice grouped according to the glucose level. Only a 0.5% methyl cellulose solution was administered to a control group.

Blood was drawn under fasting condition, which condition was started simultaneously with the administration by means of removing the feed. The blood was drawn under anesthesia from the orbital vein 3 hours after the administration. The blood thus taken was centrifuged and the glucose level was measured from the obtained plasma by the hexokinase method (glucose measurement kit). For evaluation, the percentage (%) of decrease in the glucose level of the test compound-administered group relative to the control group is shown. The results are shown in Tables 5 to 9.

**Table 5**

| Example | PTP1B inhibitory activity (IC50; µM) | blood glucose decreasing rate (%) | | |
|---|---|---|---|---|
| | | Dose (mg/kg) | 3 hours later | |
| 1-1 | 0.28 | 1 | 45 | |
| 1-15 | 0.13 | 1 | 35 | |
| 1-19 | 0.10 | 1 | 35 | |
| 1-25 | 0.13 | 1 | 25 | |
| 1-37 | 0.14 | 3 | 26 | |
| 1-39 | 0.19 1 | | 32 | |
| 1-44 | 0.22 | 1 | 20 | |
| 1-45 | 0.066 | 1 | 32 | |
| 1-48 | 0.23 | 1 | 46 | |
| 1-50 | 0.14 | --- | | |
| 1-53 | 0.24 | --- | | |
| 1-78 | 0.18 | 3 | | 24 |
| 1-141 | 0.35 | --- | | |
| 3-9 | 0.17 | 1 | | 18 |
| 3-10 | 0.20 | 1 | | 18 |
| 3-14 | 0.38 | --- | | |
| 3-15 | 0.27 | 1 | | 25 |
| 3-21 | 0.45 | 1 | | 27 |
| 3-25 | 0.14 | 1 | | 20 |
| 3-26 | 0.11 | --- | | |
| 3-29 | 0.41 | 3 | | 41 |
| 3-32 | 0.18 | 1 | | 14 |
| 3-34 | 0.16 | --- | | |

**Table 6**

| Example | PTP1B inhibitory activity (IC50; µM) | blood glucose decreasing rate (%) | |
|---|---|---|---|
| | | dose (mg/kg) | 3 hours later |
| 3-35 | 0.073 | --- | |
| 3-36 | 0.10 | 3 | 52 |
| 3-52 | 0.54 | 1 | 28 |
| 3-53 | 0.28 | 1 | 20 |
| 3-58 | 0.39 | 1 | 23 |
| 3-64 | 0.21 | 1 | 13 |
| 3-67 | 0.23 | 3 | 20 |
| 3-69 | 0.16 | --- | |
| 3-70 | 0.16 | --- | |
| 3-71 | 0.095 | --- | |
| 3-73 | 0.20 | 1 | 14 |
| 3-76 | 0.48 | 1 | 33 |
| 3-86 | 0.20 | 1 | 16 |
| 3-87 | 0.21 | --- | |
| 3-92 | 0.18 | 1 | 26 |
| 3-98 | 0.16 | 3 | 31 |
| 3-100 | 0.15 | 3 | 20 |
| 3-108 | 0.16 | 1 | 29 |
| 4-1 | 0.15 | --- | |
| 4-6 | 0.092 | --- | |
| 4-9 | 0.12 | --- | |
| 4-30 | 0.17 | --- | |
| 4-35 | 0.087 | --- | |

**Table 7**

| Example | PTP1B inhibitory activity (IC50; µM) | blood glucose decreasing rate (%) | |
|---|---|---|---|
| | | dose (mg/kg) | 3 hours later |
| 1-155 | 0.37 | --- | |
| 1-156 | 0.19 | --- | |
| 1-157 | 0.17 | --- | |
| 1-158 | 0.29 | --- | |
| 1-160 | 0.31 | --- | |
| 1-161 | 0.18 | --- | |
| 1-162 | 0.21 | --- | |
| 1-166 | 0.31 | --- | |
| 1-167 | 0.41 | --- | |
| 1-172 | 0.28 | --- | |
| 1-178 | 0.44 | --- | |
| 1-181 | 0.20 | --- | |
| 1-182 | 0.19 | --- | |
| 4-42 | 0.17 | --- | |
| 4-43 | 0.16 | --- | |
| 4-44 | 0.12 | --- | |
| 4-45 | 0.27 | --- | |
| 4-52 | 0.15 | --- | |
| 4-53 | 0.07 | --- | |
| 4-54 | 0.14 | --- | |
| 4-55 | 0.17 | --- | |
| 4-56 | 0.20 | --- | |
| 4-57 | 0.17 | --- | |

**Table 8**

| Example | PTP1B inhibitory activity (IC50; µM) | blood glucose decreasing rate (%) | |
|---|---|---|---|
| | | dose (mg/kg) | 3 hours later |
| 4-58 | 0.19 | --- | |
| 4-59 | 0.18 | --- | |
| 4-60 | <0.1 | --- | |
| 4-61 | 0.25 | --- | |
| 4-62 | 0.45 | --- | |
| 4-63 | 0.42 | --- | |
| 4-64 | 0.24 | --- | |
| 4-66 | 0.45 | --- | |
| 4-67 | 0.23 | --- | |
| 4-68 | <0.1 | --- | |
| 4-69 | 0.15 | --- | |
| 4-70 | 0.17 | --- | |
| 4-72 | 0.29 | --- | |
| 4-73 | 0.19 | --- | |
| 4-74 | 0.11 | --- | |
| 4-75 | <0.1 | --- | |
| 4-77 | 0.40 | --- | |
| 4-78 | 0.38 | --- | |
| 4-79 | <0.1 | --- | |
| 4-80 | <0.1 | --- | |
| 4-81 | <0.1 | --- | |
| 4-82 | 0.48 | --- | |
| 4-84 | 0.27 | --- | |

**Table 9**

| Example | PTP1B inhibitory activity (IC50; µM) | blood glucose decreasing rate (%) | |
|---|---|---|---|
| | | dose (mg/kg) | 3 hours later |
| 4-87 | 0.12 | --- | |
| 4-88 | <0.1 | --- | |
| 4-89 | 0.49 | --- | |
| 4-90 | 0.16 | --- | |
| 4-91 | 0.17 | --- | |
| 4-92 | <0.1 | --- | |
| 4-93 | <0.1 | --- | |
| 4-94 | 0.12 | --- | |
| 4-95 | 0.28 | --- | |
| 4-96 | 0.12 | --- | |
| 4-97 | 0.11 | --- | |
| 4-98 | <0.1 | --- | |
| 4-99 | <0.1 | --- | |
| 4-100 | <0.1 | --- | |
| 4-101 | 0.10 | --- | |
| 4-102 | 0.37 | --- | |
| 4-104 | 0.20 | --- | |
| 4-106 | 0.16 | --- | |
| 4-107 | <0.1 | --- | |

### Experimental Example 3 (effect of concomitant use with insulin on hypoglycemic activity)

A 0.5% methyl cellulose suspension of the test compound {note: Example 3-40 (30 mg/kg)} (test compound and insulin concomitant use group) or a 0.5% methyl cellulose solution alone (insulin administration group) was orally administered to 7-week-old, male SD rats once a day for 8 days, and 3 hours later, insulin (0.6 U/kg) was subcutaneously administered.

Blood was drawn on the day of the start of the administration, before administration of insulin on day 8 and 1 hour thereafter from the tail vein. For drawing blood, the animals were fasted after insulin administration. The blood thus taken was centrifuged and the glucose level was measured from the obtained plasma by the hexokinase method (glucose measurement kit). For evaluation, the percentage (%) of decrease in the glucose level of the test compound and insulin concomitant use group and insulin administration group one hour later relative to before insulin administration is shown. The results are shown in Table 10.

**Table 10**

| | dose of test compound (mg/kg) | blood sugar decreasing ratio (%) | |
|---|---|---|---|
| | | Day of start of administration | Day 8 |
| Insulin administration group | ------ | 29 | 17 |
| test compound and insulin concomitant use group (Example 3-40) | 30 | 43 | 36 |

### Experimental Example 4 (effect of concomitant use with glibenclamide on hypoglycemic activity)

To 10-week-old female ob/ob mice grouped according to glucose level were orally administered a 0.5% methyl cellulose suspension of the test compound {note: Example 4-2 (1 mg/kg)} (test compound-administered group), a 0.5% methyl cellulose suspension of glibenclamide (3 mg/kg) (glibenclamide-administered group), simultaneously both a 0.5% methyl cellulose suspension of the test compound and a 0.5% methyl cellulose suspension (glibenclamide 3 mg/kg) (test compound and glibenclamide concomitant use group), or a 0.5% methyl cellulose solution alone (control group).

Blood was drawn under fasting condition, which condition was started simultaneously with the administration by means of removing the feed. The blood was drawn under anesthesia from the orbital vein 3 and 5 hours after the administration. The blood thus taken was centrifuged and the glucose level was measured from the obtained plasma by the hexokinase method (glucose kit). For evaluation, the percentage (%) of decrease in the glucose level of each group other than the control group relative to the control group is shown. The results are shown in Table 11.

**Table 11**

| | dose of test compound (mg/kg) | blood sugar decreasing ratio (%) | |
|---|---|---|---|
| | | 3 hours later | 5 hours later |
| Glibenclamide-administered group | ----- | 44 | 40 |
| test compound-administered group (Example 4-2) | 1 | 48 | 20 |
| test compound and glibenclamide concomitant use group (Example 4-2) | 1 | 64 | 60 |

### Experimental Example 5 (effect of concomitant use with tolbutamide on hypoglycemic activity)

To 10-week-old male db/db mice grouped according to glucose level were orally administered a 0.5% methyl cellulose suspension of the test compound {note: Example 4-5 (5 mg/kg)} (test compound-administered group), a 0.5% methyl cellulose suspension of tolbutamide (30 mg/kg) (tolbutamide-administered group), simultaneously both a 0.5% methyl cellulose suspension of the test compound and a 0.5% methyl cellulose suspension (tolbutamide 30 mg/kg) (test compound and tolbutamide concomitant use group), or a 0.5% methyl cellulose solution alone (control group).

Blood was drawn under fasting condition, which condition was started simultaneously with the administration by means of removing the feed. The blood was drawn under anesthesia from the orbital vein 5 hours after the administration. The blood thus taken was centrifuged and the glucose level was measured from the obtained plasma by the hexokinase method (glucose measurement kit). For evaluation, the percentage (%) of decrease in the glucose level of each group other than the control group relative to the control group is shown. The results are shown in Table 12.

**Table 12**

| | dose of test compound (mg/kg) | blood sugar decreasing ratio (%) |
|---|---|---|
| | | 5 hours later |
| Tolbutamide-administered group | ----- | 26 |
| test compound-administered group (Example 4-5) | 5 | 26 |
| test compound and tolbutamide concomitant use group (Example 4-5) | 5 | 30 |

### Experimental Example 6 (effect of concomitant use with nateglinide on hypoglycemic activity)

To 10-week-old male db/db mice grouped according to glucose level were orally administered a 0.5% methyl cellulose suspension of the test compound {note: Example 4-5 (5 mg/kg)} (test compound-administered group), a 0.5% methyl cellulose suspension of nateglinide (30 mg/kg) (nateglinide-administered group), simultaneously both a 0.5% methyl cellulose suspension of the test compound and a 0.5% methyl cellulose suspension (nateglinide 30 mg/kg) (test compound and nateglinide concomitant use group), or a 0.5% methyl cellulose solution alone (control group).

Blood was drawn under fasting condition, which condition was started simultaneously with the administration by means of removing the feed. The blood was drawn under anesthesia from the orbital vein 2 hours after the administration. The blood thus taken was centrifuged and the glucose level was measured from the obtained plasma by the hexokinase method (glucose measurement kit). For evaluation, the percentage (%) of decrease in the glucose level of each group other than the control group relative to the control group is shown. The results are shown in Table 13.

**Table 13**

| | dose of test compound (mg/kg) | blood sugar decreasing ratio (%) |
|---|---|---|
| | | 2 hours later |
| Nateglinide-administered group | ----- | 5 |
| test compound-administered group (Example 4-5) | 5 | 11 |
| test compound and nateglinide concomitant use group (Example 4-5) | 5 | 19 |

### Experimental Example 7 (effect of concomitant use with metformin on hypoglycemic hydrochloride)

To 10-week-old female ob/ob mice grouped according to glucose level were orally administered a 0.5% methyl cellulose suspension of the test compound {note: Example 4-2 (1 mg/kg)} (test compound-administered group), a 0.5% methyl cellulose suspension of metformin chloride (30 mg/kg) (metformin-administered group), simultaneously both a 0.5% methyl cellulose suspension of the test compound and a 0.5% methyl cellulose suspension (metformin chloride 30 mg/kg) (test compound and metformin concomitant use group), or a 0.5% methyl cellulose solution alone (control group). The ob/ob mice were fasted for 3 hours before administration.

Blood was drawn under fasting condition, which condition was started simultaneously with the administration by means of removing the feed. The blood was drawn under anesthesia from the orbital vein 3 and 5 hours after the administration. The blood thus taken was centrifuged and the glucose level was measured from the obtained plasma by the hexokinase method (glucose kit). For evaluation, the percentage (%) of decrease in the glucose level of each group other than the control group relative to the control group is shown. The results are shown in Table 14.

**Table 14**

| | dose of test compound (mg/kg) | blood sugar decreasing ratio (%) | |
|---|---|---|---|
| | | 3 hours later | 5 hours later |
| Metformin-administered group | ----- | 17 | 39 |
| test compound-administered group (Example 4-2) | 1 | 19 | 20 |
| test compound and metformin concomitant use group (Example 4-2) | 1 40 | | 51 |

### Experimental Example 8 (effect of concomitant use with voglibose on hypoglycemic activity)

To 9-week-old female ob/ob mice grouped according to glucose level were orally administered a 0.5% methyl cellulose suspension of the test compound (note: Example 4-2 (1 mg/kg)} (test compound-administered group), a 0.5% methyl cellulose suspension of voglibose (0.3 mg/kg) (voglibose-administered group), simultaneously both a 0.5% methyl cellulose suspension of the test compound and a 0.5% methyl cellulose suspension (voglibose 0.3 mg/kg) (test compound and voglibose concomitant use group), or a 0.5% methyl cellulose solution alone (control group). One hour later, sucrose (2 g/5 mL/kg) was loaded.

Blood was drawn under fasting condition, which condition was started simultaneously with the loading by means of removing the feed. The blood was drawn under anesthesia from the orbital vein 1 and 2 hours after the sucrose loading. The blood thus taken was centrifuged and the glucose level was measured from the obtained plasma by the hexokinase method (glucose kit). For evaluation, the percentage (%) of decrease in the glucose level of each group other than the control group relative to the control group is shown. The results are shown in Table 15.

**Table 15**

| | dose of test compound (mg/kg) | blood sugar decreasing ratio (%) | |
|---|---|---|---|
| | | 1 hour later | 2 hours later |
| Voglibose-administered group | ----- | 35 | 38 |
| test compound-administered group (Example 4-2) | 1 | 32 | 40 |
| test compound and voglibose concomitant use group (Example 4-2) | 1 | 49 | 54 |

### Experimental Example 9 (effect of concomitant use with Pioglitazone hydrochloride on hypoglycemic activity)

To 8-week-old male db/db mice grouped according to full feed glucose level were orally administered a 0.5% methyl cellulose suspension of the test compound {note: Example 4-5 (10 mg/kg)} (test compound-administered group), a 0.5% methyl cellulose suspension of Pioglitazone hydrochloride (3 mg/kg) (Pioglitazone-administered group), simultaneously both a 0.5% methyl cellulose suspension of the test compound and a 0.5% methyl cellulose suspension (Pioglitazone hydrochloride 3 mg/kg) (test compound and Pioglitazone concomitant use group), or a 0.5% methyl cellulose solution alone (control group), once a day for 3 days.

**Table 17-1**

| Example | solvent, Hz | NMR(δ) |
|---|---|---|
| 1-1 | DMSO-d6, 300MHz | 0.69 (t, J = 7.3 Hz, 6H), 1.16 (d, J = 6.5 Hz, 6H), 1.48-1.31 (m, 2H), 1.64- 1.48 (m, 2H), 2.19- 2.06 (m, 1H), 4.23 (quint, J = 6.6 Hz, 1H), 5.67 (s, 2H), 6.62 (d, J = 8.7 Hz, 2H), 6.89 (d, J = 8.6 Hz, 2H), 7.36 (d, J = 8.2 Hz, 2H), 7.90 (d, J = 8.2 Hz, 2H), 7.99 (dd, J = 3.0, 1.5 Hz, 1H), 8.11 (s, 1H), 8.64 (d, J = 2.9 Hz, 1H), 8.72 (d, J = 1.5 Hz, 1H), 13.43 (br s, 1H). |
| 1-2 | DMSO-d6, 400MHz | 0.68 (t, J = 7.4 Hz, 6 H), 1.14 (d, J = 8.0 Hz, 6 H), 1.32-1.46 (m, 2 H), 1.50- 1.62 (m, 2 H), 2.08- 2.17 (m, 1 H), 4.23 (quint, J = 6.6 Hz, 1 H), 4.37 (s, 2 H), 5.57 (s, 2 H), 6.62 (d, J = 8.8 Hz, 2 H), 6.88 (d, J = 8.8 Hz, 2 H), 7.17 (d, J = 9.2 Hz, 2 H), 7.36 (d, J = 8.3 Hz, 2 H), 7.89 (d, J = 8.8 Hz, 2 H), 7.91 (d, J = 9.0 Hz, 2 H), 8.09 (s, 1 H), 12.64 (br s, 1 H). |
| 1-3 | DMSO-d6, 400MHz | 0.68(t, J=7.2Hz, 6H), 1.16(d, J=6.4Hz, 2H), 1.35-1.45(m, 2H), 1.50-1.60(m, 2H), 2.10-2.15(m, 1H), 4.20-4.25(m, 1H), 4.37(s, 2H), 5.75(s, 2H), 6.61(d, J=10.2Hz, 2H), 6.87(d, J=11.6Hz, 2H), 6.99(d, J=8.0Hz, 1H), 7.34(d, J=10.8Hz, 2H), 7.88(d, J=8.1Hz, 2H), 8.04(s, 1H), 8.18(d, J=8.0Hz, 1H), 8.70(s, 1H) |
| 1-4 | DMSO-d6, 400MHz | 0.79(t, J=7.2Hz, 6H), 1.05-1.11(m, 4H), 1.39-1.50(m, 4H), 2.34-2.39(m, 1H), 2.97(s, 3H), 4.52(s, 2H), 5.65(s, 2H), 6.67(d, J=8.8Hz, 2H), 6.93(d, J=8.0Hz, 2H), 7.30(d, J=8.0Hz, 2H), 7.91(d, J=8.7Hz, 2H), 7.96-7.97(m, 1H), 8.12(s, 1H), 8.62(d, J=2.8Hz, 1H), 8.71(d, J=1.4Hz, 1H) |
| 1-5 | DMSO-d6, 400MHz | 0.79(t, J=7.2Hz, 6H), 1.03-1.11(m, 4H), 1.39-1.50(m, 4H), 2.34-2.39(m, 1H), 2.97(s, 3H), 4.52(s, 2H), 5.56(s, 2H), , 6.67(d, J=8.8Hz, 2H), 6.93(d, J=8.8Hz, 2H), 7.15(d, J=8.8Hz, 2H), 7.30(d, J=8.8Hz, 2H), 7.91-7.93(m, 4H), 8.11(s, 1H) |

**Table 17-2**

| Example | solvent, Hz | NMR(δ) |
|---|---|---|
| 1-6 | DMSO-d6, 300MHz | 0.85(t, J=7.3Hz, 6H), 1.10(d, J=6.8Hz, 6H), 1.25-1.64(m, 8H), 2.65-2.74(m, 1H), 3.84-3.91(m, 1H), 4.37(s, 2H), 5.77(s, 2H), 6.66(d, J=9.1Hz, 2H), 6.94(d, J=8.7Hz, 2H), 7.06(d, J=9.4Hz, 1 H), 7.31(d, J=8.6Hz, 2H), 7.84(d, J=8.3Hz, 2H), 8.04(s, 1 H), 8.22(dd, J=2.3, 8.7Hz, 1H), 8.75(dd, J=0.7, 2.6Hz, 1H), 13.05(brs, 1H) |
| 1-7 | DMSO-d6, 300MHz | 0.85(t, J=7.2Hz, 6H), 1.11(d, J=6.8Hz, 6H), 1.25-1.64(m, 8H), 2.65-2.74(m, 1H), 3.83-3.92(m, 1H), 4.37(s, 2H), 5.65(s, 2H), 6.66(d, J=8.6Hz, 2H), 6.94(d, J=8.7Hz, 2H), 7.32(d, J=8.3Hz, 2H), 7.86(d, J=8.3Hz, 2H), 7.98-7.98(brs, 1H), 8.09(s, 1 H), 8.63-8.63(brs, 1H), 8.72(s, 1H), 13.38(brs, 1H) |
| 1-8 | DMSO-d6, 300MHz | 0.92(d, J=6.8Hz, 6H), 1.12(d, J=7.2Hz, 6H), 2.00-2.13(m, 1H), 2.67-2.78(m, 1H), 3.24(d, J=7.1Hz, 2H), 4.58(s, 2H), 5.66(s, 2H), 6.59(d, J=9.0Hz, 2H), 6.97(d, J=8.6Hz, 2H), 7.26(d, J=8.3Hz, 2H), 7.88(d, J=8.3Hz, 2H), 7.99-7.99(m, 1H), 8.10(s, 1H), 8.64(d, J=3.0Hz. 1H), 8.72(d, J=1.5Hz, 1 H), 13.46(brs, 1H) |
| 1-9 | DMSO-d6, 300MHz | 0.85(t, J=7.2Hz, 6H), 1.11(d, J=7.2Hz, 6H), 1.29-1.62(m, 8H), 2.65-2.74(m, 1H), 3.83-3.95(m, 1H), 4.37(s, 2H), 5.56(s, 2H), 6.66(d, J=8.6Hz, 2H), 6.94(d, J=8.7Hz, 2H), 7.17(d, J=9.1Hz, 2H), 7.32(d, J=8.3Hz, 2H), 7.85(d, J=8.3Hz, 2H), 7.92(d, J=9.0Hz, 2H), 8.07(s, 1H), 12.58(brs, 1H) |
| 1-10 | DMSO-d6, 300MHz | 0.85(t, J=7.2Hz, 6H), 1.11(d, J=6.8Hz, 6H), 1.25-1.68(m, 8H), 2.65-2.75(m, 1H), 3.84-3.93(m, 1H), 4.37(s, 2H), 5.55(s, 2H), 6.66(d, J=8.7Hz, 2H), 6.94(d, J=8.7Hz, 2H), 7.32(d, J=8.3Hz, 2H), 7.35-7.36(m, 1H), 7.45(t, J=7.9Hz, 1H), 7.57-7.62(m, 2H), 7.86(d, J=8.3Hz, 2H), 8.06(s, 1H), 12.91(brs, 1H) |

**Table 17-3**

| Example | solvent, Hz | NMR(δ) |
|---|---|---|
| 1-11 | DMSO-d6, 300MHz | 0.87(t, J = 6.0Hz, 6H), 1.09(d,J = 9.0Hz, 6H), 1.37-1.70(m,4H), 2.65-2.72(m,1H), 3.55-3.82(m,1H), 4.35(s,2H), 5.74(s,2H), 6.67(d,J = 9.0Hz, 2H), 6.92(d,J = 9.0Hz, 2H), 7.04(d,J = 9.0Hz, 1H), 7.31(d,J = 9.0Hz, 2H), 7.82(d,J = 9.0Hz, 2H), 8.01(s,1H), 8.20(d,J = 6.0Hz, 1H), 8.73(d,J = 3.0Hz, 1H) |
| 1-12 | DMSO-d6, 300MHz | 0.88(t, J = 6.0Hz, 6H), 1.10(t, J = 6.0Hz, 6H), 1.37-1.72(m,4H), 2.53-2.76(m,1H), 3.57-3.84(m,1H), 4.36(s,2H), 5.63(s,2H), 6.45-6.76(m,2H), 6.83-7.07(m,2H), 7.19-7.42(m,2H), 7.80-7.86(m,2H), 7.92-7.95(m,1H), 8.04-8.08(m,1H), 8.59(m,1H), 8.7(m,1H) |
| 1-13 | DMSO-d6, 400MHz | 0.68 (t, J = 7.3 Hz, 6 H), 1.16 (d, J = 6.7 Hz, 6 H), 1.33-1.46 (m, 2 H), 1.49- 1.62 (m, 2 H), 2.07- 2.17 (m, 1 H), 4.22 (quit, J = 6.5 Hz, 1 H), 4.36 (s, 2 H), 4.78 (s, 2 H), 6.61 (d, J = 8.8 Hz, 2 H), 6.88 (d, J = 8.6 Hz, 2 H), 7.33 (d, J = 8.3 Hz, 2 H), 7.50 (d, J = 8.8 Hz, 2 H), 7.84 (d, J = 8.6 Hz, 4 H), 7.92 (s, 1 H), 12.80 (br s, 1 H). |
| 1-14 | DMSO-d6, 400MHz | 0.79(t, J=7.2Hz, 6H), 1.03-1.11 (m, 4H), 1.39-1.50(m, 4H), 2.34-2.39(m, 1 H), 2.97(s, 3H), 4.50(s, 2H), 4.78(s, 2H),, 6.66(d, J=8.8Hz, 2H), 6.93(d, J=8.4Hz, 2H), 7.28(d, J=8.0Hz, 2H), 7.50(d, J=8.0Hz, 2H), 7.82-7.85(m, 4H), 7.94(s, 1H) |
| 1-15 | DMSO-d6, 300MHz | 0.79(t, J=7.1Hz, 6H), 1.02-1.16(m, 4H), 1.35-1.52(m, 4H), 2.32-2.42(m, 1H), 2.87(s, 3H), 2.96(s, 3H), 4.51(s, 2H), 4.68(s, 2H), 6.67(d, J=8.8Hz, 2H), 6.94(d, J=8.8Hz, 2H), 7.27(d, J=8.1Hz, 2H), 7.79(d, J=8.4Hz, 2H), 7.97(d, J=8.8Hz, 2H), 8.02(s, 1H), 8.13(d, J=8.8Hz, 2H), 13.20(brs, 1H) |
| 1-16 | DMSO-d6, 300MHz | 0.79(t, J=7.3Hz, 6H), 1.02-1.15(m, 4H), 1.37-1.53(m, 4H), 1.78-1.88(m, 2H), 1.99(t, J=7.0Hz, 2H), 2.32-2.42(m, 1H), 2.89(s, 3H), 2.97(s, 3H), 3.21-3.26(m, 2H), 4.52(s, 2H), 4.67(s, 2H), 6.68(d, J=8.8Hz, 2H), 6.94(d, J=8.8Hz, 2H), 7.30(d, J=8.1 Hz, 2H), 7.89(d, J=8.1 Hz, 2H), 8.04(s, 1H) |

**Table 17-4**

| Example | solvent, Hz | NMR(δ) |
|---|---|---|
| 1-17 | DMSO-d6, 400MHz | 1.11-1.37(m, 5H), 1.61-1.78(m, 5H), 2.23-2.33(m, 1H), 3.09(s, 3H), 4.22(s, 2H), 4.83(s, 2H), 5.97(brs, 1H), 6.48(d, J=8.4Hz, 2H), 6.87(d, J=8.4Hz, 2H), 7.12(s, 1H), 7.34(d. J=8.6Hz, 2H), 7.44(d, J=8.1 Hz, 2H), 7.78(d, J=8.2Hz, 2H), 7.92(d, J=8.6Hz, 2H), 12.78(brs, 1H) |
| 1-18 | DMSO-d6, 400MHz | 1.12-1.40(m, 5H), 1.63-1.82(m, 5H), 2.29-2.38(m, 1H), 2.96(s, 3H), 3.09(s, 3H), 4.49(s, 2H), 4.82(s, 2H), 6.65(d, J=8.6Hz, 2H), 6.99(d, J=8.6Hz, 2H), 7.12(s, 1H), 7.21(d, J=8.1Hz, 2H), 7.43(d, J=8.1Hz, 2H), 7.77(d, J=8.1Hz, 2H), 7.92(d, J=8.1 Hz, 2H) |
| 1-19 | DMSO-d6, 300MHz | 0.78(t, J=7.4Hz, 6H), 0.98-1.17(m, 4H), 1.32-1.54(m, 4H), 2.29-2.42(m, 1H), 2.95(s, 3H), 3.09(s, 3H), 4.49(s, 2H), 4.83(s, 2H), 6.66(d, J=8.7Hz, 2H), 6.93(d, J=8.7Hz, 2H), 7.14(s, 1H), 7.23(d, J=8.3Hz, 2H), 7.43(d, J=8.3Hz, 2H), 7.78(d, J=8.3Hz, 2H), 7.92(d, J=8.3Hz, 2H), 12.82(brs, 1H) |
| 1-20 | DMSO-d6, 300MHz | 0.68(t, J=7.2Hz, 6H), 1.15(d, J=6.4Hz, 6H), 1.32-1.47(m, 2H), 1.49-1.64(m, 2H), 2.05-2.19(m, 1H), 3.09(s, 3H), 4.15-4.28(m, 1H), 4.34(s, 2H), 4.83(s, 2H), 6.61 (d, J=8.6Hz, 2H), 6.87(d, J=8.6Hz, 2H), 7.12(s, 1H), 7.28(d, J=8.3Hz, 2H), 7.43(d, J=7.9Hz, 2H), 7.77(d, J=8.3Hz, 2H), 7.92(d, J=7.9Hz, 2H), 12.82(brs, 1H) |
| 1-21 | DMSO-d6, 300MHz | 0.68 (t, J = 7.3 Hz, 6 H), 1.15 (d, J = 6.6 Hz, 6 H), 1.33-1.46 (m, 2 H), 1.48- 1.61 (m, 2 H), 2.06- 2.18 (m, 1 H), 2.34 (s, 3 H), 3.85 (d, J = 15.0 Hz, 1 H), 4.02 (d, J = 15.4 Hz, 1H), 4.22 (quint, J = 6.6 Hz, 1 H), 4.36 (s, 2 H), 4.50 (s, 2 H), 6.62 (d, J = 8.4 Hz, 2 H), 6.88 (d, J = 8.8 Hz, 2 H), 7.32 (d, J = 8.0 Hz, 2 H), 7.34 (t, J = 7.1 Hz, 1 H), 7.40 (t, J = 7.0 Hz, 2 H), 7.47 (d, J = 7.0 Hz, 2 H), 7.84 (d, J = 8.1 Hz, 2 H), 7.93 (s, 1 H), 12.66 (br s, 1 H). |

**Table 17-5**

| Example | solvent, Hz | NMR(δ) |
|---|---|---|
| 1-22 | DMSO-d6, 300MHz | 0.68 (t, J = 7.3 Hz, 6 H), 1.16 (d, J = 6.6 Hz, 6 H), 1.30-1.47 (m, 2 H), 1.48- 1.61 (m, 2 H), 2.07- 2.19 (m, 1 H), 2.37 (s, 3 H), 2.94 (dd, J = 14.1, 8.2 Hz, 1 H), 3.08 (dd, J = 13.9, 7.0 Hz, 1 H), 3.70 (t, J = 7.7 Hz, 1 H), 4.02 (d, J = 15.8 Hz, 1 H), 4.17 (d, J = 15.8 Hz, 1 H), 4.22 (t, J = 6.0 Hz, 1 H), 4.36 (s, 2 H), 6.62 (d, J = 8.8 Hz, 2 H), 6.88 (d, J = 8.8 Hz, 2 H), 7.16- 7.30 (m, 5 H), 7.32 (d, J = 8.4 Hz, 2 H), 7.83 (d, J = 8.4 Hz, 2 H), 7.86 (s, 1 H), 12.53 (br s, 1 H). |
| 1-23 | DMSO-d6, 300MHz | 0.92 (d, J = 6.6 Hz, 6 H), 1.19 (s, 9 H), 2.05 (sept, J = 7.2 Hz, 1 H), 3.24 (d, J = 7.5 Hz, 2 H), 3.35 (s, 2 H), 3.86 (s, 2 H), 4.17 (s, 2 H), 4.57 (s, 2 H), 6.58 (d, J = 8.7 Hz, 2 H), 7.11 (d, J = 9.0 Hz, 2 H), 7.20- 7.45 (m, 5 H), 7.84 (d, J = 8.1 Hz, 2 H), 7.94 (s, 1 H), 13.38 (br s, 1 H). |
| 1-24 | DMSO-d6, 300MHz | 0.92 (d, J = 6.6 Hz, 6 H), 2.05 (sept, J = 6.8 Hz, 1 H), 3.28 (d, J = 6.8 Hz, 2 H), 3.36 (s, 2 H), 3.86 (s, 2 H), 4.17 (s, 2 H), 4.61 (s, 2 H), 6.65 (d, J = 9.0 Hz, 2 H), 7.10 (d, J = 9.0 Hz, 2 H), 7.21 (d, J = 7.8 Hz, 2 H), 7.07- 7.43 (m, 5 H), 7.85 (d, J = 8.4 Hz, 2 H), 7.95 (s, 1 H), 12.37 (br s, 1 H). |
| 1-25 | DMSO-d6, 300MHz | 0.79 (t, J = 7.1 Hz, 6 H), 1.00- 1.28 (m, 4 H), 1.34- 1.58 (m, 4 H), 2.30- 2.42 (m, 3 H), 2.97 (s, 3 H), 3.36 (s, 2 H), 3.87 (s, 2 H), 4.18 (s, 2 H), 4.51 (s, 2 H), 6.67 (d, J = 8.7 Hz, 2 H), 6.93 (d, J = 8.7 Hz, 2 H), 7.28 (d, J = 8.3 Hz, 2 H), 7.22- 7.43 (m, 5 H), 7.87 (d, J = 8.4 Hz, 2 H), 7.97 (s, 1 H), 12.38 (br s, 1 H). |
| 1-26 | MeOH-d4, 300MHz | 0.70(t, J=7.3Hz, 6H), 1.26(d, J=6.4Hz, 6H), 1.36-1.53(m, 2H), 1.53-1.70(m, 2H), 2.11-2.24(m, 1H), 3.45(s, 2H), 3.97(s, 2H), 4.16-4.30(m, 3H), 4.50(s, 2H), 6.81(d, J=8.6Hz, 2H), 6.97(d, J=8.7Hz, 2H), 7.21-7.38(m, 5H), 7.44(d, J=6.7Hz, 2H), 7.68(s, 1H), 7.78(d, J=8.3Hz, 2H) |

**Table 17-6**

| Example | solvent, Hz | NMR(δ) |
|---|---|---|
| 1-27 | DMSO-d6, 300MHz | 0.92 (d, J = 6.8 Hz, 6 H), 1.19 (s, 9 H), 2.05 (sept, J = 6.8 Hz, 1 H), 3.25 (d, J = 7.1 Hz, 2 H), 4.01 (s, 2 H), 4.58 (s, 2 H), 4.85 (s, 2 H), 6.60 (d, J = 8.7 Hz, 2 H), 6.93 (t, J = 7.3 Hz, H), 7.11 (d, J = 9.1 Hz, 2 H), 7.23 (t, J = 7.9 Hz, 2 H), 7.24 (d, J = 8.7 Hz, 2 H), 7.43 (d, J = 7.5 Hz, 2 H), 7.85 (d, J = 8.3 Hz, 2 H), 7.94 (s, 1 H), 9.47 (br s, 1 H). |
| 1-28 | DMSO-d6, 300MHz | 0.92 (d, J = 6.4 Hz, 6 H), 1.19 (s, 9 H), 2.05 (sept, J = 7.0 Hz, 1 H), 3.25 (d, J = 7.2 Hz, 2 H), 4.05- 4.25 (m, 2 H), 4.58 (s, 2 H), 4.76- 5.01 (m, 2 H), 6.59 (d, J = 8.7 Hz, 2 H), 7.11 (d, J = 8.7 Hz, 2 H), 7.26 (d, J = 7.9 Hz, 2 H), 7.33- 7.53 (m, 5 H), 7.81- 7.90 (m, 2 H), 7.97- 7.03 (m, 1 H), 12.84 (br s, 1 H). |
| 1-29 | DMSO-d6, 300MHz | 0.91 (d, J = 6.6 Hz, 6 H), 1.22 (s, 9 H), 2.04 (sept, J = 6.8 Hz, 1 H), 3.19 (d, J = 7.0 Hz, 2 H), 4.36 (br s, 2 H), 4.54 (s, 2 H), 5.07 (s, 2 H), 6.67 (d, J = 8.8 Hz, 2 H), 7.13 (d, J = 8.8 Hz, 2 H), 7.26 (d, J = 8.4 Hz, 2 H), 7.47 (ddd, J = 7.6, 4.8, 1.2 Hz, 1 H), 7.70 (dt, J = 7.7, 1.1 Hz, 1 H), 7.81 (d, J = 8.1 Hz, 2 H), 7.82 (s, 1 H), 7.91 (td, J = 7.7, 1.8 Hz, 1 H), 8.56 (d, J = 4.8 Hz, 1 H). |
| 1-30 | DMSO-d6, 300MHz | 0.92 (d, J = 6.6 Hz, 6 H), 1.22 (s, 9 H), 2.04 (sept, J = 6.8 Hz, 1 H), 3.20 (d, J = 7.0 Hz, 2 H), 4.18 (s, 2 H), 4.54 (s, 2 H), 4.93 (s, 2 H), 6.67 (d, J = 9.1 Hz, 2 H), 7.13 (d, J = 8.8 Hz, 2 H), 7.44 (ddd, J = 7.9, 5.0, 0.9 Hz, 1 H), 7.78-7.86 (m, 4 H), 8.60- 8.65 (m, 2 H). |
| 1-31 | DMSO-d6, 300MHz | 0.92 (d, J = 6.8 Hz, 6 H), 1.19 (s, 9 H), 2.05 (sept, J = 6.6 Hz, 1 H), 3.24 (d, J = 7.2 Hz, 2 H), 4.11 (s, 2 H), 4.58 (s, 2 H), 4.83 (s, 2 H), 6.58 (d, J = 9.0 Hz, 2 H), 7.11 (d, J = 8.7 Hz, 2 H), 7.23 (d, J = 8.3 Hz, 2 H), 7.55 (t, J = 7.5 Hz, 2 H), 7.63 (t, J = 7.3 Hz, 1 H), 7.76 (d, J = 8.3 Hz, 2 H), 7.86 (d, J = 7.5 Hz, 2 H), 7.95 (s, 1 H), 12.82 (br s, 1 H). |

**Table 17-7**

| Example | solvent, Hz | NMR(δ) |
|---|---|---|
| 1-32 | DMSO-d6, 300MHz | 0.69 (t, J = 7.3 Hz, 6 H), 1.16 (d, J = 6.6 Hz, 6 H), 1.33-1.46 (m, 2 H), 1.47- 1.64 (m, 2 H), 2.07- 2.18 (m, 1 H), 3.09 (dd, J = 16.5, 3.3 Hz, 1 H), 3.18 (dd, J = 16.1, 6.2 Hz, 1 H), 3.94 (d, J = 16.1 Hz, 1 H), 3.97 (dd, J = 5.9, 3.6 Hz, 1 H), 4.09 (d, J = 15.8 Hz, 1 H), 4.21 (d, J = 6.2 Hz, 1 H), 4.26 (d, J = 8.4 Hz, 1 H), 4.37 (s, 2 H), 4.39 (d, J = 15.8 Hz, 1 H), 6.62 (d, J = 8.8 Hz, 2 H), 6.89 (d, J = 8.4 Hz, 2 H), 7.01- 7.17 (m, 4 H), 7.34 (d, J = 8.1 Hz, 2 H), 7.87 (d, J = 8.0 Hz, 2 H), 7.94 (s, 1 H), 12.51 (br s, 1 H). |
| 1-33 | DMSO-d6 300MHz | 0.69(t, J=7.3Hz, 6H), 1.16(d, J=6.4Hz, 6H), 1.32-1.48(m, 2H), 1.48-1.65(m, 2H), 2.06-2.20(m, 1H), 3.09(dd, J=3.4, 16.2Hz, 1 H), 3.18(dd, J=6.0, 16.2Hz, 1H), 3.86-4.00(m, 2H), 4.09(d, J=15.4Hz, 1 H), 4.16-4.45(m, 5H), 6.62(d, J=8.7Hz, 2H), 6.88(d, J=8.7Hz, 2H), 6.99-7.19(m, 4H), 7.34(d, J=8.3Hz, 2H), 7.87(d, J=8.3Hz, 2H), 7.94(s, 1H), 12.55(brs, 1H) |
| 1-34 | DMSO-d6, 300MHz | 0.79(t, J=7.3Hz, 6H), 0.98-1.17(m, 4H), 1.33-1.58(m, 4H), 2.30-2.44(m, 1H), 2.98(s, 3H), 3.10(dd, J=2.6, 15.8Hz, 1 H), 3.19(dd, J=6.0, 15.8Hz, 1 H), 3.87-4.03(m, 2H), 4.10(d, J=15.5Hz, 1 H), 4.26(d, J=15.8Hz, 1H), 4.41(d, J=15.8Hz, 1H), 4.52(s, 2H), 6.68(d, J=7.9Hz, 2H), 6.94(d, J=8.3Hz, 2H), 6.99-7.20(m, 4H), 7.29(d, J=8.3Hz, 2H), 7.88(d, J=7.9Hz, 2H), 7.97(s, 1H) |
| 1-35 | DMSD-d6 300MHz | 0.89(t, J = 9.0Hz, 6H), 1.09(t, J = 9.0Hz, 6H), 1.32-1.73(m,4H), 2.61-2.75(m,1H), 3.09(s,2H), 3.65-3.74(m, 1H), 3.84-.89(m,2H), 4.06-4.19(m,1H), 4.24-4.25(m,1H), 4.33-4.40(m,3H), 6.68(d,J = 9.0Hz, 2H), 6.91-6.94(m,2H), 6.98-7.04(m,1H), 7.10-7.13(m,3H), 7.30(d,J = 6.0Hz, 2H), 7.81 (d,J = 6.0Hz, 2H), 7.90(s,1H) |
| 1-36 | DMSO-d6, 400MHz | 1.14-1.42(m, 5H), 1.64-1.82(m, 5H), 2.38-2.45(m, 1 H), 3.60(s, 3H), 5.08(s, 2H), 6.92(d, J=8.6Hz, 2H), 7.12(d, J=8.6Hz, 2H), 7.41(s, 1H), 7.47(d, J=8.2Hz, 2H), 7.70(d, J=8.6Hz, 2H), 7.89(d, J=8.2Hz, 2H), 7.99(d, J=8.6Hz, 2H), 12.81(brs, 1H) |

**Table 17-8**

| Example | solvent, Hz | NMR(δ) |
|---|---|---|
| 1-37 | DMSO-d6, 400MHz J=8.1Hz, 2H) | 1.14-1.42(m, 5H), 1.64-1.81 (m, 5H), 2.37-2.46(m, 1H), 3.10(s, 3H), 4.84(s, 2H), 5.06(s, 2H), 6.91 (d, J=8.6Hz, 2H), 7.12(d, J=8.6Hz, 2H), 7.20(s, 1 H), 7.43(d, J=5.4Hz, 2H), 7.45(d, J=5.4Hz, 2H), 7.86(d, J=8.1 Hz, 2H), 7.93(d, |
| 1-38 | DMSO-d6, 400MHz | 0.61(t, J=7.4Hz, 3H), 1.20(s, 6H), 1.57(q, J=7.3Hz, 2H), 3.10(s, 3H), 4.83(s, 2H), 5.06(s, 2H), 6.92(d, J=8.8Hz, 2H), 7.20-7.22(m, 3H), 7.43(d, J=8.8Hz, 4H), 7.85(d, J=8.0Hz, 2H), 7.91(d, J=8:0Hz, 2H) |
| 1-39 | DMSO-d6, 300MHz | 0.79(t, J=7.3Hz, 6H), 0.93-1.18(m, 4H), 1.32-1.59(m, 4H), 2.38-2.55(m, 1H), 3.11(s, 3H), 4.84(s, 2H), 5.06(s, 2H), 6.92(d, J=8.6Hz, 2H), 7.06(d, J=8.6Hz, 2H), 7.22(s, 1H), 7.45(d, J=8.2Hz, 4H), 7.87(d, J=8.2Hz, 2H), 7.93(d, J=8.2Hz, 2H), 12.83(brs, 1H) |
| 1-40 | DMSO-d6, 300MHz | 0.79(t, J=7.3Hz, 6H), 0.99-1.17(m, 4H), 1.38-1.60(m, 4H), 2.40-2.52(m, 1H), 3.07(s, 3H), 4.73(s, 2H), 5.06(s, 2H), 6.93(d, J=8.7Hz, 2H), 7.06(d, J=8.7Hz, 2H), 7.19-7.23(m, 3H), 7.45(d, J=8.3Hz, 2H), 7.82(d, J=7.9Hz, 2H), 7.89(d, J=8.3Hz, 2H) |
| 1-41 | MeOH-d4, 300MHz | 0.83(t, J=7.3Hz, 6H), 1.05-1.22(m, 4H), 1.40-1.64(m, 4H), 2.40-2.53(m, 1H), 2.61(s, 3H), 4.21(d, J=15.1Hz, 1H), 4.34(d, J=15.1Hz, 1 H), 4.66(s, 1 H), 5.08(s, 2H), 6.91(d, J=8.7Hz, 2H), 7.04(d, J=8.7Hz, 2H), 7.35-7.45(m, 3H), 7.46-7.59(m, 4H), 7.83(s, 1 H), 7.93(d, J=8.3Hz, 2H) |
| 1-42 | DMSO-d6, 300MHz | 0.79(t, J=7.4Hz, 6H), 0.99-1.17(m, 4H), 1.36-1.61(m, 4H), 2.40(s, 3H), 2.40-2.52(m, 1H), 2.95(dd, J=8.3, 13.9Hz, 1H), 3.08(dd, J=7.1, 13.9Hz, 1H), 3.73(t, J=7.5Hz, 1H), 4.05(d, J=15.8Hz, 1 H), 4.21 (d, J=15.8Hz, 1H), 5.07(s, 2H), 6.93(d, J=8.7Hz, 2H), 7.07(d, J=8.7Hz, 2H), 7.16-7.33(m, 5H), 7.49(d, J=8.3Hz, 2H), 7.81-8.05(m, 3H) |

**Table 17-9**

| Example | solvent, Hz | NMR(δ) |
|---|---|---|
| 1-43 | DMSO-d6, 300MHz | 1.34 (s, 9 H), 2.23 (s, 3 H), 3.37 (s, 2 H), 3.88 (s, 2 H), 4.20 (s, 2 H), 5.12 (s, 2 H), 6.96 (s, 2 H), 7.04 (s, 1 H), 7.22- 7.45 (m, 5 H), 7.53 (d, J = 8.4 Hz, 2 H), 7.97 (d, J = 8.1 Hz, 2 H), 8.05 (s, 1 H), 12.38 (br s, 1 H). |
| 1-44 | DMSO-d6, 300MHz | 0.79(t, J=7.3Hz, 6H), 0.95-1.18(m, 4H), 1.33-1.60(m, 4H), 2.40-2.54(m, 1H), 3.38(s, 2H), 3.88(s, 2H), 4.20(s, 2H), 5.08(s, 2H), 6.93(d, J=8.6Hz, 2H), 7.07(d, J=8.6Hz, 2H), 7.22-7.45(m, 5H), 7.50(d, J=8.2Hz, 2H), 7.96(d, J=8.2Hz, 2H), 8.05(s, 1H) |
| 1-45 | DMSO-d6, 300MHz | 0.80(t, J=7.3Hz, 6H), 0.99-1.18(m, 4H), 1.37-1.61 (m, 4H), 2.39-2.51(m, 1H), 3.10(dd, J=3.4, 16.2Hz, 1H), 3.19(dd, J=5.6, 15.8Hz, 1H), 3.88-4.03(m, 2H), 4.10(d, J=15.8Hz, 1H), 4.27(d, J=15.8Hz, 1 H), 4.42(d, J=15.8Hz, 1 H), 5.09(s, 2H), 6.94(d, J=8.6Hz, 2H), 7.01-7.20(m, 6H), 7.51(d, J=8.3Hz, 2H), 7.97(d, J=8.3Hz, 2H), 8.04(s, 1H), 12.56(brs, 1H) |
| 1-46 | DMSO-d6, 300MHz | 0.80(t, J=7.3Hz, 6H), 0.99-1.18(m, 4H), 1.37-1.61(m, 4H), 2.39-2.51(m, 1H), 3.10(dd, J=3.4, 16.2Hz, 1H), 3.19(dd, J=5.6, 15.8Hz, 1H), 3.88-4.03(m, 2H), 4.10(d, J=15.8Hz, 1H), 4.27(d, J=15.8Hz, 1 H), 4.42(d, J=15.8Hz, 1H), 5.09(s, 2H), 6.94(d, J=8.6Hz, 2H), 7.01-7.20(m, 6H), 7.51(d, J=8.3Hz, 2H), 7.97(d, J=8.3Hz, 2H), 8.04(s, 1H), 12.56(brs, 1H) |
| 1-47 | DMSO-d6, 300MHz | 0.80(t, J=7.4Hz, 6H), 0.98-1.17(m, 4H), 1.36-1.60(m, 4H), 2.39-2-53(m, 1H), 3.10(dd, J=2.6, 15.8Hz, 1H), 3.19(dd, J=5.6, 16.2Hz, 1H), 3.88-4.02(m, 2H), 4.10(d, J=16.2Hz, 1 H), 4.27(d, J=15.5Hz, 2H), 4.42(d, J=15.8Hz, 2H), 5.08(s, 2H), 6.94(d, J=8.3Hz, 2H), 7.01-7.21(m, 6H), 7.51(d, J=7.9Hz, 2H), 7.97(d, J=7.9Hz, 2H), 8.05(s, 1H), 12.59(brs, 1H) |

**Table 17-10**

| Example | solvent, Hz | NMR(δ) |
|---|---|---|
| 1-48 | DMSO-d6, 300MHz | 0.80(t, J=7.2Hz, 6H), 1.02-1.15(m, 4H), 1.38-1.57(m, 4H), 2.41-2.48(m, 1H), 5.10.(s, 2H), 5.69(s, 2H), 6.94(d, J=8.6Hz, 2H), 7.08(d, J=8.7Hz, 2H), 7.53(d, J=8.3Hz, 2H), 7-99-8.02(m, 3H), 8.21 (s, 1H), 8.66(d, J=3.0Hz, 1H), 8.74(d, J=1.5Hz, 1H), 13.48(brs, 1H) |
| 1-49 | DMSO-d6, 300MHz | 0.80(t, J=7.3Hz, 6H), 1.02-1.15(m, 4H), 1.38-1.58(m, 4H), 2.41-2.50(m, 1H), 5.10(s, 2H), 5.60(s, 2H), 6.94(d, J=8.7Hz, 2H), 7.07(d, J=9.0Hz, 2H), 7.19(d, J=9.1Hz, 2H), 7.53(d, J=8.3Hz, 2H), 7.93(d, J=9.1 Hz, 2H), 8.00(d, J=8.3Hz, 2H), 8.19(s, 1H), 12.66(brs, 1H) |
| 1-50 | DMSO-d6, 300MHz | 0.80(t, J=7.2Hz, 6H), 1.02-1.17(m, 4H), 1.38-1.56(m, 4H), 2.41-2.49(m, 1H), 2.88(s, 3H), 4.70(s, 2H), 5.08(s, 2H), 6.94(d, J=8.8Hz, 2H), 7.08(d, J=8.8Hz, 2H), 7.49(d, J=8.4Hz, 2H), 7.87(d, J=8.4Hz, 2H), 7.98(d, J=8.8Hz, 2H), 8.10(s, 1H), 8.14(d, J=8.8Hz, 2H), 13.38(brs, 1H) |
| 1-51 | DMSO-d6, 300MHz | 0.80(t, J=7.3Hz, 6H), 1.02-1.15(m, 4H), 1.38-1.56(m, 4H), 1.88-1.98(m, 2H), 2.41 (t, J=7.3Hz, 2H), 2.45-2.49(m, 1H), 2.92(s, 3H), 3.25-3.28(m, 2H), 4.72(s, 2H), 5.09(s, 2H), 6.94(d, J=8.8Hz, 2H), 7.08(d, J=8.8Hz, 2H), 7.52(d, J=8.4Hz, 2H), 7.97(d, J=8.1Hz, 2H), 8.13(s, 1H), 12.17(brs, 1H) |
| 1-52 | DMSO-d6, 400MHz | 1.16-1.46(m, 5H), 1.67-1.85(m, 5H), 2.40-2.56(m, 1H), 3.62(s, 3H), 7.20(d, J=8.6Hz, 2H), 7.58(s, 1H), 7.68(d, J=8.6Hz, 2H), 7.71(d, J=9.2Hz, 2H), 7.95-8.05(m, 6H), 10.14(s, 1 H), 12.75(brs, 1H) |

**Table 17-11**

| Example | solvent, Hz | NMR(δ) |
|---|---|---|
| 1-53 | DMSO-d6, 400MHz | 1.17-1.45(m, 5H), 1.67-1.83(m, 5H), 2.43-2.52(m, 1H), 3.13(s, 3H), 4.86(s, 2H), 7.19(d, J=8.6Hz, 2H), 7.39(s, 1H), 7.46(d, J=8.6Hz, 2H), 7.67(d, J=8.6Hz, 2H), 7.94(d, J=8.6Hz, 2H), 7.96(d, J=9.2Hz, 2H), 7.99(d, J=9.2Hz, 2H), 10.11(s, 1H), 12.69(brs, 1H) |
| 1-54 | DMSO-d6, 300MHz | 0.82(t, J=7.2Hz, 6H), 1.01-1.21(m, 4H), 1.41-1.63(m, 4H), 3.39(s, 2H), 3.89(s, 2H), 4.22(s, 2H), 7.14(d, J=8.6Hz, 2H), 7.24-7.46(m, 5H), 7.68(d, J=8.3Hz, 2H), 8.02(d, J=8.7Hz, 2H), 8.09(d, J=8.7Hz, 2H), 8.23(s, 1H), 10.19(s, 1H), 12.42(brs, 1H) |
| 1-55 | DMSO-d6, 300MHz | 0.79(t, J = 6.0Hz, 6H), 1.04-1.12(m,4H), 1.45-1.56(m,4H), 2.43-2.48(m,1H), 3.38(s,2H), 3.88(s,2H), 4.21(s,2H), 4.47(d,J = 6.0Hz, 2H), 7.12(d,J = 9.0Hz, 2H), 7.23-7.45(m,8H), 7.97(d,J = 9.0Hz, 2H), 8.04(d,J = 9.0Hz, 2H), 8.19(s,1H), 9.03(t, J = 6.0Hz, 1H) |
| 1-56 | DMSO-d6, 300MHz | 0.80(t, J = 6.0Hz, 6H), 1.06-1.18(m,4H), 1.47-1.54(m,4H), 2.88(s,3H), 3.37(s,2H), 3.88(s,2H), 4.20(s,2H), 4.50-4.64(m,2H), 7.17(d,J = 9.0Hz, 2H), 7.25-7.42(m,10H), 7.51(d,J = 6.0Hz, 2H), 8.00(d,J = 6.0Hz, 2H), 8.14(s,1H) |
| 1-57 | DMSO-d6, 300MHz | 0.80(t, J = 6.0Hz, 6H), 0.93-1.24(m,10H), 1.40-1.67(m,4H), 3.37(s,2H), 3.88(s,2H), 4.20(s,2H), 4.48-4.65(m,1H), 7.15-7.49(m, 12H), 8.00(d,J = 6.0Hz, 2H), 8.13(s,1H) |
| 1-58 | DMSO-d6, 300MHz | 0.77(t, J=7.3Hz, 6H), 1.00-1.10(m, 4H), 1.19(s, 6H), 1.33-1.48(m, 4H), 2.27-2.37(m, 1H), 3.40(s, 2H), 4.45(brs, 1H), 4.72(s, 2H), 5.66(s, 2H), 6.66(d, J=8.6Hz, 2H), 6-84(d, J=8.8Hz, 2H), 7.27(d, J=8.0Hz, 2H), 7.87(d, J=8.3Hz, 2H), 7.98-7.99(m, 1H), 8.10(s, 1H), 8.64(d, J=2.9Hz, 1H), 8.73(d, J=1.4Hz, 1H) |

**Table 17-12**

| Example | solvent, Hz | NMR(δ) |
|---|---|---|
| 1-59 | DMSO-d6, 400MHz | 0.92 (d, J = 6.5 Hz, 6 H), 1.19 (s, 9 H), 2.05 (sept, J = 6.7 Hz, 1 H), 3.19 (t, J = 4.5 Hz, 4 H), 3.24 (d, J = 7.2 Hz, 2 H), 3.57 (t, J = 4.5 Hz, 4 H), 3.92 (s, 2 H), 4.58 (s, 2 H), 4.68 (s, 2 H), 6.58 (d, J = 8.8 Hz, 2 H), 7.10 (d, J = 9.0 Hz, 2 H), 7.24 (d, J = 8.4 Hz, 2 H), 7.84 (d, J = 8.1 Hz, 2 H), 7.95 (s, 1 H), 12.69 (br s, 1 H). |
| 1-60 | DMSO-d6, 300MHz | 0.69 (t, J = 7.4 Hz, 6 H), 1.16 (d, J = 6.8 Hz, 6 H), 1.31-1.48 (m, 2 H), 1.48- 1.64 (m, 2 H), 2.06- 2.19 (m, 1 H), 4.23 (sept, J = 6.6 Hz, 1 H), 4.38 (s, 2 H), 5.56 (s, 2 H), 6.62 (d, J = 8.7 Hz, 2 H), 6.89 (d, J = 8.6 Hz, 2 H), 7.36 (d, J = 8.3 Hz, 2 H), 7.80 (dd, J = 3.0, 1.5 Hz, 1 H), 7.90 (d, J = 8.3 Hz, 2 H), 8.08 (s, 1 H), 8.31 (d, J = 3.0 Hz, 1 H), 8.61 (d, J = 1.1 Hz, 1 H). |
| 1-61 | DMSO-d6, 300MHz | 0.79(t, J = 9.0Hz, 6H), 1.02-1.11(m,4H), 1.40-1.56(m,4H), 2.40-2.51(m,1H), 2.95-3.00(m,1H), 3.14-3.22(m,1H), 3.31-3.36(m,2H), 3.69(d,J = 15.0Hz, 1H), 4.28-4.42(m,3H), 5.07(s,2H), 6.91-7.08(m,8H), 7.49(d,J = 9.0Hz, 2H), 7.96(d,J = 9.0Hz, 2H), 7.97(s,1H) |
| 1-62 | DMSO-d6, 300MHz | 0.80 (t, J = 7.2 Hz, 6 H), 1.00- 1.17 (m, 4 H), 1.35- 1.62 (m, 4 H), 2.40- 2.53 (m, 1 H), 5.09 (s, 2 H), 5.58 (s, 2 H), 6.94 (d, J = 8.6 Hz, 2 H), 7.08 (d, J = 9.0 Hz, 2 H), 7.53 (d, J = 8.3 Hz, 2 H), 7.80 (dd, J = 3.0, 1.5 Hz, 1 H), 8.00 (d, J = 8.3 Hz, 2 H), 8.18 (s, 1 H), 8.32 (d, J = 3.0 Hz, 1 H), 8.61 (d, J = 1.1 Hz, 1 H). |
| 1-63 | DMSO-d6, 400MHz | 0.68(t, J = 7.3 Hz, 6H), 1.16(d, J = 6.5 Hz, 6H), 1.34-1.45(m, 2H), 1.50-1.61(m, 2H), 2.09-2.16(m, 1H), 3.79 (s, 3H), 4.23(sep, J = 6.5 Hz, 1H), 4.37(s, 2H), 5.54 (s, 2H), 6.62(d, J = 8.6 Hz, 2H), 6.88(d, J = 8.6 Hz, 2H), 6.92(t, J = 2.4 Hz, 1 H), 7.10(dd, J = 2.4, 1.2 Hz, 1 H), 7.22(dd, J = 2.4, 1.2 Hz, 1H), 7.35 (d, J = 8.6 Hz, 2H), 7.89 (d, J = 8.6 Hz, 2H), 8.08(s, 1H), 13.03(brs, 1H) |

**Table 17-13**

| Example | solvent, Hz | NMR(δ) |
|---|---|---|
| 1-64 | DMSO-d6, 300MHz | 0.68(t, J = 7.3 Hz, 6H), 1.16(d, J = 6.6 Hz, 6H), 1.32-1.47(m, 2H), 1.49-1.63(m, 2H), 2.08-2.17(m. 1 H),4.05(s, 2H), 4.22(sep, J = 6.6 Hz, 1H), 4.37(s, 2H),4.87(s, 2H), 6-62(d, J = 8.4 Hz, 2H), 6.89(d, J = 8.4 Hz, 2H), 6.93(t,J = 7.3 Hz, 1H), 6.94(t, J = 7.3 Hz, 2H), 7.34(d,J = 8.4 Hz, 2H),7.44(d, J = 7.3 Hz, 2H), 7.87 (d, J = 8.4 Hz, 2H), 7.94(s, 1H), 9.49(brs, 1H) |
| 1-65 | DMSO-d6, 300MHz | 0.68(t, J = 7.3 Hz, 6H), 1.16(d, J = 6.6 Hz, 6H), 1.32-1.47(m, 2H), 1.49-1.63(m, 2H), 2.08-2.18(m, 1H), 2.22(s, 3H), 4.19-4.28(m, 3H), 4.37(s, 2H), 4.89(s, 2H), 6.62(d, J = 8.4 Hz, 2H), 6.89(d, J = 8.4 Hz, 2H), 7.04(d, J = 8.4 Hz, 2H), 7.33(d, J = 8.4 Hz, 2H), 7.34(d, J = 8.4 Hz, 2H), 7.87(d, J = 8.4 Hz, 2H), 7.95(s, 1H), 8.80(brs, 1H), 12.70(brs, 1H) |
| 1-66 | DMSO-d6, 300MHz | 0.68(t, J = 7.3 Hz, 6H), 1.16(d, J = 7.0 Hz, 12H), 1.33-1.47(m, 2H), 1.49-1.63(m, 2H), 2.06-2.17(m, 1H), 2.80(sep, J = 7.0 Hz, 1H), 4.04(s, 2H), 4.22(sep, J = 7.0 Hz, 1H), 4.37(s, 2H), 4.86(s, 2H), 6.62(d, J = 8.4 Hz, 2H), 6.88(d, J = 8.4 Hz, 2H), 7.09(d, J = 8.4 Hz, 2H), 7.34(d, J = 8.4 Hz, 4H), 7.87(d, J = 8.4 Hz, 2H), 7.93(s, 1H), 9.32(brs, 1H) |
| 1-67 | DMSO-d6, 300MHz | 0.68(t, J = 7.3 Hz, 6H), 1.16(d, J = 6.6 Hz, 6H), 1.32-1.47(m, 2H), 1.49-1.63(m, 2H), 2.08-2.18(m, 1 H), 3.08(s, 3H), 3.81(s, 2H), 4.22(sep, J = 6.6 Hz, 1H), 4.37(s, 2H), 4.63(s, 2H), 6.62 (d, J = 8.8 Hz, 2H), 6.88 (d, J = 8.8Hz, 2H), 7.10-7.15(m, 1H), 7.26-7.36(m, 6H), 7.84(d, J = 8.1 Hz, 2H), 7.93(s, 1H), 12.42(brs, 1H) |
| 1-68 | DMSO-d6, 400MHz | 0.77(t, J = 7.3 Hz, 6H), 1.02-1.11(m, 4H), 1.16(d, J = 6.5Hz, 6H), 1.33-1.51(m, 4H), 2.29-2.37(m, 1H), 4.22(sep,J = 6.5 Hz,1H), 4.36(s, 2H), 5.66 (s, 2H), 6.59(d, J = 8.8 Hz, 2H), 6.87(d, J = 8.8 Hz, 2H), 7.35(d, J = 8.4 Hz, 2H), 7.89(d, J = 8.4 Hz, 4H), 7.98(dd, J = 3.0, 1.6Hz, 1H), 8.10(s, 1H),8.63(d, J = 3.0 Hz, 1H), 8.71(d, J = 1.6 Hz, 1H), 13.44(brs, 1H) |

**Table 17-14**

| Example | solvent, Hz | NMR(δ) |
|---|---|---|
| 1-69 | DMSO-d6, 300MHz | 0.80(t, J=7.0Hz, 6H), 0.98-1.18(m, 4H), 1.36-1.60(m, 4H), 2.39-2.54(m, 1 H), 4.18(s, 2H), 4.91 (s, 2H), 5.09(s, 2H), 6.94(d, J=8.7Hz, 2H), 6.95(t, J=8.6Hz, 2H), 7.07(d, J=8.7Hz, 2H), 7.24(t, J=7.8Hz, 2H), 7.46(d, J=7.5Hz, 2H), 7.51(d, J=8.1 Hz, 2H), 7.97(d, J=8.1 Hz, 2H), 8.05(s, 1H), 9.11(brs, 1H) |
| 1-70 | DMSO-d6, 400MHz | 0.68(t, J = 7.3 Hz, 6H), 1.16(d, J = 6.6 Hz, 6H), 1.33-1.47(m, 2H), 1.49-1.60(m, 2H), 2.08-2.17(m, 7H),4.18-4.25(m, 3H), 4.37(s, 2H), 4.90 (s, 2H), 6.62(d, J = 8.8 Hz, 2H), 6.88(d, J = 8.8 Hz, 2H), 7.02(s, 2H), 7.34(d, J = 8.4 Hz, 2H), 7.86(d, J = 8.4 Hz, 2H), 7.96(s, 1H), 8.16(brs, 1H), 12.70(brs, 1H) |
| 1-71 | DMSO-d6, 400MHz | 0.68(t, J = 7.3 Hz, 6H), 1.16(d, J = 6.6 Hz, 6H), 1.33-1.47(m, 2H), 1.49-1.63(m, 2H), 2.08-2.18(m, 1H), 3.44(brs, 2H), 3.56(brs, 2H), 4.21(sep, J = 6.6 Hz,1H), 4.22(s, 2H), 4.36(s, 2H), 6.61(d, J = 8.8 Hz, 2H), 6.88 (d, J = 8.8 Hz, 2H), 7.01-7.04(m, 1H), 7.23-7.31(m, 4H), 7.61(d, J = 8.4 Hz, 2H), 7.84(d, J = 8.4 Hz, 2H), 7.94(s,1H), 10.94(brs,1H) |
| 1-72 | DMSO-d6, 300MHz 300MHz | 0.68(t, J=7.2Hz, 6H), 1.16(d, J=6.3Hz, 6H), 1.33(d, J=6.6Hz, 6H), 1.37-1.65(m, 4H), 2.06-2.21(m, 1H), 3.50-3.70(m, 1H), 4.06(s, 2H), 4.14-4.29(m, 1H), 4.37(s, 2H), 4.84(s, 2H), 6.62(d, J=8.4Hz, 2H), 6.88(d, J=8.4Hz, 2H), 7.35(d, J=7.8Hz, 2H), 7.86(d, J=8.1Hz, 2H), 8.01 (s, 1H), 12.22(brs, 1H) |
| 1-73 | DMSO-d6, 400MHz | 14.43(brs, 1H), 8.12(s, 1H), 7.88(d, J=8.4Hz, 2H), 7.35(d, J=8.4Hz, 2H), 7.07(s, 1H), 6.87(d, J=8.8Hz, 2H), 6.60(d, J=8.8Hz, 2H), 5.66(s, 2H), 4.37(s, 2H), 4.17-4.27(m, 1 H), 2.27-2.39(m, 1H), 1.32-1.52(m, 4H), 1.16(d, J=6.4Hz, 6H), 0.99-1.12(m, 4H), 0.78(t, J=7.2Hz, 6H) |

**Table 17-15**

| Example | solvent, Hz | NMR(δ) |
|---|---|---|
| 1-74 | DMSO-d6, 300MHz | 0.86 (t, J = 7.5 Hz, 6 H), 1.12 (d, J = 6.8 Hz, 6 H), 1.22-1.45 (m, 4 H), 1.70 (sept, J = 6.0 Hz, 1 H), 2.71 (sept, J = 6.8 Hz, 1 H), 3.33 (s, 2 H), 4.57 (s, 2 H), 5.66 (s, 2 H), 6.97 (d, J = 8.7 Hz, 2 H), 7.26 (d, J = 8.3 Hz, 2 H), 7.88 (d, J = 8.3 Hz, 2 H), 7.98 (dd, J = 3.0, 1.5 Hz, 1 H), 8.11 (s, 1 H), 8.64 (d, J = 3.0 Hz, 1 H), 8.72 (d, J = 1.9 Hz, 1 H), 13.44 (br s, 1 H). |
| 1-75 | DMSO-d6, 400MHz | 0.68(t, J=7.4Hz, 6H), 1.15(d, J=6.4Hz, 6H), 1.32-1.66(m, 10H), 2.06-2.16(m, 1H), 2.92-2.98(m, 2H), 3.07-3.18(m, 2H), 3.31-3.48(m, 1H), 3.72(s, 1H), 4.01-4.09(m, 1H), 4.16-4.27(m, 1H), 4.36(s, 2H), 4.62(s, 1 H), 6.60(d, J=8.8Hz, 2H), 6.87(d, J=8.4Hz, 2H), 7.32(d, J=7.6Hz, 2H), 7.84(d, J=8.0Hz, 2H), 7.91(s, 1H) |
| 1-76 | DMSO-d6, 300MHz | 0.80 (t, J = 7.3 Hz, 6 H), 0.99- 1.18 (m, 4 H), 1.35- 1.61 (m, 4 H), 2.40- 2.55 (m, 1 H), 5.09. (s, 2 H), 5.79 (s, 2 H), 6.94 (d, J = 8.7 Hz, 2 H), 7.07 (d, J = 8.7 Hz, 2 H), 7.18 (dd, J = 7.4, 5.1 Hz, 1 H), 7.52 (d, J = 8.3 Hz, 2 H), 7.99 (d, J = 8.3 Hz, 2 H), 8.20 (dd, J = 7.3, 2.1 Hz, 1 H), 8.40 (dd, J = 4.9, 1.9 Hz, 1 H), 13.08 (br s, 1 H). |
| 1-77 | DMSO-d6, 400MHz | 0.68 (t, J = 7.4 Hz, 6H), 1.15(d, J = 6.6 Hz, 6H), 1.34-1.45(m, 2H), 1.49-1.63(m, 2H), 2.08-2.15(m, 1H), 2.22(s,3H), 3.41(brs, 2H), 3.53(brs, 2H), 4.18-4.23(m, 3H), 4.35(s, 2H), 6.60(d, J = 8.8 Hz, 2H), 6.87(d, J = 8.8 Hz, 2H), 7.06(d, J = 8.4 Hz, 2H), 7.32(d, J = 8.4 Hz, 2H), 7.51(d, J = 8.4 Hz, 2H), 7.84 (d, J = 8.4 Hz, 2H), 7.93(s, 1H), 10.91(brs, 1H) |

**Table 17-16**

| Example | solvent, Hz | NMR(δ) |
|---|---|---|
| 1-78 | DMSO-d6, 400MHz | 0.68 (t, J = 7.3 Hz, 6H), 1.15(d, J = 6.5 Hz, 6H), 1.16(d, J = 6.5 Hz, 6H), 1.34-1.45(m, 2H), 1.50-1.60 (m, 2H), 2.08-2.16(m, 1H), 2.82(sep, J = 6.5 Hz, 1H), 3.55(s, 2H), 3.61 (s, 2H), 4.22(sep, J = 6.5 Hz, 1H), 4.26(s, 2H), 4.36(s, 2H), 6.60(d, J = 8.8 Hz, 2H), 6.87(d, J = 8.8 Hz, 2H), 7.14(d, J = 8.3 Hz, 2H), 7.31(d, J = 8.3 Hz, 2H), 7.49(d, J = 8.3Hz, 2H), 7.84(d, J = 8.3 Hz, 2H), 7.95(s, 1 H), 10.01(brs, 1H), 12.67(brs, 1H) |
| 1-79 | DMSO-d6, 300MHz | 0.77(t, J = 7.3 Hz, 6H), 1.01-1.10(m,4H), 1.15(d, J = 6.6 Hz, 6H), 1.34-1.50(m, 4H), 2.28-2.38(m, 1H), 3.08(s, 3H), 3.67(s, 2H), 4.22(sep, J = 6.6 Hz, 1H), 4.36(s, 2H), 4.66(s, 2H), 6.61(d, J = 8.4 Hz, 2H), 6.88(d, J = 8.4 Hz, 2H), 7.06-7.11(m, 1H), 7.25-7.35(m, 6H), 7.84(d, J = 8.1 Hz, 2H), 7.92(s, 1H) |
| 1-80 | DMSO-d6, 300MHz | 0.78(t, J = 7.3 Hz, 6H), 1.01-1.11(m,4H), 1.16(d, J = 6.6 Hz, 6H), 1.35-1.48(m, 4H), 2.23(s, 3H), 2.31-2.39(m, 1H), 4.19-4.26(m, 3H), 4.37(s, 2H), 4.89(s, 2H), 6.61(d, J = 8.4 Hz, 2H), 6.89(d, J = 8.4 Hz, 2H), 7.05(d, J = 8.4 Hz, 2H), 7.33(d, J = 8.4 Hz, 2H), 7.34(d, J = 8.4 Hz, 2H), 7.87(d, J = 8.1 Hz, 2H), 7.96(s, 1H), 8.76(brs, 1H), 12.71(brs, 1H) |
| 1-81 | DMSO-d6, 400MHz | 0.77(t, J=7.4Hz, 6H), 0.99-1.11(m, 4H), 1.15(d, J=6.4Hz, 6H), 1.31-1.52(m, 4H), 2.27-2.37(m, 1H), 3.01(t, J=8.4Hz, 2H), 3.86(t, J=8.2Hz, 2H), 4.02(s, 2H), 4.16-4.26(m, 1H), 4.36(s, 2H), 4.82(s, 2H), 6.59(d, J=8.8Hz, 2H), 6.87(d, J=8.8Hz, 2H), 6.83-6.92(m, 1H), 7.11(t, J=8.2Hz, 1H), 7.19(d, J=7.6Hz, 1H), 7.25(d, J=8.0Hz, 1H), 7.33(d, J=8.4Hz, 2H), 7.86(d, J=8.4Hz, 2H), 7.97(s, 1H), 13.03(brs, 1H) |
| 1-82 | DMSO-d6, 300MHz | 0.80 (t, J = 7.3 Hz, 6 H), 0.99- 1.19 (m, 4 H), 1.37- 1.60 (m, 4 H), 2.40- 2.53 (m, 1 H), 5.09 (s, 2 H), 5.63 (s, 2 H), 6.94 (d, J = 8.7 Hz, 2 H), 7.08 (d, J = 8.7 Hz, 2 H), 7.53 (d, J = 8.3 Hz, 2 H), 7.54 (dd, J = 8.5, 4.7 Hz, 1 H), 7.80 (dd, J = 8.7, 1.1 Hz, 1 H), 7.99 (d, J = 8.3 Hz, 2 H), 8.19 (s, 1 H), 8.23 (dd, J = 4.5, 1.1 Hz, 1 H), 13.22 (br s, 1 H). |

**Table 17-17**

| Example | solvent, Hz | NMR(δ) |
|---|---|---|
| 1-83 | DMSO-d6, 300MHz | 0.86 (t, J = 7.3 Hz, 6 H), 1.12 (d, J = 6.8 Hz, 6 H), 1.23-1.44 (m, 4 H), 1.70 (sept, J = 6.4 Hz, 1 H), 2.71 (sept, J = 7.0 Hz, 1 H), 3.30 (d, J = 7.0 Hz, 2 H), 3.52- 3.69 (m, 4 H), 4.28 (s, 2 H), 4.53 (s, 2 H), 6.59 (d, J = 8.7 Hz, 2 H), 6.97 (d, J = 8.7 Hz, 2 H), 7.04 (t, J = 7.4 Hz, 1 H), 7.22 (d, J = 8.3 Hz, 2 H), 7.29 (t, J = 7.9 Hz, 2 H), 7.59 (d, J = 7.5 Hz, 2 H), 7.84 (d, J = 7.9 Hz, 2 H), 7.96 (s, 1 H), 10.05 (br s, 1 H), 12.54 (br s, 1 H). |
| 1-84 | DMSO-d6, 300MHz | 0.86 (t, J = 7.4 Hz, 6 H), 1.12 (d, J = 6.8 Hz, 6 H), 1.17 (d, J = 6.8 Hz, 6 H), 1.26- 1.44 (m, 4 H), 1.63- 1.75 (m, 1 H), 2.71 (sept, J = 7.0 Hz, 1 H), 2.83 (sept, J = 7.0 Hz, 1 H), 3.30 (d, J = 7.0 Hz, 2 H), 3.50- 3.70 (m, 4 H), 4.27 (s, 2 H), 4.55 (s, 2 H), 6.59 (d, J = 8.7 Hz, 2 H), 6.97 (d, J = 8.6 Hz, 2 H), 7.15 (d, J = 8.3 Hz, 2 H), 7.22 (d, J = 8.3 Hz, 2 H), 7.49 (d, J = 8.7 Hz, 2 H), 7.84 (d, J = 7.9 Hz, 2 H), 7.97 (s, 1 H), 9.93 (br s, 1 H), 12.55 (br s, 1 H). |
| 1-85 | DMSO-d6, 300MHz | 0.86 (t, J = 7.4 Hz, 6 H), 1.12 (d, J = 6.8 Hz, 6 H), 1.22-1.45 (m, 4 H), 1.69 (sept, J = 6.6 Hz, 1 H), 2.71 (sept, J = 7.0 Hz, 1 H), 3.30 (d, J = 6.8 Hz, 2 H), 3.60- 3.69 (m, 4 H), 4.28 (s, 2 H), 6.59 (d, J = 8.7 Hz, 2 H), 6.97. (d, J = 8.6 Hz, 2 H), 7.22 (d, J = 8.3 Hz, 2 H), 7.70 (d, J = 9.0 Hz, 2 H), 7.83 (d, J = 8.3 Hz, 2 H), 7.88 (d, J = 8.7 Hz, 2 H), 7.95 (s, 1 H), 10.33 (s, 1 H), 12.66 (br s, 2 H). |
| 1-86 | DMSO-d6, 300MHz | 0.80 (t, J = 7.3 Hz. 6 H), 0.98- 1.18 (m, 4 H), 1.36- 1.61 (m, 4 H), 2.40- 2.53 (m, 1 H), 5.09 (s, 2 H), 5.81 (s, 2 H), 6.94 (d, J = 8.7 Hz, 2 H), 7.07 (d, J = 8.7 Hz, 2 H), 7.22 (d, J = 7.5 Hz, 1 H), 7.52 (d, J = 8.3 Hz, 2 H), 7.75 (d, J = 7.5 Hz, 1 H), 7.96 (dd, J = 8.1, 7.3 Hz, 1 H), 7.99 (d, J 8.3 Hz, 2 H), 8.15 (s, 1 H), 13.17 (br s, 1 H). |

**Table 17-18**

| Example | solvent, Hz | NMR(δ) |
|---|---|---|
| 1-87 | DMSO-d6, 300MHz | 0.78(t, J=7.2Hz, 6H), 0.92(d, J=6.6Hz, 6H), 1.01-1.14(m, 4H), 1.33-1.56(m, 4H), 1.98-2.10(m, 1H), 2.27-2.40(m, 1H), 3.24(d, J=7.2Hz, 2H), 4.58(s, 2H), 5.55(s, 2H), 6.60(d, J=8.7Hz, 2H), 6.89(d, J=8.4Hz, 2H), 7.27(d, J=8.4Hz, 2H), 7.78(dd, J=3.0, 1.5Hz, 1H), 7.89(d, J=8.1 Hz, 2H), 8.09(s, 1 H), 8.30(d, J=3.3Hz, 1 H), 8.60(d, J=0.9Hz, 1H) |
| 1-88 | DMSO-d6, 300MHz | 0.78(t, J=7.2Hz, 6H), 0.92(d, J=6.9Hz, 6H), 1.00-1.16(m, 4H), 1.32-1.50(m, 4H), 1.97-2.11 (m, 1H), 2.25-2.39(m, 1H), 3.23(d, J=7.2Hz, 2H), 3.97(brs, 2H), 4.57(s, 2H), 4.84(s, 2H), 6.59(d, J=9.3Hz, 2H), 6.86-6.97(m, 3H), 7.20-7.28(m, 4H), 7.42(d, J=8.4Hz, 2H), 7.86(d, J=7.8Hz, 2H), 7.95(s, 1H) |
| 1-89 | DMSO-d6, 300MHz | 0.68(t, J=7.3Hz, 6H), 1.16(d, J=6.8Hz, 6H), 1.35-1.63(m, 4H), 2.08-2.18(m, 1H), 3.89(s, 3H), 4.19-4.28(m, 1H), 4.38(s, 2H), 5.68(s, 2H), 6.62(d, J=9.0Hz, 2H), 6.88(d, J=8.7Hz, 2H), 7.36(d, J=8.3Hz, 2H), 7.90(d, J=8.3Hz, 2H), 8.02-8.04(m, 1 H), 8.11 (s, 1 H), 8.68(d, J=2.6Hz, 1 H), 8.74(d, J=1.9Hz, 1H) |
| 1-90 | DMSO-d6, 300MHz | 0.69 (t, J = 7.3 Hz, 6 H),1.16 (d, J = 6.5 Hz, 6 H),1.25-1.65 (m, 4 H),2.05- 2.20 (m, 1 H),4.15- 4.30 (m, 1 H),4.38 (s, 2 H),5.49 (s, 2 H),5.62 (s, 2 H),6.62 (d, J = 8.7 Hz, 2 H),6.68 (d, J = 8.2 Hz, 1 H),6.89 (d, J = 8.7 Hz, 2 H),7.35- 7.45 (m, 3 H),7.51 (br s, 1 H),7.90 (d, J = 8.2 Hz, 2 H),8.08 (s, 1 H). |
| 1-91 | DMSO-d6, 300MHz | 0.69 (t, J = 7.3 Hz, 6 H),1.16 (d, J = 6.6 Hz, 6 H),1.30-1.65 (m, 4 H),2.05- 2.20 (m, 1 H),4.15- 4.30 (m, 1 H),4.38 (s, 2 H),5.56 (s, 2 H),6.62 (d, J = 8.3 Hz, 2 H),6.68 (d, J = 8.3 Hz, 2 H),7.30- 7.40 (m, 3 H),7.45 (t, J = 7.7 Hz, 1 H),7.58 (d, J = 7.7 Hz, 1 H),7.62 (br s, 1 H),7.90 (d, J = 8.7 Hz, 2 H),8.08 (s, 1 H). |

**Table 17-19**

| Example | solvent, Hz | NMR(δ) |
|---|---|---|
| 1-92 | DMSO-d6, 300MHz | 0.69 (t, J = 7.3 Hz, 6 H),1.16 (d, J = 6.6 Hz, 6 H),1.30-1.65 (m, 4 H),2.05- 2.20 (m, 1 H),4.15- 4.30 (m, 1 H),4.37 (s, 2 H),4.65 (d, J = 6.0 Hz, 2 H),6.62 (d, J = 8.7 Hz, 2 H),6.70- 7.00 (m, 4 H),7.20- 7.30 (m, 3 H),7.34 (d, J = 8.4 Hz, 2 H),7.80- 7.90 (m, 3 H),12.66 (br s, 1 H). |
| 1-93 | DMSO-d6, 400MHz | 0.68 (t, J = 7.3 Hz, 6 H),1.16 (d, J = 6.5 Hz, 6 H),1.35-1.65 (m, 4 H),2.10- 2.20 (m, 1 H),4.20- 4.30 (m, 1 H),4.37 (s, 2 H),5.78 (s, 2 H),6.61 (d, J = 8.8 Hz, 2 H),6.88 (d, J = 8.8 Hz, 2 H),7.35 (d, J = 8.4 Hz, 2 H),7.69 (dd, J = 8.3, 1.5 Hz, 1 H),7.89 (d, J = 8.4 Hz, 2 H),8.01 (d, J = 8.3 Hz, 1 H),8.05 (d, J = 1.5 Hz, 1 H),8.10 (s, 1 H). |
| 1-94 | DMSO-d6, 300MHz | 0.77(t, J=7.3Hz, 6H), 0.98-1.21(m, 10H), 1.31-1.54(m, 4H), 2.25-2.41(m, 1H), 3.61(s, 2H), 4.14-4.43(m, 7H), 6.60(d, J=8.7Hz, 2H), 6.89(d, J=8.7Hz, 2H), 7.17-7.26(m, 2H), 7.32(d, J=8.3Hz, 2H), 7.52-7.61(m, 2H), 7.83(d, J=8.3Hz, 2H), 7.94(s, 1H) |
| 1-95 | DMSO-d6, 300MHz | 0.79 (t, J = 7.3 Hz, 6 H), 0.99- 1.18 (m, 4 H), 1.35- 1.61 (m, 4 H), 2.40- 2.54 (m, 1 H), 3.60 (s, 2 H), 3.66 (s, 2 H), 4.30 (s, 2 H), 5.08 (s, 2 H), 6.93 (d, J = 9.0 Hz, 2 H), 7.05 (t, J = 7.3 Hz, 1 H), 7.07 (d, J = 8.7 Hz, 2 H), 7.30 (t, J = 7.9 Hz, 2 H), 7.49 (d, J = 8.3 Hz, 2 H), 7.60 (d, J = 7.6 Hz, 2 H), 7.95 (d, J = 8.7 Hz, 2 H), 8.06 (s, 1 H), 10.01 (s, 1 H), 12.65 (br s, 1 H). |
| 1-96 | DMSO-d6, 300MHz | 0.80 (t, J = 7.3 Hz, 6 H), 0.96- 1.16 (m, 4 H), 1.36- 1.59 (m, 4 H), 2.38- 2.54 (m, 1 H), 3.66 (s, 2 H), 3.67 (s, 2 H), 4.31 (s, 2 H), 5.08 (s, 2 H), 6.93 (d, J = 8.7 Hz, 2 H), 7.07 (d, J = 8.7 Hz, 2 H), 7.34 (dd, J = 8.3, 4.5 Hz, 1 H), 7.49 (d, J = 8.3 Hz, 2 H), 7.95 (d, J = 8.3 Hz, 2 H), 8.05 (ddd, J = 8.5, 2.4, 1.5 Hz, 1 H), 8.05 (s, 1 H), 8.26 (dd, J = 4.9, 1.5 Hz, 1 H), 8.73 (d, J = 2.6 Hz, 1 H), 10.20 (s, 1 H), 12.63 (br s, 1 H). |

**Table 17-20**

| Example | solvent, Hz | NMR(δ) |
|---|---|---|
| 1-97 | DMSO-d6, 300MHz | 0.79 (t, J = 7.3 Hz, 6 H), 0.99- 1.17 (m, 4 H), 1.37- 1.60 (m, 4 H), 2.39- 2.52 (m, 1 H), 2.84 (s, 6 H), 3.54 (s, 2 H), 3.64 (s, 2 H), 4.28 (s, 2 H), 5.08 (s, 2 H), 6.69 (d, J = 9.1 Hz, 2 H), 6.93 (d, J = 8.7 Hz, 2 H), 7.07 (d, J = 8.6 Hz, 2 H), 7.41 (d, J = 9.0 Hz, 2 H), 7.49 (d, J = 8.3 Hz, 2 H), 7.95 (d, J = 8.3 Hz, 2 H), 8.06 (s, 1 H), 9.70 (s, 1 H), 12.63 (br s, 1 H). |
| 1-98 | DMSO-d6, 300MHz | 0.79 (t, J = 7.3 Hz, 6 H), 0.99- 1.16 (m, 4 H), 1.36- 1.60 (m, 4 H), 2.39- 2.52 (m, 1 H), 3.57 (s, 2 H), 3.65 (s, 2 H), 3.72 (s, 3 H), 4.29 (s, 2 H), 5.08 (s, 2 H), 6.88 (d, J = 9.0 Hz, 2 H), 6.93 (d, J = 8.6 Hz, 2 H), 7.07 (d, J = 8.6 Hz, 2 H), 7.49 (d, J = 8.3 Hz, 2 H), 7.51 (d, J = 9.1 Hz, 2 H), 7.95 (d, J = 8.3 Hz, 2 H), 8.06 (s, 1 H), 9.86 (s, 1 H), 12.63 (br s, 1 H). |
| 1-99 | DMSO-d6, 300MHz | 0.79 (t, J = 7.3 Hz, 6 H), 0.96 (d, J = 6.8 Hz, 6 H), 1.00-1.14 (m, 4 H), 1.36- 1.58 (m, 4 H), 2.38- 2.53 (m, 1 H), 3.16 (s, 2 H), 3.53 (s, 2 H), 4.17 (s, 2 H), 4.83 (t, J = 6.4 Hz, 1 H), 5.09 (s, 2 H), 6.94 (d, J = 8.7 Hz, 2 H), 7.07 (d, J = 8.7 Hz, 2 H), 7.10- 7.16 (m, 2 H), 7.33- 7.40 (m, 3 H), 7.51 (d, J = 8.3 Hz, 2 H), 7.93 (d, J = 8.3 Hz, 2 H), 8.01 (s, 1 H), 12.44 (br s, 1 H). |
| 1-100 | DMSO-d6, 300MHz | 0.79 (t, J = 7.3 Hz, 6 H), 0.98- 1.16 (m, 4 H), 1.36- 1.61 (m, 4 H), 2.40- 2.54 (m, 1 H), 3.64 (s, 4 H), 4.30 (s, 2 H), 5.08 (s, 2 H), 6.93 (d, J = 8.3 Hz, 2 H), 7.07 (d, J = 8.7 Hz, 2 H), 7.11 (ddd, J = 7.2, 4.8, 1.0 Hz, 1 H), 7.49 (d, J = 8.3 Hz, 2 H), 7.78 (ddd, J = 8.4, 7.2, 1.0 Hz, 1 H), 7.97 (d, J = 7.9 Hz, 2 H), 8.06 (s, 1 H), 8.09 (d, J = 8.3 Hz, 1 H), 8.31 (ddd, J = 4.9, 1.9, 0.7 Hz, 1 H), 10.22 (s, 1 H), 12.61 (br s, 1 H). |

**Table 17-21**

| Example | solvent, Hz | NMR(δ) |
|---|---|---|
| 1-101 | DMSO-d6, 300MHz | 0.79 (t, J = 7.2 Hz, 6 H), 0.99- 1.17 (m, 4 H), 1.35- 1.60 (m, 4 H), 1.71- 1.93 (m, 4 H), 2.40- 2.54 (m, 1 H), 3.11-3.51 (m, 6 H), 3.78 (br s, 2 H), 4.28 (br s, 2 H), 5.09 (s, 2 H), 6.93 (d, J = 8.7 Hz, 2 H), 7.07 (d, J = 8.7 Hz, 2 H), 7.53 (d, J = 7.9 Hz, 2 H), 8.01 (d, J = 7.9 Hz, 2 H), 8.19 (s, 1 H). |
| 1-102 1-102 | DMSO-d6, 400MHz | 0.79(t, J=7.4Hz, 6H), 0.99-1.14(m, 4H), 1.37-1.59(m, 4H), 2.32(s, 3H), 2.40-2.49(m, 1H), 3.50(s, 2H), 4.21(s, 2H), 4.28(s, 2H), 5.07(s, 2H), 6.92(d, J=8.8Hz, 2H), 7.06(d, J=8.4Hz, 2H), 7.20(brs, 1H), 7.49(d, J=8.4Hz, 2H), 7.94(d, J=8.4Hz, 2H), 8.06(s, 1H), 1.2.57(brs, 1H), |
| 1-103 | DMSO-d6, 300MH | 0.77(t, J = 7.3 Hz, 6H), 0.88-1.17(m,15H), 1.32-1.51(m, 4H), 1.56-1.72(m, 5H), 2.28-2.39(m, 1H), 3.38(d, J = 7.3 Hz, 2H), 4.22(sep, J = 6.6 Hz, 1H), 4.36(s, 2H), 4.90(s, 2H), 6.61(d, J = 8.8 Hz, 2H), 6.88(d, J = 8.8 Hz, 2H), 7.34(d, J = 8.4 Hz, 2H), 7.60(d, J = 8.4 Hz, 2H), 7.82-7.87 (m, 4H), 7.94(s, 1H), 8.88(brs, 1H), 12.54(brs, 1H) |
| 1-104 | DMSO(NaOD)-d6, 400MHz | 0.77(t, J = 7.3 Hz, 6H), 0.77(d, J = 6.7 Hz, 6H), 1.02-1.11 (m, 4H), 1.14(d, J = 6.5 Hz, 6H), 1.34-1.50(m, 4H), 1.94-2.01(m, 1H), 2.29-2.37(m, 1H), 3.31-3.38(m, 2H), 4.21 (sep, J = 6.5 Hz, 1 H), 4.36(s, 2H), 4.88(s, 2H), 6.59(d, J = 8.6 Hz, 2H), 6.87(d, J = 8.6 Hz, 2H); 7.33(d, J = 8.1 Hz, 2H), 7.38(d, J = 8.1 Hz, 2H), 7.75(d, J = 8.4 Hz, 2H), 7.86(d, J = 8.4 Hz, 2H), 7.93(s, 1H) |
| 1-105 | DMSO-d6, 300MHz | 0.78(t, J=7.1Hz, 6H), 0.92(d, J=6.3Hz, 6H), 1.00-1.13(m, 4H), 1.31-1.55(m, 4H), 1.96-2.10(m, 1H), 2.26-2.39(m, 1H), 3.08(s, 3H), 3.23(d, J=7.5Hz, 2H), 3.78(s, 2H), 4-57(s, 2H), 4.63(s, 2H), 6.59(d, J=9.0Hz, 2H), 6.88(d, J=8.7Hz, 2H), 7.09-7.14(m, 1H), 7.24-7.36(m, 6H), 7.83(d, J=8.1Hz, 2H), 7.93(s, 1H) |

**Table 17-22**

| Example | solvent, Hz | NMR(δ) |
|---|---|---|
| 1-106 | DMSO-d6, 400MHz | 0.78(t, J=7.4Hz, 6H), 0.92(d, J=6.4Hz, 6H), 1.02-1.12(m, 4H), 1.34-1.51(m, 4H), 2.00-2.07(m, 1H), 2.30-2.36(m, 1H), 3.22(d, J=6.4Hz, 2H), 3.42(brs, 2H), 3.51(s, 2H), 4.22(s, 2H), 4.56(s, 2H), 6.57(d, J=8.4Hz, 2H), 6.87(d, J=8.4Hz, 2H), 6.99-7.04(m, 1H), 7.22(d, J=8.0Hz, 2H), 7.26(dd, J=8.8, 7.2Hz, 2H), 7.65(d, J=8.8Hz, 2H), 7.82(d, J=8.8Hz, 2H), 7.93(s, 1H) |
| 1-107 | DMSO-d6, 400MHz | 0.78(t, J=7.2Hz, 6H), 0.92(d, J=6.4Hz, 6H), 1.01-1.12(m, 4H), 1.17(d, J=6.8Hz, 6H), 1.33-1.51(m, 4H), 1.98-2.09(m, 1H), 2.28-2.37(m, 1H), 2.82(q, J=9.2Hz, 1 H), 3.22(d, J=7.6Hz, 2H), 3.54(s, 2H), 3.59(s, 2H), 4.25(s, 2H), 4.56(s, 2H), 6.57(d, J=8.8Hz, 2H), 6.87(d, J=8.4Hz, 2H), 7.14(d, J=8.4Hz, 2H), 7.22(d, J=8.0Hz, 2H), 7.49(d, J=8.8Hz, 2H), 7.83(d, J=8.4Hz, 2H), 7.95(s, 1H) |
| 1-108 | DMSO-d6, 300MHz | 0.78 (t, J = 7.4 Hz, 6 H),0.98- 1.14 (m, 4 H),1.16 (d, J = 6.4 Hz, 6 H),1.30- 1.55 (m, 4 H),2.15 (s, 3 H),2.25- 2.40 (m, 1 H),4.15- 4.30 (m, 1 H),4.37 (s, 2 H),5.64 (s, 2 H),6.61 (d, J = 8.7 Hz, 2 H),6.89 (d, J = 8.7 Hz, 2 H),7.36 (d, J = 8.1 Hz, 2 H),7.57 (dd, J = 8.6, 1.9 Hz, 1 H),7.78 (d, J = 1.9 Hz, 1 H),7.91 (d, J = 8.1 Hz, 2 H),8.09 (s, 1 H),8.14 (d, J = 8.6 Hz, 1 H),9.43 (s, 1 H),12.72 (br s, 1 H). |
| 1-109 | DMSO-d6 300MHz | 0.78 (t, J = 7.3 Hz, 6 H),1.00- 1.15 (m, 4 H),1.16 (d, J = 6.4 Hz, 6 H),1.30- 1.53 (m, 4 H),2.30 (s, 6 H),2.25- 2.40 (m, 1 H),3.14 (s, 2 H),4.15- 4.30 (m, 1 H),4.37 (s, 2 H),5.67 (s, 2 H),6.61 (d, J = 8.7 Hz, 2 H),6.88 (d, J = 8.7 Hz, 2 H),7.36 (d, J = 8.5 Hz, 2 H),7.65 (dd, J = 8.5, 1.5 Hz, 1 H),7.78 (d, J = 1.5 Hz, 1 H),7.91 (d, J = 8.5 Hz, 2 H),8.14 (s, 1 H),8.41 (d, J =8.5 Hz, 1 H),10.00 (s, 1 H),12.81 (br s, 1 H). |

**Table 17-23**

| Example | solvent, Hz | NMR(δ) |
|---|---|---|
| 1-110 | DMSO-d6, 300MHz | 0.68(t, J=7.3Hz, 6H), 1.16(d, J=6.4Hz, 6H), 1.31-1.48(m, 2H), 1.49-1.64(m, 2H), 2.06-2.19(m, 1H), 3.58(s, 2H), 4.15-4.40(m, 7H), 6.62(d, J=8.7Hz, 2H), 6.88(d, J=8.7Hz, 2H), 7.14-7.22(m, 2H), 7.33(d, J=8.3Hz, 2H), 7.50-7.58(m, 2H), 7.84(d, J=8.3Hz, 2H), 7.95(s, 1H) |
| 1-111 | DMSO-d6, 300MHz | 0.79(t, J=7.2Hz, 6H), 0.97-1.18(m, 4H), 1.36-1.61(m, 4H), 2.39-2.54(m, 1H), 3.59(s, 2H), 4.20(s, 2H), 4.33(s, 2H), 5.08(s, 2H), 6.93(d, J=8.6Hz, 2H), 7.07(d, J=8.7Hz, 2H), 7.12-7.21(m, 2H), 7.44-7.58(m, 4H), 7.95(d, J=8.3Hz, 2H), 8.05(s, 1H) |
| 1-112 | DMSO-d6, 300MHz | 0.78 (t, J = 7.4 Hz, 6 H),1.00- 1.15 (m, 4 H),1.16 (d, J = 6.4 Hz, 6 H),1.30- 1.55 (m, 4 H),2.25- 2.40 (m, 1 H),3.08 (s, 3 H),4.15- 4.30 (m, 1 H),4.37 (s, 2 H),5.62 (s, 2 H),6.61 (d, J = 8.7 Hz, 2 H),6.89 (d, J = 8.7 Hz, 2 H),7.36 (d, J = 8.3 Hz, 2 H),7.45 (d, J = 8.3 Hz, 1 H),7.60 (dd, J = 8.3, 1.9 Hz, 1 H),7.81 (d, J = 1.9 Hz, 1 H),7.91 (d, J = 8.3 Hz, 2 H)8.10 (s, 1 H),9.33 (br s, 1 H),12.91 (br s, 1 H). |
| 1-113 | DMSO-d6, 300MHz | 0.78 (t, J = 7.4 Hz, 6 H),0.98- 1.15 (m, 1 H),1.11 (d, J = 6.8 Hz, 6 H),1.16 (d, J = 6.4 Hz, 6 H),1.30- 1.55 (m, 1 H),2.25- 2.40 (m, 1 H),2.75- 2.90 (m, 1 H),4.15- 4.30 (m, 1 H),4.37 (s, 2 H),5.63 (s, 2 H),6.61 (d, J = 8.7 Hz, 2 H),6.89 (d, J = 8.7 Hz, 2 H),7.36 (d, J = 8.3 Hz, 2 H),7.59 (dd, J = 8.4, 1.5 Hz, 1 H),7.78 (d, J = 1.5 Hz, 1 H),7.90 (d, J = 8.3 Hz, 2 H),8.09 (s, 1 H),8.13 (d, J = 8.4 Hz, 1 H),9.24 (s, 1 H), 12.77 (br s, 1 H). |
| 1-114 | DMSO-d6, 300MHz | 0.68 (t, J = 7.3 Hz, 6 H), 1.16 (d, J = 6.4 Hz, 6 H), 1.32-1.46 (m, 2 H), 1.48- 1.63 (m, 2 H), 2.05- 2.21 (m, 1 H), 4.23 (sept, J = 6.8 Hz, 1 H), 4.37 (s, 2 H), 4.88 (s, 2 H), 5.47 (s, 2 H), 6.61 (d, J = 9.1 Hz, 2 H), 6.88 (d, J = 8.7 Hz, 2 H), 7.12 (d, J = 8.3 Hz, 1 H), 7.34 (d, J = 8.3 Hz, 2 H), 7.62 (dd, J = 8.3, 1.9 Hz, 1 H), 7.92 (d, J = 8.3 Hz, 2 H), 8.01 (s, 1 H), 8.18 (d, J = 1.9 Hz, 1 H), 12.84 (br s, 1 H). |

**Table 17-24**

| Example | solvent, Hz | NMR(δ) |
|---|---|---|
| 1-115 | DMSO-d6, 300MHz | 0.68 (t, J = 7.3 Hz, 6 H), 1.15 (d, J = 6.4 Hz, 6 H), 1.32-1.47 (m, 2 H), 1.48- 1.64 (m, 2 H), 2.07- 2.18 (m, 1 H), 4.22 (sept, J = 6.6 Hz, 1 H), 4.37 (s, 2 H), 4.84 (s, 2 H), 5.49 (s, 2 H), 6.61 (d, J = 8.6 Hz, 2 H), 6.88 (d, J = 8.7 Hz, 2 H), 7.10 (t, J = 8.1 Hz, 1 H), 7.34 (d, J = 8.3 Hz, 2 H), 7.38 (dd, J = 7.9, 1.5 Hz, 1 H), 7.52 (dd, J = 8.1, 1.3 Hz, 1 H), 7.85 (d, J = 8.3 Hz, 2 H), 7.99 (s, 1 H), 12.91 (br s, 1 H). |
| 1-116 | DMSO-d6, 300MHz | 0.77(t, J=7.3Hz, 6H), 1.01-1.10(m, 4H), 1.16(d, J=6.6Hz, 6H), 1.32-1.50(m, 4H), 2.29-2.38(m, 1H), 3.17(s, 3H), 4.02(s, 2H), 4.18-4.26(m, 1H), 4.37(s, 2H), 4.80(s, 2H), 6.61(d, J=8.8Hz, 2H), 6.88(d, J=8.8Hz, 2H), 7.25-7.43(m, 7H), 7.87(d, J=8.4Hz, 2H), 8.02(s, 1H), 12.96(brs, 1H) |
| 1-117 | DMSO-d6, 300MHz | 0.77(t, J = 7.3 Hz, 6H), 1.00-1.14(m, 4H), 1.15(d, J = 6.6 Hz, 6H), 1.16(d, J = 6.6 Hz, 6H), 1.32-1.52(m, 4H), 2.27-2.39(m, 1H), 2.82(sep, J = 6.6 Hz, 1H), 3.51 (brs, 2H), 3.55(brs, 2H), 4.17-4.28(m, 3H), 4.36(s, 2H), 6.60(d, J = 8.8 Hz, 2H), 6.88(d, J = 8.8 Hz, 2H), 7.15(d, J = 8.4 Hz, 2H), 7.32(d, J = 8.4 Hz, 2H), 7.52(d, J = 8.4 Hz, 2H), 7.85(d, J = 8.4 Hz, 2H), 7.9.5(s, 1H) |
| 1-118 | DMSO-d6, 400MHz | 0.78(t, J = 7.3 Hz, 6H), 1.02-1.12(m, 4H), 1.15(d, J = 6.5 Hz, 6H), 1.34-1.51(m, 4H), 2.18(s, 6H), 2.30-2.37(m, 1H), 3.54(s, 2H), 3.60(s, 2H), 4.21(sep, J = 6.5 Hz, 1H), 4.25(s, 2H), 4.35(s, 2H), 6.59(d, J = 8.6 Hz, 2H), 6.66(s, 1H), 6.87(d, J = 8.6 Hz, 2H), 7.20(s, 2H), 7.31(d, J = 8.1 Hz, 2H), 7.84(d, J = 8.1 Hz, 2H), 7.95(s, 1H), 9.97(brs, 1H), 12.70(brs, 1H) |

**Table 17-25**

| Example | solvent, Hz | NMR(δ) |
|---|---|---|
| 1-119 | DMSO-d6, 400MHz | 0.77(t, J = 7.3 Hz, 6H), 1.02-1.11 (m, 4H), 1.15(d, J = 6.5 Hz, 6H), 1.30-1.50(m, 4H), 2.29-2.37(m, 1H), 2.83(s, 6H), 3.44(s, 2H), 3.52(s, 2H), 3.62(s, 2H), 4.21(sep, J = 6.5 Hz, 1H), 4.25(s, 2H), 4.35(s, 2H), 6.59(d, J = 8.6 Hz, 2H), 6.66(d, J = 9.0 Hz, 2H), 6.87(d, J = 8.6 Hz, 2H), 7.31(d, J = 8.4 Hz, 2H), 7.39(d, J = 9.0 Hz, 2H), 7.84(d, J = 8.4 Hz, 2H), 7.95(s, 1H), 9.68(s, 1H), 12.61(brs, 1H) |
| 1-120 | DMSO-d6, 400MHz | 0.77(t, J = 7.3 Hz, 6H), 1.02-1.11(m, 4H), 1.15(d, J = 6.5 Hz, 6H), 1.34-1.51(m, 4H), 2.29-2.37(m, 1 H), 3.44(s, 2H), 3.54(s, 2H), 4.18-4.25(m, 3H), 4.31(d, J = 6.0 Hz, 2H), 4.35(s, 2H), 6.59(d, J = 8.6 Hz, 2H), 6.87(d, J = 8.6 Hz, 2H), 7.17-7.28(m, 5H), 7.31(d, J = 8.4 Hz, 2H), 7.83(d, J = 8.4 Hz, 2H), 7.94(s, 1H), 8.40(t, J = 6.0 Hz, 1H), 12.59(brs, 1H) |
| 1-121 | DMSO-d6, 400MHz | 0.77(t, J = 7.3 Hz, 6H), 1.02-1.11(m, 4H), 1.15(d, J = 6.5 Hz, 6H), 1.34-1.50(m, 4H), 2.29-2.37(m, 1H), 3.29-3.58(m, 10H), 3.67(s, 2H), 4.19(s, 2H), 4.21(sep, J = 6.5 Hz, 1H), 4.35(s, 2H), 6.59(d, J = 8.6 Hz, 2H), 6.87(d, J = 8.6 Hz, 2H), 7.32(d, J = 8.4 Hz, 2H), 7.84(d, J = 8.4 Hz, 2H), 7.94(s, 1H), 12.54(brs, 1H) |
| 1-122 | DMSO-d6, 300MHz | 0.68(t, J=7.3Hz, 6H), 1.16(d, J=6.4Hz, 6H), 1.32-1.61(m, 4H), 2.05-2.17(m, 1H), 3.13(t, J=5.8Hz, 2H), 3.55(s, 2H), 4.09(t, J=5.8Hz, 2H), 4.17-4.30(m, 3H), 4.36(s, 2H), 6.62(d, J=8.6Hz, 2H), 6.87-6.93(m, 5H), 7.25(t, J=7.9Hz, 2H), 7.33(d, J=8.6Hz, 2H), 7.85(d, J=8.3Hz, 2H), 7.92(s, 1H) |
| 1-123 | DMSO-d6 300MHz | 0.67(t, J=7.4Hz, 6H), 1.14(d, J=6.4Hz, 6H), 1.31-1.62(m, 4H), 2.05-2.18(m, 1H), 2.90(t, J=6.4Hz, 2H), 3.13(t, J=6.4Hz, 2H), 3.32(s, 2H), 4.15-4.28(m, 3H), 4.34(s, 2H), 6.47-6.73(m, 5H), 6.87(d, J=8.6Hz, 2H), 7.05(t, J=7.9Hz, 2H), 7.31(d, J=8.3Hz, 2H), 7.83(d, J=7.9Hz, 2H), 7.91(s, 1H) |

**Table 17-26**

| Example | solvent, Hz | NMR(δ) |
|---|---|---|
| 1-124 | DMSO-d6, 300MHz | 0.69 (t, J = 7.17 Hz, 6H), 1.16 (d, J = 6.39 Hz, 6H), 1.33-1.45 (m, 2H), 1.48- 1.62 (m, 2H), 2.08- 2.18 (m, 1H), 3.04 (s, 3H), 4.23 (quint, J = 6.39 Hz, 1H), 4.37 (s, 2H), 4.63 (s, 2H), 4.84 (s, 2H), 6.63 (d, J = 8.67 Hz, 2H), 6.89 (d, J = 8.67 Hz, 2H), 6.94 (d, J = 9.78 Hz, 1 H), 6.99 (dd, J = 9.78, 2.64 Hz, 1H), 7.12 (d, J = 2.64 Hz, 1H), 7.34 (d, J = 8.28 Hz, 2H), 7.87 (d, J = 8.28 Hz, 2H), 7.90 (s, 1H) |
| 1-125 | DMSO-d6, 300MHz | 0.80 (t, J = 7.15 Hz, 3H), 0.98-1.14 (m, 4H), 1.41-1.57 (m, 4H), 2.31-2.41 (m, 1H), 3.05 (s, 3H), 4.64 (s, 2H), 4.86 (s, 2H), 5.08 (s, 2H), 6.92-6.97 (m, 4H), 7.06-7.12 (m. 2H), 7.51 (d, J = 8.24 Hz, 2H), 7.97 (d, J = 8.24 Hz, 2H), 7.99 (s, 1H) |
| 1-126 | DMSO-d6, 300MHz | 1HNMR(DMSO-d6,300MHz) δ .78 (t, J = 7.2 Hz, 6 H).95- 1.15 (m, 4 H),1.16 (d, J = 6.8 Hz, 6 H),1.30- 1.55 (m, 4 H),2.30- 2.40 (m, 1 H),2.34 (s, 3 H),3.27 (s, 2 H),4.15- 4.30 (m, 1 H),4.37 (s, 2 H),5.67 (s, 2 H),6.61 (d, J = 8.5 Hz, 2 H),6.89 (d, J = 8.5 Hz, 2 H),7.36 (d, J = 8.3 Hz, 2 H),7.63 (dd, J = 8.5, 1.5 Hz, 1 H),7.78 (d, J = 1.5 Hz, 1 H),7.91 (d, J = 8.3 Hz, 2 H),8.12 (s, 1 H),8.43 (d, J = 8.5 Hz, 1 H). |
| 1-127 | DMSO-d6, 400MHz | 0.79(t, J=7.2Hz, 6H), 1.01-1.14(m, 4H), 1.26(s, 9H), 1.37-1.57(m, 4H), 2.41-2.49(m, 1H), 3.42(s, 2H), 4.22(s, 2H), 4.28(s, 2H), 5.07(s, 2H), 6.92(d, J=8.8Hz, 2H), 7.06(d, J=8.8Hz, 2H), 7.16(s, 1H), 7.48(d, J=8.0Hz, 2H), 7.94(d, J=8.4Hz, 2H), 8.04(s, 1H) |
| 1-128 | DMSO-d6, 400MHz | 0.80(t, J=7.4Hz, 6H), 1.00-1.17(m, 4H), 1.38-1.57(m, 4H), 2.41-2.52(m, 1H), 4.28(s, 2H), 4.92(s, 2H), 5.08(s, 2H), 6.65-6.72(m, 1H), 6.68(d, J=8.0Hz, 2H), 6.93(d, J=8.8Hz, 2H), 7.06(d, J=8.4Hz, 2H), 7.16(dd, J=7.2, 7.2Hz, 2H), 7.51(d, J=8.4Hz, 2H), 7.97(d, J=8.0Hz, 2H), 7.99(s, 1H), 12.74(brs, 1H) |

**Table 17-27**

| Example | solvent, Hz | NMR(δ) |
|---|---|---|
| 1-129 | DMSO-d6, 300MHz | 0.79 (t, J = 7.3 Hz, 6 H),0.95- 1.20 (m, 4 H),1.35- 1.60 (m, 4 H),2.40- 2.53 (m, 1 H),4.10 (s, 2 Hx0.4),4.37 (s, 2Hx0.6),4.80- 5.12 (m, 6H),6.85- 6.98 (m, 5H),7.07 (d, J = 8.6 Hz, 2Hx0.6),7.04- 7.31 (m, 2H),7.15- 7.31 (m, 2H),7.51 (d, J = 7.9 Hz, 2Hx0.4),7.96 (d, J = 8.3 Hz, 2Hx0.6),7.97 (d, J = 7.9 Hz, 2Hx0.4),8.06 (s, 1 Hx0.6),8.13 (s, 1Hx0.4),12.73 (br s, 1 Hx0.4),13.07 (br s, 1 Hx0.6). |
| 1-130 | DMSO-d6, 300MHz | 0.79 (t, J = 7.3 Hz, 6 H),0.95- 1.20 (m, 4 H),1.35- 1.60 (m, 4 H),2.19 (s, 3 Hx0.4),2.22 (s, 3 Hx0.6),2.40- 2.53 (m, 1 H),4.10 (s, 2 Hx0.4),4.36 (s, 2 Hx0.6),4.78 (s, 2 Hx0.6),4.84 (s, 2 Hx0.6),5.03 (s, 2 Hx0.4),5.05 (s, 2 Hx0.4),5.09 (s, 2 H),6.75- 7.10 (m, 8 H),7.51 (d, J = 8.3 Hz, 2 H),7.96 (d, J = 8.3 Hz, 2 H),8.06 (s, 1 Hx0.6),8.12 (s, 1 Hx0.4),12.73 (br s, 1 Hx0.4),13.05 (br s, 1 Hx0.6). |
| 1-131 | DMSO-d6, 400MHz | 0.61-0.69(m, 6H), 0.74-0.97(m, 4H), 1.18(d, J = 6.3 Hz, 3H), 1.30-1.50(m, 4H), 1.63(d, J = 6.0 Hz, 3H), 2.39-2.48(m, 1 H), 3.56(s, 2H), 3.65(s, 2H), 4.03-4.14(m, 3H), 4.21(s, 2H), 4.60-4.68(m, 1H), 4.96-5.02(m, 1H), 7.14(d, J = 8.1 Hz, 2H), 7.39(d, J = 8.1 Hz, 2H), 7.55(d, J = 8.1 Hz, 2H), 7.67(d, J = 8.1 Hz, 2H), 7.99(s, 1H), 12.63(brs, 1H) |
| 1-132 | DMSO-d6, 300MHz | 0.68(t, J = 7.3 Hz, 6H), 1.16(d, J = 6.6 Hz, 6H), 1.26(s, 9H), 1.32-1.47(m, 2H), 1.49-1.63(m, 2H), 2.08-2.17(m, 1H), 3.45(s, 2H), 4.19-4.29(m, 5H), 4.36(s, 2H), 6.62(d, J = 8.8 Hz, 2H), 6.88(d, J = 8.8 Hz, 2H), 7.17(s, 1H), 7.33(d, J = 8.1 Hz, 2H), 7.85(d, J = 8.1 Hz, 2H), 7.96(s, 1H) |
| 1-133 | DMSO-d6, 300MHz | 0.77(t, J = 7.3 Hz, 6H), 1.01-1.13(m, 4H), 1.16(d, J = 6.2 Hz, 6H), 1.35-1.51 (m, 4H), 2.29-2.38(m, 1H), 4.22(sep, J = 6.2 Hz, 1H), 4.37(s, 2H), 5.79(s, 2H), 6.61(d, J = 8.8 Hz, 2H), 6.89(d, J = 8.8 Hz, 2H), 7.19(d, J = 8.1 Hz, 2H), 7.35(d, J = 8.1 Hz, 2H), 7.74(d, J = 7.3 Hz, 2H), 7.89(d, J = 8.4 Hz, 2H), 7.92-7.97(m, 1H), 8.05(s, 1H) |

**Table 17-28**

| Example | solvent, Hz | NMR(δ) |
|---|---|---|
| 1-134 | DMSO-d6, 300MHz | 0.79 (t, J = 7.3 Hz, 6 H),0.95- 1.20 (m, 4 H),1.35- 1.60 (m, 4 H),2.40- 2.53 (m, 1 H),4.10 (s, 2 Hx0.4),4.23 (s, 2 Hx0.6),4.29 (s, 2 Hx0.6),4.41 (s, 2 Hx0.4),4.52 (s, 2 Hx0.6),4.54 (s, 2 Hx0.4),4.84 (s, 2 Hx0.6),4.97 (s, 2x0.4 H),5.09 (s, 2 H),6.94 (d, J = 8.7 Hz, 2 H),7.07 (d, J = 8.7 Hz, 2 H),7.25- 7.40 (m, 5 H),7.45- 7.55 (m, 2 H),7.92-7.98 (m, 2 H),8.06 (s, 1 Hx0.6),8.08 (s, 1 Hx0.4),12.80 (br s, 1 H). |
| 1-135 | DMSO-d6, 300MHz | 0.79 (t, J = 7.2 Hz, 6 H),0.96- 1.21 (m, 4 H),1.13 (d, J = 6.8 Hz, 6 Hx0.4),1.16 (d, J = 7.1 Hz, 6 Hx0.6),1.34- 1.62 (m, 4 H),2.39- 2.54 (m, 1 H),2.70- 2.89 (m, 1. H),4.10 (s, 2 Hx0.4),4.36 (s, 2 Hx0.6),4.79 (s, 2 Hx0.6),4.84 (s, 2 Hx0.6),5.04 (s, 2 Hx0.4),5.05 (s, 2 Hx0.4),5.09 (s, 2 H),6.77- 6.87 (m, 2 H),6.90- 6.98 (m, 2 H),7.02- 7.15 (m, 4 H),7.51 (d, J = 8.1 Hz, 2 H),7.97 (d, J = 8.1 Hz, 2 Hx0.6),7.98 (d, J = 8.1 Hz, 2 Hx04),8.06 (s, 1 Hx0.6),8.13 (s, 1 Hx0.4),12.75 (br.s, 1 Hx0.4),13.01 (br.s, 1 Hx0.6). |
| 1-136 | DMSO-d6, 400MHz | 0.80(t, J=8.2Hz, 6H), 0.99-1.16(m, 4H), 1.37-1.58(m, 4H), 2.20(s, 3H), 2.30(s, 3H), 2.41-2.52(m, 1H), 3.46(s, 2H), 4.12(s, 2H), 4.26(s, 2H), 5.07(s, 2H), 6.93(d, J=6.8Hz, 2H), 7.06(d, J=8.8Hz, 2H), 7.49(d, J=8.4Hz, 2H), 7.94(d, J=8.0Hz, 2H), 8.05(s, 1 H), 12.65(brs, 1 H), |
| 1-137 | DMSO-d6, 400MHz | 0.79(t, J=7.2Hz, 6H), 0.99-1.15(m, 4H), 1.38-1.58(m, 4H), 2.40-2.52(m, 1H), 5.24(s, 2H), 5.56(s, 2H), 5.56(s, 2H), 6.94(d, J=7.6Hz, 2H), 7.07(d, J=8.0Hz, 2H), 7.17(brs, 1H), 7.48(brs, 1H), 7.80(s, 1H), 8.01 (s, 1H), 8.37(s, 1H), 8.63(s, 1 H) |
| 1-138 | DMSO-d6, 300MHz | 0.77(t, J = 6.0Hz, 6H), 1.03-1.09(m,4H), 1.18(d,J = 9.0Hz, 6H), 1.26(s,9H), 1.38-1.45(m,4H), 2.30-2.42(m,1H), 3.54(s,2H), 4.24(s,2H), 4.25-4.30(m,1H), 4.32(s,2H), 4.50(s,2H), 6.65(d,J = 9.0Hz, 2H), 6.90(d,J = 9.0Hz, 2H), 7.20(s,1H), 7.38(d,J = 9.0Hz, 1H), 7.62(s,1H), 7.73(d,J = 9.0Hz, 1H), 8.57(s,1H) |

**Table 17-29**

| Example | solvent, Hz | NMR(δ) |
|---|---|---|
| 1-139 | DMSO-d6 300MHz | 0.77(t, J = 6.0Hz, 6H), 1.02-1.10(m,4H), 1.18(d,J = 9.0Hz, 6H), 1.38-1.44(m,4H), 2.30-2.36(m,1H), 3.61(s,2H), 4.19(s,2H), 4.20-4.25(m,1H), 4.36(s,2H), 4.50(s,2H), 6.65(d,J = 9.0Hz, 2H), 6.90(d,J = 9.0Hz, 2H), 7.13(m,2H), 7.38(d,J = 9.0Hz, 1H), 7.53-7.56(m,2H), 7.62(s,1H), 7.73(d,J = 9.0Hz, 1 H), 8.55(s,1H) |
| 1-140 | DMSO-d6, 300MHz | 0.78(t, J = 6.0Hz, 6H), 1.02(m,4H), 1.19(d,J = 6.0Hz, 6H), 1.34-1.52(m,4H), 2.30-2.38(m,1 H), 4.20(s,2H), 4.25-4.27(m,1 H), 4.37(s,2H), 6.66(d,J = 9.0Hz, 2H), 7.03(d,J = 66.0Hz, 2H), 7.16-7.20(m,2H), 7.40-7.42(m,1H), 7.56-7.59(m,2H), 7.63(s,1H), 7.74(d,J = 9.0Hz, 1 H), 8.56(s,1H) |
| 1-141 | DMSO-d6, 400MHz | 0.79(t, J=7.2Hz, 6H), 0.99-1.14(m, 4H), 1.33(s, 9H), 1.37-1.57(m, 4H), 2.41-2.53(m, 1H), 3.43(s, 2H), 4.17(s, 2H), 4.27(s, 2H), 5.07(s, 2H), 6.92(d, J=8.4Hz, 2H), 7.06(d, J=8.4Hz, 2H), 7.41 (s, 1H), 7.49(d, J=8.4Hz, 2H), 7.94(d, J=8.4Hz, 2H), 8.05(s, 1H) |
| 1-142 | DMSO-d6, 300MHz | 0.76(t, J=7.2Hz, 6H), 0.99-1.09(m, 4H), 1.14(d, J=6.0Hz, 6H), 1.30-1.56(m, 10H), 2.25-2.38(m, 1H), 3.14(brs, 2H), 3.41 (brs, 4H), 3.62(s, 2H), 4.12(s, 2H), 4.17-4.25(m, 1H), 4.34(s, 2H), 6.59(d, J=8.4Hz, 2H), 6.87(d, J=8.4Hz, 2H), 7.32(d, J=7.5Hz, 2H), 7.84(d, J=6.9Hz, 2H), 7.92(s, 1H) |
| 1-143 | DMSO-d6, 300MHz | 0.80 (t, J = 7.3 Hz, 6 H),0.98- 1.18 (m, 4 H),1.35- 1.61 (m, 4 H),2.35- 2.53 (m, 1 H),3.18 (s, 3 H),5.08 (s, 2 H),5.22 (s, 2 H),6.94 (d. J = 8.7 Hz, 2 H),7.01 (d, J = 8.4 Hz, 1 H),7.07 (d, J = 8.7 Hz, 2 H),7.35 (d, J = 6.8 Hz, 1 H),7.51 (d, J = 7.9 Hz, 2 H),7.74 (dd, J = 8.4, 6.8 Hz, 1 H),7.96 (d, J = 7.9 Hz, 2 H),7.99 (s, 1 H),12.60 (br s, 1 H). |
| 1-144 | DMSO-d6, 300MHz | 0.77(t, J = 6.0Hz, 6H), 1.02-1.10(m,4H), 1.18(d,J = 9.0Hz, 6H), 1.38-1.48(m,4H), 2.30-2.35(m,1H), 3.40(s,2H), 4.14(s,2H), 4.22-4.28(m,1H), 4.50(s,1H), 6.65(d,J = 9.0Hz, 2H), 6.90(d,J = 9.0Hz, 2H), 7.38(d,J = 9.0Hz, 1H), 7.62(s,1H), 7.73(d,J = 9.0Hz, 1 H), 7.89(m,1H), 8.52(s,1H) |

**Table 17-30**

| Example | solvent, Hz | NMR(δ) |
|---|---|---|
| 1-145 | DMSO-d6, 300MHz | 0.61-0.83(m, 6H), 1.00-1.24(m, 6H), 1.35-1.65(m, 8H), 2.27-2.39(m, 1H), 3.19-3.34(m, 2H), 4.03-4.26(m, 2H), 4.36(brs, 1H), 4.61-4.72(m, 1H), 4.83(brs, 1H), 4.96-5.06(m, 1H), 6.62(brs, 2H), 6.88(brs, 2H), 7.17(brd, J=8.4Hz, 2H), 7.28-7.53(m, 3H), 7.69(brd, J=7.2Hz, 2H), 7.79-7.87(m, 1H), 7.91-7.99(m, 2H) |
| 1-146 | DMSO-d6, 300MHz | 0.78(t, J=7.3Hz, 6H), 1.01-1.22(m, 4H), 1.16(d, J=6.6Hz, 6H), 1.32-1.52(m, 4H), 2.20(s, 3H), 2.28-2.38(m, 1H), 2.30(s, 3H), 3.48(s, 2H), 4.11 (s, 2H), 4.18-4.28(m, 1H), 4.25(s, 2H), 4.36(s, 2H), 6.61(d, J=8.4Hz, 2H), 6.88(d, J=8.4Hz, 2H), 7.34(d, J=8.0Hz, 2H), 7.86(d, J=8.0Hz, 2H), 7.97(s, 1H), 12.57(brs, 1H) |
| 1-147 | DMSO-d6, 400MHz | 0.79(t, J=7.4Hz, 6H), 0.99-1.14(m, 4H), 1.23(s, 9H), 1.37-1.56(m, 4H), 2.39-2.49(m, 1H), 3.48(s, 2H), 4.03(s, 2H), 4.23(s, 2H), 5.07(s, 2H), 6.72(s, 1H), 6.92(d, J=8.8Hz, 2H), 7.06(d, J=8.8Hz, 2H), 7.48(d, J=8.4Hz, 2H), 7.93(d, J=8.0Hz, 2H), 8.02(s, 1H) |
| 1-148 | DMSO-d6, 300MHz | 0.79(t, J=7.4Hz, 6H), 0.98-1.16(m, 4H), 1.35-1.60(m, 4H), 2.40-2.54(m, 1H), 3.65(s, 2H), 3.96(s, 3H), 4.36(s, 2H), 4.45(s, 2H), 5.07(s, 2H), 6.93(d, J=8.7Hz, 2H), 7.07(d, J=8.7Hz, 2H), 7.31-7.44(m, 2H), 7.47(d, J=8.3Hz, 2H), 7.64-7.77(m, 2H), 7.88(d, J=8.3Hz, 2H), 7.98(s, 1H) |
| 1-149 | DMSO-d6 +D2O+NaHCO3, 300MHz | 0.78(t, J=7.4Hz, 6H), 0.87(d, J=6.4Hz, 6H), 0.99-1.13(m, 4H), 1.16(d, J=6.4Hz, 6H), 1.32-1.50(m, 4H), 1.78-1.90(m, 1H), 2.26-2.39(m, 1H), 3.08(s, 2H), 3.72(s, 2H), 3.82(d, J=6.4Hz, 2H), 4.16-426(m, 3H), 4.36(s, 2H), 6.60(d, J=8.7Hz, 2H), 6.88(d, J=8.7Hz, 2H), 7.32(d, J=8.3Hz, 2H), 7.84(d, J=8.3Hz, 2H), 7.89(s, 1H) |
| 1-150 | DMSO-d6 +D2O+NaHCO3, 300MHz | 0.78(t, J=7.2Hz, 6H), 0.86(t, J=7.4Hz, 3H), 0.98-1.12(m, 4H), 1.16(d, J=6.4Hz, 6H), 1.30-1.63(m, 6H), 2.27-2.40(m, 1H), 3.98(t, J=6.6Hz, 2H), 4.17-4.25(m, 3H), 4.35(s, 2H), 6.60(d, J=8.6Hz, 2H), 6.88(d, J=8.6Hz, 2H), 7.33(d, J=8.3Hz, 2H), 7.84(d, J=8.3Hz, 2H), 7.88(s, 1H) |

**Table 17-31**

| Example | solvent, Hz | NMR(δ) |
|---|---|---|
| 1-151 | DMSO-d6, 300MHz | 0.80(t, J=7.2Hz, 6H), 1.01-1.15(m, 4H), 1.37-1.58(m, 4H), 1.75-2.16(m, 5H), 2.43-2.46(m, 1H), 3.11 (brs, 2H), 3.55(brs, 2H), 3.86(brs, 1H), 5.10(s, 2H), 6.94(d, J=8.7Hz, 2H), 7.08(d, J=8.7Hz, 2H), 7.54(d, J=9.0Hz, 2H), 8.01(d, J=9.0Hz, 2H), 8.32(brs, 1H), 11.54(brs, 1H), 12.45(brs, 1H) |
| 1-152 | DMSO-d6, 300MHz | 0.69(t, J=7.3Hz, 6H), 1.16(d, J=6.8Hz, 6H), 1.33-1.65(m, 6H), 1.76-1.87(m, 2H), 2.08-2.30(m, 4H), 2.83-2.92(m, 2H), 3.84(s, 2H), 4.18-4.27(m, 1H), 4.36(s, 2H), 6.62(d, J=8.7Hz, 2H), 6.88(d, J=8.7Hz, 2H), 7.34(d, J=8.3Hz, 2H), 7.86(d, J=7.9Hz, 2H), 7.95(s, 1H), 12.15(s, 1H) |
| 1-153 | DMSO-d6, 300MHz | 0.80(t, J=7.2Hz, 6H), 1.01-1.18(m, 4H), 1.38-1.58(m, 4H), 2.42-2.48(m, 1H), 2.70(brs, 4H), 3.35(brs, 4H), 3.96(s, 2H), 5.09(s, 2H), 6.94(d, J=9.1 Hz, 2H), 6.98(d, J=9.4Hz, 2H), 7.08(d, J=8.7Hz, 2H), 7.51(d, J=8.3Hz, 2H), 7.78(d, J=9.0Hz, 2H), 7.98(d, J=8.3Hz, 2H), 8.08(s, 1H), 12.29(brs, 1H) |
| 1-154 | DMSO-d6, 300MHz | 0.69(t, J=7.4Hz, 6H), 1.16(d, J=6.4Hz, 6H), 1.34-1.62(m, 4H), 2.08-2.18(m, 1H), 2,68(brs, 4H), 3.36(brs, 4H), 3.94(s, 2H), 4.18-4.27(m, 1H), 4.37(s, 2H), 6.62(d, J=8.6Hz, 2H), 6.89(d, J=8.6Hz, 2H), 6.97(d, J=9.0Hz, 2H), 7.34(d, J=7.9Hz, 2H), 7.77(d, J=9.0Hz, 2H), 7.87(d, J=8.3Hz, 2H), 7.98(s, 1H), 12.29(brs, 1H) |
| 1-155 1-155 | DMSO-d6, 300MHz | 0.81(t, J=7.3Hz, 6H), 1.00-1.19(m, 4H), 1.43-1.59(m, 4H), 2.53-2.61(m, 1H), 2.81 (brs, 4H), 3.13(brs, 4H), 4.01(s, 2H), 5.09(s, 2H), 7.07(d, J=12.7Hz, 2H), 7.19(d, J=8.3Hz, 2H), 7.35-7.42(m, 3H), 7.62-7.74(m, 2H), 7-89(d, J=9.0Hz, 2H), 7.92(s, 1H), 7.98-8.01(m, 1H), 12.81(brs, 1H) |

**Table 17-32**

| Example | solvent, Hz | NMR(δ) |
|---|---|---|
| 1-156 | DMSO-d6, 300MHz | 0.81 (t, J=7.2Hz, 6H), 1.02-1.18(m, 4H), 1.45-1.61 (m, 4H), 2.55-2.60(m, 1H), 2.72(brs, 4H), 3.23(brs, 4H), 3.95(s, 2H), 5.09(s, 2H), 7.08(d, J=9.1 Hz, 2H), 7.16-7.23(m, 3H), 7.31(d, J=7.5Hz, 2H), 7.34-7.42(m, 2H), 7.47(brs, 1H), 7.89(d, J=9.0Hz, 2H), 7.90(brs, 1H), 12.84(brs, 1H) |
| 1-157 | DMSO-d6, 300MHz | 0.76(t, J = 7.3Hz, 6H), 0.93(m,4H), 1.36(m,4H), 5.45(s,2H), 5.60(s,2H), 7.09(m,9H), 7.57(d,J = 7.3Hz, 1H), 7.85(s,1H), 7.93(d,J = 8.4Hz, 2H), 8.06(s,1H), 8.11(d,J = 8.1Hz, 1H) |
| 1-158 | DMSO-d6, 300MHz | 0.74(t, J = 7.5Hz, 6H), 0.98(m,4H), 1.06(d,J = 7.2Hz, 6H), 1.48(m,4H), 2.7(m,1H), 5.59(s,2H), 5.92(s,2H), 6.99(m,6H), 7.37(s,1H), 7.42(d,J = 8.3Hz, 1H), 7.80(d,J = 8.7Hz, 2H), 8.41 (s,1H) |
| 1-159 | CDCl3, 300MHz | 0.82(t, J = 7.2Hz, 6H), 1.09(m,4H), 1.53(s,3H), 1.54(m,4H), 2.53(m,1H), 0.82(s,1H), 6.99(d,J = 6.8Hz, 1H), 7.14(m,1H), 7.25(s,3H), 7.30(d,J = 8.3Hz, 1H), 7.38(s,1H), 8.04(brs,1H) |
| 1-160 | DMSO-d6, 300MHz | 0.80(t, J=7.1 Hz, 6H), 0.99-1.16(m, 4H), 1.38-1.59(m, 4H), 2.40-2.50(m, 1H), 2.82-2.98(m, 4H), 3.84(s, 2H), 4.09(s, 2H), 5.09(s, 2H), 6.94(d, J=8.8Hz, 2H), 7.08(d, J=8.4Hz, 2H), 7.26(d, J=8.1 Hz, 1 H), 7.52(d, J=8.4Hz, 2H), 7.66(s, 1 H), 7.72(d, J=8.1 Hz, 1 H), 7.99(d, J=8.1 Hz, 2H), 8.08(s, 1H), 12.79(s, 1H) |
| 1-161 | DMSO-d6, 300MHz | 0.81 (t, J=7.3Hz, 6H), 1.00-1.18(m, 4H), 1.43-1.60(m, 4H), 1.64-1.83(m, 4H), 2.29(t, J=11.1Hz, 3H), 2.55-2.67(m, 1H), 3.06(d, J=11.3Hz, 2H), 3.90(s, 2H), 5.08(s, 2H), 7.07(d, J=8.6Hz, 2H), 7.19(d, J=8.3Hz, 2H), 7.37-7.41(m, 4H), 7.86-7.89(m, 5H) |

**Table 17-33**

| Example | solvent, Hz | NMR(δ) |
|---|---|---|
| 1-162 | DMSO-d6, 300MHz | 0.81(t, J=7.3Hz, 6H), 1.02-1.19(m, 4H), 1.43-1.60(m, 4H), 1.66-1.86(m, 4H), 2.30(t, J=10.5Hz, 2H), 2.53-2.68(m, 2H), 3.06(d, J=11.7Hz, 2H), 3.91(s, 2H), 5.09(s, 2H), 7.07(d, J=8.6Hz, 2H), 7.19(d, J=7.9Hz, 2H), 7.37-7.46(m, 3H), 7.54(d, J=7.9Hz, 1 H), 7.78(d, J=7.5Hz, 1H), 7.83(s, 1H), 7.87-7.90(m, 3H), 12.93(brs, 1H) |
| 1-163 | DMSO-d6 300MHz | 0.80 (t, J = 7.3 Hz, 6 H),0.98- 1.18 (m, 4 H),1.36- 1.60 (m, 4 H),2.39- 2.53 (m, 1 H),4.85 (d, J = 6.3 Hz, 2 H),5.09 (s, 2 H),6.94 (d, J = 8.7 Hz, 2 H),7.07 (d, J = 8.7 Hz, 2 H),7.51 (d, J = 8.3 Hz, 2 H),7.97 (d, J = 8.3 Hz, 2 H),8.01 (s, 1 H),8.20 (dd, J = 8.1, .9 Hz, 1 H),8.48 (dd, J = 8.1, 2.1 Hz, 1 H),9.14 (dd, J = 2.1, .9 Hz, 1 H),9.88 (t, J =6.3 Hz, 1 H),13.74 (br s, 1 H). |
| 1-164 | DMSO-d6, 300MHz | 0.81(t, J=7.2Hz, 6H), 1.03-1.17(m, 4H), 1.48-1.59(m, 4H), 1.70-1.80(m, 4H), 2.19-2.30(m, 2H), 2.48-2.59(m, 2H), 3.06(d, J=10.9Hz, 2H), 3.90(s, 2H), 5.08(s, 2H), 7.07(d, J=9.0Hz, 2H), 7.19(d, J=8.3Hz, 2H), 7.24-7.31(m, 1H), 7.38(d, J=8.3Hz, 2H), 7.45-7.53(m, 2H), 7.66(d, J=8.3Hz, 1H), 7.87-7.89(m, 3H) |
| 1-165 | CDCl3, 300MHz | 0.80(t, J = 7.2Hz, 6H), 1.06(m,4H), 1.43(m,4H), 2(m,4H), 2.42(m,2H), 2.64(m,1H), 3.17(m,2H), 4.17(s,2H), 5.31(s,2H), 7.02(m,4H), 7.21(m,5H), 7.33(m,2H), 7.69(s,1H), 7.99(m,1H) |
| 1-166 | CDCl3, 300MHz | 0.80(t, J = 7.5Hz, 6H), 1.03(m,4H), 1.43(m,4H), 2.41 (m,1H), 3.10-3.25(m,4H), 4.12(m,2H), 4.32(m,2H), 5.32(s,2H), 7.02-7.09(m,4H), 7.21 (m,6H), 7.34(m,2H), 7.72(s,1H), 7.77(s,1H), 7.85(d,J = 6.4Hz, 1H), 8.01(t, J = 3.0Hz, 1H) |
| 1-167 | DMSO-d6, 300MHz | 0.77(t, J = 7.3Hz, 6H), 1(m,4H), 1.42(m,4H), 4.68(s,2H), 5.44(s,2H), 6.86(m,1H); 7.1(m,9H), 7.56(d,J = 7.7Hz, 1H), 7.65(s,1H), 8.04(s,1H), 8.10(d,J = 7.3Hz, 1H) |

**Table 17-34**

| Example | solvent, Hz | NMR(δ) |
|---|---|---|
| 1-168 | DMSO-d6, 300MHz | 0.80 (t, J = 7.2 Hz, 6 H),0.98- 1.19 (m, 4 H),1.36- 1.62 (m, 4 H),2.39- 2.53 (m, 1 H),3.07- 3.32 (m, 2 H),3.66-4.10 (m, 2 H),5.02- 5.12 (m, 4 H),6.94 (d, J = 8.5 Hz, 2 H),7.08 (d, J = 8.5 Hz, 2 H),7.48- 7.58 (m, 2 H),7.84 (d, J = 7.9 Hz, 1 Hx0.5),7.89 (d, J = 8.3 Hz, 1 Hx0.5),7.92-8.14 (m, 5 H),8.12 (s, 1 Hx0.5),8.13 (s, 1 Hx0.5),8.39-8.48 (m, 1 H),9.04 (d, J = 1.5 Hz, 1 Hx0.5),9.15 (d, J = 1.5 Hz, 1 Hx0.5),13.67 (br s, 1 H). |
| 1-169 | DMSO-d6, 300MHz | 0.72(t, J=7.4Hz, 6H), 0.87-1.08(m, 4H), 1.30-1.53(m, 4H), 2.36-2.47(m, 1H), 2.78-2.92(m, 4H), 3.80(s, 2H), 4.12(s, 2H), 6.05(s, 2H), 6.98-7.09(m, 4H), 7.20-7.30(m, 3H), 7.56-7.75(m, 4H), 8.40(s, 1 H), 12.77(s, 1H) |
| 1-170 | DMSO-d6, 300MHz | 0.73(t, J=7.2Hz, 6H), 0.89-1.09(m, 4H), 1.31-1.54(m, 4H), 2.37-2.50(m, 1H), 2.69(brs, 4H), 3.19(brs, 4H), 4.00(s, 2H), 6.04(s, 2H), 7.05(s, 4H), 7.16-7.41(m, 5H), 7.46(brs, 1H), 7.57-7.74(m, 2H), 8.42(s, 1H), 12.71(brs, 1H) |
| 1-171 | DMSO-d6, 300MHz | 0.70(t, J=6.8Hz, 6H), 0.85-1.07(m, 4H), 1.30-1.51(m, 4H), 1.57-1.79(m, 4H), 2.20-2.63(m, 4H), 2.94-3.05(m, 2H), 3.96(s, 2H), 6.04(s, 2H), 7.03(s, 4H), 7.24(brs, 2H), 7.35(d, J=7.5Hz, 2H), 7.56-7.73(m, 2H), 7.87(d, J=7.5Hz, 2H), 8.39(s, 1H) |
| 1-172 | DMSO-d6, 300MHz | 0.75(t, J = 7.5Hz, 6H), 0.96(m,4H), 1.4(m,4H), 2.72(m,4H), 3.22(m,4H), 3.95(s,2H), 5.42(s,2H), 7.07-7.20(m,7H), 7.31(m,1H), 7.45(s,1H), 7.54(d,J = 7.2Hz, 1H), 7.73(s,1H), 8.09(d,J = 6.0Hz, 1H) |
| 1-173 | DMSO-d6, 300MHz | 1.64-1.85(m, 4H), 2.30(t, J=10.4Hz, 2H), 2.58-2.68(m, 1H), 3.06(d, J=11.7Hz, 2H), 3.90(s, 2H), 5.17(s, 2H), 7.08(d, J=9.0Hz, 2H), 7.40-7.54(m, 3H), 7.67(d, J=8.3Hz, 1H), 7.75(d, J=2.3Hz, 1H), 7.76-7.80(m, 1H), 7.81-7.84(m, 1H), 7.86-7.91(m, 3H) |

**Table 17-35**

| Example | solvent, Hz | NMR(δ) |
|---|---|---|
| 1-174 | DMSO-d6, 300MHz | 1.72-2.12(m, 4H), 2.26-2.46(m, 2H), 2.80-3.03(m, 1H), 3.54-3.80(m, 2H), 4.82(s, 2H), 5.38(s, 2H), 7.16(d, J=8.3Hz, 2H), 7.42-7.53(m, 2H), 7.77-7.86(m, 2H), 7.96(d, J=7.9Hz, 2H), 8.11-8.19(m, 3H), 12.94(brs, 1H) |
| 1-175 | DMSO-d6, 300MHz | 0.93(t, J=7.4Hz, 3H), 1.37-1.49(m, 2H), 1.65-1.85(m, 6H), 2.25-2.35(m, 2H), 3.02-3.10(m, 3H), 3.90(s, 2H), 3.97(t, J=6.4Hz, 2H), 5.05(s, 2H), 6.94(d, J=8.7Hz, 2H), 7.05(d, J=9.0Hz, 2H), 7.36-7.44(m, 3H), 7.49-7.54(m, 1H), 7.75-7.88(m, 5H) |
| 1-176 | DMSO-d6, 400MHz | 0.80(t, J=7.3Hz, 6H), 1.02-1.13(m, 4H), 1.40-1.57(m, 4H), 2.21(s, 3H), 2.42-2.51(m, 1H), 5.08(s, 2H), 6.00(s, 2H), 6.72(s, 1H), 6.93(d, J=8.6Hz, 2H), 7.06(d, J=8.6Hz, 2H), 7.50(d, J=8.3Hz, 2H), 7.93(d, J=8.3Hz, 2H), 8.03(s, 1H), 13.52(brs, 1H) |
| 1-177 | DMSO-d6, 400MHz | 0.79(t, J=7.3Hz, 6H), 1.02-1.13(m, 4H), 1.39-1.56(m, 4H), 2.39(s, 3H), 2.42-2.52(m, 1H), 5.08(s, 2H), 5.77(s, 2H), 6.56(s, 1H), 6.92(d, J=8.6Hz, 2H), 7.06(d, J=8.6Hz, 2H), 7.51(d, J=8.1Hz, 2H), 7.94(d, J=8.1 Hz, 2H), 8.10(s, 1H), 12.67(brs, 1H) |
| 1-178 | DMSO-d6, 300MHz | 0.80(t, J=7.3Hz, 6H), 1.01-1.15(m, 4H), 1.36(s, 9H), 1.40-1.59(m, 4H), 2.41-2.53(m, 1H), 5.09(s, 2H), 5.90(s, 2H), 6.54(s, 1H), 6.94(d, J=8.4Hz, 2H), 7.07(d, J=8.4Hz, 2H), 7.52(d, J=8.1Hz, 2H), 7.96(d, J=8.1 Hz, 2H), 8.09(s, 1 H), 12.74(brs, 1H) |
| 1-179 | DMSO-d6, 300MHz | 0.80(t, J=7.1Hz, 6H), 1.02-1.15(m, 4H), 1.28(s, 9H), 1.38-1.56(m, 4H), 2.42-2.55(m, 1H), 5.09(s, 2H), 6.04(s, 2H), 6.81(s, 1H), 6.94(d, J=8.8Hz, 2H), 7.08(d, J=8.8Hz, 2H), 7.51(d, J=8.4Hz, 2H), 7.95(d, J=8.4Hz, 2H), 8.03(s, 1H) |
| 1-180 | DMSO-d6, 300MHz | 0.76(t, J = 7.3Hz, 6H), 0.99(m,4H), 1.42(m,4H), 1.9(m,2H), 2.44(m,1H), 3.27(m,3H), 3.60(t, J = 8.1Hz, 1 H), 4.04(d,J = 15.4Hz, 1H), 4.26(m,2H), 5.43(s,2H), 7.09(m,7H), 7.54(d,J = 7.0Hz, 1H), 7.70(s,1H), 8.01(s,1H), 8.11(d,J = 7.3Hz, 1H) |

**Table 17-36**

| Example | solvent, Hz | NMR(δ) |
|---|---|---|
| 1-181 | DMSO-d6, 400MHz | 0.77(t, J=7.3Hz, 6H), 1.00-1.11(m, 4H), 1.26-1.54(m, 6H), 1.77(brd, J=14.8Hz, 2H), 1.96-2.04(m, 1H), 2.39-2.45(m, 1 H), 2.80(brt, J=12.3Hz, 2H), 2.98(d, J=6.9Hz, 2H), 3.87(brd, J=13.0Hz, 2H), 5.05(s, 2H), 6.90(d, J=8.6Hz, 4H), 7.04(d, J=8.6Hz, 2H), 7.47(d, J=8.1Hz, 2H), 7.70(d, J=9.0Hz, 2H), 7.93(d, J=8.1 Hz, 2H), 7.95(s, 1H), 12.16(brs, 1H) |
| 1-182 | DMSO-d6, 300MHz | 0.76(t, J = 7.3Hz, 6H), 0.97-1.09(m,4H), 1.39-1.52(m,4H), 2.44-2.50(m,1H), 5.44(s,2H), 5.95(s,2H), 7.10(d,J = 8.4Hz, 2H), 7.17(d,J = 8.4Hz, 2H), 7.21-7.28(m,3H), 7.54(d,J = 7.0Hz, 1 H), 7.68(d,J = 7.0Hz, 1H), 7.70(s,1H), 8.03(s,1H), 8.04-8.10(m,2H), 8.29(s,1H) |
| 1-183 | DMSO-d6, 300MHz | 0.74(t, J = 7.1 Hz, 6H), 0.95-1.10(m,4H), 1.36-1.57(m,6H), 1.77-1.80(m,2H), 2.11-2.18(m,3H), 2.40-2.43(m,1H), 2.80-2.84(m,2H), 3.80(s,2H), 5.24(s,2H), 6.63(s,1H), 6.77(d,J = 7.9Hz, 2H), 6.97(d,J = 7.9Hz, 2H), 7.10-7.20(m,2H), 7.32-7.44(m,6H), 8.16(dd, J = 1.9, 9.0Hz, 1H) |
| 1-184 | DMSO-d6, 300MHz J=1.9Hz, 1H) | 1.63-1.85(m, 4H), 2.25-2.35(m, 2H), 2.52(s, 3H), 2.59-2.74(m, 1H), 3.06(d, J=12.8Hz, 2H), 3.90(s, 2H), 5.24(s, 2H), 7.09(d, J=9.0Hz, 2H), 7.42(t, J=7.7Hz, 1 H), 7.51-7.58(m, 2H), 7.70-7.83(m, 3H), 7.86-7.92(m, 3H), 8.09(d, |
| 1-185 | DMSO-d6, 300MHz | 1.10(d, J=6.8Hz, 6H), 2.71-2.92(m, 5H), 3.11(brs, 4H), 3.71-3.82(m. 6H), 4.11(s, 2H), 6.00(s, 2H), 6.96-7.12(m, 6H), 7.24(d, J=7.9Hz, 1H), 7.53(d, J=9.1 Hz, 1H), 7.61(s, 1 H), 7.71 (d, J=8.3Hz, 1 H), 8.29(s, 1H), 12.78(brs, 1H) |
| 1-186 | DMSO-d6, 300MHz | 1.11(d, J=6.4Hz, 6H), 2.66(brs, 4H), 2.72-2.83(m, 1H), 3.07-3.22(m, 8H), 3.75(brs, 4H), 3.98(s, 2H), 6.00(s, 2H), 6.95-7.23(m, 6H), 7.28-7.41(m, 3H), 7.45(s, 1H), 7.53(d, J=9.1 Hz, 1H), 8.30(s, 1H), 12.82(brs, 1H) |

**Table 17-37**

| Example | solvent, Hz | NMR(δ) |
|---|---|---|
| 2-1 | DMSO-d6, 300MHz | 0.79 (t, J = 7.3 Hz, 6H), 0.95- 1.20 (m, 4H),1.40- 1.65 (m, 4H),2.40- 2.60 (m, 1H), 3.02 (s, 3H),3.81 (s, 2H),3.93 (s, 2H),4.57 (s, 2H),6.78 (d, J = 8.8 Hz, 2H),7.00- 7.40 (m, 9H),7.55 (d, J = 8.8 Hz, 2H),8.30 (s, 1H),12.30 (br s, 1H). |

**Table 17-38**

| Example | solvent, Hz | NMR(δ) |
|---|---|---|
| 3-1 | DMSO-d6, 400MHz | 0.81(t, J = 7.3 Hz, 6H), 1.05-1.13(m, 4H), 1.48-1.57(m, 4H), 2.5-2.56(m, 1H), 5.09(s, 2H), 5.66(5, 2H), 7.09(d, J = 9.04 Hz, 2H), 7.19(d, J = 8.32 Hz, 2H), 7.38(d, J = 8.12 Hz, 2H), 7.91(d, J = 9.04 Hz, 2H), 7.98-7.99(m, 1H), 8.03(s, 1H), 8.64(d, J = 3 Hz, 1 H), 8.72(d, J = 1.4 Hz, 1H), 13.52(s, 1H) |
| 3-2 | DMSO-d6, 400MHz | 0.81(t.J=7.3Hz, 6H), 1.05-1.13(m, 4H), 1.48-1.57(m, 4H), 2.50-2.56(m, 1H), 5.09(s, 2H), 5.57(s, 2H), 7.09(d, J=9.2Hz, 2H), 7.17-7.19(m, 4H), 7.38(d, J=12.0Hz, 2H), 7.89-7.91 (m, 4H), 8.01 (s, 1H) |
| 3-3 | DMSO-d6, 400MHz | 0.81(t.J=7.3Hz, 6H), 1.05-1.13(m, 4H), 1.48-1.57(m, 4H), 2.50-2.56(m, 1H), 4.78(s, 2H), 5.08(s, 2H), 7.07(d, J=8.8Hz, 2H), 7.18(d, J=8.1 Hz, 2H), 7.37(d, J=8.0Hz, 2H), 7.51(d, J=8.0Hz, 2H), 7.82-7.86(m, 5H) |
| 3-4 | DMSO-d6, 300MHz | 1.14-1.49(m, 5H), 1.64-1.84(m, 5H), 2.44-2.54(m, 1H), 4.77(s, 2H), 5.09(s, 2H), 7.06(d, J=9.0Hz, 2H), 7.24(d, J=8.3Hz, 2H), 7.37(d. J=8.3Hz, 2H), 7.50(d, J=8.7Hz, 2H), 7.82-7.86(m, 5H) |
| 3-5 | DMSO-d6, 400MHz | 1.16-1.45(m, 5H), 1.67-1.83(m, 5H), 2.45-2.56(m, 1H), 5.07(s, 2H), 5.37(s, 2H), 7.00(d, J=9.0Hz, 2H), 7.24(d, J=8.1Hz, 2H), 7.36(d, J=8.1 Hz, 2H), 7.65(d, J=9.0Hz, 2H), 7.94-7.96(m, 3H), 8.13(d, J=8.8Hz, 2H) |
| 3-6 | DMSO-d6, 300MHz | 0.80(t, J=7.3Hz, 6H), 1.02-1.16(m, 4H), 1.46-1.60(m, 4H), 2.27(s, 3H), 2.54-2.58(m, 1H), 2.86(s, 3H), 4.68(s, 2H), 5.19(s, 2H), 7.10(d, J=0.8Hz, 2H), 7.18(d, J=8.3Hz, 2H), 7.39(d, J=8.3Hz, 2H), 7.78(s, 1H), 7.95-7.98(m, 3H), 8.13(d, J=8.3Hz, 2H), 13.39(brs, 1H) |
| 3-7 | DMSO-d6, 300MHz | 0.78(t, J=7.4Hz, 6H), 0.97-1.16(m, 4H), 1.40-1.60(m, 4H), 2.30(s, 3H), 2.51-2.56(m, 1H), 3.17(s, 3H), 5.00(s, 2H), 5.19(s, 2H), 6.85(d, J=9.0Hz, 2H), 7.10(s, 1H), 7.11(s, 1H), 7.16(d, J=7.9Hz, 2H), 7.37(d, J=8.3Hz, 2H), 7.77(d, J=8.7Hz, 2H), 7.86(s, 1H), 7.96(s, 1H), 12.16(brs, 1H) |

**Table 17-39**

| Example | solvent, Hz | NMR(δ) |
|---|---|---|
| 3-8 | DMSO-d6, 300MHz | 2.28(s, 3H), 3.36(s, 2H), 3.84(s, 2H), 4.19(s, 2H), 5.36(s, 2H), 7.07-7.08(m, 2H), 7.27-7.42(m, 5H), 7.67-7.77(m, 4H), 7.94-7.98(m, 2H) |
| 3-9 | DMSO-d6, 300MHz | 0.80(t, J=7.3Hz, 6H), 1.02-1.14(m, 4H), 1.43-1.60(m, 4H), 2.29(s, 3H), 2.51-2.57(m, 1H), 3.35(s, 2H), 3.86(s, 2H), 4.19(s, 2H), 5.19(s, 2H), 7.10-7.10(m, 2H), 7.18(d, J=8.1Hz, 2H), 7.25-7.30(m, 1H), 7.33-7.42(m, 6H), 7.91(s, 1H), 7.94(br, 1H) |
| 3-10 | DMSO-d6, 400MHz | 0.81(t.J=7.3Hz, 6H), 1.05-1.13(m, 4H), 1.48-1.57(m, 4H), 2.50-2.56(m, 1H), 3.40(s, 2H), 3.86(s, 2H), 4.17(s, 2H), 5.07(s, 2H), 7.06(d, J=9.2Hz, 2H), 7.19(d, J=6.0Hz, 2H), 7.26-7.68(m, 5H), 7.86-7.90(m, 3H) |
| 3-11 | DMSO-d6 400MHz | 0.72(t, J=7.4Hz, 6H), 1.48-1.55(m, 2H), 1.61-1.68(m, 2H), 2.30-2.33(m, 1H), 3.35(s, 2H), 3.86(s, 2H), 4.17(s, 2H), 5.08(s, 2H), 7.06(d, J=8.4Hz, 2H), 7.19(d, J=7.2Hz, 2H), 7.26-7.38(m, 7H), 7.86-7.87(m, 3H) |
| 3-12 | DMSO-d6, 300MHz | 0.86(d, J=6.6Hz, 6H), 1.29(s, 9H), 1.79-1.88(m, 1H), 2.45(d, J=7.4Hz, 2H), 3.37(s, 2H), 3.87(s, 2H), 4.20(s, 2H), 5.21(s, 2H), 7.11-7.19(m, 3H), 7.25-7.44(m, 8H), 7.93(s, 1H), 8.18(d, J=2.6Hz, 1H), 12.41 (brs, 1H) |
| 3-13 | DMSO-d6, 300MHz | 0.80(t, J=7.3Hz, 6H), 1.02-1.16(m, 4H), 1.41-1.62(m, 4H), 2.52-2.59(m, 1H), 3.36(s, 2H), 3.86(s, 2H), 4.19(s, 2H), 5.26(s, 2H), 7.18-7.42(m, 11 H), 8-00(s, 1H), 8.11 (d, J=2.9Hz, 1H), 12.41 (brs, 1H) |
| 3-14 | DMSO-d6, 400MHz | 0.72(t, J=7.4Hz, 6H), 1.48-1.55(m, 2H), 1.61-1.68(m, 2H), 2.30-2.33(m, 1H), 3.37(s, 2H), 3.85(s, 2H), 4.16(s, 2H), 5.08(s, 2H), 7.06(d, J=8.8Hz, 2H), 7.18-7.28(m, 4H), 7.36-7.42(m, 4H), 7.86-7.89(m, 3H) |

**Table 17-40**

| Example | solvent, Hz | NMR(δ) |
|---|---|---|
| 3-15 | DMSO-d6, 300MHz | 0.72(t, J=7.4Hz, 6H), 1.19(d, J=6.9Hz, 6H), 1.45-1.53(m, 2H), 1.63-1.66(m, 2H), 2.33-2.36(m, 1H), 2.85-2.88(m, 1H), 3.35(s, 2H), 3.83(s, 2H), 4.16(s, 2H), 5.09(s, 2H), 7.07(d, J=11.2Hz, 2H), 7.17-7.23(m, 4H), 7.31 (d, J=8.1Hz, 2H), 7.39(d, J=8.1 Hz, 2H), 7.85-7.87(m, 3H) |
| 3-16 | DMSO-d6, 400MHz | 0.72(t, J=7.4Hz, 6H), 1.45-1.53(m, 2H), 1.63-1.66(m, 2H), 2.30-2.34(m, 1H),, 3.40(s, 2H), 3.97(s, 2H), 4.30(s, 2H), 5.09(s, 2H), 7.07(d, J=8.1 Hz, 2H), 7.08(d, J=8.0Hz, 2H), 7.18(d, J=8.0Hz, 2H), 7.38(d, J=8.0Hz, 2H), 7.65(d, J=8.0Hz, 2H), 7.72(d, J=8.0Hz, 2H), 7.84-7.87(m, 3H) |
| 3-17 | DMSO-d6, 400MHz | 0.72(t, J=7.4Hz, 6H), 1.45-1.53(m, 2H), 1.63-1.66(m, 2H), 2.30-2.34(m, 1H), 3.37(s, 2H), 3.85(s, 2H), 4.30(s, 2H), 5.09(s, 2H), 7.08(d, J=8.0Hz, 2H), 7.19(d, J=8.0Hz, 2H), 7.37-7.44(m, 6H), 7.86-7.90(m, 3H) |
| 3-18 | DMSO-d6, 400MHz | 0.72(t, J=7.4Hz, 6H), 1.45-1.53(m, 2H), 1.63-1.66(m, 2H), , 2.26(s, 6H), 2.30-2.40(m, 1H), 3.48(s, 2H), 3.89(s, 2H), 4.25(s, 2H), 5.09(s, 2H), 6.92(s, 1 H), 7.03-7.08(m, 4H), 7.20(d, J=8.0Hz, 2H), 7.38(d, J=8.0Hz, 2H), 7.86-7.91(m, 3H) |
| 3-19 | DMSO-d6, 400MHz | 0.81(t.J=7.3Hz, 6H), 1.05-1.13(m, 4H), 1.48-1.57(m, 4H), 2.50-2.56(m, 1H),3.45(s, 2H), 4.00(s, 2H), 4.23(s, 2H), 5.07(s, 2H), 7.06(d, J=9.2Hz, 2H), 7.19(d, J=5.6Hz, 2H), 7.36-7.37(m, 1 H), 7.35-7.37(m, 2H), 7.57-7.60(m, 1H), 7.81-7.85(m, 4H), 8.48-8.49(m, 1H) |
| 3-20 | DMSO-d6, 300MHz | 0.72(t, J=7.4Hz, 6H), 1.48-1.55(m, 2H), 1.61-1.68(m, 2H), 2.30-2.33(m, 1H), 3.46(s, 2H), 4.02(s, 2H), 4.23(s, 2H), 5.09(s, 2H), 7.07(d, J=11.6Hz, 2H), 7.18(d, J=8.1 Hz, 2H), 7.26-7.29(m, 1 H), 7.39(d, J=9.0Hz, 2H), 7.57(d, J=9.0Hz, 1H), 7.80-7.87(m, 4H), 8.50(d, J=3.9Hz, 1H) |

**Table 17-41**

| Example | solvent, Hz | NMR(δ) |
|---|---|---|
| 3-21 | DMSO-d6 300MHz | 0.80(t, J=7.2Hz, 6H), 0.99-1.19(m, 4H), 1.42-1.62(m, 4H), 2.29(s, 3H), 2.46-2.58(m, 1H), 3.45(s, 2H), 4.01(s, 2H), 4.24(s, 2H), 5.19(s, 2H), 7.10(s, 2H), 7.18(d, J=8.1Hz, 2H), 7.25-7.30(m, 1 H), 7.39(d, J=8.1Hz, 2H), 7.57(d, J=7.8Hz, 1H), 7.80(dt, J=1.9, 7.8Hz, 1H), 7.90(s, 1H), 7.93(s, 1H), 8.49-8.51(m, 1H) |
| 3-22 | DMSO-d6, 400MHz | 0.81(t.J=7.3Hz, 6H), 1.05-1.13(m, 4H), 1.48-1.57(m, 4H), 2.50-2.56(m, 1H), 4.09(s, 2HX0.55),4.21(s,2HXO.45), 4.80(s,2HX0.45),4.97(s,2HX0.55), 5.09(s, 2H), 7.08(d, J=9.2Hz, 2H), 7.20(d, J=8.0Hz, 2H), 7.36-7.38(m, 3H), 7.46-7.48(m, 4H), 7.87-7.89(m, 3H) |
| 3-23 | DMSO-d6, 300MHz | 0.72(t, J=7.4Hz, 6H), 1.45-1.53(m, 2H), 1.63-1.66(m, 2H), 2.33-2.36(m, 4H), 4.10(s, 1H), 4.20(s, 1H), 4.82(s, 1 H), 4.94(s, 1 H), 5.10(s, 2H), 7.09(d, J=9.0Hz, 2H), 7.18(d, J=8.1Hz, 2H), 7.26-7.28(m, 3H), 7.37-7.40(m, 3H), 7.86-7.91(m, 3H) |
| 3-24 | DMSO-d6, 400MHz | 0.81(t.J=7.3Hz, 6H), 1.05-1.13(m, 4H), 1.48-1.57(m, 4H), 2.50-2.56(m, 1H),3.78(s, 3H), 4.11(s, 2H), 4.86-4.92(m, 2H), 5.08(s, 2H), 7.02(d, J=8.0Hz, 2H), 7.10(d, J=8.0Hz, 2H), 7.20(d, J=4.0Hz, 2H), 7.36-7.41(m, 4H), 7.86-7.96(m, 3H) |
| 3-25 | DMSO-d6, 300MHz | 0.79(t, J=7.4Hz, 6H), 0.96-1.17(m, 4H), 1.41-1.62(m, 4H), 2.29(s, 3H), 2.46-2.57(m, 1H), 3.05-3.22(m, 2H), 3.91(d, J=15.5Hz, 1H), 3.95-3.99(m, 1H), 4.09(d, J=15.5Hz, 1H), 4.26(d, J=15.5Hz, 1H), 4.40(d, J=15.5Hz, 1 H), 5.20(s, 2H), 7.02-7.19(m, 8H), 7.39(d, J=8.3Hz, 2H), 7.92(s, 1H) |

**Table 17-42**

| Example | solvent, Hz | NMR(δ) |
|---|---|---|
| 3-26 | DMSO-d6, 400MHz | 0.81(t, J=7.3Hz, 6H), 1.04-1.15(m, 4H), 1.46-1.59(m, 4H), 2.52-2.57(m, 1H), 3.10(dd, J=3.5, 16.2Hz, 1H), 3.17(dd, J=6.0, 16.4Hz, 1 H), 3.92(d, J=15.8Hz, 1 H), 3.94-3.97(m, 1 H), 4.09(d, J=15.8Hz, 1H), 4.25(d, J=15.8Hz, 1H), 4.39(d, J=15.8Hz, 1 H), 5.08(s, 2H), 7.02-7.16(m, 6H), 7.18(d, J=8.1 Hz, 2H), 7.38(d, J=8.1 Hz, 2H), 7.86(s, 1H), 7.87(d, J=8.8Hz, 2H) |
| 3-27 | DMSO-d6, 400MHz | 0.84 (s, 9 H), 2.42 (s, 3 H), 3.03 (s, 3 H), 3.35 (s, 2 H), 3.85 (s, 2 H), 4.15 (s, 2 H), 4.58 (s, 2 H), 6.76 (d, J = 8.8 Hz, 2 H), 7.05 (d, J = 7.9 Hz, 2 H), 7.11 (d, J = 8.1 Hz, 2 H), 7.26 (t, J = 6.9 Hz, 1 H), 7.34 (t, J = 7.4 Hz, 2 H), 7.39 (d, J = 7.0 Hz, 2 H), 7.65 (s, 1 H), 7.70 (d, J = 8.8 Hz, 2H), 12.37 (br s, 1H). |
| 3-28 | DMSO-d6, 300MHz | 1.18-1.20(m,2H), 1.24(s,9H), 1.55-1.76(m,2H), 2.28-2.47(m,1H), 2.98(s,3H), 3.25(d,J = 6.0Hz, 2H), 3.36(s,2H), 3.87(s,2H), 4.16(s,2H), 6.72(d,J = 9.0Hz, 2H), 7.12(d,J = 9.0Hz, 2H), 7.23-7.28(m,3H), 7.31-7.44(m,4H), 7.65(s,1H), 7.73(d,J = 9.0Hz, 2H) |
| 3-29 | DMSO-d6, 300MHz | 1.20-1.45(m, 5H), 1.65-1.80(m, 5H), 1.40-1.45(m, 1H), 3.03(s, 3H), 3.35(s, 2H), 3.86(s, 2H), 4.15(s, 2H), 4.56(s, 2H), 6.76(d, J=12.0Hz, 2H), 7.09-7.16(m, 4H), 7.26-7.39(m, 5H), 7.66-7.72(m, 3H) |
| 3-30 | DMSO-d6, 300MHz | 1.20-1.40(m, 5H), 1.60-1.80(m, 5H), 2.45-2.50(m, 1H), 2.51(t, J=6.0Hz, 2H), 2.77(t, J=6.0Hz, 2H), 3.02(s, 3H), 3.69(s, 2H), 3.92(s, 2H), 4.56(s, 2H), 6.75(d, J=9.0Hz, 2H), 7.09-7.16(m, 4H), 7.31-7.41(m. 5H), 7.64(s, 1H), 7.71(d, J=9.0Hz, 2H) |
| 3-31 | DMSO-d6, 400MHz | 0.84 (s, 9 H),2.42 (s, 2 H),2.87 (s, 3 H),3.31 (s, 2 H),3.35 (s, 2 H),3.86 (s, 2 H),4.15 (s, 2 H),6.72 (d, J = 9.0 Hz, 2 H),7.03 (d, J = 7.9 Hz, 2 H),7.14 (d, J = 7.9 Hz, 2 H),7.23- 7.29 (m, 1 H),7.32- 7.44 (m, 4 H),7.65 (s, 1 H),7.72 (d, J = 9.0 Hz, 2 H),12.40 (br s, 1 H). |

**Table 17-43**

| Example | solvent, Hz | NMR(δ) |
|---|---|---|
| 3-32 | DMSO-d6, 300MHz | 0.89(d, J=6.5Hz, 3H), 1.01-1.05(m, 2H), 1.30-1.45(m, 3H), 1.70-1.75(m, 4H), 3.35(s, 2H), 3.86(s, 2H), 4.15(s, 2H), 4.55(s, 2H), 6.76(d, J=9.0Hz, 2H), 7.09-7.16(m, 4H), 7.26-7.29(m, 1H), 7.35-7.41(m, 4H), 7.65(s, 1H), 7.71 (d, J=8.9Hz, 2H) |
| 3-33 | DMSO-d6, 300MHz | 1.55-1.64(m,6H), 1.92-2.03(m,2H), 2.50-2.55(m,1H), 3.35(s,2H), 3.86(s,2H), 4.15(s,2H), 4.57(s,2H), 4.83(d,J = 15.0Hz, 1H), 6.77(d,J = 9.0Hz, 2H), 7.13-7.16(m,4H), 7.32-7.39(m,4H), 7.65(s,1H), 7.71(d,J = 8.8Hz, 2H) |
| 3-34 | DMSO-d6, 400MHz | 1.12-1.21(m,2H), 1.32-1.43(m,2H), 1.78(d,J = 12.0Hz, 4H), 2.40-2.50(m,2H), 2.96(s,3H), 3.24(d,J = 4.0Hz, 2H), 3.35(s,2H), 3.85(s,2H), 4.14(s,2H), 6.69(d,J = 8.0Hz, 2H), 7.63(s,1H), 7.70(d,J = 8.0Hz, 2H) |
| 3-35 | DMSO-d6, 300MHz | 0.92(s, 3H), 0.94(s, 3H), 1.23-1.63(m, 8H), 2.33-2.40(m, 1H), 3.02(s, 3H), 3.35(s, 2H), 3.85(s, 2H), 4.14(s, 2H), 4.55(s, 2H), 6.75(d, J=6.7Hz, 2H), 7.10(d, J=6.2Hz, 2H), 7.17(d, J=6.0Hz, 2H), 7.26(t, J=5.3Hz, 1H), 7.32-7.40(m, 4H), 7.64(s, 1H), 7.70(d, J=6.7Hz, 2H), 12.39(brs, 1H) |
| 3-36 | DMSO-d6, 300MHz | 0.78 (t, J = 7.3 Hz, 6 H), 1.00- 1.18 (m, 4 H), 1.38- 1.59 (m, 4 H), 2.40- 2.58 (m, 1 H), 3.03 (s, 3 H), 3.32 (s, 2 H), 3.86 (s, 2 H), 4.15 (s, 2 H), 4.57 (s, 2 H), 6.77 (d, J = 8.9 Hz, 2 H), 7.06- 7.15 (m, 4 H), 7.21- 7.44 (m, 5 H), 7.66 (s, 1 H), 7.72 (d, J = 8.8 Hz, 2 H). |
| 3-37 | DMSO-d6 300MHz | 3.08(s, 3H), 3.35(s, 2H), 3.86(s, 2H), 4.15(s, 2H), 4.66(s, 2H), 6.79(d, J=8.6Hz, 2H), 7.22-7.46(m, 10H), 7.60-7.65(m, 4H), 7.67(s, 1H), 7.73(d, J=9.0Hz, 2H) |

**Table 17-44**

| Example | solvent, Hz | NMR(δ) |
|---|---|---|
| 3-38 | DMSO-d6, 300MHz | 1.14-1.43(m, 5H), 1.63-1.82(m, 5H), 2.38-2.49(m, 1H), 2.94(s, 2H), 3.02(s, 3H), 3.90(s, 2H), 4.19(s, 2H), 4.55(s, 2H), 6.75(d, J=9.0Hz, 2H), 7.11(d, J=8.3Hz, 2H), 7.15(d, J=8.3Hz, 2H), 7.21(t, J=7.2Hz, 1 H), 7.31(t, J=7.3Hz, 2H), 7.39(d, J=7.2Hz, 2H), 7.58(s, 1H), 7.70(d, J=9.0Hz, 2H) |
| 3-39 | DMSQ-d6 400MHz | 0.85(d, J=6.8Hz, 6H), 1.75-1.81(m, 1H), 2.39(d, J=6.8Hz, 2H), 3.02(s, 3H), 3.34(s, 2H), 3.85(s, 2H), 4.14(s, 2H), 4-56(s, 2H), 6.76(d, J=8.0Hz, 2H), 7.08-7.10(m, 4H), 7.20-7.25(m, 1H), 7.33-7.40(m, 4H), 7.64(s, 1H), 7.69(d, J=8.0Hz, 2H) |
| 3-40 | DMSO-d6, 400MHz | 0.78(t, J=7.3Hz, 6H), 0.99-1.15(m, 4H), 1.40-1.56(m, 4H), 2.43-2.52(m, 1H), 2.93(s, 2H), 3.02(s, 3H), 3.90(s, 2H), 4.19(s, 2H), 4.56(s, 2H), 6.75(d, J=9.1Hz, 2H), 7.08(d, J=8.3Hz, 2H), 7.11(d, J=8.3Hz, 2H), 7.20(t, J=7.3Hz, 1H), 7.30(t, J=7.3Hz, 2H), 7.38(d, J=7.3Hz, 2H), 7.58(s, 1H), 7.69(d, J=9.1Hz, 2H) |
| 3-41 | DMSO-d6, 300MHz | 0.97 (s, 9H), 2.89 (s, 2H), 3.02 (s, 3H), 3.86 (s, 2H), 4.15 (s, 2H), 4.56 (s, 2H), 6.75 (d, J = 8.87 Hz, 2H), 7.13 (d, J = 8.25 Hz, 2H), 7.25-7.40 (m, 7H), 7.67 (s, 1H), 7.71 (d, J = 8.87, 2H) |
| 3-42 | DMSO-d6, 400MHz | 0.86 (d, J = 6.72, 6H), 1.40-1.46 (m, 2H), 1.61-1.71 (m, 1H), 2.90 (d, J = 7.64 Hz, 1H), 2.92 (d, J = 7.64 Hz, 1H), 3.03 (s, 3H), 3.85 (s, 2H), 4.15 (s, 2H), 4.57 (s, 2H), 6.74 (d, J = 9.04 Hz, 2H), 7.14 (d, J = 8.36 Hz, 2H), 7.24-7.28 (m, 3H), 7.32-7.40 (m, 4H), 7.66 (s, 1H), 7.70 (d, J = 9.04, 2H) |
| 3-43 | DMSQ-d6 300MHz | 0.81 (t, J = 7.2 Hz, 6 H), 1.44 (tq, J = 7.8, 7.2 Hz, 4 H), 3.19 (dd, J = 7.8, 7.8 Hz, 4 H), 3.34 (s, 3 H), 3.36 (s, 2 H), 3.86 (s, 2 H), 4.17 (s, 2 H), 6.40 (d, J = 8.7 Hz, 2 H), 7.12 (d, J = 9.0 Hz, 2 H), 7.17 (d, J = 8.7 Hz, 2 H), 7.21-7.43 (m, 5 H), 7.84 (d, J = 9.0 Hz, 2 H), 8.00 (s, 1 H), 12.38 (br s, 1 H). |

**Table 17-45**

| Example | solvent, Hz | NMR(δ) |
|---|---|---|
| 3-44 | DMSO-d6, 300MHz | 1.12-1.20(m,15H), 2.80-2.89(m,2H), 3.24(s,2H), 3.15-3.52(m,2H), 3.82(s,2H), 4.15(s,2H), 4.52(s,2H), 6.70(d,J = 9.0Hz, 2H), 7.13-7.22(m,8H), 7.31(d,J = 9.0Hz, 2H), 7.61(s,1H), 7.68(d,J = 9.0Hz, 2H) |
| 3-45 | DMSO-d6, 300MHz | 1.15-1.19(m,18H), 2.76-2.89(m,1H), 3.79(s,2H), 4.11(s,2H), 4.27-4.33(m,1H), 4.38(s,2H), 6.69(d,J = 9.0Hz, 2H), 7.15-7.32(m,8H), 7.60-7.66(m,3H) |
| 3-46 | DMSO-d6, 300MHz | 1.14(d,J = 6.0Hz, 6H), 1.17(s,9H), 1.24(d,J = 6.0Hz, 6H), 2.74-2.90(m,1H), 3.79(s,2H), 4.12(s,2H), 4.23-4.36(m,1H), 4.38(s,2H), 6.68(d,J = 6.0Hz, 2H), 7.14-7.17(m,3H), 7.27-7.42(m,4H), 7.60-7.72(m,3H) |
| 3-47 | DMSO-d6, 400MHz | 0.82(d, J=19.6Hz, 6H), 1.75-1.82(m, 1H), 2.40(d, J=14.4Hz, 2H), 3.03(s, 3H), 3.34(s, 2H), 3.84(s, 2H), 4.26(s, 2H), 4.56(s, 2H), 6.77(d, J=8.0Hz, 2H), 7.08-7.11(m, 4H), 7.40-7.41 (m, 4H), 7.64(s, 1H), 7.69(d, J=8.0Hz, 2H) |
| 3-48 | DMSO-d6, 300MHz | 0.79(t, J = 6.0Hz, 6H), 1.04-1.13(m,4H), 1.48-1.56(m,4H), 2.49-2.51 (m,1H), 3.05(s,3H), 3.44(m,2H), 3.91(s,2H), 4.22(s,2H), 4.62(s,2H), 6.78(d,J = 9.0Hz, 1H), 7.15(d,J = 9.0Hz, 2H), 7.20-7.24(m,3H), 7.30-7.35(m,5H), 7.69-7.74(m,2H) |
| 3-49 | DMSO-d6, 300MHz | 3.05(s,3H), 3.36(s,2H), 3.85(s,2H), 4.14(s,2H), 4.61(s,2H), 6.74-6.79(m,2H), 7.20-7.42(m,9H), 7.66(s,1H), 7.72(d,J = 6.0Hz, 2H) |
| 3-50 | DMSO-d6, 300MHz | 3.04(s,3H), 3.42(s,2H), 4.02(s,2H), 4.22(s,2H), 4.61(s,2H), 6.76(d,J = 6.0Hz, 2H), 7.20-7.44(m,8H), 7.64-7.72(m,4H) |
| 3-51 | DMSO-d6, 300MHz | 3.05(s,3H), 3.40(s,2H), 3.87(s,2H), 4.13(s,2H), 4.62(s,2H), 6.77(d,J = 9.0Hz, 2H), 7.20-7.45(m,6H), 7.58-7.74(m,6H) |

**Table 17-46**

| Example | solvent, Hz | NMR(δ) |
|---|---|---|
| 3-52 | DMSO-d6, 300MHz | 0.89(d, J=6.0Hz, 3H), 1.00-1.80(m, 2H), 1.33-1.46(m, 3H), 1.72-1.75(m, 4H), 2.35-2.43(m, 1H), 2.95(s, 2H), 3.02(s, 3H), 4.10(s, 2H), 4.22(s, 2H), 4.55(s, 2H), 6.74(d, J=9.1 Hz, 2H), 7.09-7.11 (m, 4H), 7.16-7.22(m, 1 H), 7.57-7.79(m, 5H), 8.45(d, J=4.1 Hz, 2H) |
| 3-53 | DMSO-d6, 300MHz | 0.79(t, J=7.3Hz, 6H), 0.97-1.16(m, 4H), 1.36-1.59(m, 4H), 2.43-2.53(m, 1H), 3.03(s, 3H), 3.44(s, 2H), 4.00(s, 2H), 4.21(s, 2H), 4.57(s, 2H), 6.77(d, J=9.1Hz, 2H), 7.09(d, J=8.7Hz, 2H), 7.12(d, J=8.7Hz, 2H), 7.28(dd, J=7.5, 4.5Hz, 1 H), 7.57(d, J=7.5Hz, 1 H), 7.65(s, 1 H), 7.71(d, J=9.1Hz, 2H), 7.81(dt, J=1.9, 7.5Hz, 1H), 8.50(dd, J=4.5, 1.9Hz, 1H) |
| 3-54 | DMSO-d6, 300MHz | 3.04(s,3H), 3.39(s,2H), 4.21(s,2H), 4.36(s,2H), 4.61(s,2H), 6.76(d,J = 9.0Hz, 2H), 7.20-7.35(m,5H), 7.44-7.72(m,9H), 7.85-7.91(m,2H),8.41-8.444(m,1H) |
| 3-55 | DMSO-d6, 300MHz | 3.05(s,3H), 3.51(s,2H), 4.22(s,2H), 4.27(s,2H), 4.61(s,2H), 6.76(d,J = 9.0Hz, 2H), 7.23(t, J = 9.0Hz, 3H), 7.32(t, J = 9.0Hz, 2H), 7.57-7.82(m,7H), 7.98(t, J = 6.0Hz, 2H), 8.42(d,J = 9.0Hz, 1H) |
| 3-56 | DMSO-d6, 300MHz | 3.06(s,3H), 3.96(s,1H), 4.13(d,J = 12.0Hz, 2H), 4.44(s,1H), 4.63(s,2H), 4.82(s,1H), 5.10(s,1H), 6.78(d,J = 9.0Hz, 2H), 7.21-7.37(m,7H), 7.52(d,J = 6.0Hz, 1H), 7.66-7.76(m,4H), 7.96-7.98(m,1H) |
| 3-57 | DMSO-d6, 400MHz | 0.84(t.J=7.3Hz, 6H), 1.73-1.78(m, 1H), 2.40(d, J=7.2Hz, 2H), 3.03(s, 3H), 3.70(s, 1H), 3.90(s, 1H), 4.07(s, 1H), 4.34(s, 1H), 4.57(s, 2H), 4.78(s, 1H), 5.02(s, 1H), 6.75-6.77(m, 2H), 7.10-7.12(m, 4H), 7.24-7.27(m, 2H), 7.33-7.36(m, 2H), 7.64-7.71 (m, 3H) |
| 3-58 | DMSO-d6, 400MHz | 0.846(t, J=7.4H, 6H), 1.18(t, J=8.4Hz, 6H), 1.76-1.81(m, 1H), 2.40(d, J=6.8Hz, 2H), 2.82-2.86(m, 1 H), 3.03(s, 3H), 3.63(s, 1H), 3.83(s, 1H), -4.06(s, 1H), 4.30(s, 1H), 4.55(s, 2H), 4.78(s, 1H), 4.99(s, 1H), 6.75-6.77(m, 2H), 7.07-7.16(m, 8H), 7.64-7.72(m, 3H) |

**Table 17-47**

| Example | solvent, Hz | NMR(δ) |
|---|---|---|
| 3-59 | DMSO-d6, 400MHz | 0.82-0.90(m, 12H), 1.74-1.81 (m, 1H), 2.02-2.04(m. 1H), 2.15(d, J=8.0Hz, 1H), 2.33(d, J=8.0Hz, 1 H), 2.39(d, J=7.0Hz, 2H), 3.02(s, 3H), 4.03(s, 1H), 4.24(s, 1H), 4.56(s, 2H), 4.74(s, 1H), 4.91(s, 1H), 6.72-6.76(m, 2H), 7.05-7.11(m, 4H), 7.61-7,70(m, 3H) |
| 3-60 | DMSO-d6, 300MHz | 0.85 (s, 9 H), 2.43 (s, 2 H), 3.04 (s, 3 H), 4.21 (s, 2 H), 4.59 (s, 2 H), 4.90 (s, 2 H), 6.77 (d, J = 8.9 Hz, 2 H), 6.96 (d, J = 7.3 Hz, 1 H), 7.07 (d, J = 8.2 Hz, 2 H), 7.13 (d, J = 8.1 Hz, 2 H), 7.24 (dd, J = 7.9, 7.9 Hz, 2 H), 7.45 (d, J = 7.6 Hz, 2 H), 7.67 (s, 1 H), 7.73 (d, J = 8.8 Hz, 2 H), 8.79 (s, 1 H), 12.58 (br s, 1 H). |
| 3-61 | DMSO-d6, 400MHz | 0.83(d, J=12.0Hz, 6H), 1.75-1.82(m, 1H), 2.40(d, J=16.0Hz, 2H), 3.03(s, 3H), 4.07(s, 1H), 4.19(s, 1H), 4.57(s, 2H), 4.77(s, 1H), 4.93(s, 1H), 6.76(d, J=8.0Hz, 2H), 7.09-7.12(m, 4H), 7.36-7.37(m, 1H), 7.45-7.48(m, 4H), 7.67-7.71 (m, 3H) |
| 3-62 | DMSO-d6, 400MHz | 0.81(t.J=7.3Hz, 6H), 1.05-1.13(m, 4H), 1.48-1.57(m, 4H),2.31(s, 3H), 2.50-2.56(m, 1H), 3.00(s, 3H), 3.92(s, 2HX0.58), 4.15(s, 2HX0.42), 4.57 (s, 2H), 4.78 (s,2HX0.42), 4.90(s, 2HXO.58), 6.77(d, J=8.0Hz, 2H), 7.07-7.13(m, 4H), 7.23-7.39(m, 4H), 7.69-7.73(m, 3H) |
| 3-63 | DMSO-d6, 400MHz | 0.81(t.J=7.3Hz, 6H), 1.05-1.13(m, 4H), 1.21(d, J=8.0Hz, 6H), 1.42-1.52(m, 4H), 2.48-2.50(m, 1H), 2.89-2.93(m, 1H), 3.20(s, 3H), 3.45(s, 2H), 4.57(s, 2H), 4.88(s, 2H), 6.77(d, J=8.0Hz, 2H), 7.08-7.13(m, 4H), 7.26(d, J=8.0Hz, 2H), 7.40(d, J=8.0Hz, 2H), 7.66(s, 1H), 7.73(d, J=8.0Hz, 2H) |
| 3-64 | DMSO-d6, 300MHz | 0.79(t, J = 9.0Hz, 6H), 1.02-1.12(m,4H), 1.43-1.54(m,4H), 2.96-2.06(m,5H), 3.78(d,J = 15.0Hz, 1H), 4=16(d,J = 15.0Hz, 1H), 4.56(s,2H), 4.72-4.76(m,1H), 6.76(d,J = 9.0Hz, 2H), 7.05-7.12(m,7H), 7.48(s,1H), 7.68(d,J = 9.0Hz, 2H) |

**Table 17-48**

| Example | solvent, Hz | NMR(δ) |
|---|---|---|
| 3-65 | DMSO-d6, 300MHz | 1.15-1.86(m, 13H), 2.12-2.29(m, 3H), 2.39-2.48(m, 1H), 2.82-2.91(m, 2H), 3.03(s, 3H), 3.81(s, 2H), 4.56(s, 2H), 6.75(d, J=9.1Hz, 2H), 7.11 (d, J=8.4Hz, 2H), 7.16(d, J=8.4Hz, 2H), 7.64(s, 1H), 7.71(d, J=8.8Hz, 2H), 12.11(brs, 1H) |
| 3-66 | DMSO-d6, 300MHz | 1.20-1.82(m, 14H), 2.00-2.12(m, 2H), 2.16-2.20(m, 1H), 2.45-2.50(m, 1H), 2.78-2.82(m, 1H), 2.90-2.95(m, 1H), 3.02(s, 3H), 3.78(s, 2H), 4.55(s, 2H), 6.75(d, J=9.0Hz, 2H), 7.09-7.16(m, 4H), 7.62(s, 1 H), 7.70(d, J=12.0Hz, 2H) |
| 3-67 | DMSO-d6, 300MHz | 1.20-1.40(m, 5H), 1.60-1.80(m, 7H), 2.20-2.50(m, 5H), 2.80-2.90(m, 2H), 3.03(s, 3H), 3.81(s, 2H), 4.56(s, 2H), 6.75(d, J=9.0Hz, 2H), 7.09-7.14(m, 4H), 7.25-7.28(m, 1H), 7.35-7.42(m, 4H), 7.63(s, 1H), 7.70(d, J=8.9Hz, 2H) |
| 3-68 | DMSO-d6, 300MHz | 0.84(d, J=6.6Hz, 6H), 1.02-1.50(m, 1.2H), 1.65-1.81(m, 5H), 1.95-2.04(m, 2H), 2.15-2.26(m, 2H), 2.39-2.49(m, 1H), 2.69-2.79(m, 2H), 3.03(s, 3H), 3.78(s, 2H), 4.56(s, 2H), 6.75(d, J=9.1 Hz, 2H), 7.10-7.17(m, 4H), 7.63(s, 1H), 7.70(d, J=8.8Hz, 2H) |
| 3-69 | DMSO-d6, 300MHz | 0.89(d, J=6.0Hz, 3H), 1.00-1.10(m, 2H), 1.30-1.40(m, 3H), 1.72-1.95(m, 6H), 2.30-2.50(m, 5H), 2.80-2.90(m, 2H), 3.03(s, 3H), 3.81(s, 2H), 4.56(s, 2H), 6.75(d, J=9.0Hz, 2H), 7.09-7.17(m, 4H), 7.25-7.30(m, 1H), 7.32-7.37(m, 4H), 7.64(s, 1H), 7.70(d, J=11.7Hz, 2H) |
| 3-70 | DMSO-d6, 300MHz | 1.08-1.19(m,2H), 1.30-1.44(m,2H), 1.57-1.67(m,2H), 2.35-2.44(m,2H), 2.75(d,J = 8.0Hz, 2H), 2.96(s,3H), 3.25-3.30(m,4H), 3.74(s,2H), 6.69(d,J = 8.0Hz, 2H), 7.08(t, J = 4.0Hz, 1H), 7.18-7.23(m,5H), 7.28(d,J = 8.0Hz, 2H), 7.37(d,J = 8.0Hz, 2H), 7.59(s,1H), 7.70(d,J = 12.0Hz, 2H) |

**Table 17-49**

| Example | solvent, Hz | NMR(δ) |
|---|---|---|
| 3-71 | DMSO-d6, 300MHz 7.64(s, | 0.89(d, J=4.8Hz, 3H), 1.01-1.10(m, 2H), .1.30-1.50(m, 3H), 1.70-1.80(m, 4H), 3.10-3.20(m, 1H), 3.10-3.20(m, 2H), 3.88-4.11 (m, 3H), , 4.30(dd, J=37.7, 11.6Hz, 2H), 4.56(s, 2H), 6.76(d, J=9.0Hz, 2H), 7.04-7.17(m, 8H), 1H), 7.72(d, J=8.8Hz, 2H) |
| 3-72 | DMSO-d6 300MHz | 0.84(d, J=6.8Hz, 6H), 1.70-1.80(m, 1H), 2.39(d, J=4.4Hz, 2H), 3.03(s, 3H), 3.10-3.20(m, 2H), 3.89-4.06(m, 3H), 4.22(d, J=15.6Hz, 1H), 4.36(d, J=15.6Hz, 1H), 4.57(s, 2H), 6.75(d, J=10.8Hz, 2H), 7.03-7.15(m, 8H), 7.64(s, 1H), 7,71 (d, J=10.0Hz, 2H) |
| 3-73 | DMSO-d6, 300MHz | 0.79(t, J=7.4Hz, 6H), 1.01-1.14(m, 4H), 1.39-1.56(m, 4H), 2.43-2.48(m, 1H), 3.03(s, 3H), 3.11-3.16(m, 2H), 4.00(ABq, J=15.6,41.6Hz, 2H), 3.95-3.97(m, 1H), 4.30(ABq, J=15.8, 50.1 Hz, 2H), 4.57(s, 2H), 6.77(d, J=9.1Hz, 2H), 7.02-7.16(m, 8H), 7.65(s, 1H), 7.73(d, J=8.6Hz, 2H), 12.54(brs, 1H) |
| 3-74 | DMSO-d6, 300MHz | 0.85 (s, 9 H), 2.43 (s, 2 H), 3.04 (s, 3 H), 3.10- 3.20 (m, 2 H), 3.92 (d, J = 16.1 Hz, 1 H), 3.90- 4.00 (m, 1 H), 4.10 (d, J = 7.7 Hz, 1 H), 4.23 (d, J = 15.7 Hz, 1 H), 4.37 (d, J = 15.7 Hz, 1 H), 4.59 (s, 2 H), 6.77 (d, J = 8.9 Hz, 2 H), 7.00- 7.20 (m, 8 H), 7.66 (s, 1 H), 7.72 (d, J = 8.8 Hz, 2 H), 12.50 (br s, 1 H). |
| 3-75 | DMSO-d6, 400MHz | 1.21-1.34(m, 5H), 1.69-1.74(m, 5H), 2.44-2.54(m, 1H), 3.02(s, 3H), 3.70(s, 3H), 4.55(s, 2H), 4.77(s, 2H), 6.54(s, 1H), 6.71 (d, J=8.0Hz, 2H), 6.87(s, 1H), 7.11-7.14(m, 5H), 7.64(d, J=8.0Hz, 2H) |
| 3-76 | DMSO-d6 300MHz | 1.20-1.45(m, 5H), 1.60-1.85(m, 5H), 2.45-2.50(m, 1H), 2.80-3.00(m, 2H), 3.35-3.38(m, 1H), 3.83(d, J=15.0Hz, 1H), 4.09(d, J=15.0Hz, 1H), 4.55(s, 2H), 6.74(d, J=9.0Hz, 2H), 7.09-7.25(m, 9H), 7.53(s, 1H), 7.67(d, J=9.0Hz, 2H) |

**Table 17-50**

| Example | solvent, Hz | NMR(δ) |
|---|---|---|
| 3-77 | DMSO-d6, 400MHz | 1.21-1.40(m, 5H), 1.69-1.80(m, 5H), 2.44-2.50(m, 1H), 3.90-3.96(m, 2H), 4.34(s, 1 H), 4.55(s, 2H), 6.75(d, J=8.8Hz, 2H), 7.12-7.14(m, 4H), 7.28-7.40(m, 5H), 7.60(s, 1H), 7.69(d, J=8.3Hz, 2H) |
| 3-78 | DMSO-d6, 400MHz | 1.14(d, J=12.0Hz, 3H), 1.20-1.40(m, 5H), 1.65-1.80(m, 5H), 2.44-2.50(m, 1H), 3.05-3.08(m, 1H), 3.85(d, J=16.0Hz, 1H), 4.09(d, J=16.0Hz, 1H), 4.75(s, 2H), 6.74(d, J=8.0Hz, 2H), 7.09-7.15(m, 4H), 7.57(s, 1H), 7.70(d, J=8.8Hz, 2H) |
| 3-79 | DMSO-d6, 400MHz | 0.83(d, J=14.0Hz, 6H), 1.78-1.90(m, 1H), 2.39(d, J=5.6Hz, 2H), 2.82-3.02(m, 2H), 3.02(s, 3H), 3.44-3.45(m, 1H), 3.87(d, J=16.0Hz, 1H), 4.11 (d, J=16.0Hz, 1 H), 4.56(s, 2H), 6.73(d, J=8.0Hz, 2H), 7.08-7.11 (m, 4H), 7.27-7.30(m, 4H), 7.55(s, 1 H), 7.67(d, J=10.8Hz, 2H) |
| 3-80 | DMSO-d6, 300MHz | 1.08-1.35(m, 5H), 1.60-1.77(m, 5H), 2.44-2.54(m, 1H), 2.40(s, 3H), 2.44-2.50(m, 1H), 3.03(s, 3H), 3.38(s, 3H), 4.04(s, 2H), 4.56(s, 2H), 6.76(d, J=12.0Hz, 2H), 7.09-7.17(m, 5H), 7.66-7.72(m, 2H) |
| 3-81 | DMSO-d6, 300MHz | 1.14-1.45(m, 5H), 1.63-1.83(m, 5H), 2.29(s, 3H), 2.39-2.49(m, 1H), 2.43(t, J=7.2Hz, 2H), 2.74(t, J=7.1 Hz, 2H), 3.03(s, 3H), 3.87(s, 2H), 4.56(s, 2H), 6.76(d, J=8.8Hz, 2H), 7.10-7.17(m, 4H), 7.65(s, 1H), 7.71(d, J=8.8Hz, 2H) |
| 3-82 | DMSO-d6, 400MHz | 1.19-1.41(m, 5H),1.67-1.77(m, 5H),226(s, 3H),2.44-2.47(m, 1H), 3.03(s, 3H), 3.66(s, 2H), 3.88(s, 2H), 4.56(s, 2H), 6.76(d, J=9.2, 2H), 7.10-7.16(m, 4H), 7.37(d, J=8.1, 2H), 7.66(s, 1H), 7.71(d, J=8.6, 2H), 7.87(d, J=9, 2H) |
| 3-83 | DMSO-d6, 300MHz | 1.16-1.41(m, 5H), 1.67-1.81(m, 5H), 2.41-2.48(m, 1H), 3.03(s, 3H), 3.07(s, 3H), 4.56(s, 2H), 4.84(s, 2H), 6.68(d, J=6.9Hz, 2H), 6.76(d, J=6.5Hz, 2H), 7.10-7.17(m, 4H), 7.57(s, 1 H), 7.68-7.73(m, 4H) |

**Table 17-51**

| Example | solvent, Hz | NMR(δ) |
|---|---|---|
| 3-84 | DMSO-d6, 400MHz | 1.13-1.41 (m, 5H), 1.65-1.81 (m, 5H), 2.40-2.48(m, 1H), 3.03(s, 3H), 3.20(s, 3H), 4.56(s, 2H), 5.14(s, 2H), 6.75(d, J=9.0Hz, 2H), 6.80(d, J=9.0Hz, 1 H), 7.09-7.15(m, 4H), 7.59(s, 1H), 7.70(d, J=9.0Hz, 2H), 7.97(dd, J=2.3, 9.0Hz, 1H), 8.64(dd, J=0.8, 2.4Hz, 1H), 12.46(brs, 1 H) |
| 3-85 | DMSO-d6, 400MHz | 0.83(d, J=13.6Hz, 6H), 1.70-1.80(m, 1H), 2.32-2.37(m, 5H), 2.90-3.00(m, 2H), 3.02(s, 3H), 3.60-3.70(m, 1H), 3.98(d, J=16.0Hz 1H), 4.14(d, J=16.0Hz,1H), 4.56(s, 2H), 6.74(d, J=9.2Hz, 2H), 7.08-7.11(m, 4H), 7.18-7.20(m, 1 H), 7.25-7.27(m, 4H), 7.56(s, 1 H), 7.67(d, J=8.0Hz, 2H) |
| 3-86 | DMSO-d6, 300MHz | 0.79 (t, J = 7.2 Hz, 6H), 0.96-1.15(m, 4H), 1.40-1.57(m, 4H), 2.37 (s, 3H), 2.41-2.5(m, 1 H), 2.93 (dd, J = 14.1, 7.7, 1H), 3.02 (s, 3H), 3.06 (dd, J = 14.1, 7.7 Hz, 1H), 3.69 (t, J = 7.7 Hz, 1H), 4.00 (d, J = 15.8 Hz, 1H), 4.15 (d, J = 15.8 Hz, 1 H), 4.56 (s, 2H), 6.76 (d, J = 8.9 Hz, 2H), 7.09 (d, J = 8.6 Hz, 2H), 7.12 (d, J = 8.6 Hz, 2H), 7.19-7.28 (m, 5H), 7.57 (s, 1H), 7.69 (d, J = 8.9 Hz, 2H) |
| 3-87 | DMSO-d6, 300MHz | 0.79 (t, J = 7.2 Hz, 6H), 0.96-1.15(m, 4H), 1.40-1.57(m, 4H), 2.34 (s, 3H), 2.42-2.5(m, 1H), 3.02 (s, 3H), 3.83 (d, J = 15.3 Hz, 1H), 3.98 (d, J = 15.3 Hz, 1H), 4.50 (s, 1 H), 4.56 (s, 2H), 6.76 (d, J = 8.7 Hz, 2H), 7.08 (d, J = 8.4 Hz, 2H), 7.12 (d, J = 8.4 Hz, 2H), 7.33-7.48 (m, 5H), 7.64 (s, 1H), 7.70 (d, J = 8.7 Hz, 2H) |
| 3-88 | DMSO-d6, 400MHz | 0.84 (s, 9 H), 2.33 (s, 3 H), 2.42 (s, 2 H), 3.02 (s, 3 H), 3.82 (d, J = 15.2 Hz, 1 H), 3.96 (d, J = 15.6 Hz, 1 H), 4.48 (s, 1 H), .00 (s, 2 H), 6.74 (d, J = 8.8 Hz, 2 H), 7.05 (d, J = 7.6 Hz, 2 H), 7.10 (d, J = 7.6 Hz, 2 H), 7.32 (t, J = 7.2 Hz, 1 H), 7.39 (t, J = 7.4 Hz, 2 H), 7.45 (d, J = 8.9 Hz, 2 H), 7.63 (s, 1 H), 7.65 (d, J = 15.2 Hz, 2 H). |

**Table 17-52**

| Example | solvent, Hz | NMR(δ) |
|---|---|---|
| 3-89 | DMSO-d6, 400MHz | 0.84 (s, 9 H), 2.36 (s, 3 H), 2.42 (s, 2 H), 2.93 (dd, J = 14.2, 8.1 Hz, 1 H), 3.02 (s, 3 H), 3.05 (dd, J = 14.1, 7.2 Hz, 1 H), 3.68 (t, J = 8.0 Hz, 1 H), 4.01 (d, J = 12.0 Hz, 1 H), 4.14 (d, J = 15.6 Hz, 1 H), 4.57 (s, 2 H), 6.74 (d, J = 9.0 Hz, 2 H), 7.05 (d, J = 8.1 Hz, 2 H), 7.10 (d, J = 8.1 Hz, 2 H), 7.19 (q, J = 4.4 Hz, 1 H), 7.26 (d, J = 4.6 Hz, 2 H), 7.56 (s, 1 H), 7.67 (d, J = 8.8 Hz, 2 H), 12.48 (br s, 1 H). |
| 3-90 | DMSO-d6, 400MHz | 0.79(t, J=7.3Hz, 6H), 1.00-1.14(m, 4H), 1.41-1.57(m, 4H), 2.44-2.48(m, 1H), 3.03(s, 3H), 3.51(s, 4H), 4.16(s, 2H), 4.57(s, 2H), 6.76(d, J=9.0Hz, 2H), 7.09(d, J=8.6Hz, 2H), 7.12(d, J=8.4Hz, 2H), 7.64(s, 1H), 7.70(d, J=9.0Hz, 2H) |
| 3-91 | DMSO-d6, 300MHz | 0.83(d, J=5.0Hz, 6H), 1.17-1.41 (m, 7H), 1.54-1.62(m, 1H), 1.67-1.77(m, 5H), 2.41-2.49(m, 1 H), 2.66-2.70(m, 2H), 3.02(s, 3H), 3.41(s, 2H), 4.10(s, 2H), 6.74(d, J=6.9Hz, 2H), 7.09-7.15(m, 4H), 7.61(s, 1H), 7.69(d, J=6.6Hz, 2H) |
| 3-92 | DMSO-d6, 300MHz | 0.84(d, J=6.7Hz, 6H), 0.91 (d, J=17.6Hz, 3H), 0.95-1.05(m, 2H), 1.30-1.42(m, 5H), 1.50-1.60(m, 1H), 1.72-1.76(m, 4H), 2.45-2.50(m, 1H), 2.60-2.70(m, 2H), 3.02(s, 3H), 3.41(s, 2H), 4.10(s, 2H), 4.56(s, 2H), 6.75(d, J=9.0Hz, 2H), 7.09-7.17(m, 4H), 7.62(s, 1H), 7.70(d, J=8.8Hz, 2H) |
| 3-93 | DMSO-d6, 400MHz | 0.87-0.84(m, 12H), 1.33-1.37(m, 2H), 1.58-4.61(m, 1H), 1.75-1.78(m, 1H), 2.39(d, J=7.2Hz, 2H), 2.65-2.69(m, 2H), 3.02(s, 3H), 3.40(s, 2H), 4.09(s, 2H), 4.56(s, 2H), 6.74(d, J=9.2Hz, 2H), 7.06-7.11(m, 4H), 7.61(s, 1H), 7.68(d, J=11.6Hz, 2H) |
| 3-94 | DMSO-d6, 400MHz | 0.81(t.J=7.3Hz, 6H), 1.05-1.13(m, 4H), 1.48-1.57(m, 4H), 2.50-2.56(m, 1 H), 3.00(s, 3H), 4.56(s, 2H), 5.61(s, 2H), 6.78(d, J=8.8Hz, 2H), 7.10-7.13(m, 4H), 7.75(d, J=8.4Hz, 2H), 7.81(s, 1H), 7.97(s, 1H), 8.63(s, 1H), 8.71(s, 1H) |

**Table 17-53**

| Example | solvent, Hz | NMR(δ) |
|---|---|---|
| 3-95 | DMSO-d6, 400MHz | 0.81(t.J=7.3Hz, 6H), 1.05-1.13(m, 4H), 1.48-1.57(m, 4H),2.50-2.56(m, 1H), 3.04(s, 3H), 4.58(s, 2H), 5.54(s, 2H), 6.78(d, J=8.8Hz, 2H), 7.10-7.13(m, 4H), 7.19(d, J=8.0Hz, 2H), 7.74(d, J=8.0Hz, 2H), 7.80(s, 1H), 7.91 (d, J=9.2Hz, 2H) |
| 3-96 | DMSO-d6, 400MHz | 0.81(t.J=7.3Hz, 6H), 1.05-1.13(m, 4H), 1.48-1.57(m, 4H),2.50-2.56(m, 1H), 3.03(s, 3H), 4.57(s, 2H), 4.74(s, 2H), 6.76(d, J=9.2Hz, 2H), 7.10-7.13(m, 4H), 7.52(d, J=8.0Hz, 2H), 7.63(s, 1 H), 7.69(d, J=9.2Hz, 2H), 7.83(d, J=8.0Hz, 2H) |
| 3-97 | DMSO-d6, 300MHz | 1.20-1.40(m, 5H), 1.60-1.80(m, 5H), 1.45-1.50(m, 1H), 3.02(s, 3H), 4.39(d, J=6.3Hz, 2H), 4.55(s, 2H), 6.73(d, J=9.0Hz, 2H), 7.10-7.17(m, 4H), 7.61-7.64(m, 3H), 7.94(d, J=8.4Hz, 2H), 8.11(d, J=8.4Hz, 2H), 8.80(t, J=6.3Hz, 1H) |
| 3-98 | DMSO-d6, 400MHz | 0.92 (s, 3H), 0.95 (s, 3H), 1.40-1.61 (m, 8H), 2.31-2.41 (m, 1H), 2.85 (s, 3H), 3.03 (s, 3H), 4.56 (s, 2H), 4.65 (s, 2H), 6.74 (d, J = 8.82 Hz, 2H), 7.12 (d, J = 8.10 Hz, 2H), 7.18 (d, J = 8.10 Hz, 2H), 7.61 (d, J = 8.82 Hz, 2H), 7.69 (s, 1H), 7.97 (d, J = 8.36 Hz, 2H), 8.13 (d, J = 8.36, 2H) |
| 3-99 | DMSO-d6, 400MHz | 0.92 (s, 3H), 0.95 (s, 3H), 1.40-1.61 (m, 8H), 2.31-2.41 (m, 1H), 2.91 (s, 3H), 3.03 (s, 3H), 4.04 (s, 2H), 4.57 (s, 2H), 4.67 (s, 2H), 6.76 (d, J = 8.84 Hz, 2H), 7.12 (d, J = 8.12 Hz, 2H), 7.18 (d, J = 8.12 Hz, 2H), 7.70 (s, 1H), 7.72 (d, J = 8.84 Hz, 2H) |
| 3-100 | DMSO-d6 300MHz | 1.20-1.40(m, 5H), 1.60-1.77(m, 5H), 2.40-2.50(m, 1H), 2.84(s, 3H), 3.03(s, 3H), 4.55(s, 2H), 4.65(s, 2H), 6.73(d, J=9.0Hz, 2H), 7.90-7.16(m, 4H), 7.61 (d, J=8.4Hz, 2H), 7.70(s, 1H), 7.97(d, J=8.1Hz, 2H), 8.13(d, J=8.1Hz, 2H) |

**Table 17-54**

| Example | solvent, Hz | NMR(δ) |
|---|---|---|
| 3-101 | DMSO-d6, 300MHz | 1.20-1.40(m, 5H), 1.60-1.77(m, 5H), 2.40-2.50(m, 1H), 2.85(s, 3H), 3.03(s, 3H), 4.56(s, 2H), 4.65(s, 2H), 6.74(d, J=9.0Hz, 2H), 7.09-7.17(m, 4H), 7.62(d, J=8.7Hz, 2H), 7.69(s, 1H), 7.75(t, J=7.8Hz, 1H), 8.09(d, J=8.8Hz, 1H), 8.20(d, J=7.8Hz, 1H), 8.28(s, 1H) |
| 3-102 | DMSO-d6 300MHz | 1.20-1.40(m, 5H), 1.60-1.80(m, 5H), 1.45-1.50(m, 1H), 2.90(s, 3H), 3.03(s, 3H), 3.97(s, 2H), 4.56(s, 2H), 4.66(s, 2H), 6.76(d, J=8.7Hz, 2H), 7.09-7.16(m, 4H), 7.66-7.74(m, 3H) |
| 3-103 | DMSO-d6, 300MHz | 0.92(s, 3H), 0.95(s, 3H), 1.24-1.60(m, 8H), 1.86-1.97(m, 2H), 2.34-2.42(m, 3H), 2.89(s, 3H), 3.04(s, 3H), 3.23-3-28(m, 2H), 4.57(s, 2H), 4.67(s, 2H), 6.77(d, J=8.8Hz, 2H), 7.12(d, J=8.4Hz, 2H), 7.19(d, J=8.1 Hz, 2H), 7.70-7.74(m, 3H), 12.10(brs, 1H) |
| 3-104 | DMSO-d6, 300MHz | 0.81(d, J=8.5Hz, 6H), 0.83-0.95(m, 1H), 1.20-1.45(m, 5H), 1.60-1.80(m, 5H), 2.40-2.50(m, 1H), 3.00-3.07(m, 5H), 4.22(s, 2H), 4.56(s, 2H), 4.73(s, 2H), 6.76(d, J=9.0Hz, 2H), 7.09-7.17(m, 4H), 7.70-7.73(m, 3H) |
| 3-105 | DMSO-d6, 300MHz | 1.14-1.46(m, 5H), 1.64-1.85(m, 5H), 2.38-2.49(m, 1H), 3.03(s, 3H), 4.56(s, 2H), 4.61(d, J=6.0Hz, 2H), 6.76(d, J=9.1Hz, 2H), 7.10-7.17(m, 4H), 7.64(s, 1H), 7.71(d, J=9.1Hz, 2H), 9.61(t, J=6.2Hz, 1H) |
| 3-106 | DMSO-d6, 300MHz | 1.19-1.43(m, 5H), 1.63-1.8.1(m, 5H), 2.40-2.47(m, 1H), 3.03(s, 3H), 3.23(s, 2H), 4.58(d, J=6.2Hz, 2H), 6.76(d, J=8.8Hz, 2H), 7.10-7.18(m, 4H), 7.63(s, 1H), 7.72(d, J=8.8Hz, 2H), 8.92(t, J=5.8Hz, 1H), 12.37(brs, 1H) |
| 3-107 | DMSO-d6, 300MHz-120°C | 1.17-1.45(m, 5H), 1.66-1.85(m, 5H), 2.44-2.52(m, 1H), 3.01(s, 3H), 3.03(s, 3H), 4.52(s, 2H), 4.87(s, 2H), 6.80(d, J=8.8Hz, 2H), 7.14(s, 4H), 7.54(d, J=8.4Hz, 2H), 7.57(s, 1.H), 7.71(d, J=8.8Hz, 2H), 7.99(d, J=8.4Hz, 2H) |

**Table 17-55**

| Example | solvent, Hz | NMR(δ) |
|---|---|---|
| 3-108 | DMSO-d6, 300MHz | 0.89(d, J=6.6Hz, 3H), 1.00-1.50(m, 2H), 1.35-1.42(m, 3H), 1.72-1.76(m, 4H), 2.40-2.50(m, 1H), 3.04(s, 3H), 3.92(d, J=11.2Hz, 2H), 4.50-4.56(m, 4H), 5.40(d, J=6.0Hz, 2H), 6.77(d, J=9.0Hz, 2H), 7.09-7.17(m, 4H), 7.30-7.40(m, 5H), 7.70-7.76(m, 3H) |
| 3-109 | DMSO-d6, 400MHz | 1.21-1.41(m, 5H), 1.65-1.80(m, 5H), 1.45-1.50(m, 1H), 2.93(s, 3H), 3.03(s, 3H), 3.69-3.80(m, 2H), 4.56(s, 2H), 4.71(s, 2H), 6.75(d, J=11.6Hz, 2H), 6.94-7.05(m, 1H), 7.12-7.16(m, 4H), 7.64(s, 1 H), 7.71 (d, J=8.0Hz, 2H) |
| 3-110 | DMSO-d6, 300MHz | 1.15-1.25(m,4H), 162-1.76(m,6H), 3.68(s,4H), 4.39(s,2H), 5.21 (s,2H), 7.09(dd, J = 1.5, 9.0Hz, 1H), 7.48-7.52(m,5H), 7.66-7.69(m,6H), 7.77(s,1H) |
| 3-111 | DMSO-d6, 300MHz | 1.20-1.40(m, 5H), 1.60-1.80(m, 5H), 2.60-2.75(m, 4H), 2.80-2.95(m, 4H), 3.42(s, 2H), 3.91(s, 2H), 4.56(s, 2H), 6.76(d, J=9.0Hz, 2H), 7.09-7.16(m, 4H), 7.69(d, J=4.9Hz, 2H), 7.73(s, 1H) |
| 3-112 | DMSO-d6, 300MHz | 0.81(t, J=7.3Hz, 6H), 0.99-1.21(m, 4H), 1.41-1.65(m, 4H), 2.48-2.61 (m, 1H), 2.96(dd, J=6.4, 16.2Hz, 1H), 3.19(dd, J=3.8, 16.2Hz, 1 H), 3.68(d, J=15.1Hz, 1H), 4.24-4.44(m, 3H), 5.08(s, 2H), 6.95(d, J=6.0Hz, 1H), 6.98-7.11 (m, 6H), 7.19(d, J=8.3Hz, 2H), 7.38(d, J=7.9Hz, 2H), 7.80(s, 1H), 7.87(d, J=8.7Hz, 2H) |
| 3-113 | DMSO-d6, 300MHz | 0.80(t, J=7.2Hz, 6H), 1.07-1.14(m, 4H), 1.48-1.58(m, 4H), 2.5-2.6(m, 1H), 5.09(s, 2H), 5.55(s., 2H), 7.09(d, J=9.0H, 2H), 7.19(d, J=7.8Hz, 2H), 7.39(d, J=8.1Hz, 2H), 7.77-7.79(m, 1H), 7.91(d, J=8.7Hz, 2H), 8.00(s, 1H), 8.29(d, J=3.0Hz, 1H), 8.60(d, J=1.5Hz, 1H) |
| 3-114 | DMSO-d6, 400MHz | 0.80(t, J=7.2Hz, 6H), 1.04-1.14(m, 4H), 1.48-1.58(m, 4H),, 2.37(s, 3H), 2.49-2.53(m, 1H), 5.07(s, 2H), 5.65(s, 2H), 6.91(dd, J=8.6, 2.8Hz, 1H), 6.95(d, J=2.5Hz, 1H), 7.18(d, J=8.1 Hz, 2H), 7.36(d, J=8.1 Hz, 2H), 7.54(d. J=8.6Hz, 1H), 7.73(s, 1H), 7.95-7.96(m, 1H), 8.63(d, J=3.0Hz, 1H), 8.71(d, J=1.6Hz, 1H) |

**Table 17-56**

| Example | solvent, Hz | NMR(δ) |
|---|---|---|
| 3-115 | DMSO-d6, 400MHz | 0.80(t, J=7.2Hz, 6H), 1.07-1.14(m, 4H), 1.48-1.58(m, 4H), 2.5-2.6(m, 1H), 3.83(s, 3H), 5.05(s, 2H), 5.66(s, 2H), 7.12(d, J=8.4Hz, 1H), 7.20(d, J=8.OHz, 2H), 7.37(d, J=8.0Hz, 2H), 7.50-7.54(m, 2H), 7.99(s, 1H), 8.08(s, 1H), 8.63(s, 1H), 8.71(s, 1H) |
| 3-116 | DMSO-d6, 400MHz | 0.80(t, J=7.2Hz, 6H), 1.05-1.14(m, 4H), 1.48-1.58(m, 4H) 2.37(s, 3H), 3.58(s, 2H), 3.63(s, 2H), 4.26(s, 2H), 5.07(s, 2H), 6.88(dd, J=8.0, 4.0Hz, 1 H), 6.94(d, J=4.0Hz, 1H), 7.04(t, J=6.0Hz, 1H), 7.18(d, J=8.0Hz, 2H), 7.26(d, J=8.0Hz, 1H), 7.28(d, J=8.0Hz, 1H), 7.36(d, J=8.0Hz, 2H), 7.50(d, J=8.0Hz, 1H), 7.56-7.58(m, 3H) |
| 3-117 | DMSO-d6, 400MHz | 0.80(t, J=7.2Hz, 6H), 1.05-1.14(m, 4H),1.16(d, t=7.0Hz, 6H), 1.48-1.58(m, 4H), 2.37(s, 3H), 2.50-2.55(m,1H),2.8-2.90(m,1H), 3.53(s, 4H), 4.23(s, 2H), 5.06(s, 2H), 6.88(dd, J=8.0, 4.0Hz, 1H), 6.94(d, J=4.0Hz, 1H), 7.05-7.19(m, 4H), 7.36(d, J=8.0Hz, 2H), 7.50-7.60(m, 4H) |
| 3-118 | DMSO-d6, 300MHz | 0.81(t, J=7.4Hz, 6H), 0.99-1.20(m, 4H), 1.41-1.64(m, 4H), 2.48-2.61 (m, 1H), 3.10(dd, J=2.6, 16.2Hz, 1H), 3.18(dd, J=5.7, 16.9Hz, 1H), 3.87-4.01(m, 2H), 4.09(d, J=15.8Hz, 1H), 4.25(d, J=15.8Hz, 1H), 4.40(d, J=15.5Hz, 1 H), 5.09(s, 2H), 7.00-7.23(m, 8H), 7.38(d, J=8.3Hz, 2H), 7.84-7.93(m, 3H), 12.65(brs, 1H) |
| 3-119 | DMSO-d6, 400MHz | 0.80(t, J=7.2Hz, 6H), 1.05-1.14(m, 4H),1.48-1.58(m, 4H), 2.50-2.54(m, 1H), 3.58(s, 2H), 3.64(s, 2H), 3.80(s, 3H), 4.27(s, 2H), 5.40(s, 2H), 7.04(t, J=8.0Hz, 1H), 7.08(d, J=8.0Hz, 1H), 7.18(d, J=8.0Hz, 2H), 7.28(d, J=8.0Hz, 1H), 7.30(d, J=8.0Hz, 1H), 7.36(d, J=8.0Hz, 2H), 7.46(dd, J=8.0, 4.0Hz, 1H), 7.50(d, J=4.0Hz, 1H), 7.59(d, J=8.0Hz, 2H), 7.93(s, 1H) |

**Table 17-57**

| Example | solvent, Hz | NMR(δ) |
|---|---|---|
| 3-120 | DMSO-d6, 400MHz | 0.80(t, J=7.2Hz, 6H), 1.05-1.14(m, 4H),1.16(d, t=7.0Hz, 6H), 1.48-1.58(m, 4H), 2.50-2.55(m,1H), 2.80-2.90(m,1H), 3.56(s, 2H), 3.63(s, 2H), 3.80(s, 3H), 4.26(s, 2H), 5.03(s, 2H), 7.08(d, J=8.0Hz, 1 H), 7.15-7.19(m, 4H), 7.36(d, J=8.0Hz, 2H), 7.44-7.50(m, 4H), 7.93(s, 1H) |
| 3-121 | DMSO-d6, 400MHz | 0.80(t, J=7.2Hz, 6H), 1.05-1.14(m, 4H), 1.48-1.58(m, 4H), 2.38(s, 3H), 2.50-2.54(m, 1H), 3.45(s, 2H), 4.05(s, 2H), 4.19(s, 2H), 5.02(s, 2H), 6.89(d, J=8.0Hz, 1 H), 6.94(d, J=4.0Hz, 1 H), 7.18(d, J=8.0Hz, 2H), 7.36(d, J=8.0Hz, 2H), 7.51-7.56(m, 3H), 9.07(d, J=4.0Hz, 2H) |
| 3-122 | DMSO-d6, 400MHz | 0.81(t, J=7.3Hz, 6H), 1.02-1.05(m, 4H), 1.40-1.50(m, 4H), 2.40(s, 3H), 2.49-2.53(m, 1H), 2.66(s, 3H), 3.85(s, 2H), 4.29(s, 2H), 4.51(s, 2H), 5.07(s, 2H), 6.91(dd, J=8.0, 4.0Hz, 1 H), 6.96(d, J=4.0Hz, 1 H), 7.18(d, J=8.0Hz, 2H), 7.36(d, J=8.0Hz, 2H), 7.53(d, J=8.0Hz, 1 H), 7.55(s, 1H), 7.70(s, 1H) |
| 3-123 | DMSO-d6, 400MHz | 0.81(t, J=7.3Hz, 6H), 1.05-1.15(m, 4H), 1.40-1.50(m, 4H), 2.38(s, 3H), 2.45-2.55(m, 1H), 3.58(s, 2H), 3.68(s, 2H), 4.30(s, 2H), 4.31(s, 2H), 5.07(s, 2H), 6.88(dd, J=12.0, 4.0Hz, 1 H), 6.94(d, J=2.0Hz, 1 H), 7.18(d, J=8.0Hz, 2H), 7.22-7.30(m, 5H), 7.36(d, J=8.0Hz, 2H), 7.51 (d, J=8.0Hz, 1H), 7.62(s, 1H) |
| 3-124 | DMSO-d6, 400MHz | 0.80(t, J=7.2Hz, 6H), 1.05-1.14(m, 4H),1.48-1.58(m, 4H), 2.38(s, 3H), 2.50-2.54(m, 1H), 3.54(s, 2H), 4.17(s, 2H), 4.28(s, 2H), 5.06(s, 2H), 6.89(d, J=8.0Hz, 1H), 6.94(d, J=4.0Hz, 1 H), 7.14-7.18(m, 4H), 7.35(d,J=8Hz,2H), 7.49-7.56(m, 4H) |
| 3-125 | DMSO-d6, 300MHz | 0.79(t, J=7.2Hz, 6H), 1.00-1.14(m, 4H), 1.26(s, 9H), 1.40-1.58(m, 4H), 1.89-1.98(m, 2H), 2.42-2.55(m, 1H), 2.75-2.82(m, 2H), 3.35-3.42(m, 2H), 3.49(s, 2H), 4.19(s, 2H), 4.21 (s, 2H), 4.49(s, 2H), 6.53(d, J=9.0Hz, 1H), 7.09-7.18(m, 5H), 7.45(d, J=9.0Hz, 1H), 7.49(s, 1H), 7.61 (s, 1H), 12.58(brs, 1H) |

**Table 17-58**

| Example | solvent, Hz | NMR(δ) |
|---|---|---|
| 3-126 | DMSO-d6, 300MHz | 0.81(t, J=7.2Hz, 6H), 1.00-1.19(m, 4H), 1.43-1.63(m, 4H), 1.98(brm, 4H), 2.53-2.59(m, 1H), 3.13(brs, 2H), 3.61(brs, 2H), 4.73(brs, 2H), 5.10(s, 2H), 7.12(d, J=9.1Hz, 2H), 7.20(d, J=7.9Hz, 2H), 7.38(d, J=8.3Hz, 2H), 7.93(d, J=8.7Hz, 2H), 8.14(brs, 1H), 10.74(brs, 1H), 12.52(brs, 1H) |
| 3-127 | DMSO-d6, 300MHz | 0.81(t, J=7.3Hz, 6H), 1.02-1.19(m, 4H), 1.46-1.61(m, 4H), 2.52-2.59(m, 1H), 2.69(brs, 4H), 3.35(brs, 4H), 3.94(s, 2H), 5.09(s, 2H), 6.98(d, J=9.1Hz, 2H), 7.08(d, J=9.0Hz, 2H), 7.19(d, J=8.3Hz, 2H), 7.38(d, J=7.9Hz, 2H), 7.77(d, J=9.0Hz, 2H), 7.89(d, J=9.0Hz, 2H), 7.90(s, 1H), 12.27(brs, 1H) |
| 3-128 | DMSO-d6, 300MHz | 0.71(t, J = 9.0Hz, 6H), 1.11(d,J = 6.0Hz, 6H), 1.29(t, J = 6.0Hz, 3H), 1.56-1.71(m,4H), 2.38-2.42(m,1H), 2.76-2.83(m,1H), 4.26(q, J = 6.0, 2H), 5.66(s,2H), 5.97(s,2H), 7.05-7.20(m,5H), 7.43(s,1H), 7.49(d,J = 9.0Hz, 1H), 7.87(d,J = 9.0Hz, 2H), 8.47(s,1H) |
| 3-129 | DMSO-d6, 300MHz | 0.72(t, J = 6.0Hz, 6H), 1.11(d,J = 0.9Hz, 6H), 1.52-1.74(m,4H), 2.42-2.46(m,1H), 2.75-2.84(m,1H), 5.66(s,2H), 5.99(s,2H), 7.06-7.18(m,9H), 7.44(s,1H), 7.50(d,J = 9.0Hz, 1 H), 7.87(d,J = 9.0Hz, 2H), 8.48(s,1H) |
| 3-130 | DMSO-d6, 300MHz | 0.79(t, J = 6.0Hz, 6H), 1.15(s,3H), 1.57-1.89(m,4H), 2.42-2.52(m,1H), 5.43(s,2H), 6.10(s,2H), 6.99-7.09(m,3H), 7.24-7.33(m,15H), 7.65(t, J = 9.0Hz, 1H), 7.90(d,J = 6.3Hz. 1H), 7.99-8.03(m,3H) |
| 3-131 | DMSO-d6, 300MH | 0.68(t, J = 6.0Hz, 6H), 1.48-1.76(m,4H), 2.35-2.42(m,1H), 5.61(s,2H), 6.14(s,2H), 7.07(s,1H), 7.10(d,J = 9.0Hz, 2H), 7.20(d,J = 6.0Hz, 2H), 7.35(s,1H), 7.62(d,J = 6.0Hz, 1H), 7.82(d,J = 6.0Hz, 2H), 7.83(d,J = 9.0Hz, 2H), 8.47(s,1H) |

**Table 17-59**

| Example | solvent, Hz | NMR(δ) |
|---|---|---|
| 3-132 | DMSO-d6, 300MHz | 0.74(t, J = 6.0Hz, 6H), 0.89-1.10(m,9H), 1.42-1.778(m,13H), 2.38-2.45(m,1 H), 4.58(d,J = 6.0Hz, 2H), 5.65(s,2H), 7.08-7.16(m,3H), 7.41(s,1H), 7.53(d,J = 9.0Hz, 1 H), 7.91 (d,J = 9.0Hz, 2H), 8.42(s,1H) |

**Table 17-60**

| Example | solvent, Hz | NMR(δ) |
|---|---|---|
| 4-1 | DMSO-d6, 300MHz 1H), | 0.79 (t, J = 7.3 Hz, 6H), 0.96- 1.18 (m, 4H), 1.34- 1.60 (m, 4H), 3.36 (s, 2H), 3.88 (s, 3H), 4.04 (s, 2H), 4.16 (s, 2H), 5.06 (s, 2H), 6.92 (d, J = 8.7 Hz, 2H), 7.01 (d, J = 3.4 Hz, 1H), 7.07 (d, J = 8.7 Hz, 2H), 7.14- 7.26 (m, 2H), 7.35 (d, J = 3.4 Hz, 1H), 7.46 (d, J = 8.3 Hz, 2H), 7.53 (d, J = 7.5 Hz, 7.58- 7.65 (m, 3H), 12.52 (br s, 1H) |
| 4-2 | DMSO-d6, 300MHz | 0.80(t, J=7.3Hz, 6H), 1.00-1.12(m, 4H), 1.38-1.57(m, 4H), 2.40-2.50(m, 1H), 5.07(s, 2H), 5.48(s, 2H), 6.93(d, J=8.6Hz, 2H), 7.07(d, J=8.6Hz, 2H), 7.49(d, J=7.9Hz, 2H), 7.69-7.71(m, 1H), 7.72(d, J=8.3Hz, 2H), 7.88-7.89(m, 1H), 7.91(d, J=1.5Hz, 1H), 8.56(d, J=2.6Hz, 1H), 8.69(d, J=1.5Hz, 1H), 13.41(brs, 1H) |
| 4-3 | DMSO-d6, 300MHz | 0.78 (t, J = 7.4 Hz, 6 H), 1.00- 1.12 (m, 4 H), 1.15 (d, J = 6.4 Hz, 6 H), 1.30- 1.53 (m, 4 H), 2.27- 2.41 (m, 1 H), 4.22 (sept, J = 6.8 Hz, 1 H), 4.35 (s, 2 H), 5.48 (s, 2 H), 6.60 (d, J = 8.6 Hz, 2 H), 6.88 (d, J = 9.0 Hz, 2 H), 7.32 (d, J = 8.3 Hz, 2 H), 7.62 (d, J = 8.7 Hz, 2 H), 7.65 (d, J = 1.5 Hz, 1 H), 7.83 (d, J = 1.5 Hz, 1 H), 7.89 (dd, J = 3.0, 1.5 Hz, 1 H), 8.59 (d, J = 2.6 Hz, 1 H), 8.69 (d, J = 1.5 Hz, 1 H), 13.48 (br s, 1 H). |
| 4-4 | DMSO-d6, 300MHz | 0.69 (t, J = 7.4 Hz, 6 H), 1.16 (d, J = 6.4 Hz, 6 H), 1.32-1.47 (m, 2 H), 1.48- 1.64 (m, 2 H), 2.06- 2.18 (m, 1 H), 4.23 (sept, J = 6.6 Hz, 1 H), 4.36 (s, 2 H), 5.48 (s, 2 H), 6.62 (d, J = 8.7 Hz, 2 H), 6.88 (d, J = 9.0 Hz, 2 H), 7.32 (d, J = 8.3 Hz, 2 H), 7.62 (d, J = 8.3 Hz, 2 H), 7.65 (d, J = 1.1 Hz, 1 H), 7.83 (d, J = 1.5 Hz, 1 H), 7.89 (dd, J = 2.8, 1.7 Hz, 1 H), 8.59 (d, J = 3.0 Hz, 1 H), 8.69 (d, J = 1.5 Hz, 1 H), 13.48 (br s, 1 H). |

**Table 17-61**

| Example | solvent, Hz | NMR(δ) |
|---|---|---|
| 4-5 | DMSO-d6, 300MHz | 0.79(t, J=7.2Hz, 6H), 1.01-1.14(m, 4H), 1.37-1.58(m, 4H), 2.41-2.48(m, 1H), 5.07(s, 2H), 5.48(s, 2H), 6.93(d, J=8.7Hz, 2H), 7.07(d, J=8.7Hz, 2H), 7.26(d, J=3.8Hz, 1H), 7.45(d, J=3.8Hz, 1H), 7.49(d, J=8.3Hz, 2H), 7.67(d, J=8.3Hz, 2H), 7.88-7.90(m, 1H), 8.58(d, J=2.6Hz, 1H), 8.70(d, J=1.5Hz, 1H), 13.53(brs, 1H) |
| 4-6 | DMSO-d6, 300MHz | 0.79(t, J=7.3Hz, 6H), 0.97-1.17(m, 4H), 1.35-1.60(m, 4H), 2.38-2.53(m, 1H), 3.45(s, 2H), 4.12(s, 4H), 5.06(s, 2H), 6.93(d, J=8.7Hz, 2H), 7.07(d, J=8.7Hz, 2H), 7.11-7.20(m, 2H), 7.41-7.59(m, 5H), 7.69(d, J=8.3Hz, 2H), 7.75-7.80(m, 1H) |
| 4-7 | DMSO-d6, 300MHz | 0.80 (t, J = 7.3 Hz, 6 H), 0.99- 1.17 (m, 4 H), 1.36- 1.59 (m, 4 H), 2.38- 2.53 (m, 1 H), 5.07 (s, 2 H), 5.63 (s, 2 H), 6.93 (d, J = 8.6 Hz, 2 H), 7.07 (d. J = 8.6 Hz, 2 H), 7.10 (dd, J = 8.3, 0.7 Hz, 1 H), 7.48 (d, J = 8.3 Hz, 2 H), 7.71 (d, J = 7.6 Hz, 2 H), 7.72 (d, J = 0.8 Hz, 1 H), 7.79 (d, J = 1.5 Hz, 1 H), 7.87 (d, J = 1.5 Hz, 1 H), 7.90 (dd, J = 8.3, 7.2 Hz, 1 H), 13.17(br s, 1 H). |
| 4-8 | DMSO-d6, 400MHz | 0.80(t, J=7.2Hz, 6H), 1.05-1.14(m, 4H),1.48-1.58(m, 4H), 2.45-2.50(m, 1H), 3.42(s, 2H), 4.07(s, 4H), 5.06(s, 2H), 6.89(d, J=8.0Hz, 2H), 7.00-7.05(m, 3H), 7.11-7.13(m, 2H), 7.35(d, J=4.0Hz, 1H), 7.44(d, J=12.0Hz, 2H), 7.48-7.50(m, 2H), 7.60(d, J=8.0Hz, 2H) |
| 4-9 | DMSO-d6, 400MHz | 0.77(t, J=7.3Hz, 6H), 1.02-1.05(m, 4H), 1.15(d, J=4.0Hz, 6H), 1.40-1.51(m, 4H), 2.40-2.45(m, 1H), 2.79-2.84(m, 1H), 3.44(s, 2H), 3.51(s, 2H), 4.04(s, 2H), 5.03(s, 2H), 6.89(d, J=8.0Hz, 2H), 6.99(d, J=4.0Hz, 1H), 7.03(d, J=8.0Hz, 2H), 7.14(d, J=8.0Hz, 2H), 7.33(d, J=4.0Hz, 1H), 7.43(d, J=8.0Hz, 2H), 7.48(d, J=8.0Hz, 2H), 7.58(d, J=8.0Hz, 2H) |

**Table 17-62**

| Example | solvent, Hz | NMR(δ) |
|---|---|---|
| 4-10 | DMSO-d6, 400MHz | 0.77(t, J=7.3Hz, 6H), 1.02-1.05(m, 4H), 1.40-1.51(m, 4H), 2.40-2.45(m, 1H), 3.47(s, 2H), 3.51(s, 2H), 4.70(s, 2H), 5.05(s, 2H), 6.91(d, J=8.0Hz, 2H), 7.01(d, J=2.0Hz, 1H), 7.03-7.07(m, 3H), 7.28-7.31(m, 2H), 7.35(d, J=3.0Hz, 1H), 7.45(d, J=8.0Hz, 2H), 7.59-7.62(m, 4H) |
| 4-11 | DMSO-d6, 400MHz | 0.68(t, J = 7.3 Hz, 6H), 1.15(d, J = 6.5 Hz, 6H), 1.34-1.45(m, 2H), 1.50-1.60(m, 2H), 2.08-2.15(m, 1H), 3.46(s, 2H), 3.50(s, 2H), 4.07(s, 2H), 4.21 (sep, J = 6.5Hz, 1 H), 4.34(s, 2H), 6.60(d, J = 8.8 Hz, 2H), 6.87(d, J = 8.8 Hz, 2H), 7.03(t, J = 7.3 Hz, 1H), 7.26-7.31 (m, 4H), 7.40(s, 1H), 7.56-7.61 (m, 4H), 7.69(s, 1H), 10.11 (brs, 1 H), 12.64(brs, 1H) |
| 4-12 | DMSO-d6, 400MHz | 0.68(t, J = 7.3 Hz, 6H), 1.15(d, J = 6.5 Hz, 6H), 1.34-1.45(m, 2H), 1.50-1.60(m, 2H), 2.08-2.15(m, 1H), 3.41(s, 2H), 4.07(s, 2H), 4.11(s, 2H), 4.21 (sep, J = 6.5Hz, 1 H), 4-34(s, 2H), 6.60(d, J = 8.8 Hz, 2H), 6.88(d, J = 8.8 Hz, 2H), 7.15(s, 1 H), 7.16(d, J = 8.6 Hz, 2H), 7.28(d, J = 8.1 Hz, 2H), 7.38(s, 1H), 7.58(d, J = 8.1 Hz, 2H), 7.70(s, 1H), 12.48(brs, 1H) |
| 4-13 | DMSO-d6, 400MHz | 0.79(t, J=7.2Hz, 6H), 1.01-1.14(m, 4H), 1.33(s, 9H), 1.37-1.58(m, 4H), 2.41-2.51(m, 1H), 3.37(s, 2H), 4.09(s, 4H), 5.05(s, 2H), 6.91(d, J=8.4Hz, 2H), 7.05(d, J=8.4Hz, 2H), 7.39(s, 1H), 7.42(brs, 1H), 7.45(d, J=8.4Hz, 2H), 7.68(d, J=8.0Hz, 2H), 7.77(d, J=1.2Hz, 1H) |
| 4-14 | DMSO-d6, 300MHz | 0.68 (t, J = 7.3 Hz, 6 H), 1.15 (d, J = 6.7 Hz, 6 H), 1.32-1.46 (m, 2 H), 1.48- 1.64 (m, 2 H), 2.05- 2.18 (m, 1 H), 3.43 (s, 2 H), 4.10 (s, 4 H), 4.22 (sept, J = 6.6 Hz, 1 H), 4.35 (s, 2 H), 6.61 (d, J = 8.7 Hz, 2 H), 6.88 (d, J = 8.7 Hz, 2 H), 7.14 (dd, J = 6.0, 3.0 Hz, 2 H), 7.29 (d, J = 8.3 Hz, 2 H), 7.41 (d, J = 1.1 Hz, 1 H), 7.52 (dd, J = 5.8, 3.2 Hz, 1 H), 7.59 (d, J = 8.3 Hz, 2 H), 7.69 (d, J = 1.5 Hz, 1 H), 12.43 (br s, 1 H). |

**Table 17-63**

| Example | solvent, Hz | NMR(δ) |
|---|---|---|
| 4-15 | DMSO-d6, 300MHz | 0.76 (t, J = 7.17 Hz, 6H), 0.98-1.16 (m, 4H), 1.23 (s, 9H), 1.34-1.56 (m, 4H), 2.38-2.46(m, 1H), 3.39 (s, 2H), 4.05 (s, 2H), 4.10 (s, 2H), 5.02 (s, 2H), 6.89 (d, J = 8.60 Hz, 2H), 7.03 (d, J = 8.60 Hz, 2H), 7.14 (s, 1H), 7.41 (s, 1H), 7.43 (d, J = 8.31 Hz, 2H), 7.66 (d, J = 8.31 Hz, 2H), 7.76 (s, 1H) |
| 4-16 | DMSO-d6, 300MHz | 0.76 (t, J = 7.14 Hz, 6H), 0.97-1.11 (m, 4H), 1.34-1.55 (m, 4H), 2.36-2.46(m, 1H), 3.45 (s, 2H), 3.52 (s, 2H), 4.07 (s, 2H), 5.02 (s, 2H), 6.89 (d, J = 8.67 Hz, 2H), 7.01(d, J = 6.39 Hz, 1H), 7.03 (d, J = 8.67 Hz, 2H), 7.28 (dd, J = 8.28, 7.53 Hz, 2H), 7.42 (d, J = 8.28 Hz, 2H), 7.43 (d, J = 1.5 Hz, 1H), 7.57 (d, J = 7.53 Hz, 2H), 7.65 (d, J = 8.31 Hz, 2H), 7.75 (d, J = 1.5 Hz, 1H), 9.93 (s, 1H) |
| 4-17 | DMSO-d6, 300MHz | 0.67(t, J = 4.0Hz, 6H), 1.14(d,J = 8.0Hz, 6H), 1.39-1.43(m,2H), 1.50-1.60(m,2H), 2.08-2.15(m,1H), 4.34(s,2H), 5.44(s,2H), 6.59(d,J = 8.0Hz, 2H), 6.87(d,J = 12.0Hz, 2H), 7.22(d,J = 4.0Hz, 1 H), 7.30(d,J = 8.0Hz, 2H), 7.36(d,J = 4.0Hz, 1H), 7.56(d,J = 8.0Hz. 2H), 7.85(s,1H), 8.82(s,1H), 8.66(s,1H) |
| 4-18 | DMSO-d6, 400MHz | 0.77(t, J = 8.0Hz, 6H), 1.02-1.10(m,4H), 1.14(d,J = 4.0Hz, 6H), 1.31-1.50(m,4H), 2.29-2.37(m,1H), 4.15-4.26(m,1H), 4.33(s,2H), 5.43(s,2H), 6.58(d,J = 12.0Hz, 2H), 6.87(d,J = 12.0Hz, 2H), 7.22(s,1H), 7.30(d,J = 8.0Hz, 1 H), 7.36(d,J = 4.0Hz, 2H), 7.56(d,J = 8.0Hz, 2H), 7.84(s,1H), 8.51(s,1H), 8.65(s,1H) |
| 4-19 | DMSO-d6, 400MHz | 0.63(t, J = 8.0Hz, 6H), 1.10(d,J = 8.0Hz, 6H), 1.21(s,9H), 1.33-1.38(m,2H), 1.45-1.52(m,2H), 2.27(m,1H), 3.25-3.60(m,1H), 3.98(s,2H), 4.06(s,2H), 4.29(s,2H), 6.55(d,J = 8.0Hz, 2H), 6.82(d,J = 8.0Hz, 2H), 6.92(d,J = 4.0Hz, 1H), 7.10(s,1H), 7.25(d,J = 12.0Hz, 2H), 7.24(s,1H), 7.49(d,J = 8.0Hz, 2H) |

**Table 17-64**

| Example | solvent, Hz | NMR(δ) |
|---|---|---|
| 4-20 | DMSO-d6, 300MHz | 0.78(t, J = 9.0Hz, 6H), 1.01-1.13(m,4H), 1.15(d,J = 6.0Hz, 6H), 1.26(s,9H), 1.38-1.49(m,4H), 2.28-2.35(m,1H), 3.33(s,2H), 4.06(s,2H), 4.13(s,2H), 4.14-4.24(m,1H), 4.34(s,2H), 6.60(d,J = 9.0Hz, 2H), 6.89(d,J = 9.0Hz, 2H), 6.96(d,J = 6.0Hz, 1H), 7.13(s,1H), 7.29(d,J = 12.0Hz, 2H), 7.29(s,1H), 7.54(d,J = 6.0Hz, 2H) |
| 4-21 | DMSO-d6, 300MHz | 0.67(t, J = 4.0Hz, 6H), 1.14(d,J = 8.0Hz, 6H), 1.30-1.43(m,2H), 1.48-1.60(m,2H), 2.06-2.14(m,1H), 3.40(s,2H), 4.06(s,2H), 4.07(s,2H), 4.18-4.25(m,1H), 4.33(s,2H), 6.59(d,J = 8.0Hz, 2H), 6.86(d,J = 8.0Hz, 2H), 7.00(d,J = 4.0Hz, 1H), 7.12-7.14(m,2H), 7.28(m,3H), 7.48-7.54(m,3H) |
| 4-22 | DMSO-d6, 300MHz | 0.78(t, J = 9.0Hz, 6H), 1.01-1.14(m,4H), 1.15(d,J = 6.0Hz, 6H), 1.36-1.48(m,4H), 2.24-2.40(m,1H), 4.07(s,4H), 4.17-4.25(m,1H), 4.34(s,2H), 6.60(d,J = 6.0Hz, 2H), 6.89(d,J = 9.0Hz, 2H), 6.97-7.43(m,6H), 7.43-7.69,(m,3H) |
| 4-23 | DMSO-d6 400MHz | 0.79(t, J = 7.3 Hz, 6H), 1.01-1.14(m, 4H), 1.39-1.56(m, 4H), 2.20(s, 3H), 2.29(s, 3H), 2.41-2.49(m, 1H), 3.37(s, 2H), 4.04(s, 2H), 4.08(s, 2H), 5.05(s, 2H), 6.91 (d, J = 8.6 Hz, 2H), 7.05(d, J = 8.6 Hz, 2H), 7.42(s, 1H), 7.45(d, J = 8.1 Hz, 2H), 7.68(d, J = 8.1 Hz, 2H), 7.77(s, 1H) |
| 4-24 | DMSO-d6, 400MHz | 0.68(t, J = 7.2 Hz, 6H), 1.15(d, J = 6.3 Hz, 6H), 1.34-1.45(m, 2H), 1.51-1.61 (m, 2H), 2.09-2.16(m, 1H), 3.04-3.17(m, 2H), 3.83-3.96(m, 2H), 4.00-4.09(m, 1H), 4.16-4.27(m, 3H), 4.34(s, 2H), 6.60(d, J = 8.6 Hz, 2H), 6.87(d, J = 8.6 Hz, 2H), 7.00-7.16(m, 4H), 7.29(d, J = 8.1 Hz, 2H), 7.45(brs, 3H), 7.61 (d, J = 8.1 Hz, 2H), 7.71(brs, 1H) |

**Table 17-65**

| Example | solvent, Hz | NMR(δ) |
|---|---|---|
| 4-25 | DMSO-d6, 400MHz | 0.68(t, J = 7.3 Hz, 6H), 1.15(d, J = 7.0 Hz, 6H), 1.17(d, J = 7.0 Hz, 6H), 1.34-1.45(m, 2H), 1.50-1.60(m, 2H), 2.08-2.16(m, 1H), 2.82(sep, J = 7.0 Hz, 1H), 3.45(s, 2H), 3.53(s, 2H), 4.08(s, 2H), 4.21 (sep, J = 7.0 Hz, 1H), 4.34(s, 2H), 6.60(d, J = 8.6 Hz, 2H), 6.87(d, J = 8.6 Hz, 2H), 7.16(d, J = 8.6 Hz, 2H), 7.28(d, J = 8.4 Hz, 2H), 7.41 (s, 1H), 7.50(d, J = 8.6 Hz, 2H), 7.57(d, J = 8.4 Hz, 2H), 7.69(s, 1H), 9.85(s, 1H), 12.58(brs, 1H) |
| 4-26 | DMSO-d6, 400MHz | 0.79(t, J=7.4Hz, 6H), 1.00-1.14(m, 4H), 1.39-1.56(m, 4H), 2.42-2.48(m, 1H), 3.17-3.43(m, 2H), 4.15-4.63(m, 5H), 5.07(s, 2H), 6.92(d, J=8.8Hz, 2H), 7.06(d, J=8.8Hz, 2H), 7.18-7.28(m, 5H), 7.48-7.50(m, 3H), 7.67(d, J=8.4Hz, 2H) |
| 4-27 | DMSO-d6, 300MHz | 0.79 (t, J = 7.2 Hz, 6 H),0.99- 1.18 (m, 4 H),1.18 (d, J = 6.8 Hz, 6 H),1.32- 1.60 (m, 4 H),2.39- 2.55 (m, 1 H),2.75-2.91 (m, 1 H),3.47 (s, 2 H),3.55 (s, 2 H),4.10 (s, 2 H),5.06 (s, 2 H),6.93 (d, J = 8.7 Hz, 2 H),7.07 (d, J = 8.7 Hz, 2 H),7.18 (d, J = 8.7 Hz, 2 H),7.42- 7.53 (m, 5 H),7.68 (d, J = 8.3 Hz, 2 H),7.79 (d, J = 1.5 Hz, 1 H),9.87 (s, 1 H), 12.50 (br s, 1 H). |
| 4-28 | DMSO-d6, 300MHz 2H), 7.00-7.14(m, | 0.68(t, J = 7.3 Hz, 6H), 1.14(d, J = 6.6 Hz, 6H), 1.32-1.47(m, 2H), 1.49-1.62(m, 2H), 2.08-2.17(m, 1H), 3.04(dd, J = 16.3, 3.5 Hz, 1H), 3.11(dd, J = 16.3, 6.2 Hz, 1H), 3.81-3.86(m, 2H), 4.00(d, J = 15.8 Hz, 1 H), 4.14-4.26(m, 3H), 4.34(s 2H), 6.60(d, J = 8.4 Hz, 2H), 6.88(d, J = 8.4 Hz, 5H), 7.28-7.31(m, 3H), 7.54(d, J = 8.0 Hz, 2H), 12-47(brs, 1H) |
| 4-29 | DMSO-d6, 300MHz | 0.79(t, J = 7.1 Hz, 6H), 1.01-1.14(m, 4H), 1.25(s, 9H), 1.38-1.58(m, 4H), 2.41-2.50(m, 1H), 3.39(s, 2H), 3.95(s, 2H), 4.03(s, 2H), 5.06(s, 2H), 6.75(s, 1H), 6.93(d, J = 8.4 Hz, 2H), 7.07(d, J = 8.4 Hz, 2H), 7.40(s, 1H), 7.47(d, J = 8.4 Hz, 2H), 7.69(d, J = 8.4 Hz, 2H), 7.78(s, 1H), 12.40(brs, 1H) |

**Table 17-66**

| Example | solvent, Hz | NMR(δ) |
|---|---|---|
| 4-30 | DMSO-d6, 400MHz | 0.79(t, J = 7.3 Hz, 6H), 1.00-1.14(m, 4H), 1.39-1.56(m, 4H), 2.41-2.49(m, 1H), 3.36(s, 2H), 3.86(s, 3H), 4.06(s, 2H), 4.17(s, 2H), 5.05(s, 2H), 6.91(d, J = 8.6 Hz, 2H), 7.05(d, J = 8.8 Hz, 2H), 7.16(t, J = 7.4 Hz, 1H), 7.23(t, J = 7.4 Hz, 1H), 7.41(s, 1H), 7.44(d, J = 8.1 Hz, 2H), 7.52(d, J = 7.4 Hz, 1H), 7.59(d, J = 7.4 Hz, 1 H), 7.65(d, J = 8.1 Hz, 2H), 7.75(s, 1 H) |
| 4-31 | DMSO-d6, 300MHz | 0.79(t, J=7.2Hz, 6H), 0.99-1.14(m, 4H), 1.38-1.59(m, 4H), 2.41-2.46(m, 1H), 5.06(s, 2H), 5.62(s, 2H), 6.92(d, J=8.4Hz, 2H), 7.05-7.11(m, 3H), 7.30(d, J=3.6Hz, 1H), 7.42(d, J=3.3Hz, 1H), 7.48(d, J=8.1 Hz, 2H), 7.65(d, J=7.8Hz, 2H), 7.71(d, J=7.2Hz, 1 H), 7.90(dd, J=8.1, 7.2Hz, 1H) |
| 4-32 | DMSO-d6, 300MHz | 0.69 (t, J = 7.17 Hz, 6H), 1.16 (d, J = 6.59 Hz, 6H), 1.35-1.47 (m, 2H), 1.48-1.63 (m, 2H), 2.07-2.18 (m, 1H), 4.22 (quint, J = 6.59 Hz, 1H), 4.35 (s, 2H), 5.53 (s, 2H), 6.62 (d, J = 8.66 Hz, 2H), 6.68 (dd, J = 8.10, 0.75 Hz, 1H), 6.88 (d, J = 8.66 Hz, 2H), 7.30 (d, J = 8.28 Hz, 2H), 7.41 (dd, J = 7.14, 0.75 Hz, 1H), 7.61 (dd, J = 8.10, 7.14 Hz, 1H), 7.64 (d, J = 8.28 Hz, 2H), 7.76 (d, J = 1.5 Hz, 1 H), 7.78 (d, J = 1.5 Hz, 1H) |
| 4-33 | DMSO-d6, 300MHz | 0.68(t, J = 7.1 Hz, 6H), 1.14(d, J = 6.8 Hz, 6H), 1.33(s, 9H), 1.35-1.60(m, 4H), 2.07-2.18(m, 1H), 3.41(s, 2H), 4.08(s, 4H), 4.22(sep, J = 6.6 Hz, 1H), 4.35(s, 2H), 6.61(d, J = 8.8 Hz, 2H), 6.88(d, J = 8.8 Hz, 2H), 7.30(d, J = 8.4 Hz, 2H), 7.39(s, 1H), 7.41(s, 1H), 7.60(d, J = 8.4 Hz, 2H), 7.72(s, 1H), 12.48(brs, 1H) |
| 4-34 | DMSO-d6, 300MHz | 0.68(t, J = 7.3 Hz, 6H), 1.15(d, J = 6.6 Hz, 6H), 1.32-1.47(m, 2H), 1.49-1.62(m, 2H), 2.08-2.17(m, 1H), 3.37(s, 2H), 3.86(s, 3H), 4.06(s, 2H), 4.17(s, 2H), 4.22(sep, J = 6.6 Hz, 1H), 4.34(s, 2H), 6.61 (d, J = 8.8 Hz, 2H), 6.88(d, J = 8.8 Hz, 2H), 7.16-7.30(m, 4H), 7.38(brs, 1H), 7.52-7.61(m, 4H), 7.67(brs, 1H) |

**Table 17-67**

| Example | solvent, Hz | NMR(δ) |
|---|---|---|
| 4-35 | DMSO-d6, 400MHz | 0.79(t, J=7.4Hz, 6H), 1.01-1.14(m, 4H), 1.27(s, 9H), 1.39-1.56(m, 4H), 2.41-2.48(m, 1H), 3.42(s, 2H), 4.13(s, 2H), 5.06(s, 2H), 6.92(d, J=8.8Hz, 2H), 7.00(d, J=3.6Hz, 1H), 7.06(d, J=8.4Hz, 2H), 7.16(s, 1H), 7.36(d, J=3.6Hz, 1H), 7.46(d, J=8.4Hz, 2H), 7.63(d, J=8.4Hz, 2H) |
| 4-36 | DMSO-d6, 300MHz | 0.69(t, J=7.2Hz, 6H), 1.15(d, J=6.6Hz, 6H), 1.32-1.63(m, 4H), 2.07-2.18(m, 1H), 3.36(s, 2H), 3.87(s, 3H), 4.03(s, 2H), 4.16(s, 2H), 4.18-4.26(m, 1H), 4.34(s, 2H), 6.61 (d, J=8.7Hz, 2H), 6.89(d, J=8.4Hz, 2H), 6.99(d, J=3.6Hz, 1 H), 7.16-7.31(m, 5H), 7.53(brd, J=8.4Hz, 3H), 7.60(d, J=7.2Hz, 1H) |
| 4-37 | DMSO-d6, 300MHz | 0.80(t, J=7.3Hz, 6H), 0.98-1.16(m, 4H), 1.34(s, 9H), 1.37-1.59(m, 4H), 2.39-2.53(m, 1H), 3.41 (s, 2H), 4.06(s, 2H), 4.10(s, 2H), 5.07(s, 2H), 6.93(d, J=7.9Hz, 2H), 7.01(d, J=3.0Hz, 1H), 7.07(d, J=8.3Hz, 2H), 7.37(d, J=3.4Hz, 1H), 7.41-7.43(m, 1H), 7.48(d, J=7.9Hz, 2H), 7.64(d, J=7.9Hz, 2H), 12.51(brs, 1H) |
| 4-38 | DMSO-d6, 300MHz | 0.79(t, J=7.3Hz, 6H), 0.98-1.17(m, 4H), 1.36-1.59(m, 4H), 2.39-2.54(m, 1H), 2.94(s, 2H), 3.91(s, 2H), 4.03(s, 3H), 4.16(s, 2H), 5.05(s, 2H), 6.86-6.98(m, 3H), 7.06(d, J=8.7Hz, 2H), 7.12-7.26(m, 2H), 7.30(d, J=3.4Hz, 1H), 7.44(d, J=8.3Hz, 2H), 7.51(d, J=7.2Hz, 1H), 7.55-7.63(m, 3H) |
| 4-39 | DMSO-d6, 300MHz | 0.79(t, J=7.3Hz, 6H), 0.97-1.17(m, 4H), 1.34-1.60(m, 4H), 2.37-2.55(m, 1H), 3.02(brs, 2H), 3.95(brs, 5H), 4.14(brs, 2H), 5.04(brs, 2H), 6.91(brd, J=8.3Hz, 3H), 7.06(d, J=8.6Hz, 2H), 7.10-7.27(m, 2H), 7.27-7.35(m, 1H), 7.35-7.68(m, 6H) |

**Table 17-68**

| Example | solvent, Hz | NMR(δ) |
|---|---|---|
| 4-40 | DMSO-d6, 300MHz | 0.79(t, J=7.3Hz, 6H), 0.95-1.20(m, 4H), 1.32-1.60(m, 4H), 2.28(s, 3H), 2.40-2.54(m, 1H), 3.62(s, 2H), 4.00(s, 3H), 4.19(s, 2H), 4.46(s, 2H), 5.05(s, 2H), 6.92(d, J=8.6Hz, 2H), 6.98-7.14(m, 5H), 7.25(d, J=3.6Hz, 1H), 7.36-7.58(m, 8H), 7.76(d, J=9.0Hz, 1H), 7.89(d, J=9.0Hz, 1H) |
| 4-41 | DMSO-d6, 300MHz 1H), | 0.80(t, J=7.3Hz, 6H), 0.98-1.15(m, 4H), 1.38-1.59(m, 4H), 2.39-2.54(m, 1 H), 5.07(s, 2H), 5.52(s, 2H), 6.93(d, J=8.6Hz, 2H), 7.07(d, J=8.7Hz, 2H), 7.49(d, J=8.3Hz, 2H), 7.71(s, 1H), 7.72(d, J=6.4Hz, 2H), 7.93(d, J=1.5Hz, 1H), 8.00(brs, 8.65-8.67(m, 1H), 8.73-8.76(m, 1H) |
| 4-42 | DMSO-d6, 300MHz | 0.79(t, J=7.3Hz, 6H), 1.00-1.14(m, 4H), 1.37-1.60(m, 4H), 2.40-2.54(m, 1H), 3.80(brs, 3H), 4.08-4.62(m, 2H), 4.92-5.36(m, 4H), 6.92(d, J=8.6Hz, 2H), 7.07(d, J=8.7Hz, 2H), 7.16-7.80(m, 10H) |
| 4-43 | DMSO-d6, 300MHz | 0.79(t, J=7.3Hz, 6H), 0.93-1.15(m, 4H), 1.16-1.60(m, 13H), 2.36-2.55(m, 1H), 4.16(brs, 2Hx0.45), 4.43(brs, 2Hx0.55), 4.70-5.25(m, 4H), 6.93(d, J=8.4Hz, 2H), 7.07(d, J=8.4Hz, 2H), 7.29-7.62(m, 5H), 7.72-7.75(m, 2H) |
| 4-44 | DMSO-d6, 300MHz | 0.79(t, J=7.3Hz, 6H), 0.97-1.15(m, 4H), 1.20(d, J=6.8Hz, 6H), 1.35-1.59(m, 4H), 2.39-2.54(m, 1H), 2.81-2.96(m, 1H), 4.01(brs, 2Hx0.60), 4.23(brs, 2Hx0.40), 4.63(brs, 2Hx0.40), 4.88(brs, 2Hx0.60), 5.08(s, 2H), 6.92(d, J=8.7Hz, 2H), 7.06(d, J=8.7Hz, 2H), 7.16-7.40(m, 5H), 7.41-7.56(m, 3H), 7.71(d, J=7.9Hz, 2H) |
| 4-45 | DMSO-d6, 300MHz | 0.79(t, J=7.2Hz, 6H), 0.97-1.19(m, 4H), 1.35-1.60(m, 4H), 237-256(m, 1H), 289(s, 6H), 3.84-4.30(m, 2H), 4.40-4-93(m, 2H), 5.09(s, 2H), 6.73(brd, J=7.2Hz, 2H), 6.93(d, J=8.6Hz, 2H), 7.07(d, J=8.7Hz, 2H), 7.15(brd, J=7.9Hz, 2H), 7.31(d, J=3.8Hz, 1 H), 7.44-7.57(m, 3H), 7.72(d, J=8.3Hz, 2H) |
| 4-46 | DMSO-d6, 300MHz | 0.79(t, J=7.2Hz, 6H), 0.95-1.18(m, 4H), 1.35-1.61(m, 4H), 2.39-2.55(m, 1H), 4.11(brs, 2Hx0.62), 4.46(brs, 2Hx0.38), 4.77(brs, 2Hx0.38), 4.99(brs, 2Hx0.62), 5.08(s, 2H), 6.92(d, J=8.7Hz, 2H), 7.07(d, J=8.7Hz, 2H), 7.25-7.60(m, 6H), 7.61-7.92(m, 3H), 8.42-8.72(m, 1H) |

**Table 17-69**

| Example | solvent, Hz | NMR(δ) |
|---|---|---|
| 4-47 | DMSO-d6, 300MHz | 0.79(t, J=7.4Hz, 6H), 0.97-1.18(m, 4H), 1.34-1.60(m, 4H), 2.37-2.55(m, 1H), 3.94-4.50(m, 2H), 4.59-5.07(m, 2H), 5.09(s, 2H), 6.93(d, J=8.7Hz, 2H), 7.07(d, J=8.7Hz, 2H), 7.19-7.58(m, 5H), 7.72(d, J=7.9Hz, 2H), 7.79(d, J=7.5Hz, 1H), 8.51(d, J=4.5Hz, 1H), 8.58(s, 1H) |
| 4-48 | DMSO-d6, 300MHz | 0.80(t, J=7.3Hz, 6H), 1.00-1.15(m, 4H), 1.38-1.58(m, 4H), 2.40(s, 3H), 2.42-2.50(m, 1H), 5.07(s, 2H), 5.53(s, 2H), 6.93(d, J=8.6Hz, 2H), 7.07(d, J=8.6Hz, 2H), 7.49(d, J=8.3Hz, 2H), 7.71-7.75(m, 3H), 7.93(d, J=1.5Hz, 1H), 8.04(brs, 1H), 8.69(d, J=2.6Hz, 1H), 8.76(d, J=1.5Hz, 1H) |
| 4-49 | DMSO-d6, 300MHz (d, J = | 0.79 (t, J = 7.3 Hz, 6 H),0.97- 1.18 (m, 4 H),1.35- 1.61 (m, 4 H),2.39- 2.53 (m, 1 H),3.09 (s, 3 H),3.91 (s, 2 H),4.62 (s, 2 H),5.07 (s, 2 H),6.93 (d, J = 8.7 Hz, 2 H),7.03- 7.12 (m, 3 H),7.39 (d, J = 3.8 Hz, 1 H),7.48 (d, J = 8.5 Hz, 2 H),7.66 8.5 Hz, 2 H),12.95 (br s, 1 H). |
| 4-50 | DMSO-d6, 300MHz | 0.80(t, J=7.3Hz, 6H), 0.99-1.17(m, 4H), 1.37-1.58(m, 4H), 2.40-2.54(m, 1 H), 5.07(s, 2H), 5.39(s, 2H), 6.93(d, J=8.3Hz, 2H), 7.07(d, J=8.6Hz, 2H), 7.48(d, J=8.3Hz, 2H), 7.67(brs, 1H), 7.72(d, J=8.3Hz, 2H), 7.79(dd, J=1.5, 3.0Hz, 1 H), 7.89(d, J=1.5Hz, 1H), 8.26(d, J=3.0Hz, 1 H), 8.60(d, J=1.1Hz, 1H) |
| 4-51 | DMSO-d6, 300MHz | 0.80(t, J=7.3Hz, 6H), 1.00-1.15(m, 4H), 1.38-1.58(m, 4H), 2.39-2.54(m, 1H), 5.07(s, 2H), 5.52(s, 2H), 6.93(d, J=8.6Hz, 2H), 7.07(d, J=8.6Hz, 2H), 7.49(d, J=8.3Hz, 2H), 7.71(s, 1H), 7.72(d, J=6.8Hz, 2H), 7.93(d, J=1.5Hz, 1H), 7.99(brs, 1H), 8.65-8.67(m, 1H), 8.73-8.75(m, 1H) |
| 4-52 | DMSO-d6, 300MHz | 0.80(t, J=7.3Hz, 6H), 0.97-1.19(m, 4H), 1.36-1.62(m, 4H), 2.40-2.55(m, 1H), 3.45(brs, 4H), 3.84(brs, 4H), 5.10(s, 2H), 6.96(dd, J=8.7, 12.8Hz, 4H), 7.08(d, J=8.3Hz, 2H), 7.46-7.59(m, 4H), 7.78(dd, J=8.3, 15.5Hz, 4H) |
| 4-53 | DMSO-d6, 300MHz | 0.79(t, J=7.2Hz, 6H), 0.98-1.19(m, 4H), 1.36-1.59(m, 4H), 1.60-1.80(m, 2H), 1.88(brd, J=11.3Hz, 2H), 2.40-2.54(m, 1H), 2.88-3.02(m, 1 H), 3.03-3.22(m, 2H), 4.46(brd, J=13.2Hz, 2H), 5.09(s, 2H), 6.93(d, J=8.7Hz, 2H), 7.07(d, J=8.7Hz, 2H), 7.38-7.58(m, 6H), 7.74(d, J=8.3Hz, 2H), 7.89(d, J=8.3Hz, 2H) |

**Table 17-70**

| Example | solvent, Hz | NMR(δ) |
|---|---|---|
| 4-54 | DMSO-d6, 300MHz | 0.79 (t, J = 7.4 Hz, 6 H),0.98- 1.18 (m, 4 H),1.36- 1.60 (m, 4 H),2.39- 2.53 (m, 1 H),3.64 (s, 2 H),3.99 (s, 3 H),4.21 (s, 2 H),4.48 (s, 2 H),5.06 (s, 2 H),6.93 (d, J = 8.7 Hz, 2 H),7.07 (d, J = 8.7 Hz, 2 H),7.40- 7.68 (m, 8 H),7.71- 7.76 (m, 1 H),7.85- 7.91 (m, 1 H). |
| 4-55 | 4.48(s, DMSO-d6, 300MHz | 0.79(t, J=7.3Hz, 6H), 0.98-1.18(m, 4H), 1.36-1.59(m, 4H), 2.40-2.50(m, 1H), 3.64(s, 2H), 3.99(s, 3H), 4.22(s, 2H), 2H), 5.06(s, 2H), 6.93(d, J=8.8Hz, 2H), 7.07(d, J=8.8Hz, 2H), 7.42-7.63(m, 7H), 7.66(s, 1H), 7.75(d, J=8.0Hz, 1H), 7.89(d, J=8.8Hz, 1H) |
| 4-56 | DMSO-d6, 300MHz 1 H),8.35 (d, J = | 0.79 (t, J = 7.2 Hz, 6 H),0.96- 1.18 (m, 4 H),1.35- 1.60 (m, 4 H),2.33 (s, 6 H),2.38- 2.54 (m, 1 H),5.07 (s, 2 H),5.51 (s, 2 H),6.93 (d, J = 8.7 Hz, 2 H),7.07 (d, J = 8.7 Hz, 2 H),7.49 (d, J = 7.9 Hz, 2 H),7.60- 7.78 (m, 5 H),7.93 (d, J = 1.1 Hz, 8.3 Hz, 1H),9.99 (s, 1 H),12.76 (br s, 1 H). |
| 4-57 | DMSO-d6, 300MHz | 0.80 (t, J = 7.2 Hz, 6 H),0.97- 1.16 (m, 4 H),1.10 (d, J = 6.8 Hz, 6 H),1.35- 1.60 (m, 4 H),2.39- 2.53 (m, 1 H),2.71-2.87 (m, 1 H),5.07 (s, 2 H),5.48 (s, 2 H),6.93 (d, J = 8.6 Hz, 2 H),7.07 (d, J = 8.6 Hz, 2 H),7.49 (d, J = 8.3 Hz, 2 H),7.56 (dd, J = 8.6, 1.4 Hz, 1 H),7.66- 7.73 (m, 4 H),7.90 (d, J = 1.4 Hz, 1 H),8.11 (d, J = 8.6 Hz, 1 H),9.10 (s, 1 H),12.85 (br s, 1H). |
| 4-58 | DMSO-d6 300MHz | 0.80 (t, J = 7.2 Hz, 6 H),0.97- 1.18 (m, 4 H),1.34- 1.61 (m, 4 H),2.38- 2.54 (m, 1 H),5.07 (s, 2 H),5.40 (s, 2 H),6.93 (d, J = 8.7 Hz, 2 H),7.07 (d, J = 8.7 Hz, 2 H),7.14 (d, J = 8.7 Hz, 2 H),7.49 (d, J = 7.9 Hz, 2 H),7.68- 7.75 (m, 3 H),7.87-7.94 (m, 3 H),12.66 (br s, 1 H). |
| 4-59 | DMSO-d6, 300MHz | 0.80 (t, J = 7.2 Hz, 6 H),0.98- 1.18 (m, 4 H),1.36- 1.59 (m, 4 H),2.39- 2.54 (m, 1 H),2.99 (s, 3 H),3.18 (s, 3 H),5.07 (s, 2 H),5.51 (s, 2 H),6.93 (d, J = 8.5 Hz, 2 H),7.07 (d, J = 8.5 Hz, 2 H),7.43 (d, J = 8.0 Hz, 1 H),7.49 (d, J = 8.1 Hz, 2 H),7.58 (dd, J = 8.0, 1.5 Hz, 1H),7.70 (s, 1 H),7.71 (d, J = 8.1 Hz, 2 H),7.78 (d, J = 1.5 Hz, 1 H),7.92 (s, 1 H),13.17 (br s, 1 H). |

**Table 17-71**

| Example | solvent, Hz | NMR(δ) |
|---|---|---|
| 4-60 | DMSO-d6, 300MHz (d, J = | 0.79 (t, J = 7.2 Hz, 6 H),0.98- 1.18 (m, 4 H),1.35- 1.60 (m, 4 H),2.39- 2.54 (m, 1 H),4.63 (s, 2 H),5.06 (s, 2 H),6.93 (d, J = 8.7 Hz, 2 H),7.06 (d, J = 8.7 Hz, 2 H),7.42- 7.54 (m, 5 H),7.66 (d, J = 8.3 Hz, 2 H),7.73 (d, J = 1.1 Hz, 1 H),7.86 8.3 Hz, 2 H),12.90 (br s, 1 H). |
| 4-61 | DMSO-d6, 300MHz | 0:80 (t, J = 7.3 Hz, 6 H),0.99- 1.19 (m, 4 H),1.36- 1.60 (m, 4 H),2.40- 2.54 (m, 1 H),5.07 (s, 2 H),5.33 (s, 2 H),5.54 (s, 2 H),6.67 (d, J = 8.0 Hz, 1 H),6.93 (d, J = 8.7 Hz, 2 H),7.07 (d, J = 8.7 Hz, 2 H),7.39 (dd, J = 8.0, 1.6 Hz, 1 H),7.45-7.52 (m, 3 H),7.65- 7.76 (m, 3 H),7.88 (d, J = 1.6 Hz, 1 H), 12.11 (br s, 1 H). |
| 4-62 | DMSO-d6, 400MHz | 1.11-1.41(m, 5H), 1.61-1.81(m, 5H), 2.00-3.68(m, 9H), 4.30-4.76(m, 2H), 5.41(s, 2H), 7.09-7.44(m, 13H), 7.57(d, J=7.9Hz, 1H), 7.88(d, J=7.9Hz, 1H), 7.96(s, 1H) |
| 4-63 | DMSO-d6, 300MHz | 1.12-1.44(m, 5H), 1.60-1.83(m, 5H), 2.37-2.48(m, 1H), 3.28(s, 2H), 3.83(s, 2H), 4.00(s, 2H), 5.40(s, 2H), 6.97(d, J=3.7Hz, 1 H), 7.11-7.44(m, 12H), 7.52-7.57(m, 1H), 7.85-7.90(m, 1H), 7.88(s, 1H), 12.35(brs, 1H) |
| 4-64 | DMSO-d6, 300MHz | 0.79(t, J=7.3Hz, 6H), 0.99-1.15(m, 4H), 1.38-1.59(m, 4H), 2.39-2.53(m, 1H), 3.18(s, 3H), 3.85(s, 2H), 4.65(s, 2H), 5.07(s, 2H), 6.93(d, J=8.7Hz, 2H), 7.07(d, J=8.6Hz, 2H), 7.25-7.35(m, 1H), 7.39-7.43(m, 4H), 7.45-7.51 (m, 3H), 7.68(d, J=8.2Hz, 2H), 7.84(d, J=1.6Hz, 1H) |
| 4-65 | DMSO-d6, 300MHz | 1.08-1.39(m, 5H), 1.27(s, 9H), 1.61-1.77(m, 5H), 2.34-2.47(m, 1H), 3.16(brs, 2H), 3.35(brs, 3H), 4.01 (brs, 2H), 4.08(brs, 2H), 6.97(brd, J=2.6Hz, 1H), 7.06(brd, J=8.7Hz, 2H), 7.10(brd, J=8.7Hz, 2H), 7.24(brd, J=8.7Hz, 2H), 7.32(brd, J=3.0Hz, 1H), 7.37-7.43(m, 3H) |

**Table 17-72**

| Example | solvent, Hz | NMR(δ) |
|---|---|---|
| 4-66 | DMSO-d6, 300MHz | 1.08-1.45(m, 5H), 1.13(t, J=7.0Hz, 3H), 1.62-1.84(m, 5H), 2.38-2.54(m, 1H), 3.32(s, 2H), 3.48(q, J=6.9Hz, 2H), 3.92(s, 2H), 3.97(s, 2H), 4.51(s, 2H), 6.67(d, J=8.7Hz, 2H), 6.88(d, J=3.4Hz, 2H), 7.04(d, J=3.8Hz, 2H), 7.12(d, J=8.7Hz, 2H), 7,16(d, J=8.3Hz, 2H), 7.24-7.28(m, 1H), 7.36(d, J=8.7Hz, 2H), 7.54(d, J=7.5Hz, 1 H), 7.77-7.83(m, 1H), 8.48(d, J=4.1Hz, 1H), 12.45(brs, 1H) |
| 4-67 | DMSO-d6, 300MHz | 1.09-1.44(m, 5H), 1.13(t, J=7.0Hz, 3H), 1.64-1.82(m, 5H), 2.38-2.51(m, 1H), 3.03(t, J=5.8Hz, 2H), 3.47(q, J=7.1Hz, 2H), 3.44(s, 2H), 4.05(t, J=7.2Hz, 2H), 4.03(s, 2H), 4.51(s, 2H), 6.65(s, 2H), 6.68(s, 2H), 6.85-6.94(m, 4H), 7.03(d, J=3.4Hz, 1H), 7.12(d, J=8.3Hz, 2H), 7.16(d, J=8.3Hz, 2H), 7.26(t, J=7.9Hz, 2H), 7.35(d, J=8.7Hz, 2H), 12.31(brs, 1H) |
| 4-68 | DMSO-d6, 300MHz | 1.02-1.47(m, 4H), 1.08(t, J=6.8Hz, 3H), 1.24(s, 9H), 1.61-1.87(m, 9H), 2.05-2.16(m, 2H), 2.38-2.47(m, 1H), 2.53-2.65(m, 1H), 3.02(brd, J=11.3Hz, 2H), 3.16(brd, J=6.4Hz, 2H), 3.41(q, J=6.9Hz, 2H), 3.72(s, 2H), 6.68(d, J=8.7Hz, 2H), 7.12(d, J=8.3Hz, 2H), 7.23-7.30(m, 3H), 7.38(d, J=7.9Hz, 2H), 7.42(s, 1H), 7.46(d, J=8.3Hz, 2H), 7.87(d, J=8.3Hz, 2H), 12.78(brs, 1H) |
| 4-69 | DMSO-d6, 300MHz | 0.79(t, J=7.4Hz, 6H), 1.01-1.21(m, 4H), 1.15(d, J=7.2Hz, 6H), 1.37-1.59(m, 4H), 2.40-2.51 (m, 1H), 2.72-2.87(m, 1H), 3.95(s, 2H), 4.65(s, 2Hx0.75), 4.74(s, 2Hx0.75), 4.85(s, 2Hx0.25), 4.91(s, 2Hx0.25), 5.05(s, 2H), 6.79-6.86(m, 2H), 6.92(d, J=8.3Hz, 2H), 7.03-7.13(m, 4H), 7.42-7.57(m, 3H), 7.62-7.85(m, 3H) |
| 4-70 | DMSO-d6, 300MHz | 0.79(t, J=7.2Hz, 6H), 0.99-1.15(m, 4H), 1.21(d, J=6.8Hz, 6H), 1.37-1.58(m, 4H), 2.40-2.51(m, 1H), 2.86-3.00(m, 1 H), 3.93(s, 2Hx0.5), 4.08(s, 2Hx0.5), 4.69(s, 2Hx0.5), 4.81(s, 2Hx0.5), 5.07(s, 2H), 6.93(d, J=8.7Hz, 2H), 7.07(d, J=8.7Hz, 2H), 7.26-7.61(m, 7H), 7.71 (d, J=7.9Hz, 2H), 7.81(s, 1H) |
| 4-71 | DMSO-d6, 300MHz | 0.79(t, J=7.3Hz, 6H), 0.84(d, J=6.8Hz, 6H), 1.00-1.17(m, 4H), 1.29-1.65(m, 7H), 2.41-2.51 (m, 1H), 2.63(t, J=7.3Hz, 2H), 3.30(s, 2H), 3.99(s, 2H), 5.06(s, 2H), 6.93(d, J=8.7Hz, 2H), 7.07(d, J=8.7Hz, 2H), 7.40(brs, 1H), 7.46(d, J=8.3Hz, 2H), 7.69(d, J=8.3Hz, 2H), 7.76(d, J=1.1 Hz, 1 H), 12.22(brs, 1H) |

**Table 17-73**

| Example | solvent, Hz | NMR(δ) |
|---|---|---|
| 4-72 | DMSO-d6, 300MHz | 0.79(t, J=7.3Hz, 6H), 0.97-1.16(m, 4H), 1.37-1.58(m, 4H), 2.39-2.50(m, 1H), 3.11(s, 3H), 3.70(s, 2H), 4.46(s, 2H), 5.06(s, 2H), 6.93(d, J=8.7Hz, 2H), 7.06(d, J=8.3Hz, 2H), 7-14(t, J=7.2Hz, 1 H), 7.25-7.41 (m, 5H), 7.47(d, J=8.3Hz, 2H), 7.66(d, J=8.3Hz, 2H), 7.75(d, J=1.1 Hz, 1 H), 11.96(brs, 1H) |
| 4-73 | 3.91(s, DMSO-d6, 300MHz | 0.79(t, J=7.4Hz, 6H), 1.00-1.15(m, 4H), 1.36-1.58(m, 4H), 2.41-2.50(m, 1H), 2.74(br, 2H), 2.85(br, 2H), 3.72(s, 2H), 2H), 5.09(s, 2H), 6.95(d, J=8.3Hz, 2H), 7.07(d, J=8.3Hz, 2H), 7.23(d, J=7.9Hz, 1H), 7.34-7.46(m, 3H), 7.48(s, 1H), 7.61-7.73(m, 3H), 7.79(s, 2H), 12.74(br, 1H) |
| 4-74 | DMSO-d6, 300MHz | 0.91(d, J=6.4Hz, 3H), 0.98-1.13(m, 2H), 1.34-1.52(m, 3H), 1.71-1.82(m, 4H), 2.38-2.49(m, 1H), 2.51(br, 4H), 3.27(br, 4H), 3.72(s, 2H), 5.07(s, 2H), 6.92-6.98(m, 3H), 7.03(d, J=9.0Hz, 2H), 7.21(s, 1H), 7.23(d, J=8.3Hz, 2H), 7.35(d, J=8.3Hz, 2H), 7.54(d, J=8.7Hz, 2H), 7.76(d, J=8.7Hz, 2H), 12.25(s, 1H) |
| 4-75 | DMSO-d6, 300MHz | 0.79(t, J=7.2Hz, 6H), 1.01-1.14(m, 4H), 1.38-1.58(m, 4H), 2.41-2.49(m, 1H), 3.41 (s, 2H), 4.04(s, 2H), 5.06(s, 2H), 6.92(d, J=8.7Hz, 2H), 6.98(d, J=3.8Hz, 1 H), 7.07(d, J=8.7Hz, 2H), 7.34(d, J=3.8Hz, 1H), 7.47(d, J=8.3Hz, 2H), 7.60(d, J=8.3Hz, 2H), 7.63-7.69(m, 2H), 8.06(s, 1H), 12.47(br, 1H) |
| 4-76 | DMSO-d6, 300MHz | 0.79(t, J=7.3Hz, 6H), 1.00-1.14(m, 4H), 1.38-1.57(m, 4H), 2.39-2.50(m, 1H), 3.29(s, 2H), 3.88(s, 2H), 4.00(s, 2H), 5.07(s, 2H), 6.93(d, J=8.7Hz, 2H), 7.00(d, J=3.4Hz, 1H), 7.07(d, J=8.7Hz, 2H), 7.37(d, J=3.4Hz, 1 H), 7.47(d, J=8.3Hz, 2H), 7.51(d, J=8.3Hz, 2H), 7.65(d, J=8.3Hz, 2H), 7.93(d, J=8.3Hz, 2H), 12.52(br, 2H) |
| 4-77 | DMSO-d6, 300MHz | 0.79(t, J=7.3Hz, 6H), 1.01-1.16(m, 4H), 1.38-1.58(m, 4H), 2.40-2.50(m, 1H), 3.23(s, 2H), 3.70(br, 5H), 3.97(s, 2H), 5.07(s, 2H), 6.88-6.96(m, 4H), 6.98(d, J=3.8Hz, 1H), 7.07(d, J=8.7Hz, 2H), 7.28(d, J=8.7Hz, 2H), 7.36(d, J=3.4Hz, 1H), 7.47(d, J=8.7Hz, 2H), 7.65(d, J=8.3Hz, 2H), 12.11(br, 1H) |

**Table 17-74**

| Example | solvent, Hz | NMR(δ) |
|---|---|---|
| 4-78 | DMSO-d6, 300MHz | 0.79(t, J=7.4Hz, 6H), 1.01-1.16(m, 4H), 1.38-1.58(m, 4H), 2.46(s, 3H), 3.25(s, 2H), 3.75(s, 2H), 3.98(s, 2H), 5.07(s, 2H), 6.93(d, J=8.7Hz, 2H), 6.99(d, J=3.4Hz, 1H), 7.07(d, J=8.7Hz, 2H), 7.24(d, J=8.3Hz, 2H), 7.32(d, J=8.3Hz, 2H), 7.36(d, J=3.4Hz, 1H), 7.47(d, J=8.3Hz, 2H), 7.65(d, J=8.3Hz, 2H), 12.28(br, 1H) |
| 4-79 | DMSO-d6, 300MHz-120°C | 0.73-0.96(m, 5H), 0.98-1.31(m, 5H), 1.34-1.89(m, 13H), 3.20(d, J=7.0Hz, 2H), 4.70(s, 2H), 5.08(s, 2H), 6.93(d, J=3.7Hz, 1 H), 7.02(d, J=8.8Hz, 2H), 7.13(d, J=3.7Hz, 1 H), 7.22(d, J=8.1Hz, 2H), 7.26-7.37(m, 4H), 7.50(d, J=8.8Hz, 2H), 7.69(d, J=8.4Hz, 2H) |
| 4-80 | DMSO-d6 300MHz | 0.87-1.25(m, 10H), 1.32-1.53(m, 3H), 1.55-1.82(m, 10H), 2.38-2.49(m, 1H), 3.25(d, J=6.8Hz, 2H), 4.71 (s, 2H), 5.06(s, 2H), 6.98-7.03(m, 3H), 7.18-7.25(m, 3H), 7.34(d, J=8.3Hz, 2H), 7.50(d, J=8.7Hz, 2H), 7.61(d, J=9.0Hz, 2H), 7.83(d, J=8.7Hz, 2H), 8.70(s, 1H), 12.35(br, 1H) |
| 4-81 | DMSO=d6, 300MHz | 0.80(t, J=7.3Hz, 6H), 1.00-1.17(m, 4H), 1.37-1.58(m, 4H), 2.40-2.50(m, 1H), 3.22(s, 2H), 3.97(s, 2H), 5.07(s, 2H), 5.33(s, 1H), 6.90-6.98(m, 3H), 7.07(d, J=8.7Hz, 2H), 7.19-7.27(m, 2H), 7.30-7.39(m, 5H), 7.45-7.56(m, 6H), 7.68(d, J=8.3Hz, 2H), 12.29(br, 1H) |
| 4-82 | DMSO-d6, 300MHz | 0.79(t, J=7.2Hz, 6H), 1.00-1.27(m, 7H), 1.34-1.61(m, 4H), 1.97(brs, 4H), 3.34(brs, 4H), 4.07-4.19(m, 2H), 4.60(brs, 2H), 4.76(brs, 2H), 5.08(s, 2H), 6.59(d, J=9.0Hz, 2H), 6.92(d, J=8.7Hz, 2H), 7.07(d, J=8.7Hz, 2H), 7.30(brs, 1H), 7.41-7.53(m, 3H), 7.65(d, J=7.5Hz, 2H), 7.76(d, J=7.9Hz, 2H) |
| 4-83 | DMSO-d6, 300MHz | 0.79(t, J=7.3Hz, 6H), 1.00-1.20(m, 4H), 1.15(t, J=7.2Hz, 3H), 1.38-1.58(m, 4H), 2.40-2.54(m, 1H), 3.44(s, 2H), 3.88(s, 3H), 4.04(q, J=7.2Hz, 2H), 4.02(s, 2H), 4.14(s, 2H), 5.06(s, 2H), 6.92(d, J=8.6Hz, 2H), 7.02(d, J=3.8Hz, 1H), 7.07(d, J=9.0Hz, 2H), 7.17(t, J=7.5Hz, 1 H), 7.24(t, J=7.7Hz, 1H), 7.36(d, J=3.8Hz, 1 H), 7.46(d, J=8.3Hz, 2H), 7.53(d, J=9.1Hz, 1H), 7.57-7.65(m, 3H) |

**Table 17-75**

| Example | solvent, Hz | NMR(δ) |
|---|---|---|
| 4-84 | DMSO-d6, 300MHz | 1.16-1.45(m, 5H), 1.65-1.83(m, 5H), 2.39-2.49(m, 3H), 2.70(t, J=7.2Hz, 2H), 2.93(t, J=8.1Hz, 2H), 3.31(t, J=8.5Hz, 2H), 3.61(s, 2H), 3.73(s, 2H), 4.27(s, 2H), 6.59(d, J=8.3Hz, 1H), 6.88(d, J=3.8Hz, 1 H), 7.06(d, J=3.8Hz, 1H), 7.16-7.40(m, 11H), 12.17(brs, 1H) |
| 4-85 | DMSO-d6, 300MHz | 1.03(s, 9H), 2.79(s, 2H), 3.24(s, 2H), 3.78(s, 2H), 4.00(s, 2H), 5.54(s, 2H), 7.05-7.18(m, 4H), 7.21-7.45(m, 7H), 7.58-7.64(m, 3H), 7.71(d, J=1.5Hz, 1H), 12.37(brs, 1H) |
| 4-86 | DMSO-d6, 300MHz | 0.79 (t, J = 7.4 Hz, 6 H),0.97- 1.17 (m, 4 H),1.34- 1.59 (m, 4 H),2.39- 2.55 (m, 1 H),3.26 (s, 2 H),3.76 (s, 3 H),3.96 (s, 2 H),4.03 (s, 2 H),5.06 (s, 2 H),6.93 (d, J = 8.7 Hz, 2 H),6.99- 7.20 (m, 4 H),7.26 (s, 1 H),7.36- 7.51 (m, 4 H),7.66- 7.80 (m, 4 H),12.22 (br s, 1 H). |
| 4-87 | DMSO-d6, 300MHz | 0.79 (t, J = 7.3 Hz, 6 H),0.98- 1.17 (m, 4 H),1.36- 1.58 (m, 4 H),2.38- 2.53 (m, 1 H),3.41 (s, 2 H),4.11 (s, 2 H),4.12 (s, 2 H),5.06 (s, 2 H),6.92 (d, J = 8.7 Hz, 2 H),7.07 (d, J = 8.7 Hz, 2 H),7.43- 7.48 (m, 3 H),7.54- 7.61 (m, 1 H),7.66- 7.79 (m, 5 H),7.94- 8.00 (m, 2 H),8.37 (d, J = 8.3 Hz, 1 H),12.54 (br s, 1 H). |
| 4-88 | DMSO-d6, 300MHz | 0.79 (t, J = 7.2 Hz, 6 H),0.97- 1.19 (m, 4 H),1.35- 1.60 (m, 4 H),2.39- 2.54 (m, 1 H),3.51 (s, 2 H),4.18 (s, 2 H),4.33 (s, 2 H),5.06 (s, 2 H),6.93 (d, J = 8.9 Hz, 2 H),7.07 (d, J = 8.9 Hz, 2 H),7.37- 7.53 (m, 5 H),7.70 (d, J = 8.3 Hz, 2 H),7.82 (d, J = 1.5 Hz, 1 H),7.90- 7.96 (m, 1 H),8.07- 8.13 (m, 1 H),12.58 (br s, 1 H). |
| 4-89 | DMSO-d6, 300MHz | 0.79 (t, J = 7.1 Hz, 6 H),0.98- 1.19 (m, 4 H),1.36- 1.60 (m, 4 H),2.38- 2.55 (m, 1 H),3.28 (s, 2 H),3.84 (s, 2 H),3.98 (s, 2 H),5.07 (s, 2 H),5.29 (s, 2 H),6.86 (d, J = 1.1 Hz, 1 H),6.93 (d, J = 8.4 Hz, 2 H),7.03- 7.31 (m, 8 H),7.40- 7.43 (m, 1 H),7.47 (d, J = 8.0 Hz, 2 H),7.68 (d, J = 8.0 Hz, 2 H),7.78 (d, J = 1.1 Hz, 1 H),12.62 (br s, 1 H). |

**Table 17-76**

| Example | solvent, Hz | NMR(δ) |
|---|---|---|
| 4-90 | DMSO-d6, 300MHz | 0.79 (t, J = 7.1 Hz, 6 H),0.97- 1.17 (m, 4 H),1.36- 1.59 (m, 4 H),2.39- 2.53 (m, 1 H),3.25 (s, 2 H),3.85 (s, 2 H),4.05 (s, 2 H),5.06 (s, 2 H),6.38- 6.41 (m, 1 H),6.93 (d, J = 8.8 Hz, 2 H),7.03- 7.15 (m, 3 H),7.29- 7.39 (m, 2 H),7.41- 7.52 (m, 4 H),7.70 (d, J = 8.1 Hz, 2 H),7.78 (d, J = 1.1 Hz, 1 H),11.04 (br s, 1 H),12.30 (br s, 1 H). |
| 4-91 | DMSO-d6, 300MHz | 0.79 (t, J = 7.3 Hz, 6 H),0.99- 1.16 (m, 4 H),1.36- 1.58 (m, 4 H),2.38- 2.54 (m, 1 H),3.31 (s, 2 H),3.85 (s, 2 H),4.05 (s, 2 H),5.07 (s, 2 H),6.93 (d, J = 8.8 Hz, 2 H),7.04- 7.12 (m, 3 H),7.42- 7.55 (m, 5 H),7.60- 7.75 (m, 5 H),7.79 (d, J = 1.4 Hz, 1 H),8.23- 8.26 (m, 1 H),12.42 (br s, 1 H). |
| 4-92 | DMSO-d6, 300MHz | 0.79 (t, J = 7.1 Hz, 6 H),0.98- 1.18 (m, 4 H),1.36- 1.59 (m, 4 H),2.39- 2.53 (m, 1 H),3.38 (s, 2 H),4.05 (s, 2 H),4.10 (s, 2 H),5.07 (s, 2 H),6.82 (s, 1 H),6.93 (d, J = 8.6 Hz, 2 H),7.07 (d, J = 8.6 Hz, 2 H),7.19- 7.32 (m, 2.H),7.44- 7.64 (m, 5 H),7.70 (d, J = 8.4 Hz, 2 H),7.79 (d, J = 1.4 Hz, 1 H),12.44 (br s, 1 H). |
| 4-93 | H),7.14 DMSO-d6, 300MHz | 0.79 (t, J = 7.3 Hz, 6 H),0.97- 1.17 (m, 4 H),1.35- 1.59 (m, 4 H),2.39- 2.53 (m, 1 H),3.37 (s, 2 H),4.02 (s, 2 H),4.07 (s, 2 H),5.06 (s, 2 H),6.89- 6.98 (m, 3 H),7.03- 7.10 (m, 3 (d, J = 3.7 Hz, 1 H),7.27 (dd, J = 3.3, 1.1 Hz, 1 H),7.42- 7.51 (m, 4 H),7.70 (d, J = 8.1 Hz, 2 H),7.79 (d, J = 1.1 Hz, 1 H),12.46 (br s, 1 H). |
| 4-94 | DMSO-d6, 300MHz | 0.79 (t, J = 7.2 Hz, 6 H),0.97- 1.17 (m, 4 H),1.35- 1.59 (m, 4 H),2.38- 2.54 (m, 1 H),3.34 (s, 2 H),3.83 (s, 2 H),4.01 (s, 2 H),5.06 (s, 2 H),6.93 (d, J = 8.7 Hz, 2 H),6.99- 7.11 (m, 4 H),7.27- 7.50 (m, 6 H),7.64 (d, J = 8.3 Hz, 2 H),7.93 (d, J = 7.9 Hz, 2 H),12.42 (br s, 1 H). |
| 4-95 | 2 DMSO-d6, 300MHz | 0.79 (t, J = 7.3 Hz, 6 H),0.98- 1.18 (m, 4 H),1.34- 1.59 (m, 4 H),2.38- 2.54 (m, 1 H),3.27 (s, 2 H),3.83 (s, 2 H),4.03 (s, H),5.06 (s, 2 H),6.89- 6.97 (m, 3 H),7.07 (d, J = 8.7 Hz, 2 H),7.26- 7.51 (m, 6 H),7.58- 7.66 (m, 3 H),7.79 (d, J = 7.1 Hz, 2 H),12.66 (br s, 2 H). |

**Table 17-77**

| Example | solvent, Hz | NMR(δ) |
|---|---|---|
| 4-96 | DMSO-d6, 300MHz | 0.79 (t, J = 7.3 Hz, 6 H),0.97- 1.17 (m, 4 H),1.33- 1.60 (m, 4 H),2.38- 2.53 (m, 1 H),3.51 (s, 2 H),4.15 (s, 2 H),4.21 (s, 2 H),5.06 (s, 2 H),6.93 (d, J = 8.7 Hz, 2 H),6.98- 7.11 (m, 3 H),7.32- 7.50 (m, 5 H),7.62 (d, J = 7.9 Hz, 2 H),7.68- 7.77 (m, 2 H),12.49 (br s, 1 H). |
| 4-97 | DMSO-d6, 300MHz | 0.79 (t, J = 7.3 Hz, 6 H),0.97- 1.18 (m, 4 H),1.36- 1.61 (m, 4 H),2.40- 2.54 (m, 1 H),3.40 (s, 2 H),4.08 (s, 2 H),4.16 (s, 2 H),5.07 (s, 2 H),6.88- 7.12 (m, 2 H),7.28- 7.41 (m, 3 H),7.48 (d, J = 8.3 Hz, 5 H),7.66 (d, J = 8.3 Hz, 2 H),7.73-7.81 (m, 2 H),7.90- 7.97 (m, 1 H),12.48 (br s, 1 H). |
| 4-98 | DMSO-d6 400MHz | 0.80 (t, J = 7.3 Hz, 6 H),0.98- 1.16 (m, 4 H),1.37- 1.58 (m, 4 H),2.41- 2.53 (m, 1 H),3.39 (s, 2 H),4.05 (s, 2 H),4.09 (s, 2 H),5.07 (s, 2 H),6.93 (d, J = 8.7 Hz, 2 H),7.02 (d, J = 3.7 Hz, 1 H),7.07 (d, J = 8.7 Hz, 2 H),7.25- 7.31 (m, 1 H),7.36-7.50 (m, 6 H),7.63- 7.70 (m, 4 H),7.78 (d, J = 1.4 Hz, 1 H),12.46 (br s, 1 H). |
| 4-99 | DMSO-d6, 400MHz (d, J = | 0.79 (t, J = 7.3 Hz, 6 H),1.00- 1.15 (m, 4 H),1.36- 1.57 (m, 4 H),2.40- 2.52 (m, 1 H),4.32 (s, 2 H),4.96 (s, 2 H),5.05 (s, 2H),6.91 (d, J = 8.6 Hz, 2 H),7.02- 7.13 (m, 3 H),7.21 (d, J = 3.7 Hz, 1H),7.28- 7.34 (m, 1 H),7.39- 7.54 (m, 4 H),7.63 8.3 Hz, 2 H),7.77- 7.82 (m, 1 H),12.95 (br s, 1 H). |
| 4-100 | DMSO-d6, 400MHz | 0.80 (t, J = 7.3 Hz, 6 H),0.99- 1.17 (m, 4 H),1.38- 1.58 (m, 4 H),2.40- 2.52 (m, 1 H),3.96 (s, 2 H),4.68 (s, 2 H),5.07 (s, 2 H),6.93 (d, J = 8.8 Hz, 2 H),7.03- 7.12 (m, 3 H),7.32 (d, J = 4.0 Hz, 1 H),7.37 (d, J = 3.7 Hz, 1 H),7.48 (d, J = 8.5 Hz, 2 H),7.62 (d, J = 8.5 Hz, 2 H),7.67 (d, J = 4.0 Hz, 1 H),12.97 (br s, 1 H). |
| 4-101 | DMSO-d6, 300MHz | 0.79 (t, J = 7.2 Hz, 6 H),0.96 (t, J = 7.0 Hz, 3 H),1.00-1.17 (m, 4 H),1.23 (t, J = 7.0 Hz, 3 H),1.35- 1.61 (m, 4 H),2.37- 2.55 (m, 1 H),3.16- 3.50 (m, 6 H),3.96 (s, 2 H),4.02 (s, 2 H),5.07 (s, 2 H),5.43 (s, 2 H),6.93 (d, J = 8.7 Hz, 2 H),7.00- 7.24 (m, 5 H),7.31- 7.41 (m, 2 H),7.48 (d, J = 8.6 Hz, 2 H),7.58- 7.68 (m, 3 H),12.08 (br s, 1 H). |

**Table 17-78**

| Example | solvent, Hz | NMR(δ) |
|---|---|---|
| 4-102 | DMSO-d6, 300MHz | 1.14-1.48(m, 5H), 1.64-1.85(m, 5H), 2.26(s, 3H), 2.27(s, 3H), 2.38-2.54(m, 1H), 2.87(dd, J=7.7, 14.1Hz, 1H), 3.02(dd, J=7.6, 13.9Hz, 1 H), 3.59(t, J=7.5Hz, 1 H), 3.81 (d, J=14.3Hz, 1 H), 3.96(d, J=14.3Hz, 1H), 5.07(s, 2H), 7.05(d, J=1.1Hz, 2H), 7.14-7.35(m, 11H), 7.57(d, J=1.5Hz, 1H), 12.48(brs, 1H) |
| 4-103 | DMSO-d6, 300MHz | 0.93(t, J=7.3Hz, 3H), 0.97(t, J=7.0Hz, 3H), 1.36-1.51(m, 2H), 1.65-1.77(m, 2H), 3.26(s, 2H), 3.57(q, J=7.1Hz, 2H), 3-81(s, 2H), 4.00-4.08(m, 4H), 7.07(t, J=8.5Hz, 4H), 7.23-7.44(m, 6H), 7.48(d, J=8.7Hz, 2H), 7.66(d, J=8.4Hz, 2H), 7.80(d, J=1.1 Hz, 1H) |
| 4-104 | CDCl3-300MHz | 1.17-1.51(m, 5H), 1.67-1.95(m, 5H), 2.30(s, 3H), 2.43-2.57(m, 1 H), 2.78(s, 3H), 4.99(s, 2H), 5.19(s, 2H), 6.88(d, J=8.3Hz, 1H), 7.00(dd, J=1.8, 8.4Hz, 1H), 7.14-7.36(m, 7H), 7.53(d, J=1.5Hz, 1H), 7.70(d, J=8.6Hz, 1H), 7.79(d, J=1.9Hz, 1H) |
| 4-105 | DMSO-d6, 400MHz | 3.27(s, 2H), 3.81(s, 2H), 4.03(s, 2H), 5.17(s, 2H), 7.13-7.18(m, 3H), 7.24-7.29(m, 1H), 7.32-7.40(m, 4H), 7.42-7.49(m, 3H), 7.71(d, J=8.3Hz, 2H), 7.79(d, J=1.6Hz, 1H), 12.40(brs, 1H) |
| 4-106 | DMSO-d6, 400MHz | 0.79 (t, J = 7.3 Hz, 6 H),0.99- 1.16 (m, 4 H),1.38- 1.58 (m, 4 H),2.40- 2.53 (m, 1 H),3.35 (s, 2 H),4.04 (s, 2 H),4.10 (s, 2 H),4.94- 5.17 (m, 6 H),5.92- 6.04 (m, 1 H),6.92 (d, J = 8.8 Hz, 2 H),7.02- 7.10 (m, 3 H),7.16- 7.27 (m, 2 H),7.37 (d, J = 3.5 Hz, 1 H),7.44- 7.53 (m, 3 H),7.59- 7.65 (m, 3 H),12.60 (br s, 1 H). |
| 4-107 | DMSO-d6, 300MHz | 0.79 (t, J = 7.2 Hz, 6 H),0.97- 1.16 (m, 4 H),1.36- 1.60 (m, 4 H),2.39- 2.56 (m, 1 H),4.26 (s, 2 H),4.90 (s, 2 H),5.05 (s, 2 H),6.91 (d, J = 8.7 Hz, 2 H),7.06 (d, J = 8.7 Hz, 2 H),7.19 (d, J = 3.6 Hz, 1 H),7.34 (s, 1 H),7.40 (d, J = 3.6 Hz, 1 H),7.42- 7.49 (m, 4 H),7.62 (d, J = 8.3 Hz, 2 H),7.86- 7.92 (m, 2 H),12.88 (br s, 1 H). |

### Industrial Applicability

As is clear from the foregoing test results and the like, compound [I] of the present invention has been shown to have a superior PTP1B inhibitory activity and a hypoglycemic activity. That is, the compound [I] of the present invention is expected to provide a new type of drug for the prophylaxis or treatment of diabetes, which directly improves the action of insulin and ameliorates hyperglicemic state. In addition, the compound [I] of the present invention is expected to provide a drug for the prophylaxis or treatment of diabetic complications (retinopathy, nephropathy, neuropathy, syndrome X or metabolic syndrome, ischemic heart diseases (cardiac infarction, angina pectoris etc.), strokes (cerebral infarction, cerebral hemorrhage, subarachnoid hemorrhage etc.) etc.), hyperlipidemia, obesity and the like, and further a drug for the prophylaxis or treatment of diseases mediated by PTP1B.

Moreover, the compound [I] of the present invention enhances hypoglycemic activity, as shown in the foregoing test results, by administration in combination with each of insulin, glibenclamide, tolbutamide, nateglinide, metformin hydrochloride, voglibose and Pioglitazone hydrochloride. Therefore, the compound [I] is expected to have an activity to mutually enhance and/or complement blood glucose (fasting, postprandial) lowering ability that the compound [I] of the present invention and therapeutic agents for diabetes individually have, by the use in combination with other therapeutic agents for diabetes (particularly the corresponding therapeutic agents other than therapeutic agents for diabetic complications) including insulin preparation, insulin secretagogue, biguanide, α-glucosidase inhibitor and insulin sensitivity enhancer. Furthermore, the compound [I] of the present invention is expected to simultaneously realize improvement of hyperglycemia and prophylaxis or treatment of diabetic complications (particularly, microangio complications) by the use in combination with other therapeutic agents for diabetes (particularly, therapeutic agents for diabetic complications). Moreover, the compound [I] of the present invention is expected to ameliorate hyperglycemia, high blood lipid and obesity, and further, prevent transition into diabetic complications (particularly, large artery complications) and/or suppress progression thereof, by a use in combination with other therapeutic agents for hyperlipidemia, therapeutic agents for obesity, therapeutic agents for hypertension and/or therapeutic agents for thrombosis. In other words, the compound [I] of the present invention is expected to exhibit a high prophylactic or therapeutic effect for diabetes and diabetic complications, by the use in combination with other therapeutic agents for diabetes, other therapeutic agents for hyperlipidemia, other therapeutic agents for obesity, therapeutic agents for hypertension or therapeutic agents for thrombosis, which effect is unavailable by independent use of each therapeutic agent.

This application is based on a patent application Nos. 2003-105267 and 2003-157590 filed in Japan, the contents of which are hereby incorporated by reference. The references cited herein, including patents and patent applications, are hereby incorporated in their entireties by reference, to the extent that they have been disclosed herein.

## Claims

1. A 5-membered heteroaromatic ring compound represented by the formula [I] 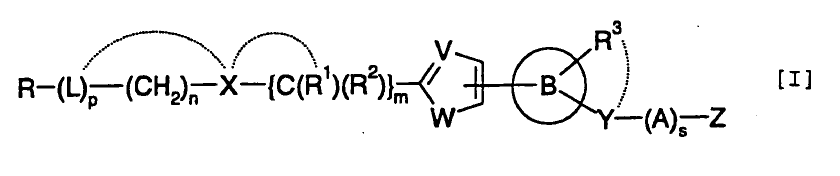 wherein
V is =N- or =CH-;
W is -S- or -O-;
m is 0, 1 or 2;
R¹ and R² are each independently a hydrogen atom or a C₁₋₄ alkyl group;
X is -N(R⁴)-, -N (R⁵) -CO-O-, -SO₂-N (R⁵)-, -N(R⁵)-SO₂-, -N (R⁶)-SO₂-N(R⁵)-, -CO-N (R⁷)-, -N (R⁸) -CO-, -N (R⁹) -CO-N (R⁵) -, -N (R¹⁰)-(CH₂)ₖ-N(R⁵)-, -N (R¹⁰)- (CH₂)ₖ-N (R⁵) -CO-, -C (R¹⁰) =N-N(R⁷) -, -N (R¹⁰)-(CH₂)ₖ-CH(R⁶)-, -O-, -S- or -SO₂-
wherein
k is 0 or an integer of 1 to 4,
R⁴, R⁵, R⁶, R⁷, R⁸, R⁹ and R¹⁰ are each independently
(1) a hydrogen atom,
(2) a C₁₋₆ alkyl group
wherein said C₁₋₆ alkyl group is optionally substituted by
(a) an optionally substituted aryl group,
(b) an optionally substituted heteroaromatic ring group,
(c) a carboxy group,
(d) a C₁₋₄ alkoxycarbonyl group,
(e) -CO-N(R¹⁵)(R¹⁶)
wherein R¹⁵ and R¹⁶ are each independently a hydrogen atom, an optionally substituted aryl group, an optionally substituted heteroaromatic ring group, a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group, or may form an indoline ring together with the nitrogen atom bonded thereto or may form a 5- to 7-membered hetero ring optionally containing at least one heteroatom selected from the group consisting of nitrogen atom, oxygen atom and sulfur atom),
(f) -N(R¹⁵)(R¹⁶)
wherein R¹⁵ and R¹⁶ are as defined above,
(g) -O-R¹⁷
wherein R¹⁷ is a hydrogen atom, an optionally substituted aryl group, an optionally substituted heteroaromatic ring group or a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group,
(h) -CO-R¹⁷
wherein R¹⁷ is as defined above,
(i) -SO₂-R¹⁷
wherein R¹⁷ is as defined above or
(j) a C₃₋₇ cycloalkyl group,
(3) -CO-N(R¹⁵) (R¹⁶)
wherein R¹⁵ and R¹⁶ are as defined above,
(4) -SO₂-N(R¹⁵) (R¹⁶)
wherein R¹⁵ and R¹⁶ are as defined above,
(5) -CO-R¹⁷
wherein R¹⁷ is as defined above,
(6) -SO₂-R¹⁷
wherein R¹⁷ is as defined above,
(7) an optionally substituted aryl group,
(8) an optionally substituted heteroaromatic ring group, or
(9) R⁴ and R¹ are optionally linked to form 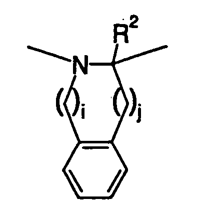 wherein i and j are each independently 0, 1 or 2,
(10) R⁵ and R⁹ are optionally linked to form 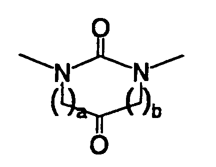 wherein a and b are each independently 0, 1 or 2,
(11) R⁵ and R¹⁰ are optionally linked to form 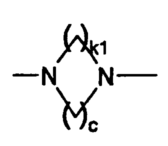 wherein k1 and c are each independently 0 or an integer of 1 to 4,
(12) R⁵ and R¹⁰ are optionally linked to form 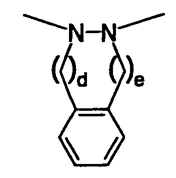 wherein d and e are each independently 0, 1 or 2,
(13) R⁶ and R¹⁰ are optionally linked to form 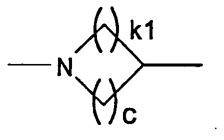 wherein k1 and c are as defined above, or
(14) R⁷ and R¹⁰ are optionally linked to form 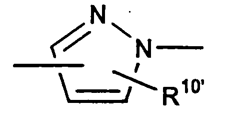 wherein R^{10'} is a hydrogen atom or a C₁₋₆ alkyl group;
n is 0 or an integer of 1 to 4;
p is 0 or 1;
L is
(1) -C(R²⁰)(R²¹)-
wherein
R²⁰ is
(a) a hydrogen atom,
(b) a C₁₋₆ alkyl group, or
(c) optionally linked with R⁴ or R⁸ to form 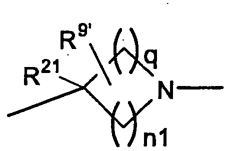 wherein n1 and q are each independently 0 or an integer of 1 to 4, R^{9'} is a hydrogen atom, a hydroxyl group, a C₁₋₆ alkyl group, a carboxy group or a C₁₋₆ alkoxy group, or
(d) optionally linked with R⁴ to form 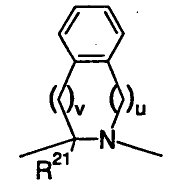
wherein u and v are each independently 0, 1 or 2,
R²¹ is a hydrogen atom, a C₁₋₆ alkyl group wherein said C₁₋₆ alkyl group is optionally substituted by an optionally substituted aryl group or an optionally substituted heteroaromatic ring group, an optionally substituted aryl group or an optionally substituted heteroaromatic ring group,
(2) 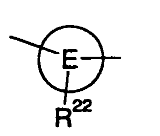 wherein
E is an aryl group or a heteroaromatic ring group,
R²² is
(a) a hydrogen atom,
(b) a halogen atom,
(c) a C₁₋₄ alkyl group,
(d) a C₁₋₄ alkoxy group optionally substituted by a carboxy group,
(e) a C₁₋₆ alkylthio group,
(f) a nitro group,
(g) -N(R²³)(R²⁴)
wherein R²³ and R²⁴ are each independently a hydrogen atom, a C₁₋₆ alkyl group, a C₁₋₄ alkylcarbonyl group wherein said C₁₋₄ alkylcarbonyl group is optionally substituted by an amino group, a C₁₋₄ alkylamino group or a di(C₁₋₄ alkyl) amino group, or a C₁₋₄ alkylsulfonyl group, or
(h) optionally linked with R⁴ to form 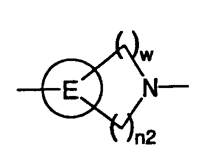 wherein n2 and w are each independently 0 or an integer of 1 to 3,
(i) optionally linked with R⁴ to form 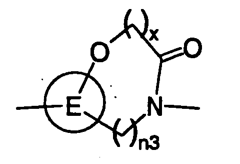 wherein n3 and x are each independently 0 or 1,
(j) optionally linked with R⁷ to form 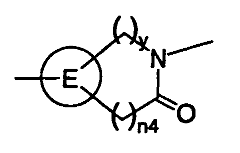 wherein n4 and y are each independently 0, 1 or 2,
(k) optionally linked with R⁷ to form 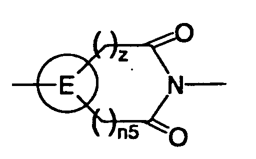 wherein n5 and z are each independently 0 or 1,
(l) optionally linked with R⁸ to form 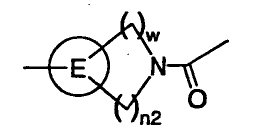 wherein n2 and w are each independently 0 or an integer of 1 to 3, or
(m) optionally linked with R⁴ to form 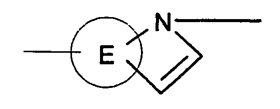 or 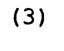 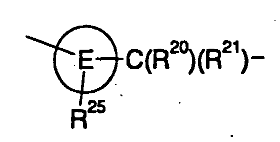 wherein
R²⁰, R²¹ and E are as defined above,
R²⁵ is
(a) a hydrogen atom,
(b) a halogen atom,
(c) a C₁₋₄ alkyl group,
(d) a C₁₋₄ alkoxy group optionally substituted by a carboxy group,
(e) a C₁₋₆ alkylthio group,
(f) a nitro group or
(g) -N(R²³)(R²⁴)
wherein R²³ and R²⁴ are as defined above;
R is -COO (R¹⁹), -A¹-COO(R¹⁹) or -O-A¹-COO(R¹⁹)
wherein A¹ is a C₁₋₄ alkylene group and R¹⁹ is a hydrogen atom or a C₁₋₄ alkyl group;
B is an aryl group or a heteroaromatic ring group;
R³ is
(1) a hydrogen atom,
(2) a halogen atom,
(3) a C₁₋₈ alkyl group,
(4) a C₁₋₆ alkoxy group,
(5) a C₁₋₆ alkylamino group,
(6) a di(C₁₋₆ alkyl)amino group,
(7) a cyano group,
(8) a nitro group,
(9) a C₁₋₄ haloalkyl group, (10) -S-R¹⁸ wherein R¹⁸ is a C₁₋₆ alkyl group or an aryl group, (11) -SO-R¹⁸ wherein R¹⁸ is a C₁₋₆ alkyl group or an aryl group, (12) -SO₂-R¹⁸ wherein R¹⁸ is a C₁₋₆ alkyl group or an aryl group,
(13) an aryl group or
(14) a heterocyclic group;
Y is -O-, -S-, -SO-, -SO₂-, -N(R¹¹)-, -N(R¹²)-CO-, -N(R¹²)-SO₂-, -SO₂-N(R¹²)-, -C(R¹³)(R¹⁴)-, -CO-, -C(R¹³)(R¹⁴)-N(R¹²)-, -CO-N(R¹²)- or -C(R¹³)(R¹⁴)-O-
wherein
R¹¹ is
(1) a hydrogen atom,
(2) a C₁₋₈ alkyl group
wherein said C₁₋₈ alkyl group is optionally substituted by substituent(s) selected from the group consisting of
(a) a C₃₋₇ cycloalkyl group,
(b) an optionally substituted aryl group,
(c) an optionally substituted heterocyclic group,
(d) a hydroxyl group,
(e) a C₁₋₄ alkylamino group and
(f) a di(C₁₋₄ alkyl) amino group,
(3) a C₂₋₄ alkenyl group,
(4) a C₁₋₄ alkylsulfonyl group,
(5) a C₁₋₄ alkylcarbonyl group
wherein said C₁₋₄ alkylcarbonyl group is optionally substituted by a hydroxyl group or a C₁₋₄ alkoxy group, or
(6) optionally linked with R³ to form 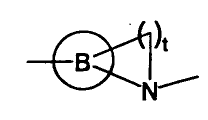
wherein t is an integer of 1 to 4,
R¹² is
(1) a hydrogen atom,
(2) a C₁₋₈ alkyl group
wherein said C₁₋₈ alkyl group is optionally substituted by substituent(s) selected from the group consisting of
(a) a C₃₋₇ cycloalkyl group,
(b) an optionally substituted aryl group,
(c) an optionally substituted heterocyclic group,
(d) a hydroxyl group,
(e) a C₁₋₄ alkylamino group and
(f) a di(C₁₋₄ alkyl)amino group,
(3) a C₂₋₄ alkenyl group,
(4) a C₁₋₄ alkylsulfonyl group or
(5) a C₁₋₄ alkylcarbonyl group
wherein said C₁₋₄ alkylcarbonyl group is optionally substituted by a hydroxyl group or a C₁₋₄ alkoxy group,
R¹³ and R¹⁴ are each independently a hydrogen atom, a C₁₋₄ alkyl group, or optionally form a C₃₋₇ cycloalkane together with the carbon atom bonded thereto, or optionally form, together with the carbon atom bonded thereto, a 5- to 7-membered hetero ring optionally having at least one heteroatom selected from the group consisting of nitrogen atom, oxygen atom and sulfur atom, provided that,
when m is 0, p is 1 and L is 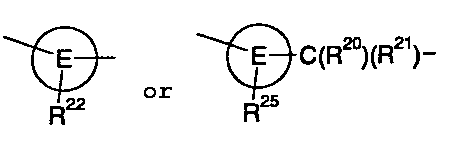 wherein R²⁰ and R²¹ are each a hydrogen atom, E is a phenyl group, R²² is a hydrogen atom, a halogen atom, a C₁₋₄ alkyl group, a C₁₋₄ alkoxy group or a nitro group, R²⁵ is a hydrogen atom, a halogen atom, a C₁₋₄ alkyl group, a C₁₋₄ alkoxy group or a nitro group,
Y should be -C(R¹³)(R¹⁴)-N(R¹²)-, -CO-N(R¹²)- or -C(R¹³)(R¹⁴)-O- wherein R¹², R¹³ and R¹⁴ are as defined above;
s is 0 or 1;
A is a C₁₋₄ alkylene group optionally substituted by a C₃₋₇ cycloalkyl group;
Z is
(1) a C₃₋₇ cycloalkyl group
wherein said C₃₋₇ cycloalkyl group is optionally substituted by an aryl group wherein said aryl group is optionally substituted by a halogen atom or a C₁₋₆ alkyl group, or a heteroaromatic ring group wherein said heteroaromatic ring group is optionally substituted by a halogen atom or a C₁₋₆ alkyl group,
(2) an aryl group
wherein said aryl group is optionally substituted by substituent(s) selected from the group consisting of
(a) a heterocyclic group optionally substituted by a C₁₋₄ alkyl group or a C₁₋₄ alkylcarbonyl group,
(b) a C₃₋₇ cycloalkyl group optionally substituted by a hydroxyl group, an oxo group, a halogen atom or a C₁₋₆ alkyl group,
(c) a carboxy group,
(d) a halogen atom,
(e) a C₁₋₈ alkyl group,
(f) a C₁₋₄ haloalkyl group,
(g) a C₁₋₄ alkylamino group,
(h) a di(C₁₋₄ alkyl) amino group,
(i) a C₁₋₆ alkylthio group,
(j) a C₁₋₄ alkoxy group,
(k) a C₁₋₄ alkylcarbonyl group and
(l) a nitro group,
(3) a heteroaromatic ring group
wherein said heteroaromatic ring group is optionally substituted by substituent(s) selected from the group consisting of
(a) a heterocyclic group optionally substituted by a C₁₋₄ alkyl group or a C₁₋₄ alkylcarbonyl group,
(b) a C₃₋₇ cycloalkyl group optionally substituted by a hydroxyl group, an oxo group, a halogen atom or a C₁₋₆ alkyl group,
(c) a carboxy group,
(d) a halogen atom,
(e) a C₁₋₈ alkyl group,
(f) a C₁₋₄ haloalkyl group,
(g) a C₁₋₄ alkylamino group,
(h) a di(C₁₋₄ alkyl) amino group,
(i) a C₁₋₆ alkylthio group,
(j) a C₁₋₄ alkoxy group,
(k) a C₁₋₄ alkylcarbonyl group and
(l) an aryl group optionally substituted by a halogen atom or a C₁₋₄ haloalkyl group,
(4) an indanyl group or
(5) a piperazinyl group
wherein said piperazinyl group is optionally substituted by substituent(s) selected from the group consisting of
(a) a phenyl group,
(b) a phenyl C₁₋₄ alkyl group,
(c) a benzoyl group optionally substituted by a halogen atom and
(d) a phenyl C₁₋₄ alkoxycarbonyl group or a prodrug thereof, or a pharmaceutically acceptable salt thereof.

2. The 5-membered heteroaromatic ring compound of claim 1, which is represented by the formula [I] 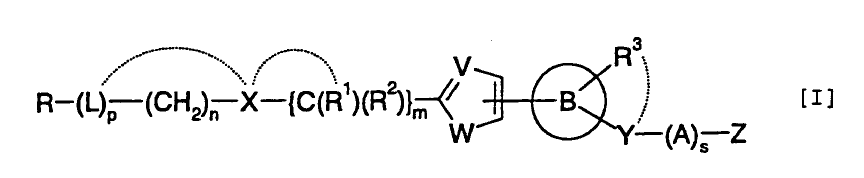 wherein
V is =N- or =CH-;
W is -S- or -O-;
m is 0, 1 or 2;
R¹ and R² are each independently a hydrogen atom or a C₁₋₄ alkyl group;
X is -N(R⁴)-, -N(R⁵)-CO-O-, -SO₂-N(R⁵)-, -N (R⁵)-SO₂-, -N (R⁶)-SO₂-N(R⁵)-, -CO-N (R⁷) -, -N (R⁸)-CO-, -N (R⁹)-CO-N (R⁵)-, -N (R¹⁰)-(CH₂)ₖ-N(R⁵)-, -O-, -S- or -SO₂-
wherein
k is 0 or an integer of 1 to 4,
R⁴, R⁵, R⁶, R⁷, R⁸, R⁹ and R¹⁰ are each independently
(1) a hydrogen atom,
(2) a C₁₋₆ alkyl group
wherein said C₁₋₆ alkyl group is optionally substituted by
(a) an optionally substituted aryl group,
(b) an optionally substituted heteroaromatic ring group,
(c) a carboxy group,
(d) a C₁₋₄ alkoxycarbonyl group,
(e) -CO-N(R¹⁵)(R¹⁶)
wherein R¹⁵ and R¹⁶ are each independently a hydrogen atom, an optionally substituted aryl group, an optionally substituted heteroaromatic ring group, a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group, or may form an indoline ring together with the nitrogen atom bonded thereto or may form a 5- to 7-membered hetero ring optionally containing at least one heteroatom selected from the group consisting of nitrogen atom, oxygen atom and sulfur atom),
(f) -N(R¹⁵)(R¹⁶)
wherein R¹⁵ and R¹⁶ are as defined above,
(g) -O-R¹⁷
wherein R¹⁷ is a hydrogen atom, an optionally substituted aryl group, an optionally substituted heteroaromatic ring group or a C₁₋₆ alkyl group wherein said C₁₋₆ alkyl group is optionally substituted by substituent(s) selected from the group consisting of an optionally substituted aryl group, an optionally substituted heteroaromatic ring group, a C₁₋₄ alkoxy group optionally substituted by an aryl group and an optionally substituted aryloxy group,
(h) -CO-R¹⁷
wherein R¹⁷ is as defined above,
(i) -SO₂-R¹⁷
wherein R¹⁷ is as defined above or
(j) a C₃₋₇ cycloalkyl group,
(3) -CO-N(R¹⁵)(R¹⁶)
wherein R¹⁵ and R¹⁶ are as defined above,
(4) -SO₂-N(R¹⁵)(R¹⁶)
wherein R¹⁵ and R¹⁶ are as defined above,
(5) -CO-R¹⁷
wherein R¹⁷ is as defined above,
(6) -SO₂-R¹⁷
wherein R¹⁷ is as defined above,
(7) an optionally substituted aryl group,
(8) an optionally substituted heteroaromatic ring group, or
(9) R⁴ and R¹ are optionally linked to form 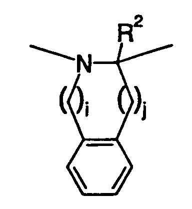 wherein i and j are each independently 0, 1 or 2,
(10) R⁵ and R⁹ are optionally linked to form 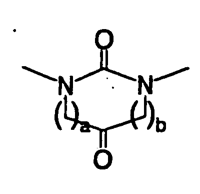 wherein a and b are each independently 0, 1 or 2,
(11) R⁵ and R¹⁰ are optionally linked to form 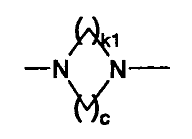 wherein k1 and c are each independently 0 or an integer of 1 to 4, or
(12) R⁵ and R¹⁰ are optionally linked to form 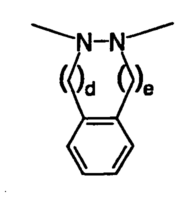
wherein d and e are each independently 0, 1 or 2;
n is 0 or an integer of 1 to 4;
p is 0 or 1;
L is
(1) -C(R²⁰)(R²¹)-
wherein
R²⁰ is
(a) a hydrogen atom,
(b) a C₁₋₆ alkyl group, or
(c) optionally linked with R⁴ to form 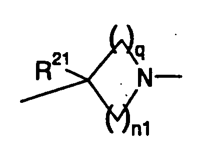 wherein n1 and q are each independently 0 or an integer of 1 to 4, or
(d) optionally linked with R⁴ to form 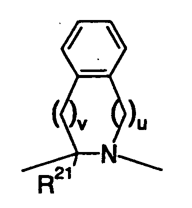
wherein u and v are each independently 0, 1 or 2,
R²¹ is a hydrogen atom, a C₁₋₆ alkyl group wherein said C₁₋₆ alkyl group is optionally substituted by an optionally substituted aryl group or an optionally substituted heteroaromatic ring group, an optionally substituted aryl group or an optionally substituted heteroaromatic ring group, 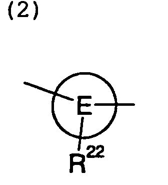 wherein
E is an aryl group or a heteroaromatic ring group,
R²² is
(a) a hydrogen atom,
(b) a halogen atom,
(c) a C₁₋₄ alkyl group,
(d) a C₁₋₄ alkoxy group optionally substituted by a carboxy group,
(e) a C₁₋₆ alkylthio group,
(f) a nitro group,
(g) -N(R²³)(R²⁴)
wherein R²³ and R²⁴ are each independently a hydrogen atom, a C₁₋₆ alkyl group, a C₁₋₄ alkylcarbonyl group wherein said C₁₋₄ alkylcarbonyl group is optionally substituted by an amino group, a C₁₋₄ alkylamino group or a di(C₁₋₄ alkyl) amino group, or a C₁₋₄ alkylsulfonyl group, or
(h) optionally linked with R⁴ to form 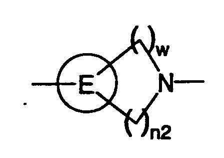 wherein n2 and w are each independently 0 or an integer of 1 to 3,
(i) optionally linked with R⁴ to form 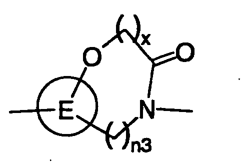 wherein n3 and x are each independently 0 or 1,
(j) optionally linked with R⁷ to form 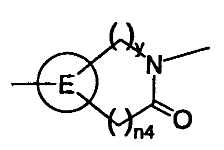 wherein n4 and y are each independently 0, 1 or 2,
(k) optionally linked with R⁷ to form 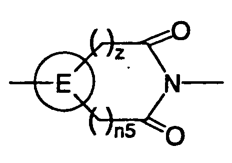 wherein n5 and z are each independently 0 or 1, or
(l) optionally linked with R⁸ to form 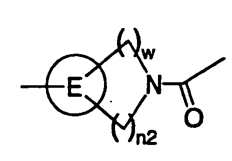
wherein n2 and w are each independently 0 or an integer of 1 to 3, or 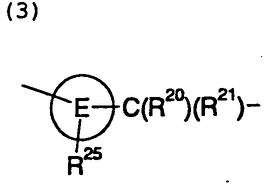 wherein
R²⁰, R²¹ and E are as defined above,
R²⁵ is
(a) a hydrogen atom,
(b) a halogen atom,
(c) a C₁₋₄ alkyl group,
(d) a C₁₋₄ alkoxy group optionally substituted by a carboxy group,
(e) a C₁₋₆ alkylthio group,
(f) a nitro group or
(g) -N(R²³)(R²⁴)
wherein R²³ and R²⁴ are as defined above;
R is -COO(R¹⁹), -A¹-COO(R¹⁹) or -O-A¹-COO(R¹⁹)
wherein A¹ is a C₁₋₄ alkylene group and R¹⁹ is a hydrogen atom or a C₁₋₄ alkyl group;
B is an aryl group or a heteroaromatic ring group;
R³ is
(1) a hydrogen atom,
(2) a halogen atom,
(3) a C₁₋₈ alkyl group,
(4) a C₁₋₆ alkoxy group,
(5) a C₁₋₆ alkylamino group,
(6) a di(C₁₋₆ alkyl)amino group,
(7) a cyano group,
(8) a nitro group,
(9) a C₁₋₄ haloalkyl group,
(10) -S-R¹⁸ wherein R¹⁸ is a C₁₋₆ alkyl group or an aryl group,
(11) -SO-R¹⁸ wherein R¹⁸ is a C₁₋₆ alkyl group or an aryl group, or
(12) -SO₂-R¹⁸ wherein R¹⁸ is a C₁₋₆ alkyl group or an aryl group; Y is -O-, -S-, -SO-, -SO₂-, -N(R¹¹)-, -N(R¹²)-CO-, -N(R¹²)-SO₂-, -SO₂-N(R¹²)-, -C(R¹³)(R¹⁴)-, -CO-, -C(R¹³)(R¹⁴)-N(R¹²)-, -CO-N(R¹²)-or -C(R¹³)(R¹⁴)-O-
wherein
R¹¹ is
(1) a hydrogen atom,
(2) a C₁₋₈ alkyl group
wherein said C₁₋₈ alkyl group is optionally substituted by substituent(s) selected from the group consisting of
(a) a C₃₋₇ cycloalkyl group,
(b) an optionally substituted aryl group,
(c) an optionally substituted heterocyclic group,
(d) a hydroxyl group,
(e) a C₁₋₄ alkylamino group and
(f) a di(C₁₋₄ alkyl)amino group,
(3) a C₂₋₄ alkenyl group,
(4) a C₁₋₄ alkylsulfonyl group,
(5) a C₁₋₄ alkylcarbonyl group
wherein said C₁₋₄ alkylcarbonyl group is optionally substituted by a hydroxyl group or a C₁₋₄ alkoxy group, or
(6) optionally linked with R³ to form 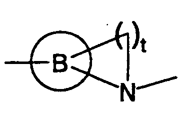
wherein t is an integer of 1 to 4,
R¹² is
(1) a hydrogen atom,
(2) a C₁₋₈ alkyl group
wherein said C₁₋₈ alkyl group is optionally substituted by substituent(s) selected from the group consisting of
(a) a C₃₋₇ cycloalkyl group,
(b) an optionally substituted aryl group,
(c) an optionally substituted heterocyclic group,
(d) a hydroxyl group,
(e) a C₁₋₄ alkylamino group and
(f) a di(C₁₋₄ alkyl) amino group,
(3) a C₂₋₄ alkenyl group,
(4) a C₁₋₄ alkylsulfonyl group or
(5) a C₁₋₄ alkylcarbonyl group
wherein said C₁₋₄ alkylcarbonyl group is optionally substituted by a hydroxyl group or a C₁₋₄ alkoxy group,
R¹³ and R¹⁴ are each independently a hydrogen atom, a C₁₋₄ alkyl group, or optionally form a C₃₋₇ cycloalkane together with the carbon atom bonded thereto, or optionally form, together with the carbon atom bonded thereto, a 5- to 7-membered hetero ring optionally having at least one heteroatom selected from the group consisting of nitrogen atom, oxygen atom and sulfur atom, provided that,
when m is 0, p is 1 and L is 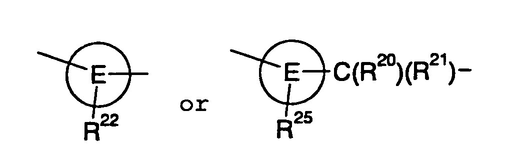 wherein R²⁰ and R²¹ are each a hydrogen atom, E is a phenyl group, R²² is a hydrogen atom, a halogen atom, a C₁₋₄ alkyl group, a C₁₋₄ alkoxy group or a nitro group, R²⁵ is a hydrogen atom, a halogen atom, a C₁₋₄ alkyl group, a C₁₋₄ alkoxy group or a nitro group,
Y should be
-C(R¹³)(R¹⁴)-N(R¹²)-, -CO-N(R¹²)- or -C(R¹³)(R¹⁴)-O- wherein R¹², R¹³ and R¹⁴ are as defined above;
s is 0 or 1;
A is a C₁₋₄ alkylene group optionally substituted by a C₃₋₇ cycloalkyl group;
Z is
(1) a C₃₋₇ cycloalkyl group
wherein said C₃₋₇ cycloalkyl group is optionally substituted by an aryl group wherein said aryl group is optionally substituted by a halogen atom or a C₁₋₆ alkyl group, or a heteroaromatic ring group wherein said heteroaromatic ring group is optionally substituted by a halogen atom or a C₁₋₆ alkyl group,
(2) an aryl group
wherein said aryl group is optionally substituted by substituent(s) selected from the group consisting of
(a) a heterocyclic group optionally substituted by a C₁₋₄ alkyl group or a C₁₋₄ alkylcarbonyl group,
(b) a C₃₋₇ cycloalkyl group optionally substituted by a hydroxyl group, an oxo group, a halogen atom or a C₁₋₆ alkyl group,
(c) a carboxy group,
(d) a halogen atom,
(e) a C₁₋₈ alkyl group,
(f) a C₁₋₄ haloalkyl group,
(g) a C₁₋₄ alkylamino group,
(h) a di(C₁₋₄ alkyl)amino group,
(i) a C₁₋₆ alkylthio group,
(j) a C₁₋₄ alkoxy group, and
(k) a C₁₋₄ alkylcarbonyl group,
(3) a heteroaromatic ring group
wherein said heteroaromatic ring group is optionally substituted by substituent(s) selected from the group consisting of
(a) a heterocyclic group optionally substituted by a C₁₋₄ alkyl group or a C₁₋₄ alkylcarbonyl group,
(b) a C₃₋₇ cycloalkyl group optionally substituted by a hydroxyl group, an oxo group, a halogen atom or a C₁₋₆ alkyl group,
(c) a carboxy group,
(d) a halogen atom,
(e) a C₁₋₈ alkyl group,
(f) a C₁₋₄ haloalkyl group,
(g) a C₁₋₄ alkylamino group,
(h) a di(C₁₋₄ alkyl)amino group,
(i) a C₁₋₆ alkylthio group,
(j) a C₁₋₄ alkoxy group,
(k) a C₁₋₄ alkylcarbonyl group and
(l) an aryl group optionally substituted by a halogen atom or a C₁₋₄ haloalkyl group,
(4) an indanyl group or
(5) a piperazinyl group
wherein said piperazinyl group is optionally substituted by substituent(s) selected from the group consisting of
(a) a phenyl group,
(b) a phenyl C₁₋₄ alkyl group,
(c) a benzoyl group optionally substituted by a halogen atom and
(d) a phenyl C₁₋₄ alkoxycarbonyl group or a prodrug thereof, or a pharmaceutically acceptable salt thereof.

3. The 5-membered heteroaromatic ring compound of claim 1,
wherein, in the formula [I],
R³ is
(1) a hydrogen atom,
(2) a halogen atom,
(3) a C₁₋₆ alkyl group or
(4) a C₁₋₄ alkoxy group;
Y is -O-, -N(R¹¹)-, -N(R¹²)-CO-, -C(R¹³)(R¹⁴)-, -C(R¹³)(R¹⁴)-N(R¹²)-, -CO-N(R¹²)- or -C(R¹³)(R¹⁴)-O-
wherein
R¹¹ is
(1) a hydrogen atom,
(2) a C₁₋₈ alkyl group, or
(3) optionally linked with R³ to form 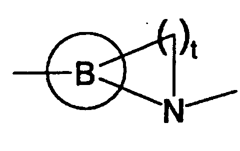
wherein t is 3,
R¹² is a hydrogen atom or a C₁₋₈ alkyl group,
R¹³ and R¹⁴ are each a hydrogen atom
provided that
when m is 0, p is 1, and L is 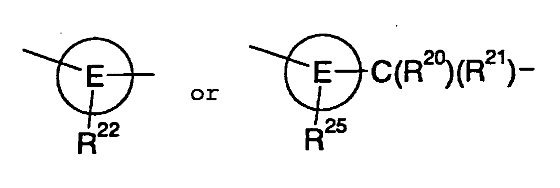 wherein R²⁰ and R²¹ are each a hydrogen atom, E is a phenyl group, R²² is a hydrogen atom, a C₁₋₄ alkoxy group or a nitro group, R²⁵ is a hydrogen atom, a C₁₋₄ alkoxy group or a nitro group,
Y should be
-C(R¹³)(R¹⁴)-N(R¹²)-, -CO-N(R¹²)- or -C(R¹³)(R¹⁴)-O- wherein R¹², R¹³ and R¹⁴ are as defined above;
s is 0 or 1;
A is a C₁₋₄ alkylene group;
Z is an aryl group substituted by substituent(s) selected from the group consisting of
(a) a C₃₋₇ cycloalkyl group optionally substituted by 1 to 3 substituent(s) selected from the group consisting of a halogen atom and a C₁₋₆ alkyl group,
(b) a halogen atom,
(c) C₁₋₈ alkyl group,
(d) a C₁₋₄ haloalkyl group,
(e) a di(C₁₋₄ alkyl) amino group,
(f) a C₁₋₆ alkylthio group and
(g) a C₁₋₄ alkylcarbonyl group, a prodrug thereof, or a pharmaceutically acceptable salt thereof.

4. The 5-membered heteroaromatic ring compound of claim 3, wherein V is =N- and W is -S- or -O-, or V is =CH- and W is -S-, or a prodrug thereof, or a pharmaceutically acceptable salt thereof.

5. The 5-membered heteroaromatic ring compound of claim 4, wherein Z is a phenyl group substituted by a C₁₋₈ alkyl group, or a prodrug thereof, or a pharmaceutically acceptable salt thereof.

6. The 5-membered heteroaromatic ring compound of claim 5, wherein Y is -C (R¹³)(R¹⁴)-N(R¹²)- or -C(R¹³)(R¹⁴)-O- wherein R¹², R¹³ and R¹⁴ are as defined in claim 3 and s is 0, or Y is -O- or -N(R¹¹)- wherein R¹¹ is as defined in claim 3, s is 1, and A is a methylene group, or a prodrug thereof, or a pharmaceutically acceptable salt thereof.

7. The 5-membered heteroaromatic ring compound of claim 6, wherein V is =CH-, W is -S-, and the position of substitution of B on the thiophene ring formed by V together with W is the 4-position or 5-position, or a prodrug thereof, or a pharmaceutically acceptable salt thereof.

8. The 5-membered heteroaromatic ring compound of claim 7, wherein B is a phenyl group, or a prodrug thereof, or a pharmaceutically acceptable salt thereof.

9. The 5-membered heteroaromatic ring compound of claim 8, wherein
m is 1;
R¹ and R² are each a hydrogen atom;
X is -N(R⁴)- or -O-
wherein R⁴ is a C₁₋₆ alkyl group optionally substituted by
(a) an aryl group,
(b) an heteroaromatic ring group optionally substituted by 1 to 3 C₁₋₈ alkyl groups or
(c) -CO-N(R¹⁵)(R¹⁶)
wherein R¹⁵ and R¹⁶ are each independently a hydrogen atom or an aryl group wherein said aryl group is optionally substituted by 1 to 3 C₁₋₈ alkyl groups;
n is 0 or 1;
p is 0 or 1;
L is
(1) -C(R²⁰)(R²¹)-
wherein R²⁰ is linked with R⁴ to form 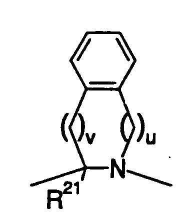 wherein u is 1 and v is 1,
R²¹ is a hydrogen atom, or 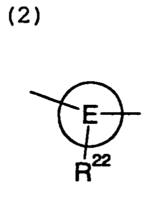
wherein E is an heteroaromatic ring group and R²² is a hydrogen atom;
R is -COO(R¹⁹)
wherein R¹⁹ is a hydrogen atom, or a prodrug thereof, or a pharmaceutically acceptable salt thereof.

10. The 5-membered heteroaromatic ring compound of claim 9, wherein X is -N(R⁴) wherein R⁴ is as defined in claim 9, n is 1 and p is 0, or a prodrug thereof, or a pharmaceutically acceptable salt thereof.

11. The 5-membered heteroaromatic ring compound of claim 10, wherein R⁴ is a methyl group substituted by a heteroaromatic ring group optionally substituted by one C₁₋₈ alkyl group, or a prodrug thereof, or a pharmaceutically acceptable salt thereof.

12. The 5-membered heteroaromatic ring compound of claim 11, wherein the heteroaromatic ring defined in claim 11 is a thiazolyl group, an oxazolyl group or a benzimidazolyl group, or a prodrug thereof, or a pharmaceutically acceptable salt thereof.

13. The 5-membered heteroaromatic ring compound of claim 10, wherein R⁴ is a methyl group substituted by -CO-N(R¹⁵)(R¹⁶) wherein R¹⁵ is a hydrogen atom and R¹⁶ is an aryl group optionally substituted by one C₁₋₈ alkyl group, or a prodrug thereof, or a pharmaceutically acceptable salt thereof.

14. The 5-membered heteroaromatic ring compound of claim 6, wherein V is =N-, W is -S-, and the position of substitution of B on the thiazole ring formed by V together with W is the 4-position, or a prodrug thereof, or a pharmaceutically acceptable salt thereof.

15. The 5-membered heteroaromatic ring compound of claim 14, wherein B is a phenyl group, or a prodrug thereof, or a pharmaceutically acceptable salt thereof.

16. The 5-membered heteroaromatic ring compound of claim 15, wherein
m is 0 or 1;
R¹ and R² are each independently a hydrogen atom or a C₁₋₄ alkyl group;
X is -N(R⁴)-, -N(R⁵)-CO-O-, -SO₂-N(R⁵)-, -CO-N(R⁷)-, -N(R⁹)-CON(R⁵)-, -N(R¹⁰)-(CH₂)ₖ-N(R⁵) -, -O-, -S- or -SO₂-
wherein
R⁴ is
(1) a hydrogen atom,
(2) a C₁₋₆ alkyl group
wherein said C₁₋₆ alkyl group is optionally substituted by
(a) an aryl group optionally substituted by 1 to 3 substituent(s) selected from the group consisting of a halogen atom, a C₁₋₈ alkyl group and a C₁₋₄ haloalkyl group,
(b) a heteroaromatic ring group optionally substituted by 1 to 3 C₁₋₈ alkyl group,
(c) a carboxy group,
(d) a C₁₋₄ alkoxycarbonyl group,
(e) -CO-N(R¹⁵)(R¹⁶)
wherein R¹⁵ and R¹⁶ are each independently a hydrogen atom, an aryl group wherein said aryl group is optionally substituted by 1 to 3 substituent(s) selected from the group consisting of a C₁₋₈ alkyl group, a C₁₋₄ alkoxy group, a carboxy group and a di(C₁₋₄ alkyl) amino group, a heteroaromatic ring group, a C₁₋₆ alkyl group wherein said C₁₋₆ alkyl group is optionally substituted by an aryl group, or may form a 5- to 7-membered hetero ring optionally containing at least one heteroatom selected from the group consisting of nitrogen atom, oxygen atom and sulfur atom, together with the nitrogen atom bonded thereto,
(f) -N(R¹⁵)(R¹⁶)
wherein R¹⁵ and R¹⁶ are each independently a hydrogen atom or an aryl group,
(g) -O-R¹⁷
wherein R¹⁷ is an aryl group, or
(h) a C₃₋₇ cycloalkyl group,
(3) -CO-N(R¹⁵)(R¹⁶)
wherein R¹⁵ and R¹⁶ are each independently a hydrogen atom, an aryl group wherein said aryl group is optionally substituted by 1 to 3 C₁₋₈ alkyl groups, a C₁₋₆ alkyl group, or may form an indoline ring together with the nitrogen atom bonded thereto, or may form a 5- to 7-membered hetero ring optionally containing at least one heteroatom selected from the group consisting of nitrogen atom, oxygen atom and sulfur atom,
(4) -SO₂-N(R¹⁵)(R¹⁶)
wherein R¹⁵ and R¹⁶ are each independently an aryl group or a C₁₋₆ alkyl group,
(5) -CO-R¹⁷
wherein R¹⁷ is an aryl group wherein said aryl group is optionally substituted by 1 to 3 substituent(s) selected from the group consisting of a C₁₋₈ alkyl group and a C₁₋₄ alkoxy group, a heteroaromatic ring group or a C₁₋₆ alkyl group wherein said C₁₋₆ alkyl group is optionally substituted by an aryl group optionally substituted by 1 to 3 substituent(s) selected from the group consisting of a halogen atom and a C₁₋₈ alkyl group, a heteroaromatic ring group, a C₁₋₄ alkoxy group optionally substituted by an aryl group or an aryloxy group optionally substituted by 1 to 3 C₁₋₈ alkyl groups,
(6) -SO₂-R¹⁷
wherein R¹⁷ is an aryl group,
(7) an aryl group, or
(8) optionally linked with R¹ to form 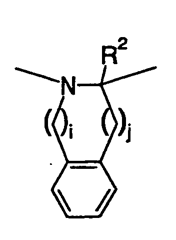
wherein i and j are each 1,
R⁵ is a hydrogen atom or an aryl group or a C₁₋₆ alkyl group optionally substituted by a C₃₋₇ cycloalkyl group,
R⁷ is a hydrogen atom or a C₁₋₆ alkyl group,
R⁹ is a hydrogen atom or a C₁₋₆ alkyl group,
k is 2,
R¹⁰ is
(1) a hydrogen atom,
(2) a C₁₋₆ alkyl group, or
(3) optionally linked with R⁵ to form 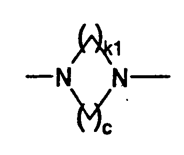
wherein k1 and c are each 2;
n is 0 or an integer of 1 to 3;
p is 0 or 1;
L is
(1) -C(R²⁰)(R²¹)-
wherein
R²⁰ is
(a) a hydrogen atom,
(b) a C₁₋₆ alkyl group, or
(c) optionally linked with R⁴ to form 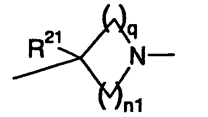 wherein n1 and q are each independently an integer of 1 to 3, or
(d) optionally linked with R⁴ to form 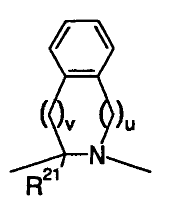
wherein u is 1 and v is 1,
R²¹ is a hydrogen atom, a C₁₋₆ alkyl group wherein said C₁₋₆ alkyl group is optionally substituted by an aryl group optionally substituted by 1 to 3 halogen atoms, or an aryl group or 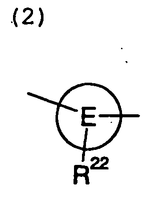 wherein
E is an aryl group or a heteroaromatic ring group,
R²² is
(a) a hydrogen atom,
(b) a C₁₋₄ alkoxy group optionally substituted by a carboxy group,
(c) a nitro group,
(d) -N(R²³)(R²⁴)
wherein R²³ and R²⁴ are each independently a hydrogen atom, a C₁₋₄ alkylcarbonyl group wherein said C₁₋₄ alkylcarbonyl group is optionally substituted by a C₁₋₄ alkylamino group or a di(C₁₋₄ alkyl)amino group or a C₁₋₄ alkylsulfonyl group, or
(e) optionally linked with R⁴ to form 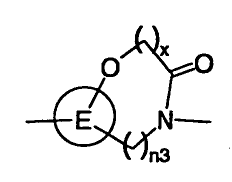
wherein n3 is 0 and x is 1;
R is -COO(R¹⁹)
wherein R¹⁹ is a hydrogen atom or a C₁₋₄ alkyl group, or a prodrug thereof, or a pharmaceutically acceptable salt thereof.

17. The 5-membered heteroaromatic ring compound of claim 16,
wherein m is 1 and R¹ and R² are each a hydrogen atom, or a prodrug thereof, or a pharmaceutically acceptable salt thereof.

18. The 5-membered heteroaromatic ring compound of claim 17,
wherein X is -N(R⁴) wherein R⁴ is as defined in claim 16, n is 1 and p is 0, or a prodrug thereof, or a pharmaceutically acceptable salt thereof.

19. The 5-membered heteroaromatic ring compound of claim 18,
wherein
R⁴ is a C₁₋₆ alkyl group optionally substituted by
(a) an aryl group optionally substituted by 1 to 3 substituent(s) selected from the group consisting of a halogen atom, a C₁₋₈ alkyl group and a C₁₋₄ haloalkyl group,
(b) a heteroaromatic ring group optionally substituted by 1 to 3 C₁₋₈ alkyl groups,
(c) a carboxy group,
(d) a C₁₋₄ alkoxycarbonyl group,
(e) -CO-N(R¹⁵)(R¹⁶)
wherein R¹⁵ and R¹⁶ are each independently a hydrogen atom, an aryl group wherein said aryl group is optionally substituted by 1 to 3 substituent(s) selected from the group consisting of a C₁₋₈ alkyl group, a C₁₋₄ alkoxy group, a carboxy group and a di(C₁₋₄ alkyl) amino group, a heteroaromatic ring group; a C₁₋₆ alkyl group wherein said C₁₋₆ alkyl group is optionally substituted by an aryl group, or may form a 5- to 7-membered hetero ring optionally containing at least one heteroatom selected from the group consisting of nitrogen atom, oxygen atom and sulfur atom, together with the nitrogen atom bonded thereto,
(f) -N(R¹⁵)(R¹⁶)
wherein R¹⁵ and R¹⁶ are each independently a hydrogen atom or an aryl group,
(g) -O-R¹⁷
wherein R¹⁷ is an aryl group, or
(h) a C₃₋₇ cycloalkyl group, or a prodrug thereof, or a pharmaceutically acceptable salt thereof.

20. The 5-membered heteroaromatic ring compound of claim 19, wherein R⁴ is a methyl group substituted by a heteroaromatic ring group optionally substituted by one C₁₋₈ alkyl group, or a prodrug thereof, or a pharmaceutically acceptable salt thereof.

21. The 5-membered heteroaromatic ring compound of claim 20, wherein the heteroaromatic ring defined in claim 20 is a thiazolyl group, an oxazolyl group, a benzimidazolyl group, a pyridyl group or a quinolyl group, or a prodrug thereof, or a pharmaceutically acceptable salt thereof.

22. The 5-membered heteroaromatic ring compound of claim 19,
wherein R⁴ is a methyl group substituted by -CO-N(R¹⁵)(R¹⁶) wherein R¹⁵ is a hydrogen atom and R¹⁶ is an aryl group optionally substituted by one C₁₋₈ alkyl group, or a prodrug thereof, or a pharmaceutically acceptable salt thereof.

23. The 5-membered heteroaromatic ring compound of any of claims 1 to 22, which is selected from the group consisting of the following compounds, or a prodrug thereof, or a pharmaceutically acceptable salt thereof:
(1) 5-{4-[4-({[4-(1-ethylpropyl)phenyl]isopropylamino}methyl)phenyl]thiazol-2-ylmethoxy}nicotinic acid;
(2) 4-{4-[4-({[4-(1-ethylpropyl)phenyl]isopropylamino}methyl)phenyl]thiazol-2-ylmethoxy}benzoic acid;
(3) 6-{4-[4-({[4-(1-ethylpropyl)phenyl]isopropylamino}methyl)phenyl]thiazol-2-ylmethoxy}nicotinic acid;
(4) 5-{4-[4-({methyl[4-(1-propylbutyl)phenyl]amino}methyl)phenyl]thiazol-2-ylmethoxy}nicotinic acid;
(5) 4-{4-[4-({methyl[4-(1-propylbutyl)phenyl]amino}methyl)phenyl]thiazol-2-ylmethoxy}benzoic acid;
(6) 6-[4-(4-{[(4-isopropylphenyl)(1-propylbutyl)amino]methyl}phenyl)thiazol-2-ylmethoxy]nicotinic acid;
(7) 5-[4-(4-{[(4-isopropylphenyl)(1-propylbutyl)amino]methyl}phenyl)thiazol-2-ylmethoxy]nicotinic acid;
(8) 5-[4-(4-{[isobutyl(4-isopropylphenyl)amino]methyl}phenyl)thiazol-2-ylmethoxy]nicotinic acid;
(9) 4-[4-(4-{[(4-isopropylphenyl)(1-propylbutyl)amino]methyl}phenyl)thiazol-2-ylmethoxy]benzoic acid;
(10) 3-[4-(4-{[(4-isopropylphenyl)(1-propylbutyl)amino]methyl}phenyl)thiazol-2-ylmethoxy]benzoic acid;
(11) 6-[4-(4-{[(1-ethylpropyl)(4-isopropylphenyl)amino]methyl}phenyl)thiazol-2-ylmethoxy]nicotinic acid;
(12) 5-[4-(4-{[(1-ethylpropyl)(4-isopropylphenyl)amino]methyl}phenyl)thiazol-2-ylmethoxy]nicotinic acid;
(13) 4-{4-[4-({[4-(1-ethylpropyl)phenyl]isopropylamino}methyl)phenyl]thiazol-2-ylmethylthio}benzoic acid;
(14) 4-{4-[4-({methyl[4-(1-propylbutyl)phenyl]amino}methyl)phenyl]thiazol-2-ylmethylthio}benzoic acid;
(15) 4-(methyl-{4-[4-({methyl[4-(1-propylbutyl)phenyl]amino}methyl)phenyl]thiazol-2-ylmethyl}sulfamoyl)benzoic acid;
(16) sodium 4-(methyl{4-[4-({methyl[4-(1-propylbutyl)phenyl]amino}methyl)phenyl]thiazol-2-ylmethyl}sulfamoyl)butyrate;
(17) 4-{[(4-{4-[(4-cyclohexylphenylamino)methyl]phenyl}thiazol-2-yl)methylamino]methyl}benzoic acid;
(18) 4-({[4-(4-{[(4-cyclohexylphenyl)methylamino]methyl}phenyl)thiazol-2-yl]methylamino}methyl)benzoic acid;
(19) 4-[(methyl-{4-[4-({methyl[4-(1-propylbutyl)phenyl]amino}methyl)phenyl]thiazol-2-yl}amino)methyl]benzoic acid;
(20) 4-[({4-[4-({[4-(1-ethylpropyl)phenyl]isopropylamino}methyl)phenyl]thiazol-2-yl}methylamino)methyl]benzoic acid;
(21) (S)-({4-[4-({[4-(1-ethylpropyl)phenyl]isopropylamino}methyl)phenyl]thiazol-2-ylmethyl}methylamino)phenylacetic acid;
(22) (S)-2-({4-[4-({[4-(1-ethylpropyl)phenyl]isopropylamino}methyl)phenyl]thiazol-2-ylmethyl}methylamino)-3-phenylpropionic acid;
(23) {benzyl[4-(4-{[(4-tert-butylphenyl)isobutylamino]methyl}phenyl)thiazol-2-ylmethyl]amino}acetic acid;
(24) {benzyl[4-(4-{[(4-chlorophenyl)isobutylamino]methyl}phenyl)thiazol-2-ylmethyl]amino}acetic acid;
(25) (benzyl{4-[4-({methyl[4-(1-propylbutyl)phenyl]amino}methyl)phenyl]thiazol-2-ylmethyl}amino)acetic acid;
(26) (benzyl{4-[4-({[4-(1-ethylpropyl)phenyl]isopropylamino}methyl)phenyl]thiazol-2-ylmethyl}amino)acetic acid;
(27) {1-[4-(4-{[(4-tert-butylphenyl)isobutylamino]methyl}phenyl)thiazol-2-ylmethyl]-3-phenylureido}acetic acid;
(28) {benzoyl-[4-(4-{[(4-tert-butylphenyl)isobutylamino]methyl}phenyl)thiazol-2-ylmethyl]amino}acetic acid;
(29) [[4-(4-{[(4-tert-butylphenyl)isobutylamino]methyl}phenyl)thiazol-2-ylmethyl]-(pyridin-2-ylcarbonyl)amino]acetic acid;
(30) [[4-(4-{[(4-tert-butylphenyl)isobutylamino]methyl}phenyl)thiazol-2-ylmethyl]-(pyridin-3-ylcarbonyl)amino]acetic acid;
(31) {benzenesulfonyl-[4-(4-{[(4-tert-butylphenyl)isobutylamino]methyl}phenyl)thiazol-2-ylmethyl]amino}acetic acid;
(32) 2-{4-[4-({[4-(1-ethylpropyl)phenyl]isopropylamino}methyl)phenyl]thiazol-2-ylmethyl}-1,2,3,4-tetrahydroisoquinoline-3-carboxylic acid;
(33) (S)-2-{4-[4-({[4-(1-ethylpropyl)phenyl]isopropylamino}methyl)phenyl]thiazol-2-ylmethyl}-1,2,3,4-tetrahydroisoquinoline-3-carboxylic acid;
(34) (S)-2-{4-[4-({methyl[4-(1-propylbutyl)phenyl]amino}methyl)phenyl]thiazol-2-ylmethyl}-1,2,3,4-tetrahydroisoquinoline-3-carboxylic acid;
(35) (S)-2-[4-(4-{[(1-ethylpropyl)-(4-isopropylphenyl)amino]methyl}phenyl)thiazol-2-ylmethyl]-1,2,3,4-tetrahydroisoquinoline-3-carboxylic acid;
(36) 4-({4-[4-(4-cyclohexylphenoxymethyl)phenyl]thiazol-2-yl}methylamino)benzoic acid;
(37) 4-[({4-[4-(4-cyclohexylphenoxymethyl)phenyl]thiazol-2-yl}methylamino)methyl]benzoic acid;
(38) 4-{[(4-{4-[4-(1,1-dimethylpropyl)phenoxymethyl]phenyl}thiazol-2-yl)methylamino]methyl}benzoic acid;
(39) 4-{[methyl-(4-{4-[4-(1-propylbutyl)phenoxymethyl]phenyl}thiazol-2-yl)amino]methyl}benzoic acid;
(40) sodium 4-{[methyl(4-{4-[4-(1-propylbutyl)phenoxymethyl]phenyl}thiazol-2-yl)amino]methyl}benzoate;
(41) (S)-[methyl-(4-{4-[4-(1-propylbutyl)phenoxymethyl]phenyl}thiazol-2-ylmethyl)amino]phenylacetic acid;
(42) (S)-2-[methyl-(4-(4-[4-(1-propylbutyl)phenoxymethyl]phenyl}thiazol-2-ylmethyl)amino]-3-phenylpropionic acid;
(43) (benzyl{4-[4-(2-tert-butyl-4-methylphenoxymethyl)phenyl]thiazol-2-ylmethyl}amino)acetic acid;
(44) [benzyl(4-{4-[4-(1-propylbutyl)phenoxymethyl]phenyl}thiazol-2-ylmethyl)amino]acetic acid;
(45) 2-(4-{4-[4-(1-propylbutyl)phenoxymethyl]phenyl}thiazol-2-ylmethyl)-1,2,3,4-tetrahydroisoquinoline-3-carboxylic acid;
(46) (S)-2-(4-{4-[4-(1-propylbutyl)phenoxymethyl]phenyl}thiazol-2-ylmethyl)-1,2,3,4-tetrahydroisoquinoline-3-carboxylic acid;
(47) (R)-2-(4-{4-[4-(1-propylbutyl)phenoxymethyl]phenyl}thiazol-2-ylmethyl)-1,2,3,4-tetrahydroisoquinoline-3-carboxylic acid;
(48) 5-(4-{4-[4-(1-propylbutyl)phenoxymethyl]phenyl}thiazol-2-ylmethoxy)nicotinic acid;
(49) 4-(4-{4-[4-(1-propylbutyl)phenoxymethyl]phenyl}thiazol-2-ylmethoxy)benzoic acid;
(50) 4-[methyl-(4-{4-[4-(1-propylbutyl)phenoxymethyl]phenyl}thiazol-2-ylmethyl)sulfamoyl]benzoic acid;
(51) 4-[methyl-(4-{4-[4-(1-propylbutyl)phenoxymethyl]phenyl}thiazol-2-ylmethyl)sulfamoyl]butyric acid;
(52) 4-({4-[4-(4-cyclohexylphenylcarbamoyl)phenyl]thiazol-2-yl}methylamino)benzoic acid;
(53) 4-[({4-[4-(4-cyclohexylphenylcarbamoyl)phenyl]thiazol-2-yl}methylamino)methyl]benzoic acid;
(54) [benzyl(4-{4-[4-(1-propylbutyl)phenylcarbamoyl]phenyl}thiazol-2-ylmethyl)amino]acetic acid;
(55) [benzyl(4-{4-[4-(1-propylbutyl)benzylcarbamoyl]phenyl}thiazol-2-ylmethyl)amino]acetic acid;
(56) {benzyl[4-(4-{methyl[4-(1-propylbutyl)benzyl]-carbamoyl}phenyl)thiazol-2-ylmethyl]amino}acetic acid;
(57) {benzyl[4-(4-{ethyl-[4-(1-propylbutyl)benzyl]-carbamoyl}phenyl)thiazol-2-ylmethyl]amino}acetic acid;
(58) 5-{4-[4-({(2-hydroxy-2-methylpropyl)-[4-(1-propylbutyl)phenyl]amino}methyl)phenyl]thiazol-2-ylmethoxy}nicotinic acid;
(59) [[4-(4-{[(4-tert-butylphenyl)isobutylamino]methyl}phenyl)thiazol-2-ylmethyl]-(morpholine-4-carbonyl)amino]acetic acid;
(60) sodium 5-{4-[4-({[4-(1-ethylpropyl)phenyl]isopropylamino}methyl)phenyl]thiazol-2-ylmethoxy}-nicotinate;
(61) sodium (S)-2-(4-{4-[4-(1-propylbutyl)phenoxymethyl]phenyl}thiazol-2-ylmethyl)-1,2,3,4-tetrahydroisoquinoline-3-carboxylate;
(62) sodium 5-(4-{4-[4-(1-propylbutyl)phenoxymethyl]phenyl}thiazol-2-ylmethoxy)nicotinate;
(63) 3-{4-[4-({[4-(1-ethylpropyl)phenyl]isopropylamino}methyl)phenyl]thiazol-2-ylmethoxy}-5-methoxy-benzoic acid;
(64) (1-{4-[4-({[4-(1-ethylpropyl)phenyl]isopropylamino}methyl)phenyl]thiazol-2-ylmethyl}-3-phenylureido)acetic acid;
(65) (1-{4-[4-({[4-(1-ethylpropyl)phenyl]isopropylamino}methyl)phenyl]thiazol-2-ylmethyl}-3-p-tolylureido)acetic acid;
(66) [1-{4-[4-({[4-(1-ethylpropyl)phenyl]isopropylamino}methyl)phenyl]thiazol-2-ylmethyl}-3-(4-isopropylphenyl)ureido]acetic acid;
(67) (1-{4-[4-({[4-(1-ethylpropyl}phenyl]isopropylamino}methyl)phenyl]thiazol-2-ylmethyl}-3-methyl-3-phenylureido)acetic acid;
(68) 5-(4-[4-({isopropyl[4-(1-propylbutyl)phenyl]amino}methyl)phenyl]thiazol-2-ylmethoxy}nicotinic acid;
(69) [3-phenyl-1-(4-{4-[4-(1-propylbutyl)phenoxymethyl]phenyl}thiazol-2-ylmethyl)ureido]acetic acid;
(70) (3-(2,6-dimethylphenyl)-1-{4-[4-({[4-(1-ethylpropyl)phenyl]isopropylamino}methyl)phenyl]thiazol-2-ylmethyl}ureido)acetic acid;
(71) ({4-[4-({[4-(1-ethylpropyl)phenyl]isopropylamino}methyl)phenyl]thiazol-2-ylmethyl}phenylcarbamoylmethylamino)acetic acid;
(72) (1-{4-[4-({ [4-(1-ethylpropyl)phenyl]isopropylamino}methyl)phenyl]thiazol-2-ylmethyl}-3-isopropyl-ureido)acetic acid;
(73) 3-{4-[4-({isopropyl[4-(1-propylbutyl)phenyl]amino}methyl)phenyl]thiazol-2-ylmethoxy}isoxazole-5-carboxylic acid;
(74) 5-[4-(4-{[(2-ethylbutyl)-(4-isopropylphenyl)amino]methyl}phenyl)thiazol-2-ylmethoxy]nicotinic acid;
(75) [{4-[4-({[4-(1-ethylpropyl)phenyl]isopropylamino}methyl)phenyl]thiazol-2-ylmethyl}-(piperidine-1-carbonyl)amino]acetic acid;
(76) 2-(4-{4-[4-(1-propylbutyl)phenoxymethyl]phenyl}thiazol-2-ylmethoxy)nicotinic acid;
(77) [{4-[4-({[4-(1-ethylpropyl)phenyl]isopropylamino}methyl)phenyl]thiazol-2-ylmethyl}-(p-tolylcarbamoylmethyl)amino]acetic acid;
(78) {{4-[4-({[4-(1-ethylpropyl)phenyl]isopropylamino}methyl)phenyl]thiazol-2-ylmethyl}-[(4-isopropylphenylcarbamoyl)methyl]amino}acetic acid;
(79) (1-{4-[4-({isopropyl[4-(1-propylbutyl)phenyl]amino}methyl)phenyl]thiazol-2-ylmethyl}-3-methyl-3-phenylureido)acetic acid;
(80) (1-{4-[4-({isopropyl[4-(1-propylbutyl)phenyl]amino}methyl)phenyl]thiazol-2-ylmethyl}-3-p-tolylureido)acetic acid;
(81) ((2,3-dihydro-indole-1-carbonyl)-{4-[4-({isopropyl[4-(1-propylbutyl)phenyl]amino}methyl)phenyl]thiazol-2-ylmethyl}amino)acetic acid;
(82) 3-(4-{4-[4-(1-propylbutyl)phenoxymethyl]phenyl}thiazol-2-ylmethoxy)pyridine-2-carboxylic acid;
(83) {[4-(4-{[(2-ethylbutyl)-(4-isopropylphenyl)amino]methyl}phenyl)thiazol-2-ylmethyl]phenylcarbamoylmethylamino}acetic acid;
(84) {[4-(4-{[(2-ethylbutyl)-(4-isopropylphenyl)amino]methyl}phenyl)thiazol-2-ylmethyl]-[(4-isopropylphenylcarbamoyl)methyl]amino}acetic acid;
(85) 4-(2-{carboxymethyl[4-(4-{[(2-ethylbutyl)-(4-isopropylphenyl)amino]methyl}phenyl)thiazol-2-ylmethyl]amino}acetylamino)benzoic acid;
(86) 6-(4-{4-[4-(l-propylbutyl)phenoxymethyl]phenyl}thiazol-2-ylmethoxy)pyridine-2-carboxylic acid;
(87) 5-{4-[4-({isobutyl[4-(1-propylbutyl)phenyl]amino}methyl)phenyl]thiazol-2-ylmethoxy}nicotinic acid;
(88) (1-{4-[4-({isobutyl[4-(1-propylbutyl)phenyl]amino}methyl)phenyl]thiazol-2-ylmethyl}-3-phenylureido)acetic acid;
(89) 5-{4-[4-({[4-(1-ethylpropyl)phenyl]isopropylamino}methyl)phenyl]thiazol-2-ylmethoxy}nicotinic acid methyl ester;
(90) 4-amino-3-{4-[4-({[4-(1-ethylpropyl)phenyl]isopropylamino}methyl)phenyl]thiazol-2-ylmethoxy}benzoic acid;
(91) 3-{4-[4-({[4-(1-ethylpropyl)phenyl]isopropylamino}methyl)phenyl]thiazol-2-ylmethoxy}benzoic acid;
(92) 3-({4-[4-({[4-(1-ethylpropyl)phenyl]isopropylamino}methyl)phenyl]thiazol-2-ylmethyl}amino)benzoic acid;
(93) 3-{4-[4-({[4-(1-ethylpropyl)phenyl]isopropylamino}methyl)phenyl]thiazol-2-ylmethoxy}-4-nitro-benzoic acid;
(94) ((1H-benzimidazol-2-ylmethyl)-{4-[4-({isopropyl[4-(1-propylbutyl)phenyl]amino}methyl)phenyl]thiazol-2-ylmethyl}amino)acetic acid;
(95) [phenylcarbamoylmethyl(4-{4-[4-(1-propylbutyl)phenoxymethyl]phenyl}thiazol-2-ylmethyl)amino]acetic acid;
(96) [(4-{4-[4-(1-propylbutyl)phenoxymethyl]phenyl}thiazol-2-ylmethyl)-(pyridin-3-ylcarbamoylmethyl)amino]acetic acid;
(97) [[(4-dimethylaminophenylcarbamoyl)methyl]-(4-{4-[4-(1-propylbutyl)phenoxymethyl]phenyl}thiazol-2-ylmethyl)amino]acetic acid;
(98) [[(4-methoxyphenylcarbamoyl)methyl]-(4-{4-[4-(1-propylbutyl)phenoxymethyl]phenyl}thiazol-2-ylmethyl)amino]acetic acid;
(99) [[(isopropylphenylcarbamoyl)methyl]-(4-{4-[4-(1-propylbutyl)phenoxymethyl]phenyl}thiazol-2-ylmethyl)amino]acetic acid;
(100) [(4-{4-[4-(1-propylbutyl)phenoxymethyl]phenyl}thiazol-2-ylmethyl)-(pyridin-2-ylcarbamoylmethyl)amino]acetic acid;
(101) [(2-oxo-2-pyrrolidin-1-yl-ethyl)-(4-{4-[4-(1-propylbutyl)phenoxymethyl]phenyl}thiazol-2-ylmethyl)amino]acetic acid;
(102) [(4-methylthiazol-2-ylmethyl)-(4-{4-[4-(1-propylbutyl)phenoxymethyl]phenyl}thiazol-2-ylmethyl)amino]acetic acid;
(103) 4-(3-cyclohexylmethyl-3-{4-[4-({isopropyl[4-(1-propylbutyl)phenyl]amino}methyl)phenyl]thiazol-2-ylmethyl}ureido)benzoic acid;
(104) 4-(3-isobutyl-3-{4-[4-({isopropyl[4-(1-propylbutyl)phenyl]amino}methyl)phenyl]thiazol-2-ylmethyl}ureido)benzoic acid;
(105) (1-{4-[4-({isobutyl[4-(1-propylbutyl)phenyl]amino}methyl)phenyl]thiazol-2-ylmethyl}-3-methyl-3-phenylureido)acetic acid;
(106) ({4-[4-({isobutyl[4-(1-propylbutyl)phenyl]amino}methyl)phenyl]thiazol-2-ylmethyl}phenylcarbamoylmethylamino)acetic acid;
(107) {{4-[4-({isobutyl[4-(1-propylbutyl)phenyl]amino}methyl)phenyl]thiazol-2-ylmethyl}-[(4-isopropylphenylcarbamoyl)methyl]amino}acetic acid;
(108) 4-acetylamino-3-{4-[4-({isopropyl[4-(1-propylbutyl)phenyl]amino}methyl)phenyl]thiazol-2-ylmethoxy}benzoic acid;
(109) 4-(2-dimethylaminoacetylamino)-3-{4-[4-({isopropyl[4-(1-propylbutyl)phenyl]amino}methyl)phenyl]thiazol-2-ylmethoxy}benzoic acid;
(110) ((1H-benzimidazol-2-ylmethyl)-{4-[4-({[4-(1-ethylpropyl)phenyl]isopropylamino}methyl)phenyl]thiazol-2-ylmethyl}amino)acetic acid;
(111) [(1H-benzimidazol-2-ylmethyl)-(4-{4-[4-(1-propylbutyl)phenoxymethyl]phenyl}thiazol-2-ylmethyl)amino]acetic acid;
(112) 3-{4-[4-({isopropyl[4-(1-propylbutyl)phenyl]amino}methyl)phenyl]thiazol-2-ylmethoxy}-4-methanesulfonylaminobenzoic acid;
(113) 4-isobutyrylamino-3-{4-[4-({isopropyl[4-(1-propylbutyl)phenyl]amino}methyl)phenyl]thiazol-2-ylmethoxy}benzoic acid;
(114) 4-{4-[4-({[4-(1-ethylpropyl)phenyl]isopropylamino}methyl)phenyl]thiazol-2-ylmethyl}-3-oxo-3,4-dihydro-2H-benzo[1,4]oxazine-6-carboxylic acid;
(115) 4-{4-[4-({[4-(1-ethylpropyl)phenyl]isopropylamino}methyl)phenyl]thiazol-2-ylmethyl}-3-oxo-3,4-dihydro-2H-benzo[1,4]oxazine-8-carboxylic acid;
(116) ({4-[4-({isopropyl[4-(1-propylbutyl)phenyl]amino}methyl)phenyl]thiazol-2-ylmethyl}{methylphenylaminosulfonyl}amino)acetic acid;
(117) ([(4-isopropylphenylcarbamoyl)methyl]-{4-[4-({isopropyl[4-(1-propylbutyl)phenyl]amino}methyl)phenyl]thiazol-2-ylmethyl}amino)acetic acid;
(118) ([(3,5-dimethylphenylcarbamoyl)methyl]-{4-[4-({isopropyl[4-(1-propylbutyl)phenyl]amino}methyl)phenyl]thiazol-2-ylmethyl}amino)acetic acid;
(119) ([(4-dimethylaminophenylcarbamoyl)methyl]-{4-[4-({isopropyl[4-(1-propylbutyl)phenyl]amino}methyl)phenyl]thiazol-2-ylmethyl}amino)acetic acid;
(120) ((benzylcarbamoylmethyl)-{4-[4-({isopropyl[4-(1-propylbutyl)phenyl]amino}methyl)phenyl]thiazol-2-ylmethyl}amino)acetic acid;
(121) [{4-[4-({isopropyl[4-(1-propylbutyl)phenyl]amino}methyl)phenyl]thiazol-2-ylmethyl}-[2-(morpholin-4-yl)-2-oxo-ethyl]amino]acetic acid;
(122) [{4-[4-({[4-(1-ethylpropyl)phenyl]isopropylamino}methyl)phenyl]thiazol-2-ylmethyl}-(2-phenoxyethyl)amino]acetic acid;
(123) [{4-[4-({[4-(1-ethylpropyl)phenyl]isopropylamino}methyl)phenyl]thiazol-2-ylmethyl}-(2-phenylamino-ethyl)amino]acetic acid;
(124) 2-carboxymethoxy-5-({4-[4-({[4-(1-ethylpropyl)phenyl]isopropylamino}methyl)phenyl]thiazol-2-ylmethyl}methylamino)benzoic acid;
(125) 2-carboxymethoxy-5-[methyl(4-{4-[4-(1-propylbutyl)phenoxymethyl]phenyl}thiazol-2-ylmethyl)amino]benzoic acid;
(126) 3-{4-[4-({isopropyl[4-(1-propylbutyl)phenyl]amino}methyl)phenyl]thiazol-2-ylmethoxy}-4-(2-methylamino-acetylamino)benzoic acid;
(127) [(4-tert-butylthiazol-2-ylmethyl)-(4-{4-[4-(1-propylbutyl)phenoxymethyl]phenyl}thiazol-2-ylmethyl)amino]acetic acid;
(128) [phenyl(4-{4-[4-(1-propylbutyl)phenoxymethyl]phenyl}thiazol-2-ylmethyl)amino]acetic acid;
(129) [(2-phenoxyacetyl)-(4-{4-[4-(1-propylbutyl)phenoxymethyl]phenyl}thiazol-2-ylmethyl)amino]acetic acid;
(130) [(4-{4-[4-(1-propylbutyl)phenoxymethyl]phenyl}thiazol-2-ylmethyl)-(2-p-tolyloxy-acetyl)amino]acetic acid;
(131) (ethoxycarbonylmethyl{4-[4-({isopropyl[4-(1-propylbutyl)phenyl]amino}methyl)phenyl]thiazol-2-ylmethyl}amino)acetic acid dihydrochloride;
(132) ((4-tert-butylthiazol-2-ylmethyl)-{4-[4-({[4-(1-ethylpropyl)phenyl]isopropylamino}methyl)phenyl]thiazol-2-ylmethyl}amino)acetic acid;
(133) 6-{4-[4-({isopropyl[4-(1-propylbutyl)phenyl]amino}methyl)phenyl]thiazol-2-ylmethoxy}pyridine-2-carboxylic acid;
(134) [(2-benzyloxy-acetyl)-(4-{4-[4-(1-propylbutyl)phenoxymethyl]phenyl}thiazol-2-ylmethyl)amino]acetic acid;
(135) [[2-(4-isopropylphenoxy)acetyl]-(4-{4-[4-(1-propylbutyl)phenoxymethyl]phenyl}thiazol-2-ylmethyl)amino]acetic acid;
(136) [(4,5-dimethylthiazol-2-ylmethyl)-(4-{4-[4-(1-propylbutyl)phenoxymethyl]phenyl}thiazol-2-ylmethyl)amino]acetic acid;
(137) 5-(4-{5-[4-(1-propylbutyl)phenoxymethyl]thiophen-2-yl}thiazol-2-ylmethoxy)nicotinic acid;
(138) ((4-tert-butylthiazol-2-ylmethyl)-{4-[6-({isopropyl[4-(1-propylbutyl)phenyl]amino}methyl)benzoxazol-2-yl]thiazol-2-ylmethyl}amino)acetic acid;
(139) ((1H-benzimidazol-2-ylmethyl)-{4-[6-({isopropyl[4-(1-propylbutyl)phenyl]amino}methyl)benzoxazol-2-yl]thiazol-2-ylmethyl}amino)acetic acid;
(140) 5-{4-[6-({isopropyl[4-(1-propylbutyl)phenyl]amino}methyl)benzoxazol-2-yl]thiazol-2-ylmethoxy}nicotinic acid;
(141) [(5-tert-butylthiazol-2-ylmethyl)-(4-{4-[4-(1-propylbutyl)phenoxymethyl]phenyl}thiazol-2-ylmethyl)amino]acetic acid;
(142) [{4-[4-({isopropyl[4-(1-propylbutyl)phenyl]amino}methyl)phenyl]thiazol-2-ylmethyl}-(2-oxo-2-piperidin-1-yl-ethyl)amino]acetic acid;
(143) 6-[methyl-(4-{4-[4-(1-propylbutyl)phenoxymethyl]phenyl}thiazol-2-ylmethyl)amino]pyridine-2-carboxylic acid;
(144) ({4-[6-({isopropyl[4-(1-propylbutyl)phenyl]amino}methyl)benzoxazol-2-yl]thiazol-2-ylmethyl}amino)acetic acid;
(145) (S)-(carboxymethyl{4-[4-({isopropyl[4-(1-propylbutyl)phenyl]amino}methyl)phenyl]thiazol-2-ylmethyl}amino)phenylacetic acid;
(146) ((4,5-dimethylthiazol-2-ylmethyl)-{4-[4-({isopropyl[4-(1-propylbutyl)phenyl]amino}methyl)phenyl]thiazol-2-ylmethyl}amino)acetic acid;
(147) [(5-tert-butyloxazol-2-ylmethyl)-(4-{4-[4-(1-propylbutyl)phenoxymethyl]phenyl}thiazol-2-ylmethyl)amino]acetic acid;
(148) [(1-methyl-1H-benzimidazol-2-ylmethyl)-(4-{4-[4-(1-propylbutyl)phenoxymethyl]phenyl}thiazol-2-ylmethyl)amino]acetic acid;
(149) (isobutoxycarbonylmethyl{4-[4-({isopropyl[4-(1-propylbutyl)phenyl]amino}methyl)phenyl]thiazol-2-ylmethyl}amino)acetic acid dihydrochloride;
(150) ({4-[4-({isopropyl[4-(1-propylbutyl)phenyl]amino}methyl)phenyl]thiazol-2-ylmethyl}propoxycarbonylmethylamino)acetic acid dihydrochloride;
(151) 1-(4-{4-[4-(1-propylbutyl)phenoxymethyl]phenyl}thiazol-2-ylmethyl)piperidine-4-carboxylic acid hydrochloride;
(152) 1-{4-[4-({[4-(1-ethylpropyl)phenyl]isopropylamino}methyl)phenyl]thiazol-2-ylmethyl}piperidine-4-carboxylic acid;
(153) 4-[4-(4-{4-[4-(1-propylbutyl)phenoxymethyl]phenyl}thiazol-2-ylmethyl)piperazin-1-yl]benzoic acid;
(154) 4-(4-{4-[4-({[4-(1-ethylpropyl)phenyl]isopropylamino}methyl)phenyl]thiazol-2-ylmethyl)-piperazin-1-yl)benzoic acid;
(155) 2-[4-(4-{4-[4-(1-propylbutyl)benzyloxy]phenyl}thiazol-2-ylmethyl)piperazin-1-yl]benzoic acid;
(156) 3-[4-(4-{4-[4-(1-propylbutyl)benzyloxy]phenyl}thiazol-2-ylmethyl)piperazin-1-yl]benzoic acid;
(157) 4-(4-{1-[4-(1-propylbutyl)benzyl]-1H-indol-3-yl)thiazol-2-ylmethoxy)benzoic acid;
(158) 4-{4-[1-(4-isopropylbenzyl)-5-(1-propylbutyl)-1H-benzimidazol-2-yl]thiazol-2-ylmethoxy}benzoic acid;
(159) 4-{4-[1-(6-methylpyridin-2-ylmethyl)-5-(1-propylbutyl)-1H-benzimidazol-2-yl]thiazol-2-ylmethoxy}benzoic acid;
(160) 2-(4-{4-[4-(1-propylbutyl)phenoxymethyl]phenyl}thiazol-2-ylmethyl)-1,2,3,4-tetrahydroisoquinoline-7-carboxylic acid;
(161) 4-[1-(4-{4-[4-(1-propylbutyl)benzyloxy]phenyl}thiazol-2-ylmethyl)piperidin-4-yl]benzoic acid;
(162) 3-[1-(4-{4-[4-(1-propylbutyl)benzyloxy]phenyl}thiazol-2-ylmethyl)piperidin-4-yl]benzoic acid;
(163) 6-[(4-{4-[4-(1-propylbutyl)phenoxymethyl]phenyl}thiazol-2-ylmethyl)carbamoyl]nicotinic acid;
(164) 2-[1-(4-{4-[4-(1-propylbutyl)benzyloxy]phenyl}thiazol-2-ylmethyl)piperidin-4-yl]benzoic acid;
(165) 1-(4-{1-[4-(1-propylbutyl)benzyl]-1H-indol-3-yl}thiazol-2-ylmethyl)piperidine-4-carboxylic acid;
(166) 2-(4-{1-[4-(1-propylbutyl)benzyl]-1H-indol-3-yl}thiazol-2-ylmethyl)-1,2,3,4-tetrahydroisoquinoline-7-carboxylic acid;
(167) 3-[(4-{1-[4-(1-propylbutyl)benzyl]-1H-indol-3-yl}thiazol-2-ylmethyl)amino]benzoic acid;
(168) 6-[(2-aminoethyl)(4-{4-[4-(1-propylbutyl)phenoxymethyl]phenyl}thiazol-2-ylmethyl)carbamoyl]nicotinic acid hydrochloride;
(169) 2-(4-{1-[4-(1-propylbutyl)benzyl]-1H-benzimidazol-2-yl}thiazol-2-ylmethyl)-1,2,3,4-tetrahydroisoquinoline-7-carboxylic acid;
(170) 3-[4-(4-{1-[4-(1-propylbutyl)benzyl]-1H-benzimidazol-2-yl}thiazol-2-ylmethyl)piperazin-1-yl]benzoic acid;
(171) 4-[1-(4-{1-[4-(1-propylbutyl)benzyl]-1H-benzimidazol-2-yl}thiazol-2-ylmethyl)piperidin-4-yl]benzoic acid;
(172) 3-[4-(4-{1-[4-(1-propylbutyl)benzyl]-1H-indol-3-yl}thiazol-2-ylmethyl)piperazin-1-yl]benzoic acid;
(173) 3-(1-{4-[4-(3,4-dichloro-benzyloxy)phenyl]thiazol-2-ylmethyllpiperidin-4-yl)benzoic acid;
(174) 3-(1-{4-[4-(3,5-bis-trifluoromethylbenzyloxy)phenyl]thiazol-2-ylmethyl}piperidin-4-yl)benzoic acid;
(175) 3-(1-{4-[4-(4-butoxy-benzyloxy)phenyl]thiazol-2-ylmethyl}piperidin-4-yl)benzoic acid;
(176) 5-methyl-2-(4-{4-[4-(1-propylbutyl)phenoxymethyl]phenyl}thiazol-2-ylmethyl)-2H-pyrazole-3-carboxylic acid;
(177) 5-methyl-1-(4-{4-[4-(1-propylbutyl)phenoxymethyl]phenyl}thiazol-2-ylmethyl)-1H-pyrazole-3-carboxylic acid;
(178) 5-tert-butyl-1-(4-{4-[4-(1-propylbutyl)phenoxymethyl]phenyl}thiazol-2-ylmethyl)-1H-pyrazole-3-carboxylic acid;
(179) 5-tert-butyl-2-(4-{4-[4-(1-propylbutyl)phenoxymethyl]phenyl}thiazol-2-ylmethyl)-2H-pyrazole-3-carboxylic acid;
(180) (2S,4R)-4-hydroxy-1-(4-{1-[4-(1-propylbutyl)benzyl]-1H-indol-3-yl}thiazol-2-ylmethyl)pyrrolidine-2-carboxylic acid;
(181) 4-[4-(4-{4-[4-(1-propylbutyl)phenoxymethyl]phenyl}thiazol-2-ylmethyl)piperidin-1-yl]benzoic acid;
(182) 1-(4-{1-[4-(1-propylbutyl)benzyl]-1H-indol-3-yl}thiazol-2-ylmethyl)-1H-indole-3-carboxylic acid;
(183) 1-(4-{2-phenyl-1-[4-(1-propylbutyl)benzyl]-1H-indol-3-yl}thiazol-2-ylmethyl)piperidine-4-carboxylic acid;
(184) 3-(1-{4-[4-(4-methyl-3-nitrobenzyloxy)phenyl]thiazol-2-ylmethyl}piperidin-4-yl)benzoic acid;
(185) 2-{4-[1-(4-isopropylbenzyl)-6-(morpholin-4-yl)-1H-benzimidazol-2-yl]thiazol-2-ylmethyl}-1,2,3,4-tetrahydroisoquinoline-7-carboxylic acid;
(186) 3-(4-{4-[1-(4-isopropylbenzyl)-6-(morpholin-4-yl)-1H-benzimidazol-2-yl]thiazol-2-ylmethyl}-piperazin-1-yl)benzoic acid;
(187) {benzyl[4-(4-{methyl[4-(1-propylbutyl)benzyl]amino}phenyl)oxazol-2-ylmethyl]amino}acetic acid;
(188) 5-(4-{4-[4-(1-propylbutyl)benzyloxy]phenyl}thiazol-2-ylmethoxy)nicotinic acid;
(189) 4-(4-{4-[4-(1-propylbutyl)benzyloxy]phenyl}thiazol-2-ylmethoxy)benzoic acid;
(190) 4-(4-{4-[4-(1-propylbutyl)benzyloxy]phenyl}thiazol-2-ylmethylthio)benzoic acid;
(191) 4-{4-[4-(4-cyclohexylbenzyloxy)phenyl]thiazol-2-ylmethylthio}benzoic acid;
(192) 4-{4-[4-(4-cyclohexylbenzyloxy)phenyl]thiazol-2-ylmethanesulfonyl}benzoic acid;
(193) 4-[methyl-(4-{5-methyl-2-[4-(1-propylbutyl)benzyloxy]phenyl}thiazol-2-ylmethyl)sulfamoyl]benzoic acid;
(194) 4-[methyl-(4-{5-methyl-2-[4-(1-propylbutyl)benzyloxy]phenyl}thiazol-2-ylmethyl)amino]benzoic acid;
(195) (benzyl{4-[5-methyl-2-(4-trifluoromethylbenzyloxy)phenyl]thiazol-2-ylmethyl}amino)acetic acid;
(196) [benzyl(4-{5-methyl-2-[4-(1-propylbutyl)benzyloxy]phenyl}thiazol-2-ylmethyl)amino]acetic acid;
(197) [benzyl(4-{4-[4-(1-propylbutyl)benzyloxy]phenyl}thiazol-2-ylmethyl)amino]acetic acid;
(198) [benzyl(4-{4-[A-(1-ethylpropyl)benzyloxy]phenyl}thiazol-2-ylmethyl)amino]acetic acid;
(199) (benzyl{4-[5-tert-butyl-2-(4-isobutylbenzyloxy)phenyl]thiazol-2-ylmethyl}amino)acetic acid;
(200) [benzyl(4-{5-chloro-2-[4-(1-propylbutyl)benzyloxy]phenyl}thiazol-2-ylmethyl)amino]acetic acid;
(201) [(4-{4-[4-(1-ethylpropyl)benzyloxy]phenyl}thiazol-2-ylmethyl)-(4-fluoro-benzyl)amino]acetic acid;
(202) [(4-{4-[4-(1-ethylpropyl)benzyloxy]phenyl}thiazol-2-ylmethyl)-(4-isopropylbenzyl)amino]acetic acid;
(203) [(4-{4-[4-(1-ethylpropyl)benzyloxy]phenyl}thiazol-2-ylmethyl)-(4-trifluoromethylbenzyl)amino]acetic acid;
(204) [(4-chlorobenzyl)-(4-{4-[4-(1-ethylpropyl)benzyloxy]phenyl}thiazol-2-ylmethyl)amino]acetic acid;
(205) [(3,5-dimethylbenzyl)-(4-{4-[4-(1-ethylpropyl)benzyloxy]phenyl}thiazol-2-ylmethyl)amino]acetic acid;
(206) [(4-{4-[4-(1-propylbutyl)benzyloxy]phenyl}thiazol-2-ylmethyl)-pyridin-2-ylmethylamino]acetic acid;
(207) [(4-{4-[4-(1-ethylpropyl)benzyloxy]phenyl}thiazol-2-ylmethyl)-pyridin-2-ylmethylamino]acetic acid;
(208) [(4-{5-methyl-2-[4-(1-propylbutyl)benzyloxy]phenyl}thiazol-2-ylmethyl)-pyridin-2-ylmethylamino]acetic acid;
(209) [benzoyl-(4-{4-[4-(1-propylbutyl)benzyloxy]phenyl}thiazol-2-ylmethyl)amino]acetic acid;
(210) [(4-{4-[4-(1-ethylpropyl)benzyloxy]phenyl}thiazol-2-ylmethyl)-(4-methylbenzoyl)amino]acetic acid;
(211) [(4-methoxybenzoyl)-(4-{4-[4-(1-propylbutyl)benzyloxy]phenyl}thiazol-2-ylmethyl)amino]acetic acid;
(212) 2-(4-{5-methyl-2-[4-(1-propylbutyl)benzyloxy]phenyl}thiazol-2-ylmethyl)-1,2,3,4-tetrahydroisoquinoline-3-carboxylic acid;
(213) (S)-2-(4-{4-[4-(1-propylbutyl)benzyloxy]phenyl}thiazol-2-ylmethyl)-1,2,3,4-tetrahydroisoquinoline-3-carboxylic acid;
(214) {benzyl[4-(4-{[4-(2,2-dimethylpropyl)benzyl]methylamino}phenyl)thiazol-2-ylmethyl]amino}acetic acid;
(215) {benzyl[4-(4-{[trans-4-(4-tert-butylphenyl)cyclohexylmethyl]methylamino}phenyl)thiazol-2-ylmethyllaminolacetic acid;
(216) [benzyl(4-{4-[(4-cyclohexylbenzyl)methylamino]phenyl}thiazol-2-ylmethyl)amino]acetic acid;
(217) 3-[benzyl(4-{4-[(4-cyclohexylbenzyl)methylamino]phenyl}thiazol-2-ylmethyl)amino]propionic acid;
(218) (benzyl{4-[4-({2-[4-(2,2-dimethylpropyl)phenyl]-ethyl}methylamino)phenyl]thiazol-2-ylmethyl}amino)acetic acid;
(219) {benzyl[4-(4-{methyl[4-(trans-4-methylcyclohexyl)benzyl]amino}phenyl)thiazol-2-ylmethyl]amino}acetic acid;
(220) {benzyl[4-(4-{[4-(cis-4-fluorocyclohexyl)benzyl]methylamino}phenyl)thiazol-2-ylmethyl]amino}acetic acid;
(221) {benzyl[4-(4-{[trans-4-(4-chlorophenyl)-cyclohexylmethyl]methylamino}phenyl)thiazol-2-ylmethyl]amino}acetic acid;
(222) {benzyl[4-(4-{[4-(4,4-dimethylcyclohexyl)benzyl]methylamino}phenyl)thiazol-2-ylmethyl]amino}acetic acid;
(223) {benzyl[4-(4-{methyl[4-(1-propylbutyl)benzyl]amino}phenyl)thiazol-2-ylmethyl]amino}acetic acid;
(224) (benzyl{4-[4-(biphenyl-4-ylmethylmethylamino)phenyl]thiazol-2-ylmethyl}amino)acetic acid;
(225) sodium [benzyl(4-{4-[(4-cyclohexylbenzyl)methylamino]phenyl}thiazol-2-ylmethyl)amino]-acetate;
(226) [benzyl(4-{4-[(4-isobutylbenzyl)methylamino]phenyl}thiazol-2-ylmethyl)amino]acetic acid;
(227) sodium {benzyl[4-(4-{methyl[4-(1-propylbutyl)benzyl]amino}phenyl)thiazol-2-ylmethyl]amino}acetate;
(228) {benzyl[4-(4-{[4-(2,2-dimethylpropylthio)benzyl]methylamino}phenyl)thiazol-2-ylmethyl]amino}acetic acid;
(229) {benzyl[4-(4-{methyl[4-(3-methylbutylthio)benzyl]amino}phenyl)thiazol-2-ylmethyl]amino}acetic acid;
(230) [benzyl(4-{4-[(4-dipropylaminobenzoyl)methylamino]phenyl}thiazol-2-ylmethyl)amino]acetic acid;
(231) [(4-{4-[ethyl(4-isopropylbenzyl)amino]phenyl}thiazol-2-ylmethyl)-(4-isopropylbenzyl)amino]acetic acid;
(232) [(4-isopropylbenzyl)-(4-{4-[isopropyl-(4-isopropylbenzyl)amino]phenyl}thiazol-2-ylmethyl)amino]acetic acid;
(233) [(4-tert-butylbenzyl)-(4-{4-[isopropyl-(4-isopropylbenzyl)amino]phenyl}thiazol-2-ylmethyl)amino]acetic acid;
(234) [(4-chlorobenzyl)-(4-{4-[(4-isobutylbenzyl)methylamino]phenyl}thiazol-2-ylmethyl)amino]acetic acid;
(235) {{4-[4-(benzylmethylamino)phenyl]thiazol-2-ylmethyl}-[4-(1-propylbutyl)benzyl]amino}acetic acid;
(236) [{4-[4-(benzylmethylamino)phenyl]thiazol-2-ylmethyl}-(4-chloro-benzyl)amino]acetic acid;
(237) [{4-[4-(benzylmethylamino)phenyl]thiazol-2-ylmethyl}-(2-chloro-benzyl)amino]acetic acid;
(238) [{4-[4-(benzylmethylamino)phenyl]thiazol-2-ylmethyl}-(3,4-dichloro-benzyl)amino]acetic acid;
(239) {[4-(4-{methyl[4-(trans-4-methylcyclohexyl)benzyl]amino}phenyl)thiazol-2-ylmethyl]pyridin-2-ylmethylamino}acetic acid;
(240) {[4-(4-{methyl[4-(1-propylbutyl)benzyl]amino}phenyl)thiazol-2-ylmethyl]pyridin-2-ylmethylamino}acetic acid;
(241) ({4-[4-(benzylmethylamino)phenyl]thiazol-2-ylmethyl}naphthalen-1-ylmethylamino)acetic acid;
(242) ({4-[4-(benzylmethylamino)phenyl]thiazol-2-ylmethyl}quinolin-2-ylmethylamino)acetic acid;
(243) ((2-benzo[b]thiophen-3-yl-acetyl)-{4-[4-(benzylmethylamino)phenyl]thiazol-2-ylmethyl}amino)acetic acid;
(244) [[2-(4-chlorophenyl)acetyl]-(4-{4-[(4-isobutylbenzyl)methylamino]phenyl}thiazol-2-ylmethyl)amino]acetic acid;
(245) {(4-{4-[(4-isobutylbenzyl)methylamino]phenyl}thiazol-2-ylmethyl)-[2-(4-isopropylphenyl)acetyl]amino}acetic acid;
(246) [(4-{4-[(4-isobutylbenzyl)methylamino]phenyl}thiazol-2-ylmethyl)-(3-methyl-butyryl)amino]acetic acid;
(247) {1-[4-(4-{[4-(2,2-dimethylpropyl)benzyl]methylamino}phenyl)thiazol-2-ylmethyl]-3-phenylureido}acetic acid;
(248) [benzoyl-(4-{4-[(4-isobutylbenzyl)methylamino]phenyl}thiazol-2-ylmethyl)amino]acetic acid;
(249) {(4-methylbenzoyl}-[4-(4-{methyl[4-(1-propylbutyl)benzyl]amino}phenyl)thiazol-2-ylmethyl]amino}acetic acid;
(250) sodium {(4-isopropylbenzoyl)-[4-(4-{methyl[4-(1-propylbutyl)benzyl]amino}phenyl)thiazol-2-ylmethyl]amino}acetate;
(251) sodium (S)-{3-[4-(4-{methyl[4-(1-propylbutyl)benzyl]amino}phenyl)thiazol-2-yl]-3,4-dihydro-1H-isoquinolin-2-yl}-acetate;
(252) 1-(4-{4-[(4-cyclohexylbenzyl)methylamino]phenyl}thiazol-2-ylmethyl)piperidine-4-carboxylic acid;
(253) 1-(4-{4-[(4-cyclohexylbenzyl)methylamino]phenyl}thiazol-2-ylmethyl)piperidine-3-carboxylic acid;
(254) 1-(4-{4-[(4-cyclohexylbenzyl)methylamino]phenyl}thiazol-2-ylmethyl)-4-phenylpiperidine-4-carboxylic acid;
(255) 1-(4-{4-[(4-cyclohexylbenzyl)methylamino]phenyl}thiazol-2-ylmethyl)-4-(3-methylbutyl)piperidine-4-carboxylic acid;
(256) 1-[4-(4-{methyl[4-(trans-4-methylcyclohexyl)benzyl]amino}phenyl)thiazol-2-ylmethyl]-4-phenylpiperidine-4-carboxylic acid;
(257) 1-[4-(4-{[trans-4-(4-chloro-phenyl)-cyclohexylmethyl]methylamino}phenyl)thiazol-2-ylmethyl]-4-phenylpiperidine-4-carboxylic acid;
(258) 2-[4-(4-{methyl[4-(trans-4-methylcyclohexyl)benzyl]amino}phenyl)thiazol-2-ylmethyl]-1,2,3,4-tetrahydroisoquinoline-3-carboxylic acid;
(259) 2-(4-{4-[(4-isobutylbenzyl)methylamino]phenyl}thiazol-2-ylmethyl)-1,2,3,4-tetrahydroisoquinoline-3-carboxylic acid;
(260) 2-[4-(4-{methyl[4-(1-propylbutyl)benzyl]amino}phenyl)thiazol-2-ylmethyl]-1,2,3,4-tetrahydroisoquinoline-3-carboxylic acid;
(261) 2-[4-(4-{[4-(2,2-dimethylpropyl)benzyl]methylamino}phenyl)thiazol-2-ylmethyl]-1,2,3,4-tetrahydroisoquinoline-3-carboxylic acid;
(262) 5-{[(4-{4-[(4-cyclohexylbenzyl)methylamino]phenyl}thiazol-2-yl)methylaminolmethyl)furan-2-carboxylic acid;
(263) 2-[(4-{4-[(4-cyclohexylbenzyl)methylamino]phenyl}thiazol-2-ylmethyl)amino]-3-phenylpropionic acid;
(264) [(4-{4-[(4-cyclohexylbenzyl)methylamino]phenyl}thiazol-2-ylmethyl)amino]phenylacetic acid;
(265) 2-[(4-{4-[(4-cyclohexylbenzyl)methylamino]phenyl}thiazol-2-ylmethyl)amino]propionic acid;
(266) 3-(4-chlorophenyl)-2-[(4-{4-[(4-isobutylbenzyl)methylamino]phenyl}thiazol-2-ylmethyl)amino]propionic acid;
(267) [(4-{4-[(4-cyclohexylbenzyl)methylamino]phenyl}thiazol-2-ylmethyl)methylamino]acetic acid;
(268) 3-[(4-{4-[(4-cyclohexylbenzyl)methylamino]phenyl}thiazol-2-ylmethyl)methylamino]propionic acid;
(269) 4-{[(4-{4-[(4-cyclohexylbenzyl)methylamino]phenyl}thiazol-2-ylmethyl)methylamino]methyl}benzoic acid;
(270) 4-[(4-{4-[(4-cyclohexylbenzyl)methylamino]phenyl}thiazol-2-ylmethyl)methylamino]benzoic acid;
(271) 6-[(4-{4-[(4-cyclohexylbenzyl)methylamino]phenyl}thiazol-2-ylmethyl)methylamino]nicotinic acid;
(272) 2-[(4-{4-[(4-isobutylbenzyl)methylamino]phenyl}thiazol-2-ylmethyl)methylamino]-3-phenylpropionic acid;
(273) (S)-2-{methyl[4-(4-{methyl[4-(1-propylbutyl)benzyl]amino}phenyl)thiazol-2-ylmethyl]amino}-3-phenylpropionic acid;
(274) (S)-{methyl[4-(4-{methyl[4-(1-propylbutyl)benzyl]amino}phenyl)thiazol-2-ylmethyl]amino}phenylacetic acid;
(275) {[4-(4-{[4-(2,2-dimethylpropyl)benzyl]methylamino}phenyl)thiazol-2-ylmethyl]methylamino}phenylacetic acid;
(276) 2-{[4-(4-{[4-(2,2-dimethylpropyl)benzyl]methylamino}phenyl)thiazol-2-ylmethyl]methylamino}-3-phenylpropionic acid;
(277) {carboxymethyl[4-(4-{methyl[4-(1-propylbutyl)benzyl]amino}phenyl)thiazol-2-ylmethyl]amino}acetic acid;
(278) [(4-{4-[(4-cyclohexylbenzyl)methylamino)phenyl}thiazol-2-ylmethyl)-(3-methylbutyl)amino]acetic acid;
(279) {(3-methylbutyl)-[4-(4-{methyl[4-(trans-4-methylcyclohexyl)benzyl]amino}phenyl)thiazol-2-ylmethyl]amino}acetic acid;
(280) [(4-{4-[(4-isobutylbenzyl)methylamino]phenyl}thiazol-2-ylmethyl)-(3-methylbutyl)amino]acetic acid;
(281) 5-[4-(4-{methyl[4-(1-propylbutyl)benzyl]amino}phenyl)thiazol-2-ylmethoxy]nicotinic acid;
(282) 4-[4-(4-{methyl[4-(1-propylbutyl)benzyl]amino}phenyl)thiazol-2-ylmethoxy]benzoic acid;
(283) 4-[4-(4-{methyl[4-(1-propylbutyl)benzyl]amino}phenyl)thiazol-2-ylmethylthio]benzoic acid;
(284) 4-[(4-{4-[(4-cyclohexylbenzyl)methylamino]phenyl}thiazol-2-ylmethyl)sulfamoyl]benzoic acid;
(285) 4-{[4-(4-{[4-(4,4-dimethylcyclohexyl)benzyl]methylamino}phenyl)thiazol-2-ylmethyl]methylsulfamoyl}benzoic acid;
(286) {[4-(4-{[4-(4,4-dimethylcyclohexyl)benzyl]methylamino}phenyl)thiazol-2-ylmethyl]methylsulfamoyl}acetic acid;
(287) 4-[(4-{4-[(4-cyclohexylbenzyl)methylamino)phenyl}thiazol-2-ylmethyl)methylsulfamoyl]benzoic acid;
(288) 3-[(4-{4-[(4-cyclohexylbenzyl)methylamino]phenyl}thiazol-2-ylmethyl)methylsulfamoyl]benzoic acid;
(289) [(4-{4-[(4-cyclohexylbenzyl)methylamino]phenyl}thiazol-2-ylmethyl)methylsulfamoyl]acetic acid;
(290) 4-{[4-(4-{[4-(4,4-dimethylcyclohexyl)benzyl]methylamino}phenyl)thiazol-2-ylmethyl]methylsulfamoyl}butyric acid;
(291) [(4-{4-[(4-cyclohexylbenzyl)methylamino]phenyl}thiazol-2-ylmethyl)isobutyl-sulfamoyl]acetic acid;
(292) N-(4-{4-[(4-cyclohexylbenzyl)methylamino]phenyl}thiazol-2-ylmethyl)oxamic acid;
(293) {benzyl[4-(4-{[4-(1-propylbutyl)benzyl]methylaminocarbonyl}phenyl)thiazol-2-ylmethyl]amino}acetic acid;
(294) N-(4-{4-[(4-cyclohexylbenzyl)methylamino]phenyl}thiazol-2-ylmethyl)-N-methylterephthalamic acid;
(295) {benzyl[4-(4-{methyl[4-(trans-4-methylcyclohexyl)benzyl]amino}phenyl)thiazol-2-ylmethoxycarbonyl]amino}acetic acid;
(296) [3-(4-{4-[(4-cyclohexylbenzyl)methylamino]phenyl}thiazol-2-ylmethyl)-3-methyl-ureido]acetic acid;
(297) (cyclohexylmethyl{4-[6-(3,4-dichlorobenzyloxy)benzoxazol-2-yl]thiazol-2-yl}amino)acetic acid;
(298) [4-(4-{4-[(4-cyclohexylbenzyl)methylamino]phenyl}thiazol-2-ylmethyl)piperazin-1-yl]acetic acid;
(299) sodium (S)-2-(4-{4-[4-(1-propylbutyl)benzyloxy]phenyl}thiazol-2-ylmethyl)-1,2,3,4-tetrahydroisoquinoline-3-carboxylate;
(300) sodium 5-(4-{4-[4-(1-propylbutyl)benzyloxy]phenyl}thiazol-2-ylmethoxy)nicotinate;
(301) 5-(4-{2-methyl-4-[4-(1-propylbutyl)benzyloxy]phenyl}thiazol-2-ylmethoxy)nicotinic acid;
(302) 5-(4-{3-methoxy-4-[4-(1-propylbutyl)benzyloxy]phenyl}thiazol-2-ylmethoxy)nicotinic acid;
(303) [(4-{2-methyl-4-[4-(1-propylbutyl)benzyloxy]phenyl}thiazol-2-ylmethyl)phenylcarbamoylmethylamino]acetic acid;
(304) [[(4-isopropylphenylcarbamoyl)methyl]-(4-{2-methyl-4-[4-(1-propylbutyl)benzyloxy]phenyl}thiazol-2-ylmethyl)amino]acetic acid;
(305) 2-(4-{4-[4-(1-propylbutyl)benzyloxy]phenyl}thiazol-2-ylmethyl)-1,2,3,4-tetrahydroisoquinoline-3-carboxylic acid;
(306) [(4-{3-methoxy-4-[4-(1-propylbutyl)benzyloxy]phenyl}thiazol-2-ylmethyl)phenylcarbamoylmethylamino]acetic acid;
(307) [[(4-isopropylphenylcarbamoyl)methyl]-(4-{3-methoxy-4-[4-(1-propylbutyl)benzyloxy]phenyl}thiazol-2-ylmethyl)amino]acetic acid;
(308) [(4-{2-methyl-4-[4-(1-propylbutyl)benzyloxy]phenyl}thiazol-2-ylmethyl)thiazol-4-ylmethylamino]acetic acid;
(309) [(4-{2-methyl-4-[4-(1-propylbutyl)benzyloxy]phenyl}thiazol-2-ylmethyl)-(2-methylthiazol-4-ylmethyl)amino]acetic acid hydrochloride;
(310) [(benzylcarbamoylmethyl)-(4-{2-methyl-4-[4-(1-propylbutyl)benzyloxy]phenyl}thiazol-2-ylmethyl)amino]acetic acid hydrochloride;
(311) [(1H-benzimidazol-2-ylmethyl)-(4-{2-methyl-4-[4-(1-propylbutyl)benzyloxy]phenyl}thiazol-2-ylmethyl)amino]acetic acid;
(312) [(4-tert-butylthiazol-2-ylmethyl)-(4-{1-[4-(1-propylbutyl)benzyl]-1,2,3,4-tetrahydroquinolin-6-yl}thiazol-2-ylmethyl)amino]acetic acid;
(313) 1-(4-{4-[4-(1-propylbutyl)benzyloxy]phenyl}thiazol-2-ylmethyl)piperidine-4-carboxylic acid hydrochloride;
(314) 4-[4-(4-{4-[4-(1-propylbutyl)benzyloxy]phenyl}thiazol-2-ylmethyl)piperazin-1-yl]benzoic acid;
(315) 4-{4-[5-(1-ethylpropyl)-1-(4-isopropylbenzyl)-1H-benzimidazol-2-yl]thiazol-2-ylmethoxy}benzoic acid ethyl ester;
(316) 4-{4-[5-(1-ethylpropyl)-1-(4-isopropylbenzyl)-1H-benzimidazol-2-yl]thiazol-2-ylmethoxy}benzoic acid;
(317) 4-{4-[1-(4-acetylbenzyl)-5-(1-ethylpropyl)-1H-benzimidazol-2-yl]thiazol-2-ylmethoxy}benzoic acid;
(318) 4-{4-[1-(4-acetylbenzyl)-6-(1-ethylpropyl)-1H-benzimidazol-2-yl]thiazol-2-ylmethoxy}benzoic acid;
(319) 4-{4-[1-cyclohexylmethyl-5-(1-ethylpropyl)-1H-benzimidazol-2-yl]thiazol-2-ylmethoxy}benzoic acid;
(320) [(1-methyl-1H-benzimidazol-2-ylmethyl)-(5-{4-[4-(1-propylbutyl)phenoxymethyl]phenyl}thiophen-2-ylmethyl)amino]acetic acid;
(321) 5-(4-{4-[4-(1-propylbutyl)phenoxymethyl]phenyl}thiophen-2-ylmethoxy)nicotinic acid;
(322) 5-{4-[4-({isopropyl[4-(1-propylbutyl)phenyl]amino}methyl)phenyl]thiophen-2-ylmethoxy}nicotinic acid;
(323) 5-{4-[4-({[4-(1-ethylpropyl)phenyl]isopropylamino}methyl)phenyl]thiophen-2-ylmethoxy}nicotinic acid;
(324) 5-(5-{4-[4-(1-propylbutyl)phenoxymethyl]phenyl}thiophen-2-ylmethoxy)nicotinic acid;
(325) [(1H-benzimidazol-2-ylmethyl)-(4-{4-[4-(1-propylbutyl)phenoxymethyl]phenyl}thiophen-2-ylmethyl)amino]acetic acid;
(326) 6-(4-{4-[4-(1-propylbutyl)phenoxymethyl]phenyl}thiophen-2-ylmethoxy)pyridine-2-carboxylic acid;
(327) [(1H-benzimidazol-2-ylmethyl)-(5-{4-[4-(1-propylbutyl)phenoxymethyl]phenyl}thiophen-2-ylmethyl)amino]acetic acid;
(328) [[(4-isopropylphenylcarbamoyl)methyl]-(5-{4-[4-(1-propylbutyl)phenoxymethyl]phenyl}thiophen-2-ylmethyl)amino]acetic acid;
(329) [phenylcarbamoylmethyl(5-{4-[4-(1-propylbutyl)phenoxymethyl]phenyl}thiophen-2-ylmethyl)amino]acetic acid;
(330) ({4-[4-({[4-(1-ethylpropyl)phenyl]isopropylamino}methyl)phenyl]thiophen-2-ylmethyl}phenylcarbamoylmethylamino)acetic acid;
(331) ((4-tert-butylthiazol-2-ylmethyl)-{4-[4-({[4-(1-ethylpropyl)phenyl]isopropylamino}methyl)phenyl]thiophen-2-ylmethyl}amino)acetic acid;
(332) [(5-tert-butylthiazol-2-ylmethyl)-(4-{4-[4-(1-propylbutyl)phenoxymethyl]phenyl}thiophen-2-ylmethyl)-amino]acetic acid;
(333) ((1H-benzimidazol-2-ylmethyl)-{4-[4-({[4-(1-ethylpropyl)phenyl]isopropylamino}methyl)phenyl]thiophen-2-ylmethyl}amino)acetic acid;
(334) [(4-tert-butylthiazol-2-ylmethyl)-(4-{4-[4-(1-propylbutyl)phenoxymethyl]phenyl}thiophen-2-ylmethyl)amino]acetic acid;
(335) [phenylcarbamoylmethyl(4-{4-[4-(1-propylbutyl)phenoxymethyl]phenyl}thiophen-2-ylmethyl)amino]acetic acid;
(336) 5-{5-[4-({[4-(1-ethylpropyl)phenyl]isopropylamino}methyl)phenyl]thiophen-2-ylmethoxy}nicotinic acid;
(337) 5-{5-[4-({isopropyl[4-(1-propylbutyl)phenyl]amino}methyl)phenyl]thiophen-2-ylmethoxy}nicotinic acid;
(338) ((4-tert-butylthiazol-2-ylmethyl)-{5-[4-({[4-(1-ethylpropyl)phenyl]isopropylamino}methyl)phenyl]thiophen-2-ylmethyl}amino)acetic acid;
(339) ((4-tert-butylthiazol-2-ylmethyl)-{5-[4-({isopropyl[4-(1-propylbutyl)phenyl]amino}methyl)phenyl]thiophen-2-ylmethyl}amino)acetic acid;
(340) ((1H-benzimidazol-2-ylmethyl)-{5-[4-({[4-(1-ethylpropyl)phenyl]isopropylamino}methyl)phenyl]thiophen-2-ylmethyl}amino)acetic acid;
(341) ((1H-benzimidazol-2-ylmethyl)-{5-[4-({isopropyl[4-(1-propylbutyl)phenyl]amino}methyl)phenyl]thiophen-2-ylmethyl}amino)acetic acid;
(342) [(4,5-dimethylthiazol-2-ylmethyl)- (4-{4-[4-(1-propylbutyl)phenoxymethyl]phenyl}thiophen-2-ylmethyl)amino]acetic acid;
(343) (S)-2-{4-[4-({[4-(1-ethylpropyl)phenyl]isopropylamino}methyl)phenyl]thiophen-2-ylmethyl}-1,2,3,4-tetrahydroisoquinoline-3-carboxylic acid;
(344) {{4-[4-({[4-(1-ethylpropyl)phenyl]isopropylamino}methyl)phenyl]thiophen-2-ylmethyl}-[(4-isopropylphenylcarbamoyl)methyl]amino}acetic acid;
(345) (S)-2-(5-{4-[4-(1-propylbutyl)phenoxymethyl]phenyl}thiophen-2-ylmethyl)-1,2,3,4-tetrahydroisoquinoline-3-carboxylic acid;
(346) [[(4-isopropylphenylcarbamoyl)methyl]-(4-{4-[4-(1-propylbutyl)phenoxymethyl]phenyl}thiophen-2-ylmethyl)amino]acetic acid;
(347) (S)-2-{5-[4-({[4-(1-ethylpropyl)phenyl]isopropylamino}methyl)phenyl]thiophen-2-ylmethyl}-1,2,3,4-tetrahydroisoquinoline-3-carboxylic acid;
(348) [(5-tert-butyloxazol-2-ylmethyl)-(4-{4-[4-(1-propylbutyl)phenoxymethyl]phenyl}thiophen-2-ylmethyl)amino]acetic acid;
(349) [(1-methyl-1H-benzimidazol-2-ylmethyl)-(4-{4-[4-(1-propylbutyl)phenoxymethyl]phenyl}thiophen-2-ylmethyl)amino]acetic acid;
(350) 6-(5-{4-[4-(1-propylbutyl)phenoxymethyl]phenyl}thiophen-2-ylmethoxy)pyridine-2-carboxylic acid;
(351) 6-{4-[4-({[4-(1-ethylpropyl)phenyl]isopropylamino}methyl)phenyl]thiophen-2-ylmethoxy}-pyridine-2-carboxylic acid;
(352) ((5-tert-butylthiazol-2-ylmethyl)-{4-[4-({[4-(1-ethylpropyl)phenyl]isopropylamino}methyl)phenyl]thiophen-2-ylmethyl}amino)acetic acid;
(353) [{4-[4-({[4-(1-ethylpropyl)phenyl]isopropylamino}methyl)phenyl]thiophen-2-ylmethyl}-(1-methyl-1H-benzimidazol-2-ylmethyl)amino]acetic acid;
(354) [(4-tert-butylthiazol-2-ylmethyl)-(5-{4-[4-(1-propylbutyl)phenoxymethyl]phenyl}thiophen-2-ylmethyl)amino]acetic acid;
(355) [{5-[4-({[4-(1-ethylpropyl)phenyl]isopropylamino}methyl)phenyl]thiophen-2-ylmethyl}-(1-methyl-1H-benzimidazol-2-ylmethyl)amino]acetic acid;
(356) [(5-tert-butylthiazol-2-ylmethyl)-(5-{4-[4-(1-propylbutyl)phenoxymethyl]phenyl}thiophen-2-ylmethyl)amino]acetic acid;
(357) sodium [(1-methyl-1H-benzimidazol-2-ylmethyl)-(5-{4-[4-(1-propylbutyl)phenoxymethyl]phenyl}thiophen-2-ylmethyl)amino]-acetate;
(358) calcium bis{[(1-methyl-1H-benzimidazol-2-ylmethyl)-(5-{4-[4-(1-propylbutyl)phenoxymethyl]phenyl}thiophen-2-ylmethyl)amino]-acetate};
(359) [(1-methyl-1H-benzimidazol-2-ylmethyl)-(5-{4-[4-(1-propylbutyl)phenoxymethyl]phenyl}thiophen-2-ylmethyl)amino]acetic acid toluene-4-sulfonate;
(360) 5-(4-{4-[4-(1-propylbutyl)phenoxymethyl]phenyl}thiophen-2-ylmethoxy)nicotinic acid sulfate;
(361) [(1-methyl-1H-benzimidazol-2-ylmethyl)-(5-{4-[4-(1-propylbutyl)phenoxymethyl]phenyl}thiophene-2-carbonyl)amino]acetic acid;
(362) [(5-tert-butylthiazol-2-ylmethyl)-(5-{4-[4-(1-propylbutyl)phenoxymethyl]phenyl}thiophene-2-carbonyl)amino]acetic acid;
(363) [(4-isopropylbenzyl)-(5-{4-[4-(1-propylbutyl)phenoxymethyl]phenyl}thiophene-2-carbonyl)amino]acetic acid;
(364) [(4-dimethylaminobenzyl)-(5-{4-[4-(1-propylbutyl)phenoxymethyl]phenyl)thiophene-2-carbonyl)amino]acetic acid;
(365) [(5-{4-[4-(1-propylbutyl)phenoxymethyl]phenyl}thiophene-2-carbonyl)-pyridin-2-ylmethylamino]acetic acid;
(366) [(5-{4-[4-(1-propylbutyl)phenoxymethyl]phenyl}thiophene-2-carbonyl)-pyridin-3-ylmethylamino]acetic acid;
(367) 5-(4-{4-[4-(1-propylbutyl)phenoxymethyl]phenyl}thiophen-2-ylmethoxy)nicotinic acid methanesulfonate;
(368) [methanesulfonyl-(5-{4-[4-(1-propylbutyl)phenoxymethyl]phenyl}thiophen-2-ylmethyl)amino]acetic acid;
(369) sodium 5-(4-(4-[4-(1-propylbutyl)phenoxymethyl]phenyl)thiophen-2-ylmethoxy)nicotinate;
(370) 5-(4-{4-[4-(1-propylbutyl)phenoxymethyl]phenyl}thiophen-2-ylmethoxy)nicotinic acid hydrochloride;
(371) 4-[4-(5-{4-[4-(1-propylbutyl)phenoxymethyl]phenyl}thiophene-2-carbonyl)piperazin-1-yl]benzoic acid;
(372) 4-[1-(5-{4-[4-(1-propylbutyl)phenoxymethyl]phenyl}thiophene-2-carbonyl)piperidin-4-yl]benzoic acid;
(373) [(1-methyl-1H-benzimidazol-2-ylmethyl)-(4-{4-[4-(1-propylbutyl)phenoxymethyl]phenyl}thiophen-2-ylmethyl)amino]acetic acid hydrochloride;
(374) [(1-methyl-1H-benzimidazol-2-ylmethyl)-(4-{4-[4-(1-propylbutyl)phenoxymethyl]phenyl}thiophen-2-ylmethyl)amino]acetic acid sulfate;
(375) 4-(2-dimethylaminoacetylamino)-3-(4-{4-[4-(1-propylbutyl)phenoxymethyl]phenyl}thiophen-2-ylmethoxy)benzoic acid;
(376) 4-isobutyrylamino-3-(4-{4-[4-(1-propylbutyl)phenoxymethyl]phenyl}thiophen-2-ylmethoxy)benzoic acid;
(377) 4-(4-{4-[4-(1-propylbutyl)phenoxymethyl]phenyl}thiophen-2-ylmethoxy)benzoic acid;
(378) 4-(methanesulfonylmethylamino)-3-(4-{4-[4-(1-propylbutyl)phenoxymethyl]phenyl}thiophen-2-ylmethoxy)benzoic acid;
(379) 4-(4-{4-[4-(1-propylbutyl)phenoxymethyl]phenyl}thiophen-2-ylmethylthio)benzoic acid;
(380) 4-amino-3-(4-{4-[4-(1-propylbutyl)phenoxymethyl]phenyl}thiophen-2-ylmethoxy)benzoic acid;
(381) 1-{5-[1-(4-cyclohexylbenzyl)-1H-indol-3-yl]thiophen-2-ylmethyl}-4-phenylpiperidine-4-carboxylic acid hydrochloride;
(382) (benzyl{5-[1-(4-cyclohexylbenzyl)-1H-indol-3-yl]thiophen-2-ylmethyl}amino)acetic acid hydrochloride;
(383) [(methylphenylsulfamoyl)(4-{4-[4-(1-propylbutyl)phenoxymethyl]phenyl)thiophen-2-ylmethyl)amino]acetic acid;
(384) [(5-tert-butylthiazol-2-ylmethyl)-(5-{4-[(4-cyclohexylphenyl)methylcarbamoyl]phenyl}thiophen-2-ylmethyl)amino]acetic acid;
(385) [(5-{4-[(4-cyclohexylbenzyl)ethylamino]phenyl}thiophen-2-ylmethyl)pyridin-2-ylmethylamino]acetic acid;
(386) [(5-{4-[(4-cyclohexylbenzyl)ethylamino]phenyl}thiophen-2-ylmethyl)-(2-phenoxyethyl)amino]acetic acid;
(387) 4-{1-[4-(4-{[trans-4-(4-tert-butylphenyl)-cyclohexylmethyl]ethylamino}phenyl)thiophen-2-ylmethyl]piperidin-4-yl}benzoic acid;
(388) [[2-(4-isopropylphenoxy)acetyl]-(4-{4-[4-(l-propylbutyl)phenoxymethyl]phenyl}thiophen-2-ylmethyl)amino]acetic acid;
(389) [(4-isopropylbenzoyl)-(4-{4-[4-(1-propylbutyl)phenoxymethyl]phenyl}thiophen-2-ylmethyl)amino]acetic acid;
(390) [(3-methylbutyl)-(4-{4-[4-(1-propylbutyl)phenoxymethyl]phenyl}thiophen-2-ylmethyl)amino]acetic acid;
(391) [3-methyl-3-phenyl-1-(4-{4-[4-(1-propylbutyl)phenoxymethyl]phenyl}thiophen-2-ylmethyl)ureido]acetic acid;
(392) 2-(4-{3-[4-(1-propylbutyl)phenoxymethyl]phenyl}thiophen-2-ylmethyl)-1,2,3,4-tetrahydroisoquinoline-7-carboxylic acid;
(393) 4-[4-(5-{4-[4-(trans-4-methylcyclohexyl)benzyloxy]phenyl}thiophen-2-ylmethyl)piperazin-1-yl]benzoic acid;
(394) [(2-chloro-5-trifluoromethylbenzyl)-(5-{4-[4-(1-propylbutyl)phenoxymethyl]phenyl}thiophen-2-ylmethyl)amino]acetic acid;
(395) 3-{[carboxymethyl(5-{4-[4-(1-propylbutyl)phenoxymethyl]phenyl}thiophen-2-ylmethyl)amino]methyl}benzoic acid;
(396) [(4-methoxybenzyl)-(5-{4-[4-(1-propylbutyl)phenoxymethyl]phenyl}thiophen-2-ylmethyl)amino]acetic acid;
(397) [(4-methylthiobenzyl)-(5-{4-[4-(1-propylbutyl)phenoxymethyl]phenyl}thiophen-2-ylmethyl)amino]acetic acid;
(398) 4-[cyclohexylmethyl(5-{4-[4-(trans-4-methylcyclohexyl)benzyloxy]phenyl}thiophen-2-ylmethyl)sulfamoyl]benzoic acid;
(399) 4-[3-cyclohexylmethyl-3-(5-{4-[4-(trans-4-methylcyclohexyl)benzyloxy]phenyl}thiophen-2-ylmethyl)ureido]benzoic acid;
(400) [benzhydryl-(5-{4-[4-(1-propylbutyl)phenoxymethyl]phenyl}thiophen-2-ylmethyl)amino]acetic acid;
(401) [[2-oxo-2-(4-pyrrolidin-1-yl-phenyl)ethyl]-(5-{4-[4-(1-propylbutyl)phenoxymethyl]phenyl}thiophen-2-ylmethyl)amino]acetic acid ethyl ester hydrochloride;
(402) [(1-methyl-1H-benzimidazol-2-ylmethyl)-(5-{4-[4-(1-propylbutyl)phenoxymethyl]phenyl}thiophen-2-ylmethyl)amino]acetic acid ethyl ester;
(403) 3-(benzyl{5-[1-(4-cyclohexylbenzyl)-2,3-dihydro-1H-indol-5-yl]thiophen-2-ylmethyl}amino)propionic acid;
(404) [benzyl(4-{4-[2-(2,2-dimethylpropyl)benzimidazol-1-ylmethyl]phenyl}thiophen-2-ylmethyl)amino]acetic acid;
(405) [(1-methyl-1H-indol-3-ylmethyl)-(4-{4-[4-(1-propylbutyl)phenoxymethyl]phenyl}thiophen-2-ylmethyl)amino]acetic acid;
(406) [(4-{4-[4-(1-propylbutyl)phenoxymethyl]phenyl}thiophen-2-ylmethyl)quinolin-2-ylmethylamino]acetic acid;
(407) [benzothiazol-2-ylmethyl(4-{4-[4-(1-propylbutyl)phenoxymethyl]phenyl}thiophen-2-ylmethyl)amino]acetic acid;
(408) [(1-benzyl-1H-imidazol-2-ylmethyl)-(4-{4-[4-(1-propylbutyl)phenoxymethyl]phenyl}thiophen-2-ylmethyl)amino]acetic acid;
(409) [(1H-indol-5-ylmethyl)-(4-{4-[4-(1-propylbutyl)phenoxymethyl]phenyl}thiophen-2-ylmethyl)amino]acetic acid;
(410) [(4-imidazol-1-ylbenzyl)-(4-{4-[4-(1-propylbutyl)phenoxymethyl]phenyl}thiophen-2-ylmethyl)amino]acetic acid;
(411) [benzofuran-2-ylmethyl(4-{4-[4-(1-propylbutyl)phenoxymethyl]phenyl}thiophen-2-ylmethyl)amino]acetic acid;
(412) [[2,2']bithiophenyl-5-ylmethyl(4-{4-[4-(1-propylbutyl)phenoxymethyl]phenyl}thiophen-2-ylmethyl)amino]acetic acid;
(413) [(2-phenyl-1H-imidazol-4-ylmethyl)-(5-{4-[4-(1-propylbutyl)phenoxymethyl]phenyl}thiophen-2-ylmethyl)amino]acetic acid;
(414) [(3-phenyl-1H-pyrazol-4-ylmethyl)-(5-{4-[4-(1-propylbutyl)phenoxymethyl]phenyl}thiophen-2-ylmethyl)amino]acetic acid;
(415) [benzoxazol-2-ylmethyl(5-{4-[4-(1-propylbutyl)phenoxymethyl)phenyl}thiophen-2-ylmethyl)amino]acetic acid;
(416) [benzo[b]thiophen-2-ylmethyl(5-{4-[4-(1-propylbutyl)phenoxymethyl]phenyl}thiophen-2-ylmethyl)amino]acetic acid;
(417) [(4-phenylthiophen-2-ylmethyl)-(5-{4-[4-(1-propylbutyl)phenoxymethyl]phenyl}thiophen-2-ylmethyl)amino]acetic acid;
(418) [benzothiazol-2-yl-(5-{4-[4-(1-propylbutyl)phenoxymethyl]phenyl}thiophen-2-ylmethyl)amino]acetic acid;
(419) [(5-chlorothiophene-2-sulfonyl)-(5-{4-[4-(1-propylbutyl)phenoxymethyl]phenyl}thiophen-2-ylmethyl)amino]acetic acid;
(420) [(1-diethylcarbamoylmethyl-1H-benzimidazol-2-ylmethyl)-(5-{4-[4-(1-propylbutyl)phenoxymethyl]phenyl}thiophen-2-ylmethyl)amino]acetic acid;
(421) (S)-({4-[2-(4-cyclohexylbenzyloxy)-5-methylphenyl]thiophen-2-ylmethyl}methylamino)-3-phenylpropionic acid;
(422) [benzyl(4-{4-[(4-butoxybenzenesulfonyl)ethylamino]phenyl}thiophen-2-ylmethyl)amino]acetic acid;
(423) N-{4-[2-(4-cyclohexylbenzyloxy)-5-methylphenyl]thiophen-2-ylmethyl}-N-(2-methylbenzothiazol-6-yl)oxamic acid;
(424) (benzyl{4-[4-(3,5-dichlorophenoxymethyl)phenyl]thiophen-2-ylmethyl}amino)acetic acid;
(425) [(1-allyl-1H-benzimidazol-2-ylmethyl)-(5-{4-[4-(1-propylbutyl)phenoxymethyl]phenyl}thiophen-2-ylmethyl)amino]acetic acid; and
(426) [[4-(4-chlorophenyl)thiazol-2-yl]-(5-{4-[4-(1-propylbutyl)phenoxymethyl]phenyl}thiophen-2-ylmethyl)amino]acetic acid.

24. The 5-membered heteroaromatic ring compound of any of claims 1 to 23, which is selected from the group consisting of the following compounds, or a prodrug thereof, or a pharmaceutically acceptable salt thereof:
(1) (S)-2-(4-{4-[4-(1-propylbutyl)phenoxymethyl]phenyl}thiazol-2-ylmethyl)-1,2,3,4-tetrahydroisoquinoline-3-carboxylic acid;
(2) {{4-[4-({[4-(1-ethylpropyl)phenyl]isopropylamino}methyl)phenyl]thiazol-2-ylmethyl}-[(4-isopropylphenylcarbamoyl)methyl]amino}acetic acid;
(3) 4-(3-isobutyl-3-{4-[4-({isopropyl[4-(1-propylbutyl)phenyl]amino}methyl)phenyl]thiazol-2-ylmethyl}ureido)benzoic acid;
(4) ({4-[4-({isobutyl[4-(1-propylbutyl)phenyl]amino}methyl)phenyl]thiazol-2-ylmethyl}phenylcarbamoylmethylamino)acetic acid;
(5) ([(4-isopropylphenylcarbamoyl)methyl]-{4-[4-({isopropyl[4-(1-propylbutyl)phenyl]amino}methyl)phenyl]thiazol-2-ylmethyl}amino)acetic acid;
(6) [(4-tert-butylthiazol-2-ylmethyl)-(4-{4-[4-(1-propylbutyl)phenoxymethyl]phenyl}thiazol-2-ylmethyl)amino]acetic acid;
(7) [(4,5-dimethylthiazol-2-ylmethyl)-(4-{4-[4-(1-propylbutyl)phenoxymethyl]phenyl}thiazol-2-ylmethyl)amino]acetic acid;
(8) [(5-tert-butylthiazol-2-ylmethyl)-(4-{4-[4-(1-propylbutyl)phenoxymethyl]phenyl}thiazol-2-ylmethyl)amino]acetic acid;
(9) [[2-(4-isopropylphenoxy)acetyl]-(4-{4-[4-(1-propylbutyl)phenoxymethyl]phenyl}thiazol-2-ylmethyl)amino]acetic acid;
(10) 4-[4-(4-{4-[4-(1-propylbutyl)phenoxymethyl]phenyl}thiazol-2-ylmethyl)piperazin-1-yl]benzoic acid;
(11) {benzyl[4-(4-{methyl[4-(1-propylbutyl)benzyl]amino}phenyl)thiazol-2-ylmethyl]amino}acetic acid;
(12) [(1-methyl-1H-benzimidazol-2-ylmethyl)-5-{4-[4-(1-propylbutyl)phenoxymethyl]phenyl}thiophen-2-ylmethyl)amino]acetic acid;
(13) 5-(4-{4-[4-(1-propylbutyl)phenoxymethyl]phenyl}thiophen-2-ylmethoxy)nicotinic acid;
(14) [(1H-benzimidazol-2-ylmethyl)-(4-{4-[4-(1-propylbutyl)phenoxymethyl]phenyl}thiophen-2-ylmethyl)amino]acetic acid;
(15) [(1H-benzimidazol-2-ylmethyl)-(5-{4-[4-(1-propylbutyl)phenoxymethyl]phenyl}thiophen-2-ylmethyl)amino]acetic acid;
(16) [[(4-isopropylphenylcarbamoyl)methyl]-(5-{4-[4-(1-propylbutyl)phenoxymethyl]phenyl}thiophen-2-ylmethyl)amino]acetic acid;
(17) (S)-2-{4-[4-({[4-(1-ethylpropyl)phenyl]isopropylamino}methyl)phenyl]thiophen-2-ylmethyl}-1,2,3,4-tetrahydroisoquinoline-3-carboxylic acid;
(18) {{4-[4-({[4-(1-ethylpropyl)phenyl]isopropylamino}methyl)phenyl]thiophen-2-ylmethyl}-[(4-isopropylphenylcarbamoyl)methyl]amino}acetic acid;
(19) [(1-methyl-1H-benzimidazol-2-ylmethyl)-(4-{4-[4-(1-propylbutyl)phenoxymethyl]phenyl}thiophen-2-ylmethyl)amino]acetic acid; and
(20) [(4-tert-butylthiazol-2-ylmethyl)-(5-{4-[4-(1-propylbutyl)phenoxymethyl]phenyl}thiophen-2-ylmethyl)amino]acetic acid.

25. A pharmaceutical composition comprising the 5-membered heteroaromatic ring compound of any of claims 1 to 24, or a prodrug thereof, or a pharmaceutically acceptable salt thereof and a pharmaceutically acceptable carrier.

26. A pharmaceutical composition for inhibition of a receptor tyrosine kinase negative regulator, which comprises a 5-membered heteroaromatic ring compound of any of claims 1 to 24, or a prodrug thereof, or a pharmaceutically acceptable salt thereof and a pharmaceutically acceptable carrier.

27. A pharmaceutical composition for inhibition of protein tyrosine phosphatase 1B, which comprises a 5-membered heteroaromatic ring compound of any of claims 1 to 24, or a prodrug thereof, or a pharmaceutically acceptable salt thereof and a pharmaceutically acceptable carrier.

28. A pharmaceutical composition for the prophylaxis or treatment of diabetes, which comprises a 5-membered heteroaromatic ring compound of any of claims 1 to 24, or a prodrug thereof, or a pharmaceutically acceptable salt thereof and a pharmaceutically acceptable carrier.

29. A pharmaceutical composition for the prophylaxis or treatment of hyperlipidemia, which comprises a 5-membered heteroaromatic ring compound of any of claims 1 to 24, or a prodrug thereof, or a pharmaceutically acceptable salt thereof and a pharmaceutically acceptable carrier.

30. A pharmaceutical composition for the prophylaxis or treatment of obesity, which comprises a 5-membered heteroaromatic ring compound of any of claims 1 to 24, or a prodrug thereof, or a pharmaceutically acceptable salt thereof and a pharmaceutically acceptable carrier.

31. A pharmaceutical composition for the prophylaxis or treatment of diabetic complications, which comprises a 5-membered heteroaromatic ring compound of any of claims 1 to 24, or a prodrug thereof, or a pharmaceutically acceptable salt thereof and a pharmaceutically acceptable carrier.

32. The pharmaceutical composition of claim 25, which is used in combination with a therapeutic agent for hyperlipidemia.

33. The pharmaceutical composition of claim 32, wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.

34. The pharmaceutical composition of claim 33, wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).

35. The pharmaceutical composition of claim 25, which is used in combination with a therapeutic agent for diabetes.

36. The pharmaceutical composition of claim 35, wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors,antioxidants, carnitine acetyltransferase stimulant, antidepressants, glucocorticoids, retilin, radical formation agonists and transketolase activators.

37. The pharmaceutical composition of claim 36, wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.

38. The pharmaceutical composition of claim 25, which is used in combination with a therapeutic agent for obesity.

39. The pharmaceutical composition of claim 38, wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.

40. The pharmaceutical composition of claim 39, wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.

41. The pharmaceutical composition of claim 25, which is used in combination with a therapeutic agent for hypertension.

42. The pharmaceutical composition of claim 41, wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.

43. The pharmaceutical composition of claim 42, wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.

44. The pharmaceutical composition of claim 25, which is used in combination with a therapeutic agent for thrombosis.

45. The pharmaceutical composition of claim 44, wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.

46. The pharmaceutical composition of claim 45, wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.

47. The pharmaceutical composition for the prophylaxis or treatment of diabetes according to claim 28, which is used in combination with a therapeutic agent for hyperlipidemia.

48. The pharmaceutical composition of claim 47, wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.

49. The pharmaceutical composition of claim 48, wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).

50. The pharmaceutical composition for the prophylaxis or treatment of diabetes according to claim 28, which is used in combination with a different therapeutic agent for diabetes.

51. The pharmaceutical composition of claim 50, wherein the different therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors,antioxidants, carnitine acetyltransferase stimulant, antidepressants, glucocorticoids, retilin, radical formation agonists and transketolase activators.

52. The pharmaceutical composition of claim 51, wherein the different therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.

53. The pharmaceutical composition for the prophylaxis or treatment of diabetes according to claim 28, which is used in combination with a therapeutic agent for obesity.

54. The pharmaceutical composition of claim 53, wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.

55. The pharmaceutical composition of claim 54, wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.

56. The pharmaceutical composition for the prophylaxis or treatment of diabetes according to claim 28, which is used in combination with a therapeutic agent for hypertension.

57. The pharmaceutical composition of claim 56, wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.

58. The pharmaceutical composition of claim 57, wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.

59. A pharmaceutical composition for the prophylaxis or treatment of diabetes according to claim 28, which is used in combination with a therapeutic agent for thrombosis.

60. The pharmaceutical composition of claim 59, wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.

61. The pharmaceutical composition of claim 60, wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.

62. A pharmaceutical composition for the prophylaxis or treatment of hyperlipidemia according to claim 29, which is used in combination with a different therapeutic agent for hyperlipidemia.

63. The pharmaceutical composition of claim 62, wherein the different therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.

64. The pharmaceutical composition of claim 63, wherein the different therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).

65. A pharmaceutical composition for the prophylaxis or treatment of hyperlipidemia according to claim 29, which is used in combination with a therapeutic agent for diabetes.

66. The pharmaceutical composition of claim 65, wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors,antioxidants, carnitine acetyltransferase stimulant, antidepressants, glucocorticoids, retilin, radical formation agonists and transketolase activators.

67. The pharmaceutical composition of claim 66, wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.

68. A pharmaceutical composition for the prophylaxis or treatment of hyperlipidemia according to claim 29, which is used in combination with a therapeutic agent for obesity.

69. The pharmaceutical composition of claim 68, wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.

70. The pharmaceutical composition of claim 69, wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.

71. A pharmaceutical composition for the prophylaxis or treatment of hyperlipidemia according to claim 29, which is used in combination with a therapeutic agent for hypertension.

72. The pharmaceutical composition of claim 71, wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alphal-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel.activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.

73. The pharmaceutical composition of claim 72, wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.

74. A pharmaceutical composition for the prophylaxis or treatment of hyperlipidemia according to claim 29, which is used in combination with a therapeutic agent for thrombosis.

75. The pharmaceutical composition of claim 74, wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.

76. The pharmaceutical composition of claim 75, wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.

77. A pharmaceutical composition for the prophylaxis or treatment of obesity according to claim 30, which is used in combination with a therapeutic agent for hyperlipidemia.

78. The pharmaceutical composition of claim 77, wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.

79. The pharmaceutical composition of claim 78, wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).

80. A pharmaceutical composition for the prophylaxis or treatment of obesity according to claim 30, which is used in combination with a therapeutic agent for diabetes.

81. The pharmaceutical composition of claim 80, wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors,antioxidants, carnitine acetyltransferase stimulant, antidepressants, glucocorticoids, retilin, radical formation agonists and transketolase activators.

82. The pharmaceutical composition of claim 81, wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.

83. A pharmaceutical composition for the prophylaxis or treatment of obesity according to claim 30, which is used in combination with a different therapeutic agent for obesity.

84. The pharmaceutical composition of claim 83, wherein the different therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.

85. The pharmaceutical composition of claim 84, wherein the different therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.

86. A pharmaceutical composition for the prophylaxis or treatment of obesity according to claim 30, which is used in combination with a therapeutic agent for hypertensiona.

87. The pharmaceutical composition of claim 86, wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alpha1-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.

88. The pharmaceutical composition of claim 87, wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.

89. A pharmaceutical composition for the prophylaxis or treatment of obesity according to claim 30, which is used in combination with a therapeutic agent for thrombosis.

90. The pharmaceutical composition of claim 89, wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.

91. The pharmaceutical composition of claim 90, wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.

92. A pharmaceutical composition for the prophylaxis or treatment of diabetic complications according to claim 31, which is used in combination with a therapeutic agent for hyperlipidemia.

93. The pharmaceutical composition of claim 92, wherein the therapeutic agent for hyperlipidemia is one or more pharmaceutical agents selected from the group consisting of HMG-CoA reductase inhibitors (statins), fibrates, TNFSF6 expression inhibitors, HDL-cholesterol increasing agents, ApoA1 expression enhancers, SPP1 (osteopontin) expression inhibitors, drugs acting on peroxisome proliferator-activated receptors (PPAR), PPAR-alpha agonists, lipase clearing factor stimulants, cholesterol antagonists, platelet aggregation antagonists, antioxidants, cholesterol biosynthesis inhibitors, LDL-receptor up-regulators, bile acid sequestrants, cholesterol absorption inhibitors and nicotinic acids.

94. The pharmaceutical composition of claim 93, wherein the therapeutic agent for hyperlipidemia is one or more pharmaceutical agents selected from the group consisting of lovastatin, pravastatin (eptastatin) sodium, fluvastatin (fluindostainin) sodium, rosuvastatin calcium, atorvastatin calcium, simvastatin (synvinolin), pitavastatin (itavastatin, nisvastatin) calcium, ronifibrate (ronifibrato), binifibrate (binifibrato), clinofibrate, ciprofibrate, clofibrate, etofibrate, fenofibrate, bezafibrate, gemfibrozil, acipimox, eicosapentaenoic acid (icosapent, icopenate, icosapentate) ethyl ester, probucol, policosanol, colesevelam hydrochloride, colestyramine (cholestyramine resin), colestipol hydrochloride, colestimide (colestilan), ezetimibe and niacin (nicotinic acid).

95. A pharmaceutical composition for the prophylaxis or treatment of diabetic complications according to claim 31, which is used in combination with a therapeutic agent for diabetes.

96. The pharmaceutical composition of claim 95, wherein the therapeutic agent for diabetes is one or more pharmaceutical agents selected from the group consisting of insulin secretagogues, biguanides, a-glucosidase inhibitors, insulin preparations, insulin analogs, insulin sensitivity enhancers, IL-11, anti-CD25 (IL-2 Receptor) agents, angiotensin (AT1) antagonists, angiotensin-converting enzyme (ACE) inhibitors, aldose reductase inhibitors, antioxidants, carnitine acetyltransferase stimulant, antidepressants, glucocorticoids, retilin, radical formation agonists and transketolase activators.

97. The pharmaceutical composition of claim 96, wherein the therapeutic agent for diabetes is one or more pharmaceutical agents selected from the group consisting of nateglinide, glimepiride, glibenclamide, gliclazide, acetohexamide, tolbutamide, glyclopyramide, tolazamide, glybuzole, glipizide, glibornuride, gliquidone, repaglinide, metformin hydrochloride, buformin hydrochloride, voglibose, acarbose, epalrestat, miglitol, insulin, pioglitazone hydrochloride, rosiglitazone maleate, chromium picolinate/biotin, V-411, recombinant human interleukin-11, dacliximab (daclizumab), losartan potassium, captopril, imidapril hydrochloride, alpha-lipoic acid, levacecarnine (acetyl-L-carnitine, levocarnitine acetyl) hydrochloride, captopril, retilin, verteporfin, benfotiamine and fluocinolone acetonide.

98. A pharmaceutical composition for the prophylaxis or treatment of diabetic complications according to claim 31, which is used in combination with a therapeutic agent for obesity.

99. The pharmaceutical composition of claim 98, wherein the therapeutic agent for obesity is one or more pharmaceutical agents selected from the group consisting of mazindol, lipase inhibitors, 5-HT/norepinephrine reuptake dual inhibitors, 5-HT reuptake inhibitors, supplements containing herbal ephedrine and caffeine, human chorionic gonadotropins, adrenoceptor agonists, methamphetamine, phentermine and amfepramone.

100. The pharmaceutical composition of claim 99, wherein the therapeutic agent for obesity is one or more pharmaceutical agents selected from the group consisting of mazindol, orlistat, sibutramine hydrochloride monohydrate, fluoxetine hydrochloride, chorionic gonadotropin (human), VNS therapy using NCP System, metaraminol, d-methamphetamine hydrochloride, phentermine, amfepramone hydrochloride (diethylpropion), benzfetamine hydrochloride and phendimetrazine tartrate.

101. A pharmaceutical composition for the prophylaxis or treatment of diabetic complications according to claim 31, which is used in combination with a therapeutic agent for hypertension.

102. The pharmaceutical composition of claim 101, wherein the therapeutic agent for hypertension is one or more pharmaceutical agents selected from the group consisting of thiazides, aldosterone antagonists, adrenergic neuron blockers, calcium channel blockers; dopamine D2 antagonists, beta-adrenoceptor antagonists, alpha2-adrenoceptor agonists, guanylate cyclase activators, beta1-adrenoceptor antagonists, alphal-adrenoceptor antagonists, antioxidants, angiotensin-I converting enzyme (ACE) inhibitors, Na+/H+ exchange inhibitors, alpha-adrenoceptor antagonists, nitric oxide donors, 5-HT2 antagonists, K(ATP) channel activators, potassium sparing diuretic prostaglandin synthase stimulants, imidazoline I1 receptor agonists, angiotensin AT1 antagonists, dopamine D1 agonists, guanylate cyclase stimulants, endothelin ETA receptor antagonists, endothelin ETB receptor antagonists, NOS3 expression enhancers, prostacyclin analogs, prostaglandins, angiotensin II antagonists, electrolyte absorption agonists, nicotinic antagonists, dopamine D2 agonists, prolactin inhibitors, platelet-activating factor (PAF) antagonists, platelet aggregation antagonists, tumor necrosis factor antagonists, Rho kinase inhibitors, PPAR-alpha agonists, AMPA receptor modulators, GABA(A) receptor antagonists and phosphodiesterase V (PDE5A) inhibitors.

103. The pharmaceutical composition of claim 102, wherein the therapeutic agent for hypertension is one or more pharmaceutical agents selected from the group consisting of chlorothiazide, hydrochlorothiazide, hydroflumethiazide, methyclothiazide, polythiazide, xipamide, cyclopenthiazide, bendroflumethiazide (bendrofluazide), spironolactone, epoxymexrenone (eplerenone), guanethidine monosulfate, guanadrel sulfate, verapamil, propranolol hydrochloride, alprenolol hydrochloride, pindolol, oxprenolol hydrochloride, timolol maleate, sotalol hydrochloride, acebutolol hydrochloride, carteolol hydrochloride, mepindolol sulfate, arotinolol hydrochloride, indenolol hydrochloride, tertatolol hydrochloride, celiprolol hydrochloride, tilisolol hydrochloride, nebivolol, penbutolol sulfate, nadolol, cloranolol hydrochloride, bevantol (bevantolol) hydrochloride, clonidine, guanfacine hydrochloride, diltiazem hydrochloride, nicardipine hydrochloride, nitrendipine, felodipine, nilvadipine; nivadipine, nisoldipine, benidipine hydrochloride, amlodipine besylate, franidipine (manidipine) hydrochloride, lacidipine, isradipine, barnidipine (mepirodipine) hydrochloride, efonidipine hydrochloride ethanol, cinaldipine (cilnidipine), aranidipine, lercanidipine (masnidipine) hydrochloride, azelnidipine, amlodipine, manidipine (franidipine), sodium nitroprusside, atenolol, metoprolol tartrate, betaxolol hydrochloride, bopindolol, bisoprolol fumarate, esmolol hydrochloride, carvedilol, metoprolol succinate, talinolol, prazosin hydrochloride, urapidil, indoramin hydrochloride, bunazosin hydrochloride, terazosin hydrochloride, doxazosin mesylate, urapidil, nifedipine, captopril, enalapril maleate, lisinopril, perindopril, alacepril, ramipril, quinapril hydrochloride, delapril hydrochloride, benazepril hydrochloride, cilazapril, fosinoprilat, fosinopril sodium, trandolapril, spirapril, temocapril hydrochloride, moexipril hydrochloride, imidapril hydrochloride, zofenopril calcium, enalaprilat, zofenoprilat, amiloride hydrochloride, labetalol hydrochloride, nipradilol (nipradolol), linsidomine, ketanserin, pinacidil, cicletanine (cycletanide), amosulalol hydrochloride, moxonidine hydrochloride hydrate, losartan potassium, valsartan, eprosartan mesylate, candesartan cilexetil (hexetil), irbesartan, telmisartan, olmesartan medoxomil, fenoldopam mesilate, cadralazine, rilmenidine dihydrogen phosphate, bosentan, beraprost sodium, limaprost alfadex (alpha-cyclodextrin), uniprost (treprostinil sodium), iloprost (ciloprost), mecamylamine hydrochloride, metergoline, guanabenz acetate, cloricromene, fasudil, doconexent (docosahexaenoic acid), cyclothiazide, sildenafil citrate, chlortalidone (chlorthalidone), quinethazone, indapamide, metolazone, phenoxybenzamine hydrochloride, metirosine (metyrosine), diazoxide, torasemide (torsemide), clopamide, hydralazine hydrochloride, reserpine and methyldopa.

104. A pharmaceutical composition for the prophylaxis or treatment of diabetes according to claim 31, which is used in combination with a therapeutic agent for thrombosis.

105. The pharmaceutical composition of claim 104, wherein the therapeutic agent for thrombosis is one or more pharmaceutical agents selected from the group consisting of heparin preparations, low molecular weight heparins, heparin analogs, anticoagulants, thrombin inhibitors, anti-thrombin preparations, antiplatelet agents and thrombolytic agents.

106. The pharmaceutical composition of claim 105, wherein the therapeutic agent for thrombosis is one or more pharmaceutical agents selected from the group consisting of heparin calcium, heparin sodium, dalteparin sodium, parnaparin sodium, reviparin sodium, danaparoid sodium, warfarin potassium, argatroban, gabexate mesylate, nafamostat mesylate, human anti-thrombin III, aspirin, dipyridamole, ticlopidine hydrochloride, cilostazol, limaprost alfadex, sodium ozagrel, sarpogrelate hydrochloride, ethyl icosapentate, beraprost sodium, urokinase, tisokinase, alteplase, nasaruplase, nateplase, monteplase, pamiteplase, batroxobin, sodium citrate and protein C.

107. A pharmaceutical composition for inhibition of a receptor tyrosine kinase negative regulator, which is used in combination with a therapeutic agent for hyperlipidemia.

108. A pharmaceutical composition for inhibition of a receptor tyrosine kinase negative regulator, which is used in combination with a therapeutic agent for diabetes.

109. A pharmaceutical composition for inhibition of a receptor tyrosine kinase negative regulator, which is used in combination with a therapeutic agent for obesity.

110. A pharmaceutical composition for inhibition of a receptor tyrosine kinase negative regulator, which is used in combination with a therapeutic agent for hypertension.

111. A pharmaceutical composition for inhibition of a receptor tyrosine kinase negative regulator, which is used in combination with a therapeutic agent for thrombosis.

112. A 5-membered heteroaromatic ring compound represented by the formula [II] 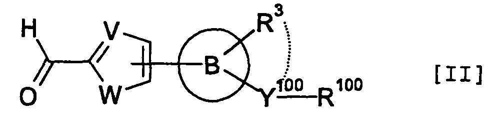 wherein Y¹⁰⁰ is -C(R¹³)(R¹⁴)- (R¹³ and R¹⁴ are each as defined in claim 1) ;
R¹⁰⁰ is a hydroxyl group or a halogen atom, and
V, W and R³ are each as defined in claim 1, or a pharmaceutically acceptable salt thereof.

113. The 5-membered heteroaromatic ring compound of claim 112,
wherein, in the formula [II], R¹³ and R¹⁴ are each a hydrogen atom, V is =CH- and W is -S-, or a pharmaceutically acceptable salt thereof.
